(19) Europäisches Patentamt
European Patent Office
Office européen des brevets

(11) **EP 4 026 918 A1**

(12) **EUROPEAN PATENT APPLICATION**

(43) Date of publication:
**13.07.2022 Bulletin 2022/28**

(21) Application number: **21151054.0**

(22) Date of filing: **12.01.2021**

(51) International Patent Classification (IPC):
***C12Q 1/6886*** *(2018.01)*

(52) Cooperative Patent Classification (CPC):
**C12Q 1/6886;** C12Q 2600/118; C12Q 2600/158

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **HOFFMANN, Ralf Dieter**
  **5656 AE Eindhoven (NL)**
- **STOFFELS, Monique**
  **5656 AE Eindhoven (NL)**

(74) Representative: **Philips Intellectual Property & Standards**
**High Tech Campus 52**
**5656 AG Eindhoven (NL)**

# (54) PREDICTION OF AN OUTCOME OF A COLORECTAL CANCER SUBJECT

(57) The invention relates to a method of predicting an outcome of a colorectal cancer subject, comprising determining or receiving the result of a determination of a first gene expression profile for each of one or more immune defense response genes, and/or of a second gene expression profile for each of one or more T-Cell receptor signaling genes, and/or of a third gene expression profile for each of one or more PDE4D7 correlated genes, said first, second, and third expression profile(s) being determined in a biological sample obtained from the subject, determining the prediction of outcome based on the first gene expression profile(s), or on the second gene expression profile(s), or on the third gene expression profile(s), or on the first, second, and third gene expression profile(s), and, optionally, providing the prediction to a medical caregiver or the subject.

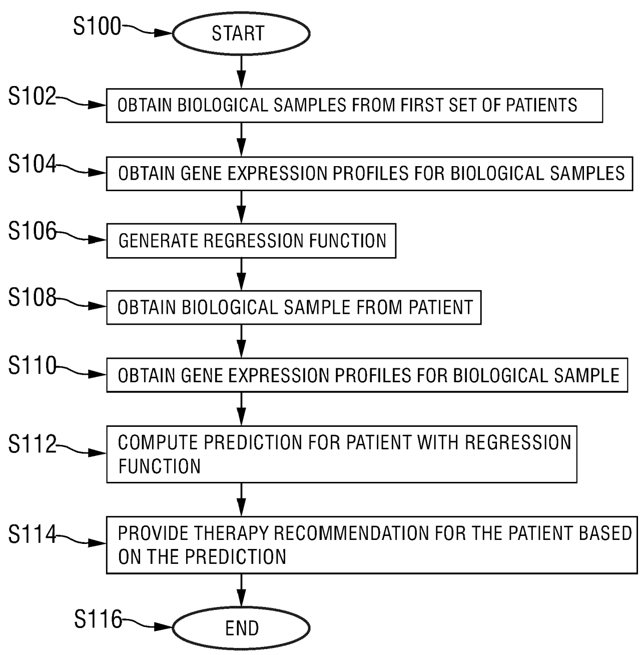


FIG. 1

EP 4 026 918 A1

SEQUENCE LISTING

<110> Koninklijke Philips N.V.

<120> Prediction of an outcome of a colorectal cancer subject

<130> 2020PF00762

<160> 166

<170> PatentIn version 3.5

<210> 1
<211> 2009
<212> DNA
<213> Homo sapiens

<400> 1
gggaatgctt tgtgcagcgc gcttgcgcgg tgtggcggcc gatgccgcta taaaggcttg 60

ttttgctgca gggctcatgc tcgggagcgt ggttgagcgg ctggcgcggt tgtcctggag 120

caggggcgca ggaattctga tgtgaaacta acagtctgtg agccctggaa cctccactca 180

gagaagatga aggatatcga cataggaaaa gagtatatca tccccagtcc tgggtataga 240

agtgtgaggg agagaaccag cacttctggg acgcacagag accgtgaaga ttccaagttc 300

aggagaactc gaccgttgga atgccaagat gccttggaaa cagcagcccg agccgagggc 360

ctctctcttg atgcctccat gcattctcag ctcagaatcc tggatgagga gcatcccaag 420

ggaaagtacc atcatggctt gagtgctctg aagcccatcc ggactacttc caaacaccag 480

cacccagtgg acaatgctgg gctttttcc tgtatgactt tttcgtggct ttcttctctg 540

gcccgtgtgg cccacaagaa gggggagctc tcaatggaag acgtgtggtc tctgtccaag 600

cacgagtctt ctgacgtgaa ctgcagaaga ctagagagac tgtggcaaga agagctgaat 660

gaagttgggc cagacgctgc ttccctgcga agggttgtgt ggatcttctg ccgcaccagg 720

ctcatcctgt ccatcgtgtg cctgatgatc acgcagctgg ctggcttcag tggaccaaat 780

tttcaggatg gctgtattct gcggtcagaa tgagagagtc aagctgggca gaatctctcg 840

ccaagagttc agccttcctt tggagactgc tccatcagtg ccgaggtgtg tgggaacagg 900

cttcactgca ccgccatctt actgagttgc ttcacgtgag gaaaaggggg ctttggccct 960

gtgactcagt tccacatttt ggattgcata ctggaaaaga agccaatctt cttgctagta 1020

aaccagcaac ccggctgtat acagtggtga cccaagcaat ggatataaac ctaaaaatct 1080

gagggagggg agaggtggaa tacagtagtt cttggaatct gaagtctcct atttgatcag 1140

gttatttcct gggacttggc aaaaatctga ttggtgggga tctcctagga cctagtggac 1200

atctggtatt aatttaatct caggaaaaac aagaaattaa cccagagaga gtctgggttt 1260

tggaattcag cgtagctacc tccagaccgt ggtgtctggc ctccattttt gtctgtcatt 1320

cagctctgac ttacagctgc agtcaccttt gctataaggc acctgggtag aagggtggat 1380

gggcttcaca tcaatttttt tcttccttta gggtggggga ttggtttggc tttcttttgt 1440

tgtggttttt tgttttattt ttgtcaagat tgatttttag atgcaaggac ttgaaaagac 1500

ccagaaggat gccaccagtt tttccttgag gcctaggatt ttttattctg tcccgagcag 1560

aggtaattcc tcacaactta gtgcaccagt agcaccagcc attttgagca gagtacctct 1620

ttggggagct tttcgttttg ttttgttttt aattctcttt ccttagcagc aaggtctttt 1680

ttcctagaga atctactccg ttgcagaatc attgcaacct caggagccct cactgattga 1740

gtgctgtcag cctgatatac tactttggac tctggaaaca gatatgggtt ctattctcta 1800

tttctactgt gtgtcgttaa acaaccgtcg gagaccagat gacctgttag atggctagtc 1860

ctgtataact cgactctgta tgtttcaatg tatgttactg caatgcttca cctgctgtac 1920

agtgtttgtg agatgctctt tgaagatggt acttttatat tttttcattt tcaataaaag 1980

tacattcctt cccacaaaaa aaaaaaaaa 2009

<210> 2
<211> 5872
<212> DNA
<213> Homo sapiens

<400> 2
gggaatgctt tgtgcagcgc gcttgcgcgg tgtggcggcc gatgccgcta taaaggcttg 60

ttttgctgca gggctcatgc tcgggagcgt ggttgagcgg ctggcgcggt tgtcctggag 120

caggggcgca ggaattctga tgtgaaacta acagtctgtg agccctggaa cctccactca 180

gagaagatga aggatatcga cataggaaaa gagtatatca tccccagtcc tgggtataga 240

agtgtgaggg agagaaccag cacttctggg acgcacagag accgtgaaga ttccaagttc 300

aggagaactc gaccgttgga atgccaagat gccttggaaa cagcagcccg agccgagggc 360

ctctctcttg atgcctccat gcattctcag ctcagaatcc tggatgagga gcatcccaag 420

ggaaagtacc atcatggctt gagtgctctg aagcccatcc ggactacttc caaacaccag 480

cacccagtgg acaatgctgg gctttttttcc tgtatgactt tttcgtggct ttcttctctg 540

gcccgtgtgg cccacaagaa gggggagctc tcaatggaag acgtgtggtc tctgtccaag 600

cacgagtctt ctgacgtgaa ctgcagaaga ctagagagac tgtggcaaga agagctgaat 660

gaagttgggc cagacgctgc ttccctgcga agggttgtgt ggatcttctg ccgcaccagg 720

ctcatcctgt ccatcgtgtg cctgatgatc acgcagctgg ctggcttcag tggaccagcc 780

ttcatggtga aacacctctt ggagtatacc caggcaacag agtctaacct gcagtacagc 840

ttgttgttag tgctgggcct cctcctgacg gaaatcgtgc ggtcttggtc gcttgcactg 900

acttgggcat tgaattaccg aaccggtgtc cgcttgcggg gggccatcct aaccatggca 960

tttaagaaga tccttaagtt aaagaacatt aaagagaaat ccctgggtga gctcatcaac 1020

atttgctcca acgatgggca gagaatgttt gaggcagcag ccgttggcag cctgctggct 1080

ggaggacccg ttgttgccat cttaggcatg atttataatg taattattct gggaccaaca 1140

ggcttcctgg gatcagctgt ttttatcctc ttttacccag caatgatgtt tgcatcacgg 1200

ctcacagcat atttcaggag aaaatgcgtg gccgccacgg atgaacgtgt ccagaagatg 1260

aatgaagttc ttacttacat taaatttatc aaaatgtatg cctgggtcaa agcattttct 1320

cagagtgttc aaaaaatccg cgaggaggag cgtcggatat tggaaaaagc tgggtacttc 1380

cagagcatca ctgtgggtgt ggctcccatt gtggtggtga ttgccagcgt ggtgaccttc 1440

tctgttcata tgaccctggg cttcgatctg acagcagcac aggctttcac agtggtgaca 1500

gtcttcaatt ccatgacttt tgctttgaaa gtaacaccgt tttcagtaaa gtccctctca 1560

gaagcctcag tggctgttga cagatttaag agtttgtttc taatggaaga ggttcacatg 1620

ataaagaaca aaccagccag tcctcacatc aagatagaga tgaaaaatgc caccttggca 1680

tgggactcct cccactccag tatccagaac tcgcccaagc tgacccccaa aatgaaaaaa 1740

gacaagaggg cttccagggg caagaaagag aaggtgaggc agctgcagcg cactgagcat 1800

caggcggtgc tggcagagca gaaaggccac ctcctcctgg acagtgacga gcggcccagt 1860

cccgaagagg aagaaggcaa gcacatccac ctgggccacc tgcgcttaca gaggacactg 1920

cacagcatcg atctggagat ccaagagggt aaactggttg gaatctgtgg cagtgtggga 1980

agtggaaaaa cctctctcat ttcagccatt ttaggccaga tgacgcttct agagggcagc 2040

attgcaatca gtggaacctt cgcttatgtg gcccagcagg cctggatcct caatgctact 2100

ctgagagaca acatcctgtt tgggaaggaa tatgatgaag aaagatacaa ctctgtgctg 2160

aacagctgct gcctgaggcc tgacctggcc attcttccca gcagcgacct gacggagatt 2220

ggagagcgag gagccaacct gagcggtggg cagcgccaga ggatcagcct tgcccgggcc 2280

ttgtatagtg acaggagcat ctacatcctg gacgaccccc tcagtgcctt agatgcccat 2340

gtgggcaacc acatcttcaa tagtgctatc cggaaacatc tcaagtccaa gacagttctg 2400

tttgttaccc accagttaca gtacctggtt gactgtgatg aagtgatctt catgaaagag 2460

ggctgtatta cggaaagagg cacccatgag gaactgatga atttaaatgg tgactatgct 2520

accattttta ataacctgtt gctgggagag acaccgccag ttgagatcaa ttcaaaaaag 2580

gaaaccagtg gttcacagaa gaagtcacaa gacaagggtc ctaaaacagg atcagtaaag 2640

aaggaaaaag cagtaaagcc agaggaaggg cagcttgtgc agctggaaga gaaagggcag 2700

ggttcagtgc cctggtcagt atatggtgtc tacatccagg ctgctggggg ccccttggca 2760

ttcctggtta ttatggccct tttcatgctg aatgtaggca gcaccgcctt cagcacctgg 2820

tggttgagtt actggatcaa gcaaggaagc gggaacacca ctgtgactcg agggaacgag 2880

acctcggtga gtgacagcat gaaggacaat cctcatatgc agtactatgc cagcatctac 2940
gccctctcca tggcagtcat gctgatcctg aaagccattc gaggagttgt ctttgtcaag 3000
ggcacgctgc gagcttcctc ccggctgcat gacgagcttt tccgaaggat ccttcgaagc 3060
cctatgaagt tttttgacac gacccccaca gggaggattc tcaacaggtt ttccaaagac 3120
atggatgaag ttgacgtgcg gctgccgttc caggccgaga tgttcatcca gaacgttatc 3180
ctggtgttct tctgtgtggg aatgatcgca ggagtcttcc cgtggttcct tgtggcagtg 3240
gggcccttg tcatcctctt ttcagtcctg cacattgtct ccagggtcct gattcgggag 3300
ctgaagcgtc tggacaatat cacgcagtca cctttcctct cccacatcac gtccagcata 3360
cagggccttg ccaccatcca cgcctacaat aaagggcagg agtttctgca cagataccag 3420
gagctgctgg atgacaacca agctcctttt tttttgttta cgtgtgcgat gcggtggctg 3480
gctgtgcggc tggacctcat cagcatcgcc ctcatcacca ccacggggct gatgatcgtt 3540
cttatgcacg ggcagattcc cccagcctat gcgggtctcg ccatctctta tgctgtccag 3600
ttaacggggc tgttccagtt tacggtcaga ctggcatctg agacagaagc tcgattcacc 3660
tcggtggaga ggatcaatca ctacattaag actctgtcct tggaagcacc tgccagaatt 3720
aagaacaagg ctccctcccc tgactggccc caggagggag aggtgacctt tgagaacgca 3780
gagatgaggt accgagaaaa cctccctctc gtcctaaaga aagtatcctt cacgatcaaa 3840
cctaaagaga agattggcat tgtggggcgg acaggatcag ggaagtcctc gctggggatg 3900
gccctcttcc gtctggtgga gttatctgga ggctgcatca agattgatgg agtgagaatc 3960
agtgatattg gccttgccga cctccgaagc aaactctcta tcattcctca agagccggtg 4020
ctgttcagtg gcactgtcag atcaaatttg gaccccttca accagtacac tgaagaccag 4080
atttgggatg ccctggagag gacacacatg aaagaatgta ttgctcagct acctctgaaa 4140
cttgaatctg aagtgatgga gaatggggat aacttctcag tgggggaacg gcagctcttg 4200
tgcatagcta gagccctgct ccgccactgt aagattctga ttttagatga agccacagct 4260
gccatggaca cagagacaga cttattgatt caagagacca tccgagaagc atttgcagac 4320
tgtaccatgc tgaccattgc ccatcgcctg cacacggttc taggctccga taggattatg 4380
gtgctggccc agggacaggt ggtggagttt gacaccccat cggtccttct gtccaacgac 4440
agttcccgat tctatgccat gtttgctgct gcagagaaca aggtcgctgt caagggctga 4500
ctcctccctg ttgacgaagt ctcttttctt tagagcattg ccattccctg cctggggcgg 4560
gcccctcatc gcgtcctcct accgaaacct tgcctttctc gattttatct ttcgcacagc 4620
agttccggat tggcttgtgt gtttcacttt tagggagagt catattttga ttattgtatt 4680
tattccatat tcatgtaaac aaaatttagt ttttgttctt aattgcactc taaaaggttc 4740
agggaaccgt tattataatt gtatcagagg cctataatga agctttatac gtgtagctat 4800

atctatatat aattctgtac atagcctata tttacagtga aaatgtaagc tgtttatttt 4860

atattaaaat aagcactgtg ctaataacag tgcatattcc tttctatcat ttttgtacag 4920

tttgctgtac tagagatctg gttttgctat tagactgtag gaagagtagc atttcattct 4980

tctctagctg gtggtttcac ggtgccaggt tttctgggtg tccaaaggaa gacgtgtggc 5040

aatagtgggc cctccgacag ccccctctgc cgcctcccca cggccgctcc aggggtggct 5100

ggagacgggt gggcggctgg agaccatgca gagcgccgtg agttctcagg gctcctgcct 5160

tctgtcctgg tgtcacttac tgtttctgtc aggagagcag cggggcgaag cccaggcccc 5220

ttttcactcc ctccatcaag aatggggatc acagagacat tcctccgagc cggggagttt 5280

ctttcctgcc ttcttctttt tgctgttgtt tctaaacaag aatcagtcta tccacagaga 5340

gtcccactgc ctcaggttcc tatggctggc cactgcacag agctctccag ctccaagacc 5400

tgttggttcc aagccctgga gccaactgct gctttttgag gtggcacttt ttcatttgcc 5460

tattcccaca cctccacagt tcagtggcag ggctcaggat ttcgtgggtc tgttttcctt 5520

tctcaccgca gtcgtcgcac agtctctctc tctctctccc ctcaaagtct gcaactttaa 5580

gcagctcttg ctaatcagtg tctcacactg gcgtagaagt ttttgtactg taaagagacc 5640

tacctcaggt tgctggttgc tgtgtggttt ggtgtgttcc cgcaaacccc ctttgtgctg 5700

tggggctggt agctcaggtg ggcgtggtca ctgctgtcat caattgaatg gtcagcgttg 5760

catgtcgtga ccaactagac attctgtcgc cttagcatgt ttgctgaaca ccttgtggaa 5820

gcaaaaatct gaaaatgtga ataaaattat tttggatttt gtaaaaaaaa aa 5872

<210> 3
<211> 208
<212> PRT
<213> Homo sapiens

<400> 3

Met Lys Asp Ile Asp Ile Gly Lys Glu Tyr Ile Ile Pro Ser Pro Gly
1 5 10 15

Tyr Arg Ser Val Arg Glu Arg Thr Ser Thr Ser Gly Thr His Arg Asp
20 25 30

Arg Glu Asp Ser Lys Phe Arg Arg Thr Arg Pro Leu Glu Cys Gln Asp
35 40 45

Ala Leu Glu Thr Ala Ala Arg Ala Glu Gly Leu Ser Leu Asp Ala Ser
50 55 60

Met His Ser Gln Leu Arg Ile Leu Asp Glu Glu His Pro Lys Gly Lys
65 70 75 80

Tyr His His Gly Leu Ser Ala Leu Lys Pro Ile Arg Thr Thr Ser Lys
85 90 95

His Gln His Pro Val Asp Asn Ala Gly Leu Phe Ser Cys Met Thr Phe
100 105 110

Ser Trp Leu Ser Ser Leu Ala Arg Val Ala His Lys Lys Gly Glu Leu
115 120 125

Ser Met Glu Asp Val Trp Ser Leu Ser Lys His Glu Ser Ser Asp Val
130 135 140

Asn Cys Arg Arg Leu Glu Arg Leu Trp Gln Glu Glu Leu Asn Glu Val
145 150 155 160

Gly Pro Asp Ala Ala Ser Leu Arg Arg Val Val Trp Ile Phe Cys Arg
165 170 175

Thr Arg Leu Ile Leu Ser Ile Val Cys Leu Met Ile Thr Gln Leu Ala
180 185 190

Gly Phe Ser Gly Pro Asn Phe Gln Asp Gly Cys Ile Leu Arg Ser Glu
195 200 205

<210> 4
<211> 1437
<212> PRT
<213> Homo sapiens

<400> 4

Met Lys Asp Ile Asp Ile Gly Lys Glu Tyr Ile Ile Pro Ser Pro Gly
1 5 10 15

Tyr Arg Ser Val Arg Glu Arg Thr Ser Thr Ser Gly Thr His Arg Asp
20 25 30

Arg Glu Asp Ser Lys Phe Arg Arg Thr Arg Pro Leu Glu Cys Gln Asp
35 40 45

Ala Leu Glu Thr Ala Ala Arg Ala Glu Gly Leu Ser Leu Asp Ala Ser
50 55 60

Met His Ser Gln Leu Arg Ile Leu Asp Glu Glu His Pro Lys Gly Lys
65 70 75 80

Tyr His His Gly Leu Ser Ala Leu Lys Pro Ile Arg Thr Thr Ser Lys
85 90 95

His Gln His Pro Val Asp Asn Ala Gly Leu Phe Ser Cys Met Thr Phe
100 105 110

Ser Trp Leu Ser Ser Leu Ala Arg Val Ala His Lys Lys Gly Glu Leu
115 120 125

Ser Met Glu Asp Val Trp Ser Leu Ser Lys His Glu Ser Ser Asp Val
130 135 140

Asn Cys Arg Arg Leu Glu Arg Leu Trp Gln Glu Glu Leu Asn Glu Val
145 150 155 160

Gly Pro Asp Ala Ala Ser Leu Arg Arg Val Val Trp Ile Phe Cys Arg
165 170 175

Thr Arg Leu Ile Leu Ser Ile Val Cys Leu Met Ile Thr Gln Leu Ala
180 185 190

Gly Phe Ser Gly Pro Ala Phe Met Val Lys His Leu Leu Glu Tyr Thr
195 200 205

Gln Ala Thr Glu Ser Asn Leu Gln Tyr Ser Leu Leu Leu Val Leu Gly
210 215 220

Leu Leu Leu Thr Glu Ile Val Arg Ser Trp Ser Leu Ala Leu Thr Trp
225 230 235 240

Ala Leu Asn Tyr Arg Thr Gly Val Arg Leu Arg Gly Ala Ile Leu Thr
245 250 255

Met Ala Phe Lys Lys Ile Leu Lys Leu Lys Asn Ile Lys Glu Lys Ser
260 265 270

Leu Gly Glu Leu Ile Asn Ile Cys Ser Asn Asp Gly Gln Arg Met Phe
275 280 285

Glu Ala Ala Ala Val Gly Ser Leu Leu Ala Gly Gly Pro Val Val Ala
290 295 300

Ile Leu Gly Met Ile Tyr Asn Val Ile Ile Leu Gly Pro Thr Gly Phe
305 310 315 320

Leu Gly Ser Ala Val Phe Ile Leu Phe Tyr Pro Ala Met Met Phe Ala
325 330 335

Ser Arg Leu Thr Ala Tyr Phe Arg Arg Lys Cys Val Ala Ala Thr Asp

340 345 350

Glu Arg Val Gln Lys Met Asn Glu Val Leu Thr Tyr Ile Lys Phe Ile
355 360 365

Lys Met Tyr Ala Trp Val Lys Ala Phe Ser Gln Ser Val Gln Lys Ile
370 375 380

Arg Glu Glu Glu Arg Arg Ile Leu Glu Lys Ala Gly Tyr Phe Gln Ser
385 390 395 400

Ile Thr Val Gly Val Ala Pro Ile Val Val Val Ile Ala Ser Val Val
405 410 415

Thr Phe Ser Val His Met Thr Leu Gly Phe Asp Leu Thr Ala Ala Gln
420 425 430

Ala Phe Thr Val Val Thr Val Phe Asn Ser Met Thr Phe Ala Leu Lys
435 440 445

Val Thr Pro Phe Ser Val Lys Ser Leu Ser Glu Ala Ser Val Ala Val
450 455 460

Asp Arg Phe Lys Ser Leu Phe Leu Met Glu Glu Val His Met Ile Lys
465 470 475 480

Asn Lys Pro Ala Ser Pro His Ile Lys Ile Glu Met Lys Asn Ala Thr
485 490 495

Leu Ala Trp Asp Ser Ser His Ser Ser Ile Gln Asn Ser Pro Lys Leu
500 505 510

Thr Pro Lys Met Lys Lys Asp Lys Arg Ala Ser Arg Gly Lys Lys Glu
515 520 525

Lys Val Arg Gln Leu Gln Arg Thr Glu His Gln Ala Val Leu Ala Glu
530 535 540

Gln Lys Gly His Leu Leu Leu Asp Ser Asp Glu Arg Pro Ser Pro Glu
545 550 555 560

Glu Glu Glu Gly Lys His Ile His Leu Gly His Leu Arg Leu Gln Arg
565 570 575

Thr Leu His Ser Ile Asp Leu Glu Ile Gln Glu Gly Lys Leu Val Gly
580 585 590

Ile Cys Gly Ser Val Gly Ser Gly Lys Thr Ser Leu Ile Ser Ala Ile
595 600 605

Leu Gly Gln Met Thr Leu Leu Glu Gly Ser Ile Ala Ile Ser Gly Thr
610 615 620

Phe Ala Tyr Val Ala Gln Gln Ala Trp Ile Leu Asn Ala Thr Leu Arg
625 630 635 640

Asp Asn Ile Leu Phe Gly Lys Glu Tyr Asp Glu Glu Arg Tyr Asn Ser
645 650 655

Val Leu Asn Ser Cys Cys Leu Arg Pro Asp Leu Ala Ile Leu Pro Ser
660 665 670

Ser Asp Leu Thr Glu Ile Gly Glu Arg Gly Ala Asn Leu Ser Gly Gly
675 680 685

Gln Arg Gln Arg Ile Ser Leu Ala Arg Ala Leu Tyr Ser Asp Arg Ser
690 695 700

Ile Tyr Ile Leu Asp Asp Pro Leu Ser Ala Leu Asp Ala His Val Gly
705 710 715 720

Asn His Ile Phe Asn Ser Ala Ile Arg Lys His Leu Lys Ser Lys Thr
725 730 735

Val Leu Phe Val Thr His Gln Leu Gln Tyr Leu Val Asp Cys Asp Glu
740 745 750

Val Ile Phe Met Lys Glu Gly Cys Ile Thr Glu Arg Gly Thr His Glu
755 760 765

Glu Leu Met Asn Leu Asn Gly Asp Tyr Ala Thr Ile Phe Asn Asn Leu
770 775 780

Leu Leu Gly Glu Thr Pro Pro Val Glu Ile Asn Ser Lys Lys Glu Thr
785 790 795 800

Ser Gly Ser Gln Lys Lys Ser Gln Asp Lys Gly Pro Lys Thr Gly Ser
805 810 815

Val Lys Lys Glu Lys Ala Val Lys Pro Glu Glu Gly Gln Leu Val Gln
820 825 830

Leu Glu Glu Lys Gly Gln Gly Ser Val Pro Trp Ser Val Tyr Gly Val
835 840 845

Tyr Ile Gln Ala Ala Gly Gly Pro Leu Ala Phe Leu Val Ile Met Ala
850 855 860

Leu Phe Met Leu Asn Val Gly Ser Thr Ala Phe Ser Thr Trp Trp Leu
865 870 875 880

Ser Tyr Trp Ile Lys Gln Gly Ser Gly Asn Thr Thr Val Thr Arg Gly
885 890 895

Asn Glu Thr Ser Val Ser Asp Ser Met Lys Asp Asn Pro His Met Gln
900 905 910

Tyr Tyr Ala Ser Ile Tyr Ala Leu Ser Met Ala Val Met Leu Ile Leu
915 920 925

Lys Ala Ile Arg Gly Val Val Phe Val Lys Gly Thr Leu Arg Ala Ser
930 935 940

Ser Arg Leu His Asp Glu Leu Phe Arg Arg Ile Leu Arg Ser Pro Met
945 950 955 960

Lys Phe Phe Asp Thr Thr Pro Thr Gly Arg Ile Leu Asn Arg Phe Ser
965 970 975

Lys Asp Met Asp Glu Val Asp Val Arg Leu Pro Phe Gln Ala Glu Met
980 985 990

Phe Ile Gln Asn Val Ile Leu Val Phe Phe Cys Val Gly Met Ile Ala
995 1000 1005

Gly Val Phe Pro Trp Phe Leu Val Ala Val Gly Pro Leu Val Ile
1010 1015 1020

Leu Phe Ser Val Leu His Ile Val Ser Arg Val Leu Ile Arg Glu
1025 1030 1035

Leu Lys Arg Leu Asp Asn Ile Thr Gln Ser Pro Phe Leu Ser His
1040 1045 1050

Ile Thr Ser Ser Ile Gln Gly Leu Ala Thr Ile His Ala Tyr Asn
1055 1060 1065

Lys Gly Gln Glu Phe Leu His Arg Tyr Gln Glu Leu Leu Asp Asp
1070 1075 1080

Asn Gln Ala Pro Phe Phe Leu Phe Thr Cys Ala Met Arg Trp Leu
1085 1090 1095

Ala Val Arg Leu Asp Leu Ile Ser Ile Ala Leu Ile Thr Thr Thr
1100 1105 1110

Gly Leu Met Ile Val Leu Met His Gly Gln Ile Pro Pro Ala Tyr
1115 1120 1125

Ala Gly Leu Ala Ile Ser Tyr Ala Val Gln Leu Thr Gly Leu Phe
1130 1135 1140

Gln Phe Thr Val Arg Leu Ala Ser Glu Thr Glu Ala Arg Phe Thr
1145 1150 1155

Ser Val Glu Arg Ile Asn His Tyr Ile Lys Thr Leu Ser Leu Glu
1160 1165 1170

Ala Pro Ala Arg Ile Lys Asn Lys Ala Pro Ser Pro Asp Trp Pro
1175 1180 1185

Gln Glu Gly Glu Val Thr Phe Glu Asn Ala Glu Met Arg Tyr Arg
1190 1195 1200

Glu Asn Leu Pro Leu Val Leu Lys Lys Val Ser Phe Thr Ile Lys
1205 1210 1215

Pro Lys Glu Lys Ile Gly Ile Val Gly Arg Thr Gly Ser Gly Lys
1220 1225 1230

Ser Ser Leu Gly Met Ala Leu Phe Arg Leu Val Glu Leu Ser Gly
1235 1240 1245

Gly Cys Ile Lys Ile Asp Gly Val Arg Ile Ser Asp Ile Gly Leu
1250 1255 1260

Ala Asp Leu Arg Ser Lys Leu Ser Ile Ile Pro Gln Glu Pro Val
1265 1270 1275

Leu Phe Ser Gly Thr Val Arg Ser Asn Leu Asp Pro Phe Asn Gln
1280 1285 1290

Tyr Thr Glu Asp Gln Ile Trp Asp Ala Leu Glu Arg Thr His Met
1295 1300 1305

Lys Glu Cys Ile Ala Gln Leu Pro Leu Lys Leu Glu Ser Glu Val
1310 1315 1320

Met Glu Asn Gly Asp Asn Phe Ser Val Gly Glu Arg Gln Leu Leu

1325 1330 1335

Cys Ile Ala Arg Ala Leu Leu Arg His Cys Lys Ile Leu Ile Leu
1340 1345 1350

Asp Glu Ala Thr Ala Ala Met Asp Thr Glu Thr Asp Leu Leu Ile
1355 1360 1365

Gln Glu Thr Ile Arg Glu Ala Phe Ala Asp Cys Thr Met Leu Thr
1370 1375 1380

Ile Ala His Arg Leu His Thr Val Leu Gly Ser Asp Arg Ile Met
1385 1390 1395

Val Leu Ala Gln Gly Gln Val Val Glu Phe Asp Thr Pro Ser Val
1400 1405 1410

Leu Leu Ser Asn Asp Ser Ser Arg Phe Tyr Ala Met Phe Ala Ala
1415 1420 1425

Ala Glu Asn Lys Val Ala Val Lys Gly
1430 1435

<210> 5
<211> 1394
<212> DNA
<213> Homo sapiens

<400> 5
agaagtgtca gagtctttgt agctttgaaa gtcacctagg ttatttgggc atgctctcct 60

gagtcctctg ctagttaagc tctctgaaaa gaaggtggca gacccggttt gctgatcgcc 120

ccagggatca ggaggctgat cccaaagttg tcagatggag agtaaataca aggagatact 180

cttgctaaca ggcctggata acatcactga tgaggaactg gataggttta agttctttct 240

ttcagacgag tttaatattg ccacaggcaa actacatact gcaaacagaa tacaagtagc 300

taccttgatg attcaaaatg ctggggcggt gtctgcagtg atgaagacca ttcgtatttt 360

tcagaagttg aattatatgc ttttggcaaa acgtcttcag gaggagaagg agaaagttga 420

taagcaatac aaatcggtaa caaaaccaaa gccactaagt caagctgaaa tgagtcctgc 480

tgcatctgca gccatcagaa atgatgtcgc aaagcaacgt gctgcaccaa aagtctctcc 540

tcatgttaag cctgaacaga aacagatggt ggcccagcag gaatctatca gagaagggtt 600

tcagaagcgc tgtttgccag ttatggtact gaaagcaaag aagcccttca cgtttgagac 660

ccaagaaggc aagcaggaga tgtttcatgc tacagtggct acagaaaagg aattcttctt 720

tgtaaaagtt tttaatacac tgctgaaaga taaattcatt ccaaagagaa taattataat 780

agcaagatat tatcggcaca gtggtttctt agaggtaaat agcgcctcac gtgtgttaga 840

tgctgaatct gaccaaaagg ttaatgtccc gctgaacatt atcagaaaag ctggtgaaac 900

cccgaagatc aacacgcttc aaactcagcc ccttggaaca attgtgaatg gtttgtttgt 960

agtccagaag gtaacagaaa agaagaaaaa catattattt gacctaagtg acaacactgg 1020

gaaaatggaa gtactggggg ttagaaacga ggacacaatg aaatgtaagg aaggagataa 1080

ggttcgactt acattcttca cactgtcaaa aaatggagaa aaactacagc tgacatctgg 1140

agttcatagc accataaagg ttattaaggc caaaaaaaaa acatagagaa gtaaaaagga 1200

ccaattcaag ccaactggtc taagcagcat ttaattgaag aatatgtgat acagcctctt 1260

caatcagatt gtaagttacc tgaaagctgc agttcacagg ctcctctctc caccaaatta 1320

ggatagaata attgctggat aaacaaattc agaatatcaa cagatgatca caataaacat 1380

ctgtttctca ttca 1394

<210> 6
<211> 343
<212> PRT
<213> Homo sapiens

<400> 6

Met Glu Ser Lys Tyr Lys Glu Ile Leu Leu Leu Thr Gly Leu Asp Asn
1 5 10 15

Ile Thr Asp Glu Glu Leu Asp Arg Phe Lys Phe Phe Leu Ser Asp Glu
20 25 30

Phe Asn Ile Ala Thr Gly Lys Leu His Thr Ala Asn Arg Ile Gln Val
35 40 45

Ala Thr Leu Met Ile Gln Asn Ala Gly Ala Val Ser Ala Val Met Lys
50 55 60

Thr Ile Arg Ile Phe Gln Lys Leu Asn Tyr Met Leu Leu Ala Lys Arg
65 70 75 80

Leu Gln Glu Glu Lys Glu Lys Val Asp Lys Gln Tyr Lys Ser Val Thr
85 90 95

Lys Pro Lys Pro Leu Ser Gln Ala Glu Met Ser Pro Ala Ala Ser Ala
100 105 110

Ala Ile Arg Asn Asp Val Ala Lys Gln Arg Ala Ala Pro Lys Val Ser
115 120 125

Pro His Val Lys Pro Glu Gln Lys Gln Met Val Ala Gln Gln Glu Ser

130 135 140

Ile Arg Glu Gly Phe Gln Lys Arg Cys Leu Pro Val Met Val Leu Lys
145 150 155 160

Ala Lys Lys Pro Phe Thr Phe Glu Thr Gln Glu Gly Lys Gln Glu Met
165 170 175

Phe His Ala Thr Val Ala Thr Glu Lys Glu Phe Phe Phe Val Lys Val
180 185 190

Phe Asn Thr Leu Leu Lys Asp Lys Phe Ile Pro Lys Arg Ile Ile Ile
195 200 205

Ile Ala Arg Tyr Tyr Arg His Ser Gly Phe Leu Glu Val Asn Ser Ala
210 215 220

Ser Arg Val Leu Asp Ala Glu Ser Asp Gln Lys Val Asn Val Pro Leu
225 230 235 240

Asn Ile Ile Arg Lys Ala Gly Glu Thr Pro Lys Ile Asn Thr Leu Gln
245 250 255

Thr Gln Pro Leu Gly Thr Ile Val Asn Gly Leu Phe Val Val Gln Lys
260 265 270

Val Thr Glu Lys Lys Lys Asn Ile Leu Phe Asp Leu Ser Asp Asn Thr
275 280 285

Gly Lys Met Glu Val Leu Gly Val Arg Asn Glu Asp Thr Met Lys Cys
290 295 300

Lys Glu Gly Asp Lys Val Arg Leu Thr Phe Phe Thr Leu Ser Lys Asn
305 310 315 320

Gly Glu Lys Leu Gln Leu Thr Ser Gly Val His Ser Thr Ile Lys Val
325 330 335

Ile Lys Ala Lys Lys Lys Thr
340

<210> 7
<211> 1358
<212> DNA
<213> Homo sapiens

<400> 7
gttggtgaag atcttaacac cacgccttga gcaagtcgca agagcgggag gacacagacc 60

aggaaccgag aagggacaag cacatggaag ccagcccagc atccgggccc agacacttga 120

tggatccaca catattcact tccaacttta acaatggcat tggaaggcat aagacctacc 180

tgtgctacga agtggagcgc ctggacaatg gcacctcggt caagatggac cagcacaggg 240

gctttctaca caaccaggct aagaatcttc tctgtggctt ttacggccgc catgcggagc 300

tgcgcttctt ggacctggtt ccttctttgc agttggaccc ggcccagatc tacagggtca 360

cttggttcat ctcctggagc ccctgcttct cctggggctg tgccggggaa gtgcgtgcgt 420

tccttcagga gaacacacac gtgagactgc gtatcttcgc tgcccgcatc tatgattacg 480

acccccctata taaggaggca ctgcaaatgc tgcgggatgc tggggcccaa gtctccatca 540

tgacctacga tgaatttaag cactgctggg acacctttgt ggaccaccag ggatgtccct 600

tccagccctg ggatggacta gatgagcaca gccaagccct gagtgggagg ctgcgggcca 660

ttctccagaa tcagggaaac tgaaggatgg gcctcagtct ctaaggaagg cagagacctg 720

ggttgagcag cagaataaaa gatcttcttc caagaaatgc aaacagaccg ttcaccacca 780

tctccagctg ctcacagacg ccagcaaagc agtatgctcc cgatcaagta gatttttaaa 840

aaatcagagt gggccgggcg cggtggctca cgcctgtaat cccagcactt tggaggccaa 900

ggcgggtgga tcacgaggtc aggagatcga gaccatcctg gctaacacgg tgaaaccctg 960

tctctactaa aaatacaaaa aattagccag gcgtggtggc gggcgcctgt agtcccagct 1020

actctggagg ctgaggcagg agagtagcgt gaacccggga ggcagagctt gcggtgagcc 1080

gagattgcgc tactgcactc cagcctgggc gacagtacca gactccatct caaaaaaaaa 1140

aaaaccagac tgaattaatt ttaactgaaa atttctctta tgttccaagt acacaatagt 1200

aagattatgc tcaatattct cagaataatt ttcaatgtat taatgaaatg aaatgataat 1260

ttggcttcat atctagacta acacaaaatt aagaatcttc cataattgct tttgctcagt 1320

aactgtgtca tgaattgcaa gagtttccac aaacacta 1358

<210> 8
<211> 199
<212> PRT
<213> Homo sapiens

<400> 8

Met Glu Ala Ser Pro Ala Ser Gly Pro Arg His Leu Met Asp Pro His
1 5 10 15

Ile Phe Thr Ser Asn Phe Asn Asn Gly Ile Gly Arg His Lys Thr Tyr
20 25 30

Leu Cys Tyr Glu Val Glu Arg Leu Asp Asn Gly Thr Ser Val Lys Met
35 40 45

Asp Gln His Arg Gly Phe Leu His Asn Gln Ala Lys Asn Leu Leu Cys
50 55 60

Gly Phe Tyr Gly Arg His Ala Glu Leu Arg Phe Leu Asp Leu Val Pro
65 70 75 80

Ser Leu Gln Leu Asp Pro Ala Gln Ile Tyr Arg Val Thr Trp Phe Ile
85 90 95

Ser Trp Ser Pro Cys Phe Ser Trp Gly Cys Ala Gly Glu Val Arg Ala
100 105 110

Phe Leu Gln Glu Asn Thr His Val Arg Leu Arg Ile Phe Ala Ala Arg
115 120 125

Ile Tyr Asp Tyr Asp Pro Leu Tyr Lys Glu Ala Leu Gln Met Leu Arg
130 135 140

Asp Ala Gly Ala Gln Val Ser Ile Met Thr Tyr Asp Glu Phe Lys His
145 150 155 160

Cys Trp Asp Thr Phe Val Asp His Gln Gly Cys Pro Phe Gln Pro Trp
165 170 175

Asp Gly Leu Asp Glu His Ser Gln Ala Leu Ser Gly Arg Leu Arg Ala
180 185 190

Ile Leu Gln Asn Gln Gly Asn
195

<210> 9
<211> 1565
<212> DNA
<213> Homo sapiens

<400> 9
agtctcactt cagttccttt tgcatgaaga gctcagaatc aaaagaggaa accaacccct 60

aagatgagct ttccatgtaa atttgtagcc agcttccttc tgattttcaa tgtttcttcc 120

aaaggtgcag tctccaaaga gattacgaat gccttggaaa cctggggtgc cttgggtcag 180

gacatcaact tggacattcc tagttttcaa atgagtgatg atattgacga tataaaatgg 240

gaaaaaactt cagacaagaa aaagattgca caattcagaa aagagaaaga gactttcaag 300

gaaaaagata catataagct atttaaaaat ggaactctga aaattaagca tctgaagacc 360

gatgatcagg atatctacaa ggtatcaata tatgatacaa aaggaaaaaa tgtgttggaa 420

aaaatatttg atttgaagat tcaagagagg gtctcaaaac caaagatctc ctggacttgt 480

atcaacacaa ccctgacctg tgaggtaatg aatggaactg accccgaatt aaacctgtat 540

caagatggga aacatctaaa actttctcag agggtcatca cacacaagtg gaccaccagc 600

ctgagtgcaa aattcaagtg cacagcaggg aacaaagtca gcaaggaatc cagtgtcgag 660

cctgtcagct gtccagagaa aggtctggac atctatctca tcattggcat atgtggagga 720

ggcagcctct tgatggtctt tgtggcactg ctcgttttct atatcaccaa aaggaaaaaa 780

cagaggagtc ggagaaatga tgaggagctg gagacaagag cccacagagt agctactgaa 840

gaaaggggcc ggaagcccca ccaaattcca gcttcaaccc ctcagaatcc agcaacttcc 900

caacatcctc ctccaccacc tggtcatcgt tcccaggcac ctagtcatcg tcccccgcct 960

cctggacacc gtgttcagca ccagcctcag aagaggcctc ctgctccgtc gggcacacaa 1020

gttcaccagc agaaaggccc gcccctcccc agacctcgag ttcagccaaa acctccccat 1080

ggggcagcag aaaactcatt gtccccttcc tctaattaaa aaagatagaa actgtctttt 1140

tcaataaaaa gcactgtgga tttctgccct cctgatgtgc atatccgtac ttccatgagg 1200

tgttttctgt gtgcagaaca ttgtcacctc ctgaggctgt gggccacagc cacctctgca 1260

tcttcgaact cagccatgtg gtcaacatct ggagtttttg gtctcctcag agagctccat 1320

cacaccagta aggagaagca atataagtgt gattgcaaga atggtagagg accgagcaca 1380

gaaatcttag agatttcttg tcccctctca ggtcatgtgt agatgcgata aatcaagtga 1440

ttggtgtgcc tgggtctcac tacaagcagc ctatctgctt aagagactct ggagtttctt 1500

atgtgccctg gtggacactt gcccaccatc ctgtgagtaa aagtgaaata aaagctttga 1560

ctaga 1565

<210> 10
<211> 351
<212> PRT
<213> Homo sapiens

<400> 10

Met Ser Phe Pro Cys Lys Phe Val Ala Ser Phe Leu Leu Ile Phe Asn
1 5 10 15

Val Ser Ser Lys Gly Ala Val Ser Lys Glu Ile Thr Asn Ala Leu Glu
20 25 30

Thr Trp Gly Ala Leu Gly Gln Asp Ile Asn Leu Asp Ile Pro Ser Phe
35 40 45

Gln Met Ser Asp Asp Ile Asp Asp Ile Lys Trp Glu Lys Thr Ser Asp
50 55 60

Lys Lys Lys Ile Ala Gln Phe Arg Lys Glu Lys Glu Thr Phe Lys Glu

65 70 75 80

Lys Asp Thr Tyr Lys Leu Phe Lys Asn Gly Thr Leu Lys Ile Lys His
85 90 95

Leu Lys Thr Asp Asp Gln Asp Ile Tyr Lys Val Ser Ile Tyr Asp Thr
100 105 110

Lys Gly Lys Asn Val Leu Glu Lys Ile Phe Asp Leu Lys Ile Gln Glu
115 120 125

Arg Val Ser Lys Pro Lys Ile Ser Trp Thr Cys Ile Asn Thr Thr Leu
130 135 140

Thr Cys Glu Val Met Asn Gly Thr Asp Pro Glu Leu Asn Leu Tyr Gln
145 150 155 160

Asp Gly Lys His Leu Lys Leu Ser Gln Arg Val Ile Thr His Lys Trp
165 170 175

Thr Thr Ser Leu Ser Ala Lys Phe Lys Cys Thr Ala Gly Asn Lys Val
180 185 190

Ser Lys Glu Ser Ser Val Glu Pro Val Ser Cys Pro Glu Lys Gly Leu
195 200 205

Asp Ile Tyr Leu Ile Ile Gly Ile Cys Gly Gly Gly Ser Leu Leu Met
210 215 220

Val Phe Val Ala Leu Leu Val Phe Tyr Ile Thr Lys Arg Lys Lys Gln
225 230 235 240

Arg Ser Arg Arg Asn Asp Glu Glu Leu Glu Thr Arg Ala His Arg Val
245 250 255

Ala Thr Glu Glu Arg Gly Arg Lys Pro His Gln Ile Pro Ala Ser Thr
260 265 270

Pro Gln Asn Pro Ala Thr Ser Gln His Pro Pro Pro Pro Pro Gly His
275 280 285

Arg Ser Gln Ala Pro Ser His Arg Pro Pro Pro Pro Gly His Arg Val
290 295 300

Gln His Gln Pro Gln Lys Arg Pro Pro Ala Pro Ser Gly Thr Gln Val
305 310 315 320

```
His Gln Gln Lys Gly Pro Pro Leu Pro Arg Pro Arg Val Gln Pro Lys
                325                 330                 335

Pro Pro His Gly Ala Ala Glu Asn Ser Leu Ser Pro Ser Ser Asn
            340                 345                 350
```

<210> 11
<211> 1674
<212> DNA
<213> Homo sapiens

<400> 11

```
gtcctccact tcctggggag gtagctgcag aataaaacca gcagagactc cttttctcct      60
aaccgtcccg gccaccgctg cctcagcctc tgcctcccag cctctttctg agggaaagga     120
caagatgaag tggaaggcgc ttttcaccgc ggccatcctg caggcacagt tgccgattac     180
agaggcacag agctttggcc tgctggatcc caaactctgc tacctgctgg atggaatcct     240
cttcatctat ggtgtcattc tcactgcctt gttcctgaga gtgaagttca gcaggagcgc     300
agacgccccc gcgtaccagc agggccagaa ccagctctat aacgagctca atctaggacg     360
aagagaggag tacgatgttt tggacaagag acgtggccgg gaccctgaga tggggggaaa     420
gccgagaagg aagaaccctc aggaaggcct gtacaatgaa ctgcagaaag ataagatggc     480
ggaggcctac agtgagattg ggatgaaagg cgagcgccgg aggggcaagg ggcacgatgg     540
cctttaccag ggtctcagta cagccaccaa ggacacctac gacgcccttc acatgcaggc     600
cctgccccct cgctaacagc caggggattt caccactcaa aggccagacc tgcagacgcc     660
cagattatga gacacaggat gaagcattta caacccggtt cactcttctc agccactgaa     720
gtattcccct ttatgtacag gatgctttgg ttatatttag ctccaaacct tcacacacag     780
actgttgtcc ctgcactctt taagggagtg tactcccagg gcttacggcc ctggccttgg     840
gccctctggt ttgccggtgg tgcaggtaga cctgtctcct ggcggttcct cgttctccct     900
gggaggcggg cgcactgcct ctcacagctg agttgttgag tctgttttgt aaagtcccca     960
gagaaagcgc agatgctagc acatgcccta atgtctgtat cactctgtgt ctgagtggct    1020
tcactcctgc tgtaaatttg gcttctgttg tcaccttcac ctcctttcaa ggtaactgta    1080
ctgggccatg ttgtgcctcc ctggtgagag ggccgggcag aggggcagat ggaaaggagc    1140
ctaggccagg tgcaaccagg gagctgcagg ggcatgggaa ggtgggcggg caggggaggg    1200
tcagccaggg cctgcgaggg cagcgggagc ctccctgcct caggcctctg tgccgcacca    1260
ttgaactgta ccatgtgcta caggggccag aagatgaaca gactgacctt gatgagctgt    1320
gcacaaagtg gcataaaaaa catgtggtta cacagtgtga ataaagtgct gcggagcaag    1380
aggaggccgt tgattcactt cacgctttca gcgaatgaca aaatcatctt tgtgaaggcc    1440
tcgcaggaag acccaacaca tgggacctat aactgcccag cggacagtgg caggacagga    1500
```

aaaacccgtc aatgtactag gatactgctg cgtcattaca gggcacaggc catggatgga 1560

aaacgctctc tgctctgctt tttttctact gttttaattt atactggcat gctaaagcct 1620

tcctattttg cataataaat gcttcagtga aaaaaaaaaa aaaaaaaaaa aaaa 1674

<210> 12
<211> 1690
<212> DNA
<213> Homo sapiens

<400> 12
tgctttctca aaggccccac agtcctccac ttcctgggga ggtagctgca gaataaaacc 60

agcagagact ccttttctcc taaccgtccc ggccaccgct gcctcagcct ctgcctccca 120

gcctctttct gagggaaagg acaagatgaa gtggaaggcg cttttcaccg cggccatcct 180

gcaggcacag ttgccgatta cagaggcaca gagctttggc ctgctggatc ccaaactctg 240

ctacctgctg gatggaatcc tcttcatcta tggtgtcatt ctcactgcct tgttcctgag 300

agtgaagttc agcaggagcg cagacgcccc cgcgtaccag cagggccaga accagctcta 360

taacgagctc aatctaggac gaagagagga gtacgatgtt ttggacaaga gacgtggccg 420

ggaccctgag atggggggaa agccgcagag aaggaagaac cctcaggaag gcctgtacaa 480

tgaactgcag aaagataaga tggcggaggc ctacagtgag attgggatga aaggcgagcg 540

ccggaggggc aaggggcacg atggccttta ccagggtctc agtacagcca ccaaggacac 600

ctacgacgcc cttcacatgc aggccctgcc ccctcgctaa cagccagggg atttcaccac 660

tcaaaggcca gacctgcaga cgcccagatt atgagacaca ggatgaagca tttacaaccc 720

ggttcactct tctcagccac tgaagtattc ccctttatgt acaggatgct ttggttatat 780

ttagctccaa accttcacac acagactgtt gtccctgcac tctttaaggg agtgtactcc 840

cagggcttac ggccctggcc ttgggccctc tggtttgccg gtggtgcagg tagacctgtc 900

tcctggcggt tcctcgttct ccctgggagg cgggcgcact gcctctcaca gctgagttgt 960

tgagtctgtt ttgtaaagtc cccagagaaa gcgcagatgc tagcacatgc cctaatgtct 1020

gtatcactct gtgtctgagt ggcttcactc ctgctgtaaa tttggcttct gttgtcacct 1080

tcacctcctt tcaaggtaac tgtactgggc catgttgtgc ctccctggtg agagggccgg 1140

gcagaggggc agatggaaag gagcctaggc caggtgcaac cagggagctg caggggcatg 1200

ggaaggtggg cgggcagggg agggtcagcc agggcctgcg agggcagcgg gagcctccct 1260

gcctcaggcc tctgtgccgc accattgaac tgtaccatgt gctacagggg ccagaagatg 1320

aacagactga ccttgatgag ctgtgcacaa agtggcataa aaaacatgtg gttacacagt 1380

gtgaataaag tgctgcggag caagaggagg ccgttgattc acttcacgct ttcagcgaat 1440

gacaaaatca tctttgtgaa ggcctcgcag gaagacccaa cacatgggac ctataactgc 1500

ccagcggaca gtggcaggac aggaaaaacc cgtcaatgta ctaggatact gctgcgtcat 1560

tacagggcac aggccatgga tggaaaacgc tctctgctct gcttttttc tactgtttta 1620

atttatactg gcatgctaaa gccttcctat tttgcataat aaatgcttca gtgaaaatgc 1680

aaaaaaaaaa 1690

<210> 13
<211> 163
<212> PRT
<213> Homo sapiens

<400> 13

Met Lys Trp Lys Ala Leu Phe Thr Ala Ala Ile Leu Gln Ala Gln Leu
1 5 10 15

Pro Ile Thr Glu Ala Gln Ser Phe Gly Leu Leu Asp Pro Lys Leu Cys
20 25 30

Tyr Leu Leu Asp Gly Ile Leu Phe Ile Tyr Gly Val Ile Leu Thr Ala
35 40 45

Leu Phe Leu Arg Val Lys Phe Ser Arg Ser Ala Asp Ala Pro Ala Tyr
50 55 60

Gln Gln Gly Gln Asn Gln Leu Tyr Asn Glu Leu Asn Leu Gly Arg Arg
65 70 75 80

Glu Glu Tyr Asp Val Leu Asp Lys Arg Arg Gly Arg Asp Pro Glu Met
85 90 95

Gly Gly Lys Pro Arg Arg Lys Asn Pro Gln Glu Gly Leu Tyr Asn Glu
100 105 110

Leu Gln Lys Asp Lys Met Ala Glu Ala Tyr Ser Glu Ile Gly Met Lys
115 120 125

Gly Glu Arg Arg Arg Gly Lys Gly His Asp Gly Leu Tyr Gln Gly Leu
130 135 140

Ser Thr Ala Thr Lys Asp Thr Tyr Asp Ala Leu His Met Gln Ala Leu
145 150 155 160

Pro Pro Arg

<210> 14
<211> 164

<212> PRT
<213> Homo sapiens

<400> 14

Met Lys Trp Lys Ala Leu Phe Thr Ala Ala Ile Leu Gln Ala Gln Leu
1               5                   10                  15

Pro Ile Thr Glu Ala Gln Ser Phe Gly Leu Leu Asp Pro Lys Leu Cys
            20                  25                  30

Tyr Leu Leu Asp Gly Ile Leu Phe Ile Tyr Gly Val Ile Leu Thr Ala
        35                  40                  45

Leu Phe Leu Arg Val Lys Phe Ser Arg Ser Ala Asp Ala Pro Ala Tyr
    50                  55                  60

Gln Gln Gly Gln Asn Gln Leu Tyr Asn Glu Leu Asn Leu Gly Arg Arg
65                  70                  75                  80

Glu Glu Tyr Asp Val Leu Asp Lys Arg Arg Gly Arg Asp Pro Glu Met
                85                  90                  95

Gly Gly Lys Pro Gln Arg Arg Lys Asn Pro Gln Glu Gly Leu Tyr Asn
            100                 105                 110

Glu Leu Gln Lys Asp Lys Met Ala Glu Ala Tyr Ser Glu Ile Gly Met
        115                 120                 125

Lys Gly Glu Arg Arg Arg Gly Lys Gly His Asp Gly Leu Tyr Gln Gly
    130                 135                 140

Leu Ser Thr Ala Thr Lys Asp Thr Tyr Asp Ala Leu His Met Gln Ala
145                 150                 155                 160

Leu Pro Pro Arg

<210> 15
<211> 4721
<212> DNA
<213> Homo sapiens

<400> 15
acacttcggg ttcctcgggg aggaggggct ggaaccctag cccatcgtca ggacaaagat 60

gctcaggctg ctcttggctc tcaacttatt cccttcaatt caagtaacag gaaacaagat 120

tttggtgaag cagtcgccca tgcttgtagc gtacgacaat gcggtcaacc ttagctgcaa 180

gtattcctac aatctcttct caaggagtt ccgggcatcc cttcacaaag gactggatag 240

tgctgtggaa gtctgtgttg tatatgggaa ttactcccag cagcttcagg tttactcaaa 300
aacggggttc aactgtgatg ggaaattggg caatgaatca gtgacattct acctccagaa 360
tttgtatgtt aaccaaacag atatttactt ctgcaaaatt gaagttatgt atcctcctcc 420
ttacctagac aatgagaaga gcaatggaac cattatccat gtgaaaggga aacacctttg 480
tccaagtccc ctatttcccg gaccttctaa gcccttttgg gtgctggtgg tggttggtgg 540
agtcctggct tgctatagct tgctagtaac agtggccttt attattttct gggtgaggag 600
taagaggagc aggctcctgc acagtgacta catgaacatg actccccgcc gccccgggcc 660
cacccgcaag cattaccagc cctatgcccc accacgcgac ttcgcagcct atcgctcctg 720
acacggacgc ctatccagaa gccagccggc tggcagcccc catctgctca atatcactgc 780
tctggatagg aaatgaccgc catctccagc cggccacctc aggcccctgt tgggccacca 840
atgccaattt ttctcgagtg actagaccaa atatcaagat cattttgaga ctctgaaatg 900
aagtaaaaga gatttcctgt gacaggccaa gtcttacagt gccatggccc acattccaac 960
ttaccatgta cttagtgact tgactgagaa gttagggtag aaaacaaaaa gggagtggat 1020
tctgggagcc tcttcccttt ctcactcacc tgcacatctc agtcaagcaa agtgtggtat 1080
ccacagacat tttagttgca gaagaaaggc taggaaatca ttccttttgg ttaaatgggt 1140
gtttaatctt ttggttagtg ggttaaacgg ggtaagttag agtaggggga gggataggaa 1200
gacatattta aaaaccatta aaacactgtc tcccactcat gaaatgagcc acgtagttcc 1260
tatttaatgc tgttttcctt tagtttagaa atacatagac attgtctttt atgaattctg 1320
atcatattta gtcattttga ccaaatgagg gatttggtca aatgagggat tccctcaaag 1380
caatatcagg taaaccaagt tgctttcctc actccctgtc atgagacttc agtgttaatg 1440
ttcacaatat actttcgaaa gaataaaata gttctcctac atgaagaaag aatatgtcag 1500
gaaataaggt cactttatgt caaaattatt tgagtactat gggacctggc gcagtggctc 1560
atgcttgtaa tcccagcact ttgggaggcc gaggtgggca gatcacttga gatcaggacc 1620
agcctggtca agatggtgaa actccgtctg tactaaaaat acaaaattta gcttggcctg 1680
gtggcaggca cctgtaatcc cagctgccca agaggctgag gcatgagaat cgcttgaacc 1740
tggcaggcgg aggttgcagt gagccgagat agtgccacag ctctccagcc tgggcgacag 1800
agtgagactc catctcaaac aacaacaaca acaacaacaa caacaacaaa ccacaaaatt 1860
atttgagtac tgtgaaggat tatttgtcta acagttcatt ccaatcagac caggtaggag 1920
ctttcctgtt tcatatgttt cagggttgca cagttggtct ctttaatgtc ggtgtggaga 1980
tccaaagtgg gttgtggaaa gagcgtccat aggagaagtg agaatactgt gaaaaaggga 2040
tgttagcatt cattagagta tgaggatgag tcccaagaag gttctttgga aggaggacga 2100
atagaatgga gtaatgaaat tcttgccatg tgctgaggag atagccagca ttaggtgaca 2160

atcttccaga agtggtcagg cagaaggtgc cctggtgaga gctcctttac agggacttta 2220
tgtggtttag ggctcagagc tccaaaactc tgggctcagc tgctcctgta ccttggaggt 2280
ccattcacat gggaaagtat tttggaatgt gtcttttgaa gagagcatca gagttcttaa 2340
gggactgggt aaggcctgac cctgaaatga ccatggatat ttttctacct acagtttgag 2400
tcaactagaa tatgcctggg gaccttgaag aatggccctt cagtggccct caccatttgt 2460
tcatgcttca gttaattcag gtgttgaagg agcttaggtt ttagaggcac gtagacttgg 2520
ttcaagtctc gttagtagtt gaatagcctc aggcaagtca ctgcccacct aagatgatgg 2580
ttcttcaact ataaaatgga gataatggtt acaaatgtct cttcctatag tataatctcc 2640
ataagggcat ggcccaagtc tgtctttgac tctgcctatc cctgacattt agtagcatgc 2700
ccgacataca atgttagcta ttggtattat tgccatatag ataaattatg tataaaaatt 2760
aaactgggca atagcctaag aagggggaa tattgtaaca caaatttaaa cccactacgc 2820
agggatgagg tgctataata tgaggacctt ttaacttcca tcattttcct gtttcttgaa 2880
atagtttatc ttgtaatgaa atataaggca cctcccactt ttatgtatag aaagaggtct 2940
tttaattttt ttttaatgtg agaaggaagg gaggagtagg aatcttgaga ttccagatcg 3000
aaaatactgt actttggttg atttttaagt gggcttccat tccatggatt taatcagtcc 3060
caagaagatc aaactcagca gtacttgggt gctgaagaac tgttggattt accctggcac 3120
gtgtgccact tgccagcttc ttgggcacac agagttcttc aatccaagtt atcagattgt 3180
atttgaaaat gacagagctg gagagttttt tgaaatggca gtggcaaata aataaatact 3240
ttttttttaaa tggaaagact tgatctatgg taataaatga ttttgttttc tgactggaaa 3300
aataggccta ctaaagatga atcacacttg agatgtttct tactcactct gcacagaaac 3360
aaagaagaaa tgttatacag ggaagtccgt tttcactatt agtatgaacc aagaaatggt 3420
tcaaaaacag tggtaggagc aatgctttca tagtttcaga tatggtagtt atgaagaaaa 3480
caatgtcatt tgctgctatt attgtaagag tcttataatt aatggtactc ctataatttt 3540
tgattgtgag ctcacctatt tgggttaagc atgccaattt aaagagacca agtgtatgta 3600
cattatgttc tacatattca gtgataaaat tactaaacta ctatatgtct gctttaaatt 3660
tgtactttaa tattgtcttt tggtattaag aaagatatgc tttcagaata gatatgcttc 3720
gctttggcaa ggaatttgga tagaacttgc tatttaaaag aggtgtgggg taaatccttg 3780
tataaatctc cagtttagcc ttttttgaaa aagctagact ttcaaatact aatttcactt 3840
caagcagggt acgtttctgg tttgtttgct tgacttcagt cacaatttct tatcagacca 3900
atggctgacc tctttgagat gtcaggctag gcttacctat gtgttctgtg tcatgtgaat 3960
gctgagaagt ttgacagaga tccaacttca gccttgaccc catcagtccc tcgggttaac 4020

taactgagcc accggtcctc atggctattt taatgagggt attgatggtt aaatgcatgt 4080

ctgatccctt atcccagcca tttgcactgc cagctgggaa ctataccaga cctggatact 4140

gatcccaaag tgttaaattc aactacatgc tggagattag agatggtgcc aataaaggac 4200

ccagaaccag gatcttgatt gctatagact tattaataat ccaggtcaaa gagagtgaca 4260

cacactctct caagacctgg ggtgagggag tctgtgttat ctgcaaggcc atttgaggct 4320

cagaaagtct ctctttccta tagatatatg catactttct gacatatagg aatgtatcag 4380

gaatactcaa ccatcacagg catgttccta cctcagggcc tttacatgtc ctgtttactc 4440

tgtctagaat gtccttctgt agatgacctg gcttgcctcg tcacccttca ggtccttgct 4500

caagtgtcat cttctcccct agttaaacta ccccacaccc tgtctgcttt ccttgcttat 4560

ttttctccat agcattttac catctcttac attagacatt tttcttattt atttgtagtt 4620

tataagcttc atgaggcaag taactttgct ttgtttcttg ctgtatctcc agtgcccaga 4680

gcagtgcctg gtatataata aatatttatt gactgagtga a 4721

<210> 16
<211> 4543
<212> DNA
<213> Homo sapiens

<400> 16
taaagtcatc aaaacaacgt tatatcctgt gtgaaatgct gcagtcagga tgccttgtgg 60

tttgagtgcc ttgatcatgt gccctaaggg gatggtggcg gtggtggtgg ccgtggatga 120

cggagactct caggccttgg caggtgcgtc tttcagttcc cctcacactt cgggttcctc 180

ggggaggagg ggctggaacc ctagcccatc gtcaggacaa agatgctcag gctgctcttg 240

gctctcaact tattcccttc aattcaagta acagggaaac acctttgtcc aagtccccta 300

tttcccggac cttctaagcc cttttgggtg ctggtggtgg ttggtggagt cctggcttgc 360

tatagcttgc tagtaacagt ggcctttatt attttctggg tgaggagtaa gaggagcagg 420

ctcctgcaca gtgactacat gaacatgact ccccgccgcc ccgggcccac ccgcaagcat 480

taccagccct atgccccacc acgcgacttc gcagcctatc gctcctgaca cggacgccta 540

tccagaagcc agccggctgg cagcccccat ctgctcaata tcactgctct ggataggaaa 600

tgaccgccat ctccagccgg ccacctcagg cccctgttgg gccaccaatg ccaatttttc 660

tcgagtgact agaccaaata tcaagatcat tttgagactc tgaaatgaag taaaagagat 720

ttcctgtgac aggccaagtc ttacagtgcc atggcccaca ttccaactta ccatgtactt 780

agtgacttga ctgagaagtt agggtagaaa acaaaaaggg agtggattct gggagcctct 840

tccctttctc actcacctgc acatctcagt caagcaaagt gtggtatcca cagacatttt 900

agttgcagaa gaaaggctag gaaatcattc cttttggtta aatgggtgtt taatcttttg 960

gttagtgggt taaacggggt aagttagagt agggggaggg ataggaagac atatttaaaa 1020

accattaaaa cactgtctcc cactcatgaa atgagccacg tagttcctat ttaatgctgt 1080

tttcctttag tttagaaata catagacatt gtcttttatg aattctgatc atatttagtc 1140

attttgacca aatgagggat ttggtcaaat gagggattcc ctcaaagcaa tatcaggtaa 1200

accaagttgc tttcctcact ccctgtcatg agacttcagt gttaatgttc acaatatact 1260

ttcgaaagaa taaaatagtt ctcctacatg aagaaagaat atgtcaggaa ataaggtcac 1320

tttatgtcaa aattatttga gtactatggg acctggcgca gtggctcatg cttgtaatcc 1380

cagcactttg ggaggccgag gtgggcagat cacttgagat caggaccagc ctggtcaaga 1440

tggtgaaact ccgtctgtac taaaaataca aaatttagct tggcctggtg gcaggcacct 1500

gtaatcccag ctgcccaaga ggctgaggca tgagaatcgc ttgaacctgg caggcggagg 1560

ttgcagtgag ccgagatagt gccacagctc tccagcctgg gcgacagagt gagactccat 1620

ctcaaacaac aacaacaaca acaacaacaa caacaaacca caaaattatt tgagtactgt 1680

gaaggattat ttgtctaaca gttcattcca atcagaccag gtaggagctt tcctgtttca 1740

tatgtttcag ggttgcacag ttggtctctt taatgtcggt gtggagatcc aaagtgggtt 1800

gtggaaagag cgtccatagg agaagtgaga atactgtgaa aaagggatgt tagcattcat 1860

tagagtatga ggatgagtcc caagaaggtt ctttggaagg aggacgaata gaatggagta 1920

atgaaattct tgccatgtgc tgaggagata gccagcatta ggtgacaatc ttccagaagt 1980

ggtcaggcag aaggtgccct ggtgagagct cctttacagg gactttatgt ggtttagggc 2040

tcagagctcc aaaactctgg gctcagctgc tcctgtacct tggaggtcca ttcacatggg 2100

aaagtatttt ggaatgtgtc ttttgaagag agcatcagag ttcttaaggg actgggtaag 2160

gcctgaccct gaaatgacca tggatatttt tctacctaca gtttgagtca actagaatat 2220

gcctggggac cttgaagaat ggcccttcag tggccctcac catttgttca tgcttcagtt 2280

aattcaggtg ttgaaggagc ttaggtttta gaggcacgta gacttggttc aagtctcgtt 2340

agtagttgaa tagcctcagg caagtcactg cccacctaag atgatggttc ttcaactata 2400

aaatggagat aatggttaca aatgtctctt cctatagtat aatctccata agggcatggc 2460

ccaagtctgt ctttgactct gcctatccct gacatttagt agcatgcccg acatacaatg 2520

ttagctattg gtattattgc catatagata aattatgtat aaaaattaaa ctgggcaata 2580

gcctaagaag gggggaatat tgtaacacaa atttaaaccc actacgcagg gatgaggtgc 2640

tataatatga ggacctttta acttccatca ttttcctgtt tcttgaaata gtttatcttg 2700

taatgaaata taaggcacct cccactttta tgtatagaaa gaggtctttt aattttttt 2760

taatgtgaga aggaagggag gagtaggaat cttgagattc cagatcgaaa atactgtact 2820

ttggttgatt tttaagtggg cttccattcc atggatttaa tcagtcccaa gaagatcaaa 2880

```
ctcagcagta cttgggtgct gaagaactgt tggatttacc ctggcacgtg tgccacttgc     2940
cagcttcttg ggcacacaga gttcttcaat ccaagttatc agattgtatt tgaaaatgac     3000
agagctggag agtttttgga aatggcagtg gcaaataaat aaatactttt ttttaaatgg     3060
aaagacttga tctatggtaa taaatgattt tgttttctga ctggaaaaat aggcctacta     3120
aagatgaatc acacttgaga tgtttcttac tcactctgca cagaaacaaa gaagaaatgt     3180
tatacaggga agtccgtttt cactattagt atgaaccaag aaatggttca aaaacagtgg     3240
taggagcaat gctttcatag tttcagatat ggtagttatg aagaaaacaa tgtcatttgc     3300
tgctattatt gtaagagtct tataattaat ggtactccta taatttttga ttgtgagctc     3360
acctatttgg gttaagcatg ccaatttaaa gagaccaagt gtatgtacat tatgttctac     3420
atattcagtg ataaaattac taaactacta tatgtctgct ttaaatttgt actttaatat     3480
tgtcttttgg tattaagaaa gatatgcttt cagaatagat atgcttcgct ttggcaagga     3540
atttggatag aacttgctat ttaaaagagg tgtggggtaa atccttgtat aaatctccag     3600
tttagccttt tttgaaaaag ctagactttc aaatactaat ttcacttcaa gcagggtacg     3660
tttctggttt gtttgcttga cttcagtcac aatttcttat cagaccaatg gctgacctct     3720
ttgagatgtc aggctaggct tacctatgtg ttctgtgtca tgtgaatgct gagaagtttg     3780
acagagatcc aacttcagcc ttgaccccat cagtccctcg ggttaactaa ctgagccacc     3840
ggtcctcatg gctattttaa tgagggtatt gatggttaaa tgcatgtctg atcccttatc     3900
ccagccattt gcactgccag ctgggaacta taccagacct ggatactgat cccaaagtgt     3960
taaattcaac tacatgctgg agattagaga tggtgccaat aaaggaccca gaaccaggat     4020
cttgattgct atagacttat taataatcca ggtcaaagag agtgacacac actctctcaa     4080
gacctggggt gagggagtct gtgttatctg caaggccatt tgaggctcag aaagtctctc     4140
tttcctatag atatatgcat actttctgac atataggaat gtatcaggaa tactcaacca     4200
tcacaggcat gttcctacct cagggccttt acatgtcctg tttactctgt ctagaatgtc     4260
cttctgtaga tgacctggct tgcctcgtca cccttcaggt ccttgctcaa gtgtcatctt     4320
ctcccctagt taaactaccc cacaccctgt ctgctttcct tgcttatttt tctccatagc     4380
attttaccat ctcttacatt agacattttt cttatttatt tgtagtttat aagcttcatg     4440
aggcaagtaa ctttgctttg tttcttgctg tatctccagt gcccagagca gtgcctggta     4500
tataataaat atttattgac tgagtgaaaa aaaaaaaaaa aaa                       4543
```

<210> 17
<211> 220
<212> PRT
<213> Homo sapiens

<400> 17

Met Leu Arg Leu Leu Leu Ala Leu Asn Leu Phe Pro Ser Ile Gln Val
1               5                   10                  15

Thr Gly Asn Lys Ile Leu Val Lys Gln Ser Pro Met Leu Val Ala Tyr
            20                  25                  30

Asp Asn Ala Val Asn Leu Ser Cys Lys Tyr Ser Tyr Asn Leu Phe Ser
        35                  40                  45

Arg Glu Phe Arg Ala Ser Leu His Lys Gly Leu Asp Ser Ala Val Glu
    50                  55                  60

Val Cys Val Val Tyr Gly Asn Tyr Ser Gln Gln Leu Gln Val Tyr Ser
65                  70                  75                  80

Lys Thr Gly Phe Asn Cys Asp Gly Lys Leu Gly Asn Glu Ser Val Thr
                85                  90                  95

Phe Tyr Leu Gln Asn Leu Tyr Val Asn Gln Thr Asp Ile Tyr Phe Cys
            100                 105                 110

Lys Ile Glu Val Met Tyr Pro Pro Pro Tyr Leu Asp Asn Glu Lys Ser
        115                 120                 125

Asn Gly Thr Ile Ile His Val Lys Gly Lys His Leu Cys Pro Ser Pro
    130                 135                 140

Leu Phe Pro Gly Pro Ser Lys Pro Phe Trp Val Leu Val Val Val Gly
145                 150                 155                 160

Gly Val Leu Ala Cys Tyr Ser Leu Leu Val Thr Val Ala Phe Ile Ile
                165                 170                 175

Phe Trp Val Arg Ser Lys Arg Ser Arg Leu Leu His Ser Asp Tyr Met
            180                 185                 190

Asn Met Thr Pro Arg Arg Pro Gly Pro Thr Arg Lys His Tyr Gln Pro
        195                 200                 205

Tyr Ala Pro Pro Arg Asp Phe Ala Ala Tyr Arg Ser
    210                 215                 220

<210> 18
<211> 101
<212> PRT
<213> Homo sapiens

<400> 18

Met Leu Arg Leu Leu Leu Ala Leu Asn Leu Phe Pro Ser Ile Gln Val
1 5 10 15

Thr Gly Lys His Leu Cys Pro Ser Pro Leu Phe Pro Gly Pro Ser Lys
20 25 30

Pro Phe Trp Val Leu Val Val Val Gly Gly Val Leu Ala Cys Tyr Ser
35 40 45

Leu Leu Val Thr Val Ala Phe Ile Ile Phe Trp Val Arg Ser Lys Arg
50 55 60

Ser Arg Leu Leu His Ser Asp Tyr Met Asn Met Thr Pro Arg Arg Pro
65 70 75 80

Gly Pro Thr Arg Lys His Tyr Gln Pro Tyr Ala Pro Pro Arg Asp Phe
85 90 95

Ala Ala Tyr Arg Ser
100

<210> 19
<211> 1534
<212> DNA
<213> Homo sapiens

<400> 19
tattgtcaga gtcctcttgt ttggccttct aggaaggctg tgggacccag ctttcttcaa 60

ccagtccagg tggaggcctc tgccttgaac gtttccaagt gaggtaaaac ccgcaggccc 120

agaggcctct ctacttcctg tgtggggttc agaaaccctc ctcccctccc agcctcaggt 180

gcctgcttca gaaaatgaag tagtaagtct gctggcctcc gccatcttag taaagtaaca 240

gtcccatgaa acaaagatgc agtcgggcac tcactggaga gttctgggcc tctgcctctt 300

atcagttggc gtttgggggc aagatggtaa tgaagaaatg ggtggtatta cacagacacc 360

atataaagtc tccatctctg gaaccacagt aatattgaca tgccctcagt atcctggatc 420

tgaaatacta tggcaacaca atgataaaaa cataggcggt gatgaggatg ataaaaacat 480

aggcagtgat gaggatcacc tgtcactgaa ggaattttca gaattggagc aaagtggtta 540

ttatgtctgc taccccagag gaagcaaacc agaagatgcg aacttttatc tctacctgag 600

ggcaagagtg tgtgagaact gcatggagat ggatgtgatg tcggtggcca caattgtcat 660

agtggacatc tgcatcactg gggggcttgct gctgctggtt tactactgga gcaagaatag 720

aaaggccaag gccaagcctg tgacacgagg agcgggtgct ggcggcaggc aaaggggaca 780

aaacaaggag aggccaccac ctgttcccaa cccagactat gagcccatcc ggaaaggcca 840

```
gcgggacctg tattctggcc tgaatcagag acgcatctga ccctctggag aacactgcct      900

cccgctggcc caggtctcct ctccagtccc cctgcgactc cctgtttcct gggctagtct      960

tggaccccac gagagagaat cgttcctcag cctcatggtg aactcgcgcc ctccagcctg     1020

atcccccgct ccctcctccc tgccttctct gctggtaccc agtcctaaaa tattgctgct     1080

tcctcttcct ttgaagcatc atcagtagtc acaccctcac agctggcctg ccctcttgcc     1140

aggatattta tttgtgctat tcactccctt ccctttggat gtaacttctc cgttcagttc     1200

cctccttttc ttgcatgtaa gttgtccccc atcccaaagt attccatcta cttttctatc     1260

gccgtcccct tttgcagccc tctctgggga tggactgggt aaatgttgac agaggccctg     1320

ccccgttcac agatcctggc cctgagccag ccctgtgctc ctccctcccc caacactccc     1380

taccaacccc ctaatcccct actccctcca cccccctcc actgtaggcc actggatggt      1440

catttgcatc tccgtaaatg tgctctgctc ctcagctgag agagaaaaaa ataaactgta     1500

tttggctgca agaaaaaaaa aaaaaaaaaa aaaa                                 1534
```

<210> 20
<211> 207
<212> PRT
<213> Homo sapiens

<400> 20

```
Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys
    50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
        115                 120                 125
```

```
Ser Val Ala Thr Ile Val Ile Val Asp Ile Cys Ile Thr Gly Gly Leu
    130                 135                 140

Leu Leu Leu Val Tyr Tyr Trp Ser Lys Asn Arg Lys Ala Lys Ala Lys
145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190

Lys Gly Gln Arg Asp Leu Tyr Ser Gly Leu Asn Gln Arg Arg Ile
        195                 200                 205


<210>  21
<211>  2690
<212>  DNA
<213>  Homo sapiens

<400>  21
agtctagctg ctgcacaggc tggctggctg gctggctgct aagggctgct ccacgctttt      60

gccggaggac agagactgac atggaacagg ggaagggcct ggctgtcctc atcctggcta     120

tcattcttct tcaaggtact ttggcccagt caatcaaagg aaaccacttg gttaaggtgt     180

atgactatca agaagatggt tcggtacttc tgacttgtga tgcagaagcc aaaaatatca     240

catggtttaa agatgggaag atgatcggct tcctaactga agataaaaaa aaatggaatc     300

tgggaagtaa tgccaaggac cctcgaggga tgtatcagtg taaaggatca cagaacaagt     360

caaaaccact ccaagtgtat tacagaatgt gtcagaactg cattgaacta aatgcagcca     420

ccatatctgg ctttctcttt gctgaaatcg tcagcatttt cgtccttgct gttggggtct     480

acttcattgc tggacaggat ggagttcgcc agtcgagagc ttcagacaag cagactctgt     540

tgcccaatga ccagctctac cagcccctca aggatcgaga agatgaccag tacagccacc     600

ttcaaggaaa ccagttgagg aggaattgaa ctcaggactc agagtagtcc aggtgttctc     660

ctcctattca gttcccagaa tcaaagcaat gcattttgga aagctcctag cagagagact     720

ttcagcccta aatctagact caaggttccc agagatgaca aatggagaag aaaggccatc     780

agagcaaatt tgggggtttc tcaaataaaa taaaaataaa aacaaatact gtgtttcaga     840

agcgccacct attggggaaa attgtaaaag aaaaatgaaa agatcaaata accccctgga     900

tttgaatata attttttgtg ttgtaatttt tatttcgttt ttgtataggt tataattcac     960

atggctcaaa tattcagtga aagctctccc tccaccgcca tcccctgcta cccagtgacc    1020

ctgttgccct cttcagagac aaattagttt ctcttttttt tttttttttt tttttttttg    1080
```

agacagtctg gctctgtcac ccaggctgaa atgcagtggc accatctcgg ctcactgcaa 1140

cctctgcctc ctgggttcaa gcgattctcc tgcctcagcc tcccgggcag ctgggattac 1200

aggcacacac taccacacct ggctaatttt tgtattttta gtagagacag ggttttgctc 1260

tgttggccaa gctggtctcg aactcctgac ctcaagtgat ccgcccgcct cagcctccca 1320

aagtgctggg attacaggtg tgagccacca tgcctggtct taaaaccagt ttcttatata 1380

tctctctgga ggtattctag gcatatatga gcacattctc aagtacatat tatcctccct 1440

tcccctatct tttagacaaa tgatatcaaa ctatacatct tgtgagatta ttgcatacca 1500

ttatatgaag ataccattat atccttttta atgcaaccat attgtacaaa tagactatga 1560

tttatttaac ctgttatcta tcagtggata tttaagttgg tagttggttc caatcttttg 1620

ctcttacaac aattctgcaa tgactaacat tgtataaata tcatttttaa aaataattgc 1680

attgaagcat aatgtacatg ccataaaatc cacccatctt aagtgatttc acctgttctc 1740

agaaattttt agtaaattta actaattgta cagccattac cataatccag ctttaggaca 1800

ttttcttttt tttcttttct tttcttttt ttcttttttt tttttttttg aagtggaatc 1860

ttgctctgtg gcccaggctg gagtgcagtg gcgcgatctc agctcactgc aacctccacc 1920

tcctgggttc aagcgattct cttgccttgg cctcccgagt agctgagact acaggcacat 1980

gccaccacgc ccagctcatt ttttgtgtat ttagtatttg tgtatctagt atttgtgtac 2040

ttagtagaga cagggtttca ccatgttggc caggctggtc tccaattcct gacctcaggc 2100

gatccacccg ccttgacctc ccaaagtgct gggattacag gtgtgagcca ccgcgccagg 2160

cccgtaactg tattttaata tagccattct atggatttaa tatggtattt tattatggcc 2220

ttaatttgca tttccctaga tactaaccat gctgagtgtc ctgtcttgtg tttattaacc 2280

attcatatat ttttagtgaa atgtgtatca aatcttttgc ccatttttaa gttgacttat 2340

ttgtttgtct tcttactatt gggttgcata tgtttttgat ataagtcctt tatcagatat 2400

atgatttgga aatattttct accaatctgt ggtttgtttt tcttaatggt gtcttttgaa 2460

gtgcaaaagg tttgaatttt gaagtacatt ttattgattt tttcttctat atattgtgct 2520

tttggtatca tgtctaataa atctttacca aacccacagt tacaaagatt ttctcctgtc 2580

ttctttttat actttttaca gctttatggt tttagctcta acaataaatg tgattttgaa 2640

catacataag actatttgta acaaacacaa ataaattgaa ttgttgggca 2690

<210> 22
<211> 182
<212> PRT
<213> Homo sapiens

<400> 22

Met Glu Gln Gly Lys Gly Leu Ala Val Leu Ile Leu Ala Ile Ile Leu
1               5                   10                  15

Leu Gln Gly Thr Leu Ala Gln Ser Ile Lys Gly Asn His Leu Val Lys
            20                  25                  30

Val Tyr Asp Tyr Gln Glu Asp Gly Ser Val Leu Leu Thr Cys Asp Ala
        35                  40                  45

Glu Ala Lys Asn Ile Thr Trp Phe Lys Asp Gly Lys Met Ile Gly Phe
    50                  55                  60

Leu Thr Glu Asp Lys Lys Lys Trp Asn Leu Gly Ser Asn Ala Lys Asp
65                  70                  75                  80

Pro Arg Gly Met Tyr Gln Cys Lys Gly Ser Gln Asn Lys Ser Lys Pro
                85                  90                  95

Leu Gln Val Tyr Tyr Arg Met Cys Gln Asn Cys Ile Glu Leu Asn Ala
            100                 105                 110

Ala Thr Ile Ser Gly Phe Leu Phe Ala Glu Ile Val Ser Ile Phe Val
        115                 120                 125

Leu Ala Val Gly Val Tyr Phe Ile Ala Gly Gln Asp Gly Val Arg Gln
    130                 135                 140

Ser Arg Ala Ser Asp Lys Gln Thr Leu Leu Pro Asn Asp Gln Leu Tyr
145                 150                 155                 160

Gln Pro Leu Lys Asp Arg Glu Asp Asp Gln Tyr Ser His Leu Gln Gly
                165                 170                 175

Asn Gln Leu Arg Arg Asn
            180

<210> 23
<211> 3049
<212> DNA
<213> Homo sapiens

<400> 23
ctctcttcat ttaagcacga ctctgcagaa ggaacaaagc accctcccca ctgggctcct 60

ggttgcagag ctccaagtcc tcacacagat acgcctgttt gagaagcagc gggcaagaaa 120

gacgcaagcc cagaggccct gccatttctg tgggctcagg tccctactgg ctcaggcccc 180

tgcctccctc ggcaaggcca caatgaaccg gggagtccct tttaggcact tgcttctggt 240

gctgcaactg gcgctcctcc cagcagccac tcagggaaag aaagtggtgc tgggcaaaaa 300

aggggataca gtggaactga cctgtacagc ttcccagaag aagagcatac aattccactg 360
gaaaaactcc aaccagataa agattctggg aaatcagggc tccttcttaa ctaaaggtcc 420
atccaagctg aatgatcgcg ctgactcaag aagaagcctt tgggaccaag gaaactttcc 480
cctgatcatc aagaatctta agatagaaga ctcagatact tacatctgtg aagtggagga 540
ccagaaggag gaggtgcaat tgctagtgtt cggattgact gccaactctg acacccacct 600
gcttcagggg cagagcctga ccctgacctt ggagagcccc cctggtagta gcccctcagt 660
gcaatgtagg agtccaaggg gtaaaaacat acaggggggg aagaccctct ccgtgtctca 720
gctggagctc caggatagtg gcacctggac atgcactgtc ttgcagaacc agaagaaggt 780
ggagttcaaa atagacatcg tggtgctagc tttccagaag gcctccagca tagtctataa 840
gaaagagggg gaacaggtgg agttctcctt cccactcgcc tttacagttg aaaagctgac 900
gggcagtggc gagctgtggt ggcaggcgga gagggcttcc tcctccaagt cttggatcac 960
ctttgacctg aagaacaagg aagtgtctgt aaaacgggtt acccaggacc ctaagctcca 1020
gatgggcaag aagctcccgc tccacctcac cctgccccag gccttgcctc agtatgctgg 1080
ctctggaaac ctcaccctgg cccttgaagc gaaaacagga aagttgcatc aggaagtgaa 1140
cctggtggtg atgagagcca ctcagctcca gaaaaatttg acctgtgagg tgtggggacc 1200
cacctcccct aagctgatgc tgagtttgaa actggagaac aaggaggcaa aggtctcgaa 1260
gcgggagaag gcggtgtggg tgctgaaccc tgaggcgggg atgtggcagt gtctgctgag 1320
tgactcggga caggtcctgc tggaatccaa catcaaggtt ctgcccacat ggtccacccc 1380
ggtgcagcca atggccctga ttgtgctggg gggcgtcgcc ggcctcctgc ttttcattgg 1440
gctaggcatc ttcttctgtg tcaggtgccg gcaccgaagg cgccaagcag agcggatgtc 1500
tcagatcaag agactcctca gtgagaagaa gacctgccag tgtcctcacc ggtttcagaa 1560
gacatgtagc cccatttgag gcacgaggcc aggcagatcc cacttgcagc ctccccaggt 1620
gtctgccccg cgtttcctgc ctgcggacca gatgaatgta gcagatcccc agcctctggc 1680
ctcctgttcg cctcctctac aatttgccat tgtttctcct gggttaggcc ccggcttcac 1740
tggttgagtg ttgctctcta gtttccagag gcttaatcac accgtcctcc acgccatttc 1800
cttttccttc aagcctagcc cttctctcat tatttctctc tgaccctctc cccactgctc 1860
atttggatcc caggggagtg ttcagggcca gccctggctg gcatggaggg tgaggctggg 1920
tgtctggaag catggagcat gggactgttc ttttacaaga caggaccctg ggaccacaga 1980
gggcaggaac ttgcacaaaa tcacacagcc aagccagtca aggatggatg cagatccaga 2040
ggtttctggc agccagtacc tcctgcccca tgctgcccgc ttctcaccct atgtgggtgg 2100
gaccacagac tcacatcctg accttgcaca aacagcccct ctggacacag ccccatgtac 2160

acggcctcaa gggatgtctc acatcctctg tctatttgag acttagaaaa atcctacaag 2220

gctggcagtg acagaactaa gatgatcatc tccagtttat agaccagaac cagagctcag 2280

agaggctaga tgattgatta ccaagtgccg gactagcaag tgctggagtc gggactaacc 2340

caggtccctt gtcccaagtt ccactgctgc ctcttgaatg cagggacaaa tgccacacgg 2400

ctctcaccag tggctagtgg tgggtactca atgtgtactt ttgggttcac agaagcacag 2460

cacccatggg aagggtccat ctcagagaat ttacgagcag ggatgaaggc ctccctgtct 2520

aaaatccctc cttcatcccc cgctggtggc agaatctgtt accagaggac aaagcctttg 2580

gctcttctaa tcagagcgca agctgggagc acaggcactg caggagagaa tgcccagtga 2640

ccagtcactg accctgtgca gaacctcctg gaagcgagct ttgctgggag agggggtagc 2700

tagcctgaga gggaaccctc taagggacct caaaggtgat tgtgccaggc tctgcgcctg 2760

ccccacaccc tcccttaccc tcctccagac cattcaggac acagggaaat cagggttaca 2820

aatcttcttg atccacttct ctcaggatcc cctctcttcc taccettcct caccacttcc 2880

ctcagtccca actccttttc cctatttcct tctcctcctg tctttaaagc ctgcctcttc 2940

caggaagacc cccctattgc tgctggggct ccccatttgc ttactttgca tttgtgccca 3000

ctctccaccc ctgctcccct gagctgaaat aaaaatacaa taaacttac 3049

<210> 24
<211> 458
<212> PRT
<213> Homo sapiens

<400> 24

Met Asn Arg Gly Val Pro Phe Arg His Leu Leu Leu Val Leu Gln Leu
1 5 10 15

Ala Leu Leu Pro Ala Ala Thr Gln Gly Lys Lys Val Val Leu Gly Lys
20 25 30

Lys Gly Asp Thr Val Glu Leu Thr Cys Thr Ala Ser Gln Lys Lys Ser
35 40 45

Ile Gln Phe His Trp Lys Asn Ser Asn Gln Ile Lys Ile Leu Gly Asn
50 55 60

Gln Gly Ser Phe Leu Thr Lys Gly Pro Ser Lys Leu Asn Asp Arg Ala
65 70 75 80

Asp Ser Arg Arg Ser Leu Trp Asp Gln Gly Asn Phe Pro Leu Ile Ile
85 90 95

Lys Asn Leu Lys Ile Glu Asp Ser Asp Thr Tyr Ile Cys Glu Val Glu

100 105 110

Asp Gln Lys Glu Glu Val Gln Leu Leu Val Phe Gly Leu Thr Ala Asn
115 120 125

Ser Asp Thr His Leu Leu Gln Gly Gln Ser Leu Thr Leu Thr Leu Glu
130 135 140

Ser Pro Pro Gly Ser Ser Pro Ser Val Gln Cys Arg Ser Pro Arg Gly
145 150 155 160

Lys Asn Ile Gln Gly Gly Lys Thr Leu Ser Val Ser Gln Leu Glu Leu
165 170 175

Gln Asp Ser Gly Thr Trp Thr Cys Thr Val Leu Gln Asn Gln Lys Lys
180 185 190

Val Glu Phe Lys Ile Asp Ile Val Val Leu Ala Phe Gln Lys Ala Ser
195 200 205

Ser Ile Val Tyr Lys Lys Glu Gly Glu Gln Val Glu Phe Ser Phe Pro
210 215 220

Leu Ala Phe Thr Val Glu Lys Leu Thr Gly Ser Gly Glu Leu Trp Trp
225 230 235 240

Gln Ala Glu Arg Ala Ser Ser Ser Lys Ser Trp Ile Thr Phe Asp Leu
245 250 255

Lys Asn Lys Glu Val Ser Val Lys Arg Val Thr Gln Asp Pro Lys Leu
260 265 270

Gln Met Gly Lys Lys Leu Pro Leu His Leu Thr Leu Pro Gln Ala Leu
275 280 285

Pro Gln Tyr Ala Gly Ser Gly Asn Leu Thr Leu Ala Leu Glu Ala Lys
290 295 300

Thr Gly Lys Leu His Gln Glu Val Asn Leu Val Val Met Arg Ala Thr
305 310 315 320

Gln Leu Gln Lys Asn Leu Thr Cys Glu Val Trp Gly Pro Thr Ser Pro
325 330 335

Lys Leu Met Leu Ser Leu Lys Leu Glu Asn Lys Glu Ala Lys Val Ser
340 345 350

Lys Arg Glu Lys Ala Val Trp Val Leu Asn Pro Glu Ala Gly Met Trp
355 360 365

Gln Cys Leu Leu Ser Asp Ser Gly Gln Val Leu Leu Glu Ser Asn Ile
370 375 380

Lys Val Leu Pro Thr Trp Ser Thr Pro Val Gln Pro Met Ala Leu Ile
385 390 395 400

Val Leu Gly Gly Val Ala Gly Leu Leu Leu Phe Ile Gly Leu Gly Ile
405 410 415

Phe Phe Cys Val Arg Cys Arg His Arg Arg Arg Gln Ala Glu Arg Met
420 425 430

Ser Gln Ile Lys Arg Leu Leu Ser Glu Lys Lys Thr Cys Gln Cys Pro
435 440 445

His Arg Phe Gln Lys Thr Cys Ser Pro Ile
450 455

<210> 25
<211> 3968
<212> DNA
<213> Homo sapiens

<400> 25
ggcggaagcg ggagcctctg tcggccgcgg aagcctggag tgggcggtac gcagacgcgc 60

gcggtgagac ccgctgtctg ctcagcggac tctgcccgcc cccacctccc cctgcgtcgg 120

gccgacatga aggactcgct ggtgctgctg ggccgtgtcc cggcgcaccc ggactcccgc 180

tgctggttcc tggcctggaa ccccgcgggg accctgctgg cctcgtgcgg cggcgaccgg 240

agaatccgca tctggggcac ggagggtgac agctggatct gcaagtctgt cctttctgaa 300

ggccaccagc gcaccgtgcg gaaggtagcc tggtccccct gcggtaatta cctggcctct 360

gccagctttg atgctaccac ttgcatttgg aagaagaacc aggatgactt tgagtgtgta 420

accactctcg agggccatga aaatgaggtc aagtcagtgg cttgggcccc atctggcaac 480

ctcctggcca cttgcagccg agataagagc gtttgggtct gggaagttga tgaagaggat 540

gagtatgaat gtgtcagtgt tctcaactcc cacacacagg atgtcaagca tgtggtttgg 600

cacccaagtc aggagctctt agcttctgcc agctatgatg acacagtgaa gctgtaccgg 660

gaggaagagg atgactgggt atgctgtgcc acccttgagg gccatgaatc cactgtgtgg 720

agcttggcct ttgacccgag tggccagcgc ctggcgtctt gtagtgatga ccgtactgtg 780

cgtatctggc gtcagtatct accaggcaat gaacaagggg tggcatgcag cggctctgac 840

cccagttgga aatgtatctg tactttgtcc ggcttccact caaggaccat ttatgacatt 900

gcttggtgtc agctgacagg ggctctggcc acagcttgtg gggatgacgc gatccgcgtg 960

tttcaggagg atcccaactc ggatccacag cagcccacct tctccctgac agcccacttg 1020

catcaggccc attcccagga tgtcaactgt gtggcctgga accccaagga gccagggcta 1080

ctggcctcct gcagtgatga tggggaggtg gccttctgga agtatcagcg gcctgaaggc 1140

ctctgagcta cctcgacttt ggacagagta atgactcccc agaaaacgtc atataagact 1200

ttaccagccc ctgagaggac caggaggagc atccttgacc ttcatttaac ttggctcact 1260

tctcttcaga cttgggtaga agtgcagagc cacagaattg ctttccttcc ccgcctttga 1320

catgaggcct tcagtaaaga gctacagaac atgagtacat tgttatacca cagatttttc 1380

ttgcattagg gcacagtgtt aaatttttg gaggtaaata tactatttat aatcactata 1440

tatagtagga gggggtatgt gtctcaggct tttctgaagt tgcaagactt aaagaaataa 1500

tccatctgca tcccaagtcc tattttataa ggatattcat aaaaattcca tggtgaatcc 1560

ttgtctgaaa taggtcctcc cttcccagtt tctgtgtaag tctttgcatt taagacatcc 1620

aatcaataat gaaggaaatt tttttctgaa tgtaggtttg agtgaggggc acctctgctt 1680

tcccttagca accctcatat acctccctgc accgttacgc tgtgatggca actggggata 1740

gaaaaaaaat ggggaaagac aggaatccta aaagggagag ttattactgg ccacaagccc 1800

tgtattctca acagggatgc aaattggttc ttcagtaagg ataaaaaaaa atcacaagca 1860

gttgtttgtg gccctcctaa ggcccacagc acatatagtg tgtctgtgat attccatttt 1920

catggcaggg agtgatcagg aagaaggctt cctaggggac tggcgattta aaccagttga 1980

gaaacactgc catcagcagg cagtttcaga ctcactcaag ttgtctcttg acagtcactt 2040

ctaaatgggt tctaatgtga caatggcctc caaaactaca gccttccctg aagtttaagc 2100

tgtgacctta gattttagaa ggacagtggg gctgtaccta gaatagtggt tctcgaagaa 2160

tgcggcctgc agatcctggg agtcccaaga ccctttcagg gaggatctgt gaggtcaact 2220

gttggcactg tggcatgaat caaggtggtg gcagcaaact tctagtagtt ttgatatgtc 2280

cttgatagaa caaatagcaa tggttaacta ttaaatgttg acctagccag cgcagtggct 2340

catgcctgta atcccagcac tttgggaggc tgaggcgggc ggatcacctg aggtcgggag 2400

ttcgaggcca gcctgaccaa catggagaaa ccccgtctct tctaaaaata caaaattagc 2460

tgggcatggt ggtgcatgcc tgtaattcca gctactcggg aggctgaggc aagagaatcg 2520

cttgaatccg gtaggtggag gttgcagtga gccgagatca taccattgca ctccagccca 2580

ggcaacaaga gtgaaaccct gtctcaaaaa gaaaaaaaaa gttgaccttg agaatttata 2640

atattctgag aaaactggaa gcatgcataa agcccctctg ctgtgcactg aagtatgggt 2700

gccttgagga aaagcagtta cacagttgag ttgcaagctg aattggctgt gttcaaggca 2760

tgccctttag aattgaaaga actagcagat tacggtattt agacttgaat atttggctga 2820

tattttctgg aaattaatgg aatgagcctc tcacctcaag ggaaacaact gatagtgttg 2880

ccagtgataa agctttcaag caaaaattgg aatttccgaa aatctgtact ccaccatgag 2940

ctttattgtt ggggatatta acaaatgtga tttgtataat gaaatgcatt tcatttggaa 3000

gaattcaatg aaccattttt ccaagtgacc agtacgtgat gttacaaaat catgcatggc 3060

tcaaagattg attcaaaagt gtaagacagg ccagtggatt ttaatgtatt aacaatataa 3120

gagtgcatta agttttcgga gtctacattg cctttaagaa actatgactt gtagtaagcc 3180

gggcgcggtg gctcacgcct gtaatcccaa cactttggga ggccaaggtg ggtggatcac 3240

aaggtcagga gttcaagacc agcctggcca atatggtgaa agtccgtctc tactaaaatt 3300

acaaaaatta gccgggcgtg gtggcagatc ccttgtagtc ccagctactc gggaggctga 3360

ggcaggagaa tagcttgaac ccgggaggtg gaggttgcag tgagtcgaga tcgtgccact 3420

ggactccagc ctgggtgaca gagcgagact ccatttcaaa aaaaaaaaaa aaaaaaaaaa 3480

atcacttgta gtcttggtgt ggtatcaaag aatagccaca attagctgaa aaggctattt 3540

taaaaacttt tccaactgcg tatctgtgtg aagtcaactt acttcaacaa aaaagtttgg 3600

atgtagaagc agctgtaaga attcaactgt ttattataac aagatactaa agagactgta 3660

aaatgccacc cttctccttg gattgttttg gaagttattc ttcataaaaa atgttaacgt 3720

gggctgggca tggtggctca tgcctgtaat cccagcactc tgggaggctg aggtgggcgg 3780

atcacttgag ctcaggaatt caaggtcagc ctgggcaaca tggctaaact ctgtctctat 3840

taagaaaaaa aatgttaaca ttatgattta aaagtgcatt aaccttaatc tagataataa 3900

aagcttttg gggcaacctc cagaactgtg aaaaataaat ttgttattta aaaagaaaaa 3960

aaaaaaaa 3968

<210> 26
<211> 339
<212> PRT
<213> Homo sapiens

<400> 26

Met Lys Asp Ser Leu Val Leu Leu Gly Arg Val Pro Ala His Pro Asp
1 5 10 15

Ser Arg Cys Trp Phe Leu Ala Trp Asn Pro Ala Gly Thr Leu Leu Ala
20 25 30

Ser Cys Gly Gly Asp Arg Arg Ile Arg Ile Trp Gly Thr Glu Gly Asp
35 40 45

Ser Trp Ile Cys Lys Ser Val Leu Ser Glu Gly His Gln Arg Thr Val

50 55 60

Arg Lys Val Ala Trp Ser Pro Cys Gly Asn Tyr Leu Ala Ser Ala Ser
65 70 75 80

Phe Asp Ala Thr Thr Cys Ile Trp Lys Lys Asn Gln Asp Asp Phe Glu
85 90 95

Cys Val Thr Thr Leu Glu Gly His Glu Asn Glu Val Lys Ser Val Ala
100 105 110

Trp Ala Pro Ser Gly Asn Leu Leu Ala Thr Cys Ser Arg Asp Lys Ser
115 120 125

Val Trp Val Trp Glu Val Asp Glu Glu Asp Glu Tyr Glu Cys Val Ser
130 135 140

Val Leu Asn Ser His Thr Gln Asp Val Lys His Val Val Trp His Pro
145 150 155 160

Ser Gln Glu Leu Leu Ala Ser Ala Ser Tyr Asp Asp Thr Val Lys Leu
165 170 175

Tyr Arg Glu Glu Glu Asp Asp Trp Val Cys Cys Ala Thr Leu Glu Gly
180 185 190

His Glu Ser Thr Val Trp Ser Leu Ala Phe Asp Pro Ser Gly Gln Arg
195 200 205

Leu Ala Ser Cys Ser Asp Asp Arg Thr Val Arg Ile Trp Arg Gln Tyr
210 215 220

Leu Pro Gly Asn Glu Gln Gly Val Ala Cys Ser Gly Ser Asp Pro Ser
225 230 235 240

Trp Lys Cys Ile Cys Thr Leu Ser Gly Phe His Ser Arg Thr Ile Tyr
245 250 255

Asp Ile Ala Trp Cys Gln Leu Thr Gly Ala Leu Ala Thr Ala Cys Gly
260 265 270

Asp Asp Ala Ile Arg Val Phe Gln Glu Asp Pro Asn Ser Asp Pro Gln
275 280 285

Gln Pro Thr Phe Ser Leu Thr Ala His Leu His Gln Ala His Ser Gln
290 295 300

Asp Val Asn Cys Val Ala Trp Asn Pro Lys Glu Pro Gly Leu Leu Ala
305 310 315 320

Ser Cys Ser Asp Asp Gly Glu Val Ala Phe Trp Lys Tyr Gln Arg Pro
325 330 335

Glu Gly Leu

<210> 27
<211> 2755
<212> DNA
<213> Homo sapiens

<400> 27
ccgggccgcg cttcctctcg ccaggcctgc gagcttcctc ccagcggagc cctgggcgag 60

ccgaggttgg ccgccgccgc cgccgagccc gctgccgccc tcccgctcct gccccacccg 120

cgccttgccc gggggcttct gccggggtgg ggtccgagcc gggcgaccgc ccggctgcgc 180

cgccgtcggg gccgtaaccc ggcccgccgt ccctcccgcc ccagccagcc tctggccgcc 240

ggagcccgcg gggcgtggag cgcgaggagc cccgcggccc cgatcgagcg tccggggcgg 300

cccccggcag ccagcgcgac gttccaaaat cgaacctcag tggcggcgct cggaagcgga 360

actctgccgg ggccgcgccg gctacattgt ttcctccccc cgactccctc ccgccccctt 420

cccccgcctt tcttccctcc gcgacccggg ccgtgcgtcc gtcccctgc ctctgcctgg 480

cggtccctcc tcccctctcc ttgcacccat acctctttgt accgcacccc ctggggaccc 540

ctgcgcccct cccctccccc ctgaccgcat ggaccgtccc gcaggccgct gatgccgccc 600

gcggcgaggt ggcccggacc gcagtgcccc aagagagctc taatggtacc aagtgacagg 660

ttggctttac tgtgactcgg ggacgccaga gctcctgaga agatgtcagc aatacaggcc 720

gcctggccat ccggtacaga atgtattgcc aagtacaact tccacggcac tgccgagcag 780

gacctgccct tctgcaaagg agacgtgctc accattgtgg ccgtcaccaa ggaccccaac 840

tggtacaaag ccaaaaacaa ggtgggccgt gagggcatca tcccagccaa ctacgtccag 900

aagcgggagg gcgtgaaggc gggtaccaaa ctcagcctca tgccttggtt ccacggcaag 960

atcacacggg agcaggctga gcggcttctg tacccgccgg agacaggcct gttcctggtg 1020

cgggagagca ccaactaccc cggagactac acgctgtgcg tgagctgcga cggcaaggtg 1080

gagcactacc gcatcatgta ccatgccagc aagctcagca tcgacgagga ggtgtacttt 1140

gagaacctca tgcagctggt ggagcactac acctcagacg cagatggact ctgtacgcgc 1200

ctcattaaac caaaggtcat ggagggcaca gtggcggccc aggatgagtt ctaccgcagc 1260

ggctgggccc tgaacatgaa ggagctgaag ctgctgcaga ccatcgggaa gggggagttc 1320

ggagacgtga tgctgggcga ttaccgaggg aacaaagtcg ccgtcaagtg cattaagaac 1380

```
gacgccactg cccaggcctt cctggctgaa gcctcagtca tgacgcaact gcggcatagc      1440
aacctggtgc agctcctggg cgtgatcgtg gaggagaagg gcgggctcta catcgtcact      1500
gagtacatgg ccaaggggag ccttgtggac tacctgcggt ctaggggtcg gtcagtgctg      1560
ggcggagact gtctcctcaa gttctcgcta gatgtctgcg aggccatgga atacctggag      1620
ggcaacaatt tcgtgcatcg agacctggct gcccgcaatg tgctggtgtc tgaggacaac      1680
gtggccaagg tcagcgactt tggtctcacc aaggaggcgt ccagcaccca ggacacgggc      1740
aagctgccag tcaagtggac agcccctgag gccctgagag agaagaaatt ctccactaag      1800
tctgacgtgt ggagtttcgg aatccttctc tgggaaatct actcctttgg gcgagtgcct      1860
tatccaagaa ttcccctgaa ggacgtcgtc cctcgggtgg agaagggcta caagatggat      1920
gcccccgacg gctgcccgcc cgcagtctat gaagtcatga agaactgctg gcacctggac      1980
gccgccatgc ggccctcctt cctacagctc cgagagcagc ttgagcacat caaaacccac      2040
gagctgcacc tgtgacggct ggcctccgcc tgggtcatgg gcctgtgggg actgaacctg      2100
gaagatcatg gacctggtgc ccctgctcac tgggcccgag cctgaactga gccccagcgg      2160
gctggcgggc ctttttcctg cgtcccagcc tgcacccctc cggccccgtc tctcttggac      2220
ccacctgtgg ggcctgggga gcccactgag gggccaggga ggaaggaggc cacggagcgg      2280
gaggcagcgc cccaccacgt cgggcttccc tggcctcccg ccactcgcct tcttagagtt      2340
ttattccttt cctttttga gattttttt ccgtgtgttt atttttatt atttttcaag       2400
ataaggagaa agaaagtacc cagcaaatgg gcattttaca agaagtacga atcttatttt      2460
tcctgtcctg cccgtgaggg tgggggggac cgggcccctc tctagggacc cctcgcccca      2520
gcctcattcc ccattctgtg tcccatgtcc cgtgtctcct cggtcgcccc gtgtttgcgc      2580
ttgaccatgt tgcactgttt gcatgcgccc gaggcagacg tctgtcaggg gcttggattt      2640
cgtgtgccgc tgccacccgc ccacccgcct tgtgagatgg aattgtaata aaccacgcca      2700
tgaggacacc gccgcccgcc tcggcgcttc ctccaccgag aaaaaaaaaa aaaaa           2755
```

<210> 28
<211> 450
<212> PRT
<213> Homo sapiens

<400> 28

```
Met Ser Ala Ile Gln Ala Ala Trp Pro Ser Gly Thr Glu Cys Ile Ala
1               5                   10                  15

Lys Tyr Asn Phe His Gly Thr Ala Glu Gln Asp Leu Pro Phe Cys Lys
            20                  25                  30
```

Gly Asp Val Leu Thr Ile Val Ala Val Thr Lys Asp Pro Asn Trp Tyr
35 40 45

Lys Ala Lys Asn Lys Val Gly Arg Glu Gly Ile Ile Pro Ala Asn Tyr
50 55 60

Val Gln Lys Arg Glu Gly Val Lys Ala Gly Thr Lys Leu Ser Leu Met
65 70 75 80

Pro Trp Phe His Gly Lys Ile Thr Arg Glu Gln Ala Glu Arg Leu Leu
85 90 95

Tyr Pro Pro Glu Thr Gly Leu Phe Leu Val Arg Glu Ser Thr Asn Tyr
100 105 110

Pro Gly Asp Tyr Thr Leu Cys Val Ser Cys Asp Gly Lys Val Glu His
115 120 125

Tyr Arg Ile Met Tyr His Ala Ser Lys Leu Ser Ile Asp Glu Glu Val
130 135 140

Tyr Phe Glu Asn Leu Met Gln Leu Val Glu His Tyr Thr Ser Asp Ala
145 150 155 160

Asp Gly Leu Cys Thr Arg Leu Ile Lys Pro Lys Val Met Glu Gly Thr
165 170 175

Val Ala Ala Gln Asp Glu Phe Tyr Arg Ser Gly Trp Ala Leu Asn Met
180 185 190

Lys Glu Leu Lys Leu Leu Gln Thr Ile Gly Lys Gly Glu Phe Gly Asp
195 200 205

Val Met Leu Gly Asp Tyr Arg Gly Asn Lys Val Ala Val Lys Cys Ile
210 215 220

Lys Asn Asp Ala Thr Ala Gln Ala Phe Leu Ala Glu Ala Ser Val Met
225 230 235 240

Thr Gln Leu Arg His Ser Asn Leu Val Gln Leu Leu Gly Val Ile Val
245 250 255

Glu Glu Lys Gly Gly Leu Tyr Ile Val Thr Glu Tyr Met Ala Lys Gly
260 265 270

Ser Leu Val Asp Tyr Leu Arg Ser Arg Gly Arg Ser Val Leu Gly Gly
275 280 285

Asp Cys Leu Leu Lys Phe Ser Leu Asp Val Cys Glu Ala Met Glu Tyr
290 295 300

Leu Glu Gly Asn Asn Phe Val His Arg Asp Leu Ala Ala Arg Asn Val
305 310 315 320

Leu Val Ser Glu Asp Asn Val Ala Lys Val Ser Asp Phe Gly Leu Thr
325 330 335

Lys Glu Ala Ser Ser Thr Gln Asp Thr Gly Lys Leu Pro Val Lys Trp
340 345 350

Thr Ala Pro Glu Ala Leu Arg Glu Lys Lys Phe Ser Thr Lys Ser Asp
355 360 365

Val Trp Ser Phe Gly Ile Leu Leu Trp Glu Ile Tyr Ser Phe Gly Arg
370 375 380

Val Pro Tyr Pro Arg Ile Pro Leu Lys Asp Val Val Pro Arg Val Glu
385 390 395 400

Lys Gly Tyr Lys Met Asp Ala Pro Asp Gly Cys Pro Pro Ala Val Tyr
405 410 415

Glu Val Met Lys Asn Cys Trp His Leu Asp Ala Ala Met Arg Pro Ser
420 425 430

Phe Leu Gln Leu Arg Glu Gln Leu Glu His Ile Lys Thr His Glu Leu
435 440 445

His Leu
450

<210> 29
<211> 6855
<212> DNA
<213> Homo sapiens

<400> 29
ggccggaggg cgcccgaggg gccccgggcc gcggcgctca gggcccgggc ggccggcggc 60

ggcccccgggg ctggggggag tccagcccgg atattgagtg cagccattga gaaaagccaa 120

actcttgtgt gtgcgcgtct cgatagcccc caagatggcc gccaatgtgg gatcgatgtt 180

tcaatattgg aagcgatttg atctacggcg actccagaag gagcttaatt ccgtcgcttc 240

tgagctgtct gcacggcagg aggagagtga acattctcat aaacatttaa ttgaactccg 300

ccgggaattt aagaaaaatg tacctgagga aatcagagag atggtggctc ctgtattaaa 360

aagcttccaa gccgaggtgg tggcccttag taagagaagt caggaggcgg aggctgcttt 420
tctgagtgtt tacaagcaat taattgaagc accagacccc gtgcctgtgt ttgaggcggc 480
acgcagccta gacgacagac tgcagccccc cagctttgac cccagtgggc agccccggcg 540
agacctccac acttcgtgga agaggaaccc cgagctcctc agccccaaag agcagagaga 600
ggggacgtcg cctgccgggc ccacgctgac cgagggaagc cgcctcccag gcattcccgg 660
gaaagccctc ctgacagaaa ccttgctgca gagaaatgag gcggaaaaac aaaagggcct 720
tcaagaagta cagatcactt tggcggccag actgggggag gcagaggaga aaatcaaagt 780
cctacattca gcgctaaagg ctacgcaggc agagctgcta gagctgcggc ggaagtacga 840
cgaggaggca gcatccaagg cagatgaagt cggcctgatc atgaccaacc tggagaaagc 900
taatcagcga gctgaggctg cccagcggga ggtggaaagt ctccgggaac agctggcctc 960
tgtcaacagc tccatccgcc tggcttgctg ctctccccag ggcccagtg gggataaggt 1020
gaacttcact ctgtgctcgg gccctcggct ggaggccgcg ctggcctcca aggacaggga 1080
gatcctgcgg ctgctgaagg acgtgcagca cctccagagc tcactgcagg agctggagga 1140
ggcatccgcc aaccagatcg ccgacctgga gcggcagctc acggccaagt ccgaggccat 1200
agaaaagctg gaagagaagc tccaggccca gtctgactat gaggaaatta aaacggagct 1260
gagcatcctg aaagccatga agctggcctc cagcacctgc agcctccccc agggcatggc 1320
caagcctgaa gactcactgc ttattgcaaa ggaggccttc ttccccacgc agaaattcct 1380
tctggagaag cccagcctcc tggccagccc tgaggaagac ccatcagagg acgattccat 1440
caaggattca ctgggcacgg agcagtccta cccctcccct cagcagctcc cacctccacc 1500
agggccagaa gaccccctgt ctcccagccc cgggcagccc ctgctgggcc ccagcttggg 1560
gcctgacggc actcggactt tctcgctgtc cccttcccc agcctggcat caggggagag 1620
actgatgatg cccccagccg ccttcaaggg agaggcgggc ggcctgctgg tgttcccccc 1680
agccttctat ggcgccaagc cccccacagc ccctgccacc ccggcccctg gccctgagcc 1740
actgggcggt cctgagcccg cggatggtgg tgggggcgga gcggcggggc ccggggcaga 1800
ggaggagcag ctggacacgg cagagatcgc cttccaggtg aaggagcagc tgctgaaaca 1860
caacatcggg cagcgggtgt ttgggcatta cgtgctgggg ctgtcgcagg gctcggtcag 1920
cgagatccta gcccggccca agccctggcg caagctcacg gtgaagggca aggagccctt 1980
catcaagatg aagcagttcc tgtcggatga gcagaatgta ctggcgctca ggaccatcca 2040
agtgcggcag cgaggcagca tcaccccgag aatccgcacg cctgagacag gctcagacga 2100
cgccatcaag agcattctag agcaggccaa gaaggagatc gagtcgcaga agggcggcga 2160
gcccaagacc tcggtggccc cgctgagcat cgccaacggc acgacccccg ccagcacctc 2220
ggaggacgcc atcaagagca tcctggagca ggcacgccgt gagatgcagg cgcaacagca 2280

ggcgctgctg gagatggagg tggcgcccag gggccgctcg gtgcccccct cgcccccgga 2340

gcggccatca ctggccaccg cgagccagaa cggggccccg gccttggtga agcaggagga 2400

gggcagcggg ggccccgcgc aggcgccgct cccggtcctg tcccccgccg ccttcgtgca 2460

gagcatcatc cgcaaggtca agtccgagat cggcgacgcc ggctacttcg accaccactg 2520

ggcctccgac cgcggcctgc tcagccgccc ctacgcctcc gtgtcgccct cgctgtcctc 2580

ctcctcctcc tctggctact ctggccagcc caacggccgc gcctggcccc gcggggacga 2640

ggcccctgtg ccccccgagg acgaggcggc ggcaggggcg gaggacgaac cccccaggac 2700

gggcgagctc aaggctgagg gcgcgacggc cgaggcgggc gcgcggctgc cctactaccc 2760

ggcctacgtg ccgcgcaccc tgaagcccac cgtgccgccg ctgacccccg agcagtacga 2820

gctgtacatg taccgtgagg tagacacgct ggagctcacc cgccaggtca aggagaagct 2880

ggccaagaac ggcatctgcc agaggatctt cggggagaag gtgctgggcc tgtcacaggg 2940

cagcgtgagc gacatgctgt cccggccgaa gccatggagc aagctgacgc agaaggggcg 3000

ggagcccttc atccgcatgc agctgtggct ctctgaccag ctcggccagg cagtgggcca 3060

gcagcctggt gcctcccagg ccagtcccac agaaccaagg tcctcaccat ccccaccccc 3120

cagccccaca gagcctgaga agagctccca ggagccgttg agcctgtccc tggagagcag 3180

caaggagaac cagcagccag agggccgctc cagctcctcg ttgagcggga agatgtactc 3240

aggcagccag gccccagggg gcatccagga gatcgtggcc atgtccccgg agctggacac 3300

gtactccatc accaagaggg tgaaggaggt cctcacagac aacaatctag ggcagcggct 3360

gtttggggaa agcatcctgg gtctgacaca gggctccgtg tctgacctgc tgtcccggcc 3420

caaaccctgg cacaagctga gcctgaaggg gcgggagcct tttgtccgca tgcagctgtg 3480

gctcaatgac ccccataacg tggagaagct gagggatatg aagaagctgg agaagaaagc 3540

ctacctgaaa cgtcgctatg gcctcatcag caccggctca gacagtgagt ccccggccac 3600

ccgctcagag tgccccagcc cctgcctgca gccccaggac ctgagcctcc tgcagatcaa 3660

gaagccccgg gtggtgctgg cacccgagga gaaggaggca ctgcggaagg cctatcagct 3720

ggaaccctac ccctcgcagc agaccatcga gctcctctcc ttccagctca acctcaagac 3780

caacaccgtc atcaactggt tccacaacta caggtcccgg atgcgccggg agatgttggt 3840

ggaggggacc caggatgagc cagaccttga tccaagcggg ggtcctggaa tcctaccgcc 3900

aggccactcc cacccagacc ccaccccgca gagccctgac tctgagactg aggaccagaa 3960

gccaaccgtg aaggaactgg agcttcagga gggccctgag gagaacagca cacccctgac 4020

cacccaggac aaggcccaag tgaggatcaa gcaggaacag atggaggagg atgctgagga 4080

agaggcaggc agccagcccc aggactcagg ggagctggac aaaggccaag gtccccccaa 4140

agaggagcat cccgaccctc cgggtaatga tggactccca aaagtggctc ccgggcccct 4200
ccttccaggt ggatccaccc cagactgtcc ctcacttcat ccccaacagg agagtgaggc 4260
cggggagcga cttcacccgg accctttaag ttttaagtca gcctcagagt cctcacgctg 4320
cagcctggag gtgtcactga actcgccctc ggccgcctcc tcaccaggcc tcatgatgtc 4380
tgtgtcacct gtcccctcct cctcagctcc catctcccca tccccacctg gcgccccccc 4440
tgccaaagtg ccgagtgcca gccccactgc tgacatggct ggagccttgc accccagtgc 4500
caaggtgaac cccaacttgc agcggcggca tgagaagatg gccaatctga acaacatcat 4560
ttaccgagta gagcgggctg ccaatcggga ggaggccctg gagtgggagt tctgaaggca 4620
gggtgagggg gcaagggaca taccctggta actaccttcc ttctcgcact tactctcctc 4680
aacaggatgg ggtaagggag ggaggaactc aaccatcaaa atgtggacag caatgttatg 4740
ccgtttacgt tttttgttgt aatcctagtt ctatgaagct gtgtgagcag gtgggtcaaa 4800
tgccattgcc tccacttttc tgcacccccc tgctcctctt caccctgacc cctctgcagg 4860
aggcagaagc aaaatggcac cacatattca cctgaaaact ccaaactctt ttagaaaaat 4920
aaataaatat ttatagacct cttttagata ttttaataaa ggatcctttg gaatttatcc 4980
cagctgatgc tgttttgata ttacagagag ttataaaatc aggatgctgt cacaactgtt 5040
gcgaagtata cactgaagtt gtgtcgtttt tgccactaga tgagattaaa agaagacaat 5100
tattcaaagc catcacaaaa cactataaga ctgaccaaaa tttagataac ctttgaacca 5160
cgattttttt ccacatctgt ctgtgagaca cagcgcaatg ctactgccct tccagaaact 5220
gtgctaaaaa gagaaagtcc aaaagactct aaacaaaaac ctcgacgccg ttgaggatgt 5280
gtttcattct ggtggtctgt tttgcaagct tgataacaga atgtccgtgc cattgtaaat 5340
gttgtagaga tgtgggccgt ggcccaaccg tcctatatga gatgtagcat ggtacagaac 5400
aaactgctta cacaggtctc actagttaga aacctgtggg ccatggaggt cagacatcca 5460
tcttgtccat ctataggcaa gaagtgtttc cagatccttt ggaaaggtgg gcatggggca 5520
ggtgcttgga gagtggcgtt tgagccagag cgaccccatt tcccgtgtga accataggca 5580
caacccagga agtttcccca cttgtaggag tgtgggtatt ccagagcaag actgtggcca 5640
ccatcttccc ctcttggtgt tttccgaaag tgacagtgtt ggtcatccca tgaccactga 5700
agcttagtaa ccagcgccaa aaagtagatt catcaaacta gagaccccag ctccccttct 5760
cgccatcttc tttctcaagt tgaccgtggt gctgtttctg gaaggcatct gcaactccaa 5820
gtccatgcag aactctggaa ggccaagttc atcgcagcat gttcaccata tcccagcctc 5880
caaatctatc ctcctacctt ccaacgcatg acctgttggg gagcagagac ttaaccccca 5940
actcagagga acccttcctc cagcgtcttt ggcatggttt ctagggtgag agttcccaat 6000
ttggatagaa cggccaccat attggttact gaatctctct cccttgtttt tattacgttt 6060

cctttttcaa actgtccatg ggaaggctga attgagtgac tccccagaat gaagatgaga 6120

aggtgaatat aatcaatgcc aatgtaatgc cagcgggtga gatggccgat ggaggtttca 6180

aagatgtagc tagcattttg aaaccatatg ggcaaaaccc ggcaaccaga aggggacaga 6240

taaggaccgt tccagaaatc ccaactctca cacccagccc aggctgcagt ctccacacca 6300

aacagtcaac aaaacacaaa ccctgaagga aaacctttc catacaccca ggctatgcat 6360

tgaagagttt tccactgtat acatttttat ccagatgaag gtatttttat attttgacaa 6420

taggaaacag tgaccatttt cagagtaatc aaatctggaa caaatgaaac atcttttagc 6480

caccaccacc ctgttgcaat taagacaacc gtgggggaac acaccacttt ttactgttga 6540

aaccaacaca acgttgaaat ccaggcttat acgcagactc cgattcctag agaactaaat 6600

ttggctttag tgtgacggga tttgattaag cacttagtat agtcttttga acacggaaat 6660

cctgttgtac ttaaagctag cggacccgtg aacaactttg tcaggttcac gtcctataac 6720

ggttaaaaaa cacacacaca catacacaaa ccgtttctat gagagattga tgaactttgt 6780

ttaaaatttt aaaaaaagga acacgttctg taaacgagtc gctaaataca gaattgtata 6840

ataaaaaaaa aaaaa 6855

<210> 30
<211> 1486
<212> PRT
<213> Homo sapiens

<400> 30

Met Ala Ala Asn Val Gly Ser Met Phe Gln Tyr Trp Lys Arg Phe Asp
1               5                   10                  15

Leu Arg Arg Leu Gln Lys Glu Leu Asn Ser Val Ala Ser Glu Leu Ser
            20                  25                  30

Ala Arg Gln Glu Glu Ser Glu His Ser His Lys His Leu Ile Glu Leu
        35                  40                  45

Arg Arg Glu Phe Lys Lys Asn Val Pro Glu Glu Ile Arg Glu Met Val
    50                  55                  60

Ala Pro Val Leu Lys Ser Phe Gln Ala Glu Val Val Ala Leu Ser Lys
65                  70                  75                  80

Arg Ser Gln Glu Ala Glu Ala Ala Phe Leu Ser Val Tyr Lys Gln Leu
                85                  90                  95

Ile Glu Ala Pro Asp Pro Val Pro Val Phe Glu Ala Ala Arg Ser Leu
            100                 105                 110

```
Asp Asp Arg Leu Gln Pro Pro Ser Phe Asp Pro Ser Gly Gln Pro Arg
        115                 120                 125

Arg Asp Leu His Thr Ser Trp Lys Arg Asn Pro Glu Leu Leu Ser Pro
    130                 135                 140

Lys Glu Gln Arg Glu Gly Thr Ser Pro Ala Gly Pro Thr Leu Thr Glu
145                 150                 155                 160

Gly Ser Arg Leu Pro Gly Ile Pro Gly Lys Ala Leu Leu Thr Glu Thr
                165                 170                 175

Leu Leu Gln Arg Asn Glu Ala Glu Lys Gln Lys Gly Leu Gln Glu Val
            180                 185                 190

Gln Ile Thr Leu Ala Ala Arg Leu Gly Glu Ala Glu Glu Lys Ile Lys
        195                 200                 205

Val Leu His Ser Ala Leu Lys Ala Thr Gln Ala Glu Leu Leu Glu Leu
    210                 215                 220

Arg Arg Lys Tyr Asp Glu Glu Ala Ala Ser Lys Ala Asp Glu Val Gly
225                 230                 235                 240

Leu Ile Met Thr Asn Leu Glu Lys Ala Asn Gln Arg Ala Glu Ala Ala
                245                 250                 255

Gln Arg Glu Val Glu Ser Leu Arg Glu Gln Leu Ala Ser Val Asn Ser
            260                 265                 270

Ser Ile Arg Leu Ala Cys Cys Ser Pro Gln Gly Pro Ser Gly Asp Lys
        275                 280                 285

Val Asn Phe Thr Leu Cys Ser Gly Pro Arg Leu Glu Ala Ala Leu Ala
    290                 295                 300

Ser Lys Asp Arg Glu Ile Leu Arg Leu Leu Lys Asp Val Gln His Leu
305                 310                 315                 320

Gln Ser Ser Leu Gln Glu Leu Glu Glu Ala Ser Ala Asn Gln Ile Ala
                325                 330                 335

Asp Leu Glu Arg Gln Leu Thr Ala Lys Ser Glu Ala Ile Glu Lys Leu
            340                 345                 350

Glu Glu Lys Leu Gln Ala Gln Ser Asp Tyr Glu Glu Ile Lys Thr Glu
```

355 360 365

Leu Ser Ile Leu Lys Ala Met Lys Leu Ala Ser Ser Thr Cys Ser Leu
370 375 380

Pro Gln Gly Met Ala Lys Pro Glu Asp Ser Leu Leu Ile Ala Lys Glu
385 390 395 400

Ala Phe Phe Pro Thr Gln Lys Phe Leu Leu Glu Lys Pro Ser Leu Leu
405 410 415

Ala Ser Pro Glu Glu Asp Pro Ser Glu Asp Asp Ser Ile Lys Asp Ser
420 425 430

Leu Gly Thr Glu Gln Ser Tyr Pro Ser Pro Gln Gln Leu Pro Pro Pro
435 440 445

Pro Gly Pro Glu Asp Pro Leu Ser Pro Ser Pro Gly Gln Pro Leu Leu
450 455 460

Gly Pro Ser Leu Gly Pro Asp Gly Thr Arg Thr Phe Ser Leu Ser Pro
465 470 475 480

Phe Pro Ser Leu Ala Ser Gly Glu Arg Leu Met Met Pro Pro Ala Ala
485 490 495

Phe Lys Gly Glu Ala Gly Gly Leu Leu Val Phe Pro Pro Ala Phe Tyr
500 505 510

Gly Ala Lys Pro Pro Thr Ala Pro Ala Thr Pro Ala Pro Gly Pro Glu
515 520 525

Pro Leu Gly Gly Pro Glu Pro Ala Asp Gly Gly Gly Gly Gly Ala Ala
530 535 540

Gly Pro Gly Ala Glu Glu Glu Gln Leu Asp Thr Ala Glu Ile Ala Phe
545 550 555 560

Gln Val Lys Glu Gln Leu Leu Lys His Asn Ile Gly Gln Arg Val Phe
565 570 575

Gly His Tyr Val Leu Gly Leu Ser Gln Gly Ser Val Ser Glu Ile Leu
580 585 590

Ala Arg Pro Lys Pro Trp Arg Lys Leu Thr Val Lys Gly Lys Glu Pro
595 600 605

Phe Ile Lys Met Lys Gln Phe Leu Ser Asp Glu Gln Asn Val Leu Ala
610 615 620

Leu Arg Thr Ile Gln Val Arg Gln Arg Gly Ser Ile Thr Pro Arg Ile
625 630 635 640

Arg Thr Pro Glu Thr Gly Ser Asp Asp Ala Ile Lys Ser Ile Leu Glu
645 650 655

Gln Ala Lys Lys Glu Ile Glu Ser Gln Lys Gly Gly Glu Pro Lys Thr
660 665 670

Ser Val Ala Pro Leu Ser Ile Ala Asn Gly Thr Thr Pro Ala Ser Thr
675 680 685

Ser Glu Asp Ala Ile Lys Ser Ile Leu Glu Gln Ala Arg Arg Glu Met
690 695 700

Gln Ala Gln Gln Gln Ala Leu Leu Glu Met Glu Val Ala Pro Arg Gly
705 710 715 720

Arg Ser Val Pro Pro Ser Pro Pro Glu Arg Pro Ser Leu Ala Thr Ala
725 730 735

Ser Gln Asn Gly Ala Pro Ala Leu Val Lys Gln Glu Glu Gly Ser Gly
740 745 750

Gly Pro Ala Gln Ala Pro Leu Pro Val Leu Ser Pro Ala Ala Phe Val
755 760 765

Gln Ser Ile Ile Arg Lys Val Lys Ser Glu Ile Gly Asp Ala Gly Tyr
770 775 780

Phe Asp His His Trp Ala Ser Asp Arg Gly Leu Leu Ser Arg Pro Tyr
785 790 795 800

Ala Ser Val Ser Pro Ser Leu Ser Ser Ser Ser Ser Ser Gly Tyr Ser
805 810 815

Gly Gln Pro Asn Gly Arg Ala Trp Pro Arg Gly Asp Glu Ala Pro Val
820 825 830

Pro Pro Glu Asp Glu Ala Ala Ala Gly Ala Glu Asp Glu Pro Pro Arg
835 840 845

Thr Gly Glu Leu Lys Ala Glu Gly Ala Thr Ala Glu Ala Gly Ala Arg
850 855 860

```
Leu Pro Tyr Tyr Pro Ala Tyr Val Pro Arg Thr Leu Lys Pro Thr Val
865                 870                 875                 880

Pro Pro Leu Thr Pro Glu Gln Tyr Glu Leu Tyr Met Tyr Arg Glu Val
                885                 890                 895

Asp Thr Leu Glu Leu Thr Arg Gln Val Lys Glu Lys Leu Ala Lys Asn
            900                 905                 910

Gly Ile Cys Gln Arg Ile Phe Gly Glu Lys Val Leu Gly Leu Ser Gln
        915                 920                 925

Gly Ser Val Ser Asp Met Leu Ser Arg Pro Lys Pro Trp Ser Lys Leu
    930                 935                 940

Thr Gln Lys Gly Arg Glu Pro Phe Ile Arg Met Gln Leu Trp Leu Ser
945                 950                 955                 960

Asp Gln Leu Gly Gln Ala Val Gly Gln Gln Pro Gly Ala Ser Gln Ala
                965                 970                 975

Ser Pro Thr Glu Pro Arg Ser Ser Pro Ser Pro Pro Pro Ser Pro Thr
            980                 985                 990

Glu Pro Glu Lys Ser Ser Gln Glu  Pro Leu Ser Leu Ser  Leu Glu Ser
        995                 1000                 1005

Ser Lys  Glu Asn Gln Gln Pro  Glu Gly Arg Ser Ser  Ser Ser Leu
    1010                 1015                 1020

Ser Gly  Lys Met Tyr Ser Gly  Ser Gln Ala Pro Gly  Gly Ile Gln
    1025                 1030                 1035

Glu Ile  Val Ala Met Ser Pro  Glu Leu Asp Thr Tyr  Ser Ile Thr
    1040                 1045                 1050

Lys Arg  Val Lys Glu Val Leu  Thr Asp Asn Asn Leu  Gly Gln Arg
    1055                 1060                 1065

Leu Phe  Gly Glu Ser Ile Leu  Gly Leu Thr Gln Gly  Ser Val Ser
    1070                 1075                 1080

Asp Leu  Leu Ser Arg Pro Lys  Pro Trp His Lys Leu  Ser Leu Lys
    1085                 1090                 1095

Gly Arg  Glu Pro Phe Val Arg  Met Gln Leu Trp Leu  Asn Asp Pro
    1100                 1105                 1110
```

His Asn Val Glu Lys Leu Arg Asp Met Lys Lys Leu Glu Lys Lys
1115 1120 1125

Ala Tyr Leu Lys Arg Arg Tyr Gly Leu Ile Ser Thr Gly Ser Asp
1130 1135 1140

Ser Glu Ser Pro Ala Thr Arg Ser Glu Cys Pro Ser Pro Cys Leu
1145 1150 1155

Gln Pro Gln Asp Leu Ser Leu Leu Gln Ile Lys Lys Pro Arg Val
1160 1165 1170

Val Leu Ala Pro Glu Glu Lys Glu Ala Leu Arg Lys Ala Tyr Gln
1175 1180 1185

Leu Glu Pro Tyr Pro Ser Gln Gln Thr Ile Glu Leu Leu Ser Phe
1190 1195 1200

Gln Leu Asn Leu Lys Thr Asn Thr Val Ile Asn Trp Phe His Asn
1205 1210 1215

Tyr Arg Ser Arg Met Arg Arg Glu Met Leu Val Glu Gly Thr Gln
1220 1225 1230

Asp Glu Pro Asp Leu Asp Pro Ser Gly Gly Pro Gly Ile Leu Pro
1235 1240 1245

Pro Gly His Ser His Pro Asp Pro Thr Pro Gln Ser Pro Asp Ser
1250 1255 1260

Glu Thr Glu Asp Gln Lys Pro Thr Val Lys Glu Leu Glu Leu Gln
1265 1270 1275

Glu Gly Pro Glu Glu Asn Ser Thr Pro Leu Thr Thr Gln Asp Lys
1280 1285 1290

Ala Gln Val Arg Ile Lys Gln Glu Gln Met Glu Glu Asp Ala Glu
1295 1300 1305

Glu Glu Ala Gly Ser Gln Pro Gln Asp Ser Gly Glu Leu Asp Lys
1310 1315 1320

Gly Gln Gly Pro Pro Lys Glu Glu His Pro Asp Pro Pro Gly Asn
1325 1330 1335

Asp Gly Leu Pro Lys Val Ala Pro Gly Pro Leu Leu Pro Gly Gly

1340 1345 1350

Ser Thr Pro Asp Cys Pro Ser Leu His Pro Gln Gln Glu Ser Glu
1355 1360 1365

Ala Gly Glu Arg Leu His Pro Asp Pro Leu Ser Phe Lys Ser Ala
1370 1375 1380

Ser Glu Ser Ser Arg Cys Ser Leu Glu Val Ser Leu Asn Ser Pro
1385 1390 1395

Ser Ala Ala Ser Ser Pro Gly Leu Met Met Ser Val Ser Pro Val
1400 1405 1410

Pro Ser Ser Ser Ala Pro Ile Ser Pro Ser Pro Pro Gly Ala Pro
1415 1420 1425

Pro Ala Lys Val Pro Ser Ala Ser Pro Thr Ala Asp Met Ala Gly
1430 1435 1440

Ala Leu His Pro Ser Ala Lys Val Asn Pro Asn Leu Gln Arg Arg
1445 1450 1455

His Glu Lys Met Ala Asn Leu Asn Asn Ile Ile Tyr Arg Val Glu
1460 1465 1470

Arg Ala Ala Asn Arg Glu Glu Ala Leu Glu Trp Glu Phe
1475 1480 1485

<210> 31
<211> 4628
<212> DNA
<213> Homo sapiens

<400> 31
gaacgtagct agctgcaagc agaggccggc atgaccaccg agcagcgacg cagcctgcaa 60

gccttccagg attatatccg gaagaccctg gaccctacct acatcctgag ctacatggcc 120

ccctggttta gggaggaaga ggtgcagtat attcaggctg agaaaaacaa caagggccca 180

atggaggctg ccacactttt tctcaagttc ctgttggagc tccaggagga aggctggttc 240

cgtggctttt tggatgccct agaccatgca ggttattctg gactttatga agccattgaa 300

agttgggatt tcaaaaaaat tgaaaagttg gaggagtata gattactttt aaaacgttta 360

caaccagaat ttaaaaccag aattatccca accgatatca tttctgatct gtctgaatgt 420

ttaattaatc aggaatgtga agaaattcta cagatttgct ctactaaggg gatgatggca 480

ggtgcagaga aattggtgga atgccttctc agatcagaca aggaaaactg gcccaaaact 540

ttgaaacttg ctttggagaa agaaaggaac aagttcagtg aactgtggat tgtagagaaa 600
ggtataaaag atgttgaaac agaagatctt gaggataaga tggaaacttc tgacatacag 660
attttctacc aagaagatcc agaatgccag aatcttagtg agaattcatg tccaccttca 720
gaagtgtctg atacaaactt gtacagccca tttaaaccaa gaaattacca attagagctt 780
gctttgcctg ctatgaaagg aaaaaacaca ataatatgtg ctcctacagg ttgtggaaaa 840
acctttgttt cactgcttat atgtgaacat catcttaaaa aattcccaca aggacaaaag 900
gggaaagttg tctttttgc gaatcagatc ccagtgtatg aacagcagaa atctgtattc 960
tcaaaatact ttgaaagaca tgggtataga gttacaggca tttctggagc aacagctgag 1020
aatgtcccag tggaacagat tgttgagaac aatgacatca tcattttaac tccacagatt 1080
cttgtgaaca accttaaaaa gggaacgatt ccatcactat ccatctttac tttgatgata 1140
tttgatgaat gccacaacac tagtaaacaa cacccgtaca atatgatcat gtttaattat 1200
ctagatcaga aacttggagg atcttcaggc ccactgcccc aggtcattgg gctgactgcc 1260
tcggttggtg ttggggatgc caaaaacaca gatgaagcct tggattatat ctgcaagctg 1320
tgtgcttctc ttgatgcgtc agtgatagca acagtcaaac acaatctgga ggaactggag 1380
caagttgttt ataagcccca gaagtttttc aggaaagtgg aatcacggat tagcgacaaa 1440
tttaaataca tcatagctca gctgatgagg gacacagaga gtctggcaaa gagaatctgc 1500
aaagacctcg aaaacttatc tcaaattcaa aatagggaat ttggaacaca gaaatatgaa 1560
caatggattg ttacagttca gaaagcatgc atggtgttcc agatgccaga caaagatgaa 1620
gagagcagga tttgtaaagc cctgttttta tacacttcac atttgcggaa atataatgat 1680
gccctcatta tcagtgagca tgcacgaatg aaagatgctc tggattactt gaaagacttc 1740
ttcagcaatg tccgagcagc aggattcgat gagattgagc aagatcttac tcagagattt 1800
gaagaaaagc tgcaggaact agaaagtgtt tccagggatc ccagcaatga gaatcctaaa 1860
cttgaagacc tctgcttcat cttacaagaa gagtaccact taaacccaga gacaataaca 1920
attctctttg tgaaaaccag agcacttgtg gacgctttaa aaaattggat tgaaggaaat 1980
cctaaactca gttttctaaa acctggcata ttgactggac gtggcaaaac aaatcagaac 2040
acaggaatga ccctcccggc acagaagtgt atattggatg cattcaaagc cagtggagat 2100
cacaatattc tgattgccac ctcagttgct gatgaaggca ttgacattgc acagtgcaat 2160
cttgtcatcc tttatgagta tgtgggcaat gtcatcaaaa tgatccaaac cagaggcaga 2220
ggaagagcaa gaggtagcaa gtgcttcctt ctgactagta atgctggtgt aattgaaaaa 2280
gaacaaataa acatgtacaa agaaaaaatg atgaatgact ctattttacg ccttcagaca 2340
tgggacgaag cagtatttag ggaaaagatt ctgcatatac agactcatga aaaattcatc 2400
agagatagtc aagaaaaacc aaaacctgta cctgataagg aaaataaaaa actgctctgc 2460

agaaagtgca aagccttggc atgttacaca gctgacgtaa gagtgataga ggaatgccat 2520

tacactgtgc ttggagatgc ttttaaggaa tgctttgtga gtagaccaca tcccaagcca 2580

aagcagtttt caagttttga aaaaagagca aagatattct gtgcccgaca gaactgcagc 2640

catgactggg gaatccatgt gaagtacaag acatttgaga ttccagttat aaaaattgaa 2700

agttttgtgg tggaggatat tgcaactgga gttcagacac tgtactcgaa gtggaaggac 2760

tttcattttg agaagatacc atttgatcca gcagaaatgt ccaaatgata tcaggtcctc 2820

aatcttcagc tacagggaat gagtaacttt gagtggagaa gaaacaaaca tagtgggtat 2880

aatcatggat cgcttgtacc cctgtgaaaa tatatttttt aaaaatatct ttagcagttt 2940

gtactatatt atatatgcaa agcacaaatg agtgaatcac agcactgagt attttgtagg 3000

ccaacagagc tcatagtact tgggaaaaat taaaaagcct catttctagc cttcttttta 3060

gagtcaactg ccaacaaaca cacagtaatc actctgtaca cactgggata gatgaatgaa 3120

tggaatgttg ggaattttta tctccctttg tctccttaac ctactgtaaa ctggcttttg 3180

cccttaacaa tctactgaaa ttgttctttt gaaggttacc agtgactctg gttgccaaat 3240

ccactgggca cttcttaacc ttctatttga cctctgcgca tttggccctg ttgagcactc 3300

ttcttgaagc tctccctggg cttctctctc ttctagttct attctagtct tttttattg 3360

agtcctcctc tttgctgatc ccttccaagg gttcaatata tatacatgta tatactgtac 3420

atatgtatat gtaactaata tacatacata caggtatgta tatgtaatgg ttatatgtac 3480

tcatgttcct ggtgtagcaa cgtgtggtat ggctacacag agaacatgag aacataaagc 3540

catttttatg cttactacta aaagctgtcc actgtagagt tgctgtatgt agcaatgtgt 3600

atccactcta cagtggtcag cttttagtag agagcataaa aatgataaaa tacttcttga 3660

aaacttagtt tactatacat cttgccctat taatatgttc tcttaacgtg tgccattgtt 3720

ctctttgacc attttcctat aatgatgttg atgttcaaca cctggactga atgtctgttc 3780

tcagatccct tggatgttac agatgaggca gtctgactgt cctttctact tgaaagatta 3840

gaatatgtat ccaaatggca ttcacgtgtc acttagcaag gtttgctgat gcttcaaaga 3900

gcttagtttg cggtttcctg gacgtggaaa caagtatctg agttccctgg agatcaacgg 3960

gatgaggtgt tacagctgcc tccctcttca tgcaatctgg tgagcagtgg tgcaggcggg 4020

gagccagaga aacttgccag ttatataact tctctttggc ttttcttcat ctgtaaaaca 4080

aggataatac tgaactgtaa gggttagtgg agagttttta attaaaagaa tgtgtgaaaa 4140

gtacatgaca cagtagttgc ttgataatag ttactagtag tagtattctt actaagaccc 4200

aatacaaatg gattatttaa accaagttta tgagttggtt ttttttcatt ttctatttgt 4260

attttattaa gagtgtcttt tcttatgtga tttttttaa ttgctatttg atatggtttg 4320

gctatatgtc cccacccaaa tctcatcttg aattataatc cccatgtgtc aagggaggga 4380

cctgacggga ggtgattgga tcacgggggc agttgtcccc atgctgttct tgggatagtg 4440

agttagttct catgagatct gatggtttta taagtgtttg acaattcctc ctttacacac 4500

actctctctc tcatctgctg ccatgtaaga cttgcctgct tccccttctg ccatgattgt 4560

aagtttcctg aggcctcctc agccatgtgg aactgtgaat ctattaagcc tcttttcttt 4620

ataaatga 4628

<210> 32
<211> 925
<212> PRT
<213> Homo sapiens

<400> 32

Met Thr Thr Glu Gln Arg Arg Ser Leu Gln Ala Phe Gln Asp Tyr Ile
1 5 10 15

Arg Lys Thr Leu Asp Pro Thr Tyr Ile Leu Ser Tyr Met Ala Pro Trp
20 25 30

Phe Arg Glu Glu Glu Val Gln Tyr Ile Gln Ala Glu Lys Asn Asn Lys
35 40 45

Gly Pro Met Glu Ala Ala Thr Leu Phe Leu Lys Phe Leu Leu Glu Leu
50 55 60

Gln Glu Glu Gly Trp Phe Arg Gly Phe Leu Asp Ala Leu Asp His Ala
65 70 75 80

Gly Tyr Ser Gly Leu Tyr Glu Ala Ile Glu Ser Trp Asp Phe Lys Lys
85 90 95

Ile Glu Lys Leu Glu Glu Tyr Arg Leu Leu Leu Lys Arg Leu Gln Pro
100 105 110

Glu Phe Lys Thr Arg Ile Ile Pro Thr Asp Ile Ile Ser Asp Leu Ser
115 120 125

Glu Cys Leu Ile Asn Gln Glu Cys Glu Glu Ile Leu Gln Ile Cys Ser
130 135 140

Thr Lys Gly Met Met Ala Gly Ala Glu Lys Leu Val Glu Cys Leu Leu
145 150 155 160

Arg Ser Asp Lys Glu Asn Trp Pro Lys Thr Leu Lys Leu Ala Leu Glu
165 170 175

Lys Glu Arg Asn Lys Phe Ser Glu Leu Trp Ile Val Glu Lys Gly Ile
180 185 190

Lys Asp Val Glu Thr Glu Asp Leu Glu Asp Lys Met Glu Thr Ser Asp
195 200 205

Ile Gln Ile Phe Tyr Gln Glu Asp Pro Glu Cys Gln Asn Leu Ser Glu
210 215 220

Asn Ser Cys Pro Pro Ser Glu Val Ser Asp Thr Asn Leu Tyr Ser Pro
225 230 235 240

Phe Lys Pro Arg Asn Tyr Gln Leu Glu Leu Ala Leu Pro Ala Met Lys
245 250 255

Gly Lys Asn Thr Ile Ile Cys Ala Pro Thr Gly Cys Gly Lys Thr Phe
260 265 270

Val Ser Leu Leu Ile Cys Glu His His Leu Lys Lys Phe Pro Gln Gly
275 280 285

Gln Lys Gly Lys Val Val Phe Phe Ala Asn Gln Ile Pro Val Tyr Glu
290 295 300

Gln Gln Lys Ser Val Phe Ser Lys Tyr Phe Glu Arg His Gly Tyr Arg
305 310 315 320

Val Thr Gly Ile Ser Gly Ala Thr Ala Glu Asn Val Pro Val Glu Gln
325 330 335

Ile Val Glu Asn Asn Asp Ile Ile Ile Leu Thr Pro Gln Ile Leu Val
340 345 350

Asn Asn Leu Lys Lys Gly Thr Ile Pro Ser Leu Ser Ile Phe Thr Leu
355 360 365

Met Ile Phe Asp Glu Cys His Asn Thr Ser Lys Gln His Pro Tyr Asn
370 375 380

Met Ile Met Phe Asn Tyr Leu Asp Gln Lys Leu Gly Gly Ser Ser Gly
385 390 395 400

Pro Leu Pro Gln Val Ile Gly Leu Thr Ala Ser Val Gly Val Gly Asp
405 410 415

Ala Lys Asn Thr Asp Glu Ala Leu Asp Tyr Ile Cys Lys Leu Cys Ala
420 425 430

Ser Leu Asp Ala Ser Val Ile Ala Thr Val Lys His Asn Leu Glu Glu
435 440 445

Leu Glu Gln Val Val Tyr Lys Pro Gln Lys Phe Phe Arg Lys Val Glu
450 455 460

Ser Arg Ile Ser Asp Lys Phe Lys Tyr Ile Ile Ala Gln Leu Met Arg
465 470 475 480

Asp Thr Glu Ser Leu Ala Lys Arg Ile Cys Lys Asp Leu Glu Asn Leu
485 490 495

Ser Gln Ile Gln Asn Arg Glu Phe Gly Thr Gln Lys Tyr Glu Gln Trp
500 505 510

Ile Val Thr Val Gln Lys Ala Cys Met Val Phe Gln Met Pro Asp Lys
515 520 525

Asp Glu Glu Ser Arg Ile Cys Lys Ala Leu Phe Leu Tyr Thr Ser His
530 535 540

Leu Arg Lys Tyr Asn Asp Ala Leu Ile Ile Ser Glu His Ala Arg Met
545 550 555 560

Lys Asp Ala Leu Asp Tyr Leu Lys Asp Phe Phe Ser Asn Val Arg Ala
565 570 575

Ala Gly Phe Asp Glu Ile Glu Gln Asp Leu Thr Gln Arg Phe Glu Glu
580 585 590

Lys Leu Gln Glu Leu Glu Ser Val Ser Arg Asp Pro Ser Asn Glu Asn
595 600 605

Pro Lys Leu Glu Asp Leu Cys Phe Ile Leu Gln Glu Glu Tyr His Leu
610 615 620

Asn Pro Glu Thr Ile Thr Ile Leu Phe Val Lys Thr Arg Ala Leu Val
625 630 635 640

Asp Ala Leu Lys Asn Trp Ile Glu Gly Asn Pro Lys Leu Ser Phe Leu
645 650 655

Lys Pro Gly Ile Leu Thr Gly Arg Gly Lys Thr Asn Gln Asn Thr Gly
660 665 670

Met Thr Leu Pro Ala Gln Lys Cys Ile Leu Asp Ala Phe Lys Ala Ser

675 680 685

Gly Asp His Asn Ile Leu Ile Ala Thr Ser Val Ala Asp Glu Gly Ile
690 695 700

Asp Ile Ala Gln Cys Asn Leu Val Ile Leu Tyr Glu Tyr Val Gly Asn
705 710 715 720

Val Ile Lys Met Ile Gln Thr Arg Gly Arg Gly Arg Ala Arg Gly Ser
725 730 735

Lys Cys Phe Leu Leu Thr Ser Asn Ala Gly Val Ile Glu Lys Glu Gln
740 745 750

Ile Asn Met Tyr Lys Glu Lys Met Met Asn Asp Ser Ile Leu Arg Leu
755 760 765

Gln Thr Trp Asp Glu Ala Val Phe Arg Glu Lys Ile Leu His Ile Gln
770 775 780

Thr His Glu Lys Phe Ile Arg Asp Ser Gln Glu Lys Pro Lys Pro Val
785 790 795 800

Pro Asp Lys Glu Asn Lys Lys Leu Leu Cys Arg Lys Cys Lys Ala Leu
805 810 815

Ala Cys Tyr Thr Ala Asp Val Arg Val Ile Glu Glu Cys His Tyr Thr
820 825 830

Val Leu Gly Asp Ala Phe Lys Glu Cys Phe Val Ser Arg Pro His Pro
835 840 845

Lys Pro Lys Gln Phe Ser Ser Phe Glu Lys Arg Ala Lys Ile Phe Cys
850 855 860

Ala Arg Gln Asn Cys Ser His Asp Trp Gly Ile His Val Lys Tyr Lys
865 870 875 880

Thr Phe Glu Ile Pro Val Ile Lys Ile Glu Ser Phe Val Val Glu Asp
885 890 895

Ile Ala Thr Gly Val Gln Thr Leu Tyr Ser Lys Trp Lys Asp Phe His
900 905 910

Phe Glu Lys Ile Pro Phe Asp Pro Ala Glu Met Ser Lys
915 920 925

<210> 33
<211> 4493
<212> DNA
<213> Homo sapiens

<400> 33
gccatttcgc cgattcctcc atgcgagttg ctgtgcgttt ctctgttgtc tcggtagaag 60

gccagagtca cacacggtcc taagagctgg gcaccaggaa gcgaaggctg atctgaagaa 120

gacacttgaa tcatgggtga cgttaaaaat tttctgtatg cctggtgtgg caaaaggaag 180

atgaccccat cctatgaaat tagagcagtg gggaacaaaa acaggcagaa attcatgtgt 240

gaggttcagg tggaaggtta taattacact ggcatgggaa attccaccaa taaaaaagat 300

gcacaaagca atgctgccag agactttgtt aactatttgg ttcgaataaa tgaaataaag 360

agtgaagaag ttccagcttt tggggtagca tctccgcccc cacttactga tactcctgac 420

actacagcaa atgctgaagg agatttacca acaaccatgg gaggacctct tcctccacat 480

ctggctctca aagcagaaaa taattctgag gtaggggcct ctggctatgg tgttcctggg 540

cccacctggg accgaggagc caacttgaag gattactact caagaaagga agaacaagaa 600

gtgcaagcga ctctagaatc agaagaagtg gatttaaatg ctgggcttca tggaaactgg 660

accttggaaa atgctaaagc tcgtctaaac caatattttc agaaagaaaa gatccaagga 720

gaatataagt acacccaagt gggtcctgat cacaacagga gctttattgc agaaatgacc 780

atttatatca agcagctggg cagaaggatt tttgcacgag aacatggatc aaataagaaa 840

ttggcagcac agtcctgtgc cctgtcactt gtcagacaac tgtaccatct tggagtggtt 900

gaagcttact ccggacttac aaagaagaag gaaggagaga cagtggagcc ttacaaagta 960

aacctctctc aagatttaga gcatcagctg caaaacatca ttcaagagct aaatcttgag 1020

attttgcccc cgcctgaaga tccttctgtg ccagttgcac tcaacattgg caaattggct 1080

cagttcgaac catctcagcg acaaaaccaa gtgggtgtgg ttccttggtc acctccacaa 1140

tccaactgga atccttggac tagtagcaac attgatgagg ggcctctggc ttttgctact 1200

ccagagcaaa taagcatgga cctcaagaat gaattgatgt accagttgga acaggatcat 1260

gatttgcaag caatcttgca ggagagagag ttactgcctg tgaagaaatt tgaaagtgag 1320

attctggaag caatcagcca aaattcagtt gtcattatta gaggggctac tggatgtggg 1380

aaaaccacac aggttcccca gttcattcta gatgacttta tccagaatga ccgagcagca 1440

gagtgtaaca tcgtagtaac tcagcccaga agaatcagtg cggtttctgt ggcagagcga 1500

gttgcatttg aaagaggaga agagcctgga aaaagctgtg gctacagcgt tcgatttgag 1560

tctatacttc ctcgtcctca tgccagtata atgttttgta ctgtaggtgt gctcctgaga 1620

aaattagaag caggcattcg aggaatcagt catgtaattg tagatgaaat acatgaaaga 1680

gatattaata ctgacttcct tttggtagta ctgcgtgatg ttgttcaggc ttatcctgaa 1740

gttcgcattg ttcttatgtc tgctactatt gataccagca tgttttgtga atatttcttc 1800

aattgcccca tcattgaagt ttatgggagg acttacccag ttcaagaata ttttctggaa 1860

gactgcattc agatgaccca ctttgttcct ccaccaaaag acaaaaagaa gaaggataag 1920

gatgatgatg gtggtgagga tgatgatgca aattgcaact tgatctgtgg tgatgaatat 1980

ggtccagaaa caaggttgag catgtctcaa ttgaacgaaa aggaaactcc ttttgaactc 2040

atcgaggctc tacttaagta cattgaaacc cttaatgttc ctggagctgt gttggttttt 2100

ttgcctggct ggaatctgat ttatactatg cagaagcatt tggaaatgaa tccacatttt 2160

ggaagccatc ggtatcagat tctacccctg cattctcaga ttcctcgaga ggaacagcgc 2220

aaagtgtttg atccagtacc agttggagta accaaggtta ttttgtccac aaatattgct 2280

gaaacaagca ttaccataaa cgatgttgtt tatgtcattg actcctgcaa gcagaaagtg 2340

aaactcttca ctgctcacaa caatatgacc aactatgcta ccgtatgggc atcaaaaaca 2400

aaccttgagc aacggaaagg gcgagctggc cgagtacggc ctggattctg ctttcacctg 2460

tgcagccgag ctcgttttga gagacttgaa acccacatga caccagagat gttccgaaca 2520

ccattgcatg aaattgctct tagcataaaa cttctgcgtc taggaggaat tggccaattt 2580

ctggccaaag caattgaacc tccccctttg gatgctgtga ttgaagcaga acacactctt 2640

agagagcttg atgcattaga tgccaatgat gagttgactc ctttgggacg aatcctggct 2700

aaactcccca ttgagcctcg ttttggcaaa atgatgataa tggggtgtat tttctacgtg 2760

ggagatgcta tctgtaccat tgctgctgct acctgctttc cagagccttt catcaatgaa 2820

ggaaagcggc tgggctatat ccatcgaaat tttgctggaa acagattttc tgatcacgta 2880

gccctttttat cagtattcca agcctgggat gatgctagaa tgggtggaga agaagcagag 2940

atacgttttt gtgagcacaa aagacttaat atggctacac taagaatgac ttgggaagcc 3000

aaagttcagc tcaaagagat tttgattaat tctgggtttc cagaagattg tttgttgaca 3060

caagtgttta ctaacactgg accagataat aatttggatg ttgttatctc cctcctggcc 3120

tttggtgtgt accccaatgt atgctatcat aaggaaaaga ggaagattct caccactgaa 3180

gggcgtaatg cacttatcca caaatcatct gttaattgtc cttttagtag ccaagacatg 3240

aagtacccat ctcccttctt tgtatttggt gaaaagattc gaactcgagc catctctgct 3300

aaaggcatga ctttagtcac ccccctgcag ttgcttctct ttgcctccaa gaaagtccaa 3360

tctgatgggc agattgtgct tgtagatgac tggattaaac tgcaaatatc tcatgaagct 3420

gctgcctgta tcactggtct ccgggcagcc atggaggctt tggttgttga agtaaccaaa 3480

caacctgcta tcatcagcca gttggacccc gtaaatgaac gtatgctgaa catgatccgt 3540

cagatctcta gaccctcagc tgctggtatc aaccttatga ttggcagtac acggtatgga 3600

gatggtccac gtcctcccaa gatggcccga tacgacaatg gaagcggata tagaagggga 3660

ggttctagtt acagtggtgg aggctatggc ggtggctata gcagtggagg ctatggtagc 3720

ggaggctatg gtggcagcgc caactccttt cgggcaggat atggtgcagg tgttggtgga 3780

ggctatagag gagtttcccg aggtggcttt agaggcaact ctggaggaga ctacagaggg 3840

cctagtggag gctacagagg atctgggga ttccagcgag gaggtggtag gggggcctat 3900

ggaactggct actttggaca gggaagagga ggtggcggct attaaaactt ggttatgtca 3960

gttcctgtgt gtagacagta aggaaaaaaa ggcatgctat gtgttacgtg ttttttccag 4020

tatgtttatt tgccaccaaa aagtaaatgc attttcaccc attctgtggt tcattgtagt 4080

ttaaggaaac caagcatata gatgcattag tgattttgtt tatattatgt aaaatataac 4140

gatctcttaa aaataccaca gtttgtattt tttctttaag gagtaaagat ttgcctttaa 4200

ataacttggt attttcctgg ctttcgttta atacaataga aaataaagta ttacaccgaa 4260

tacttgccgt gtagtttgtt tgttgacctc gtatgttaga aaattttaca atgccagcta 4320

catctgttga ttttaaatgt cagagaagtt gtaccctgtt tcaaaagtat actaagtgat 4380

actacttgta atagaataaa tcatcttgga attgaattgt taccttttga agtaaatact 4440

ggcaagtgca caagccacat aaacctgaat aaaacttttg acctagggtt gaa 4493

<210> 34
<211> 1270
<212> PRT
<213> Homo sapiens

<400> 34

Met Gly Asp Val Lys Asn Phe Leu Tyr Ala Trp Cys Gly Lys Arg Lys
1 5 10 15

Met Thr Pro Ser Tyr Glu Ile Arg Ala Val Gly Asn Lys Asn Arg Gln
20 25 30

Lys Phe Met Cys Glu Val Gln Val Glu Gly Tyr Asn Tyr Thr Gly Met
35 40 45

Gly Asn Ser Thr Asn Lys Lys Asp Ala Gln Ser Asn Ala Ala Arg Asp
50 55 60

Phe Val Asn Tyr Leu Val Arg Ile Asn Glu Ile Lys Ser Glu Glu Val
65 70 75 80

Pro Ala Phe Gly Val Ala Ser Pro Pro Pro Leu Thr Asp Thr Pro Asp
85 90 95

Thr Thr Ala Asn Ala Glu Gly Asp Leu Pro Thr Thr Met Gly Gly Pro

```
            100                 105                 110

Leu Pro Pro His Leu Ala Leu Lys Ala Glu Asn Asn Ser Glu Val Gly
        115                 120                 125

Ala Ser Gly Tyr Gly Val Pro Gly Pro Thr Trp Asp Arg Gly Ala Asn
    130                 135                 140

Leu Lys Asp Tyr Tyr Ser Arg Lys Glu Glu Gln Glu Val Gln Ala Thr
145                 150                 155                 160

Leu Glu Ser Glu Glu Val Asp Leu Asn Ala Gly Leu His Gly Asn Trp
                165                 170                 175

Thr Leu Glu Asn Ala Lys Ala Arg Leu Asn Gln Tyr Phe Gln Lys Glu
            180                 185                 190

Lys Ile Gln Gly Glu Tyr Lys Tyr Thr Gln Val Gly Pro Asp His Asn
        195                 200                 205

Arg Ser Phe Ile Ala Glu Met Thr Ile Tyr Ile Lys Gln Leu Gly Arg
    210                 215                 220

Arg Ile Phe Ala Arg Glu His Gly Ser Asn Lys Lys Leu Ala Ala Gln
225                 230                 235                 240

Ser Cys Ala Leu Ser Leu Val Arg Gln Leu Tyr His Leu Gly Val Val
                245                 250                 255

Glu Ala Tyr Ser Gly Leu Thr Lys Lys Lys Glu Gly Glu Thr Val Glu
            260                 265                 270

Pro Tyr Lys Val Asn Leu Ser Gln Asp Leu Glu His Gln Leu Gln Asn
        275                 280                 285

Ile Ile Gln Glu Leu Asn Leu Glu Ile Leu Pro Pro Pro Glu Asp Pro
    290                 295                 300

Ser Val Pro Val Ala Leu Asn Ile Gly Lys Leu Ala Gln Phe Glu Pro
305                 310                 315                 320

Ser Gln Arg Gln Asn Gln Val Gly Val Val Pro Trp Ser Pro Pro Gln
                325                 330                 335

Ser Asn Trp Asn Pro Trp Thr Ser Ser Asn Ile Asp Glu Gly Pro Leu
            340                 345                 350
```

```
Ala Phe Ala Thr Pro Glu Gln Ile Ser Met Asp Leu Lys Asn Glu Leu
        355                 360                 365

Met Tyr Gln Leu Glu Gln Asp His Asp Leu Gln Ala Ile Leu Gln Glu
    370                 375                 380

Arg Glu Leu Leu Pro Val Lys Lys Phe Glu Ser Glu Ile Leu Glu Ala
385                 390                 395                 400

Ile Ser Gln Asn Ser Val Val Ile Ile Arg Gly Ala Thr Gly Cys Gly
                405                 410                 415

Lys Thr Thr Gln Val Pro Gln Phe Ile Leu Asp Asp Phe Ile Gln Asn
            420                 425                 430

Asp Arg Ala Ala Glu Cys Asn Ile Val Val Thr Gln Pro Arg Arg Ile
        435                 440                 445

Ser Ala Val Ser Val Ala Glu Arg Val Ala Phe Glu Arg Gly Glu Glu
    450                 455                 460

Pro Gly Lys Ser Cys Gly Tyr Ser Val Arg Phe Glu Ser Ile Leu Pro
465                 470                 475                 480

Arg Pro His Ala Ser Ile Met Phe Cys Thr Val Gly Val Leu Leu Arg
                485                 490                 495

Lys Leu Glu Ala Gly Ile Arg Gly Ile Ser His Val Ile Val Asp Glu
            500                 505                 510

Ile His Glu Arg Asp Ile Asn Thr Asp Phe Leu Leu Val Val Leu Arg
        515                 520                 525

Asp Val Val Gln Ala Tyr Pro Glu Val Arg Ile Val Leu Met Ser Ala
    530                 535                 540

Thr Ile Asp Thr Ser Met Phe Cys Glu Tyr Phe Phe Asn Cys Pro Ile
545                 550                 555                 560

Ile Glu Val Tyr Gly Arg Thr Tyr Pro Val Gln Glu Tyr Phe Leu Glu
                565                 570                 575

Asp Cys Ile Gln Met Thr His Phe Val Pro Pro Pro Lys Asp Lys Lys
            580                 585                 590

Lys Lys Asp Lys Asp Asp Asp Gly Gly Glu Asp Asp Asp Ala Asn Cys
        595                 600                 605
```

Asn Leu Ile Cys Gly Asp Glu Tyr Gly Pro Glu Thr Arg Leu Ser Met
610 615 620

Ser Gln Leu Asn Glu Lys Glu Thr Pro Phe Glu Leu Ile Glu Ala Leu
625 630 635 640

Leu Lys Tyr Ile Glu Thr Leu Asn Val Pro Gly Ala Val Leu Val Phe
645 650 655

Leu Pro Gly Trp Asn Leu Ile Tyr Thr Met Gln Lys His Leu Glu Met
660 665 670

Asn Pro His Phe Gly Ser His Arg Tyr Gln Ile Leu Pro Leu His Ser
675 680 685

Gln Ile Pro Arg Glu Glu Gln Arg Lys Val Phe Asp Pro Val Pro Val
690 695 700

Gly Val Thr Lys Val Ile Leu Ser Thr Asn Ile Ala Glu Thr Ser Ile
705 710 715 720

Thr Ile Asn Asp Val Val Tyr Val Ile Asp Ser Cys Lys Gln Lys Val
725 730 735

Lys Leu Phe Thr Ala His Asn Asn Met Thr Asn Tyr Ala Thr Val Trp
740 745 750

Ala Ser Lys Thr Asn Leu Glu Gln Arg Lys Gly Arg Ala Gly Arg Val
755 760 765

Arg Pro Gly Phe Cys Phe His Leu Cys Ser Arg Ala Arg Phe Glu Arg
770 775 780

Leu Glu Thr His Met Thr Pro Glu Met Phe Arg Thr Pro Leu His Glu
785 790 795 800

Ile Ala Leu Ser Ile Lys Leu Leu Arg Leu Gly Gly Ile Gly Gln Phe
805 810 815

Leu Ala Lys Ala Ile Glu Pro Pro Pro Leu Asp Ala Val Ile Glu Ala
820 825 830

Glu His Thr Leu Arg Glu Leu Asp Ala Leu Asp Ala Asn Asp Glu Leu
835 840 845

Thr Pro Leu Gly Arg Ile Leu Ala Lys Leu Pro Ile Glu Pro Arg Phe
850 855 860

Gly Lys Met Met Ile Met Gly Cys Ile Phe Tyr Val Gly Asp Ala Ile
865 870 875 880

Cys Thr Ile Ala Ala Ala Thr Cys Phe Pro Glu Pro Phe Ile Asn Glu
885 890 895

Gly Lys Arg Leu Gly Tyr Ile His Arg Asn Phe Ala Gly Asn Arg Phe
900 905 910

Ser Asp His Val Ala Leu Leu Ser Val Phe Gln Ala Trp Asp Asp Ala
915 920 925

Arg Met Gly Gly Glu Glu Ala Glu Ile Arg Phe Cys Glu His Lys Arg
930 935 940

Leu Asn Met Ala Thr Leu Arg Met Thr Trp Glu Ala Lys Val Gln Leu
945 950 955 960

Lys Glu Ile Leu Ile Asn Ser Gly Phe Pro Glu Asp Cys Leu Leu Thr
965 970 975

Gln Val Phe Thr Asn Thr Gly Pro Asp Asn Asn Leu Asp Val Val Ile
980 985 990

Ser Leu Leu Ala Phe Gly Val Tyr Pro Asn Val Cys Tyr His Lys Glu
995 1000 1005

Lys Arg Lys Ile Leu Thr Thr Glu Gly Arg Asn Ala Leu Ile His
1010 1015 1020

Lys Ser Ser Val Asn Cys Pro Phe Ser Ser Gln Asp Met Lys Tyr
1025 1030 1035

Pro Ser Pro Phe Phe Val Phe Gly Glu Lys Ile Arg Thr Arg Ala
1040 1045 1050

Ile Ser Ala Lys Gly Met Thr Leu Val Thr Pro Leu Gln Leu Leu
1055 1060 1065

Leu Phe Ala Ser Lys Lys Val Gln Ser Asp Gly Gln Ile Val Leu
1070 1075 1080

Val Asp Asp Trp Ile Lys Leu Gln Ile Ser His Glu Ala Ala Ala
1085 1090 1095

Cys Ile Thr Gly Leu Arg Ala Ala Met Glu Ala Leu Val Val Glu

1100 1105 1110

Val Thr Lys Gln Pro Ala Ile Ile Ser Gln Leu Asp Pro Val Asn
1115 1120 1125

Glu Arg Met Leu Asn Met Ile Arg Gln Ile Ser Arg Pro Ser Ala
1130 1135 1140

Ala Gly Ile Asn Leu Met Ile Gly Ser Thr Arg Tyr Gly Asp Gly
1145 1150 1155

Pro Arg Pro Pro Lys Met Ala Arg Tyr Asp Asn Gly Ser Gly Tyr
1160 1165 1170

Arg Arg Gly Gly Ser Ser Tyr Ser Gly Gly Gly Tyr Gly Gly Gly
1175 1180 1185

Tyr Ser Ser Gly Gly Tyr Gly Ser Gly Gly Tyr Gly Gly Ser Ala
1190 1195 1200

Asn Ser Phe Arg Ala Gly Tyr Gly Ala Gly Val Gly Gly Gly Tyr
1205 1210 1215

Arg Gly Val Ser Arg Gly Gly Phe Arg Gly Asn Ser Gly Gly Asp
1220 1225 1230

Tyr Arg Gly Pro Ser Gly Gly Tyr Arg Gly Ser Gly Gly Phe Gln
1235 1240 1245

Arg Gly Gly Gly Arg Gly Ala Tyr Gly Thr Gly Tyr Phe Gly Gln
1250 1255 1260

Gly Arg Gly Gly Gly Gly Tyr
1265 1270

<210> 35
<211> 3069
<212> DNA
<213> Homo sapiens

<400> 35
gagtgtcggg cgcggcagga ggacgaggca gggcgggcgg gcgctctaag ggttctgctc 60

tgactccagg ttgggacagc gtcttcgctg ctgctggata gtcgtgtttt cggggatcga 120

ggatactcac cagaaaccga aaatgccgaa accaatcaat gtccgagtta ccaccatgga 180

tgcagagctg gagtttgcaa tccagccaaa tacaactgga aaacagcttt ttgatcaggt 240

ggtaaagact atcggcctcc gggaagtgtg gtactttggc ctccactatg tggataataa 300

aggatttcct acctggctga agctggataa gaaggtgtct gcccaggagg tcaggaagga 360
gaatcccctc cagttcaagt tccgggccaa gttctaccct gaagatgtgg ctgaggagct 420
catccaggac atcacccaga aacttttctt cctccaagtg aaggaaggaa tccttagcga 480
tgagatctac tgccccctg agactgccgt gctcttgggg tcctacgctg tgcaggccaa 540
gtttggggac tacaacaaag aagtgcacaa gtctgggtac ctcagctctg agcggctgat 600
ccctcaaaga gtgatggacc agcacaaact taccagggac cagtgggagg accggatcca 660
ggtgtggcat gcggaacacc gtgggatgct caaagataat gctatgttgg aatacctgaa 720
gattgctcag gacctggaaa tgtatggaat caactatttc gagataaaaa acaagaaagg 780
aacagacctt tggcttggag ttgatgccct tggactgaat atttatgaga aagatgataa 840
gttaacccca aagattggct ttccttggag tgaaatcagg aacatctctt tcaatgacaa 900
aaagtttgtc attaaaccca tcgacaagaa ggcacctgac tttgtgtttt atgccccacg 960
tctgagaatc aacaagcgga tcctgcagct ctgcatgggc aaccatgagt tgtatatgcg 1020
ccgcaggaag cctgacacca tcgaggtgca gcagatgaag gcccaggccc gggaggagaa 1080
gcatcagaag cagctggagc ggcaacagct ggaaacagag aagaaaagga gagaaaccgt 1140
ggagagagag aaagagcaga tgatgcgcga gaaggaggag ttgatgctgc ggctgcagga 1200
ctatgaggag aagacaaaga aggcagagag agagctctcg gagcagattc agagggccct 1260
gcagctggag gaggagagga agcgggcaca ggaggaggcc gagcgcctag aggctgaccg 1320
tatggctgca ctgcgggcta aggaggagct ggagagacag gcggtggatc agataaagag 1380
ccaggagcag ctggctgcgg agcttgcaga atacactgcc aagattgccc tcctggaaga 1440
ggcgcggagg cgcaaggagg atgaagttga agagtggcag cacagggcca aagaagccca 1500
ggatgacctg gtgaagacca aggaggagct gcacctggtg atgacagcac ccccgccccc 1560
accacccccc gtgtacgagc cggtgagcta ccatgtccag gagagcttgc aggatgaggg 1620
cgcagagccc acgggctaca gcgcggagct gtctagtgag ggcatccggg atgaccgcaa 1680
tgaggagaag cgcatcactg aggcagagaa gaacgagcgt gtgcagcggc agctgctgac 1740
gctgagcagc gagctgtccc aggcccgaga tgagaataag aggacccaca atgacatcat 1800
ccacaacgag aacatgaggc aaggccggga caagtacaag acgctgcggc agatccggca 1860
gggcaacacc aagcagcgca tcgacgagtt cgaggccctg taacagccag gccaggacca 1920
agggcagagg ggtgctcata gcgggcgctg ccagccccgc cacgcttgtg tctttagtgc 1980
tccaagtcta ggaactccct cagatcccag ttcctttaga aagcagttac ccaacagaaa 2040
cattctgggc tgggaaccag ggaggcgccc tggtttgttt tccccagttg taatagtgcc 2100
aagcaggcct gattctcgcg attattctcg aatcacctcc tgtgttgtgc tgggagcagg 2160
actgattgaa ttacggaaaa tgcctgtaaa gtctgagtaa gaaacttcat gctggcctgt 2220

gtgatacaag agtcagcatc attaaaggaa acgtggcagg acttccatct gtgccatact 2280

tgttctgtat tcgaaatgag ctcaaattga ttttttaatt tctatgaagg atccatcttt 2340

gtatatttac atgcttagag gggtgaaaat tattttggaa attgagtctg aagcactctc 2400

gcacacacag tgattccctc ctcccgtcac tccacgcagc tggcagagag cacagtgatc 2460

accagcgtga gtggtggagg aggacacttg gatttttttt tttgtttttt ttttttttgc 2520

ttaacagttt tagaatacat tgtacttata caccttatta atgatcagct atatactatt 2580

tatatacaag tgataataca gatttgtaac attagtttta aaaagggaaa gttttgttct 2640

gtatattttg ttacctttta cagaataaaa gaattacata tgaaaaaccc tctaaaccat 2700

ggcacttgat gtgatgtggc aggagggcag tggtggagct ggacctgcct gctgcagtca 2760

cgtgtaaaca ggattattat tagtgtttta tgcatgtaat ggactatgca cacttttaat 2820

tttgtcagat tcacacatgc cactatgagc tttcagactc cagctgtgaa gagactctgt 2880

ttgcttgtgt ttgtttgttt gcagtctctc tctgccatgg ccttggcagg ctgctggaag 2940

gcagcttgtg gaggccgttg gttccgccca ctcattcctt ctcgtgcact gctttctcct 3000

tcacagctaa gatgccatgt gcaggtggat tccatgccgc agacatgaaa taaaagcttt 3060

gcaaaggca 3069

<210> 36
<211> 586
<212> PRT
<213> Homo sapiens

<400> 36

Met Pro Lys Pro Ile Asn Val Arg Val Thr Thr Met Asp Ala Glu Leu
1 5 10 15

Glu Phe Ala Ile Gln Pro Asn Thr Thr Gly Lys Gln Leu Phe Asp Gln
20 25 30

Val Val Lys Thr Ile Gly Leu Arg Glu Val Trp Tyr Phe Gly Leu His
35 40 45

Tyr Val Asp Asn Lys Gly Phe Pro Thr Trp Leu Lys Leu Asp Lys Lys
50 55 60

Val Ser Ala Gln Glu Val Arg Lys Glu Asn Pro Leu Gln Phe Lys Phe
65 70 75 80

Arg Ala Lys Phe Tyr Pro Glu Asp Val Ala Glu Glu Leu Ile Gln Asp
85 90 95

Ile Thr Gln Lys Leu Phe Phe Leu Gln Val Lys Glu Gly Ile Leu Ser
100 105 110

Asp Glu Ile Tyr Cys Pro Pro Glu Thr Ala Val Leu Leu Gly Ser Tyr
115 120 125

Ala Val Gln Ala Lys Phe Gly Asp Tyr Asn Lys Glu Val His Lys Ser
130 135 140

Gly Tyr Leu Ser Ser Glu Arg Leu Ile Pro Gln Arg Val Met Asp Gln
145 150 155 160

His Lys Leu Thr Arg Asp Gln Trp Glu Asp Arg Ile Gln Val Trp His
165 170 175

Ala Glu His Arg Gly Met Leu Lys Asp Asn Ala Met Leu Glu Tyr Leu
180 185 190

Lys Ile Ala Gln Asp Leu Glu Met Tyr Gly Ile Asn Tyr Phe Glu Ile
195 200 205

Lys Asn Lys Lys Gly Thr Asp Leu Trp Leu Gly Val Asp Ala Leu Gly
210 215 220

Leu Asn Ile Tyr Glu Lys Asp Asp Lys Leu Thr Pro Lys Ile Gly Phe
225 230 235 240

Pro Trp Ser Glu Ile Arg Asn Ile Ser Phe Asn Asp Lys Lys Phe Val
245 250 255

Ile Lys Pro Ile Asp Lys Lys Ala Pro Asp Phe Val Phe Tyr Ala Pro
260 265 270

Arg Leu Arg Ile Asn Lys Arg Ile Leu Gln Leu Cys Met Gly Asn His
275 280 285

Glu Leu Tyr Met Arg Arg Arg Lys Pro Asp Thr Ile Glu Val Gln Gln
290 295 300

Met Lys Ala Gln Ala Arg Glu Glu Lys His Gln Lys Gln Leu Glu Arg
305 310 315 320

Gln Gln Leu Glu Thr Glu Lys Lys Arg Arg Glu Thr Val Glu Arg Glu
325 330 335

Lys Glu Gln Met Met Arg Glu Lys Glu Glu Leu Met Leu Arg Leu Gln
340 345 350

```
Asp Tyr Glu Glu Lys Thr Lys Lys Ala Glu Arg Glu Leu Ser Glu Gln
        355                 360                 365

Ile Gln Arg Ala Leu Gln Leu Glu Glu Glu Arg Lys Arg Ala Gln Glu
    370                 375                 380

Glu Ala Glu Arg Leu Glu Ala Asp Arg Met Ala Ala Leu Arg Ala Lys
385                 390                 395                 400

Glu Glu Leu Glu Arg Gln Ala Val Asp Gln Ile Lys Ser Gln Glu Gln
                405                 410                 415

Leu Ala Ala Glu Leu Ala Glu Tyr Thr Ala Lys Ile Ala Leu Leu Glu
            420                 425                 430

Glu Ala Arg Arg Arg Lys Glu Asp Glu Val Glu Glu Trp Gln His Arg
        435                 440                 445

Ala Lys Glu Ala Gln Asp Asp Leu Val Lys Thr Lys Glu Glu Leu His
    450                 455                 460

Leu Val Met Thr Ala Pro Pro Pro Pro Pro Pro Pro Val Tyr Glu Pro
465                 470                 475                 480

Val Ser Tyr His Val Gln Glu Ser Leu Gln Asp Glu Gly Ala Glu Pro
                485                 490                 495

Thr Gly Tyr Ser Ala Glu Leu Ser Ser Glu Gly Ile Arg Asp Asp Arg
            500                 505                 510

Asn Glu Glu Lys Arg Ile Thr Glu Ala Glu Lys Asn Glu Arg Val Gln
        515                 520                 525

Arg Gln Leu Leu Thr Leu Ser Ser Glu Leu Ser Gln Ala Arg Asp Glu
    530                 535                 540

Asn Lys Arg Thr His Asn Asp Ile Ile His Asn Glu Asn Met Arg Gln
545                 550                 555                 560

Gly Arg Asp Lys Tyr Lys Thr Leu Arg Gln Ile Arg Gln Gly Asn Thr
                565                 570                 575

Lys Gln Arg Ile Asp Glu Phe Glu Ala Leu
            580                 585
```

<210>  37
<211>  3628

<212> DNA
<213> Homo sapiens

<400> 37
agagcatcag caagagtagc agcgagcagc cgcgctggtg gcggcggcgc gtcgttgcag 60

ttgcgccatc tgtcaggagc ggagccggcg aggagggggc tgccgcgggc gaggaggagg 120

ggtcgccgcg agccgaaggc cttcgagacc cgcccgccgc ccggcggcga gagtagaggc 180

gaggttgttg tgcgagcggc gcgtcctctc ccgcccgggc gcgccgcgct tctcccagcg 240

caccgaggac cgcccgggcg cacacaaagc cgccgcccgc gccgcaccgc ccggcggccg 300

ccgcccgcgc cagggaggga ttcggccgcc gggccgggga caccccggcg ccgccccctc 360

ggtgctctcg gaaggcccac cggctcccgg gcccgccggg gacccccegg agccgcctcg 420

gccgcgccgg aggagggcgg ggagaggacc atgtgagtgg gctccggagc ctcagcgccg 480

cgcagttttt ttgaagaagc aggatgctga tctaaacgtg gaaaaagacc agtcctgcct 540

ctgttgtaga agacatgtgg tgtatataaa gtttgtgatc gttggcggac attttggaat 600

ttagataatg ggctgtgtgc aatgtaagga taaagaagca acaaaactga cggaggagag 660

ggacggcagc ctgaaccaga gctctgggta ccgctatggc acagacccca cccctcagca 720

ctaccccagc ttcggtgtga cctccatccc caactacaac aacttccacg cagccggggg 780

ccaaggactc accgtctttg gaggtgtgaa ctcttcgtct catacgggga ccttgcgtac 840

gagaggagga acaggagtga cactctttgt ggccctttat gactatgaag cacggacaga 900

agatgacctg agttttcaca aaggagaaaa atttcaaata ttgaacagct cggaaggaga 960

ttggtgggaa gcccgctcct tgacaactgg agagacaggt tacattccca gcaattatgt 1020

ggctccagtt gactctatcc aggcagaaga gtggtacttt ggaaaacttg gccgaaaaga 1080

tgctgagcga cagctattgt cctttggaaa cccaagaggt acctttctta tccgcgagag 1140

tgaaaccacc aaaggtgcct attcactttc tatccgtgat tgggatgata tgaaaggaga 1200

ccatgtcaaa cattataaaa ttcgcaaact tgacaatggt ggatactaca ttaccacccg 1260

ggcccagttt gaaacacttc agcagcttgt acaacattac tcagagagag ctgcaggtct 1320

ctgctgccgc ctagtagttc cctgtcacaa agggatgcca aggcttaccg atctgtctgt 1380

caaaaccaaa gatgtctggg aaatccctcg agaatccctg cagttgatca agagactggg 1440

aaatgggcag tttggggaag tatggatggg tacctggaat ggaaacacaa aagtagccat 1500

aaagactctt aaaccaggca caatgtcccc cgaatcattc cttgaggaag cgcagatcat 1560

gaagaagctg aagcacgaca agctggtcca gctctatgca gtggtgtctg aggagcccat 1620

ctacatcgtc accgagtata tgaacaaagg aagtttactg gatttcttaa aagatggaga 1680

aggaagagct ctgaaattac caaatcttgt ggacatggca gcacaggtgg ctgcaggaat 1740

ggcttacatc gagcgcatga attatatcca tagagatctg cgatcagcaa acattctagt 1800

ggggaatgga ctcatatgca agattgctga cttcggattg gcccgattga tagaagacaa 1860
tgagtacaca gcaagacaag gtgcaaagtt ccccatcaag tggacggccc ccgaggcagc 1920
cctgtacggg aggttcacaa tcaagtctga cgtgtggtct tttggaatct tactcacaga 1980
gctggtcacc aaaggaagag tgccataccc aggcatgaac aaccgggagg tgctggagca 2040
ggtggagcga ggctacagga tgccctgccc gcaggactgc cccatctctc tgcatgagct 2100
catgatccac tgctggaaaa aggaccctga agaacgcccc acttttgagt acttgcagag 2160
cttcctggaa gactacttta ccgcgacaga gccccagtac caacctggtg aaaacctgta 2220
aggcccgggt ctgcggagag aggccttgtc ccagaggctg ccccacccct ccccattagc 2280
tttcaattcc gtagccagct gctccccagc agcggaaccg cccaggatca gattgcatgt 2340
gactctgaag ctgacgaact tccatggccc tcattaatga cacttgtccc caaatccgaa 2400
cctcctctgt gaagcattcg agacagaacc ttgttatttc tcagactttg gaaaatgcat 2460
tgtatcgatg ttatgtaaaa ggccaaacct ctgttcagtg taaatagtta ctccagtgcc 2520
aacaatccta gtgctttcct tttttaaaaa tgcaaatcct atgtgatttt aactctgtct 2580
tcacctgatt caactaaaaa aaaaaaagta ttattttcca aaagtggcct ctttgtctaa 2640
aacaataaaa tttttttca tgttttaaca aaaaccaatc aggacaggtg tttgtttttg 2700
ttttcttttt tataaatatg aatatatata atatatatgt ccctgtacat atacaatgtg 2760
ggtgctaatg tggagactgt ggccggcctg agccaccaag ctgcgggacc cagagggagg 2820
attttactgc aagtcagcat caaagcaccg gtgttattct gaaaacacca gtggcctcat 2880
ttttggcttt tgcaaagcat gaatttttc atttggattg cactttcctg gttcatgact 2940
gtacctgtag gtggttgtta ctttgactct tttcaggaac cacccccaa gctgaattta 3000
caagttctgt tagcactatt tgcttcaact tactgcgatt tgttctcaaa acttaaaaat 3060
aagcaagcaa atggctgata ctaccaagag aactggaaga tggataccac acaaacttct 3120
tgtataaaaa tatgaatgct gaaatgtttc agacattttt aatttaataa acctgtaacc 3180
acatttaagt gatctaaaac ccatagcatt gtagtcatgg caacccgcta aactttctca 3240
tgcaactaaa atttctgggg gaaatgaggg tgggggttgt acatttccca ttgtaaaata 3300
agtgttttaa atgtcctgta ctgctaacga atgactttct atatgtccag gagttctcca 3360
gtggaataac tatgcactac tttacatttc atggggatgc acaaaaacaa aaaagtatta 3420
catttttagt tgctgtttgt accaacctta aattacatat gtttaacaac aacaaatcaa 3480
aaatcctatt tctattgagt ttttaatact gactagcaac tctgaagtct taattccttt 3540
tttgttatga tttatttgtg agtttacatt tttaaattgt ttaactttct taatttagta 3600
attaaaaaga gagcatttta catttgaa 3628

<210> 38
<211> 3238
<212> DNA
<213> Homo sapiens

<400> 38
ggctcccggg cccgccgggg accccccgga gccgcctcgg ccgcgccgga ggagggcggg 60

gagaggacca tgtgagtggg ctccggagcc tcagcgccgc gcagtttttt tgaagaagca 120

ggatgctgat ctaaacgtgg aaaaagacca gtcctgcctc tgttgtagaa gacatgtggt 180

gtatataaag tttgtgatcg ttggcggaca ttttggaatt tagataatgg gctgtgtgca 240

atgtaaggat aaagaagcaa caaaactgac ggaggagagg gacggcagcc tgaaccagag 300

ctctgggtac cgctatggca cagaccccac ccctcagcac taccccagct tcggtgtgac 360

ctccatcccc aactacaaca acttccacgc agccgggggc caaggactca ccgtctttgg 420

aggtgtgaac tcttcgtctc atacggggac cttgcgtacg agaggaggaa caggagtgac 480

actctttgtg gccctttatg actatgaagc acggacagaa gatgacctga gttttcacaa 540

aggagaaaaa tttcaaatat tgaacagctc ggaaggagat tggtgggaag cccgctcctt 600

gacaactgga gagacaggtt acattcccag caattatgtg gctccagttg actctatcca 660

ggcagaagag tggtactttg gaaaacttgg ccgaaaagat gctgagcgac agctattgtc 720

ctttggaaac ccaagaggta cctttcttat ccgcgagagt gaaaccacca aaggtgccta 780

ttcactttct atccgtgatt gggatgatat gaaaggagac catgtcaaac attataaaat 840

tcgcaaactt gacaatggtg gatactacat taccacccgg gcccagtttg aaacacttca 900

gcagcttgta caacattact cagagaaagc tgatggtttg tgttttaact taactgtgat 960

tgcatcgagt tgtaccccac aaacttctgg attggctaaa gatgcttggg aagttgcacg 1020

tcgttcgttg tgtctggaga agaagctggg tcaggggtgt ttcgctgaag tgtggcttgg 1080

tacctggaat ggaaacacaa aagtagccat aaagactctt aaaccaggca caatgtcccc 1140

cgaatcattc cttgaggaag cgcagatcat gaagaagctg aagcacgaca agctggtcca 1200

gctctatgca gtggtgtctg aggagcccat ctacatcgtc accgagtata tgaacaaagg 1260

aagtttactg gatttcttaa aagatggaga aggaagagct ctgaaattac caaatcttgt 1320

ggacatggca gcacaggtgg ctgcaggaat ggcttacatc gagcgcatga attatatcca 1380

tagagatctg cgatcagcaa acattctagt ggggaatgga ctcatatgca agattgctga 1440

cttcggattg gcccgattga tagaagacaa tgagtacaca gcaagacaag gtgcaaagtt 1500

ccccatcaag tggacggccc ccgaggcagc cctgtacggg aggttcacaa tcaagtctga 1560

cgtgtggtct tttggaatct tactcacaga gctggtcacc aaaggaagag tgccataccc 1620

aggcatgaac aaccgggagg tgctggagca ggtggagcga ggctacagga tgccctgccc 1680

gcaggactgc cccatctctc tgcatgagct catgatccac tgctggaaaa aggaccctga 1740

agaacgcccc acttttgagt acttgcagag cttcctggaa gactacttta ccgcgacaga 1800

gccccagtac caacctggtg aaaacctgta aggcccgggt ctgcggagag aggccttgtc 1860

ccagaggctg ccccacccct ccccattagc tttcaattcc gtagccagct gctccccagc 1920

agcggaaccg cccaggatca gattgcatgt gactctgaag ctgacgaact tccatggccc 1980

tcattaatga cacttgtccc caaatccgaa cctcctctgt gaagcattcg agacagaacc 2040

ttgttatttc tcagactttg gaaaatgcat tgtatcgatg ttatgtaaaa ggccaaacct 2100

ctgttcagtg taaatagtta ctccagtgcc aacaatccta gtgctttcct tttttaaaaa 2160

tgcaaatcct atgtgatttt aactctgtct tcacctgatt caactaaaaa aaaaaaagta 2220

ttattttcca aaagtggcct ctttgtctaa aacaataaaa tttttttca tgttttaaca 2280

aaaaccaatc aggacaggtg tttgttttg ttttctttt tataaatatg aatatatata 2340

atatatatgt ccctgtacat atacaatgtg ggtgctaatg tggagactgt ggccggcctg 2400

agccaccaag ctgcgggacc cagagggagg attttactgc aagtcagcat caaagcaccg 2460

gtgttattct gaaaacacca gtggcctcat ttttggcttt tgcaaagcat gaatttttc 2520

atttggattg cactttcctg gttcatgact gtacctgtag gtggttgtta ctttgactct 2580

tttcaggaac caccccccaa gctgaattta caagttctgt tagcactatt tgcttcaact 2640

tactgcgatt tgttctcaaa acttaaaaat aagcaagcaa atggctgata ctaccaagag 2700

aactggaaga tggataccac acaaacttct tgtataaaaa tatgaatgct gaaatgtttc 2760

agacattttt aatttaataa acctgtaacc acatttaagt gatctaaaac ccatagcatt 2820

gtagtcatgg caacccgcta aactttctca tgcaactaaa atttctgggg gaaatgaggg 2880

tgggggttgt acatttccca ttgtaaaata agtgttttaa atgtcctgta ctgctaacga 2940

atgactttct atatgtccag gagttctcca gtggaataac tatgcactac tttacatttc 3000

atggggatgc acaaaaacaa aaaagtatta catttttagt tgctgtttgt accaacctta 3060

aattacatat gtttaacaac aacaaatcaa aaatcctatt tctattgagt ttttaatact 3120

gactagcaac tctgaagtct taattccttt tttgttatga tttatttgtg agtttacatt 3180

tttaaattgt ttaactttct taatttagta attaaaaaga gagcatttta catttgaa 3238

<210> 39
<211> 2959
<212> DNA
<213> Homo sapiens

<400> 39
aaatgatcaa gtgttgggta taagccaagg agctgagaga gggggagacc agcgcaggtc 60

tgaggagctg agaagggagg cttacgtgaa gggaatttag ataatgggct gtgtgcaatg 120

taaggataaa gaagcaacaa aactgacgga ggagagggac ggcagcctga accagagctc 180

tgggtaccgc tatggcacag accccacccc tcagcactac cccagcttcg gtgtgacctc 240

catccccaac tacaacaact tccacgcagc cggggggccaa ggactcaccg tctttggagg 300

tgtgaactct tcgtctcata cggggacctt gcgtacgaga ggaggaacag gagtgacact 360

ctttgtggcc ctttatgact atgaagcacg gacagaagat gacctgagtt ttcacaaagg 420

agaaaaattt caaatattga acagctcgga aggagattgg tgggaagccc gctccttgac 480

aactggagag acaggttaca ttcccagcaa ttatgtggct ccagttgact ctatccaggc 540

agaagagtgg tactttggaa aacttggccg aaaagatgct gagcgacagc tattgtcctt 600

tggaaaccca agaggtacct ttcttatccg cgagagtgaa accaccaaag gtgcctattc 660

actttctatc cgtgattggg atgatatgaa aggagaccat gtcaaacatt ataaaattcg 720

caaacttgac aatggtggat actacattac cacccgggcc cagtttgaaa cacttcagca 780

gcttgtacaa cattactcag gtacctggaa tggaaacaca aaagtagcca taaagactct 840

taaaccaggc acaatgtccc ccgaatcatt ccttgaggaa gcgcagatca tgaagaagct 900

gaagcacgac aagctggtcc agctctatgc agtggtgtct gaggagccca tctacatcgt 960

caccgagtat atgaacaaag gaagtttact ggatttctta aaagatggag aaggaagagc 1020

tctgaaatta ccaaatcttg tggacatggc agcacaggtg gctgcaggaa tggcttacat 1080

cgagcgcatg aattatatcc atagagatct gcgatcagca aacattctag tggggaatgg 1140

actcatatgc aagattgctg acttcggatt ggcccgattg atagaagaca atgagtacac 1200

agcaagacaa ggtgcaaagt tccccatcaa gtggacggcc cccgaggcag ccctgtacgg 1260

gaggttcaca atcaagtctg acgtgtggtc ttttggaatc ttactcacag agctggtcac 1320

caaaggaaga gtgccatacc caggcatgaa caaccgggag gtgctggagc aggtggagcg 1380

aggctacagg atgccctgcc cgcaggactg ccccatctct ctgcatgagc tcatgatcca 1440

ctgctggaaa aaggaccctg aagaacgccc cacttttgag tacttgcaga gcttcctgga 1500

agactacttt accgcgacag agccccagta ccaacctggt gaaaacctgt aaggcccggg 1560

tctgcggaga gaggccttgt cccagaggct gccccacccc tccccattag ctttcaattc 1620

cgtagccagc tgctccccag cagcggaacc gcccaggatc agattgcatg tgactctgaa 1680

gctgacgaac ttccatggcc ctcattaatg acacttgtcc ccaaatccga acctcctctg 1740

tgaagcattc gagacagaac cttgttattt ctcagacttt ggaaaatgca ttgtatcgat 1800

gttatgtaaa aggccaaacc tctgttcagt gtaaatagtt actccagtgc caacaatcct 1860

agtgctttcc ttttttaaaa atgcaaatcc tatgtgattt taactctgtc ttcacctgat 1920

tcaactaaaa aaaaaaaagt attattttcc aaaagtggcc tctttgtcta aaacaataaa 1980

atttttttc atgttttaac aaaaaccaat caggacaggt gtttgttttt gttttctttt 2040

ttataaatat gaatatatat aatatatatg tccctgtaca tatacaatgt gggtgctaat 2100

gtggagactg tggccggcct gagccaccaa gctgcgggac ccagagggag gattttactg 2160

caagtcagca tcaaagcacc ggtgttattc tgaaaacacc agtggcctca tttttggctt 2220

ttgcaaagca tgaatttttt catttggatt gcactttcct ggttcatgac tgtacctgta 2280

ggtggttgtt actttgactc ttttcaggaa ccacccccca agctgaattt acaagttctg 2340

ttagcactat ttgcttcaac ttactgcgat ttgttctcaa aacttaaaaa taagcaagca 2400

aatggctgat actaccaaga gaactggaag atggatacca cacaaacttc ttgtataaaa 2460

atatgaatgc tgaaatgttt cagacatttt taatttaata aacctgtaac cacatttaag 2520

tgatctaaaa cccatagcat tgtagtcatg gcaacccgct aaactttctc atgcaactaa 2580

aatttctggg ggaaatgagg gtgggggttg tacatttccc attgtaaaat aagtgtttta 2640

aatgtcctgt actgctaacg aatgactttc tatatgtcca ggagttctcc agtggaataa 2700

ctatgcacta ctttacattt catggggatg cacaaaaaca aaaaagtatt acatttttag 2760

ttgctgtttg taccaacctt aaattacata tgtttaacaa caacaaatca aaaatcctat 2820

ttctattgag tttttaatac tgactagcaa ctctgaagtc ttaattcctt ttttgttatg 2880

atttatttgt gagtttacat ttttaaattg tttaactttc ttaatttagt aattaaaaag 2940

agagcatttt acatttgaa 2959

<210> 40
<211> 537
<212> PRT
<213> Homo sapiens

<400> 40

Met Gly Cys Val Gln Cys Lys Asp Lys Glu Ala Thr Lys Leu Thr Glu
1 5 10 15

Glu Arg Asp Gly Ser Leu Asn Gln Ser Ser Gly Tyr Arg Tyr Gly Thr
20 25 30

Asp Pro Thr Pro Gln His Tyr Pro Ser Phe Gly Val Thr Ser Ile Pro
35 40 45

Asn Tyr Asn Asn Phe His Ala Ala Gly Gly Gln Gly Leu Thr Val Phe
50 55 60

Gly Gly Val Asn Ser Ser Ser His Thr Gly Thr Leu Arg Thr Arg Gly
65 70 75 80

Gly Thr Gly Val Thr Leu Phe Val Ala Leu Tyr Asp Tyr Glu Ala Arg
85 90 95

Thr Glu Asp Asp Leu Ser Phe His Lys Gly Glu Lys Phe Gln Ile Leu
100 105 110

Asn Ser Ser Glu Gly Asp Trp Trp Glu Ala Arg Ser Leu Thr Thr Gly
115 120 125

Glu Thr Gly Tyr Ile Pro Ser Asn Tyr Val Ala Pro Val Asp Ser Ile
130 135 140

Gln Ala Glu Glu Trp Tyr Phe Gly Lys Leu Gly Arg Lys Asp Ala Glu
145 150 155 160

Arg Gln Leu Leu Ser Phe Gly Asn Pro Arg Gly Thr Phe Leu Ile Arg
165 170 175

Glu Ser Glu Thr Thr Lys Gly Ala Tyr Ser Leu Ser Ile Arg Asp Trp
180 185 190

Asp Asp Met Lys Gly Asp His Val Lys His Tyr Lys Ile Arg Lys Leu
195 200 205

Asp Asn Gly Gly Tyr Tyr Ile Thr Thr Arg Ala Gln Phe Glu Thr Leu
210 215 220

Gln Gln Leu Val Gln His Tyr Ser Glu Arg Ala Ala Gly Leu Cys Cys
225 230 235 240

Arg Leu Val Val Pro Cys His Lys Gly Met Pro Arg Leu Thr Asp Leu
245 250 255

Ser Val Lys Thr Lys Asp Val Trp Glu Ile Pro Arg Glu Ser Leu Gln
260 265 270

Leu Ile Lys Arg Leu Gly Asn Gly Gln Phe Gly Glu Val Trp Met Gly
275 280 285

Thr Trp Asn Gly Asn Thr Lys Val Ala Ile Lys Thr Leu Lys Pro Gly
290 295 300

Thr Met Ser Pro Glu Ser Phe Leu Glu Glu Ala Gln Ile Met Lys Lys
305 310 315 320

Leu Lys His Asp Lys Leu Val Gln Leu Tyr Ala Val Val Ser Glu Glu
325 330 335

Pro Ile Tyr Ile Val Thr Glu Tyr Met Asn Lys Gly Ser Leu Leu Asp

340 345 350

Phe Leu Lys Asp Gly Glu Gly Arg Ala Leu Lys Leu Pro Asn Leu Val
355 360 365

Asp Met Ala Ala Gln Val Ala Ala Gly Met Ala Tyr Ile Glu Arg Met
370 375 380

Asn Tyr Ile His Arg Asp Leu Arg Ser Ala Asn Ile Leu Val Gly Asn
385 390 395 400

Gly Leu Ile Cys Lys Ile Ala Asp Phe Gly Leu Ala Arg Leu Ile Glu
405 410 415

Asp Asn Glu Tyr Thr Ala Arg Gln Gly Ala Lys Phe Pro Ile Lys Trp
420 425 430

Thr Ala Pro Glu Ala Ala Leu Tyr Gly Arg Phe Thr Ile Lys Ser Asp
435 440 445

Val Trp Ser Phe Gly Ile Leu Leu Thr Glu Leu Val Thr Lys Gly Arg
450 455 460

Val Pro Tyr Pro Gly Met Asn Asn Arg Glu Val Leu Glu Gln Val Glu
465 470 475 480

Arg Gly Tyr Arg Met Pro Cys Pro Gln Asp Cys Pro Ile Ser Leu His
485 490 495

Glu Leu Met Ile His Cys Trp Lys Lys Asp Pro Glu Glu Arg Pro Thr
500 505 510

Phe Glu Tyr Leu Gln Ser Phe Leu Glu Asp Tyr Phe Thr Ala Thr Glu
515 520 525

Pro Gln Tyr Gln Pro Gly Glu Asn Leu
530 535

<210> 41
<211> 534
<212> PRT
<213> Homo sapiens

<400> 41

Met Gly Cys Val Gln Cys Lys Asp Lys Glu Ala Thr Lys Leu Thr Glu
1 5 10 15

Glu Arg Asp Gly Ser Leu Asn Gln Ser Ser Gly Tyr Arg Tyr Gly Thr

```
            20                  25                  30

Asp Pro Thr Pro Gln His Tyr Pro Ser Phe Gly Val Thr Ser Ile Pro
        35                  40                  45

Asn Tyr Asn Asn Phe His Ala Ala Gly Gly Gln Gly Leu Thr Val Phe
    50                  55                  60

Gly Gly Val Asn Ser Ser Ser His Thr Gly Thr Leu Arg Thr Arg Gly
65                  70                  75                  80

Gly Thr Gly Val Thr Leu Phe Val Ala Leu Tyr Asp Tyr Glu Ala Arg
                85                  90                  95

Thr Glu Asp Asp Leu Ser Phe His Lys Gly Glu Lys Phe Gln Ile Leu
            100                 105                 110

Asn Ser Ser Glu Gly Asp Trp Trp Glu Ala Arg Ser Leu Thr Thr Gly
        115                 120                 125

Glu Thr Gly Tyr Ile Pro Ser Asn Tyr Val Ala Pro Val Asp Ser Ile
    130                 135                 140

Gln Ala Glu Glu Trp Tyr Phe Gly Lys Leu Gly Arg Lys Asp Ala Glu
145                 150                 155                 160

Arg Gln Leu Leu Ser Phe Gly Asn Pro Arg Gly Thr Phe Leu Ile Arg
                165                 170                 175

Glu Ser Glu Thr Thr Lys Gly Ala Tyr Ser Leu Ser Ile Arg Asp Trp
            180                 185                 190

Asp Asp Met Lys Gly Asp His Val Lys His Tyr Lys Ile Arg Lys Leu
        195                 200                 205

Asp Asn Gly Gly Tyr Tyr Ile Thr Thr Arg Ala Gln Phe Glu Thr Leu
    210                 215                 220

Gln Gln Leu Val Gln His Tyr Ser Glu Lys Ala Asp Gly Leu Cys Phe
225                 230                 235                 240

Asn Leu Thr Val Ile Ala Ser Ser Cys Thr Pro Gln Thr Ser Gly Leu
                245                 250                 255

Ala Lys Asp Ala Trp Glu Val Ala Arg Arg Ser Leu Cys Leu Glu Lys
            260                 265                 270
```

Lys Leu Gly Gln Gly Cys Phe Ala Glu Val Trp Leu Gly Thr Trp Asn
275 280 285

Gly Asn Thr Lys Val Ala Ile Lys Thr Leu Lys Pro Gly Thr Met Ser
290 295 300

Pro Glu Ser Phe Leu Glu Glu Ala Gln Ile Met Lys Lys Leu Lys His
305 310 315 320

Asp Lys Leu Val Gln Leu Tyr Ala Val Val Ser Glu Glu Pro Ile Tyr
325 330 335

Ile Val Thr Glu Tyr Met Asn Lys Gly Ser Leu Leu Asp Phe Leu Lys
340 345 350

Asp Gly Glu Gly Arg Ala Leu Lys Leu Pro Asn Leu Val Asp Met Ala
355 360 365

Ala Gln Val Ala Ala Gly Met Ala Tyr Ile Glu Arg Met Asn Tyr Ile
370 375 380

His Arg Asp Leu Arg Ser Ala Asn Ile Leu Val Gly Asn Gly Leu Ile
385 390 395 400

Cys Lys Ile Ala Asp Phe Gly Leu Ala Arg Leu Ile Glu Asp Asn Glu
405 410 415

Tyr Thr Ala Arg Gln Gly Ala Lys Phe Pro Ile Lys Trp Thr Ala Pro
420 425 430

Glu Ala Ala Leu Tyr Gly Arg Phe Thr Ile Lys Ser Asp Val Trp Ser
435 440 445

Phe Gly Ile Leu Leu Thr Glu Leu Val Thr Lys Gly Arg Val Pro Tyr
450 455 460

Pro Gly Met Asn Asn Arg Glu Val Leu Glu Gln Val Glu Arg Gly Tyr
465 470 475 480

Arg Met Pro Cys Pro Gln Asp Cys Pro Ile Ser Leu His Glu Leu Met
485 490 495

Ile His Cys Trp Lys Lys Asp Pro Glu Glu Arg Pro Thr Phe Glu Tyr
500 505 510

Leu Gln Ser Phe Leu Glu Asp Tyr Phe Thr Ala Thr Glu Pro Gln Tyr
515 520 525

Gln Pro Gly Glu Asn Leu
    530

<210>  42
<211>  482
<212>  PRT
<213>  Homo sapiens

<400>  42

Met Gly Cys Val Gln Cys Lys Asp Lys Glu Ala Thr Lys Leu Thr Glu
1               5                   10                  15

Glu Arg Asp Gly Ser Leu Asn Gln Ser Ser Gly Tyr Arg Tyr Gly Thr
            20                  25                  30

Asp Pro Thr Pro Gln His Tyr Pro Ser Phe Gly Val Thr Ser Ile Pro
        35                  40                  45

Asn Tyr Asn Asn Phe His Ala Ala Gly Gly Gln Gly Leu Thr Val Phe
    50                  55                  60

Gly Gly Val Asn Ser Ser Ser His Thr Gly Thr Leu Arg Thr Arg Gly
65                  70                  75                  80

Gly Thr Gly Val Thr Leu Phe Val Ala Leu Tyr Asp Tyr Glu Ala Arg
                85                  90                  95

Thr Glu Asp Asp Leu Ser Phe His Lys Gly Glu Lys Phe Gln Ile Leu
            100                 105                 110

Asn Ser Ser Glu Gly Asp Trp Trp Glu Ala Arg Ser Leu Thr Thr Gly
        115                 120                 125

Glu Thr Gly Tyr Ile Pro Ser Asn Tyr Val Ala Pro Val Asp Ser Ile
    130                 135                 140

Gln Ala Glu Glu Trp Tyr Phe Gly Lys Leu Gly Arg Lys Asp Ala Glu
145                 150                 155                 160

Arg Gln Leu Leu Ser Phe Gly Asn Pro Arg Gly Thr Phe Leu Ile Arg
                165                 170                 175

Glu Ser Glu Thr Thr Lys Gly Ala Tyr Ser Leu Ser Ile Arg Asp Trp
            180                 185                 190

Asp Asp Met Lys Gly Asp His Val Lys His Tyr Lys Ile Arg Lys Leu
        195                 200                 205

```
Asp Asn Gly Gly Tyr Tyr Ile Thr Thr Arg Ala Gln Phe Glu Thr Leu
    210                 215                 220

Gln Gln Leu Val Gln His Tyr Ser Gly Thr Trp Asn Gly Asn Thr Lys
225                 230                 235                 240

Val Ala Ile Lys Thr Leu Lys Pro Gly Thr Met Ser Pro Glu Ser Phe
                245                 250                 255

Leu Glu Glu Ala Gln Ile Met Lys Lys Leu Lys His Asp Lys Leu Val
            260                 265                 270

Gln Leu Tyr Ala Val Val Ser Glu Glu Pro Ile Tyr Ile Val Thr Glu
        275                 280                 285

Tyr Met Asn Lys Gly Ser Leu Leu Asp Phe Leu Lys Asp Gly Glu Gly
    290                 295                 300

Arg Ala Leu Lys Leu Pro Asn Leu Val Asp Met Ala Ala Gln Val Ala
305                 310                 315                 320

Ala Gly Met Ala Tyr Ile Glu Arg Met Asn Tyr Ile His Arg Asp Leu
                325                 330                 335

Arg Ser Ala Asn Ile Leu Val Gly Asn Gly Leu Ile Cys Lys Ile Ala
            340                 345                 350

Asp Phe Gly Leu Ala Arg Leu Ile Glu Asp Asn Glu Tyr Thr Ala Arg
        355                 360                 365

Gln Gly Ala Lys Phe Pro Ile Lys Trp Thr Ala Pro Glu Ala Ala Leu
    370                 375                 380

Tyr Gly Arg Phe Thr Ile Lys Ser Asp Val Trp Ser Phe Gly Ile Leu
385                 390                 395                 400

Leu Thr Glu Leu Val Thr Lys Gly Arg Val Pro Tyr Pro Gly Met Asn
                405                 410                 415

Asn Arg Glu Val Leu Glu Gln Val Glu Arg Gly Tyr Arg Met Pro Cys
            420                 425                 430

Pro Gln Asp Cys Pro Ile Ser Leu His Glu Leu Met Ile His Cys Trp
        435                 440                 445

Lys Lys Asp Pro Glu Glu Arg Pro Thr Phe Glu Tyr Leu Gln Ser Phe
    450                 455                 460
```

Leu Glu Asp Tyr Phe Thr Ala Thr Glu Pro Gln Tyr Gln Pro Gly Glu
465 470 475 480

Asn Leu

<210> 43
<211> 2750
<212> DNA
<213> Homo sapiens

<400> 43
attgtttcct actccatttt ctctggggca atagcagaat aggagcaagc cagcactagt 60

cagctaacta agtgactcaa ccaaggcctt ttttccttgt tatctttgca gatacttcat 120

tttcttagcg tttctggaga ttacaacatc ctgcggttcc gtttctggga actttactga 180

tttatctccc ccctcacaca aataagcatt gattcctgca tttctgaaga tctcaagatc 240

tggactactg ttgaaaaaat ttccagtgag gctcacttat gtctgtaaag atgggaaaaa 300

aatacaagaa cattgttcta ctaaaaggat tagaggtcat caatgattat cattttagaa 360

tggttaagtc cttactgagc aacgatttaa aacttaattt aaaaatgaga gaagagtatg 420

acaaaattca gattgctgac ttgatggaag aaaagttccg aggtgatgct ggtttgggca 480

aactaataaa aattttcgaa gatataccaa cgcttgaaga cctggctgaa actcttaaaa 540

aagaaaagtt aaaagtaaaa ggaccagccc tatcaagaaa gaggaagaag gaagtggatg 600

ctacttcacc tgcaccctcc acaagcagca ctgtcaaaac tgaaggagca gaggcaactc 660

ctggagctca gaaaagaaaa aaatcaacca aagaaaaggc tggacccaaa gggagtaagg 720

tgtccgagga acagactcag cctccctctc ctgcaggagc cggcatgtcc acagccatgg 780

gccgttcccc atctcccaag acctcattgt cagctccacc caacagttct tcaactgaga 840

acccgaaaac agtggccaaa tgtcaggtaa ctcccagaag aaatgttctc caaaaacgcc 900

cagtgatagt gaaggtactg agtacaacaa agccatttga atatgagacc ccagaaatgg 960

agaaaaaaat aatgtttcat gctacagtgg ctacacagac acagttcttc catgtgaagg 1020

ttttaaacac cagcttgaag gagaaattca atggaaagaa aatcatcatc atatcagatt 1080

atttggaata tgatagtctc ctagaggtca atgaagaatc tactgtatct gaagctggtc 1140

ctaaccaaac gtttgaggtt ccaaataaaa tcatcaacag agcaaaggaa actctgaaga 1200

ttgatattct tcacaaacaa gcttcaggaa atattgtata tggggtattt atgctacata 1260

agaaaacagt aaatcagaag accacaatct acgaaattca ggatgataga ggaaaaatgg 1320

atgtagtggg gacaggacaa tgtcacaata tcccctgtga agaaggagat aagctccaac 1380

ttttctgctt tcgacttaga aaaaagaacc agatgtcaaa actgatttca gaaatgcata 1440

gttttatcca gataaagaaa aaaacaaacc cgagaaacaa tgaccccaag agcatgaagc 1500

taccccagga acagcgtcag cttccatatc cttcagaggc cagcacaacc ttccctgaga 1560

gccatcttcg gactcctcag atgccaccaa caactccatc cagcagtttc ttcaccaaga 1620

aaagtgaaga cacaatctcc aaaatgaatg acttcatgag gatgcagata ctgaaggaag 1680

ggagtcattt tccaggaccg ttcatgacca gcataggccc agctgagagc catccccaca 1740

ctcctcagat gcctccatca acaccaagca gcagtttctt aaccacgttg aaaccaagac 1800

tgaagactga acctgaagaa gtttccatag aagacagtgc ccagagtgac ctcaaagaag 1860

tgatggtgct gaacgcaaca gaatcatttg tatatgagcc caaagagcag aagaaaatgt 1920

ttcatgccac agtggcaact gagaatgaag tcttccgagt gaaggttttt aatattgacc 1980

taaaggagaa gttcacccca aagaagatca ttgccatagc aaattatgtt tgccgcaatg 2040

ggttcctgga ggtatatcct ttcacacttg tggctgatgt gaatgctgac cgaaacatgg 2100

agatcccaaa aggattgatt agaagtgcca gcgtaactcc taaaatcaat cagctttgct 2160

cacaaactaa aggaagtttt gtgaatgggg tgtttgaggt acataagaaa aatgtaaggg 2220

gtgaattcac ttattatgaa atacaagata atacagggaa gatggaagtg gtggtgcatg 2280

gacgactgac cacaatcaac tgtgaggaag gagataaact gaaactcacc tgctttgaat 2340

tggcaccgaa aagtgggaat accggggagt tgagatctgt aattcatagt cacatcaagg 2400

tcatcaagac caggaaaaac aagaaagaca tactcaatcc tgattcaagt atggaaactt 2460

caccagactt tttcttctaa aatctggatg tcattgacga taatgtttat ggagataagg 2520

tctaagtgcc taaaaaaatg tacatatacc tggttgaaat acaacactat acatacacac 2580

caccatatat actagctgtt aatcctatgg aatggggtat tgggagtgct tttttaattt 2640

ttcatagttt ttttttaata aaatggcata ttttgcatct acaacttcta taatttgaaa 2700

aaataaataa acattatctt ttttgtgaaa ggaaaaaaaa aaaaaaaaaa 2750

<210> 44
<211> 729
<212> PRT
<213> Homo sapiens

<400> 44

Met Gly Lys Lys Tyr Lys Asn Ile Val Leu Leu Lys Gly Leu Glu Val
1 5 10 15

Ile Asn Asp Tyr His Phe Arg Met Val Lys Ser Leu Leu Ser Asn Asp
20 25 30

Leu Lys Leu Asn Leu Lys Met Arg Glu Glu Tyr Asp Lys Ile Gln Ile
35 40 45

Ala Asp Leu Met Glu Glu Lys Phe Arg Gly Asp Ala Gly Leu Gly Lys
    50                  55                  60

Leu Ile Lys Ile Phe Glu Asp Ile Pro Thr Leu Glu Asp Leu Ala Glu
65                  70                  75                  80

Thr Leu Lys Lys Glu Lys Leu Lys Val Lys Gly Pro Ala Leu Ser Arg
                85                  90                  95

Lys Arg Lys Lys Glu Val Asp Ala Thr Ser Pro Ala Pro Ser Thr Ser
            100                 105                 110

Ser Thr Val Lys Thr Glu Gly Ala Glu Ala Thr Pro Gly Ala Gln Lys
        115                 120                 125

Arg Lys Lys Ser Thr Lys Glu Lys Ala Gly Pro Lys Gly Ser Lys Val
    130                 135                 140

Ser Glu Glu Gln Thr Gln Pro Pro Ser Pro Ala Gly Ala Gly Met Ser
145                 150                 155                 160

Thr Ala Met Gly Arg Ser Pro Ser Pro Lys Thr Ser Leu Ser Ala Pro
                165                 170                 175

Pro Asn Ser Ser Ser Thr Glu Asn Pro Lys Thr Val Ala Lys Cys Gln
            180                 185                 190

Val Thr Pro Arg Arg Asn Val Leu Gln Lys Arg Pro Val Ile Val Lys
        195                 200                 205

Val Leu Ser Thr Thr Lys Pro Phe Glu Tyr Glu Thr Pro Glu Met Glu
    210                 215                 220

Lys Lys Ile Met Phe His Ala Thr Val Ala Thr Gln Thr Gln Phe Phe
225                 230                 235                 240

His Val Lys Val Leu Asn Thr Ser Leu Lys Glu Lys Phe Asn Gly Lys
                245                 250                 255

Lys Ile Ile Ile Ile Ser Asp Tyr Leu Glu Tyr Asp Ser Leu Leu Glu
            260                 265                 270

Val Asn Glu Glu Ser Thr Val Ser Glu Ala Gly Pro Asn Gln Thr Phe
        275                 280                 285

Glu Val Pro Asn Lys Ile Ile Asn Arg Ala Lys Glu Thr Leu Lys Ile

290 295 300

Asp Ile Leu His Lys Gln Ala Ser Gly Asn Ile Val Tyr Gly Val Phe
305 310 315 320

Met Leu His Lys Lys Thr Val Asn Gln Lys Thr Thr Ile Tyr Glu Ile
325 330 335

Gln Asp Asp Arg Gly Lys Met Asp Val Val Gly Thr Gly Gln Cys His
340 345 350

Asn Ile Pro Cys Glu Glu Gly Asp Lys Leu Gln Leu Phe Cys Phe Arg
355 360 365

Leu Arg Lys Lys Asn Gln Met Ser Lys Leu Ile Ser Glu Met His Ser
370 375 380

Phe Ile Gln Ile Lys Lys Lys Thr Asn Pro Arg Asn Asn Asp Pro Lys
385 390 395 400

Ser Met Lys Leu Pro Gln Glu Gln Arg Gln Leu Pro Tyr Pro Ser Glu
405 410 415

Ala Ser Thr Thr Phe Pro Glu Ser His Leu Arg Thr Pro Gln Met Pro
420 425 430

Pro Thr Thr Pro Ser Ser Ser Phe Phe Thr Lys Lys Ser Glu Asp Thr
435 440 445

Ile Ser Lys Met Asn Asp Phe Met Arg Met Gln Ile Leu Lys Glu Gly
450 455 460

Ser His Phe Pro Gly Pro Phe Met Thr Ser Ile Gly Pro Ala Glu Ser
465 470 475 480

His Pro His Thr Pro Gln Met Pro Pro Ser Thr Pro Ser Ser Ser Phe
485 490 495

Leu Thr Thr Leu Lys Pro Arg Leu Lys Thr Glu Pro Glu Glu Val Ser
500 505 510

Ile Glu Asp Ser Ala Gln Ser Asp Leu Lys Glu Val Met Val Leu Asn
515 520 525

Ala Thr Glu Ser Phe Val Tyr Glu Pro Lys Glu Gln Lys Lys Met Phe
530 535 540

His Ala Thr Val Ala Thr Glu Asn Glu Val Phe Arg Val Lys Val Phe
545 550 555 560

Asn Ile Asp Leu Lys Glu Lys Phe Thr Pro Lys Lys Ile Ile Ala Ile
565 570 575

Ala Asn Tyr Val Cys Arg Asn Gly Phe Leu Glu Val Tyr Pro Phe Thr
580 585 590

Leu Val Ala Asp Val Asn Ala Asp Arg Asn Met Glu Ile Pro Lys Gly
595 600 605

Leu Ile Arg Ser Ala Ser Val Thr Pro Lys Ile Asn Gln Leu Cys Ser
610 615 620

Gln Thr Lys Gly Ser Phe Val Asn Gly Val Phe Glu Val His Lys Lys
625 630 635 640

Asn Val Arg Gly Glu Phe Thr Tyr Tyr Glu Ile Gln Asp Asn Thr Gly
645 650 655

Lys Met Glu Val Val Val His Gly Arg Leu Thr Thr Ile Asn Cys Glu
660 665 670

Glu Gly Asp Lys Leu Lys Leu Thr Cys Phe Glu Leu Ala Pro Lys Ser
675 680 685

Gly Asn Thr Gly Glu Leu Arg Ser Val Ile His Ser His Ile Lys Val
690 695 700

Ile Lys Thr Arg Lys Asn Lys Lys Asp Ile Leu Asn Pro Asp Ser Ser
705 710 715 720

Met Glu Thr Ser Pro Asp Phe Phe Phe
725

<210> 45
<211> 3617
<212> DNA
<213> Homo sapiens

<400> 45
atcgaaacag aaaccaaagt caggcaaact ctgtaagaac tgcctgacag aaagctggac 60

tcaaagctcc tacccgagtg tgcagcagga tcgccccggt ccgggacccc aggcgcacac 120

cgcagagtcc aaagtgccgc gcctgccggc cgcacctgcc tgccgcggcc ccgcgcgccg 180

ccccgctgcc cacctgcccg cctgcccacc tgcccaggtg cgagtgcagc cccgcgcgcc 240

ggcctgagag ccctgtggac aacctcgtca ttgtcaggca cagagcggta gaccctgctt 300

ctctaagtgg gcagcggaca gcggcacgca catttcacct gtcccgcaga caacagcacc 360
atctgcttgg gagaaccctc tcccttctct gagaaagaaa gatgtcgaat gggtattcca 420
cagacgagaa tttccgctat ctcatctcgt gcttcagggc cagggtgaaa atgtacatcc 480
aggtggagcc tgtgctggac tacctgacct ttctgcctgc agaggtgaag gagcagattc 540
agaggacagt cgccacctcc gggaacatgc aggcagttga actgctgctg agcaccttgg 600
agaagggagt ctggcacctt ggttggactc gggaattcgt ggaggccctc cggagaaccg 660
gcagccctct ggccgcccgc tacatgaacc ctgagctcac ggacttgccc tctccatcgt 720
ttgagaacgc tcatgatgaa tatctccaac tgctgaacct ccttcagccc actctggtgg 780
acaagcttct agttagagac gtcttggata agtgcatgga ggaggaactg ttgacaattg 840
aagacagaaa ccggattgct gctgcagaaa acaatggaaa tgaatcaggt gtaagagagc 900
tactaaaaag gattgtgcag aaagaaaact ggttctctgc atttctgaat gttcttcgtc 960
aaacaggaaa caatgaactt gtccaagagt taacaggctc tgattgctca gaaagcaatg 1020
cagagattga gaatttatca caagttgatg gtcctcaagt ggaagagcaa cttctttcaa 1080
ccacagttca gccaaatctg gagaaggagg tctggggcat ggagaataac tcatcagaat 1140
catcttttgc agattcttct gtagtttcag aatcagacac aagtttggca gaaggaagtg 1200
tcagctgctt agatgaaagt cttggacata acagcaacat gggcagtgat tcaggcacca 1260
tgggaagtga ttcagatgaa gagaatgtgg cagcaagagc atccccggag ccagaactcc 1320
agctcaggcc ttaccaaatg gaagttgccc agccagcctt ggaagggaag aatatcatca 1380
tctgcctccc tacagggagt ggaaaaacca gagtggctgt ttacattgcc aaggatcact 1440
tagacaagaa gaaaaaagca tctgagcctg gaaaagttat agttcttgtc aataaggtac 1500
tgctagttga acagctcttc cgcaaggagt tccaaccatt tttgaagaaa tggtatcgtg 1560
ttattggatt aagtggtgat acccaactga aaatatcatt tccagaagtt gtcaagtcct 1620
gtgatattat tatcagtaca gctcaaatcc ttgaaaactc cctcttaaac ttggaaaatg 1680
gagaagatgc tggtgttcaa ttgtcagact tttccctcat tatcattgat gaatgtcatc 1740
acaccaacaa agaagcagtg tataataaca tcatgaggca ttatttgatg cagaagttga 1800
aaaacaatag actcaagaaa gaaaacaaac cagtgattcc ccttcctcag atactgggac 1860
taacagcttc acctggtgtt ggagggcca cgaagcaagc caaagctgaa gaacacattt 1920
taaaactatg tgccaatctt gatgcattta ctattaaaac tgttaaagaa aaccttgatc 1980
aactgaaaaa ccaaatacag gagccatgca agaagtttgc cattgcagat gcaaccagag 2040
aagatccatt taaagagaaa cttctagaaa taatgacaag gattcaaact tattgtcaaa 2100
tgagtccaat gtcagatttt ggaactcaac cctatgaaca atgggccatt caaatggaaa 2160

aaaaagctgc aaaagaagga aatcgcaaag aacgtgtttg tgcagaacat ttgaggaagt 2220

acaatgaggc cctacaaatt aatgacacaa ttcgaatgat agatgcgtat actcatcttg 2280

aaactttcta taatgaagag aaagataaga agtttgcagt catagaagat gatagtgatg 2340

agggtggtga tgatgagtat tgtgatggtg atgaagatga ggatgattta aagaaacctt 2400

tgaaactgga tgaaacagat agatttctca tgactttatt ttttgaaaac aataaaatgt 2460

tgaaaaggct ggctgaaaac ccagaatatg aaaatgaaaa gctgaccaaa ttaagaaata 2520

ccataatgga gcaatatact aggactgagg aatcagcacg aggaataatc tttacaaaaa 2580

cacgacagag tgcatatgcg ctttcccagt ggattactga aaatgaaaaa tttgctgaag 2640

taggagtcaa agcccaccat ctgattggag ctggacacag cagtgagttc aaacccatga 2700

cacagaatga acaaaaagaa gtcattagta aatttcgcac tggaaaaata aatctgctta 2760

tcgctaccac agtggcagaa gaaggtctgg atattaaaga atgtaacatt gttatccgtt 2820

atggtctcgt caccaatgaa atagccatgg tccaggcccg tggtcgagcc agagctgatg 2880

agagcaccta cgtcctggtt gctcacagtg gttcaggagt tatcgaacat gagacagtta 2940

atgatttccg agagaagatg atgtataaag ctatacattg tgttcaaaat atgaaaccag 3000

aggagtatgc tcataagatt ttggaattac agatgcaaag tataatggaa aagaaaatga 3060

aaaccaagag aaatattgcc aagcattaca agaataaccc atcactaata actttccttt 3120

gcaaaaactg cagtgtgcta gcctgttctg ggaagatat ccatgtaatt gagaaaatgc 3180

atcacgtcaa tatgacccca gaattcaagg aactttacat tgtaagagaa aacaaagcac 3240

tgcaaaagaa gtgtgccgac tatcaaataa atggtgaaat catctgcaaa tgtggccagg 3300

cttggggaac aatgatggtg cacaaaggct tagatttgcc ttgtctcaaa ataaggaatt 3360

ttgtagtggt tttcaaaaat aattcaacaa agaaacaata caaaaagtgg gtagaattac 3420

ctatcacatt tcccaatctt gactattcag aatgctgttt atttagtgat gaggattagc 3480

acttgattga agattctttt aaaatactat cagttaaaca tttaatatga ttatgattaa 3540

tgtattcatt atgctacaga actgacataa gaatcaataa aatgattgtt ttactctgca 3600

aaaaaaaaaa aaaaaaa 3617

<210> 46
<211> 1025
<212> PRT
<213> Homo sapiens

<400> 46

Met Ser Asn Gly Tyr Ser Thr Asp Glu Asn Phe Arg Tyr Leu Ile Ser
1 5 10 15

Cys Phe Arg Ala Arg Val Lys Met Tyr Ile Gln Val Glu Pro Val Leu

```
            20                  25                  30

Asp Tyr Leu Thr Phe Leu Pro Ala Glu Val Lys Glu Gln Ile Gln Arg
        35                  40                  45

Thr Val Ala Thr Ser Gly Asn Met Gln Ala Val Glu Leu Leu Leu Ser
    50                  55                  60

Thr Leu Glu Lys Gly Val Trp His Leu Gly Trp Thr Arg Glu Phe Val
65                  70                  75                  80

Glu Ala Leu Arg Arg Thr Gly Ser Pro Leu Ala Ala Arg Tyr Met Asn
                85                  90                  95

Pro Glu Leu Thr Asp Leu Pro Ser Pro Ser Phe Glu Asn Ala His Asp
            100                 105                 110

Glu Tyr Leu Gln Leu Leu Asn Leu Leu Gln Pro Thr Leu Val Asp Lys
        115                 120                 125

Leu Leu Val Arg Asp Val Leu Asp Lys Cys Met Glu Glu Glu Leu Leu
    130                 135                 140

Thr Ile Glu Asp Arg Asn Arg Ile Ala Ala Ala Glu Asn Asn Gly Asn
145                 150                 155                 160

Glu Ser Gly Val Arg Glu Leu Leu Lys Arg Ile Val Gln Lys Glu Asn
                165                 170                 175

Trp Phe Ser Ala Phe Leu Asn Val Leu Arg Gln Thr Gly Asn Asn Glu
            180                 185                 190

Leu Val Gln Glu Leu Thr Gly Ser Asp Cys Ser Glu Ser Asn Ala Glu
        195                 200                 205

Ile Glu Asn Leu Ser Gln Val Asp Gly Pro Gln Val Glu Glu Gln Leu
    210                 215                 220

Leu Ser Thr Thr Val Gln Pro Asn Leu Glu Lys Glu Val Trp Gly Met
225                 230                 235                 240

Glu Asn Asn Ser Ser Glu Ser Ser Phe Ala Asp Ser Ser Val Val Ser
                245                 250                 255

Glu Ser Asp Thr Ser Leu Ala Glu Gly Ser Val Ser Cys Leu Asp Glu
            260                 265                 270
```

Ser Leu Gly His Asn Ser Asn Met Gly Ser Asp Ser Gly Thr Met Gly
275 280 285

Ser Asp Ser Asp Glu Glu Asn Val Ala Ala Arg Ala Ser Pro Glu Pro
290 295 300

Glu Leu Gln Leu Arg Pro Tyr Gln Met Glu Val Ala Gln Pro Ala Leu
305 310 315 320

Glu Gly Lys Asn Ile Ile Ile Cys Leu Pro Thr Gly Ser Gly Lys Thr
325 330 335

Arg Val Ala Val Tyr Ile Ala Lys Asp His Leu Asp Lys Lys Lys Lys
340 345 350

Ala Ser Glu Pro Gly Lys Val Ile Val Leu Val Asn Lys Val Leu Leu
355 360 365

Val Glu Gln Leu Phe Arg Lys Glu Phe Gln Pro Phe Leu Lys Lys Trp
370 375 380

Tyr Arg Val Ile Gly Leu Ser Gly Asp Thr Gln Leu Lys Ile Ser Phe
385 390 395 400

Pro Glu Val Val Lys Ser Cys Asp Ile Ile Ile Ser Thr Ala Gln Ile
405 410 415

Leu Glu Asn Ser Leu Leu Asn Leu Glu Asn Gly Glu Asp Ala Gly Val
420 425 430

Gln Leu Ser Asp Phe Ser Leu Ile Ile Ile Asp Glu Cys His His Thr
435 440 445

Asn Lys Glu Ala Val Tyr Asn Asn Ile Met Arg His Tyr Leu Met Gln
450 455 460

Lys Leu Lys Asn Asn Arg Leu Lys Lys Glu Asn Lys Pro Val Ile Pro
465 470 475 480

Leu Pro Gln Ile Leu Gly Leu Thr Ala Ser Pro Gly Val Gly Gly Ala
485 490 495

Thr Lys Gln Ala Lys Ala Glu Glu His Ile Leu Lys Leu Cys Ala Asn
500 505 510

Leu Asp Ala Phe Thr Ile Lys Thr Val Lys Glu Asn Leu Asp Gln Leu
515 520 525

Lys Asn Gln Ile Gln Glu Pro Cys Lys Lys Phe Ala Ile Ala Asp Ala
    530                 535                 540

Thr Arg Glu Asp Pro Phe Lys Glu Lys Leu Leu Glu Ile Met Thr Arg
545                 550                 555                 560

Ile Gln Thr Tyr Cys Gln Met Ser Pro Met Ser Asp Phe Gly Thr Gln
                565                 570                 575

Pro Tyr Glu Gln Trp Ala Ile Gln Met Glu Lys Lys Ala Ala Lys Glu
            580                 585                 590

Gly Asn Arg Lys Glu Arg Val Cys Ala Glu His Leu Arg Lys Tyr Asn
        595                 600                 605

Glu Ala Leu Gln Ile Asn Asp Thr Ile Arg Met Ile Asp Ala Tyr Thr
    610                 615                 620

His Leu Glu Thr Phe Tyr Asn Glu Glu Lys Asp Lys Lys Phe Ala Val
625                 630                 635                 640

Ile Glu Asp Asp Ser Asp Glu Gly Gly Asp Asp Glu Tyr Cys Asp Gly
                645                 650                 655

Asp Glu Asp Glu Asp Asp Leu Lys Lys Pro Leu Lys Leu Asp Glu Thr
            660                 665                 670

Asp Arg Phe Leu Met Thr Leu Phe Phe Glu Asn Asn Lys Met Leu Lys
        675                 680                 685

Arg Leu Ala Glu Asn Pro Glu Tyr Glu Asn Glu Lys Leu Thr Lys Leu
    690                 695                 700

Arg Asn Thr Ile Met Glu Gln Tyr Thr Arg Thr Glu Glu Ser Ala Arg
705                 710                 715                 720

Gly Ile Ile Phe Thr Lys Thr Arg Gln Ser Ala Tyr Ala Leu Ser Gln
                725                 730                 735

Trp Ile Thr Glu Asn Glu Lys Phe Ala Glu Val Gly Val Lys Ala His
            740                 745                 750

His Leu Ile Gly Ala Gly His Ser Ser Glu Phe Lys Pro Met Thr Gln
        755                 760                 765

Asn Glu Gln Lys Glu Val Ile Ser Lys Phe Arg Thr Gly Lys Ile Asn
    770                 775                 780

```
Leu Leu Ile Ala Thr Thr Val Ala Glu Glu Gly Leu Asp Ile Lys Glu
785                 790                 795                 800

Cys Asn Ile Val Ile Arg Tyr Gly Leu Val Thr Asn Glu Ile Ala Met
                805                 810                 815

Val Gln Ala Arg Gly Arg Ala Arg Ala Asp Glu Ser Thr Tyr Val Leu
            820                 825                 830

Val Ala His Ser Gly Ser Gly Val Ile Glu His Glu Thr Val Asn Asp
        835                 840                 845

Phe Arg Glu Lys Met Met Tyr Lys Ala Ile His Cys Val Gln Asn Met
    850                 855                 860

Lys Pro Glu Glu Tyr Ala His Lys Ile Leu Glu Leu Gln Met Gln Ser
865                 870                 875                 880

Ile Met Glu Lys Lys Met Lys Thr Lys Arg Asn Ile Ala Lys His Tyr
                885                 890                 895

Lys Asn Asn Pro Ser Leu Ile Thr Phe Leu Cys Lys Asn Cys Ser Val
            900                 905                 910

Leu Ala Cys Ser Gly Glu Asp Ile His Val Ile Glu Lys Met His His
        915                 920                 925

Val Asn Met Thr Pro Glu Phe Lys Glu Leu Tyr Ile Val Arg Glu Asn
    930                 935                 940

Lys Ala Leu Gln Lys Lys Cys Ala Asp Tyr Gln Ile Asn Gly Glu Ile
945                 950                 955                 960

Ile Cys Lys Cys Gly Gln Ala Trp Gly Thr Met Met Val His Lys Gly
                965                 970                 975

Leu Asp Leu Pro Cys Leu Lys Ile Arg Asn Phe Val Val Val Phe Lys
            980                 985                 990

Asn Asn Ser Thr Lys Lys Gln Tyr  Lys Lys Trp Val Glu  Leu Pro Ile
        995                 1000                1005

Thr Phe  Pro Asn Leu Asp Tyr  Ser Glu Cys Cys Leu  Phe Ser Asp
    1010                1015                1020

Glu Asp
```

1025

<210> 47
<211> 4411
<212> DNA
<213> Homo sapiens

<400> 47
aactgaaaat ccacaagaca gaatagccag atctcagagg agcctggcta agcaaaaccc 60

tgcagaacgg ctgcctaatt tacagcaacc atgaggccac ttaaggatgc agcaagaagg 120

agccatctgc aatccaggaa gaaattcctt gccaggaacc aaattggttg tcaccttcat 180

ctaggacttc tagcctcgag aacttacaaa tggtgatgat catcaggtca aggatagtct 240

ggagcaattg agatgtcact ttacatggga gttatccatt gatgacgatg aaatgcctga 300

tttagaaaac agagtcttgg atcagattga attcctagac accaaataca gtgtgggaat 360

acacaaccta ctagcctatg tgaaacacct gaaaggccag aatgaggaag ccctgaagag 420

cttaaaagaa gctgaaaact taatgcagga agaacatgac aaccaagcaa atgtgaggag 480

tctggtgacc tggggcaact ttgcctggat gtattaccac atgggcagac tggcagaagc 540

ccagacttac ctggacaagg tggagaacat ttgcaagaag ctttcaaatc ccttccgcta 600

tagaatggag tgtccagaaa tagactgtga ggaaggatgg gccttgctga agtgtggagg 660

aaaaaattat gaacgggcca aggcctgctt tgaaaaggtg cttgaagtgg accctgaaaa 720

ccctgaatcc agcgctgggt atgcgatctc tgcctatcgc ctggatggct ttaaattagc 780

cacaaaaaat cacaagccat ttctcttgct tcccctaagg caggctgtcc gcttaaatcc 840

agacaatgga tatattaagg ttctccttgc cctgaagctt caggatgaag gacaggaagc 900

tgaaggagaa aagtacattg aagaagctct agccaacatg tcctcacaga cctatgtctt 960

tcgatatgca gccaagtttt accgaagaaa aggctctgtg gataaagctc ttgagttatt 1020

aaaaaaggcc ttgcaggaaa cacccacttc tgtcttactg catcaccaga tagggctttg 1080

ctacaaggca caaatgatcc aaatcaagga ggctacaaaa gggcagccta gagggcagaa 1140

cagagaaaag ctagacaaaa tgataagatc agccatattt cattttgaat ctgcagtgga 1200

aaaaaagccc acatttgagg tggctcatct agacctggca agaatgtata tagaagcagg 1260

caatcacaga aaagctgaag agaattttca aaaattgtta tgcatgaaac cagtggtaga 1320

agaaacaatg caagacatac atttccacta tggtcggttt caggaatttc aaaagaaatc 1380

tgacgtcaat gcaattatcc attatttaaa agctataaaa atagaacagg catcattaac 1440

aagggataaa agtatcaatt ctttgaagaa attggtttta aggaaacttc ggagaaaggc 1500

attagatctg gaaagcttga gcctccttgg gttcgtctac aaattggaag gaaatatgaa 1560

tgaagccctg gagtactatg agcgggccct gagactggct gctgactttg agaactctgt 1620

gagacaaggt ccttaggcac ccagatatca gccactttca catttcattt cattttatgc 1680
taacatttac taatcatctt ttctgcttac tgttttcaga aacattataa ttcactgtaa 1740
tgatgtaatt cttgaataat aaatctgaca aaatattagt tgtgttcaac aattagtgaa 1800
acagaatgtg tgtatgcatg taagaaagag aaatcatttg tatgagtgct atgtagtaga 1860
gaaaaaatgt tagttaactt tgtaggaaat aaaacattgg acttacacta aatgtttaat 1920
tcattcattt tattgtgaaa taaaaataaa atccttagct cctccaccaa ctgaacagac 1980
cctcttggcc aaggagaccc cagaaacctt aaaaactaag tttcccaacc atgacaagat 2040
gagagatcat tcacacctca ttatattccc tcccttgcta actgccattg gactttttcc 2100
actgagttaa acagaaaccc atggaaaaca aagaacagaa gactcactcc ttggctgact 2160
tcacctagct cactccacgt agcgccacag ccagactccc ctcccctctt gcggtttcca 2220
catgacaact gatcagcctt ccctcctgat aagtgaccac tgcccacaga ctggttctgg 2280
ccagtccatg gaggctgcac acagggtgcc tctatgtcct ttgtttcacc ttttgatata 2340
gaaaggctaa ttttgctgta ttttaatgtt aagtctccac cacagagtga acacagaatg 2400
catgtgacat acatgtttac ataccactat tgtgtgactg cccctcatga atattcatag 2460
ccccccataa cctgttaact atgtgtgtct agccaatcca ccaaccataa aacttctgta 2520
ataccctccc ttcctccaag agcctgcttt tggttgctgt ggtaggctct gcttcccagg 2580
ctgcaggttg caggagagga ggctgcagtg gctcacgcct gtaatctcag cacttcgatg 2640
ggacgaggca ggcagatcac ctgaacccag gagttcgaga gcagccttgg caatggcaaa 2700
accaaccgtc tctacaaaaa atgcaaaaac ttagctgggt gtggtggcat gcacctgtag 2760
cttcagttcc agctactcag gaggctgagg tgagtggact gctggagcca gggagttcga 2820
ggctgcagtg tcgagatctt gccactgcac tccattctgg atgatagaac gagaccccat 2880
ctcaaaaaaa aaaaaagttc tctccaattg tatatagctt gtgattttat gtcaacacta 2940
tcaataaata gctttcagtg caagaaacca aaaatactgt aataaacagg cacatattct 3000
tcccaaacct catgcagttt acaatctagt gagagacaca gatagcagta cagagtcaat 3060
taaaggttag ttttcttcat gaagatgttt taattttaat tcaatgtgaa agggttccaa 3120
ggagtttatc ttgttttatg ccattttatt tgaagcacta cttactaagt catttgctga 3180
tattaatcta gttaaatcaa gaaatattac atgaaaatgt tgctaaatca gagatcatgg 3240
gtaacaatca cctttgatta tgaataatca tattttattg aaaggcaagg cacaacaaat 3300
aataagaagg aaaaaataaa taagcaatgt tattgatctt tcattctgta tatgttttgg 3360
ggggaatata ctagtttctt ttagtggctg taacaaatta ccacaaactt ggtgacttaa 3420
aatttcacag atttactctt tcttacagtt ctggaggtca gaagtctgaa atgggtttca 3480
atgagccaaa gtcaaggtat tgatgacgct acactcctcc ggaggctcta ggcagatagc 3540

cttttccagc ttccagaggc tgcctgaatt ctttcatcca tcttaaaaac caacagtgta 3600

gtagcctcaa atctctctct ctgcttcctt cttcacatct ccttctctcc tctgactctt 3660

ttgcctcttt cttctaagga cgcaccaggt ccacctgcat aatccagaat aattgcccca 3720

tccgcaaatc cttaatttaa taacatctgc aaagtccctt ttgctatgta aagtagcatg 3780

ttcacaggtt ctggagactt ggccatggat acgattgcgg gggggcatt attcttacca 3840

cagagcaccc caagaaaatc tccaaatttt gggcttccaa tccattttgc ttcaattatt 3900

taatattttt actccttcca gtagatactg atttcatcca ttgcccttaa gaaggtagga 3960

cagagattat ggcacatctc acattaaatg ctatattttc gttggaaata catttttgc 4020

ttcaactttt attttaaatt caagggtaca tgtgcaggat gttcaggttt gttacacagg 4080

taaacgtgtg ccatggcggt ttgctgaaca gatcatccca tcaccaacag atcatcccat 4140

tgagaggtga agccggctgg gcttctgggt tgggtgggga cttggagaac ttttctgtct 4200

agctaaagta ttgtaaaatg gaccagtcaa cactctgtaa aatggaccaa tcagctctct 4260

gtaaaatgga ccaatcagca ggatgtgggt ggggccaagt aagggaataa aagcaggcca 4320

cccgagctgg cagcggcaac ccgctcgggt ccccttccat gctgtggaag ttttgttctt 4380

tcgctctttc aataaatctt gctgctgctc a 4411

<210> 48
<211> 4302
<212> DNA
<213> Homo sapiens

<400> 48
aactgaaaat ccacaagaca gaatagccag atctcagagg agcctggcta agcaaaaccc 60

tgcagaacgg ctgcctaatt tacagcaacc atgagtacaa atggtgatga tcatcaggtc 120

aaggatagtc tggagcaatt gagatgtcac tttacatggg agttatccat tgatgacgat 180

gaaatgcctg atttagaaaa cagagtcttg gatcagattg aattcctaga caccaaatac 240

agtgtgggaa tacacaacct actagcctat gtgaaacacc tgaaaggcca gaatgaggaa 300

gccctgaaga gcttaaaaga agctgaaaac ttaatgcagg aagaacatga caaccaagca 360

aatgtgagga gtctggtgac ctggggcaac tttgcctgga tgtattacca catgggcaga 420

ctggcagaag cccagactta cctggacaag gtggagaaca tttgcaagaa gctttcaaat 480

cccttccgct atagaatgga gtgtccagaa atagactgtg aggaaggatg ggccttgctg 540

aagtgtggag gaaaaaatta tgaacgggcc aaggcctgct ttgaaaaggt gcttgaagtg 600

gaccctgaaa accctgaatc cagcgctggg tatgcgatct ctgcctatcg cctggatggc 660

tttaaattag ccacaaaaaa tcacaagcca ttttctttgc ttcccctaag gcaggctgtc 720

cgcttaaatc cagacaatgg atatattaag gttctccttg ccctgaagct tcaggatgaa 780

ggacaggaag ctgaaggaga aaagtacatt gaagaagctc tagccaacat gtcctcacag 840
acctatgtct ttcgatatgc agccaagttt taccgaagaa aaggctctgt ggataaagct 900
cttgagttat taaaaaaggc cttgcaggaa acacccactt ctgtcttact gcatcaccag 960
atagggcttt gctacaaggc acaaatgatc caaatcaagg aggctacaaa agggcagcct 1020
agagggcaga acagagaaaa gctagacaaa atgataagat cagccatatt tcattttgaa 1080
tctgcagtgg aaaaaaagcc cacatttgag gtggctcatc tagacctggc aagaatgtat 1140
atagaagcag gcaatcacag aaaagctgaa gagaattttc aaaaattgtt atgcatgaaa 1200
ccagtggtag aagaaacaat gcaagacata catttccact atggtcggtt tcaggaattt 1260
caaaagaaat ctgacgtcaa tgcaattatc cattatttaa aagctataaa aatagaacag 1320
gcatcattaa caagggataa aagtatcaat tctttgaaga aattggtttt aaggaaactt 1380
cggagaaagg cattagatct ggaaagcttg agcctccttg ggttcgtcta caaattggaa 1440
ggaaatatga atgaagccct ggagtactat gagcgggccc tgagactggc tgctgacttt 1500
gagaactctg tgagacaagg tccttaggca cccagatatc agccactttc acatttcatt 1560
tcattttatg ctaacattta ctaatcatct tttctgctta ctgttttcag aaacattata 1620
attcactgta atgatgtaat tcttgaataa taaatctgac aaaatattag ttgtgttcaa 1680
caattagtga aacagaatgt gtgtatgcat gtaagaaaga gaaatcattt gtatgagtgc 1740
tatgtagtag agaaaaaatg ttagttaact ttgtaggaaa taaaacattg gacttacact 1800
aaatgtttaa ttcattcatt ttattgtgaa ataaaaataa aatccttagc tcctccacca 1860
actgaacaga ccctcttggc caaggagacc ccagaaacct taaaaactaa gtttcccaac 1920
catgacaaga tgagagatca ttcacacctc attatattcc ctcccttgct aactgccatt 1980
ggactttttc cactgagtta aacagaaacc catggaaaac aaagaacaga agactcactc 2040
cttggctgac ttcacctagc tcactccacg tagcgccaca gccagactcc cctcccctct 2100
tgcggtttcc acatgacaac tgatcagcct tccctcctga taagtgacca ctgcccacag 2160
actggttctg gccagtccat ggaggctgca cacagggtgc ctctatgtcc tttgtttcac 2220
cttttgatat agaaaggcta attttgctgt attttaatgt taagtctcca ccacagagtg 2280
aacacagaat gcatgtgaca tacatgttta cataccacta ttgtgtgact gcccctcatg 2340
aatattcata gccccccata acctgttaac tatgtgtgtc tagccaatcc accaaccata 2400
aaacttctgt aataccctcc cttcctccaa gagcctgctt ttggttgctg tggtaggctc 2460
tgcttcccag gctgcaggtt gcaggagagg aggctgcagt ggctcacgcc tgtaatctca 2520
gcacttcgat gggacgaggc aggcagatca cctgaaccca ggagttcgag agcagccttg 2580
gcaatggcaa aaccaaccgt ctctacaaaa aatgcaaaaa cttagctggg tgtggtggca 2640

tgcacctgta gcttcagttc cagctactca ggaggctgag gtgagtggac tgctggagcc 2700

agggagttcg aggctgcagt gtcgagatct tgccactgca ctccattctg gatgatagaa 2760

cgagacccca tctcaaaaaa aaaaaaagtt ctctccaatt gtatatagct tgtgatttta 2820

tgtcaacact atcaataaat agctttcagt gcaagaaacc aaaaatactg taataaacag 2880

gcacatattc ttcccaaacc tcatgcagtt tacaatctag tgagagacac agatagcagt 2940

acagagtcaa ttaaaggtta gttttcttca tgaagatgtt ttaattttaa ttcaatgtga 3000

aagggttcca aggagtttat cttgttttat gccattttat ttgaagcact acttactaag 3060

tcatttgctg atattaatct agttaaatca agaaatatta catgaaaatg ttgctaaatc 3120

agagatcatg ggtaacaatc acctttgatt atgaataatc atattttatt gaaaggcaag 3180

gcacaacaaa taataagaag gaaaaaataa ataagcaatg ttattgatct ttcattctgt 3240

atatgttttg gggggaatat actagtttct tttagtggct gtaacaaatt accacaaact 3300

tggtgactta aaatttcaca gatttactct ttcttacagt tctggaggtc agaagtctga 3360

aatgggtttc aatgagccaa agtcaaggta ttgatgacgc tacactcctc cggaggctct 3420

aggcagatag ccttttccag cttccagagg ctgcctgaat tctttcatcc atcttaaaaa 3480

ccaacagtgt agtagcctca aatctctctc tctgcttcct tcttcacatc tccttctctc 3540

ctctgactct tttgcctctt tcttctaagg acgcaccagg tccacctgca taatccagaa 3600

taattgcccc atccgcaaat ccttaattta ataacatctg caaagtccct tttgctatgt 3660

aaagtagcat gttcacaggt tctggagact tggccatgga tacgattgcg gggggggcat 3720

tattcttacc acagagcacc ccaagaaaat ctccaaattt tgggcttcca atccattttg 3780

cttcaattat ttaatatttt tactccttcc agtagatact gatttcatcc attgccctta 3840

agaaggtagg acagagatta tggcacatct cacattaaat gctatatttt cgttggaaat 3900

acattttttg cttcaacttt tattttaaat tcaagggtac atgtgcagga tgttcaggtt 3960

tgttacacag gtaaacgtgt gccatggcgg tttgctgaac agatcatccc atcaccaaca 4020

gatcatccca ttgagaggtg aagccggctg ggcttctggg ttgggtgggg acttggagaa 4080

cttttctgtc tagctaaagt attgtaaaat ggaccagtca acactctgta aaatggacca 4140

atcagctctc tgtaaaatgg accaatcagc aggatgtggg tggggccaag taagggaata 4200

aaagcaggcc acccgagctg gcagcggcaa cccgctcggg tccccttcca tgctgtggaa 4260

gttttgttct ttcgctcttt caataaatct tgctgctgct ca 4302

<210> 49
<211> 447
<212> PRT
<213> Homo sapiens

<400> 49

Met Pro Asp Leu Glu Asn Arg Val Leu Asp Gln Ile Glu Phe Leu Asp
1               5                   10                  15

Thr Lys Tyr Ser Val Gly Ile His Asn Leu Leu Ala Tyr Val Lys His
            20                  25                  30

Leu Lys Gly Gln Asn Glu Glu Ala Leu Lys Ser Leu Lys Glu Ala Glu
        35                  40                  45

Asn Leu Met Gln Glu Glu His Asp Asn Gln Ala Asn Val Arg Ser Leu
    50                  55                  60

Val Thr Trp Gly Asn Phe Ala Trp Met Tyr Tyr His Met Gly Arg Leu
65                  70                  75                  80

Ala Glu Ala Gln Thr Tyr Leu Asp Lys Val Glu Asn Ile Cys Lys Lys
                85                  90                  95

Leu Ser Asn Pro Phe Arg Tyr Arg Met Glu Cys Pro Glu Ile Asp Cys
            100                 105                 110

Glu Glu Gly Trp Ala Leu Leu Lys Cys Gly Gly Lys Asn Tyr Glu Arg
        115                 120                 125

Ala Lys Ala Cys Phe Glu Lys Val Leu Glu Val Asp Pro Glu Asn Pro
    130                 135                 140

Glu Ser Ser Ala Gly Tyr Ala Ile Ser Ala Tyr Arg Leu Asp Gly Phe
145                 150                 155                 160

Lys Leu Ala Thr Lys Asn His Lys Pro Phe Ser Leu Leu Pro Leu Arg
                165                 170                 175

Gln Ala Val Arg Leu Asn Pro Asp Asn Gly Tyr Ile Lys Val Leu Leu
            180                 185                 190

Ala Leu Lys Leu Gln Asp Glu Gly Gln Glu Ala Glu Gly Glu Lys Tyr
        195                 200                 205

Ile Glu Glu Ala Leu Ala Asn Met Ser Ser Gln Thr Tyr Val Phe Arg
    210                 215                 220

Tyr Ala Ala Lys Phe Tyr Arg Arg Lys Gly Ser Val Asp Lys Ala Leu
225                 230                 235                 240

Glu Leu Leu Lys Lys Ala Leu Gln Glu Thr Pro Thr Ser Val Leu Leu
                245                 250                 255

His His Gln Ile Gly Leu Cys Tyr Lys Ala Gln Met Ile Gln Ile Lys
260 265 270

Glu Ala Thr Lys Gly Gln Pro Arg Gly Gln Asn Arg Glu Lys Leu Asp
275 280 285

Lys Met Ile Arg Ser Ala Ile Phe His Phe Glu Ser Ala Val Glu Lys
290 295 300

Lys Pro Thr Phe Glu Val Ala His Leu Asp Leu Ala Arg Met Tyr Ile
305 310 315 320

Glu Ala Gly Asn His Arg Lys Ala Glu Glu Asn Phe Gln Lys Leu Leu
325 330 335

Cys Met Lys Pro Val Val Glu Glu Thr Met Gln Asp Ile His Phe His
340 345 350

Tyr Gly Arg Phe Gln Glu Phe Gln Lys Lys Ser Asp Val Asn Ala Ile
355 360 365

Ile His Tyr Leu Lys Ala Ile Lys Ile Glu Gln Ala Ser Leu Thr Arg
370 375 380

Asp Lys Ser Ile Asn Ser Leu Lys Lys Leu Val Leu Arg Lys Leu Arg
385 390 395 400

Arg Lys Ala Leu Asp Leu Glu Ser Leu Ser Leu Leu Gly Phe Val Tyr
405 410 415

Lys Leu Glu Gly Asn Met Asn Glu Ala Leu Glu Tyr Tyr Glu Arg Ala
420 425 430

Leu Arg Leu Ala Ala Asp Phe Glu Asn Ser Val Arg Gln Gly Pro
435 440 445

<210> 50
<211> 478
<212> PRT
<213> Homo sapiens

<400> 50

Met Ser Thr Asn Gly Asp Asp His Gln Val Lys Asp Ser Leu Glu Gln
1 5 10 15

Leu Arg Cys His Phe Thr Trp Glu Leu Ser Ile Asp Asp Asp Glu Met
20 25 30

Pro Asp Leu Glu Asn Arg Val Leu Asp Gln Ile Glu Phe Leu Asp Thr
35 40 45

Lys Tyr Ser Val Gly Ile His Asn Leu Leu Ala Tyr Val Lys His Leu
50 55 60

Lys Gly Gln Asn Glu Glu Ala Leu Lys Ser Leu Lys Glu Ala Glu Asn
65 70 75 80

Leu Met Gln Glu Glu His Asp Asn Gln Ala Asn Val Arg Ser Leu Val
85 90 95

Thr Trp Gly Asn Phe Ala Trp Met Tyr Tyr His Met Gly Arg Leu Ala
100 105 110

Glu Ala Gln Thr Tyr Leu Asp Lys Val Glu Asn Ile Cys Lys Lys Leu
115 120 125

Ser Asn Pro Phe Arg Tyr Arg Met Glu Cys Pro Glu Ile Asp Cys Glu
130 135 140

Glu Gly Trp Ala Leu Leu Lys Cys Gly Gly Lys Asn Tyr Glu Arg Ala
145 150 155 160

Lys Ala Cys Phe Glu Lys Val Leu Glu Val Asp Pro Glu Asn Pro Glu
165 170 175

Ser Ser Ala Gly Tyr Ala Ile Ser Ala Tyr Arg Leu Asp Gly Phe Lys
180 185 190

Leu Ala Thr Lys Asn His Lys Pro Phe Ser Leu Leu Pro Leu Arg Gln
195 200 205

Ala Val Arg Leu Asn Pro Asp Asn Gly Tyr Ile Lys Val Leu Leu Ala
210 215 220

Leu Lys Leu Gln Asp Glu Gly Gln Glu Ala Glu Gly Glu Lys Tyr Ile
225 230 235 240

Glu Glu Ala Leu Ala Asn Met Ser Ser Gln Thr Tyr Val Phe Arg Tyr
245 250 255

Ala Ala Lys Phe Tyr Arg Arg Lys Gly Ser Val Asp Lys Ala Leu Glu
260 265 270

Leu Leu Lys Lys Ala Leu Gln Glu Thr Pro Thr Ser Val Leu Leu His

275 280 285

His Gln Ile Gly Leu Cys Tyr Lys Ala Gln Met Ile Gln Ile Lys Glu
290 295 300

Ala Thr Lys Gly Gln Pro Arg Gly Gln Asn Arg Glu Lys Leu Asp Lys
305 310 315 320

Met Ile Arg Ser Ala Ile Phe His Phe Glu Ser Ala Val Glu Lys Lys
325 330 335

Pro Thr Phe Glu Val Ala His Leu Asp Leu Ala Arg Met Tyr Ile Glu
340 345 350

Ala Gly Asn His Arg Lys Ala Glu Glu Asn Phe Gln Lys Leu Leu Cys
355 360 365

Met Lys Pro Val Val Glu Glu Thr Met Gln Asp Ile His Phe His Tyr
370 375 380

Gly Arg Phe Gln Glu Phe Gln Lys Lys Ser Asp Val Asn Ala Ile Ile
385 390 395 400

His Tyr Leu Lys Ala Ile Lys Ile Glu Gln Ala Ser Leu Thr Arg Asp
405 410 415

Lys Ser Ile Asn Ser Leu Lys Lys Leu Val Leu Arg Lys Leu Arg Arg
420 425 430

Lys Ala Leu Asp Leu Glu Ser Leu Ser Leu Leu Gly Phe Val Tyr Lys
435 440 445

Leu Glu Gly Asn Met Asn Glu Ala Leu Glu Tyr Tyr Glu Arg Ala Leu
450 455 460

Arg Leu Ala Ala Asp Phe Glu Asn Ser Val Arg Gln Gly Pro
465 470 475

<210> 51
<211> 2407
<212> DNA
<213> Homo sapiens

<400> 51
gtggaaacct cttcagcatt tgcttggaat cagtaagcta aaaacaaaat caaccgggac 60

cccagctttt cagaactgca gggaaacagc catcatgagt gaggtcacca agaattccct 120

ggagaaaatc cttccacagc tgaaatgcca tttcacctgg aacttattca aggaagacag 180

tgtctcaagg gatctagaag atagagtgtg taaccagatt gaatttttaa acactgagtt 240

caaagctaca atgtacaact tgttggccta cataaaacac ctagatggta acaacgaggc 300

agccctggaa tgcttacggc aagctgaaga gttaatccag caagaacatg ctgaccaagc 360

agaaatcaga agtctagtca cttggggaaa ctacgcctgg gtctactatc acttgggcag 420

actctcagat gctcagattt atgtagataa ggtgaaacaa acctgcaaga aattttcaaa 480

tccatacagt attgagtatt ctgaacttga ctgtgaggaa gggtggacac aactgaagtg 540

tggaagaaat gaaagggcga aggtgtgttt tgagaaggct ctggaagaaa agcccaacaa 600

cccagaattc tcctctggac tggcaattgc gatgtaccat ctggataatc acccagagaa 660

acagttctct actgatgttt tgaagcaggc cattgagctg agtcctgata accaatacgt 720

caaggttctc ttgggcctga aactgcagaa gatgaataaa gaagctgaag gagagcagtt 780

tgttgaagaa gccttggaaa agtctccttg ccaaacagat gtcctccgca gtgcagccaa 840

attttacaga agaaaaggtg acctagacaa agctattgaa ctgtttcaac gggtgttgga 900

atccacacca aacaatggct acctctatca ccagattggg tgctgctaca aggcaaaagt 960

aagacaaatg cagaatacag gagaatctga agctagtgga aataaagaga tgattgaagc 1020

actaaagcaa tatgctatgg actattcgaa taaagctctt gagaagggac tgaatcctct 1080

gaatgcatac tccgatctcg ctgagttcct ggagacggaa tgttatcaga caccattcaa 1140

taaggaagtc cctgatgctg aaaagcaaca atcccatcag cgctactgca accttcagaa 1200

atataatggg aagtctgaag acactgctgt gcaacatggt ttagagggtt tgtccataag 1260

caaaaaatca actgacaagg aagagatcaa agaccaacca cagaatgtat ctgaaaatct 1320

gcttccacaa aatgcaccaa attattggta tcttcaagga ttaattcata agcagaatgg 1380

agatctgctg caagcagcca aatgttatga gaaggaactg ggccgcctgc taagggatgc 1440

cccttcaggc ataggcagta ttttcctgtc agcatctgag cttgaggatg gtagtgagga 1500

aatgggccag ggcgcagtca gctccagtcc cagagagctc ctctctaact cagagcaact 1560

gaactgagac agaggaggaa aacagagcat cagaagcctg cagtggtggt tgtgacgggt 1620

aggacgatag gaagacaggg ggccccaacc tgggattgct gagcagggaa gctttgcatg 1680

ttgctctaag gtacattttt aaagagttgt tttttggccg ggcgcagtgg ctcatgcctg 1740

taatcccagc actttgggag gccgaggtgg gcggatcacg aggtctggag tttgagacca 1800

tcctggctaa cacagtgaaa tcccgtctct actaaaaata caaaaaatta gccaggcgtg 1860

gtggctggca cctgtagtcc cagctacttg ggaggctgag gcaggagaat ggcgtgaacc 1920

tggaaggaag aggttgcagt gagccaagat tgcgcccctg cactccagcc tgggcaacag 1980

agcaagactc catctcaaaa aaaaaaaaaa aaaaaaaaaa gagttgtttt ctcatgttca 2040

ttatagttca ttacagttac atagtccgaa ggtcttacaa ctaatcactg gtagcaataa 2100

atgcttcagg cccacatgat gctgattagt tctcagtttt cattcagttc acaatataac 2160

caccattcct gccctccctg ccaagggtca taaatggtga ctgcctaaca acaaaatttg 2220

cagtctcatc tcattttcat ccagacttct ggaactcaaa gattaacttt tgactaaccc 2280

tggaatatct cttatctcac ttatagcttc aggcatgtat ttatatgtat tcttgatagc 2340

aataccataa tcaatgtgta ttcctgatag taatgctaca ataaatccaa acatttcaac 2400

tctgtta 2407

<210> 52
<211> 490
<212> PRT
<213> Homo sapiens

<400> 52

Met Ser Glu Val Thr Lys Asn Ser Leu Glu Lys Ile Leu Pro Gln Leu
1 5 10 15

Lys Cys His Phe Thr Trp Asn Leu Phe Lys Glu Asp Ser Val Ser Arg
20 25 30

Asp Leu Glu Asp Arg Val Cys Asn Gln Ile Glu Phe Leu Asn Thr Glu
35 40 45

Phe Lys Ala Thr Met Tyr Asn Leu Leu Ala Tyr Ile Lys His Leu Asp
50 55 60

Gly Asn Asn Glu Ala Ala Leu Glu Cys Leu Arg Gln Ala Glu Glu Leu
65 70 75 80

Ile Gln Gln Glu His Ala Asp Gln Ala Glu Ile Arg Ser Leu Val Thr
85 90 95

Trp Gly Asn Tyr Ala Trp Val Tyr Tyr His Leu Gly Arg Leu Ser Asp
100 105 110

Ala Gln Ile Tyr Val Asp Lys Val Lys Gln Thr Cys Lys Lys Phe Ser
115 120 125

Asn Pro Tyr Ser Ile Glu Tyr Ser Glu Leu Asp Cys Glu Glu Gly Trp
130 135 140

Thr Gln Leu Lys Cys Gly Arg Asn Glu Arg Ala Lys Val Cys Phe Glu
145 150 155 160

Lys Ala Leu Glu Glu Lys Pro Asn Asn Pro Glu Phe Ser Ser Gly Leu
165 170 175

Ala Ile Ala Met Tyr His Leu Asp Asn His Pro Glu Lys Gln Phe Ser
180 185 190

Thr Asp Val Leu Lys Gln Ala Ile Glu Leu Ser Pro Asp Asn Gln Tyr
195 200 205

Val Lys Val Leu Leu Gly Leu Lys Leu Gln Lys Met Asn Lys Glu Ala
210 215 220

Glu Gly Glu Gln Phe Val Glu Glu Ala Leu Glu Lys Ser Pro Cys Gln
225 230 235 240

Thr Asp Val Leu Arg Ser Ala Ala Lys Phe Tyr Arg Arg Lys Gly Asp
245 250 255

Leu Asp Lys Ala Ile Glu Leu Phe Gln Arg Val Leu Glu Ser Thr Pro
260 265 270

Asn Asn Gly Tyr Leu Tyr His Gln Ile Gly Cys Cys Tyr Lys Ala Lys
275 280 285

Val Arg Gln Met Gln Asn Thr Gly Glu Ser Glu Ala Ser Gly Asn Lys
290 295 300

Glu Met Ile Glu Ala Leu Lys Gln Tyr Ala Met Asp Tyr Ser Asn Lys
305 310 315 320

Ala Leu Glu Lys Gly Leu Asn Pro Leu Asn Ala Tyr Ser Asp Leu Ala
325 330 335

Glu Phe Leu Glu Thr Glu Cys Tyr Gln Thr Pro Phe Asn Lys Glu Val
340 345 350

Pro Asp Ala Glu Lys Gln Gln Ser His Gln Arg Tyr Cys Asn Leu Gln
355 360 365

Lys Tyr Asn Gly Lys Ser Glu Asp Thr Ala Val Gln His Gly Leu Glu
370 375 380

Gly Leu Ser Ile Ser Lys Lys Ser Thr Asp Lys Glu Glu Ile Lys Asp
385 390 395 400

Gln Pro Gln Asn Val Ser Glu Asn Leu Leu Pro Gln Asn Ala Pro Asn
405 410 415

Tyr Trp Tyr Leu Gln Gly Leu Ile His Lys Gln Asn Gly Asp Leu Leu

420 425 430

Gln Ala Ala Lys Cys Tyr Glu Lys Glu Leu Gly Arg Leu Leu Arg Asp
435 440 445

Ala Pro Ser Gly Ile Gly Ser Ile Phe Leu Ser Ala Ser Glu Leu Glu
450 455 460

Asp Gly Ser Glu Glu Met Gly Gln Gly Ala Val Ser Ser Ser Pro Arg
465 470 475 480

Glu Leu Leu Ser Asn Ser Glu Gln Leu Asn
485 490

<210> 53
<211> 12379
<212> DNA
<213> Homo sapiens

<400> 53
gacccgagag ccgctgagcc gcgaggccgg gccggggcgc cggccgggaa tgccgggggc 60

gcggcgccga cgccgaggcg cggccatgga ggggaagccc cgcgccgggg tcgcgctggc 120

cccggggccg agcggccgac ggccttccgc ccgctgcgcc cgccgccgcc gcccggggct 180

gctgcttcct ggcctctggc tgctgctgct ggcccggccg gcctcgtgcg ccccagatga 240

gctctctccg gaacagcaca acctttcttt atactccatg gagctcgtgc tgaagaaaag 300

cactgggcac agcgctgcac aagtggcctt aacagaaact gctcccggct cccagcacag 360

cagtcctctc catgtcacag ccccgccgtc tgccactact tttgatacag cctttttaa 420

ccaaggaaaa cagaccaaaa gtacagcaga tcccagcatc tttgtggcaa cttacgtgtc 480

agtgacgagt aaagaggtgg ccgtcaatga cgatgagatg gataactttc tgccagatac 540

tcactggacc actccacgga tggtttctcc aatacagtat atcacagtca gcccaccagg 600

gctgcccagg gaagcattag aacctatgct cactccatca ttacccatgg tttctttaca 660

agatgaagaa gtgacatcgg gctggcagaa cacaacgcga caaccagcgg catatgctga 720

gtccgccagt catttccaca cctttcggtc agcttttcgc acctctgagg gcatcgttcc 780

aactcctggc aggaatttgg tgctttatcc tactgatgct tacagtcatt tatcaagcag 840

gactctgcca gagattgtgg cttccctaac agagggtgtg gaaaccaccc ttttttaag 900

ctcccggtct ttaatgccac agccgttagg cgacggcatt actataccgt tgccctcctt 960

gggggaggtc tcacagcctc cagaggaggt ttgggccaca agtgcagaca gatacactga 1020

tgtgaccact gtgttgagtc aaagcctaga agaaaccatc tctccaagaa cataccccac 1080

tgtgactgca tcgcacgcag cccttgcatt cagcaggaca cattctccat tgctttcaac 1140

tcctcttgca tttgcgtcct ctgcttcacc aactgatgtt tcatctaacc cctttctccc 1200

tagcgactcc agcaaaacat ccgaattgca tagcaattca gccctccccg gtcctgtgga 1260

caacactcat atcctgagcc cggtgtcctc attcagacca tacacttggt gtgcggcctg 1320

cactgtgcct tcacctcagc aagttctggc cacgagcctc atggagaaag acgtgggatc 1380

aggggatggt gccgagactc tgtgcatgac cgtgctggaa gaaagcagca tctctctaat 1440

gagtagcgtc gtagcagact tctctgaatt tgaggaagat cctcaagtat ttaatacgct 1500

tttcccctcc agacctatcg tcccactttc ttctagatcc atggaaatct cagagacgag 1560

tgttggcatt tctgccgagg tggatatgag tagtgttaca accacacagg ttcccccctgc 1620

ccacggccgc ctctctgtgc cggcgtcact tgatcctact gctggctcct tgtctgttgc 1680

tgaaacccaa gtgacgccat ccagcgtgac cactgcattt ttctcggtca tcaccagcat 1740

tctccttgac tcatctttct ctgtcatagc aaacaaaaac acaccgtcgc ttgccgtcag 1800

agacccgagt gtttttacgc cttatagtct ggttccttca gtggagtctt cacttttctc 1860

tgaccaagaa cgttccagtt tttctgagca taaacccaga ggtgctttgg attttgcatc 1920

cagctttttc tcaacacccc cgctggaact cagcggctcc atctcttcgc cttcggaagc 1980

acctgcgtct ctgtctctga tgccgagtga cttgtccccc ttcacatctc agtctttttc 2040

tcccttggtt gagacattta cattgtttga ctctagtgat ctgcagtcat ctcagctgtc 2100

tcttcccagt tccacaaatc ttgagttttc gcagctccag ccaagttccg agctgccttt 2160

aaacaccatc atgttgctac ctagccgttc tgaggtgtca ccatggtcaa gcttcccttc 2220

tgattctctc gagtttgttg aagcgtctac ggtttcactg acggattcag aagctcattt 2280

tacctcagct ttcattgaaa ctacctccta tcttgagtct tcactcattt cccatgaatc 2340

cgcagtcact gcactggtgc cccccggctc tgagtctttt gacattttga ctgccgggat 2400

tcaagcaaca tcaccattga ccactgtcca cacaacgccc attttaactg agtcttcttt 2460

gttctcaact ctgacacctc ctgacgacca aatcagtgct ctagacggtc acgtgtctgt 2520

cctggcctct ttctccaaag ccattcccac tggtacggtg ttgatcactg acgcgtacct 2580

gccatcagga tcctcgtttg tttctgaagc aaccccccttc cctctgccca cagagctgac 2640

cgtcgtgggc ccatcactca cacccacaga ggtgccactg aacacctcca cggaagtgag 2700

cacaaccagc accggtgctg ccactggtgg tcccctcgac tccaccctga tgggtgacgc 2760

cgcaagtcag agccccccag agagtagtgc tgctcctccc ctgccatccc tgcgtcccgt 2820

gactgccttc actctcgaag caacagtcga cacaccaaca ctggctactg ccaagccgcc 2880

atatgtttgt gatatcacag tccccgatgc ctatctgatc acaactgtgc tggccagaag 2940

agctgtgcag gagtacatca ttacagcaat caaagaagta ctgaggattc acttcaaccg 3000

tgcagtggaa ctgaaggttt acgaactatt tactgacttc acttttctgg taacatccgg 3060

tcctttcgtt tacacggcaa tatccgtcat aaatgtgctt ataaacagta agcttgtccg 3120
tgaccagact cctttaatcc tgtctgtgaa accttctttc cttgtgccag agtccaggtt 3180
ccaagttcaa acagtacttc agtttgtgcc tccgagtgtg gatactggct tctgcaactt 3240
cacccagcgc attgagaaag gcctaatgac agctctcttt gaagtgagaa aacaccacca 3300
gggaacgtat aacctcacgg tgcagatctt gaatatcacc atcagttcct caagggtgac 3360
tcctcggcgg ggcccggtga atatcatctt tgcggttaaa agcacacagg gatttttgaa 3420
tgggtcggaa gtgagcgagc tgctcagaaa cttgagtgtg gtggagttca gtttctatct 3480
gggataccca gtgctgcaga tcgcagagcc cttccagtat ccacagctca acttatctca 3540
gttgctgaag tcctcttggg tcagaacagt tctcctgggc gtcatggaga agcaactcca 3600
gaatgaagtg tttcaagccg agatggaacg caagctggcc cagctgctca gcgaggtttc 3660
caccagaagg cggatgtgga gaagggccac tgtagctgca gggaacagtg tggtgcaggt 3720
ggtaaatgtg tcgaggctgg agggagatga caatccggta cagctcatct actttgtgga 3780
ggatcaagat ggagaaagac tcagtgcagt caagtcttcg gacctgatta acaaaatgga 3840
cctccagaga gcagccatca tcttgggtta ccgaattcaa ggtgtcattg cccagcctgt 3900
cgacagggtg aagaggccgt ctccggaatc ccagagcaac aacttgtggg tcattgttgg 3960
cgtggtcatc ccagtgctgg tggtgatggt gattgttgtc atcctctact ggaaactatg 4020
ccgcacagac aagctagact ttcagcctga cactgtggcc aacattcagc agcgtcagaa 4080
gctgcagatc cctagtgtga agggcttcga ttttgctaag cagcatctgg gtcagcacaa 4140
taaagacgac atattgatta ttcatgagcc agcgccactg ccaggacctc tgaaggacca 4200
caccacgccc tcggaaaatg gagacgtgcc aagccccaag tcaaagatcc cttccaagaa 4260
tgttcgtcac agaggaagag tttctccctc agatgctgac tctacggtca gtgaagagtc 4320
cagcgagagg gacgcaggag ataagacgcc gggagccgtc aacgatggca ggtcccacag 4380
agctccgcag agcgggccac cactgcccag ttcgggaaat gagcagcact catcagcctc 4440
catcttcgag cacgtggaca ggatctcccg ccccccggag gctagccggc gggtccccag 4500
taagatccag cttatcgcca tgcagccgat cccggcacct cccgtccagc gcccctcccc 4560
agccgaccga gtggcggaaa gcaataaaat caacaaagag attcagaccg cgctgcggca 4620
caagtctgag atcgagcacc atcgcaacaa gatccgcctg cgcgccaagc gccgcgggca 4680
ctacgagttc ccggtggtag acgacctgtc ctcgggcgac actaaggagc gacaccgggt 4740
gtaccgcagg gcacagatgc agatcgacaa gatcctggac cccacggcca gcgtgccctc 4800
cgtgttcata gagcccagga agagctcacg gataaaacgt tctcccaagc ctcgccggaa 4860
acaccaggtc aacggctgtc ctgccgacgc tgagaaggac cggctcatca ccacagacag 4920

cgatggcacc tacaggaggc cccccggcgt ccacaactca gcctacatcg gatgcccatc 4980
ggatcctgac ctcccagccg atgtgcagac accatcctcg gtggaactgg ggaggtatcc 5040
agcccttccc ttcccggcct cccagtacat cccaccccag ccgtccatcg aggaggcacg 5100
ccagaccatg cactccctcc tggacgacgc ctttgccctc gtggccccca gcagccagcc 5160
tgccagcacc gcaggtgtag gccccggagt cccacccggc ctgcccgcaa acagcacccc 5220
ttcccaggaa gagaggcgag ccacccagtg gggtccttc tacagcccag cccagacggc 5280
caacaatccc tgcagtagat acgaagacta tggaatgact cccccgacgg gtccattgcc 5340
aagaccaggt tttggccccg gtttgctgca gtctacagag ctggtgcccc ctgaccctca 5400
gcagccacag gcctccgccg aagccccatt tgctgccaga gggatctact cggaggagat 5460
gccgtcggtg gcccggcctc ggcctgtcgg gggtaccaca ggctcccaga tccagcacct 5520
gacacaggtg gggattgcca gcagaattgg agctcagcca gtggaaatcc cgccaagcag 5580
aggcagccag tatgggggc caggctggcc ttcgtacggg gaggacgaag cggggcgaag 5640
agaggccgtg ccaaggactt caggcaggga gccctcagct ccttccggga acctccccca 5700
ccggggactg cagggccctg ggctgggtta ccccaccagc tccacggaag acctccagcc 5760
tggccactcc tcggcctctc tcatcaaagc aatccgcgag gagctcctcc ggctctccca 5820
gaaacagagc accgtgcaga acttccacag ctgatcggcc tcgcctcgca gatttgccaa 5880
gtatccgctt cctgtggaag caagaccaaa aggaaatcaa ctgagtgggt gtttggaaga 5940
ggaaggagca actctcgggc agcctgccca agggagggag caagttgcaa tttagaagat 6000
gccatacgtc gtgtgacagc tcatgagcct ttcactgggc tggcaattgt ctgaacactt 6060
gggttcagtt gaaatatatg tattttggcc aaaagccagc agcacttcac aaaaacaaaa 6120
cacaaaccta agctaacaaa atgactgcat tcgtctcttt tttaaaggta gagattaaac 6180
tgtatagaca gcatagggat gaaaggaacc aagcgtttct gtgggattga gactggtacg 6240
tgtacgatga acctgctgct ttgttttctg agaagaggtt tgaagacatt ttattaacag 6300
cttaattttt ctcttttact ccataggaac ttattttaat agtaacatta acaacaagaa 6360
tactaagact gtttgggaat tttaaaaagc tactagtgag aaaccaaatg ataggttgta 6420
gagcctgatg actccaaaca aagccatcac ccgcattctt cctccttctt ctggtgctac 6480
agctccaagg gcccttcacc ttcatgtctg aaatggaact ttggcttttt cagtggaaga 6540
atatgttgaa ggtttcattt tgttctagaa aaaaaaaatc cctcccaaag tggggcaaaa 6600
agctttatat ttatttgatt atccaaaata cagatcaaag tttagatcta cattcttcat 6660
tgtatttgct gtttcttaat tgggcacaca caactcctgg tcatgtccca gttcagcacc 6720
gatcgctaag gccgatcctg tagaatgcgg ctttcaagag gtcctaactg atcctttctt 6780
ccactttgct gttgatttgt tcacaaatta tgtctgaatg agaaatcttc tgtaagcaga 6840

agttatttaa taattcccag caccacgaaa ttgttataca tacatcccta ccagtcacat 6900
tccctgtgtt cagagcctgg aaatgaaatt gagttcagtt cagcctctct gtaatgaatc 6960
aagaaatgta aaagagcttt gagaccccaa gggaaaggag agagttgctt ctcatttctg 7020
attttattgt gctgttactc tgtcgagtgg ctattaaaat tgtcttatgc tctttgggct 7080
aagaggggat catctcgctt aagatgtact cctgatgtaa acatttcccc cactttccaa 7140
ataatggtaa agaaaacagt ggcaagggct gttctgtttt ctggtacctg agtgaaactc 7200
agaagaaaag caggcttagc taagagattt tcactattaa cctgttttta ttagatcagc 7260
gaagacagtc atgcatcgct taatgatggg gatgcattct gagaaatgtg tcattaggct 7320
gttttgttgc tgtgccaaca tcctagattg tacttacaca acctacatgg tatagcctac 7380
tacacacctg ggctgtgtgg catggcctat tcctcttagg ctacaaacct gtacagcatg 7440
ttactgtact gaacacaatg gtaagtattt gtgtatctaa acatagctaa acatagaaaa 7500
ggtatggtaa aaatacaatt ttataatctt atgggactac catcaatatg tagaccgtca 7560
ttgaccaaaa catcctgatg tgtgcatgac tgtacttttc attaaataac agataagggg 7620
ctatagaatt tggggctctg actgatcaaa acctgtgtat ctgtgtcaag ttttaagacc 7680
cctgaaacag ctaaaacagg tttccaaagt gtaatcactg gatttcaaaa ccatgtttta 7740
gcccttcatt aatgaagcct gttgtacaga tttagagtct tacaatatga gggaagttgg 7800
ttctgggaag gtaagatgta gccagttttc atctgggcac ctctgaaaga gaaggagtgc 7860
aggagagtaa gtctctcaag gacgttcaac aaaggaccag cagcatctta tcaggcgatc 7920
ctcagaggct ccaaggagca ttggagagaa tgcccctttc tgggcttgat gtcttggttt 7980
tggtttaata ccaaatttct cttggtcttg gtttaatacc cgactcctcc caaattgaac 8040
gattatcttt gtatgtcact caaaaatacc aggtttcctg aatatgttat taagaatttc 8100
agatatccaa gcagaatttt attatggaca ccttgtaatg aattcaaatc gtgtgacagc 8160
aaacgggata aatggcccct tctcacccag ttccgctcag ggccatacag gcttgcacat 8220
agagtgtgct aaaaatggta acctcctttg agcattgcag aaagagggtt ctgtttcaaa 8280
ttgggctgac cgtaaaagca attttgatgt ctttcaaact accataaagg gattttaact 8340
gtatttttat tcttagaaac aatacacctt taaacagatg tcaagaccaa agatgatgta 8400
taataaacag ccggtggtta tggtgtggtg tgagcaggcc cccggatcac cagccctctg 8460
ctggtggtca taaagcacac acagtttgta cttgcttcct ctcgacgcct gccacaacag 8520
ttttgccagt caccaaactc agaaaaaact agctgtgtga gcagcaccgt accctagcgg 8580
tcttcagaca ttaagttcac gtcatccact gaaaggaagg gtccttgcgt tgacagcgtg 8640
tggctttgga gtccgtttat ggaagcactc tacaatgaac agttgaattt tatgtctatt 8700

ctttttttcct attttaaaag ttctagtggt aacacaaact gtaaatttga gtcagaatgt 8760
ttggagaaat gttgtttttt tttttttcag agactacttt gtcactcact gaccatgtct 8820
ggttccttct ctgaacttca ttctccccat aagccaaaca agaacagacc tcccccgcca 8880
cctcccaggc accgtagttg tgagaatcag atgtgatcat gtgtgcagat gtcctgtgag 8940
gagggagcat aaagcactgt gaaaatgtag catgctgtct atgtggacgc cctaggatga 9000
gtgatgtgaa acgctgcact actgcggtga atgggttcac acatgggtaa tgagcaaaac 9060
cgaaagctcg gtgtgactca gtttcctcac tcccattttg ctttaatttc acttccttca 9120
ccaattttcc gattgcattt attaagcatc tttctgctcc cgactttctg gtgtctctgg 9180
caccaaataa cccagcagac atgagccttg ccttctgaga ttttagaatc taagatagca 9240
cgagtgggta tagaagatgg aagataccga taaataacat ggtttaagtg tcaataaaat 9300
tcattcgaag agatcaggcg cggtggctca cgcctgtaat cccagcattt tgggaggctg 9360
aggcaggtgg atcacttgag atcaagagct taaaaccagc ccaggcaaca tggcaaaacc 9420
ccgtctctac aaaaaaatac aaaaactagc tgggtgtggt ggcgcatgcc tgtagtccca 9480
gctgaggcac gataattgct tgaacccggg gggtggaggt tgcagtgagc tgagattgca 9540
ccactgtact ccaacctggg cgacagagca agactgtgtc tcaaaaagaa aaaaatttgt 9600
tcaaaagaca taaatgaatt aggcacccag aagaagttgc atgtttggaa atgcaatatc 9660
ttggggagac gtcaacttct gaagtgactt tgaaggcagg gcagctaccc aggcaaatgg 9720
cgtggaagga agagctgaga gtgggcctgg gaaaggctgt agggaaggag tgaagctgtc 9780
actgaaatga acttgatctt gatctgcttt gatatgggga cagaatcccc gactgcacta 9840
ttgagggagt ttcagaagag aaggaagagg tgctggaagg atgcttttgc agtcatgcag 9900
caaggaaacg accagggctg tgaagatcta cagaggaagt actccagtgt gggggaggca 9960
agggaagagg acgaaaatga tgagaatgac actgaggtca ttgtttagag cagatctcaa 10020
gcagccgttt ggccacgcca tatcctttgt ggagcatagt gggttatcta tctgtctgtc 10080
tgtctatgta tctgtctatc tatctatata tatattcagc ttctttactg ctacctacac 10140
atttttctcc aaattttatt aatttcatag tgttttgatt tgggtggcag atgactttta 10200
agcagtgggt gtttgccggc cagatctttc ctggcatgcg gactgtgagg caaagcacgg 10260
gtgaaggtag gcgctaaagg ctttgggcta aagccagcac gcggttctgt gctataggag 10320
tctcccgttt cccgtggaca ggttcagcgt tccttctttc gcacaacttt tttctaagtg 10380
ttccagtgac caagccagtc attcggacac tgatttgcag tgcattggca gtaattcaca 10440
aattagttgt tatataagtc tctctcatcc ctttcacact agattctcag acatgaatga 10500
atttgtcgtt tggaaggaaa gctgggtaac gttttgggca caggggaagg aggactccgg 10560
tcttaactcc cacgctaact ttagctcaag tggagttttc accgtggtca tttctacctc 10620

cggagcaagg tgccagcgcc agtactagag cctgcttatc cacatttgcc ctggacagga 10680

gcaggaggaa gtccacttct gtaccggcag acagagcatg tgaacacaaa acacatttct 10740

atggcatagt caactgaact tcatttttac atttaatcta acatgttaac acgttctaac 10800

agggtttcta tgagcagctg ctgtaacata ctcatcaact atgatagact taacacttgt 10860

tacctaatga acaaggagga tgtgcatttc gggtttcttt tgatattcgt ggaggtgatt 10920

gtcagtgttt aaacaggact actttctcca ctatgaagat gacttggaaa tcgcaacatc 10980

atggtacatt gctaagtcca tgcttgtgtg tgtgacagta ggcttgataa tttatcttaa 11040

aacacagcag cattgaaatt taggaaaaga atattaaatg cctttggaaa catgaaacaa 11100

agttaggagc cagtaagaaa gtgacagaag caatgaatgt cttaatgcag tatagttaaa 11160

atggcttccc acagggtaga taattatcca ggatccttcc ttttccttct tcctccaggt 11220

ttttcagggg tcacttcaca tttctaaaga aagagtgaat aggctgggca tggtggctca 11280

agcctctaat ctcaacgctt tgggaggctg aggcaggagg atcgcttgag gccaggagtt 11340

tgagaccacc ttgggcaaca taacgagacc ccgtctctac aaaaaaaatt taaaaattag 11400

ctgagcatgg tggcatgtgc cagtagtccc agctactcga aaggctaaga ctggaggatc 11460

gcttgagcca atgagttgga ggctgcagtg agctataatc acgccactgc actccagcct 11520

gggctgcagg gtgaggtcct gtctctggaa aaaaaaaaaa aaaaaaaagg aataggtaaa 11580

agggacagag gtgaaatttt gagtgacttg agtctcttgc agtccctgat tacacagaac 11640

ctttctgggc tacttggagc atcacgaata gtctttcctg tacttaccag atttcaagta 11700

ttcataactt gactccctaa gtgtacaagt tgggaatagt acagggccaa gttcaagtcg 11760

catatgctgt actgttcctc ctgcaaatgt ggggaaagaa gagggagata ctagaggaac 11820

tgaggctcca cccattcatt cagttgctct aagcaccaga ggacttgttt cagaaaaggg 11880

gagtgggaac gccctcgact ttgccctcct ccggagcatc tctgggacgc agggagtctg 11940

gctagcgtta ataggaaagg ttgctcggca gagctgccct ggagtactga cttgtctctc 12000

cctcctttgt caaggtccat gtttttctgg ctcttcctgc acactcatcc ctagattatg 12060

agcgttaatg tacgaaatgt gcagagcaaa ttgaggccaa ctgtggcatc aatactgcaa 12120

cttaaacttg acaatcatgg tttgctggtc ctcaaacagg cactgaaatc tcagtatggg 12180

tatacgattt aataatcggc ccctcccttc tatttgtcgg ttttatgttt ctatccttca 12240

atagctgcac tgttttctaa tgtgctgtag agtttttgaa ataatttatg caagtatttg 12300

gtttccatgt ttacaattta tacagctttc ctagcatttt aaatggaaat gtcaataaat 12360

gctatgaatt ggtgtcaaa 12379

<210> 54

<211> 12498
<212> DNA
<213> Homo sapiens

<400> 54
ctctccggcc tccctccgcg ccggtgcgcg gcgccccgtc ccagccgcag ccgcagcggc 60

cgcccctccc ggacccgaga gccgctgagc cgcgaggccg ggccggggcg ccggccggga 120

atgccggggg cgcggcgccg acgccgaggc gcggccatgg aggggaagcc ccgcgccggg 180

gtcgcgctgg ccccggggcc gagcggccga cggccttccg cccgctgcgc ccgccgccgc 240

cgcccggggc tgctgcttcc tggcctctgg ctgctgctgc tggcccggcc ggcctcgtgc 300

gccccagatg agctctctcc ggaacagcac aacctttctt tatactccat ggagctcgtg 360

ctgaagaaaa gcactgggca cagcgctgca caagtggcct taacagaaac tgctcccggc 420

tcccagcaca gcagtcctct ccatgtcaca gccccgccgt ctgccactac ttttgataca 480

gccttttttta accaaggaaa acagaccaaa agtacagcag atcccagcat ctttgtggca 540

acttacgtgt cagtgacgag taaagaggtg gccgtcaatg acgatgagat ggataacttt 600

ctgccagata ctcactggac cactccacgg atggtttctc caatacagta tatcacagtc 660

agcccaccag ggctgcccag ggaagcatta gaacctatgc tcactccatc attacccatg 720

gtttctttac aagatgaaga agtgacatcg ggctggcaga acacaacgcg acaaccagcg 780

gcatatgctg agtccgccag tcatttccac acctttcggt cagcttttcg cacctctgag 840

ggcatcgttc caactcctgg caggaatttg gtgctttatc ctactgatgc ttacagtcat 900

ttatcaagca ggactctgcc agagattgtg gcttccctaa cagagggtgt ggaaaccacc 960

cttttttttaa gctcccggtc tttaatgcca cagccgttag gcgacggcat tactataccg 1020

ttgccctcct tgggggaggt ctcacagcct ccagaggagg tttgggccac aagtgcagac 1080

agatacactg atgtgaccac tgtgttgagt caaagcctag aagaaaccat ctctccaaga 1140

acataccccca ctgtgactgc atcgcacgca gcccttgcat tcagcaggac acattctcca 1200

ttgctttcaa ctcctcttgc atttgcgtcc tctgcttcac caactgatgt ttcatctaac 1260

ccctttctcc ctagcgactc cagcaaaaca tccgaattgc atagcaattc agccctcccc 1320

ggtcctgtgg acaacactca tatcctgagc ccggtgtcct cattcagacc atacacttgg 1380

tgtgcggcct gcactgtgcc ttcacctcag caagttctgg ccacgagcct catggagaaa 1440

gacgtgggat caggggatgg tgccgagact ctgtgcatga ccgtgctgga agaaagcagc 1500

atctctctaa tgagtagcgt cgtagcagac ttctctgaat ttgaggaaga tcctcaagta 1560

tttaatacgc ttttcccctc cagacctatc gtcccacttt cttctagatc catggaaatc 1620

tcagagacga gtgttggcat ttctgccgag gtggatatga gtagtgttac aaccacacag 1680

gttccccctg cccacggccg cctctctgtg ccggcgtcac ttgatcctac tgctggctcc 1740

ttgtctgttg ctgaaaccca agtgacgcca tccagcgtga ccactgcatt tttctcggtc 1800
atcaccagca ttctccttga ctcatctttc tctgtcatag caaacaaaaa cacaccgtcg 1860
cttgccgtca gagacccgag tgtttttacg ccttatagtc tggttccttc agtggagtct 1920
tcacttttct ctgaccaaga acgttccagt ttttctgagc ataaacccag aggtgctttg 1980
gattttgcat ccagcttttt ctcaacaccc ccgctggaac tcagcggctc catctcttcg 2040
ccttcggaag cacctgcgtc tctgtctctg atgccgagtg acttgtcccc cttcacatct 2100
cagtcttttt ctcccttggt tgagacattt acattgtttg actctagtga tctgcagtca 2160
tctcagctgt ctcttcccag ttccacaaat cttgagtttt cgcagctcca gccaagttcc 2220
gagctgcctt taaacaccat catgttgcta cctagccgtt ctgaggtgtc accatggtca 2280
agcttccctt ctgattctct cgagtttgtt gaagcgtcta cggtttcact gacggattca 2340
gaagctcatt ttacctcagc tttcattgaa actacctcct atcttgagtc ttcactcatt 2400
tcccatgaat ccgcagtcac tgcactggtg ccccccggct ctgagtcttt tgacattttg 2460
actgccggga ttcaagcaac atcaccattg accactgtcc acacaacgcc cattttaact 2520
gagtcttctt tgttctcaac tctgacacct cctgacgacc aaatcagtgc tctagacggt 2580
cacgtgtctg tcctggcctc tttctccaaa gccattccca ctggtacggt gttgatcact 2640
gacgcgtacc tgccatcagg atcctcgttt gtttctgaag caaccccctt ccctctgccc 2700
acagagctga ccgtcgtggg cccatcactc acacccacag aggtgccact gaacacctcc 2760
acggaagtga gcacaaccag caccggtgct gccactggtg gtcccctcga ctccaccctg 2820
atgggtgacg ccgcaagtca gagcccccca gagagtagtg ctgctcctcc cctgccatcc 2880
ctgcgtcccg tgactgcctt cactctcgaa gcaacagtcg acacaccaac actggctact 2940
gccaagccgc catatgtttg tgatatcaca gtccccgatg cctatctgat cacaactgtg 3000
ctggccagaa gagctgtgca ggagtacatc attacagcaa tcaaagaagt actgaggatt 3060
cacttcaacc gtgcagtgga actgaaggtt tacgaactat ttactgactt cacttttctg 3120
gtaacatccg gtcctttcgt ttacacggca atatccgtca taaatgtgct tataaacagt 3180
aagcttgtcc gtgaccagac tcctttaatc ctgtctgtga aaccttcttt ccttgtgcca 3240
gagtccaggt tccaagttca aacagtactt cagtttgtgc ctccgagtgt ggatactggc 3300
ttctgcaact tcacccagcg cattgagaaa ggcctaatga cagctctctt tgaagtgaga 3360
aaacaccacc agggaacgta taacctcacg gtgcagatct tgaatatcac catcagttcc 3420
tcaagggtga ctcctcggcg gggcccggtg aatatcatct ttgcggttaa aagcacacag 3480
ggatttttga atgggtcgga agtgagcgag ctgctcagaa acttgagtgt ggtggagttc 3540
agtttctatc tgggataccc agtgctgcag atcgcagagc ccttccagta tccacagctc 3600
aacttatctc agttgctgaa gtcctcttgg gtcagaacag ttctcctggg cgtcatggag 3660

aagcaactcc agaatgaagt gtttcaagcc gagatggaac gcaagctggc ccagctgctc 3720
agcgaggttt ccaccagaag gcggatgtgg agaagggcca ctgtagctgc agggaacagt 3780
gtggtgcagg tggtaaatgt gtcgaggctg gagggagatg acaatccggt acagctcatc 3840
tactttgtgg aggatcaaga tggagaaaga ctcagtgcag tcaagtcttc ggacctgatt 3900
aacaaaatgg acctccagag agcagccatc atcttgggtt accgaattca aggtgtcatt 3960
gcccagcctg tcgacagggt gaagaggccg tctccggaat cccagagcaa caacttgtgg 4020
gtcattgttg gcgtggtcat cccagtgctg gtggtgatgg tgattgttgt catcctctac 4080
tggaaactat gccgcacaga caagctagac tttcagcctg acactgtggc caacattcag 4140
cagcgtcaga agctgcagat ccctagtgtg aagggcttcg attttgctaa gcagcatctg 4200
ggtcagcaca ataaagacga catattgatt attcatgagc cagcgccact gccaggacct 4260
ctgaaggacc acaccacgcc ctcggaaaat ggagacgtgc caagccccaa gtcaaagatc 4320
ccttccaaga atgttcgtca cagaggaaga gtttctccct cagatgctga ctctacggtc 4380
agtgaagagt ccagcgagag ggacgcagga gataagacgc cgggagccgt caacgatggc 4440
aggtcccaca gagctccgca gagcgggcca ccactgccca gttcgggaaa tgagcagcac 4500
tcatcagcct ccatcttcga gcacgtggac aggatctccc gcccccggga ggctagccgg 4560
cgggtcccca gtaagatcca gcttatcgcc atgcagccga tcccggcacc tcccgtccag 4620
cgcccctccc cagccgaccg agtggcggaa agcaataaaa tcaacaaaga gattcagacc 4680
gcgctgcggc acaagtctga gatcgagcac catcgcaaca agatccgcct gcgcgccaag 4740
cgccgcgggc actacgagtt cccggtggta gacgacctgt cctcgggcga cactaaggag 4800
cgacaccggg tgtaccgcag ggcacagatg cagatcgaca agatcctgga ccccacggcc 4860
agcgtgccct ccgtgttcat agagcccagg aagagctcac ggataaaacg ttctcccaag 4920
cctcgccgga aacaccaggt caacggctgt cctgccgacg ctgagaagga ccggctcatc 4980
accacagaca gcgatggcac ctacaggagg cccccggcg tccacaactc agcctacatc 5040
ggatgcccat cggatcctga cctcccagcc gatgtgcaga caccatcctc ggtggaactg 5100
gggaggtatc cagcccttcc cttcccggcc tcccagtaca tcccacccca gccgtccatc 5160
gaggaggcac gccagaccat gcactccctc ctggacgacg cctttgccct cgtggccccc 5220
agcagccagc ctgccagcac cgcaggtgta ggccccggag tcccacccgg cctgcccgca 5280
aacagcaccc cttcccagga agagaggcga gccacccagt gggggtcctt ctacagccca 5340
gcccagacgg ccaacaatcc ctgcagtaga tacgaagact atggaatgac tcccccgacg 5400
ggtccattgc caagaccagg ttttggcccc ggtttgctgc agtctacaga gctggtgccc 5460
cctgaccctc agcagccaca ggcctccgcc gaagccccat ttgctgccag agggatctac 5520

tcggaggaga tgccgtcggt ggcccggcct cggcctgtcg ggggtaccac aggctcccag 5580

atccagcacc tgacacaggt ggggattgcc agcagaattg gagctcagcc agtggaaatc 5640

ccgccaagca gaggcagcca gtatgggggg ccaggctggc cttcgtacgg ggaggacgaa 5700

gcggggcgaa gagaggccac acacatgctc ggacatcaag agtattcttc ttcaccgcta 5760

tttcaggtgc caaggacttc aggcagggag ccctcagctc cttccgggaa cctcccccac 5820

cggggactgc agggccctgg gctgggttac cccaccagct ccacggaaga cctccagcct 5880

ggccactcct cggcctctct catcaaagca atccgcgagg agctcctccg gctctcccag 5940

aaacagagca ccgtgcagaa cttccacagc tgatcggcct cgcctcgcag atttgccaag 6000

tatccgcttc ctgtggaagc aagaccaaaa ggaaatcaac tgagtgggtg tttggaagag 6060

gaaggagcaa ctctcgggca gcctgcccaa gggagggagc aagttgcaat ttagaagatg 6120

ccatacgtcg tgtgacagct catgagcctt tcactgggct ggcaattgtc tgaacacttg 6180

ggttcagttg aaatatatgt attttggcca aaagccagca gcacttcaca aaaacaaaac 6240

acaaacctaa gctaacaaaa tgactgcatt cgtctctttt ttaaaggtag agattaaact 6300

gtatagacag catagggatg aaaggaacca agcgtttctg tgggattgag actggtacgt 6360

gtacgatgaa cctgctgctt tgttttctga gaagaggttt gaagacattt tattaacagc 6420

ttaatttttc tcttttactc cataggaact tattttaata gtaacattaa caacaagaat 6480

actaagactg tttgggaatt ttaaaaagct actagtgaga aaccaaatga taggttgtag 6540

agcctgatga ctccaaacaa agccatcacc cgcattcttc ctccttcttc tggtgctaca 6600

gctccaaggg cccttcacct tcatgtctga aatggaactt tggctttttc agtggaagaa 6660

tatgttgaag gtttcatttt gttctagaaa aaaaaaatcc ctcccaaagt ggggcaaaaa 6720

gctttatatt tatttgatta tccaaaatac agatcaaagt ttagatctac attcttcatt 6780

gtatttgctg tttcttaatt gggcacacac aactcctggt catgtcccag ttcagcaccg 6840

atcgctaagg ccgatcctgt agaatgcggc tttcaagagg tcctaactga tcctttcttc 6900

cactttgctg ttgatttgtt cacaaattat gtctgaatga gaaatcttct gtaagcagaa 6960

gttatttaat aattcccagc accacgaaat tgttatacat acatccctac cagtcacatt 7020

ccctgtgttc agagcctgga aatgaaattg agttcagttc agcctctctg taatgaatca 7080

agaaatgtaa aagagctttg agaccccaag ggaaaggaga gagttgcttc tcatttctga 7140

ttttattgtg ctgttactct gtcgagtggc tattaaaatt gtcttatgct ctttgggcta 7200

agaggggatc atctcgctta agatgtactc ctgatgtaaa catttccccc actttccaaa 7260

taatggtaaa gaaaacagtg gcaagggctg ttctgttttc tggtacctga gtgaaactca 7320

gaagaaaagc aggcttagct aagagatttt cactattaac ctgtttttat tagatcagcg 7380

aagacagtca tgcatcgctt aatgatgggg atgcattctg agaaatgtgt cattaggctg 7440

ttttgttgct gtgccaacat cctagattgt acttacacaa cctacatggt atagcctact 7500

acacacctgg gctgtgtggc atggcctatt cctcttaggc tacaaacctg tacagcatgt 7560

tactgtactg aacacaatgg taagtatttg tgtatctaaa catagctaaa catagaaaag 7620

gtatggtaaa aatacaattt tataatctta tgggactacc atcaatatgt agaccgtcat 7680

tgaccaaaac atcctgatgt gtgcatgact gtacttttca ttaaataaca gataaggggc 7740

tatagaattt ggggctctga ctgatcaaaa cctgtgtatc tgtgtcaagt tttaagaccc 7800

ctgaaacagc taaaacaggt ttccaaagtg taatcactgg atttcaaaac catgttttag 7860

cccttcatta atgaagcctg ttgtacagat ttagagtctt acaatatgag ggaagttggt 7920

tctgggaagg taagatgtag ccagttttca tctgggcacc tctgaaagag aaggagtgca 7980

ggagagtaag tctctcaagg acgttcaaca aaggaccagc agcatcttat caggcgatcc 8040

tcagaggctc caaggagcat tggagagaat gcccctttct gggcttgatg tcttggtttt 8100

ggtttaatac caaatttctc ttggtcttgg tttaataccc gactcctccc aaattgaacg 8160

attatctttg tatgtcactc aaaaatacca ggtttcctga atatgttatt aagaatttca 8220

gatatccaag cagaatttta ttatggacac cttgtaatga attcaaatcg tgtgacagca 8280

aacgggataa atggcccctt ctcacccagt tccgctcagg gccatacagg cttgcacata 8340

gagtgtgcta aaaatggtaa cctcctttga gcattgcaga aagagggttc tgtttcaaat 8400

tgggctgacc gtaaaagcaa ttttgatgtc tttcaaacta ccataaaggg attttaactg 8460

tatttttatt cttagaaaca atacaccttt aaacagatgt caagaccaaa gatgatgtat 8520

aataaacagc cggtggttat ggtgtggtgt gagcaggccc ccggatcacc agccctctgc 8580

tggtggtcat aaagcacaca cagtttgtac ttgcttcctc tcgacgcctg ccacaacagt 8640

tttgccagtc accaaactca gaaaaaacta gctgtgtgag cagcaccgta ccctagcggt 8700

cttcagacat taagttcacg tcatccactg aaaggaaggg tccttgcgtt gacagcgtgt 8760

ggctttggag tccgtttatg gaagcactct acaatgaaca gttgaatttt atgtctattc 8820

ttttttccta ttttaaaagt tctagtggta acacaaactg taaatttgag tcagaatgtt 8880

tggagaaatg ttgttttttt ttttttcaga gactactttg tcactcactg accatgtctg 8940

gttccttctc tgaacttcat tctccccata agccaaacaa gaacagacct cccccgccac 9000

ctcccaggca ccgtagttgt gagaatcaga tgtgatcatg tgtgcagatg tcctgtgagg 9060

agggagcata aagcactgtg aaaatgtagc atgctgtcta tgtggacgcc ctaggatgag 9120

tgatgtgaaa cgctgcacta ctgcggtgaa tgggttcaca catgggtaat gagcaaaacc 9180

gaaagctcgg tgtgactcag tttcctcact cccattttgc tttaatttca cttccttcac 9240

caattttccg attgcattta ttaagcatct ttctgctccc gactttctgg tgtctctggc 9300

accaaataac ccagcagaca tgagccttgc cttctgagat tttagaatct aagatagcac 9360
gagtgggtat agaagatgga agataccgat aaataacatg gtttaagtgt caataaaatt 9420
cattcgaaga gatcaggcgc ggtggctcac gcctgtaatc ccagcatttt gggaggctga 9480
ggcaggtgga tcacttgaga tcaagagctt aaaaccagcc caggcaacat ggcaaaaccc 9540
cgtctctaca aaaaaataca aaaactagct gggtgtggtg gcgcatgcct gtagtcccag 9600
ctgaggcacg ataattgctt gaacccgggg ggtggaggtt gcagtgagct gagattgcac 9660
cactgtactc caacctgggc gacagagcaa gactgtgtct caaaaagaaa aaaatttgtt 9720
caaaagacat aaatgaatta ggcacccaga agaagttgca tgtttggaaa tgcaatatct 9780
tggggagacg tcaacttctg aagtgacttt gaaggcaggg cagctaccca ggcaaatggc 9840
gtggaaggaa gagctgagag tgggcctggg aaaggctgta gggaaggagt gaagctgtca 9900
ctgaaatgaa cttgatcttg atctgctttg atatggggac agaatccccg actgcactat 9960
tgagggagtt tcagaagaga aggaagaggt gctggaagga tgcttttgca gtcatgcagc 10020
aaggaaacga ccagggctgt gaagatctac agaggaagta ctccagtgtg ggggaggcaa 10080
gggaagagga cgaaaatgat gagaatgaca ctgaggtcat tgtttagagc agatctcaag 10140
cagccgtttg gccacgccat atcctttgtg gagcatagtg ggttatctat ctgtctgtct 10200
gtctatgtat ctgtctatct atctatatat atattcagct tctttactgc tacctacaca 10260
tttttctcca aattttatta atttcatagt gttttgattt gggtggcaga tgacttttaa 10320
gcagtgggtg tttgccggcc agatctttcc tggcatgcgg actgtgaggc aaagcacggg 10380
tgaaggtagg cgctaaaggc tttgggctaa agccagcacg cggttctgtg ctataggagt 10440
ctcccgtttc ccgtggacag gttcagcgtt ccttctttcg cacaactttt ttctaagtgt 10500
tccagtgacc aagccagtca ttcggacact gatttgcagt gcattggcag taattcacaa 10560
attagttgtt atataagtct ctctcatccc tttcacacta gattctcaga catgaatgaa 10620
tttgtcgttt ggaaggaaag ctgggtaacg ttttgggcac aggggaagga ggactccggt 10680
cttaactccc acgctaactt tagctcaagt ggagttttca ccgtggtcat ttctacctcc 10740
ggagcaaggt gccagcgcca gtactagagc ctgcttatcc acatttgccc tggacaggag 10800
caggaggaag tccacttctg taccggcaga cagagcatgt gaacacaaaa cacatttcta 10860
tggcatagtc aactgaactt catttttaca tttaatctaa catgttaaca cgttctaaca 10920
gggtttctat gagcagctgc tgtaacatac tcatcaacta tgatagactt aacacttgtt 10980
acctaatgaa caaggaggat gtgcatttcg ggtttctttt gatattcgtg gaggtgattg 11040
tcagtgttta aacaggacta ctttctccac tatgaagatg acttggaaat cgcaacatca 11100
tggtacattg ctaagtccat gcttgtgtgt gtgacagtag gcttgataat ttatcttaaa 11160
acacagcagc attgaaattt aggaaaagaa tattaaatgc ctttggaaac atgaaacaaa 11220

gttaggagcc agtaagaaag tgacagaagc aatgaatgtc ttaatgcagt atagttaaaa 11280

tggcttccca cagggtagat aattatccag gatccttcct tttccttctt cctccaggtt 11340

tttcaggggt cacttcacat ttctaaagaa agagtgaata ggctgggcat ggtggctcaa 11400

gcctctaatc tcaacgcttt gggaggctga ggcaggagga tcgcttgagg ccaggagttt 11460

gagaccacct tgggcaacat aacgagaccc cgtctctaca aaaaaaattt aaaaattagc 11520

tgagcatggt ggcatgtgcc agtagtccca gctactcgaa aggctaagac tggaggatcg 11580

cttgagccaa tgagttggag gctgcagtga gctataatca cgccactgca ctccagcctg 11640

ggctgcaggg tgaggtcctg tctctggaaa aaaaaaaaaa aaaaaaagga ataggtaaaa 11700

gggacagagg tgaaattttg agtgacttga gtctcttgca gtccctgatt acacagaacc 11760

tttctgggct acttggagca tcacgaatag tctttcctgt acttaccaga tttcaagtat 11820

tcataacttg actccctaag tgtacaagtt gggaatagta cagggccaag ttcaagtcgc 11880

atatgctgta ctgttcctcc tgcaaatgtg gggaaagaag agggagatac tagaggaact 11940

gaggctccac ccattcattc agttgctcta agcaccagag gacttgtttc agaaaagggg 12000

agtgggaacg ccctcgactt tgccctcctc cggagcatct ctgggacgca gggagtctgg 12060

ctagcgttaa taggaaaggt tgctcggcag agctgccctg gagtactgac ttgtctctcc 12120

ctcctttgtc aaggtccatg tttttctggc tcttcctgca cactcatccc tagattatga 12180

gcgttaatgt acgaaatgtg cagagcaaat tgaggccaac tgtggcatca atactgcaac 12240

ttaaacttga caatcatggt ttgctggtcc tcaaacaggc actgaaatct cagtatgggt 12300

atacgattta ataatcggcc cctcccttct atttgtcggt tttatgtttc tatccttcaa 12360

tagctgcact gttttctaat gtgctgtaga gtttttgaaa taatttatgc aagtatttgg 12420

tttccatgtt tacaatttat acagctttcc tagcatttta aatggaaatg tcaataaatg 12480

ctatgaattg gtgtcaaa 12498

<210> 55
<211> 1934
<212> PRT
<213> Homo sapiens

<400> 55

Met Pro Gly Ala Arg Arg Arg Arg Arg Gly Ala Ala Met Glu Gly Lys
1 5 10 15

Pro Arg Ala Gly Val Ala Leu Ala Pro Gly Pro Ser Gly Arg Arg Pro
20 25 30

Ser Ala Arg Cys Ala Arg Arg Arg Arg Pro Gly Leu Leu Leu Pro Gly
35 40 45

Leu Trp Leu Leu Leu Leu Ala Arg Pro Ala Ser Cys Ala Pro Asp Glu
    50                  55                  60

Leu Ser Pro Glu Gln His Asn Leu Ser Leu Tyr Ser Met Glu Leu Val
65                  70                  75                  80

Leu Lys Lys Ser Thr Gly His Ser Ala Ala Gln Val Ala Leu Thr Glu
                85                  90                  95

Thr Ala Pro Gly Ser Gln His Ser Ser Pro Leu His Val Thr Ala Pro
            100                 105                 110

Pro Ser Ala Thr Thr Phe Asp Thr Ala Phe Phe Asn Gln Gly Lys Gln
        115                 120                 125

Thr Lys Ser Thr Ala Asp Pro Ser Ile Phe Val Ala Thr Tyr Val Ser
    130                 135                 140

Val Thr Ser Lys Glu Val Ala Val Asn Asp Asp Glu Met Asp Asn Phe
145                 150                 155                 160

Leu Pro Asp Thr His Trp Thr Thr Pro Arg Met Val Ser Pro Ile Gln
                165                 170                 175

Tyr Ile Thr Val Ser Pro Pro Gly Leu Pro Arg Glu Ala Leu Glu Pro
            180                 185                 190

Met Leu Thr Pro Ser Leu Pro Met Val Ser Leu Gln Asp Glu Glu Val
        195                 200                 205

Thr Ser Gly Trp Gln Asn Thr Thr Arg Gln Pro Ala Ala Tyr Ala Glu
    210                 215                 220

Ser Ala Ser His Phe His Thr Phe Arg Ser Ala Phe Arg Thr Ser Glu
225                 230                 235                 240

Gly Ile Val Pro Thr Pro Gly Arg Asn Leu Val Leu Tyr Pro Thr Asp
                245                 250                 255

Ala Tyr Ser His Leu Ser Ser Arg Thr Leu Pro Glu Ile Val Ala Ser
            260                 265                 270

Leu Thr Glu Gly Val Glu Thr Thr Leu Phe Leu Ser Ser Arg Ser Leu
        275                 280                 285

Met Pro Gln Pro Leu Gly Asp Gly Ile Thr Ile Pro Leu Pro Ser Leu

290 295 300

Gly Glu Val Ser Gln Pro Pro Glu Glu Val Trp Ala Thr Ser Ala Asp
305 310 315 320

Arg Tyr Thr Asp Val Thr Thr Val Leu Ser Gln Ser Leu Glu Glu Thr
325 330 335

Ile Ser Pro Arg Thr Tyr Pro Thr Val Thr Ala Ser His Ala Ala Leu
340 345 350

Ala Phe Ser Arg Thr His Ser Pro Leu Leu Ser Thr Pro Leu Ala Phe
355 360 365

Ala Ser Ser Ala Ser Pro Thr Asp Val Ser Ser Asn Pro Phe Leu Pro
370 375 380

Ser Asp Ser Ser Lys Thr Ser Glu Leu His Ser Asn Ser Ala Leu Pro
385 390 395 400

Gly Pro Val Asp Asn Thr His Ile Leu Ser Pro Val Ser Ser Phe Arg
405 410 415

Pro Tyr Thr Trp Cys Ala Ala Cys Thr Val Pro Ser Pro Gln Gln Val
420 425 430

Leu Ala Thr Ser Leu Met Glu Lys Asp Val Gly Ser Gly Asp Gly Ala
435 440 445

Glu Thr Leu Cys Met Thr Val Leu Glu Glu Ser Ser Ile Ser Leu Met
450 455 460

Ser Ser Val Val Ala Asp Phe Ser Glu Phe Glu Glu Asp Pro Gln Val
465 470 475 480

Phe Asn Thr Leu Phe Pro Ser Arg Pro Ile Val Pro Leu Ser Ser Arg
485 490 495

Ser Met Glu Ile Ser Glu Thr Ser Val Gly Ile Ser Ala Glu Val Asp
500 505 510

Met Ser Ser Val Thr Thr Thr Gln Val Pro Pro Ala His Gly Arg Leu
515 520 525

Ser Val Pro Ala Ser Leu Asp Pro Thr Ala Gly Ser Leu Ser Val Ala
530 535 540

Glu Thr Gln Val Thr Pro Ser Ser Val Thr Thr Ala Phe Phe Ser Val
545 550 555 560

Ile Thr Ser Ile Leu Leu Asp Ser Ser Phe Ser Val Ile Ala Asn Lys
565 570 575

Asn Thr Pro Ser Leu Ala Val Arg Asp Pro Ser Val Phe Thr Pro Tyr
580 585 590

Ser Leu Val Pro Ser Val Glu Ser Ser Leu Phe Ser Asp Gln Glu Arg
595 600 605

Ser Ser Phe Ser Glu His Lys Pro Arg Gly Ala Leu Asp Phe Ala Ser
610 615 620

Ser Phe Phe Ser Thr Pro Pro Leu Glu Leu Ser Gly Ser Ile Ser Ser
625 630 635 640

Pro Ser Glu Ala Pro Ala Ser Leu Ser Leu Met Pro Ser Asp Leu Ser
645 650 655

Pro Phe Thr Ser Gln Ser Phe Ser Pro Leu Val Glu Thr Phe Thr Leu
660 665 670

Phe Asp Ser Ser Asp Leu Gln Ser Ser Gln Leu Ser Leu Pro Ser Ser
675 680 685

Thr Asn Leu Glu Phe Ser Gln Leu Gln Pro Ser Ser Glu Leu Pro Leu
690 695 700

Asn Thr Ile Met Leu Leu Pro Ser Arg Ser Glu Val Ser Pro Trp Ser
705 710 715 720

Ser Phe Pro Ser Asp Ser Leu Glu Phe Val Glu Ala Ser Thr Val Ser
725 730 735

Leu Thr Asp Ser Glu Ala His Phe Thr Ser Ala Phe Ile Glu Thr Thr
740 745 750

Ser Tyr Leu Glu Ser Ser Leu Ile Ser His Glu Ser Ala Val Thr Ala
755 760 765

Leu Val Pro Pro Gly Ser Glu Ser Phe Asp Ile Leu Thr Ala Gly Ile
770 775 780

Gln Ala Thr Ser Pro Leu Thr Thr Val His Thr Thr Pro Ile Leu Thr
785 790 795 800

Glu Ser Ser Leu Phe Ser Thr Leu Thr Pro Pro Asp Asp Gln Ile Ser
805 810 815

Ala Leu Asp Gly His Val Ser Val Leu Ala Ser Phe Ser Lys Ala Ile
820 825 830

Pro Thr Gly Thr Val Leu Ile Thr Asp Ala Tyr Leu Pro Ser Gly Ser
835 840 845

Ser Phe Val Ser Glu Ala Thr Pro Phe Pro Leu Pro Thr Glu Leu Thr
850 855 860

Val Val Gly Pro Ser Leu Thr Pro Thr Glu Val Pro Leu Asn Thr Ser
865 870 875 880

Thr Glu Val Ser Thr Thr Ser Thr Gly Ala Ala Thr Gly Gly Pro Leu
885 890 895

Asp Ser Thr Leu Met Gly Asp Ala Ala Ser Gln Ser Pro Pro Glu Ser
900 905 910

Ser Ala Ala Pro Pro Leu Pro Ser Leu Arg Pro Val Thr Ala Phe Thr
915 920 925

Leu Glu Ala Thr Val Asp Thr Pro Thr Leu Ala Thr Ala Lys Pro Pro
930 935 940

Tyr Val Cys Asp Ile Thr Val Pro Asp Ala Tyr Leu Ile Thr Thr Val
945 950 955 960

Leu Ala Arg Arg Ala Val Gln Glu Tyr Ile Ile Thr Ala Ile Lys Glu
965 970 975

Val Leu Arg Ile His Phe Asn Arg Ala Val Glu Leu Lys Val Tyr Glu
980 985 990

Leu Phe Thr Asp Phe Thr Phe Leu Val Thr Ser Gly Pro Phe Val Tyr
995 1000 1005

Thr Ala Ile Ser Val Ile Asn Val Leu Ile Asn Ser Lys Leu Val
1010 1015 1020

Arg Asp Gln Thr Pro Leu Ile Leu Ser Val Lys Pro Ser Phe Leu
1025 1030 1035

Val Pro Glu Ser Arg Phe Gln Val Gln Thr Val Leu Gln Phe Val
1040 1045 1050

Pro Pro Ser Val Asp Thr Gly Phe Cys Asn Phe Thr Gln Arg Ile
1055 1060 1065

Glu Lys Gly Leu Met Thr Ala Leu Phe Glu Val Arg Lys His His
1070 1075 1080

Gln Gly Thr Tyr Asn Leu Thr Val Gln Ile Leu Asn Ile Thr Ile
1085 1090 1095

Ser Ser Ser Arg Val Thr Pro Arg Arg Gly Pro Val Asn Ile Ile
1100 1105 1110

Phe Ala Val Lys Ser Thr Gln Gly Phe Leu Asn Gly Ser Glu Val
1115 1120 1125

Ser Glu Leu Leu Arg Asn Leu Ser Val Val Glu Phe Ser Phe Tyr
1130 1135 1140

Leu Gly Tyr Pro Val Leu Gln Ile Ala Glu Pro Phe Gln Tyr Pro
1145 1150 1155

Gln Leu Asn Leu Ser Gln Leu Leu Lys Ser Ser Trp Val Arg Thr
1160 1165 1170

Val Leu Leu Gly Val Met Glu Lys Gln Leu Gln Asn Glu Val Phe
1175 1180 1185

Gln Ala Glu Met Glu Arg Lys Leu Ala Gln Leu Leu Ser Glu Val
1190 1195 1200

Ser Thr Arg Arg Arg Met Trp Arg Arg Ala Thr Val Ala Ala Gly
1205 1210 1215

Asn Ser Val Val Gln Val Val Asn Val Ser Arg Leu Glu Gly Asp
1220 1225 1230

Asp Asn Pro Val Gln Leu Ile Tyr Phe Val Glu Asp Gln Asp Gly
1235 1240 1245

Glu Arg Leu Ser Ala Val Lys Ser Ser Asp Leu Ile Asn Lys Met
1250 1255 1260

Asp Leu Gln Arg Ala Ala Ile Ile Leu Gly Tyr Arg Ile Gln Gly
1265 1270 1275

Val Ile Ala Gln Pro Val Asp Arg Val Lys Arg Pro Ser Pro Glu

1280 1285 1290

Ser Gln Ser Asn Asn Leu Trp Val Ile Val Gly Val Val Ile Pro
1295 1300 1305

Val Leu Val Val Met Val Ile Val Val Ile Leu Tyr Trp Lys Leu
1310 1315 1320

Cys Arg Thr Asp Lys Leu Asp Phe Gln Pro Asp Thr Val Ala Asn
1325 1330 1335

Ile Gln Gln Arg Gln Lys Leu Gln Ile Pro Ser Val Lys Gly Phe
1340 1345 1350

Asp Phe Ala Lys Gln His Leu Gly Gln His Asn Lys Asp Asp Ile
1355 1360 1365

Leu Ile Ile His Glu Pro Ala Pro Leu Pro Gly Pro Leu Lys Asp
1370 1375 1380

His Thr Thr Pro Ser Glu Asn Gly Asp Val Pro Ser Pro Lys Ser
1385 1390 1395

Lys Ile Pro Ser Lys Asn Val Arg His Arg Gly Arg Val Ser Pro
1400 1405 1410

Ser Asp Ala Asp Ser Thr Val Ser Glu Glu Ser Ser Glu Arg Asp
1415 1420 1425

Ala Gly Asp Lys Thr Pro Gly Ala Val Asn Asp Gly Arg Ser His
1430 1435 1440

Arg Ala Pro Gln Ser Gly Pro Pro Leu Pro Ser Ser Gly Asn Glu
1445 1450 1455

Gln His Ser Ser Ala Ser Ile Phe Glu His Val Asp Arg Ile Ser
1460 1465 1470

Arg Pro Pro Glu Ala Ser Arg Arg Val Pro Ser Lys Ile Gln Leu
1475 1480 1485

Ile Ala Met Gln Pro Ile Pro Ala Pro Pro Val Gln Arg Pro Ser
1490 1495 1500

Pro Ala Asp Arg Val Ala Glu Ser Asn Lys Ile Asn Lys Glu Ile
1505 1510 1515

Gln Thr Ala Leu Arg His Lys Ser Glu Ile Glu His His Arg Asn
1520 1525 1530

Lys Ile Arg Leu Arg Ala Lys Arg Arg Gly His Tyr Glu Phe Pro
1535 1540 1545

Val Val Asp Asp Leu Ser Ser Gly Asp Thr Lys Glu Arg His Arg
1550 1555 1560

Val Tyr Arg Arg Ala Gln Met Gln Ile Asp Lys Ile Leu Asp Pro
1565 1570 1575

Thr Ala Ser Val Pro Ser Val Phe Ile Glu Pro Arg Lys Ser Ser
1580 1585 1590

Arg Ile Lys Arg Ser Pro Lys Pro Arg Arg Lys His Gln Val Asn
1595 1600 1605

Gly Cys Pro Ala Asp Ala Glu Lys Asp Arg Leu Ile Thr Thr Asp
1610 1615 1620

Ser Asp Gly Thr Tyr Arg Arg Pro Pro Gly Val His Asn Ser Ala
1625 1630 1635

Tyr Ile Gly Cys Pro Ser Asp Pro Asp Leu Pro Ala Asp Val Gln
1640 1645 1650

Thr Pro Ser Ser Val Glu Leu Gly Arg Tyr Pro Ala Leu Pro Phe
1655 1660 1665

Pro Ala Ser Gln Tyr Ile Pro Pro Gln Pro Ser Ile Glu Glu Ala
1670 1675 1680

Arg Gln Thr Met His Ser Leu Leu Asp Asp Ala Phe Ala Leu Val
1685 1690 1695

Ala Pro Ser Ser Gln Pro Ala Ser Thr Ala Gly Val Gly Pro Gly
1700 1705 1710

Val Pro Pro Gly Leu Pro Ala Asn Ser Thr Pro Ser Gln Glu Glu
1715 1720 1725

Arg Arg Ala Thr Gln Trp Gly Ser Phe Tyr Ser Pro Ala Gln Thr
1730 1735 1740

Ala Asn Asn Pro Cys Ser Arg Tyr Glu Asp Tyr Gly Met Thr Pro
1745 1750 1755

Pro Thr Gly Pro Leu Pro Arg Pro Gly Phe Gly Pro Gly Leu Leu
1760 1765 1770

Gln Ser Thr Glu Leu Val Pro Pro Asp Pro Gln Gln Pro Gln Ala
1775 1780 1785

Ser Ala Glu Ala Pro Phe Ala Ala Arg Gly Ile Tyr Ser Glu Glu
1790 1795 1800

Met Pro Ser Val Ala Arg Pro Arg Pro Val Gly Gly Thr Thr Gly
1805 1810 1815

Ser Gln Ile Gln His Leu Thr Gln Val Gly Ile Ala Ser Arg Ile
1820 1825 1830

Gly Ala Gln Pro Val Glu Ile Pro Pro Ser Arg Gly Ser Gln Tyr
1835 1840 1845

Gly Gly Pro Gly Trp Pro Ser Tyr Gly Glu Asp Glu Ala Gly Arg
1850 1855 1860

Arg Glu Ala Val Pro Arg Thr Ser Gly Arg Glu Pro Ser Ala Pro
1865 1870 1875

Ser Gly Asn Leu Pro His Arg Gly Leu Gln Gly Pro Gly Leu Gly
1880 1885 1890

Tyr Pro Thr Ser Ser Thr Glu Asp Leu Gln Pro Gly His Ser Ser
1895 1900 1905

Ala Ser Leu Ile Lys Ala Ile Arg Glu Glu Leu Leu Arg Leu Ser
1910 1915 1920

Gln Lys Gln Ser Thr Val Gln Asn Phe His Ser
1925 1930

<210> 56
<211> 1950
<212> PRT
<213> Homo sapiens

<400> 56

Met Pro Gly Ala Arg Arg Arg Arg Arg Gly Ala Ala Met Glu Gly Lys
1 5 10 15

Pro Arg Ala Gly Val Ala Leu Ala Pro Gly Pro Ser Gly Arg Arg Pro
20 25 30

Ser Ala Arg Cys Ala Arg Arg Arg Arg Pro Gly Leu Leu Leu Pro Gly
35 40 45

Leu Trp Leu Leu Leu Leu Ala Arg Pro Ala Ser Cys Ala Pro Asp Glu
50 55 60

Leu Ser Pro Glu Gln His Asn Leu Ser Leu Tyr Ser Met Glu Leu Val
65 70 75 80

Leu Lys Lys Ser Thr Gly His Ser Ala Ala Gln Val Ala Leu Thr Glu
85 90 95

Thr Ala Pro Gly Ser Gln His Ser Ser Pro Leu His Val Thr Ala Pro
100 105 110

Pro Ser Ala Thr Thr Phe Asp Thr Ala Phe Phe Asn Gln Gly Lys Gln
115 120 125

Thr Lys Ser Thr Ala Asp Pro Ser Ile Phe Val Ala Thr Tyr Val Ser
130 135 140

Val Thr Ser Lys Glu Val Ala Val Asn Asp Asp Glu Met Asp Asn Phe
145 150 155 160

Leu Pro Asp Thr His Trp Thr Thr Pro Arg Met Val Ser Pro Ile Gln
165 170 175

Tyr Ile Thr Val Ser Pro Pro Gly Leu Pro Arg Glu Ala Leu Glu Pro
180 185 190

Met Leu Thr Pro Ser Leu Pro Met Val Ser Leu Gln Asp Glu Glu Val
195 200 205

Thr Ser Gly Trp Gln Asn Thr Thr Arg Gln Pro Ala Ala Tyr Ala Glu
210 215 220

Ser Ala Ser His Phe His Thr Phe Arg Ser Ala Phe Arg Thr Ser Glu
225 230 235 240

Gly Ile Val Pro Thr Pro Gly Arg Asn Leu Val Leu Tyr Pro Thr Asp
245 250 255

Ala Tyr Ser His Leu Ser Ser Arg Thr Leu Pro Glu Ile Val Ala Ser
260 265 270

Leu Thr Glu Gly Val Glu Thr Thr Leu Phe Leu Ser Ser Arg Ser Leu
275 280 285

Met Pro Gln Pro Leu Gly Asp Gly Ile Thr Ile Pro Leu Pro Ser Leu
    290                 295                 300

Gly Glu Val Ser Gln Pro Pro Glu Glu Val Trp Ala Thr Ser Ala Asp
305                 310                 315                 320

Arg Tyr Thr Asp Val Thr Thr Val Leu Ser Gln Ser Leu Glu Glu Thr
                325                 330                 335

Ile Ser Pro Arg Thr Tyr Pro Thr Val Thr Ala Ser His Ala Ala Leu
            340                 345                 350

Ala Phe Ser Arg Thr His Ser Pro Leu Leu Ser Thr Pro Leu Ala Phe
        355                 360                 365

Ala Ser Ser Ala Ser Pro Thr Asp Val Ser Ser Asn Pro Phe Leu Pro
    370                 375                 380

Ser Asp Ser Ser Lys Thr Ser Glu Leu His Ser Asn Ser Ala Leu Pro
385                 390                 395                 400

Gly Pro Val Asp Asn Thr His Ile Leu Ser Pro Val Ser Ser Phe Arg
                405                 410                 415

Pro Tyr Thr Trp Cys Ala Ala Cys Thr Val Pro Ser Pro Gln Gln Val
            420                 425                 430

Leu Ala Thr Ser Leu Met Glu Lys Asp Val Gly Ser Gly Asp Gly Ala
        435                 440                 445

Glu Thr Leu Cys Met Thr Val Leu Glu Glu Ser Ser Ile Ser Leu Met
    450                 455                 460

Ser Ser Val Val Ala Asp Phe Ser Glu Phe Glu Glu Asp Pro Gln Val
465                 470                 475                 480

Phe Asn Thr Leu Phe Pro Ser Arg Pro Ile Val Pro Leu Ser Ser Arg
                485                 490                 495

Ser Met Glu Ile Ser Glu Thr Ser Val Gly Ile Ser Ala Glu Val Asp
            500                 505                 510

Met Ser Ser Val Thr Thr Thr Gln Val Pro Pro Ala His Gly Arg Leu
        515                 520                 525

Ser Val Pro Ala Ser Leu Asp Pro Thr Ala Gly Ser Leu Ser Val Ala

```
        530                 535                 540

Glu Thr Gln Val Thr Pro Ser Ser Val Thr Thr Ala Phe Phe Ser Val
545                 550                 555                 560

Ile Thr Ser Ile Leu Leu Asp Ser Ser Phe Ser Val Ile Ala Asn Lys
                565                 570                 575

Asn Thr Pro Ser Leu Ala Val Arg Asp Pro Ser Val Phe Thr Pro Tyr
            580                 585                 590

Ser Leu Val Pro Ser Val Glu Ser Ser Leu Phe Ser Asp Gln Glu Arg
        595                 600                 605

Ser Ser Phe Ser Glu His Lys Pro Arg Gly Ala Leu Asp Phe Ala Ser
    610                 615                 620

Ser Phe Phe Ser Thr Pro Pro Leu Glu Leu Ser Gly Ser Ile Ser Ser
625                 630                 635                 640

Pro Ser Glu Ala Pro Ala Ser Leu Ser Leu Met Pro Ser Asp Leu Ser
                645                 650                 655

Pro Phe Thr Ser Gln Ser Phe Ser Pro Leu Val Glu Thr Phe Thr Leu
            660                 665                 670

Phe Asp Ser Ser Asp Leu Gln Ser Ser Gln Leu Ser Leu Pro Ser Ser
        675                 680                 685

Thr Asn Leu Glu Phe Ser Gln Leu Gln Pro Ser Ser Glu Leu Pro Leu
    690                 695                 700

Asn Thr Ile Met Leu Leu Pro Ser Arg Ser Glu Val Ser Pro Trp Ser
705                 710                 715                 720

Ser Phe Pro Ser Asp Ser Leu Glu Phe Val Glu Ala Ser Thr Val Ser
                725                 730                 735

Leu Thr Asp Ser Glu Ala His Phe Thr Ser Ala Phe Ile Glu Thr Thr
            740                 745                 750

Ser Tyr Leu Glu Ser Ser Leu Ile Ser His Glu Ser Ala Val Thr Ala
        755                 760                 765

Leu Val Pro Pro Gly Ser Glu Ser Phe Asp Ile Leu Thr Ala Gly Ile
    770                 775                 780
```

Gln Ala Thr Ser Pro Leu Thr Thr Val His Thr Thr Pro Ile Leu Thr
785 790 795 800

Glu Ser Ser Leu Phe Ser Thr Leu Thr Pro Pro Asp Asp Gln Ile Ser
805 810 815

Ala Leu Asp Gly His Val Ser Val Leu Ala Ser Phe Ser Lys Ala Ile
820 825 830

Pro Thr Gly Thr Val Leu Ile Thr Asp Ala Tyr Leu Pro Ser Gly Ser
835 840 845

Ser Phe Val Ser Glu Ala Thr Pro Phe Pro Leu Pro Thr Glu Leu Thr
850 855 860

Val Val Gly Pro Ser Leu Thr Pro Thr Glu Val Pro Leu Asn Thr Ser
865 870 875 880

Thr Glu Val Ser Thr Thr Ser Thr Gly Ala Ala Thr Gly Gly Pro Leu
885 890 895

Asp Ser Thr Leu Met Gly Asp Ala Ala Ser Gln Ser Pro Pro Glu Ser
900 905 910

Ser Ala Ala Pro Pro Leu Pro Ser Leu Arg Pro Val Thr Ala Phe Thr
915 920 925

Leu Glu Ala Thr Val Asp Thr Pro Thr Leu Ala Thr Ala Lys Pro Pro
930 935 940

Tyr Val Cys Asp Ile Thr Val Pro Asp Ala Tyr Leu Ile Thr Thr Val
945 950 955 960

Leu Ala Arg Arg Ala Val Gln Glu Tyr Ile Ile Thr Ala Ile Lys Glu
965 970 975

Val Leu Arg Ile His Phe Asn Arg Ala Val Glu Leu Lys Val Tyr Glu
980 985 990

Leu Phe Thr Asp Phe Thr Phe Leu Val Thr Ser Gly Pro Phe Val Tyr
995 1000 1005

Thr Ala Ile Ser Val Ile Asn Val Leu Ile Asn Ser Lys Leu Val
1010 1015 1020

Arg Asp Gln Thr Pro Leu Ile Leu Ser Val Lys Pro Ser Phe Leu
1025 1030 1035

Val Pro Glu Ser Arg Phe Gln Val Gln Thr Val Leu Gln Phe Val
1040 1045 1050

Pro Pro Ser Val Asp Thr Gly Phe Cys Asn Phe Thr Gln Arg Ile
1055 1060 1065

Glu Lys Gly Leu Met Thr Ala Leu Phe Glu Val Arg Lys His His
1070 1075 1080

Gln Gly Thr Tyr Asn Leu Thr Val Gln Ile Leu Asn Ile Thr Ile
1085 1090 1095

Ser Ser Ser Arg Val Thr Pro Arg Arg Gly Pro Val Asn Ile Ile
1100 1105 1110

Phe Ala Val Lys Ser Thr Gln Gly Phe Leu Asn Gly Ser Glu Val
1115 1120 1125

Ser Glu Leu Leu Arg Asn Leu Ser Val Val Glu Phe Ser Phe Tyr
1130 1135 1140

Leu Gly Tyr Pro Val Leu Gln Ile Ala Glu Pro Phe Gln Tyr Pro
1145 1150 1155

Gln Leu Asn Leu Ser Gln Leu Leu Lys Ser Ser Trp Val Arg Thr
1160 1165 1170

Val Leu Leu Gly Val Met Glu Lys Gln Leu Gln Asn Glu Val Phe
1175 1180 1185

Gln Ala Glu Met Glu Arg Lys Leu Ala Gln Leu Leu Ser Glu Val
1190 1195 1200

Ser Thr Arg Arg Arg Met Trp Arg Arg Ala Thr Val Ala Ala Gly
1205 1210 1215

Asn Ser Val Val Gln Val Val Asn Val Ser Arg Leu Glu Gly Asp
1220 1225 1230

Asp Asn Pro Val Gln Leu Ile Tyr Phe Val Glu Asp Gln Asp Gly
1235 1240 1245

Glu Arg Leu Ser Ala Val Lys Ser Ser Asp Leu Ile Asn Lys Met
1250 1255 1260

Asp Leu Gln Arg Ala Ala Ile Ile Leu Gly Tyr Arg Ile Gln Gly
1265 1270 1275

Val Ile Ala Gln Pro Val Asp Arg Val Lys Arg Pro Ser Pro Glu
1280 1285 1290

Ser Gln Ser Asn Asn Leu Trp Val Ile Val Gly Val Val Ile Pro
1295 1300 1305

Val Leu Val Val Met Val Ile Val Val Ile Leu Tyr Trp Lys Leu
1310 1315 1320

Cys Arg Thr Asp Lys Leu Asp Phe Gln Pro Asp Thr Val Ala Asn
1325 1330 1335

Ile Gln Gln Arg Gln Lys Leu Gln Ile Pro Ser Val Lys Gly Phe
1340 1345 1350

Asp Phe Ala Lys Gln His Leu Gly Gln His Asn Lys Asp Asp Ile
1355 1360 1365

Leu Ile Ile His Glu Pro Ala Pro Leu Pro Gly Pro Leu Lys Asp
1370 1375 1380

His Thr Thr Pro Ser Glu Asn Gly Asp Val Pro Ser Pro Lys Ser
1385 1390 1395

Lys Ile Pro Ser Lys Asn Val Arg His Arg Gly Arg Val Ser Pro
1400 1405 1410

Ser Asp Ala Asp Ser Thr Val Ser Glu Glu Ser Ser Glu Arg Asp
1415 1420 1425

Ala Gly Asp Lys Thr Pro Gly Ala Val Asn Asp Gly Arg Ser His
1430 1435 1440

Arg Ala Pro Gln Ser Gly Pro Pro Leu Pro Ser Ser Gly Asn Glu
1445 1450 1455

Gln His Ser Ser Ala Ser Ile Phe Glu His Val Asp Arg Ile Ser
1460 1465 1470

Arg Pro Pro Glu Ala Ser Arg Arg Val Pro Ser Lys Ile Gln Leu
1475 1480 1485

Ile Ala Met Gln Pro Ile Pro Ala Pro Pro Val Gln Arg Pro Ser
1490 1495 1500

Pro Ala Asp Arg Val Ala Glu Ser Asn Lys Ile Asn Lys Glu Ile

1505 1510 1515

Gln Thr Ala Leu Arg His Lys Ser Glu Ile Glu His His Arg Asn
1520 1525 1530

Lys Ile Arg Leu Arg Ala Lys Arg Arg Gly His Tyr Glu Phe Pro
1535 1540 1545

Val Val Asp Asp Leu Ser Ser Gly Asp Thr Lys Glu Arg His Arg
1550 1555 1560

Val Tyr Arg Arg Ala Gln Met Gln Ile Asp Lys Ile Leu Asp Pro
1565 1570 1575

Thr Ala Ser Val Pro Ser Val Phe Ile Glu Pro Arg Lys Ser Ser
1580 1585 1590

Arg Ile Lys Arg Ser Pro Lys Pro Arg Arg Lys His Gln Val Asn
1595 1600 1605

Gly Cys Pro Ala Asp Ala Glu Lys Asp Arg Leu Ile Thr Thr Asp
1610 1615 1620

Ser Asp Gly Thr Tyr Arg Arg Pro Pro Gly Val His Asn Ser Ala
1625 1630 1635

Tyr Ile Gly Cys Pro Ser Asp Pro Asp Leu Pro Ala Asp Val Gln
1640 1645 1650

Thr Pro Ser Ser Val Glu Leu Gly Arg Tyr Pro Ala Leu Pro Phe
1655 1660 1665

Pro Ala Ser Gln Tyr Ile Pro Pro Gln Pro Ser Ile Glu Glu Ala
1670 1675 1680

Arg Gln Thr Met His Ser Leu Leu Asp Asp Ala Phe Ala Leu Val
1685 1690 1695

Ala Pro Ser Ser Gln Pro Ala Ser Thr Ala Gly Val Gly Pro Gly
1700 1705 1710

Val Pro Pro Gly Leu Pro Ala Asn Ser Thr Pro Ser Gln Glu Glu
1715 1720 1725

Arg Arg Ala Thr Gln Trp Gly Ser Phe Tyr Ser Pro Ala Gln Thr
1730 1735 1740

```
Ala Asn Asn Pro Cys Ser Arg Tyr Glu Asp Tyr Gly Met Thr Pro
    1745                1750                1755

Pro Thr Gly Pro Leu Pro Arg Pro Gly Phe Gly Pro Gly Leu Leu
    1760                1765                1770

Gln Ser Thr Glu Leu Val Pro Pro Asp Pro Gln Gln Pro Gln Ala
    1775                1780                1785

Ser Ala Glu Ala Pro Phe Ala Ala Arg Gly Ile Tyr Ser Glu Glu
    1790                1795                1800

Met Pro Ser Val Ala Arg Pro Arg Pro Val Gly Gly Thr Thr Gly
    1805                1810                1815

Ser Gln Ile Gln His Leu Thr Gln Val Gly Ile Ala Ser Arg Ile
    1820                1825                1830

Gly Ala Gln Pro Val Glu Ile Pro Pro Ser Arg Gly Ser Gln Tyr
    1835                1840                1845

Gly Gly Pro Gly Trp Pro Ser Tyr Gly Glu Asp Glu Ala Gly Arg
    1850                1855                1860

Arg Glu Ala Thr His Met Leu Gly His Gln Glu Tyr Ser Ser Ser
    1865                1870                1875

Pro Leu Phe Gln Val Pro Arg Thr Ser Gly Arg Glu Pro Ser Ala
    1880                1885                1890

Pro Ser Gly Asn Leu Pro His Arg Gly Leu Gln Gly Pro Gly Leu
    1895                1900                1905

Gly Tyr Pro Thr Ser Ser Thr Glu Asp Leu Gln Pro Gly His Ser
    1910                1915                1920

Ser Ala Ser Leu Ile Lys Ala Ile Arg Glu Glu Leu Leu Arg Leu
    1925                1930                1935

Ser Gln Lys Gln Ser Thr Val Gln Asn Phe His Ser
    1940                1945                1950


<210>  57
<211>  1671
<212>  DNA
<213>  Homo sapiens

<400>  57
acagcttcct gccgcaggcg ggcgggaggg cgggcacgga gaggcgggcg ccgaggaggg      60
```

```
gcaggtaggg ctgggacgca ggggtaactg gatcccccga cttcagccca ggccctggtc      120
tgaccaccct gggagcaggg actttccaca gtcagctgga cgcacactca gcccagtaaa      180
agaggggacc catcccggga gccccgggga gggcacagct gcctcctccc gggctcccct      240
gccacctggt gcctacctgc cccctgctcc ctgccgggtc cggtcctcac cccatcttca      300
tctggccttg actctgccct tgaggggcct aggggtgcag ccagcctgct ccgagctccc      360
ctgcagatgg aggaggccat cctggtcccc tgcgtgctgg ggctcctgct gctgcccatc      420
ctggccatgt tgatggcact gtgtgtgcac tgccacagac tgccaggctc ctacgacagc      480
acatcctcag atagtttgta tccaaggggc atccagttca aacggcctca cacggttgcc      540
ccctggccac ctgcctaccc acctgtcacc tcctacccac ccctgagcca gccagacctg      600
ctccccatcc caagatcccc gcagcccctt gggggctccc accggacgcc atcttcccgg      660
cgggattctg atggtgccaa cagtgtggcg agctacgaga acgaggaacc agcctgtgag      720
gatgcggatg aggatgagga cgactatcac aacccaggct acctggtggt gcttcctgac      780
agcaccccgg ccactagcac tgctgcccca tcagctcctg cactcagcac ccctggcatc      840
cgagacagtg ccttctccat ggagtccatt gatgattacg tgaacgttcc ggagagcggg      900
gagagcgcag aagcgtctct ggatggcagc cgggagtatg tgaatgtgtc ccaggaactg      960
catcctggag cggctaagac tgagcctgcc gccctgagtt cccaggaggc agaggaagtg     1020
gaggaagagg gggctccaga ttacgagaat ctgcaggagc tgaactgagg gcctgtggag     1080
gccgagtctg tcctggaacc aggcttgcct gggacggctg agctgggcag ctggaagtgg     1140
ctctggggtc ctcacatggc gtcctgccct tgctccagcc tgacaacagc ctgagaaatc     1200
cccccgtaac ttattatcac tttggggttc ggcctgtgtc ccccgaacgc tctgcacctt     1260
ctgacgcagc ctgagaatga cctgccctgg ccccagccct actctgtgta atagaataaa     1320
ggcctgcgtg tgtctgtgtt gagcgtgcgt ctgtgtgtgc ctgtgtgcga gtctgagtca     1380
gagatttgga gatgtctctg tgtgtttgtg tgtatctgtg ggtctccatc ctccatgggg     1440
gctcagccag gtgctgtgac accccccttc tgaatgaagc cttctgacct gggctggcac     1500
tgctgggggt gaggacacat tgccccatga gacagtccca gaacacggca gctgctggct     1560
gtgacaatgg tttcaccatc cttagaccaa gggatgggac ctgatgacct gggaggactc     1620
tcttagttct taccttttgt ggttctcaat aaaacagaac ttaaaaaatt g              1671
```

<210> 58
<211> 1758
<212> DNA
<213> Homo sapiens

<400> 58

```
acagcttcct gccgcaggcg ggcgggaggg cgggcacgga gaggcgggcg ccgaggaggg       60
```

gcaggtaggg ctgggacgca ggggtaactg gatccccga cttcagccca ggccctggtc 120

tgaccaccct gggagcaggg actttccaca gtcagctgga cgcacactca gcccagtaaa 180

agaggggacc catcccggga gccccgggga gggcacagct gcctcctccc gggctcccct 240

gccacctggt gcctacctgc cccctgctcc ctgccgggtc cggtcctcac cccatcttca 300

tctggccttg actctgccct tgaggggcct aggggtgcag ccagcctgct ccgagctccc 360

ctgcagatgg aggaggccat cctggtcccc tgcgtgctgg ggctcctgct gctgcccatc 420

ctggccatgt tgatggcact gtgtgtgcac tgccacagac tgccaggctc ctacgacagc 480

acatcctcag atagtttgta tccaaggggc atccagttca aacggcctca cacggttgcc 540

ccctggccac ctgcctaccc acctgtcacc tcctacccac ccctgagcca gccagacctg 600

ctccccatcc caagatcccc gcagcccctt gggggctccc accggacgcc atcttcccgg 660

cgggattctg atggtgccaa cagtgtggcg agctacgaga acgagggtgc gtctgggatc 720

cgaggtgccc aggctgggtg gggagtctgg ggtccgtcct ggactaggct gacccctgtg 780

tcgttacccc cagaaccagc ctgtgaggat gcggatgagg atgaggacga ctatcacaac 840

ccaggctacc tggtggtgct tcctgacagc accccggcca ctagcactgc tgccccatca 900

gctcctgcac tcagcacccc tggcatccga gacagtgcct tctccatgga gtccattgat 960

gattacgtga acgttccgga gagcggggag agcgcagaag cgtctctgga tggcagccgg 1020

gagtatgtga atgtgtccca ggaactgcat cctggagcgg ctaagactga gcctgccgcc 1080

ctgagttccc aggaggcaga ggaagtggag gaagaggggg ctccagatta cgagaatctg 1140

caggagctga actgagggcc tgtggaggcc gagtctgtcc tggaaccagg cttgcctggg 1200

acggctgagc tgggcagctg gaagtggctc tggggtcctc acatggcgtc ctgcccttgc 1260

tccagcctga caacagcctg agaaatcccc ccgtaactta ttatcacttt ggggttcggc 1320

ctgtgtcccc cgaacgctct gcaccttctg acgcagcctg agaatgacct gccctggccc 1380

cagccctact ctgtgtaata gaataaaggc ctgcgtgtgt ctgtgttgag cgtgcgtctg 1440

tgtgtgcctg tgtgcgagtc tgagtcagag atttggagat gtctctgtgt gtttgtgtgt 1500

atctgtgggt ctccatcctc catgggggct cagccaggtg ctgtgacacc cccttctga 1560

atgaagcctt ctgacctggg ctggcactgc tgggggtgag gacacattgc cccatgagac 1620

agtcccagaa cacggcagct gctggctgtg acaatggttt caccatcctt agaccaaggg 1680

atgggacctg atgacctggg aggactctct tagttcttac cttttgtggt tctcaataaa 1740

acagaactta aaaaattg 1758

<210> 59
<211> 233
<212> PRT

<213>  Homo sapiens

<400>  59

Met Glu Glu Ala Ile Leu Val Pro Cys Val Leu Gly Leu Leu Leu Leu
1               5                   10                  15

Pro Ile Leu Ala Met Leu Met Ala Leu Cys Val His Cys His Arg Leu
            20                  25                  30

Pro Gly Ser Tyr Asp Ser Thr Ser Ser Asp Ser Leu Tyr Pro Arg Gly
        35                  40                  45

Ile Gln Phe Lys Arg Pro His Thr Val Ala Pro Trp Pro Pro Ala Tyr
    50                  55                  60

Pro Pro Val Thr Ser Tyr Pro Pro Leu Ser Gln Pro Asp Leu Leu Pro
65                  70                  75                  80

Ile Pro Arg Ser Pro Gln Pro Leu Gly Gly Ser His Arg Thr Pro Ser
                85                  90                  95

Ser Arg Arg Asp Ser Asp Gly Ala Asn Ser Val Ala Ser Tyr Glu Asn
            100                 105                 110

Glu Glu Pro Ala Cys Glu Asp Ala Asp Glu Asp Glu Asp Asp Tyr His
        115                 120                 125

Asn Pro Gly Tyr Leu Val Val Leu Pro Asp Ser Thr Pro Ala Thr Ser
    130                 135                 140

Thr Ala Ala Pro Ser Ala Pro Ala Leu Ser Thr Pro Gly Ile Arg Asp
145                 150                 155                 160

Ser Ala Phe Ser Met Glu Ser Ile Asp Asp Tyr Val Asn Val Pro Glu
                165                 170                 175

Ser Gly Glu Ser Ala Glu Ala Ser Leu Asp Gly Ser Arg Glu Tyr Val
            180                 185                 190

Asn Val Ser Gln Glu Leu His Pro Gly Ala Ala Lys Thr Glu Pro Ala
        195                 200                 205

Ala Leu Ser Ser Gln Glu Ala Glu Glu Val Glu Glu Glu Gly Ala Pro
    210                 215                 220

Asp Tyr Glu Asn Leu Gln Glu Leu Asn
225                 230

<210> 60
<211> 262
<212> PRT
<213> Homo sapiens

<400> 60

Met Glu Glu Ala Ile Leu Val Pro Cys Val Leu Gly Leu Leu Leu Leu
1               5                   10                  15

Pro Ile Leu Ala Met Leu Met Ala Leu Cys Val His Cys His Arg Leu
            20                  25                  30

Pro Gly Ser Tyr Asp Ser Thr Ser Ser Asp Ser Leu Tyr Pro Arg Gly
        35                  40                  45

Ile Gln Phe Lys Arg Pro His Thr Val Ala Pro Trp Pro Pro Ala Tyr
    50                  55                  60

Pro Pro Val Thr Ser Tyr Pro Pro Leu Ser Gln Pro Asp Leu Leu Pro
65                  70                  75                  80

Ile Pro Arg Ser Pro Gln Pro Leu Gly Gly Ser His Arg Thr Pro Ser
                85                  90                  95

Ser Arg Arg Asp Ser Asp Gly Ala Asn Ser Val Ala Ser Tyr Glu Asn
            100                 105                 110

Glu Gly Ala Ser Gly Ile Arg Gly Ala Gln Ala Gly Trp Gly Val Trp
        115                 120                 125

Gly Pro Ser Trp Thr Arg Leu Thr Pro Val Ser Leu Pro Pro Glu Pro
    130                 135                 140

Ala Cys Glu Asp Ala Asp Glu Asp Glu Asp Asp Tyr His Asn Pro Gly
145                 150                 155                 160

Tyr Leu Val Val Leu Pro Asp Ser Thr Pro Ala Thr Ser Thr Ala Ala
                165                 170                 175

Pro Ser Ala Pro Ala Leu Ser Thr Pro Gly Ile Arg Asp Ser Ala Phe
            180                 185                 190

Ser Met Glu Ser Ile Asp Asp Tyr Val Asn Val Pro Glu Ser Gly Glu
        195                 200                 205

Ser Ala Glu Ala Ser Leu Asp Gly Ser Arg Glu Tyr Val Asn Val Ser
    210                 215                 220

Gln Glu Leu His Pro Gly Ala Ala Lys Thr Glu Pro Ala Ala Leu Ser
225 230 235 240

Ser Gln Glu Ala Glu Glu Val Glu Glu Glu Gly Ala Pro Asp Tyr Glu
245 250 255

Asn Leu Gln Glu Leu Asn
260

<210> 61
<211> 2091
<212> DNA
<213> Homo sapiens

<400> 61
gagacaggtg gtggctacga cggcgaaggg agctgagact gtccaggcag ccaggttagg 60

ccaggaggac catgtgaatg gggccagagg gctcccgggc tgggcaggga ccatgggctg 120

tggctgcagc tcacacccgg aagatgactg gatggaaaac atcgatgtgt gtgagaactg 180

ccattatccc atagtcccac tggatggcaa gggcacgctg ctcatccgaa atggctctga 240

ggtgcgggac ccactggtta cctacgaagg ctccaatccg ccggcttccc cactgcaaga 300

caacctggtt atcgctctgc acagctatga gccctctcac gacggagatc tgggctttga 360

gaagggggaa cagctccgca tcctggagca gagcggcgag tggtggaagg cgcagtccct 420

gaccacgggc caggaaggct tcatcccctt caattttgtg gccaaagcga acagcctgga 480

gcccgaaccc tggttcttca agaacctgag ccgcaaggac gcggagcggc agctcctggc 540

gcccgggaac actcacggct ccttcctcat ccgggagagc gagagcaccg cgggatcgtt 600

ttcactgtcg gtccgggact tcgaccagaa ccagggagag gtggtgaaac attacaagat 660

ccgtaatctg gacaacggtg gcttctacat ctcccctcga atcacttttc ccggcctgca 720

tgaactggtc cgccattaca ccaatgcttc agatgggctg tgcacacggt tgagccgccc 780

ctgccagacc cagaagcccc agaagccgtg gtgggaggac gagtgggagg ttcccaggga 840

gacgctgaag ctggtggagc ggctgggggc tggacagttc ggggaggtgt ggatggggta 900

ctacaacggg cacacgaagg tggcggtgaa gagcctgaag cagggcagca tgtccccgga 960

cgccttcctg gccgaggcca acctcatgaa gcagctgcaa caccagcggc tggttcggct 1020

ctacgctgtg gtcacccagg agcccatcta catcatcact gaatacatgg agaatggag 1080

tctagtggat ttttctcaaga ccccttcagg catcaagttg accatcaaca aactcctgga 1140

catggcagcc caaattgcag aaggcatggc attcattgaa gagcggaatt atattcatcg 1200

tgaccttcgg gctgccaaca ttctggtgtc tgacaccctg agctgcaaga ttgcagactt 1260

tggcctagca cgcctcattg aggacaacga gtacacagcc agggaggggg ccaagtttcc 1320

cattaagtgg acagcgccag aagccattaa ctacgggaca ttcaccatca agtcagatgt 1380

gtggtctttt gggatcctgc tgacggaaat tgtcacccac ggccgcatcc cttacccagg 1440

gatgaccaac ccggaggtga ttcagaacct ggagcgaggc taccgcatgg tgcgccctga 1500

caactgtcca gaggagctgt accaactcat gaggctgtgc tggaaggagc gcccagagga 1560

ccggcccacc tttgactacc tgcgcagtgt gctggaggac ttcttcacgg ccacagaggg 1620

ccagtaccag cctcagcctt gagaggcctt gagaggccct ggggttctcc ccctttctct 1680

ccagcctgac ttggggagat ggagttcttg tgccatagtc acatggccta tgcacatatg 1740

gactctgcac atgaatccca cccacatgtg acacatatgc accttgtgtc tgtacacgtg 1800

tcctgtagtt gcgtggactc tgcacatgtc ttgtacatgt gtagcctgtg catgtatgtc 1860

ttggacactg tacaaggtac ccctttctgg ctctcccatt tcctgagacc acagagagag 1920

gggagaagcc tgggattgac agaagcttct gcccacctac ttttctttcc tcagatcatc 1980

cagaagttcc tcaagggcca ggactttatc taatacctct gtgtgctcct ccttggtgcc 2040

tggcctggca cacatcagga gttcaataaa tgtctgttga tgactgttgt a 2091

<210> 62
<211> 509
<212> PRT
<213> Homo sapiens

<400> 62

Met Gly Cys Gly Cys Ser Ser His Pro Glu Asp Asp Trp Met Glu Asn
1 5 10 15

Ile Asp Val Cys Glu Asn Cys His Tyr Pro Ile Val Pro Leu Asp Gly
20 25 30

Lys Gly Thr Leu Leu Ile Arg Asn Gly Ser Glu Val Arg Asp Pro Leu
35 40 45

Val Thr Tyr Glu Gly Ser Asn Pro Pro Ala Ser Pro Leu Gln Asp Asn
50 55 60

Leu Val Ile Ala Leu His Ser Tyr Glu Pro Ser His Asp Gly Asp Leu
65 70 75 80

Gly Phe Glu Lys Gly Glu Gln Leu Arg Ile Leu Glu Gln Ser Gly Glu
85 90 95

Trp Trp Lys Ala Gln Ser Leu Thr Thr Gly Gln Glu Gly Phe Ile Pro
100 105 110

Phe Asn Phe Val Ala Lys Ala Asn Ser Leu Glu Pro Glu Pro Trp Phe

115 120 125

Phe Lys Asn Leu Ser Arg Lys Asp Ala Glu Arg Gln Leu Leu Ala Pro
130 135 140

Gly Asn Thr His Gly Ser Phe Leu Ile Arg Glu Ser Glu Ser Thr Ala
145 150 155 160

Gly Ser Phe Ser Leu Ser Val Arg Asp Phe Asp Gln Asn Gln Gly Glu
165 170 175

Val Val Lys His Tyr Lys Ile Arg Asn Leu Asp Asn Gly Gly Phe Tyr
180 185 190

Ile Ser Pro Arg Ile Thr Phe Pro Gly Leu His Glu Leu Val Arg His
195 200 205

Tyr Thr Asn Ala Ser Asp Gly Leu Cys Thr Arg Leu Ser Arg Pro Cys
210 215 220

Gln Thr Gln Lys Pro Gln Lys Pro Trp Trp Glu Asp Glu Trp Glu Val
225 230 235 240

Pro Arg Glu Thr Leu Lys Leu Val Glu Arg Leu Gly Ala Gly Gln Phe
245 250 255

Gly Glu Val Trp Met Gly Tyr Tyr Asn Gly His Thr Lys Val Ala Val
260 265 270

Lys Ser Leu Lys Gln Gly Ser Met Ser Pro Asp Ala Phe Leu Ala Glu
275 280 285

Ala Asn Leu Met Lys Gln Leu Gln His Gln Arg Leu Val Arg Leu Tyr
290 295 300

Ala Val Val Thr Gln Glu Pro Ile Tyr Ile Ile Thr Glu Tyr Met Glu
305 310 315 320

Asn Gly Ser Leu Val Asp Phe Leu Lys Thr Pro Ser Gly Ile Lys Leu
325 330 335

Thr Ile Asn Lys Leu Leu Asp Met Ala Ala Gln Ile Ala Glu Gly Met
340 345 350

Ala Phe Ile Glu Glu Arg Asn Tyr Ile His Arg Asp Leu Arg Ala Ala
355 360 365

Asn Ile Leu Val Ser Asp Thr Leu Ser Cys Lys Ile Ala Asp Phe Gly
    370                 375                 380

Leu Ala Arg Leu Ile Glu Asp Asn Glu Tyr Thr Ala Arg Glu Gly Ala
385                 390                 395                 400

Lys Phe Pro Ile Lys Trp Thr Ala Pro Glu Ala Ile Asn Tyr Gly Thr
                405                 410                 415

Phe Thr Ile Lys Ser Asp Val Trp Ser Phe Gly Ile Leu Leu Thr Glu
            420                 425                 430

Ile Val Thr His Gly Arg Ile Pro Tyr Pro Gly Met Thr Asn Pro Glu
        435                 440                 445

Val Ile Gln Asn Leu Glu Arg Gly Tyr Arg Met Val Arg Pro Asp Asn
    450                 455                 460

Cys Pro Glu Glu Leu Tyr Gln Leu Met Arg Leu Cys Trp Lys Glu Arg
465                 470                 475                 480

Pro Glu Asp Arg Pro Thr Phe Asp Tyr Leu Arg Ser Val Leu Glu Asp
                485                 490                 495

Phe Phe Thr Ala Thr Glu Gly Gln Tyr Gln Pro Gln Pro
            500                 505

<210> 63
<211> 4438
<212> DNA
<213> Homo sapiens

<400> 63
ccgtgggacg ggcggaggcg cccgagtccc gcttccccgg cgccccttcca ccccgagccc 60

gactcagccc gcggccacct gcgccccgcc cctgtcggcc gcgcccgagc ccagcgccgc 120

gagccgctcc ccggcgggct ggctcctggc cccggaagcg cgagcgttca cttagcggcg 180

agtggctccg tctccgcgga cagagcgcgc gccccctggc ccggcccgcg aggggctccc 240

ggcgcggtcc ccgagcattt cccgccgggt ggagcgggcc gagcccggca ggatgaccag 300

ccccgcggcc gctcaaagcc gggagatcga ctgtttgagc ccggaagcgc agaagctggc 360

ggaagcccgg ctcgctgcaa aacgggcggc ccgcgcggag gctcgcgaga tccgcatgaa 420

ggagctggag cggcagcaga aggaggaaga cagtgagcgc tactctcgta gatccagaag 480

aaacacatcg gcttctgatg aagacgagcg catgtcagtg ggtagtcgtg gaagcctgag 540

ggtagaagag agaccagaaa aagattttac tgagaagggg tctcgtaaca tgccgggcct 600

gtctgcagcc acgctggcct ctctgggtgg gacttcctct cggagaggca gcggagacac 660

ctccatctcc atcgacaccg aggcatccat cagggaaatc aaggactctc tagcagaagt 720
tgaagagaaa tataagaagg ctatggtttc caatgctcag ctagacaatg aaaagacaaa 780
cttcatgtac caggttgata ccctaaaaga tatgttgctg gagcttgaag aacagctggc 840
tgaatctagg cggcagtacg aagagaaaaa caaagaattt gaaagggaaa aacacgccca 900
cagtatactg caatttcagt ttgctgaagt caaggaggcc ctgaagcaaa gagaggaaat 960
gctcgagaaa catggaataa tcctaaattc agaaatagct accaatggag agacttccga 1020
caccctcaat aatgttggat accaaggtcc taccaagatg acaaaagaag agttaaatgc 1080
cctcaagtcg acaggggatg ggaccctagg aagagccagt gaagtggagg tgaaaaatga 1140
aatcgtggcg aatgtgggga aaagagaaat cttgcacaat actgagaaag aacaacacac 1200
agaggacaca gtgaaggact gtgtggacat agaggtattc cctgctggtg agaataccga 1260
ggaccagaaa tcctctgaag acactgcccc attcctagga accttagcag gtgctaccta 1320
tgaggaacag gttcaaagcc aaattcttga gagcagttct ctccctgaaa acacagtaca 1380
ggttgagtca aatgaggtca tgggtgcacc agatgacagg accagaactc cccttgagcc 1440
atccaactgt tggagtgact tagatggtgg gaaccacaca gagaatgtgg gagaggcagc 1500
agtgactcag gttgaagagc aggcaggcac agtggcctcg tgtcctttag ggcatagtga 1560
tgacacagtt tatcatgatg acaaatgtat ggtagaggtc ccccaagagt tagagacaag 1620
cacagggcat agtttagaga aagaattcac caaccaggaa gcagctgagc ccaaggaggt 1680
tccagcgcac agtacagaag taggtaggga tcacaacgaa gaagagggtg aagaaacagg 1740
attaagggac gagaaaccaa tcaagacaga agttcctggt tctccagcag gaactgaggg 1800
caactgtcag gaagcgacag gtccaagtac agtagacact caaaatgaac ccttagatat 1860
gaaagagccc gatgaagaaa agagtgacca acagggagag gcattggact catcgcagaa 1920
gaagacaaag aacaagaaaa agaaaaacaa gaagaaaaaa tccccagtac ccgtagaaac 1980
ccttaaagat gttaaaaaag agttaacgta tcagaacaca gatttaagtg aaattaagga 2040
agaagagcag gtaaagtcta ctgacagaaa gtcagcagtg gaagcccaaa acgaggtgac 2100
tgaaaatcca aaacagaaaa ttgcagcaga aagcagtgaa aatgttgatt gtccggagaa 2160
tcctaaaatt aagttggatg gaaaacttga ccaagaaggt gatgatgtac aaacagcagc 2220
tgaggaggta ctagctgatg gagacacatt agattttgag gatgacaccg ttcaatcatc 2280
aggcccgagg gctggtggtg aagaattaga tgaaggtgtt gcaaaagata atgctaaaat 2340
agatggtgcc actcaaagca gtcctgcaga accaaagagc gaagacgcag atcgctgcac 2400
cctgcccgaa catgaaagtc cctcacagga cattagtgat gcctgtgaag cagaaagtac 2460
agagaggtgt gagatgtcag aacatccaag tcagaccgtc aggaaagctt tagacagcaa 2520

tagcctagag aacgatgact tgtcggcacc aggaagagag ccagggcact tcaatccaga 2580
aagcagagaa gataccagag gagggaatga gaagggcaaa agcaaagaag actgtaccat 2640
gtcctaagct gaggcaggcg gcaggcgcgg tgcacaggaa gtctcagtgt gaaggggtct 2700
tttctctcca ctgccaatgt aagtagaatg ttctaaattc atagagaggc actgtatgac 2760
aattaccagg tgctctactg ctttaagtta tagactgtta cttgtagatt tccatgtaat 2820
cattgaggtt atcacccaga ttagaaagac atatttgtta tcagtgtacg ttctaattga 2880
gagcattcca gtagtatcaa acaataatgt ctactgttta tagtccactt aataaaaata 2940
gaggcattta ctatttgcct taggctgata ggaatgtggg ttttcttgac caaatatatc 3000
agcatctaat tgaaatgacc aaatagcatt cttagacttc tgtattatga atataattga 3060
tatttaaatt aatgtcttgt tcacatatgt gtactttcat atttgatttt aaaatgtaca 3120
ttataacctg tatggtattt tatttaaagg agataaacag ccaaatagca aataggtcac 3180
tgaatgataa gatttgcacc ttagaacaat aatcatttta aggataacaa gtaaatgtct 3240
gaaagcatga ggggctttat ttgcctttac ctcatatgag tctttgatct tgaaccgata 3300
cttttggatc tcattgttga tatacctgaa tttactttgt aagagatttt aacttcactt 3360
catgctgatg atgtatcaaa ttcattttat agaaagattt aaagtttttt tctggaagtg 3420
atatatgtca aattacattt cctactgcag tatttgagca gggacagtca ttttttaaat 3480
gtttttggcc gggcgtggtg gctcatgcct gtaatctcag tacattggga ggccaaggca 3540
ggtggatcac ctgaggtcaa gagttcgagg ccagcctggc caacatggtg aaaccctgtc 3600
tctactaaaa atacaaaaaa ttggccgggc gtgatggtgg gcgcctgtaa tcccagccac 3660
tccagaggct gaggcaggag aatcgcttga acctgcgagg cagagattgc agtgagccaa 3720
gatcaagcca ttgtactcca gcctggacaa caagagcgaa actctgtcta aaaaaaaaaa 3780
aaaaaacaca cacacacaca acacaatgtt ttcacgcctg taaacctagc acattgggaa 3840
gccaaggtgg gaggattgct tgaggccagg agttcaaggc tgcagtgagc tatgattgca 3900
cactgtactc tagcctggga gacagagtga gacactgtct ctaaaaaaaa aaaaaaaaaa 3960
aaaaaaagtt tttgaacctt aaaatacttt gtttgaattt ctaatcatca ttcaaaagag 4020
cagtaaaaaa tggttacttg ttcttgtaca agctactaat tagactatag taggatattt 4080
taaagagctg aatcactttt ggtattttgg tataaatatt ttcatttgtt atgtcccagt 4140
atattcttac tggaaaattc ttgttttgat ctgcctgaag aaaatatctg ttttctatat 4200
aaaaaaattt tttaaaataa ttgtaaagtt agatttaaaa ttgtaaaata taaaatcaca 4260
aaggaatgta ccttatgaat gttgttgaca ttttatgaaa ttatgtggat tcatattact 4320
gttacaagat agaattgaat gcaaaaagac caaaacctca ataaaatttg aggaaaacgt 4380
gttattatgt aattgaaata aaaacatttt ataattgtgc aaaaaaaaaa aaaaaaaa 4438

<210> 64
<211> 4109
<212> DNA
<213> Homo sapiens

<400> 64
cgggccgggg cggcgcgagc ggctggagca acgggccccg cggcagctgc gggcgacgcg 60

gtcgatggac atgggcaccc agggatcggg gcgcaagcgg ctccccaacc gggagcggct 120

cacggcggag gacgacgcgc tcaaccagat cgcgcgggag gcggaagccc ggctcgctgc 180

aaaacgggcg gcccgcgcgg aggctcgcga gatccgcatg aaggagctgg agcggcagca 240

gaaggagatc tatcaggtcc aaaagaaata ttatgggctg gatacaaaat ggggtgacat 300

cgagcagtgg atggaagaca gtgagcgcta ctctcgtaga tccagaagaa acacatcggc 360

ttctgatgaa gacgagcgca tgtcagtggg tagtcgtgga agcctgaggt cgcagcctga 420

cttggagtat gggggtcctt acgcctggac aaatggttat gatggagaat tgtatggatc 480

acagtccctg aatagaagat ctggcaggcc ctcctgtctg tacagcgctg cccggccttc 540

ggggagttac cgggcgtctg tgttggatga aggcagcttc ggtgggaccc gacggggcag 600

cacctccggc tcccgtgctc cctcggagta cagcggccac ctcaactcca gctcccgcgc 660

ctcctccagg gccagctcgg cccgggccag ccctgtggta gaagagagac cagaaaaaga 720

ttttactgag aaggggtctc gtaacatgcc gggcctgtct gcagccacgc tggcctctct 780

gggtgggact tcctctcgga gaggcagcgg agacacctcc atctccatcg acaccgaggc 840

atccatcagg gaaatcaagg aactcaatga gttaaaggac cagattcagg atgtagaagg 900

caaatacatg cagggattga aagagatgaa ggactctcta gcagaagttg aagagaaata 960

taagaaggct atggtttcca atgctcagct agacaatgaa aagacaaact tcatgtacca 1020

ggttgatacc ctaaaagata tgttgctgga gcttgaagaa cagctggctg aatctaggcg 1080

gcagtacgaa gagaaaaaca aagaatttga aagggaaaaa cacgcccaca gtatactgca 1140

atttcagttt gctgaagtca aggaggccct gaagcaaaga gaggaaatgc tcgaggaaat 1200

ccgacagcta cagcagaaac aggcgagttc tatcagggag atttctgatc ttcaggaaac 1260

aatagagtgg aaagacaaaa agataggggc attagagagg cagaaagagt tctttgattc 1320

cgtaaggagt gaacgggatg atcttagaga agaagtagtc atgctgaaag aggaattaaa 1380

gaaacatgga ataatcctaa attcagaaat agctaccaat ggagagactt ccgacaccct 1440

caataatgtt ggataccaag gtcctaccaa gatgacaaaa gaagagttaa atgccctcaa 1500

gtcgacaggg gatgggaccc tagatattag gttgaaaaag ctggttgatg aacgggaatg 1560

cttattggaa cagattaaga aactcaaagg gcagctggag gagagacaga agattggcaa 1620

actagacaat cttcgatctg aagatgatgt cttggaaaac gggacagaca tgcatgtaat 1680

ggacctacaa agggatgcca acagacagat cagcgacctc aaatttaaac ttgcaaaatc 1740

tgagcaagag ataactgcat tagaacaaaa tgtaataagg ttagagagtc aagtatcacg 1800

ttacaaatca gcggctgaaa atgcagaaaa aatagaagat gaacttaagg cagaaaaacg 1860

gaaactccaa agagagctcc gctctgcatt ggataaaaca gaagagctcg aggtgagcaa 1920

cggccactta gtgaagcgtc tggaaaaaat gaaagcaaat cggagtgcac tcttgtccca 1980

gcagtaaatt ccagctctga tcaggcaact ggttggtgac tggagagcat tgtttcatag 2040

gcttttctct gtcctatctg ggagcgctgc ttcttcccct gccttccgag agacgaagac 2100

cgtggcgagc ttggcgctta ggggctcccg tgccatggct caccccaggg agccccagca 2160

gccaccaggt gcctctgtct gcagacccct ggcccgggct ggcgccgacg ctcagaacct 2220

gcaggtactt cataagcaca caggggcctc gagggagctc tgtgtctgac cgcacagcag 2280

cctctgaatg ccgctggaag tgatgatcaa agtaaagatt cagttgggac ttgagttttt 2340

tttttttttc atgtgtcttg ctgaagatta aggggaaatg ttacagtgtt gggacttcct 2400

ttcatggcag aatctacaat ttgagcgact tcagtagtat ctcttagtct acgcttttca 2460

tacacaaaac actgtggaac cacaagccat taccaagcaa aactctttca ctggaaacaa 2520

gggggcagtc tagaagtaaa agtgacctta agaagactct ttacaggcaa caaatgaagc 2580

ttttctaagg gatttttgca tcagttcagt cataagaata ctttttttcca gggtaattag 2640

gcaatagctt cactgaaaat gacagctttt cattgcatta tttaatcctt atatttggaa 2700

ttgaagtcgt taacttcttt taaagaatgt actattagaa aaattaaaaa tgaaatgttg 2760

agagacttca gcaatgtggt tctaattttt ttccactgag aaagaagatc tttaatttca 2820

tattaatggt tctgtatatt ttgggtcatc tttttatttt ttaagaatat caagtcaatt 2880

catttttctt tccctattta aaaaaaaagg tgttttcaca gaatgagtgc acttaaaaag 2940

tgaagtgaag gaggaggtaa cagtagagac gatggcaata tcatcaagga caaaagtaaa 3000

aacgtttagc tacctgctga tttttagtga ctgttcatat atgttgtatt tcaagtatgg 3060

ctggtgaagc cagtcagctt ttcgggacgt tagcaagtgg aaactgagtc agtatcatcc 3120

aaaaccatat ctagtcttaa cacatggaga atgctggagt gagggttgtg agttcagggt 3180

atataatcaa gaaaacactc ccagcataat gctaggggtc accagtgtcc atcccccaga 3240

actgtatgga tctaggatat acacagctgc gttgcattaa gaaagagatg aaatctctat 3300

taaaatacac aagatttttg tatctccttg tgcagaggat atttgccact gcccattggg 3360

aagcagacaa gttatagggg gctgggggcc aacactggca agtaggaaac cacgggtcgg 3420

acaggtgagc aaaatgtgct ggcaggtggg caccactggg agacccacac tgcacacccg 3480

ggcaccgtat gaacaggaaa gaggaaggaa gctggacgaa gcccctcagg gaccctgtgc 3540

tgaccatgct gggcccaccg gcaaaaggga gatattcagt tccttgtctc atccttaagg 3600

tttcttccac aacatctgaa tacaagcatg tttaactggg aaaatgtcta tgtcatgcgt 3660

gaataacacc agcagcaaac actcacacat cacgcagaca cggccggcag catgctgacg 3720

cttttaggta tttttcactc atgcaatttt cacatatttt cactcatttc atttgcacgg 3780

aaattctatg aggtagatgc tgttatcaaa tccacattac agatgaggga cccagggtcc 3840

aggaaggtga actggcagaa gtctcccagc tggtagaaca gggctgcaag gcatcgattc 3900

ccaggtgtct cacagccctg agaagatggc gttttcccta tcagtggctc tgaggaagtc 3960

aagccttcag tctctacctc tcccaccaat tcttttggaa acagcaaacc aatgttacac 4020

acacttccta atccagagga agctagaaca cgatttttaa atttatttag taaaataaaa 4080

ctttttttgc agatgtaacg aaaaaaaaa 4109

<210> 65
<211> 4285
<212> DNA
<213> Homo sapiens

<400> 65
gcccttccac cccgagcccg actcagcccg cggccacctg cgccccgccc ctgtcggccg 60

cgcccgagcc cagcgccgcg agccgctccc cggcgggctg gctcctggcc ccggaagcgc 120

gagcgttcac ttagcggcga gtggctccgt ctccgcggac agagcgcgcg ccccctggcc 180

cggcccgcga ggggctcccg gcgcggtccc cgagcatttc ccgccgggtg gagcgggccg 240

agcccggcag gatgaccagc cccgcggccg ctcaaagccg ggagatcgac tgtttgagcc 300

cggaagcgca gaagctggcg gaagcccggc tcgctgcaaa acgggcggcc cgcgcggagg 360

ctcgcgagat ccgcatgaag gagctggagc ggcagcagaa ggaggtagaa gagagaccag 420

aaaaagattt tactgagaag gggtctcgta acatgccggg cctgtctgca gccacgctgg 480

cctctctggg tgggacttcc tctcggagag gcagcggaga cacctccatc tccatcgaca 540

ccgaggcatc catcagggaa atcaaggact ctctagcaga agttgaagag aaatataaga 600

aggctatggt ttccaatgct cagctagaca atgaaaagac aaacttcatg taccaggttg 660

ataccctaaa agatatgttg ctggagcttg aagaacagct ggctgaatct aggcggcagt 720

acgaagagaa aaacaaagaa tttgaaaggg aaaaacacgc ccacagtata ctgcaatttc 780

agtttgctga agtcaaggag gccctgaagc aaagagagga aatgctcgag aaacatggaa 840

taatcctaaa ttcagaaata gctaccaatg gagagacttc cgacaccctc aataatgttg 900

gataccaagg tcctaccaag atgacaaaag aagagttaaa tgccctcaag tcgacagggg 960

atgggaccct aggaagagcc agtgaagtgg aggtgaaaaa tgaaatcgtg gcgaatgtgg 1020

ggaaaagaga aatcttgcac aatactgaga aagaacaaca cacagaggac acagtgaagg 1080

actgtgtgga catagaggta ttccctgctg gtgagaatac cgaggaccag aaatcctctg 1140
aagacactgc cccattccta ggaaccttag caggtgctac ctatgaggaa caggttcaaa 1200
gccaaattct tgagagcagt tctctccctg aaaacacagt acaggttgag tcaaatgagg 1260
tcatgggtgc accagatgac aggaccagaa ctccccttga gccatccaac tgttggagtg 1320
acttagatgg tgggaaccac acagagaatg tgggagaggc agcagtgact caggttgaag 1380
agcaggcagg cacagtggcc tcgtgtcctt tagggcatag tgatgacaca gtttatcatg 1440
atgacaaatg tatggtagag gtcccccaag agttagagac aagcacaggg catagtttag 1500
agaaagaatt caccaaccag gaagcagctg agcccaagga ggttccagcg cacagtacag 1560
aagtaggtag ggatcacaac gaagaagagg gtgaagaaac aggattaagg gacgagaaac 1620
caatcaagac agaagttcct ggttctccag caggaactga gggcaactgt caggaagcga 1680
caggtccaag tacagtagac actcaaaatg aacccttaga tatgaaagag cccgatgaag 1740
aaaagagtga ccaacaggga gaggcattgg actcatcgca gaagaagaca aagaacaaga 1800
aaaagaaaaa caagaagaaa aaatccccag tacccgtaga aacccttaaa gatgttaaaa 1860
aagagttaac gtatcagaac acagatttaa gtgaaattaa ggaagaagag caggtaaagt 1920
ctactgacag aaagtcagca gtggaagccc aaaacgaggt gactgaaaat ccaaaacaga 1980
aaattgcagc agaaagcagt gaaaatgttg attgtccgga gaatcctaaa attaagttgg 2040
atggaaaact tgaccaagaa ggtgatgatg tacaaacagc agctgaggag gtactagctg 2100
atggagacac attagatttt gaggatgaca ccgttcaatc atcaggcccg agggctggtg 2160
gtgaagaatt agatgaaggt gttgcaaaag ataatgctaa aatagatggt gccactcaaa 2220
gcagtcctgc agaaccaaag agcgaagacg cagatcgctg caccctgccc gaacatgaaa 2280
gtccctcaca ggacattagt gatgcctgtg aagcagaaag tacagagagg tgtgagatgt 2340
cagaacatcc aagtcagacc gtcaggaaag ctttagacag caatagccta gagaacgatg 2400
acttgtcggc accaggaaga gagccagggc acttcaatcc agaaagcaga gaagatacca 2460
gaggagggaa tgagaagggc aaaagcaaag aagactgtac catgtcctaa gctgaggcag 2520
gcggcaggcg cggtgcacag gaagtctcag tgtgaagggg tcttttctct ccactgccaa 2580
tgtaagtaga atgttctaaa ttcatagaga ggcactgtat gacaattacc aggtgctcta 2640
ctgctttaag ttatagactg ttacttgtag atttccatgt aatcattgag gttatcaccc 2700
agattagaaa gacatatttg ttatcagtgt acgttctaat tgagagcatt ccagtagtat 2760
caaacaataa tgtctactgt ttatagtcca cttaataaaa atagaggcat ttactatttg 2820
ccttaggctg ataggaatgt gggttttctt gaccaaatat atcagcatct aattgaaatg 2880
accaaatagc attcttagac ttctgtatta tgaatataat tgatatttaa attaatgtct 2940
tgttcacata tgtgtacttt catatttgat tttaaaatgt acattataac ctgtatggta 3000

ttttatttaa aggagataaa cagccaaata gcaaataggt cactgaatga taagatttgc 3060

accttagaac aataatcatt ttaaggataa caagtaaatg tctgaaagca tgaggggctt 3120

tatttgcctt tacctcatat gagtctttga tcttgaaccg atacttttgg atctcattgt 3180

tgatatacct gaatttactt tgtaagagat tttaacttca cttcatgctg atgatgtatc 3240

aaattcattt tatagaaaga tttaaagttt ttttctggaa gtgatatatg tcaaattaca 3300

tttcctactg cagtatttga gcagggacag tcattttta aatgtttttg gccgggcgtg 3360

gtggctcatg cctgtaatct cagtacattg ggaggccaag gcaggtggat cacctgaggt 3420

caagagttcg aggccagcct ggccaacatg gtgaaaccct gtctctacta aaaatacaaa 3480

aaattggccg ggcgtgatgg tgggcgcctg taatcccagc cactccagag gctgaggcag 3540

gagaatcgct tgaacctgcg aggcagagat tgcagtgagc caagatcaag ccattgtact 3600

ccagcctgga caacaagagc gaaactctgt ctaaaaaaaa aaaaaaaaac acacacacac 3660

acaacacaat gttttcacgc ctgtaaacct agcacattgg gaagccaagg tgggaggatt 3720

gcttgaggcc aggagttcaa ggctgcagtg agctatgatt gcacactgta ctctagcctg 3780

ggagacagag tgagacactg tctctaaaaa aaaaaaaaaa aaaaaaaaaa gtttttgaac 3840

cttaaaatac tttgtttgaa tttctaatca tcattcaaaa gagcagtaaa aaatggttac 3900

ttgttcttgt acaagctact aattagacta tagtaggata ttttaaagag ctgaatcact 3960

tttggtattt tggtataaat attttcattt gttatgtccc agtatattct tactggaaaa 4020

ttcttgtttt gatctgcctg aagaaaatat ctgttttcta tataaaaaaa ttttttaaaa 4080

taattgtaaa gttagattta aaattgtaaa atataaaatc acaaaggaat gtaccttatg 4140

aatgttgttg acattttatg aaattatgtg gattcatatt actgttacaa gatagaattg 4200

aatgcaaaaa gaccaaaacc tcaataaaat ttgaggaaaa cgtgttatta tgtaattgaa 4260

ataaaaacat tttataattg tgcaa 4285

<210> 66
<211> 4453
<212> DNA
<213> Homo sapiens

<400> 66
gcccttccac cccgagcccg actcagcccg cggccacctg cgcccccgccc ctgtcggccg 60

cgcccgagcc cagcgccgcg agccgctccc cggcgggctg gctcctggcc ccggaagcgc 120

gagcgttcac ttagcggcga gtggctccgt ctccgcggac agagcgcgcg ccccctggcc 180

cggcccgcga ggggctcccg gcgcggtccc cgagcatttc ccgccgggtg gagcgggccg 240

agcccggcag gatgaccagc cccgcggccg ctcaaagccg ggagatcgac tgtttgagcc 300

cggaagcgca gaagctggcg gaagcccggc tcgctgcaaa acgggcggcc cgcgcggagg 360

ctcgcgagat ccgcatgaag gagctggagc ggcagcagaa ggaggaagac agtgagcgct 420

actctcgtag atccagaaga aacacatcgg cttctgatga agacgagcgc atgtcagtgg 480

gtagtcgtgg aagcctgagg gtagaagaga gaccagaaaa agattttact gagaaggggt 540

ctcgtaacat gccgggcctg tctgcagcca cgctggcctc tctgggtggg acttcctctc 600

ggagaggcag cggagacacc tccatctcca tcgacaccga ggcatccatc agggaaatca 660

aggaactcaa tgagttaaag gaccagattc aggatgtaga aggcaaatac atgcagggat 720

tgaaagagat gaaggactct ctagcagaag ttgaagagaa atataagaag gctatggttt 780

ccaatgctca gctagacaat gaaaagacaa acttcatgta ccaggttgat accctaaaag 840

atatgttgct ggagcttgaa gaacagctgg ctgaatctag gcggcagtac gaagagaaaa 900

acaaagaatt tgaaagggaa aaacacgccc acagtatact gcaatttcag tttgctgaag 960

tcaaggaggc cctgaagcaa agagaggaaa tgctcgagaa acatggaata atcctaaatt 1020

cagaaatagc taccaatgga gagacttccg acaccctcaa taatgttgga taccaaggtc 1080

ctaccaagat gacaaaagaa gagttaaatg ccctcaagtc gacaggggat gggaccctag 1140

gaagagccag tgaagtggag gtgaaaaatg aaatcgtggc gaatgtgggg aaaagagaaa 1200

tcttgcacaa tactgagaaa gaacaacaca cagaggacac agtgaaggac tgtgtggaca 1260

tagaggtatt ccctgctggt gagaataccg aggaccagaa atcctctgaa gacactgccc 1320

cattcctagg aaccttagca ggtgctacct atgaggaaca ggttcaaagc caaattcttg 1380

agagcagttc tctccctgaa aacacagtac aggttgagtc aaatgaggtc atgggtgcac 1440

cagatgacag gaccagaact ccccttgagc catccaactg ttggagtgac ttagatggtg 1500

ggaaccacac agagaatgtg ggagaggcag cagtgactca ggttgaagag caggcaggca 1560

cagtggcctc gtgtccttta gggcatagtg atgacacagt ttatcatgat gacaaatgta 1620

tggtagaggt cccccaagag ttagagacaa gcacagggca tagtttagag aaagaattca 1680

ccaaccagga agcagctgag cccaaggagg ttccagcgca cagtacagaa gtaggtaggg 1740

atcacaacga agaagagggt gaagaaacag gattaaggga cgagaaacca atcaagacag 1800

aagttcctgg ttctccagca ggaactgagg gcaactgtca ggaagcgaca ggtccaagta 1860

cagtagacac tcaaaatgaa cccttagata tgaaagagcc cgatgaagaa aagagtgacc 1920

aacagggaga ggcattggac tcatcgcaga agaagacaaa gaacaagaaa aagaaaaaca 1980

agaagaaaaa atccccagta cccgtagaaa cccttaaaga tgttaaaaaa gagttaacgt 2040

atcagaacac agatttaagt gaaattaagg aagaagagca ggtaaagtct actgacagaa 2100

agtcagcagt ggaagcccaa aacgaggtga ctgaaaatcc aaaacagaaa attgcagcag 2160

aaagcagtga aaatgttgat tgtccggaga atcctaaaat taagttggat ggaaaacttg 2220

accaagaagg tgatgatgta caaacagcag ctgaggaggt actagctgat ggagacacat 2280
tagattttga ggatgacacc gttcaatcat caggcccgag ggctggtggt gaagaattag 2340
atgaaggtgt tgcaaaagat aatgctaaaa tagatggtgc cactcaaagc agtcctgcag 2400
aaccaaagag cgaagacgca gatcgctgca ccctgcccga acatgaaagt ccctcacagg 2460
acattagtga tgcctgtgaa gcagaaagta cagagaggtg tgagatgtca gaacatccaa 2520
gtcagaccgt caggaaagct ttagacagca atagcctaga gaacgatgac ttgtcggcac 2580
caggaagaga gccagggcac ttcaatccag aaagcagaga agataccaga ggagggaatg 2640
agaagggcaa aagcaaagaa gactgtacca tgtcctaagc tgaggcaggc ggcaggcgcg 2700
gtgcacagga agtctcagtg tgaaggggtc ttttctctcc actgccaatg taagtagaat 2760
gttctaaatt catagagagg cactgtatga caattaccag gtgctctact gctttaagtt 2820
atagactgtt acttgtagat ttccatgtaa tcattgaggt tatcacccag attagaaaga 2880
catatttgtt atcagtgtac gttctaattg agagcattcc agtagtatca aacaataatg 2940
tctactgttt atagtccact taataaaaat agaggcattt actatttgcc ttaggctgat 3000
aggaatgtgg gttttcttga ccaaatatat cagcatctaa ttgaaatgac caaatagcat 3060
tcttagactt ctgtattatg aatataattg atatttaaat taatgtcttg ttcacatatg 3120
tgtactttca tatttgattt taaaatgtac attataacct gtatggtatt ttatttaaag 3180
gagataaaca gccaaatagc aaataggtca ctgaatgata agatttgcac cttagaacaa 3240
taatcatttt aaggataaca agtaaatgtc tgaaagcatg aggggcttta tttgccttta 3300
cctcatatga gtctttgatc ttgaaccgat acttttggat ctcattgttg atatacctga 3360
atttactttg taagagattt taacttcact tcatgctgat gatgtatcaa attcatttta 3420
tagaaagatt taaagttttt ttctggaagt gatatatgtc aaattacatt tcctactgca 3480
gtatttgagc agggacagtc attttttaaa tgtttttggc cgggcgtggt ggctcatgcc 3540
tgtaatctca gtacattggg aggccaaggc aggtggatca cctgaggtca agagttcgag 3600
gccagcctgg ccaacatggt gaaaccctgt ctctactaaa aatacaaaaa attggccggg 3660
cgtgatggtg ggcgcctgta atcccagcca ctccagaggc tgaggcagga gaatcgcttg 3720
aacctgcgag gcagagattg cagtgagcca agatcaagcc attgtactcc agcctggaca 3780
acaagagcga aactctgtct aaaaaaaaaa aaaaaaacac acacacacac aacacaatgt 3840
tttcacgcct gtaaacctag cacattggga agccaaggtg ggaggattgc ttgaggccag 3900
gagttcaagg ctgcagtgag ctatgattgc acactgtact ctagcctggg agacagagtg 3960
agacactgtc tctaaaaaaa aaaaaaaaaa aaaaaaaagt ttttgaacct taaaatactt 4020
tgtttgaatt tctaatcatc attcaaaaga gcagtaaaaa atggttactt gttcttgtac 4080
aagctactaa ttagactata gtaggatatt ttaaagagct gaatcacttt tggtattttg 4140

gtataaatat tttcatttgt tatgtcccag tatattctta ctggaaaatt cttgttttga 4200

tctgcctgaa gaaaatatct gttttctata taaaaaaatt ttttaaaata attgtaaagt 4260

tagatttaaa attgtaaaat ataaaatcac aaaggaatgt accttatgaa tgttgttgac 4320

attttatgaa attatgtgga ttcatattac tgttacaaga tagaattgaa tgcaaaaaga 4380

ccaaaacctc aataaaattt gaggaaaacg tgttattatg taattgaaat aaaaacattt 4440

tataattgtg caa 4453

<210> 67
<211> 784
<212> PRT
<213> Homo sapiens

<400> 67

Met Thr Ser Pro Ala Ala Ala Gln Ser Arg Glu Ile Asp Cys Leu Ser
1 5 10 15

Pro Glu Ala Gln Lys Leu Ala Glu Ala Arg Leu Ala Ala Lys Arg Ala
20 25 30

Ala Arg Ala Glu Ala Arg Glu Ile Arg Met Lys Glu Leu Glu Arg Gln
35 40 45

Gln Lys Glu Glu Asp Ser Glu Arg Tyr Ser Arg Arg Ser Arg Arg Asn
50 55 60

Thr Ser Ala Ser Asp Glu Asp Glu Arg Met Ser Val Gly Ser Arg Gly
65 70 75 80

Ser Leu Arg Val Glu Glu Arg Pro Glu Lys Asp Phe Thr Glu Lys Gly
85 90 95

Ser Arg Asn Met Pro Gly Leu Ser Ala Ala Thr Leu Ala Ser Leu Gly
100 105 110

Gly Thr Ser Ser Arg Arg Gly Ser Gly Asp Thr Ser Ile Ser Ile Asp
115 120 125

Thr Glu Ala Ser Ile Arg Glu Ile Lys Asp Ser Leu Ala Glu Val Glu
130 135 140

Glu Lys Tyr Lys Lys Ala Met Val Ser Asn Ala Gln Leu Asp Asn Glu
145 150 155 160

Lys Thr Asn Phe Met Tyr Gln Val Asp Thr Leu Lys Asp Met Leu Leu
165 170 175

Glu Leu Glu Glu Gln Leu Ala Glu Ser Arg Arg Gln Tyr Glu Glu Lys
180 185 190

Asn Lys Glu Phe Glu Arg Glu Lys His Ala His Ser Ile Leu Gln Phe
195 200 205

Gln Phe Ala Glu Val Lys Glu Ala Leu Lys Gln Arg Glu Glu Met Leu
210 215 220

Glu Lys His Gly Ile Ile Leu Asn Ser Glu Ile Ala Thr Asn Gly Glu
225 230 235 240

Thr Ser Asp Thr Leu Asn Asn Val Gly Tyr Gln Gly Pro Thr Lys Met
245 250 255

Thr Lys Glu Glu Leu Asn Ala Leu Lys Ser Thr Gly Asp Gly Thr Leu
260 265 270

Gly Arg Ala Ser Glu Val Glu Val Lys Asn Glu Ile Val Ala Asn Val
275 280 285

Gly Lys Arg Glu Ile Leu His Asn Thr Glu Lys Glu Gln His Thr Glu
290 295 300

Asp Thr Val Lys Asp Cys Val Asp Ile Glu Val Phe Pro Ala Gly Glu
305 310 315 320

Asn Thr Glu Asp Gln Lys Ser Ser Glu Asp Thr Ala Pro Phe Leu Gly
325 330 335

Thr Leu Ala Gly Ala Thr Tyr Glu Glu Gln Val Gln Ser Gln Ile Leu
340 345 350

Glu Ser Ser Ser Leu Pro Glu Asn Thr Val Gln Val Glu Ser Asn Glu
355 360 365

Val Met Gly Ala Pro Asp Asp Arg Thr Arg Thr Pro Leu Glu Pro Ser
370 375 380

Asn Cys Trp Ser Asp Leu Asp Gly Gly Asn His Thr Glu Asn Val Gly
385 390 395 400

Glu Ala Ala Val Thr Gln Val Glu Glu Gln Ala Gly Thr Val Ala Ser
405 410 415

Cys Pro Leu Gly His Ser Asp Asp Thr Val Tyr His Asp Asp Lys Cys

420 425 430

Met Val Glu Val Pro Gln Glu Leu Glu Thr Ser Thr Gly His Ser Leu
435 440 445

Glu Lys Glu Phe Thr Asn Gln Glu Ala Ala Glu Pro Lys Glu Val Pro
450 455 460

Ala His Ser Thr Glu Val Gly Arg Asp His Asn Glu Glu Glu Gly Glu
465 470 475 480

Glu Thr Gly Leu Arg Asp Glu Lys Pro Ile Lys Thr Glu Val Pro Gly
485 490 495

Ser Pro Ala Gly Thr Glu Gly Asn Cys Gln Glu Ala Thr Gly Pro Ser
500 505 510

Thr Val Asp Thr Gln Asn Glu Pro Leu Asp Met Lys Glu Pro Asp Glu
515 520 525

Glu Lys Ser Asp Gln Gln Gly Glu Ala Leu Asp Ser Ser Gln Lys Lys
530 535 540

Thr Lys Asn Lys Lys Lys Lys Asn Lys Lys Lys Lys Ser Pro Val Pro
545 550 555 560

Val Glu Thr Leu Lys Asp Val Lys Lys Glu Leu Thr Tyr Gln Asn Thr
565 570 575

Asp Leu Ser Glu Ile Lys Glu Glu Glu Gln Val Lys Ser Thr Asp Arg
580 585 590

Lys Ser Ala Val Glu Ala Gln Asn Glu Val Thr Glu Asn Pro Lys Gln
595 600 605

Lys Ile Ala Ala Glu Ser Ser Glu Asn Val Asp Cys Pro Glu Asn Pro
610 615 620

Lys Ile Lys Leu Asp Gly Lys Leu Asp Gln Glu Gly Asp Asp Val Gln
625 630 635 640

Thr Ala Ala Glu Glu Val Leu Ala Asp Gly Asp Thr Leu Asp Phe Glu
645 650 655

Asp Asp Thr Val Gln Ser Ser Gly Pro Arg Ala Gly Gly Glu Glu Leu
660 665 670

Asp Glu Gly Val Ala Lys Asp Asn Ala Lys Ile Asp Gly Ala Thr Gln
675 680 685

Ser Ser Pro Ala Glu Pro Lys Ser Glu Asp Ala Asp Arg Cys Thr Leu
690 695 700

Pro Glu His Glu Ser Pro Ser Gln Asp Ile Ser Asp Ala Cys Glu Ala
705 710 715 720

Glu Ser Thr Glu Arg Cys Glu Met Ser Glu His Pro Ser Gln Thr Val
725 730 735

Arg Lys Ala Leu Asp Ser Asn Ser Leu Glu Asn Asp Asp Leu Ser Ala
740 745 750

Pro Gly Arg Glu Pro Gly His Phe Asn Pro Glu Ser Arg Glu Asp Thr
755 760 765

Arg Gly Gly Asn Glu Lys Gly Lys Ser Lys Glu Asp Cys Thr Met Ser
770 775 780

<210> 68
<211> 640
<212> PRT
<213> Homo sapiens

<400> 68

Met Asp Met Gly Thr Gln Gly Ser Gly Arg Lys Arg Leu Pro Asn Arg
1 5 10 15

Glu Arg Leu Thr Ala Glu Asp Asp Ala Leu Asn Gln Ile Ala Arg Glu
20 25 30

Ala Glu Ala Arg Leu Ala Ala Lys Arg Ala Ala Arg Ala Glu Ala Arg
35 40 45

Glu Ile Arg Met Lys Glu Leu Glu Arg Gln Gln Lys Glu Ile Tyr Gln
50 55 60

Val Gln Lys Lys Tyr Tyr Gly Leu Asp Thr Lys Trp Gly Asp Ile Glu
65 70 75 80

Gln Trp Met Glu Asp Ser Glu Arg Tyr Ser Arg Arg Ser Arg Arg Asn
85 90 95

Thr Ser Ala Ser Asp Glu Asp Glu Arg Met Ser Val Gly Ser Arg Gly
100 105 110

Ser Leu Arg Ser Gln Pro Asp Leu Glu Tyr Gly Gly Pro Tyr Ala Trp
        115                 120                 125

Thr Asn Gly Tyr Asp Gly Glu Leu Tyr Gly Ser Gln Ser Leu Asn Arg
    130                 135                 140

Arg Ser Gly Arg Pro Ser Cys Leu Tyr Ser Ala Ala Arg Pro Ser Gly
145                 150                 155                 160

Ser Tyr Arg Ala Ser Val Leu Asp Glu Gly Ser Phe Gly Gly Thr Arg
                165                 170                 175

Arg Gly Ser Thr Ser Gly Ser Arg Ala Pro Ser Glu Tyr Ser Gly His
            180                 185                 190

Leu Asn Ser Ser Ser Arg Ala Ser Ser Arg Ala Ser Ser Ala Arg Ala
        195                 200                 205

Ser Pro Val Val Glu Glu Arg Pro Glu Lys Asp Phe Thr Glu Lys Gly
    210                 215                 220

Ser Arg Asn Met Pro Gly Leu Ser Ala Ala Thr Leu Ala Ser Leu Gly
225                 230                 235                 240

Gly Thr Ser Ser Arg Arg Gly Ser Gly Asp Thr Ser Ile Ser Ile Asp
                245                 250                 255

Thr Glu Ala Ser Ile Arg Glu Ile Lys Glu Leu Asn Glu Leu Lys Asp
            260                 265                 270

Gln Ile Gln Asp Val Glu Gly Lys Tyr Met Gln Gly Leu Lys Glu Met
        275                 280                 285

Lys Asp Ser Leu Ala Glu Val Glu Glu Lys Tyr Lys Lys Ala Met Val
    290                 295                 300

Ser Asn Ala Gln Leu Asp Asn Glu Lys Thr Asn Phe Met Tyr Gln Val
305                 310                 315                 320

Asp Thr Leu Lys Asp Met Leu Leu Glu Leu Glu Glu Gln Leu Ala Glu
                325                 330                 335

Ser Arg Arg Gln Tyr Glu Glu Lys Asn Lys Glu Phe Glu Arg Glu Lys
            340                 345                 350

His Ala His Ser Ile Leu Gln Phe Gln Phe Ala Glu Val Lys Glu Ala
        355                 360                 365

```
Leu Lys Gln Arg Glu Glu Met Leu Glu Glu Ile Arg Gln Leu Gln Gln
    370                 375                 380

Lys Gln Ala Ser Ser Ile Arg Glu Ile Ser Asp Leu Gln Glu Thr Ile
385                 390                 395                 400

Glu Trp Lys Asp Lys Lys Ile Gly Ala Leu Glu Arg Gln Lys Glu Phe
                405                 410                 415

Phe Asp Ser Val Arg Ser Glu Arg Asp Asp Leu Arg Glu Glu Val Val
            420                 425                 430

Met Leu Lys Glu Glu Leu Lys Lys His Gly Ile Ile Leu Asn Ser Glu
        435                 440                 445

Ile Ala Thr Asn Gly Glu Thr Ser Asp Thr Leu Asn Asn Val Gly Tyr
    450                 455                 460

Gln Gly Pro Thr Lys Met Thr Lys Glu Glu Leu Asn Ala Leu Lys Ser
465                 470                 475                 480

Thr Gly Asp Gly Thr Leu Asp Ile Arg Leu Lys Lys Leu Val Asp Glu
                485                 490                 495

Arg Glu Cys Leu Leu Glu Gln Ile Lys Lys Leu Lys Gly Gln Leu Glu
            500                 505                 510

Glu Arg Gln Lys Ile Gly Lys Leu Asp Asn Leu Arg Ser Glu Asp Asp
        515                 520                 525

Val Leu Glu Asn Gly Thr Asp Met His Val Met Asp Leu Gln Arg Asp
    530                 535                 540

Ala Asn Arg Gln Ile Ser Asp Leu Lys Phe Lys Leu Ala Lys Ser Glu
545                 550                 555                 560

Gln Glu Ile Thr Ala Leu Glu Gln Asn Val Ile Arg Leu Glu Ser Gln
                565                 570                 575

Val Ser Arg Tyr Lys Ser Ala Ala Glu Asn Ala Glu Lys Ile Glu Asp
            580                 585                 590

Glu Leu Lys Ala Glu Lys Arg Lys Leu Gln Arg Glu Leu Arg Ser Ala
        595                 600                 605

Leu Asp Lys Thr Glu Glu Leu Glu Val Ser Asn Gly His Leu Val Lys
    610                 615                 620
```

Arg Leu Glu Lys Met Lys Ala Asn Arg Ser Ala Leu Leu Ser Gln Gln
625                 630                 635                 640

<210>  69
<211>  752
<212>  PRT
<213>  Homo sapiens

<400>  69

Met Thr Ser Pro Ala Ala Ala Gln Ser Arg Glu Ile Asp Cys Leu Ser
1               5                   10                  15

Pro Glu Ala Gln Lys Leu Ala Glu Ala Arg Leu Ala Ala Lys Arg Ala
            20                  25                  30

Ala Arg Ala Glu Ala Arg Glu Ile Arg Met Lys Glu Leu Glu Arg Gln
        35                  40                  45

Gln Lys Glu Val Glu Glu Arg Pro Glu Lys Asp Phe Thr Glu Lys Gly
    50                  55                  60

Ser Arg Asn Met Pro Gly Leu Ser Ala Ala Thr Leu Ala Ser Leu Gly
65                  70                  75                  80

Gly Thr Ser Ser Arg Arg Gly Ser Gly Asp Thr Ser Ile Ser Ile Asp
                85                  90                  95

Thr Glu Ala Ser Ile Arg Glu Ile Lys Asp Ser Leu Ala Glu Val Glu
            100                 105                 110

Glu Lys Tyr Lys Lys Ala Met Val Ser Asn Ala Gln Leu Asp Asn Glu
        115                 120                 125

Lys Thr Asn Phe Met Tyr Gln Val Asp Thr Leu Lys Asp Met Leu Leu
    130                 135                 140

Glu Leu Glu Glu Gln Leu Ala Glu Ser Arg Arg Gln Tyr Glu Glu Lys
145                 150                 155                 160

Asn Lys Glu Phe Glu Arg Glu Lys His Ala His Ser Ile Leu Gln Phe
                165                 170                 175

Gln Phe Ala Glu Val Lys Glu Ala Leu Lys Gln Arg Glu Glu Met Leu
            180                 185                 190

Glu Lys His Gly Ile Ile Leu Asn Ser Glu Ile Ala Thr Asn Gly Glu
        195                 200                 205

Thr Ser Asp Thr Leu Asn Asn Val Gly Tyr Gln Gly Pro Thr Lys Met
210 215 220

Thr Lys Glu Glu Leu Asn Ala Leu Lys Ser Thr Gly Asp Gly Thr Leu
225 230 235 240

Gly Arg Ala Ser Glu Val Glu Val Lys Asn Glu Ile Val Ala Asn Val
245 250 255

Gly Lys Arg Glu Ile Leu His Asn Thr Glu Lys Glu Gln His Thr Glu
260 265 270

Asp Thr Val Lys Asp Cys Val Asp Ile Glu Val Phe Pro Ala Gly Glu
275 280 285

Asn Thr Glu Asp Gln Lys Ser Ser Glu Asp Thr Ala Pro Phe Leu Gly
290 295 300

Thr Leu Ala Gly Ala Thr Tyr Glu Glu Gln Val Gln Ser Gln Ile Leu
305 310 315 320

Glu Ser Ser Ser Leu Pro Glu Asn Thr Val Gln Val Glu Ser Asn Glu
325 330 335

Val Met Gly Ala Pro Asp Asp Arg Thr Arg Thr Pro Leu Glu Pro Ser
340 345 350

Asn Cys Trp Ser Asp Leu Asp Gly Gly Asn His Thr Glu Asn Val Gly
355 360 365

Glu Ala Ala Val Thr Gln Val Glu Glu Gln Ala Gly Thr Val Ala Ser
370 375 380

Cys Pro Leu Gly His Ser Asp Asp Thr Val Tyr His Asp Asp Lys Cys
385 390 395 400

Met Val Glu Val Pro Gln Glu Leu Glu Thr Ser Thr Gly His Ser Leu
405 410 415

Glu Lys Glu Phe Thr Asn Gln Glu Ala Ala Glu Pro Lys Glu Val Pro
420 425 430

Ala His Ser Thr Glu Val Gly Arg Asp His Asn Glu Glu Glu Gly Glu
435 440 445

Glu Thr Gly Leu Arg Asp Glu Lys Pro Ile Lys Thr Glu Val Pro Gly

450 455 460

Ser Pro Ala Gly Thr Glu Gly Asn Cys Gln Glu Ala Thr Gly Pro Ser
465 470 475 480

Thr Val Asp Thr Gln Asn Glu Pro Leu Asp Met Lys Glu Pro Asp Glu
485 490 495

Glu Lys Ser Asp Gln Gln Gly Glu Ala Leu Asp Ser Ser Gln Lys Lys
500 505 510

Thr Lys Asn Lys Lys Lys Lys Asn Lys Lys Lys Lys Ser Pro Val Pro
515 520 525

Val Glu Thr Leu Lys Asp Val Lys Lys Glu Leu Thr Tyr Gln Asn Thr
530 535 540

Asp Leu Ser Glu Ile Lys Glu Glu Glu Gln Val Lys Ser Thr Asp Arg
545 550 555 560

Lys Ser Ala Val Glu Ala Gln Asn Glu Val Thr Glu Asn Pro Lys Gln
565 570 575

Lys Ile Ala Ala Glu Ser Ser Glu Asn Val Asp Cys Pro Glu Asn Pro
580 585 590

Lys Ile Lys Leu Asp Gly Lys Leu Asp Gln Glu Gly Asp Asp Val Gln
595 600 605

Thr Ala Ala Glu Glu Val Leu Ala Asp Gly Asp Thr Leu Asp Phe Glu
610 615 620

Asp Asp Thr Val Gln Ser Ser Gly Pro Arg Ala Gly Gly Glu Glu Leu
625 630 635 640

Asp Glu Gly Val Ala Lys Asp Asn Ala Lys Ile Asp Gly Ala Thr Gln
645 650 655

Ser Ser Pro Ala Glu Pro Lys Ser Glu Asp Ala Asp Arg Cys Thr Leu
660 665 670

Pro Glu His Glu Ser Pro Ser Gln Asp Ile Ser Asp Ala Cys Glu Ala
675 680 685

Glu Ser Thr Glu Arg Cys Glu Met Ser Glu His Pro Ser Gln Thr Val
690 695 700

Arg Lys Ala Leu Asp Ser Asn Ser Leu Glu Asn Asp Asp Leu Ser Ala
705 710 715 720

Pro Gly Arg Glu Pro Gly His Phe Asn Pro Glu Ser Arg Glu Asp Thr
725 730 735

Arg Gly Gly Asn Glu Lys Gly Lys Ser Lys Glu Asp Cys Thr Met Ser
740 745 750

<210> 70
<211> 808
<212> PRT
<213> Homo sapiens

<400> 70

Met Thr Ser Pro Ala Ala Ala Gln Ser Arg Glu Ile Asp Cys Leu Ser
1 5 10 15

Pro Glu Ala Gln Lys Leu Ala Glu Ala Arg Leu Ala Ala Lys Arg Ala
20 25 30

Ala Arg Ala Glu Ala Arg Glu Ile Arg Met Lys Glu Leu Glu Arg Gln
35 40 45

Gln Lys Glu Glu Asp Ser Glu Arg Tyr Ser Arg Arg Ser Arg Arg Asn
50 55 60

Thr Ser Ala Ser Asp Glu Asp Glu Arg Met Ser Val Gly Ser Arg Gly
65 70 75 80

Ser Leu Arg Val Glu Glu Arg Pro Glu Lys Asp Phe Thr Glu Lys Gly
85 90 95

Ser Arg Asn Met Pro Gly Leu Ser Ala Ala Thr Leu Ala Ser Leu Gly
100 105 110

Gly Thr Ser Ser Arg Arg Gly Ser Gly Asp Thr Ser Ile Ser Ile Asp
115 120 125

Thr Glu Ala Ser Ile Arg Glu Ile Lys Glu Leu Asn Glu Leu Lys Asp
130 135 140

Gln Ile Gln Asp Val Glu Gly Lys Tyr Met Gln Gly Leu Lys Glu Met
145 150 155 160

Lys Asp Ser Leu Ala Glu Val Glu Glu Lys Tyr Lys Lys Ala Met Val
165 170 175

Ser Asn Ala Gln Leu Asp Asn Glu Lys Thr Asn Phe Met Tyr Gln Val
180 185 190

Asp Thr Leu Lys Asp Met Leu Leu Glu Leu Glu Glu Gln Leu Ala Glu
195 200 205

Ser Arg Arg Gln Tyr Glu Glu Lys Asn Lys Glu Phe Glu Arg Glu Lys
210 215 220

His Ala His Ser Ile Leu Gln Phe Gln Phe Ala Glu Val Lys Glu Ala
225 230 235 240

Leu Lys Gln Arg Glu Glu Met Leu Glu Lys His Gly Ile Ile Leu Asn
245 250 255

Ser Glu Ile Ala Thr Asn Gly Glu Thr Ser Asp Thr Leu Asn Asn Val
260 265 270

Gly Tyr Gln Gly Pro Thr Lys Met Thr Lys Glu Glu Leu Asn Ala Leu
275 280 285

Lys Ser Thr Gly Asp Gly Thr Leu Gly Arg Ala Ser Glu Val Glu Val
290 295 300

Lys Asn Glu Ile Val Ala Asn Val Gly Lys Arg Glu Ile Leu His Asn
305 310 315 320

Thr Glu Lys Glu Gln His Thr Glu Asp Thr Val Lys Asp Cys Val Asp
325 330 335

Ile Glu Val Phe Pro Ala Gly Glu Asn Thr Glu Asp Gln Lys Ser Ser
340 345 350

Glu Asp Thr Ala Pro Phe Leu Gly Thr Leu Ala Gly Ala Thr Tyr Glu
355 360 365

Glu Gln Val Gln Ser Gln Ile Leu Glu Ser Ser Ser Leu Pro Glu Asn
370 375 380

Thr Val Gln Val Glu Ser Asn Glu Val Met Gly Ala Pro Asp Asp Arg
385 390 395 400

Thr Arg Thr Pro Leu Glu Pro Ser Asn Cys Trp Ser Asp Leu Asp Gly
405 410 415

Gly Asn His Thr Glu Asn Val Gly Glu Ala Ala Val Thr Gln Val Glu
420 425 430

```
Glu Gln Ala Gly Thr Val Ala Ser Cys Pro Leu Gly His Ser Asp Asp
        435                 440                 445

Thr Val Tyr His Asp Asp Lys Cys Met Val Glu Val Pro Gln Glu Leu
    450                 455                 460

Glu Thr Ser Thr Gly His Ser Leu Glu Lys Glu Phe Thr Asn Gln Glu
465                 470                 475                 480

Ala Ala Glu Pro Lys Glu Val Pro Ala His Ser Thr Glu Val Gly Arg
                485                 490                 495

Asp His Asn Glu Glu Glu Gly Glu Glu Thr Gly Leu Arg Asp Glu Lys
            500                 505                 510

Pro Ile Lys Thr Glu Val Pro Gly Ser Pro Ala Gly Thr Glu Gly Asn
        515                 520                 525

Cys Gln Glu Ala Thr Gly Pro Ser Thr Val Asp Thr Gln Asn Glu Pro
    530                 535                 540

Leu Asp Met Lys Glu Pro Asp Glu Glu Lys Ser Asp Gln Gln Gly Glu
545                 550                 555                 560

Ala Leu Asp Ser Ser Gln Lys Lys Thr Lys Asn Lys Lys Lys Lys Asn
                565                 570                 575

Lys Lys Lys Lys Ser Pro Val Pro Val Glu Thr Leu Lys Asp Val Lys
            580                 585                 590

Lys Glu Leu Thr Tyr Gln Asn Thr Asp Leu Ser Glu Ile Lys Glu Glu
        595                 600                 605

Glu Gln Val Lys Ser Thr Asp Arg Lys Ser Ala Val Glu Ala Gln Asn
    610                 615                 620

Glu Val Thr Glu Asn Pro Lys Gln Lys Ile Ala Ala Glu Ser Ser Glu
625                 630                 635                 640

Asn Val Asp Cys Pro Glu Asn Pro Lys Ile Lys Leu Asp Gly Lys Leu
                645                 650                 655

Asp Gln Glu Gly Asp Asp Val Gln Thr Ala Ala Glu Glu Val Leu Ala
            660                 665                 670

Asp Gly Asp Thr Leu Asp Phe Glu Asp Asp Thr Val Gln Ser Ser Gly
        675                 680                 685
```

Pro Arg Ala Gly Gly Glu Glu Leu Asp Glu Gly Val Ala Lys Asp Asn
    690                 695                 700

Ala Lys Ile Asp Gly Ala Thr Gln Ser Ser Pro Ala Glu Pro Lys Ser
705                 710                 715                 720

Glu Asp Ala Asp Arg Cys Thr Leu Pro Glu His Glu Ser Pro Ser Gln
                725                 730                 735

Asp Ile Ser Asp Ala Cys Glu Ala Glu Ser Thr Glu Arg Cys Glu Met
            740                 745                 750

Ser Glu His Pro Ser Gln Thr Val Arg Lys Ala Leu Asp Ser Asn Ser
        755                 760                 765

Leu Glu Asn Asp Asp Leu Ser Ala Pro Gly Arg Glu Pro Gly His Phe
    770                 775                 780

Asn Pro Glu Ser Arg Glu Asp Thr Arg Gly Gly Asn Glu Lys Gly Lys
785                 790                 795                 800

Ser Lys Glu Asp Cys Thr Met Ser
                805

<210> 71
<211> 2691
<212> DNA
<213> Homo sapiens

<400> 71
aatgcgaccc gaccgcgctg aggctccagg accgcccgcc atggctgcag gaggtcccgg 60

cgcggggtct gcggccccgg tctcctccac atcctccctt cccctggctg ctctcaacat 120

gcgagtgcgg cgccgcctgt ctctgttctt gaacgtgcgg acacaggtgg cggccgactg 180

gaccgcgctg gcggaggaga tggactttga gtacttggag atccggcaac tggagacaca 240

agcggacccc actggcaggc tgctggacgc ctggcaggga cgccctggcg cctctgtagg 300

ccgactgctc gagctgctta ccaagctggg ccgcgacgac gtgctgctgg agctgggacc 360

cagcattgag gaggattgcc aaaagtatat cttgaagcag cagcaggagg aggctgagaa 420

gcctttacag gtggccgctg tagacagcag tgtcccacgg acagcagagc tggcgggcat 480

caccacactt gatgaccccc tggggcatat gcctgagcgt ttcgatgcct tcatctgcta 540

ttgccccagc gacatccagt ttgtgcagga gatgatccgg caactggaac agacaaacta 600

tcgactgaag ttgtgtgtgt ctgaccgcga tgtcctgcct ggcacctgtg tctggtctat 660

tgctagtgag ctcatcgaaa agaggttggc tagaaggcca cggggtgggt gccgccggat 720

ggtggtggtt gtctctgatg attacctgca gagcaaggaa tgtgacttcc agaccaaatt 780

tgcactcagc ctctctccag gtgcccatca gaagcgactg atccccatca agtacaaggc 840

aatgaagaaa gagttcccca gcatcctgag gttcatcact gtctgcgact acaccaaccc 900

ctgcaccaaa tcttggttct ggactcgcct tgccaaggcc ttgtccctgc cctgaagact 960

gttctgaggc cctgggtgtg tgtgtatctg tctgcctgtc catgtacttc tgccctgcct 1020

cctcctttcg ttgtaggagg aatctgtgct ctacttacct ctcaattcct ggagatgcca 1080

acttcacaga cacgtctgca gcagctggac atcacatttc atgtcctgca tggaaccagt 1140

ggctgtgagt ggcatgtcca cttgctggat tatcagccag gacactatag aacaggacca 1200

gctgagacta agaaggacca gcagagccag ctcagctctg agccattcac acatcttcac 1260

cctcagtttc ctcacttgag gagtgggatg gggagaacag agagtagctg tgtttgaatc 1320

cctgtaggaa atggtgaagc atagctctgg gtctcctggg ggagaccagg cttggctgcg 1380

ggagagctgg ctgttgctgg actacatgct ggccactgct gtgaccacga cactgctggg 1440

gcagcttctt ccacagtgat gcctactgat gcttcagtgc ctctgcacac cgcccattcc 1500

acttcctcct tccccacagg gcaggtgggg aagcagtttg gcccagccca aggagacccc 1560

accttgagcc ttatttccta atgggtccac ctctcatctg catctttcac acctcccagc 1620

ttctgcccaa ccttcagcag tgacaagtcc ccaagagact cgcctgagca gcttgggctg 1680

cttttcattt ccacctgtca ggatgcctgt ggtcatgctc tcagctccac ctggcatgag 1740

aagggatcct ggcctctggc atattcatca agtatgagtt ctggggatga gtcactgtaa 1800

tgatgtgagc agggagcctt cctccctggg ccacctgcag agagctttcc caccaacttt 1860

gtaccttgat tgccttacaa agttatttgt ttacaaacag cgaccatata aaagcctcct 1920

gccccaaagc ttgtgggcac atgggcacat acagactcac atacagacac acacatatat 1980

gtacagacat gtactctcac acacacaggc accagcatac acacgttttt ctaggtacag 2040

ctcccaggaa cagctaggtg ggaaagtccc atcactgagg gagcctaacc atgtccctga 2100

acaaaaattg ggcactcatc tattcctttt ctcttgtgtc cctactcatt gaaaccaaac 2160

tctggaaagg acccaatgta ccagtattta tacctctaat gaagcacaga gagaggaaga 2220

gagctgctta aactcacaca acaatgaact gcagacacag ctgttctctc cctctctcct 2280

tcccagagca atttatactt taccctcagg ctgtcctctg gggagaaggt gccatggtct 2340

taggtgtctg tgccccagga cagaccctag gaccctaaat ccaatagaaa atgcatatct 2400

ttgctccact ttcagccagg ctggagcaag gtaccttttc ttaggatctt gggagggaat 2460

ggatgcccct ctctgcatga tcttgttgag gcatttagct gccatgcacc tgtcccctt 2520

taatactggg cattttaaag ccatctcaag aggcatcttc tacatgtttt gtacgcatta 2580

aaataatttc aaagatatct gagaaaagcc gatatttgcc attcttccta tatcctggaa 2640

tatatcttgc atcctgagtt tataataata aataatattc taccttggaa a 2691

<210> 72
<211> 2727
<212> DNA
<213> Homo sapiens

<400> 72
agattcctac ttcttacgcc ccccacatca cccgcctcga gacctcaagg gtagaggtgg 60

gcaccccgc ctccgcactt ttgctcgggg ctccagattg tagggcaggg cggcgcttct 120

cggaaagcga aagccggcgg ggcggggcgg gtgccgcagg agaaagagga agcgctggca 180

gacaatgcga cccgaccgcg ctgaggctcc aggaccgccc gccatggctg caggaggtcc 240

cggcgcgggg tctgcggccc cggtctcctc cacatcctcc cttcccctgg ctgctctcaa 300

catgcgagtg cggcgccgcc tgtctctgtt cttgaacgtg cggacacagg tggcggccga 360

ctggaccgcg ctggcggagg agatggactt tgagtacttg gagatccggc aactggagac 420

acaagcggac cccactggca ggctgctgga cgcctggcag ggacgccctg gcgcctctgt 480

aggccgactg ctcgagctgc ttaccaagct gggccgcgac gacgtgctgc tggagctggg 540

acccagcatt gggcatatgc ctgagcgttt cgatgccttc atctgctatt gccccagcga 600

catccagttt gtgcaggaga tgatccggca actggaacag acaaactatc gactgaagtt 660

gtgtgtgtct gaccgcgatg tcctgcctgg cacctgtgtc tggtctattg ctagtgagct 720

catcgaaaag aggtgccgcc ggatggtggt ggttgtctct gatgattacc tgcagagcaa 780

ggaatgtgac ttccagacca aatttgcact cagcctctct ccaggtgccc atcagaagcg 840

actgatcccc atcaagtaca aggcaatgaa gaaagagttc cccagcatcc tgaggttcat 900

cactgtctgc gactacacca acccctgcac caaatcttgg ttctggactc gccttgccaa 960

ggccttgtcc ctgccctgaa gactgttctg aggccctggg tgtgtgtgta tctgtctgcc 1020

tgtccatgta cttctgccct gcctcctcct ttcgttgtag gaggaatctg tgctctactt 1080

acctctcaat tcctggagat gccaacttca cagacacgtc tgcagcagct ggacatcaca 1140

tttcatgtcc tgcatggaac cagtggctgt gagtggcatg tccacttgct ggattatcag 1200

ccaggacact atagaacagg accagctgag actaagaagg accagcagag ccagctcagc 1260

tctgagccat tcacacatct tcaccctcag tttcctcact tgaggagtgg gatggggaga 1320

acagagagta gctgtgtttg aatccctgta ggaaatggtg aagcatagct ctgggtctcc 1380

tgggggagac caggcttggc tgcgggagag ctggctgttg ctggactaca tgctggccac 1440

tgctgtgacc acgacactgc tggggcagct tcttccacag tgatgcctac tgatgcttca 1500

gtgcctctgc acaccgccca ttccacttcc tccttcccca cagggcaggt ggggaagcag 1560

tttggcccag cccaaggaga ccccaccttg agccttattt cctaatgggt ccacctctca 1620
tctgcatctt tcacacctcc cagcttctgc ccaaccttca gcagtgacaa gtccccaaga 1680
gactcgcctg agcagcttgg gctgcttttc atttccacct gtcaggatgc ctgtggtcat 1740
gctctcagct ccacctggca tgagaaggga tcctggcctc tggcatattc atcaagtatg 1800
agttctgggg atgagtcact gtaatgatgt gagcagggag ccttcctccc tgggccacct 1860
gcagagagct ttcccaccaa ctttgtacct tgattgcctt acaaagttat ttgtttacaa 1920
acagcgacca tataaaagcc tcctgcccca aagcttgtgg gcacatgggc acatacagac 1980
tcacatacag acacacacat atatgtacag acatgtactc tcacacacac aggcaccagc 2040
atacacacgt tttctaggt acagctccca ggaacagcta ggtgggaaag tcccatcact 2100
gagggagcct aaccatgtcc ctgaacaaaa attgggcact catctattcc ttttctcttg 2160
tgtccctact cattgaaacc aaactctgga aaggacccaa tgtaccagta tttatacctc 2220
taatgaagca cagagagagg aagagagctg cttaaactca cacaacaatg aactgcagac 2280
acagctgttc tctccctctc tccttcccag agcaatttat actttaccct caggctgtcc 2340
tctggggaga aggtgccatg gtcttaggtg tctgtgcccc aggacagacc ctaggaccct 2400
aaatccaata gaaaatgcat atctttgctc cactttcagc caggctggag caaggtacct 2460
tttcttagga tcttgggagg gaatggatgc cctctctgc atgatcttgt tgaggcattt 2520
agctgccatg cacctgtccc cctttaatac tgggcatttt aaagccatct caagaggcat 2580
cttctacatg ttttgtacgc attaaaataa tttcaaagat atctgagaaa agccgatatt 2640
tgccattctt cctatatcct ggaatatatc ttgcatcctg agtttataat aataaataat 2700
attctacctt ggaaaaaaaa aaaaaaa 2727

<210> 73
<211> 2486
<212> DNA
<213> Homo sapiens

<400> 73
aatgcgaccc gaccgcgctg aggctccagg accgcccgcc atggctgcag gaggtcccgg 60
cgcggggtct gcggccccgg tctcctccac atcctccctt cccctggctg ctctcaacat 120
gcgagtgcgg cgccgcctgt ctctgttctt gaacgtgcgg acacaggtgg cggccgactg 180
gaccgcgctg gcggaggaga tggactttga gtacttggag atccggcaac tggagacaca 240
agcggacccc actggcaggc tgctggacgc ctggcaggga cgccctggcg cctctgtagg 300
ccgactgctc gagctgctta ccaagctggg ccgcgacgac gtgctgctgg agctgggacc 360
cagcattgag gaggattgcc aaaagtatat cttgaagcag cagcaggagg aggctgagaa 420
gcctttacag gtggccgctg tagacagcag tgtcccacgg acagcagagc tggcgggcat 480

caccacactt gatgaccccc tgggtgccgc cggatggtgg tggttgtctc tgatgattac 540

ctgcagagca aggaatgtga cttccagacc aaatttgcac tcagcctctc tccaggtgcc 600

catcagaagc gactgatccc catcaagtac aaggcaatga agaaagagtt ccccagcatc 660

ctgaggttca tcactgtctg cgactacacc aacccctgca ccaaatcttg gttctggact 720

cgccttgcca aggccttgtc cctgccctga agactgttct gaggccctgg gtgtgtgtgt 780

atctgtctgc ctgtccatgt acttctgccc tgcctcctcc tttcgttgta ggaggaatct 840

gtgctctact tacctctcaa ttcctggaga tgccaacttc acagacacgt ctgcagcagc 900

tggacatcac atttcatgtc ctgcatggaa ccagtggctg tgagtggcat gtccacttgc 960

tggattatca gccaggacac tatagaacag gaccagctga gactaagaag gaccagcaga 1020

gccagctcag ctctgagcca ttcacacatc ttcaccctca gtttcctcac ttgaggagtg 1080

ggatggggag aacagagagt agctgtgttt gaatccctgt aggaaatggt gaagcatagc 1140

tctgggtctc ctgggggaga ccaggcttgg ctgcgggaga gctggctgtt gctggactac 1200

atgctggcca ctgctgtgac cacgacactg ctggggcagc ttcttccaca gtgatgccta 1260

ctgatgcttc agtgcctctg cacaccgccc attccacttc ctccttcccc acagggcagg 1320

tggggaagca gtttggccca gcccaaggag accccacctt gagccttatt tcctaatggg 1380

tccacctctc atctgcatct ttcacacctc ccagcttctg cccaaccttc agcagtgaca 1440

agtccccaag agactcgcct gagcagcttg ggctgctttt catttccacc tgtcaggatg 1500

cctgtggtca tgctctcagc tccacctggc atgagaaggg atcctggcct ctggcatatt 1560

catcaagtat gagttctggg gatgagtcac tgtaatgatg tgagcaggga gccttcctcc 1620

ctgggccacc tgcagagagc tttcccacca actttgtacc ttgattgcct tacaaagtta 1680

tttgtttaca aacagcgacc atataaaagc ctcctgcccc aaagcttgtg ggcacatggg 1740

cacatacaga ctcacataca gacacacaca tatatgtaca gacatgtact ctcacacaca 1800

caggcaccag catacacacg tttttctagg tacagctccc aggaacagct aggtgggaaa 1860

gtcccatcac tgagggagcc taaccatgtc cctgaacaaa aattgggcac tcatctattc 1920

cttttctctt gtgtccctac tcattgaaac caaactctgg aaaggaccca atgtaccagt 1980

atttatacct ctaatgaagc acagagagag gaagagagct gcttaaactc acacaacaat 2040

gaactgcaga cacagctgtt ctctccctct ctccttccca gagcaattta tactttaccc 2100

tcaggctgtc ctctggggag aaggtgccat ggtcttaggt gtctgtgccc caggacagac 2160

cctaggaccc taaatccaat agaaaatgca tatctttgct ccactttcag ccaggctgga 2220

gcaaggtacc ttttcttagg atcttgggag ggaatggatg cccctctctg catgatcttg 2280

ttgaggcatt tagctgccat gcacctgtcc ccctttaata ctgggcattt taaagccatc 2340

tcaagaggca tcttctacat gttttgtacg cattaaaata atttcaaaga tatctgagaa 2400

aagccgatat ttgccattct tcctatatcc tggaatatat cttgcatcct gagtttataa 2460

taataaataa tattctacct tggaaa 2486

<210> 74
<211> 2351
<212> DNA
<213> Homo sapiens

<400> 74
aatgcgaccc gaccgcgctg aggctccagg accgcccgcc atggctgcag gaggtcccgg 60

cgcggggtct gcggccccgg tctcctccac atcctccctt cccctggctg ctctcaacat 120

gcgagtgcgg cgccgcctgt ctctgttctt gaacgtgcgg acacaggtgg cggccgactg 180

gaccgcgctg gcggaggaga tggactttga gtacttggag atccggcaac tggagacaca 240

agcggacccc actggcaggc tgctggacgc ctggcaggga cgccctggcg cctctgtagg 300

ccgactgctc gagctgctta ccaagctggg ccgcgacgac gtgctgctgg agctgggacc 360

cagcattggt gccgccggat ggtggtggtt gtctctgatg attacctgca gagcaaggaa 420

tgtgacttcc agaccaaatt tgcactcagc ctctctccag gtgcccatca gaagcgactg 480

atccccatca agtacaaggc aatgaagaaa gagttcccca gcatcctgag gttcatcact 540

gtctgcgact acaccaaccc ctgcaccaaa tcttggttct ggactcgcct tgccaaggcc 600

ttgtccctgc cctgaagact gttctgaggc cctgggtgtg tgtgtatctg tctgcctgtc 660

catgtacttc tgccctgcct cctcctttcg ttgtaggagg aatctgtgct ctacttacct 720

ctcaattcct ggagatgcca acttcacaga cacgtctgca gcagctggac atcacatttc 780

atgtcctgca tggaaccagt ggctgtgagt ggcatgtcca cttgctggat tatcagccag 840

gacactatag aacaggacca gctgagacta agaaggacca gcagagccag ctcagctctg 900

agccattcac acatcttcac cctcagtttc ctcacttgag gagtgggatg gggagaacag 960

agagtagctg tgtttgaatc cctgtaggaa atggtgaagc atagctctgg gtctcctggg 1020

ggagaccagg cttggctgcg ggagagctgg ctgttgctgg actacatgct ggccactgct 1080

gtgaccacga cactgctggg gcagcttctt ccacagtgat gcctactgat gcttcagtgc 1140

ctctgcacac cgcccattcc acttcctcct tccccacagg gcaggtgggg aagcagtttg 1200

gcccagccca aggagacccc accttgagcc ttatttccta atgggtccac ctctcatctg 1260

catctttcac acctcccagc ttctgcccaa ccttcagcag tgacaagtcc ccaagagact 1320

cgcctgagca gcttgggctg cttttcattt ccacctgtca ggatgcctgt ggtcatgctc 1380

tcagctccac ctggcatgag aagggatcct ggcctctggc atattcatca agtatgagtt 1440

ctggggatga gtcactgtaa tgatgtgagc agggagcctt cctccctggg ccacctgcag 1500

agagctttcc caccaacttt gtaccttgat tgccttacaa agttatttgt ttacaaacag 1560

cgaccatata aaagcctcct gccccaaagc ttgtgggcac atgggcacat acagactcac 1620

atacagacac acacatatat gtacagacat gtactctcac acacacaggc accagcatac 1680

acacgttttt ctaggtacag ctcccaggaa cagctaggtg ggaaagtccc atcactgagg 1740

gagcctaacc atgtccctga acaaaaattg ggcactcatc tattcctttt ctcttgtgtc 1800

cctactcatt gaaaccaaac tctggaaagg acccaatgta ccagtattta tacctctaat 1860

gaagcacaga gagaggaaga gagctgctta aactcacaca acaatgaact gcagacacag 1920

ctgttctctc cctctctcct tcccagagca atttatactt taccctcagg ctgtcctctg 1980

gggagaaggt gccatggtct taggtgtctg tgccccagga cagaccctag gaccctaaat 2040

ccaatagaaa atgcatatct ttgctccact ttcagccagg ctggagcaag gtaccttttc 2100

ttaggatctt gggagggaat ggatgcccct ctctgcatga tcttgttgag gcatttagct 2160

gccatgcacc tgtccccctt taatactggg cattttaaag ccatctcaag aggcatcttc 2220

tacatgtttt gtacgcatta aaataatttc aaagatatct gagaaaagcc gatatttgcc 2280

attcttccta tatcctggaa tatatcttgc atcctgagtt tataataata aataatattc 2340

taccttggaa a 2351

<210> 75
<211> 2862
<212> DNA
<213> Homo sapiens

<400> 75
agattcctac ttcttacgcc ccccacatca cccgcctcga gacctcaagg gtagaggtgg 60

gcaccccccgc ctccgcactt ttgctcgggg ctccagattg tagggcaggg cggcgcttct 120

cggaaagcga aagccggcgg ggcggggcgg gtgccgcagg agaaagagga agcgctggca 180

gacaatgcga cccgaccgcg ctgaggctcc aggaccgccc gccatggctg caggaggtcc 240

cggcgcgggg tctgcggccc cggtctcctc cacatcctcc cttcccctgg ctgctctcaa 300

catgcgagtg cggcgccgcc tgtctctgtt cttgaacgtg cggacacagg tggcggccga 360

ctggaccgcg ctggcggagg agatggactt tgagtacttg gagatccggc aactggagac 420

acaagcggac cccactggca ggctgctgga cgcctggcag ggacgccctg gcgcctctgt 480

aggccgactg ctcgagctgc ttaccaagct gggccgcgac gacgtgctgc tggagctggg 540

acccagcatt gaggaggatt gccaaaagta tatcttgaag cagcagcagg aggaggctga 600

gaagccttta caggtggccg ctgtagacag cagtgtccca cggacagcag agctggcggg 660

catcaccaca cttgatgacc ccctggggca tatgcctgag cgtttcgatg ccttcatctg 720

ctattgcccc agcgacatcc agtttgtgca ggagatgatc cggcaactgg aacagacaaa 780

ctatcgactg aagttgtgtg tgtctgaccg cgatgtcctg cctggcacct gtgtctggtc 840

tattgctagt gagctcatcg aaaagaggtg ccgccggatg gtggtggttg tctctgatga 900

ttacctgcag agcaaggaat gtgacttcca gaccaaattt gcactcagcc tctctccagg 960

tgcccatcag aagcgactga tccccatcaa gtacaaggca atgaagaaag agttccccag 1020

catcctgagg ttcatcactg tctgcgacta caccaacccc tgcaccaaat cttggttctg 1080

gactcgcctt gccaaggcct tgtccctgcc ctgaagactg ttctgaggcc ctgggtgtgt 1140

gtgtatctgt ctgcctgtcc atgtacttct gccctgcctc ctcctttcgt tgtaggagga 1200

atctgtgctc tacttacctc tcaattcctg gagatgccaa cttcacagac acgtctgcag 1260

cagctggaca tcacatttca tgtcctgcat ggaaccagtg gctgtgagtg gcatgtccac 1320

ttgctggatt atcagccagg acactataga acaggaccag ctgagactaa gaaggaccag 1380

cagagccagc tcagctctga gccattcaca catcttcacc ctcagtttcc tcacttgagg 1440

agtgggatgg ggagaacaga gagtagctgt gtttgaatcc ctgtaggaaa tggtgaagca 1500

tagctctggg tctcctgggg gagaccaggc ttggctgcgg gagagctggc tgttgctgga 1560

ctacatgctg gccactgctg tgaccacgac actgctgggg cagcttcttc cacagtgatg 1620

cctactgatg cttcagtgcc tctgcacacc gcccattcca cttcctcctt ccccacaggg 1680

caggtgggga agcagtttgg cccagcccaa ggagacccca ccttgagcct tatttcctaa 1740

tgggtccacc tctcatctgc atctttcaca cctcccagct tctgcccaac cttcagcagt 1800

gacaagtccc caagagactc gcctgagcag cttgggctgc ttttcatttc cacctgtcag 1860

gatgcctgtg gtcatgctct cagctccacc tggcatgaga agggatcctg gcctctggca 1920

tattcatcaa gtatgagttc tggggatgag tcactgtaat gatgtgagca gggagccttc 1980

ctccctgggc cacctgcaga gagctttccc accaactttg taccttgatt gccttacaaa 2040

gttatttgtt tacaaacagc gaccatataa aagcctcctg ccccaaagct tgtgggcaca 2100

tgggcacata cagactcaca tacagacaca cacatatatg tacagacatg tactctcaca 2160

cacacaggca ccagcataca cacgtttttc taggtacagc tcccaggaac agctaggtgg 2220

gaaagtccca tcactgaggg agcctaacca tgtccctgaa caaaaattgg gcactcatct 2280

attccttttc tcttgtgtcc ctactcattg aaaccaaact ctggaaagga cccaatgtac 2340

cagtatttat acctctaatg aagcacagag agaggaagag agctgcttaa actcacacaa 2400

caatgaactg cagacacagc tgttctctcc ctctctcctt cccagagcaa tttatacttt 2460

accctcaggc tgtcctctgg ggagaaggtg ccatggtctt aggtgtctgt gccccaggac 2520

agaccctagg accctaaatc caatagaaaa tgcatatctt tgctccactt tcagccaggc 2580

tggagcaagg taccttttct taggatcttg ggagggaatg gatgcccctc tctgcatgat 2640

cttgttgagg catttagctg ccatgcacct gtcccccttt aatactgggc attttaaagc 2700

catctcaaga ggcatcttct acatgttttg tacgcattaa aataatttca aagatatctg 2760

agaaaagccg atatttgcca ttcttcctat atcctggaat atatcttgca tcctgagttt 2820

ataataataa ataatattct accttggaaa aaaaaaaaaa aa 2862

<210> 76
<211> 304
<212> PRT
<213> Homo sapiens

<400> 76

```
Met Ala Ala Gly Gly Pro Gly Ala Gly Ser Ala Ala Pro Val Ser Ser
1               5                   10                  15

Thr Ser Ser Leu Pro Leu Ala Ala Leu Asn Met Arg Val Arg Arg Arg
            20                  25                  30

Leu Ser Leu Phe Leu Asn Val Arg Thr Gln Val Ala Ala Asp Trp Thr
        35                  40                  45

Ala Leu Ala Glu Glu Met Asp Phe Glu Tyr Leu Glu Ile Arg Gln Leu
    50                  55                  60

Glu Thr Gln Ala Asp Pro Thr Gly Arg Leu Leu Asp Ala Trp Gln Gly
65                  70                  75                  80

Arg Pro Gly Ala Ser Val Gly Arg Leu Leu Glu Leu Leu Thr Lys Leu
                85                  90                  95

Gly Arg Asp Asp Val Leu Leu Glu Leu Gly Pro Ser Ile Glu Glu Asp
            100                 105                 110

Cys Gln Lys Tyr Ile Leu Lys Gln Gln Gln Glu Glu Ala Glu Lys Pro
        115                 120                 125

Leu Gln Val Ala Ala Val Asp Ser Ser Val Pro Arg Thr Ala Glu Leu
    130                 135                 140

Ala Gly Ile Thr Thr Leu Asp Asp Pro Leu Gly His Met Pro Glu Arg
145                 150                 155                 160

Phe Asp Ala Phe Ile Cys Tyr Cys Pro Ser Asp Ile Gln Phe Val Gln
                165                 170                 175

Glu Met Ile Arg Gln Leu Glu Gln Thr Asn Tyr Arg Leu Lys Leu Cys
            180                 185                 190

Val Ser Asp Arg Asp Val Leu Pro Gly Thr Cys Val Trp Ser Ile Ala
```

195 200 205

Ser Glu Leu Ile Glu Lys Arg Leu Ala Arg Arg Pro Arg Gly Gly Cys
210 215 220

Arg Arg Met Val Val Val Val Ser Asp Asp Tyr Leu Gln Ser Lys Glu
225 230 235 240

Cys Asp Phe Gln Thr Lys Phe Ala Leu Ser Leu Ser Pro Gly Ala His
245 250 255

Gln Lys Arg Leu Ile Pro Ile Lys Tyr Lys Ala Met Lys Lys Glu Phe
260 265 270

Pro Ser Ile Leu Arg Phe Ile Thr Val Cys Asp Tyr Thr Asn Pro Cys
275 280 285

Thr Lys Ser Trp Phe Trp Thr Arg Leu Ala Lys Ala Leu Ser Leu Pro
290 295 300

<210> 77
<211> 251
<212> PRT
<213> Homo sapiens

<400> 77

Met Ala Ala Gly Gly Pro Gly Ala Gly Ser Ala Ala Pro Val Ser Ser
1 5 10 15

Thr Ser Ser Leu Pro Leu Ala Ala Leu Asn Met Arg Val Arg Arg Arg
20 25 30

Leu Ser Leu Phe Leu Asn Val Arg Thr Gln Val Ala Ala Asp Trp Thr
35 40 45

Ala Leu Ala Glu Glu Met Asp Phe Glu Tyr Leu Glu Ile Arg Gln Leu
50 55 60

Glu Thr Gln Ala Asp Pro Thr Gly Arg Leu Leu Asp Ala Trp Gln Gly
65 70 75 80

Arg Pro Gly Ala Ser Val Gly Arg Leu Leu Glu Leu Leu Thr Lys Leu
85 90 95

Gly Arg Asp Asp Val Leu Leu Glu Leu Gly Pro Ser Ile Gly His Met
100 105 110

Pro Glu Arg Phe Asp Ala Phe Ile Cys Tyr Cys Pro Ser Asp Ile Gln

115 120 125

Phe Val Gln Glu Met Ile Arg Gln Leu Glu Gln Thr Asn Tyr Arg Leu
130 135 140

Lys Leu Cys Val Ser Asp Arg Asp Val Leu Pro Gly Thr Cys Val Trp
145 150 155 160

Ser Ile Ala Ser Glu Leu Ile Glu Lys Arg Cys Arg Arg Met Val Val
165 170 175

Val Val Ser Asp Asp Tyr Leu Gln Ser Lys Glu Cys Asp Phe Gln Thr
180 185 190

Lys Phe Ala Leu Ser Leu Ser Pro Gly Ala His Gln Lys Arg Leu Ile
195 200 205

Pro Ile Lys Tyr Lys Ala Met Lys Lys Glu Phe Pro Ser Ile Leu Arg
210 215 220

Phe Ile Thr Val Cys Asp Tyr Thr Asn Pro Cys Thr Lys Ser Trp Phe
225 230 235 240

Trp Thr Arg Leu Ala Lys Ala Leu Ser Leu Pro
245 250

<210> 78
<211> 191
<212> PRT
<213> Homo sapiens

<400> 78

Met Ala Ala Gly Gly Pro Gly Ala Gly Ser Ala Ala Pro Val Ser Ser
1 5 10 15

Thr Ser Ser Leu Pro Leu Ala Ala Leu Asn Met Arg Val Arg Arg Arg
20 25 30

Leu Ser Leu Phe Leu Asn Val Arg Thr Gln Val Ala Ala Asp Trp Thr
35 40 45

Ala Leu Ala Glu Glu Met Asp Phe Glu Tyr Leu Glu Ile Arg Gln Leu
50 55 60

Glu Thr Gln Ala Asp Pro Thr Gly Arg Leu Leu Asp Ala Trp Gln Gly
65 70 75 80

Arg Pro Gly Ala Ser Val Gly Arg Leu Leu Glu Leu Leu Thr Lys Leu

85 90 95

Gly Arg Asp Asp Val Leu Leu Glu Leu Gly Pro Ser Ile Glu Glu Asp
100 105 110

Cys Gln Lys Tyr Ile Leu Lys Gln Gln Gln Glu Glu Ala Glu Lys Pro
115 120 125

Leu Gln Val Ala Ala Val Asp Ser Ser Val Pro Arg Thr Ala Glu Leu
130 135 140

Ala Gly Ile Thr Thr Leu Asp Asp Pro Leu Gly Ala Ala Gly Trp Trp
145 150 155 160

Trp Leu Ser Leu Met Ile Thr Cys Arg Ala Arg Asn Val Thr Ser Arg
165 170 175

Pro Asn Leu His Ser Ala Ser Leu Gln Val Pro Ile Arg Ser Asp
180 185 190

<210> 79
<211> 146
<212> PRT
<213> Homo sapiens

<400> 79

Met Ala Ala Gly Gly Pro Gly Ala Gly Ser Ala Ala Pro Val Ser Ser
1 5 10 15

Thr Ser Ser Leu Pro Leu Ala Ala Leu Asn Met Arg Val Arg Arg Arg
20 25 30

Leu Ser Leu Phe Leu Asn Val Arg Thr Gln Val Ala Ala Asp Trp Thr
35 40 45

Ala Leu Ala Glu Glu Met Asp Phe Glu Tyr Leu Glu Ile Arg Gln Leu
50 55 60

Glu Thr Gln Ala Asp Pro Thr Gly Arg Leu Leu Asp Ala Trp Gln Gly
65 70 75 80

Arg Pro Gly Ala Ser Val Gly Arg Leu Leu Glu Leu Leu Thr Lys Leu
85 90 95

Gly Arg Asp Asp Val Leu Leu Glu Leu Gly Pro Ser Ile Gly Ala Ala
100 105 110

Gly Trp Trp Trp Leu Ser Leu Met Ile Thr Cys Arg Ala Arg Asn Val

115 120 125

Thr Ser Arg Pro Asn Leu His Ser Ala Ser Leu Gln Val Pro Ile Arg
130 135 140

Ser Asp
145

<210> 80
<211> 296
<212> PRT
<213> Homo sapiens

<400> 80

Met Ala Ala Gly Gly Pro Gly Ala Gly Ser Ala Ala Pro Val Ser Ser
1 5 10 15

Thr Ser Ser Leu Pro Leu Ala Ala Leu Asn Met Arg Val Arg Arg Arg
20 25 30

Leu Ser Leu Phe Leu Asn Val Arg Thr Gln Val Ala Ala Asp Trp Thr
35 40 45

Ala Leu Ala Glu Glu Met Asp Phe Glu Tyr Leu Glu Ile Arg Gln Leu
50 55 60

Glu Thr Gln Ala Asp Pro Thr Gly Arg Leu Leu Asp Ala Trp Gln Gly
65 70 75 80

Arg Pro Gly Ala Ser Val Gly Arg Leu Leu Glu Leu Leu Thr Lys Leu
85 90 95

Gly Arg Asp Asp Val Leu Leu Glu Leu Gly Pro Ser Ile Glu Glu Asp
100 105 110

Cys Gln Lys Tyr Ile Leu Lys Gln Gln Gln Glu Glu Ala Glu Lys Pro
115 120 125

Leu Gln Val Ala Ala Val Asp Ser Ser Val Pro Arg Thr Ala Glu Leu
130 135 140

Ala Gly Ile Thr Thr Leu Asp Asp Pro Leu Gly His Met Pro Glu Arg
145 150 155 160

Phe Asp Ala Phe Ile Cys Tyr Cys Pro Ser Asp Ile Gln Phe Val Gln
165 170 175

Glu Met Ile Arg Gln Leu Glu Gln Thr Asn Tyr Arg Leu Lys Leu Cys

180 185 190

Val Ser Asp Arg Asp Val Leu Pro Gly Thr Cys Val Trp Ser Ile Ala
195 200 205

Ser Glu Leu Ile Glu Lys Arg Cys Arg Arg Met Val Val Val Val Ser
210 215 220

Asp Asp Tyr Leu Gln Ser Lys Glu Cys Asp Phe Gln Thr Lys Phe Ala
225 230 235 240

Leu Ser Leu Ser Pro Gly Ala His Gln Lys Arg Leu Ile Pro Ile Lys
245 250 255

Tyr Lys Ala Met Lys Lys Glu Phe Pro Ser Ile Leu Arg Phe Ile Thr
260 265 270

Val Cys Asp Tyr Thr Asn Pro Cys Thr Lys Ser Trp Phe Trp Thr Arg
275 280 285

Leu Ala Lys Ala Leu Ser Leu Pro
290 295

<210> 81
<211> 1614
<212> DNA
<213> Homo sapiens

<400> 81
gcatttccag agcagagttc agagaaaggc tgggctgctt gttgctggct aaaggacaaa 60

gggtaagttt caggaagcag aagagtgagc agatgaaatt cagcactggg atcaggggag 120

tgtctgattt gcaaaaggaa agtgcaaaga cagctcctcc cttctgagga aacgaaacca 180

acagcagtcc aagctcagtc agcagaagag ataaaagcaa acaggtctgg gaggcagttc 240

tgttgccact ctctctcctg tcaatgatgg atctcagaaa taccccagcc aaatctctgg 300

acaagttcat tgaagactat ctcttgccag acacgtgttt ccgcatgcaa atcaaccatg 360

ccattgacat catctgtggg ttcctgaagg aaaggtgctt ccgaggtagc tcctaccctg 420

tgtgtgtgtc caaggtggta aagggtggct cctcaggcaa gggcaccacc ctcagaggcc 480

gatctgacgc tgacctggtt gtcttcctca gtcctctcac cacttttcag gatcagttaa 540

atcgccgggg agagttcatc caggaaatta ggagacagct ggaagcctgt caaagagaga 600

gagcattttc cgtgaagttt gaggtccagg ctccacgctg gggcaacccc cgtgcgctca 660

gcttcgtact gagttcgctc cagctcgggg agggggtgga gttcgatgtg ctgcctgcct 720

ttgatgccct gggtcagttg actggcggct ataaacctaa cccccaaatc tatgtcaagc 780

tcatcgagga gtgcaccgac ctgcagaaag agggcgagtt ctccacctgc ttcacagaac 840

tacagagaga cttcctgaag cagcgcccca ccaagctcaa gagcctcatc cgcctagtca 900

agcactggta ccaaaattgt aagaagaagc ttgggaagct gccacctcag tatgccctgg 960

agctcctgac ggtctatgct tgggagcgag ggagcatgaa aacacatttc aacacagccc 1020

agggatttcg gacggtcttg gaattagtca taaactacca gcaactctgc atctactgga 1080

caaagtatta tgactttaaa aaccccatta ttgaaaagta cctgagaagg cagctcacga 1140

aacccaggcc tgtgatcctg gacccggcgg accctacagg aaacttgggt ggtggagacc 1200

caaagggttg gaggcagctg gcacaagagg ctgaggcctg gctgaattac ccatgcttta 1260

agaattggga tgggtcccca gtgagctcct ggattctgct ggtaaacctc acactggttg 1320

gcagaaggaa ctataccaat aattagtgaa catgcggtga atttgcaaca gacaagagga 1380

gcctcattat cctatagttt ccaggttgct tagggaggca gaaatcacag caaggaaaac 1440

cttcaataat aaacagacgt ctcataaaat taattgcaac ccaacctctc tctctactta 1500

aaattagcat ctatttccag ctctgctttc aatgccccat atgaatacat gtgaactccc 1560

tccctctctt cctccctgtc tccttctctc tctctctgtc cctcattaaa aaat 1614

<210> 82
<211> 1627
<212> DNA
<213> Homo sapiens

<400> 82
gcatttccag agcagagttc agagaaaggc tgggctgctt gttgctggct aaaggacaaa 60

gggtaagttt caggaagcag aagagtgagc agatgaaatt cagcactggg atcaggggag 120

tgtctgattt gcaaaaggaa agtgcaaaga cagctcctcc cttctgagga aacgaaacca 180

acagcagtcc aagctcagtc agcagaagag ataaaagcaa acaggtctgg gaggcagttc 240

tgttgccact ctctctcctg tcaatgatgg atctcagaaa taccccagcc aaatctctgg 300

acaagttcat tgaagactat ctcttgccag acacgtgttt ccgcatgcaa atcaaccatg 360

ccattgacat catctgtggg ttcctgaagg aaaggtgctt ccgaggtagc tcctaccctg 420

tgtgtgtgtc caaggtggta aagggtggct cctcaggcaa gggcaccacc ctcagaggcc 480

gatctgacgc tgacctggtt gtcttcctca gtcctctcac cacttttcag gatcagttaa 540

atcgccgggg agagttcatc caggaaatta ggagacagct ggaagcctgt caaagagaga 600

gagcattttc cgtgaagttt gaggtccagg ctccacgctg gggcaacccc cgtgcgctca 660

gcttcgtact gagttcgctc cagctcgggg aggggtgga gttcgatgtg ctgcctgcct 720

ttgatgccct gggtcagttg actggcggct ataaacctaa cccccaaatc tatgtcaagc 780

tcatcgagga gtgcaccgac ctgcagaaag agggcgagtt ctccacctgc ttcacagaac 840

tacagagaga cttcctgaag cagcgcccca ccaagctcaa gagcctcatc cgcctagtca 900

agcactggta ccaaaattgt aagaagaagc ttgggaagct gccacctcag tatgccctgg 960

agctcctgac ggtctatgct tgggagcgag ggagcatgaa aacacatttc aacacagccc 1020

agggatttcg gacggtcttg gaattagtca taaactacca gcaactctgc atctactgga 1080

caaagtatta tgactttaaa aaccccatta ttgaaaagta cctgagaagg cagctcacga 1140

aacccaggcc tgtgatcctg gacccggcgg accctacagg aaacttgggt ggtggagacc 1200

caaagggttg gaggcagctg gcacaagagg ctgaggcctg gctgaattac ccatgcttta 1260

agaattggga tgggtcccca gtgagctcct ggattctgct ggtgagacct cctgcttcct 1320

ccctgccatt catccctgcc cctctccatg aagcttgaga catatagctg gagaccattc 1380

tttccaaaga acttacctct tgccaaaggc catttatatt catatagtga caggctgtgc 1440

tccatatttt acagtcattt tggtcacaat cgagggtttc tggaattttc acatcccttg 1500

tccagaattc attcccctaa gagtaataat aaataatctc taacaccatt tattgactgt 1560

ctgcttcggg ctcaggttct gtcctaagcc ctttaatatg cactctctca ttaaatagtc 1620

acaacaa 1627

<210> 83
<211> 1533
<212> DNA
<213> Homo sapiens

<400> 83
agtccaagct cagtcagcag aagagataaa agcaaacagg tctgggaggc agttctgttg 60

ccactctctc tcctgtcaat gatggatctc agaaataccc cagccaaatc tctggacaag 120

ttcattgaag actatctctt gccagacacg tgtttccgca tgcaaatcaa ccatgccatt 180

gacatcatct gtgggttcct gaaggaaagg tgcttccgag gtagctccta ccctgtgtgt 240

gtgtccaagg tggtaaaggg tggctcctca ggcaagggca ccaccctcag aggccgatct 300

gacgctgacc tggttgtctt cctcagtcct ctcaccactt ttcaggatca gttaaatcgc 360

cggggagagt tcatccagga aattaggaga cagctggaag cctgtcaaag agagagagca 420

ttttccgtga agtttgaggt ccaggctcca cgctggggca acccccgtgc gctcagcttc 480

gtactgagtt cgctccagct cggggagggg gtggagttcg atgtgctgcc tgcctttgat 540

gccctgggtc agttgactgg cggctataaa cctaaccccc aaatctatgt caagctcatc 600

gaggagtgca ccgacctgca gaaagagggc gagttctcca cctgcttcac agaactacag 660

agagacttcc tgaagcagcg ccccaccaag ctcaagagcc tcatccgcct agtcaagcac 720

tggtaccaaa attgtaagaa gaagcttggg aagctgccac ctcagtatgc cctggagctc 780

ctgacggtct atgcttggga gcgagggagc atgaaaacac atttcaacac agcccaggga 840

```
tttcggacgg tcttggaatt agtcataaac taccagcaac tctgcatcta ctggacaaag       900

tattatgact ttaaaaaccc cattattgaa aagtacctga gaaggcagct cacgaaaccc       960

aggcctgtga tcctggaccc ggcggaccct acaggaaact tgggtggtgg agacccaaag      1020

ggttggaggc agctggcaca agaggctgag gcctggctga attacccatg ctttaagaat      1080

tgggatgggt ccccagtgag ctcctggatt ctgctgaccc agcacactcc aggcagcatc      1140

caccccacag gcagaagagg actggacctg caccatcctc tgaatgccag tgcatcttgg      1200

gggaaagggc tccagtgtta tctggaccag ttccttcatt ttcaggtggg actcttgatc      1260

cagagaggac aaagctcctc agtgagctgg tgtataatcc aggacagaac ccaggtctcc      1320

tgactcctgg ccttctatgc cctctatcct atcatagata acattctcca cagcctcact      1380

tcattccacc tattctctga aaatattccc tgagagagaa cagagagatt tagataagag      1440

aatgaaattc cagccttgac tttcttctgt gcacctgatg ggagggtaat gtctaatgta      1500

ttatcaataa caataaaaat aaagcaaata cca                                   1533


<210>  84
<211>  1631
<212>  DNA
<213>  Homo sapiens

<400>  84
agtccaagct cagtcagcag aagagataaa agcaaacagg tctgggaggc agttctgttg        60

ccactctctc tcctgtcaat gatggatctc agaaataccc cagccaaatc tctggacaag       120

ttcattgaag actatctctt gccagacacg tgtttccgca tgcaaatcaa ccatgccatt       180

gacatcatct gtgggttcct gaaggaaagg tgcttccgag gtagctccta ccctgtgtgt       240

gtgtccaagg tggtaaaggg tggctcctca ggcaagggca ccaccctcag aggccgatct       300

gacgctgacc tggttgtctt cctcagtcct ctcaccactt ttcaggatca gttaaatcgc       360

cggggagagt tcatccagga aattaggaga cagctggaag cctgtcaaag agagagagca       420

ttttccgtga agtttgaggt ccaggctcca cgctggggca acccccgtgc gctcagcttc       480

gtactgagtt cgctccagct cggggagggg gtggagttcg atgtgctgcc tgcctttgat       540

gccctgggtc agttgactgg cggctataaa cctaaccccc aaatctatgt caagctcatc       600

gaggagtgca ccgacctgca gaaagagggc gagttctcca cctgcttcac agaactacag       660

agagacttcc tgaagcagcg ccccaccaag ctcaagagcc tcatccgcct agtcaagcac       720

tggtaccaaa attgtaagaa gaagcttggg aagctgccac ctcagtatgc cctggagctc       780

ctgacggtct atgcttggga gcgagggagc atgaaaacac atttcaacac agcccaggga       840

tttcggacgg tcttggaatt agtcataaac taccagcaac tctgcatcta ctggacaaag       900

tattatgact ttaaaaaccc cattattgaa aagtacctga gaaggcagct cacgaaaccc       960
```

aggcctgtga tcctggaccc ggcggaccct acaggaaact tgggtggtgg agacccaaag 1020

ggttggaggc agctggcaca agaggctgag gcctggctga attacccatg ctttaagaat 1080

tgggatgggt ccccagtgag ctcctggatt ctgctggctg aaagcaacag tgcagacgat 1140

gagaccgacg atcccaggag gtatcagaaa tatggttaca ttggaacaca tgagtaccct 1200

catttctctc atagacccag cacactccag gcagcatcca ccccacaggc agaagaggac 1260

tggacctgca ccatcctctg aatgccagtg catcttgggg gaaagggctc cagtgttatc 1320

tggaccagtt ccttcatttt caggtgggac tcttgatcca gagaggacaa agctcctcag 1380

tgagctggtg tataatccag gacagaaccc aggtctcctg actcctggcc ttctatgccc 1440

tctatcctat catagataac attctccaca gcctcacttc attccaccta ttctctgaaa 1500

atattccctg agagagaaca gagagattta gataagagaa tgaaattcca gccttgactt 1560

tcttctgtgc acctgatggg agggtaatgt ctaatgtatt atcaataaca ataaaaataa 1620

agcaaatacc a 1631

<210> 85
<211> 360
<212> PRT
<213> Homo sapiens

<400> 85

Met Met Asp Leu Arg Asn Thr Pro Ala Lys Ser Leu Asp Lys Phe Ile
1 5 10 15

Glu Asp Tyr Leu Leu Pro Asp Thr Cys Phe Arg Met Gln Ile Asn His
20 25 30

Ala Ile Asp Ile Ile Cys Gly Phe Leu Lys Glu Arg Cys Phe Arg Gly
35 40 45

Ser Ser Tyr Pro Val Cys Val Ser Lys Val Val Lys Gly Gly Ser Ser
50 55 60

Gly Lys Gly Thr Thr Leu Arg Gly Arg Ser Asp Ala Asp Leu Val Val
65 70 75 80

Phe Leu Ser Pro Leu Thr Thr Phe Gln Asp Gln Leu Asn Arg Arg Gly
85 90 95

Glu Phe Ile Gln Glu Ile Arg Arg Gln Leu Glu Ala Cys Gln Arg Glu
100 105 110

Arg Ala Phe Ser Val Lys Phe Glu Val Gln Ala Pro Arg Trp Gly Asn
115 120 125

Pro Arg Ala Leu Ser Phe Val Leu Ser Ser Leu Gln Leu Gly Glu Gly
    130                 135                 140

Val Glu Phe Asp Val Leu Pro Ala Phe Asp Ala Leu Gly Gln Leu Thr
145                 150                 155                 160

Gly Gly Tyr Lys Pro Asn Pro Gln Ile Tyr Val Lys Leu Ile Glu Glu
                165                 170                 175

Cys Thr Asp Leu Gln Lys Glu Gly Glu Phe Ser Thr Cys Phe Thr Glu
            180                 185                 190

Leu Gln Arg Asp Phe Leu Lys Gln Arg Pro Thr Lys Leu Lys Ser Leu
        195                 200                 205

Ile Arg Leu Val Lys His Trp Tyr Gln Asn Cys Lys Lys Lys Leu Gly
    210                 215                 220

Lys Leu Pro Pro Gln Tyr Ala Leu Glu Leu Leu Thr Val Tyr Ala Trp
225                 230                 235                 240

Glu Arg Gly Ser Met Lys Thr His Phe Asn Thr Ala Gln Gly Phe Arg
                245                 250                 255

Thr Val Leu Glu Leu Val Ile Asn Tyr Gln Gln Leu Cys Ile Tyr Trp
            260                 265                 270

Thr Lys Tyr Tyr Asp Phe Lys Asn Pro Ile Ile Glu Lys Tyr Leu Arg
        275                 280                 285

Arg Gln Leu Thr Lys Pro Arg Pro Val Ile Leu Asp Pro Ala Asp Pro
    290                 295                 300

Thr Gly Asn Leu Gly Gly Gly Asp Pro Lys Gly Trp Arg Gln Leu Ala
305                 310                 315                 320

Gln Glu Ala Glu Ala Trp Leu Asn Tyr Pro Cys Phe Lys Asn Trp Asp
                325                 330                 335

Gly Ser Pro Val Ser Ser Trp Ile Leu Leu Val Asn Leu Thr Leu Val
            340                 345                 350

Gly Arg Arg Asn Tyr Thr Asn Asn
        355                 360

<210> 86
<211> 364

<212> PRT
<213> Homo sapiens

<400> 86

Met Met Asp Leu Arg Asn Thr Pro Ala Lys Ser Leu Asp Lys Phe Ile
1 5 10 15

Glu Asp Tyr Leu Leu Pro Asp Thr Cys Phe Arg Met Gln Ile Asn His
20 25 30

Ala Ile Asp Ile Ile Cys Gly Phe Leu Lys Glu Arg Cys Phe Arg Gly
35 40 45

Ser Ser Tyr Pro Val Cys Val Ser Lys Val Val Lys Gly Gly Ser Ser
50 55 60

Gly Lys Gly Thr Thr Leu Arg Gly Arg Ser Asp Ala Asp Leu Val Val
65 70 75 80

Phe Leu Ser Pro Leu Thr Thr Phe Gln Asp Gln Leu Asn Arg Arg Gly
85 90 95

Glu Phe Ile Gln Glu Ile Arg Arg Gln Leu Glu Ala Cys Gln Arg Glu
100 105 110

Arg Ala Phe Ser Val Lys Phe Glu Val Gln Ala Pro Arg Trp Gly Asn
115 120 125

Pro Arg Ala Leu Ser Phe Val Leu Ser Ser Leu Gln Leu Gly Glu Gly
130 135 140

Val Glu Phe Asp Val Leu Pro Ala Phe Asp Ala Leu Gly Gln Leu Thr
145 150 155 160

Gly Gly Tyr Lys Pro Asn Pro Gln Ile Tyr Val Lys Leu Ile Glu Glu
165 170 175

Cys Thr Asp Leu Gln Lys Glu Gly Glu Phe Ser Thr Cys Phe Thr Glu
180 185 190

Leu Gln Arg Asp Phe Leu Lys Gln Arg Pro Thr Lys Leu Lys Ser Leu
195 200 205

Ile Arg Leu Val Lys His Trp Tyr Gln Asn Cys Lys Lys Lys Leu Gly
210 215 220

Lys Leu Pro Pro Gln Tyr Ala Leu Glu Leu Leu Thr Val Tyr Ala Trp
225 230 235 240

Glu Arg Gly Ser Met Lys Thr His Phe Asn Thr Ala Gln Gly Phe Arg
245 250 255

Thr Val Leu Glu Leu Val Ile Asn Tyr Gln Gln Leu Cys Ile Tyr Trp
260 265 270

Thr Lys Tyr Tyr Asp Phe Lys Asn Pro Ile Ile Glu Lys Tyr Leu Arg
275 280 285

Arg Gln Leu Thr Lys Pro Arg Pro Val Ile Leu Asp Pro Ala Asp Pro
290 295 300

Thr Gly Asn Leu Gly Gly Gly Asp Pro Lys Gly Trp Arg Gln Leu Ala
305 310 315 320

Gln Glu Ala Glu Ala Trp Leu Asn Tyr Pro Cys Phe Lys Asn Trp Asp
325 330 335

Gly Ser Pro Val Ser Ser Trp Ile Leu Leu Val Arg Pro Pro Ala Ser
340 345 350

Ser Leu Pro Phe Ile Pro Ala Pro Leu His Glu Ala
355 360

<210> 87
<211> 414
<212> PRT
<213> Homo sapiens

<400> 87

Met Met Asp Leu Arg Asn Thr Pro Ala Lys Ser Leu Asp Lys Phe Ile
1 5 10 15

Glu Asp Tyr Leu Leu Pro Asp Thr Cys Phe Arg Met Gln Ile Asn His
20 25 30

Ala Ile Asp Ile Ile Cys Gly Phe Leu Lys Glu Arg Cys Phe Arg Gly
35 40 45

Ser Ser Tyr Pro Val Cys Val Ser Lys Val Val Lys Gly Gly Ser Ser
50 55 60

Gly Lys Gly Thr Thr Leu Arg Gly Arg Ser Asp Ala Asp Leu Val Val
65 70 75 80

Phe Leu Ser Pro Leu Thr Thr Phe Gln Asp Gln Leu Asn Arg Arg Gly
85 90 95

Glu Phe Ile Gln Glu Ile Arg Arg Gln Leu Glu Ala Cys Gln Arg Glu
100 105 110

Arg Ala Phe Ser Val Lys Phe Glu Val Gln Ala Pro Arg Trp Gly Asn
115 120 125

Pro Arg Ala Leu Ser Phe Val Leu Ser Ser Leu Gln Leu Gly Glu Gly
130 135 140

Val Glu Phe Asp Val Leu Pro Ala Phe Asp Ala Leu Gly Gln Leu Thr
145 150 155 160

Gly Gly Tyr Lys Pro Asn Pro Gln Ile Tyr Val Lys Leu Ile Glu Glu
165 170 175

Cys Thr Asp Leu Gln Lys Glu Gly Glu Phe Ser Thr Cys Phe Thr Glu
180 185 190

Leu Gln Arg Asp Phe Leu Lys Gln Arg Pro Thr Lys Leu Lys Ser Leu
195 200 205

Ile Arg Leu Val Lys His Trp Tyr Gln Asn Cys Lys Lys Lys Leu Gly
210 215 220

Lys Leu Pro Pro Gln Tyr Ala Leu Glu Leu Leu Thr Val Tyr Ala Trp
225 230 235 240

Glu Arg Gly Ser Met Lys Thr His Phe Asn Thr Ala Gln Gly Phe Arg
245 250 255

Thr Val Leu Glu Leu Val Ile Asn Tyr Gln Gln Leu Cys Ile Tyr Trp
260 265 270

Thr Lys Tyr Tyr Asp Phe Lys Asn Pro Ile Ile Glu Lys Tyr Leu Arg
275 280 285

Arg Gln Leu Thr Lys Pro Arg Pro Val Ile Leu Asp Pro Ala Asp Pro
290 295 300

Thr Gly Asn Leu Gly Gly Gly Asp Pro Lys Gly Trp Arg Gln Leu Ala
305 310 315 320

Gln Glu Ala Glu Ala Trp Leu Asn Tyr Pro Cys Phe Lys Asn Trp Asp
325 330 335

Gly Ser Pro Val Ser Ser Trp Ile Leu Leu Thr Gln His Thr Pro Gly

340 345 350

Ser Ile His Pro Thr Gly Arg Arg Gly Leu Asp Leu His His Pro Leu
355 360 365

Asn Ala Ser Ala Ser Trp Gly Lys Gly Leu Gln Cys Tyr Leu Asp Gln
370 375 380

Phe Leu His Phe Gln Val Gly Leu Leu Ile Gln Arg Gly Gln Ser Ser
385 390 395 400

Ser Val Ser Trp Cys Ile Ile Gln Asp Arg Thr Gln Val Ser
405 410

<210> 88
<211> 400
<212> PRT
<213> Homo sapiens

<400> 88

Met Met Asp Leu Arg Asn Thr Pro Ala Lys Ser Leu Asp Lys Phe Ile
1 5 10 15

Glu Asp Tyr Leu Leu Pro Asp Thr Cys Phe Arg Met Gln Ile Asn His
20 25 30

Ala Ile Asp Ile Ile Cys Gly Phe Leu Lys Glu Arg Cys Phe Arg Gly
35 40 45

Ser Ser Tyr Pro Val Cys Val Ser Lys Val Val Lys Gly Gly Ser Ser
50 55 60

Gly Lys Gly Thr Thr Leu Arg Gly Arg Ser Asp Ala Asp Leu Val Val
65 70 75 80

Phe Leu Ser Pro Leu Thr Thr Phe Gln Asp Gln Leu Asn Arg Arg Gly
85 90 95

Glu Phe Ile Gln Glu Ile Arg Arg Gln Leu Glu Ala Cys Gln Arg Glu
100 105 110

Arg Ala Phe Ser Val Lys Phe Glu Val Gln Ala Pro Arg Trp Gly Asn
115 120 125

Pro Arg Ala Leu Ser Phe Val Leu Ser Ser Leu Gln Leu Gly Glu Gly
130 135 140

Val Glu Phe Asp Val Leu Pro Ala Phe Asp Ala Leu Gly Gln Leu Thr

145 150 155 160

Gly Gly Tyr Lys Pro Asn Pro Gln Ile Tyr Val Lys Leu Ile Glu Glu
165 170 175

Cys Thr Asp Leu Gln Lys Glu Gly Glu Phe Ser Thr Cys Phe Thr Glu
180 185 190

Leu Gln Arg Asp Phe Leu Lys Gln Arg Pro Thr Lys Leu Lys Ser Leu
195 200 205

Ile Arg Leu Val Lys His Trp Tyr Gln Asn Cys Lys Lys Lys Leu Gly
210 215 220

Lys Leu Pro Pro Gln Tyr Ala Leu Glu Leu Leu Thr Val Tyr Ala Trp
225 230 235 240

Glu Arg Gly Ser Met Lys Thr His Phe Asn Thr Ala Gln Gly Phe Arg
245 250 255

Thr Val Leu Glu Leu Val Ile Asn Tyr Gln Gln Leu Cys Ile Tyr Trp
260 265 270

Thr Lys Tyr Tyr Asp Phe Lys Asn Pro Ile Ile Glu Lys Tyr Leu Arg
275 280 285

Arg Gln Leu Thr Lys Pro Arg Pro Val Ile Leu Asp Pro Ala Asp Pro
290 295 300

Thr Gly Asn Leu Gly Gly Gly Asp Pro Lys Gly Trp Arg Gln Leu Ala
305 310 315 320

Gln Glu Ala Glu Ala Trp Leu Asn Tyr Pro Cys Phe Lys Asn Trp Asp
325 330 335

Gly Ser Pro Val Ser Ser Trp Ile Leu Leu Ala Glu Ser Asn Ser Ala
340 345 350

Asp Asp Glu Thr Asp Asp Pro Arg Arg Tyr Gln Lys Tyr Gly Tyr Ile
355 360 365

Gly Thr His Glu Tyr Pro His Phe Ser His Arg Pro Ser Thr Leu Gln
370 375 380

Ala Ala Ser Thr Pro Gln Ala Glu Glu Asp Trp Thr Cys Thr Ile Leu
385 390 395 400

<210> 89
<211> 10744
<212> DNA
<213> Homo sapiens

<400> 89
attctccgcg gcgcggcggc gagtgctggc tgcggtaccc tcccgctcgc ttgtccgtct 60

gcgcgcccgc gatgtgacca gccgactggg ggcgggctgg cggctctgcc tgcggcagcg 120

catcggtttt tctcctggca ggtccctaat tgagccgtga ttcccttgga gccagagaca 180

cccaccaggg gctcggagcg ccgcgctccg gggctggagg cagctgcagc gctcgggtcc 240

gcgcggccgg gtgcagtagc ctcagtccca gccgccgcct cgctgagtcg tgtgccctgg 300

cgaatgcccg gcgcccggca cagctgagcc aaggcgactg gtgcagagcg gcggcgcctg 360

gtcctgcact tgccgctgct gccgggctgt gccggggcgg cggcgctgcg cgccggagtt 420

tgcagaggct gcctccgagc gccgtccgcc aggaagactc cctgcctcct gagtcccaaa 480

aacacgagat ttttctccta tttcagcagt tggccacaac ttcattgctg ggaaaaggcc 540

aagaaagaag agagaactct caccacaaca tttcaaaggg aatacattgc cagaacttgg 600

aatactctac tggacaaaca tttcctccaa ggacacagct ctctgcctcc atgtcaccac 660

ctttgaagga ctgactgatt ccctcggctg gtgccagtgc ctgctcctgc catggggccc 720

gcggggagcc tgctgggcag cggacagatg cagatcaccc tgtggggaag tctggctgct 780

gtcgccattt tcttcgtcat caccttcctc atcttcctgt gctctagttg tgacagggaa 840

aagaagccgc gacagcatag tggggaccat gagaacctga tgaacgtgcc ttcagacaag 900

gagatgttca gccgttcagt tactagcctg gcaacagatg ctcctgccag cagtgagcag 960

aatggggcac tcaccaatgg ggacattctt tcagaggaca gtactctgac ctgcatgcag 1020

cattacgagg aagtccagac atcggcctcg gatctgctgg attcccagga cagcacaggg 1080

aaaccaaaat gtcatcagag tcgggagctg cccagaatcc ctcccgagag cgcagtggat 1140

accatgctca cggcgagaag tgtggacggg gaccaggggc tggggatgga agggccctat 1200

gaagtgctca aggacagctc ctcccaagaa aacatggtgg aggactgctt gtatgaaact 1260

gtgaaagaga tcaaggaggt ggctgcagct gcacacctgg agaaaggcca cagtggcaag 1320

gcaaaatcta cttctgcctc gaaagagctc ccagggcccc agactgaagg caaagctgag 1380

tttgctgaat atgcctcggt ggacagaaac aaaaaatgtc gtcaaagtgt taatgtagag 1440

agtatccttg gaaattcatg tgatccagaa gaggaggccc caccacctgt ccctgttaag 1500

cttctggacg agaatgaaaa ccttcaggag aaggaagggg gagaggcgga agagagtgcc 1560

acagacacga ccagtgaaac taacaagaga tttagctcat tgtcatacaa gtctcgggaa 1620

gaagacccca ctctcacaga agaagagatc tcagctatgt actcatcagt aaataaacct 1680

ggacagttag tgaataaatc ggggcagtcg cttacagttc cggagtccac ctacacctcc 1740

attcaagggg acccacagag gtcaccctcc tcctgtaatg atctctatgc tactgttaaa 1800
gacttcgaaa aaactccaaa cagcacactt ccaccagcag ggaggcccag cgaggagcca 1860
gagcctgatt atgaagcgat acagactctc aacagagagg aagaaaaggc caccctgggg 1920
accaatggcc accacggtct cgtcccaaag gagaacgact acgagagcat aagtgacttg 1980
cagcaaggca gagatattac caggctctag caacccagaa gacaaccctg ggtagcctgt 2040
gatcagtgtc tggagacgtt tcttctgtgg aagagaagaa gtgacacaaa cctatacttc 2100
atatgctgct ttagtcacct gaagatggtt ggagaggccc tgtcgactgt tctcccagtt 2160
gttcagtttc tgagacagag aggtacggac taggctgcac ctgagtgtgc ccctgcctgc 2220
cagatggaca ggtacaccca agcacatctc cctgctgcac cctcaccacc cacaaaagat 2280
cccagctgtc agtggtctca tctcattagt gaggaaagcc aagctgtatg gaaaagctgc 2340
actcaccaag gaccacaatg cccccggcct aaagtactgc cattcagaaa agcaggtttt 2400
tcttcctctc tttccttttc tctgtctgct actgacttta aggctttttc cccttgaaa 2460
tgtccagatt cctgtggttc atcccaagga aattttcaca caaagcttgg cctttgccct 2520
caatataggt gttttaggat ggtgacaaac catggctgct gctttctgcc cagctcgcca 2580
gtcctcccca aagagttgcg catcagcacc tggggatctg gaccctgcgg gtgaagggat 2640
ggggagggac gtccctggag tctcttctgt ctttgttcct tcttattttg gcattcgata 2700
tcagcagcct ctccccaaag tacttgaagt cagttttaga tgctttattt tatttttcta 2760
gtcaaaaacg tgtttccccc agtgtttgaa aactcgtccg aatcttttca gtattttcca 2820
tgagtattgt ggtacttcta gacttgttta agcccagaac tcattccttc aaaacagaga 2880
gccttaatct ttatgttggg acacagacca catatttgga cggcagccat gcatccatcg 2940
ctgaagggct gtggacatga atgtgtattt cccatggtct ccgctgccca caccaacagt 3000
gtggcatctc ataagttaac tgctacccta aggtaatcta agattaaaat gtaaacattt 3060
atttttgtta tgtaagttta taagatgttt tatgttcaat gcctaatttc tcaaaagtgc 3120
cagaaaaaaa tgtatattag ctattttgat tttatgtaca atgatttata ctctcctttt 3180
gaaaagatac cataaagcac ataagctaga tcactacaag gagctgttat ctttttttcta 3240
atcaagtgtt taaaacactg atggttttta aagactcacc tttttaaatg gtacttggag 3300
ctcctgattc aaattaccta gaccccctag agaaataaat ggaatataca taaataatca 3360
ttttcagtgg tttatggtgg gcaatattgc aatatttgaa atggtaaaaa tggaaagaag 3420
aacaaaatat gatgagaggt ggctgtgaat tataaacctc ataaaagtgt cataattcca 3480
ttaaggttta attatatttt ttcagaaaac agtgatgaat tctgtagtcc agtgcttgcc 3540
aatgcaaatt gcctattgga atcttcttcc tatattttac aaacatcagt ggctgaaata 3600

gctcagagta agagctcagc ctggtttgaa tttaatcatc tctttagatc ttataaggcc 3660
agcattagga aacttgttca cttttcattt tcaaaggagc ctagttgaag tgctattatg 3720
agtgtgggct atggaaagac agcttttcct acactgataa agaaaaaaaa tgaggaaatt 3780
atttcatccc cttgtgacat ctgtgacttt ttggatttaa taatcttgct gtttttcctc 3840
tttatgacaa agaatataat tgggaggatg aagtgtctta aaaattgtag agaccagctc 3900
actggaatgt ttttccatcc ctgtattcat ggcttgactt tgtgactgct ctacactgca 3960
tgtctgacat tgcagagtga gctatgttga ggtaaactgg ttggttgtca ttattttgca 4020
atcagcctgg tctctcccat gaagatgtcg tgtgcataag cacaatcatc actgattaga 4080
agatcacagc agaataccct tggattagag agaagttcgt accttgcatt tctctgaatt 4140
ctagtctctc ataagcactg ctttgctgga tgattttcac tgctttgtgt taatgacttt 4200
gagcgatctc tcacatgatg gggttcttta gtacatggta acagccatgt catcttacac 4260
acctagcatt gtgaatgctg tagtgacatc ctttataggc accttacagc tcaaaacttt 4320
tgtttcattt catgccttac ttatcaaaaa ggcaggaaag taggtatgat ctctaaagta 4380
aaaaaaaaaa aaaaaaaaaa actttttata gaaagctcat aaataatcat gtcattttgc 4440
aattttgtta ccaaaatttc ccccaagagt tttcaaatat tagttctgca atgtggctat 4500
gaaatatgca ctgaaatata ccttttaatt tgagaaccag tggttagaat aagctgtgat 4560
ataaagtatt ttcagtgtac ttttaaagga actataaggc cctccagcat aaacgctaaa 4620
agaatagatg gtagcacagg ccatgagggc tgggggagag aagcagagtg aaccttagaa 4680
agatggctca gctatttgga gcactggata ttttactgaa gttatttact gaggcaccat 4740
cactgttttg actgtacagt atagtttttc ataaatttca tcacatttac tttgttcaga 4800
atctgggctt gaatctttga gttggacaaa agcctatggt ttcttttaaa aagtttcatc 4860
ttgagctaat gctacagttt aaataaaatg tatgaaaggt agttatcaaa aacaatttgt 4920
atatttaaaa ttctattttg acatccattt tttacagcga attatagcag gttatgtgaa 4980
atttaaaaat aaattttagg aaattttttt atcttgtaag taagaattat aaatctatct 5040
cagttaaaag taggatcttt gtttttattc agatattttg aaagtttaga agcattttgt 5100
atgctacttt ggtatttctt aagtatagtc agcaggaaag ggtaacctgt tttatgcaaa 5160
atttttttaa tttgtcaaat gtttttagtg tgtctacttt ctgaaatagt ctcaatatcc 5220
tgtctgtatc ctaggcagat aactacacaa gaacaaaacg ctatatagaa aagaaaatat 5280
ttgaggaaat cttaaattcc ataagaaagt aattcttgca agaatgttgg ctacatattt 5340
ttttttctgt cctgaataaa ttacatcatt aggtctgttg aagcttttga agctaccact 5400
attctttctg ggataaaggc taattactga tttatctgcc ctcaaaatcc cagagttaat 5460
tctaattgag tactaaatta acagtaagta gttaacacag agaactaaaa tttaaccgca 5520

ttatatcact gtatatattt ctcatgattt ttgctaatta gagcatttac ttaaccagta 5580

ataaactaca cacacacaca cacacacaca cacgtatacg tgtgtgtgtg tatatacttg 5640

gctctgacac aactctggtg cttaattagg atatgttcta ttatgtattt tgaatgttta 5700

tcctgctagt gtgatgaact tttgttggaa aagcagaatt agaaggaaca gtttttcaat 5760

cagtttcatt tggtaattac aggaaaacct tgagttgctc taatttaatt tttccaatta 5820

atacatttag actttaaatt gcatccgtaa ttctcttggc tgaaaaacag tggtatttta 5880

agacagtttt gttatacatg tgacttactt ttgactcagc ctgagaggag gaaatgtaaa 5940

gagataagag atttggccca aagcagcaag agactagtaa ccttgagcca ggacgaagcc 6000

aagacaacgt gactcccaag tagcaggagc agaggtcggt cacctgtggc attctgtttc 6060

attccctttt aaaattatga gactttatag gcagtgtaaa acatcagcag gcagcgatgt 6120

gataggatgt gcgaaaaagc tggtctgata ccatggctat aattacagag ctttagttca 6180

attaggattt tgtaaatgag caaatgacct tttttccag tgccccttgt aatagttaat 6240

atgagtccat gcaatcttgt gatgccattc tcctgaaggt gttgatttct ggtacccagc 6300

cagcttagct ttggctgctg gaagcacagt aggtactgat gattacttcc tggaaatctt 6360

gacaggctta ttgtactgtg taatggggaa aagttaaagt ataatgtttg gtgttaataa 6420

atgactgatg tgaaaatagg aacatgtgtc tttaatatca aatatggctt tatttgcagt 6480

gaaatctaaa ttccttattc tacagtagta ttttcttgcc ctgtgttgta tattttccta 6540

aatacatttt tcctgacagt ggctttaggc cagttgaaca cacttagact gaaagtgttt 6600

aacttaaaga gatcagtcca gaaaccatga taaattagaa tctttatctg gaattaccaa 6660

attaaaattt aaaatcatgg tccagaagag aacaaacatt catggttttt tttatcctac 6720

tgctcatttt taggtctgtg tttacatcta gtctctacta tttgtgaagt atgtcattat 6780

ttttttcaag ttcttctttg acttaactga aaaataatat tccaaattct taatgtaaca 6840

tgaaaagaga aatacaattt ttgcttaaaa gacatttttt aaaaagcgtt caattcagta 6900

atcatttctg ttaatacagg aagttttttt ggcttgccag ttatatactg tgggtatttt 6960

ttaaaatgtg ctacccttgg gttgtctcat atcaccttgc gtaatcatgt attacaaagg 7020

ttgataattg tcatttctca gatgtctgta tgttacattg gcagcagtaa aatgttttaa 7080

tgttgttacc ttttaaataa ttgtgttgta ttaacatgcc tcatattttc tggggaaact 7140

tgaaatatat ctaaacaaaa aaggtctgtt ataaatagga attggcattt cattgttaat 7200

gtttttttcct cataaaaata gttacaccaa aagtgacata ttgtctatta catgggccca 7260

atcagtatta aagtactccc cttactcaaa aatattaaaa aataattcat gacacttagt 7320

agcattttca ttgattgttt caaaagatct tcataatggt caaattgtcc aattcacaaa 7380

agagtgagaa agttgttttc agtactggga atttttaaac cttagtttta agaccaaaaa 7440
tatctcttat tagcttgaac attttgtgat tacttttccc tccccggatc tagtcctttt 7500
aaggagtaag tctaaagaat gaggccatga agtcactatt tcctcccacc tcagtttgtc 7560
tcctggtact tagctggcct atcgctttgt gtggcaatac ccctgggtcg cttccccact 7620
cagccttctg tatctgctgt tcacaggaca tgccattatg tctttccagt acggatcaca 7680
gtgctgccat atcacagacc ctgccagccg gcacagaacc atgcctcgtc acagctctgc 7740
ccaactcaca ggctgccaag atgaggccac aggtccacct agggcggcag atgctggtgc 7800
tatttgtcac gacttttgcc aaaacatgta cactgttgct cacatctttc gtaagaatgg 7860
ttgacactaa ggggccatgg aaaagcactt ttaaaaaatt atgtggtgga atcaaactca 7920
cagaggcaga aatgttagtg ccgtgctcta accaagtaag cttagtgttc cccacagtga 7980
tctgtatctg gcctgagtct cctcaagcag caacccccac tgtccagcat gtggaaggta 8040
gatccttatg acaacaccag acccatagtt accaggaaag aaaaaggaag ctgagtgttg 8100
tgaaggggaa gaaatccttt ttataagaag taatcatttt taggccgggt gcagtggctc 8160
atgcctgtaa tcccagcact ttgggaggct gagacaggtg gatcacttga ggtcaggagt 8220
ttgagacctg cctggccaac atgatgaaac cctgtctcta ctaaaaatgc aaaaaaaatt 8280
agccaggcat ggtggcatgt gcctgtagtc ccaactactc aggaggctga ggcaggagaa 8340
tcacttgaac ctgggaggca gaggttgcaa tgagctgaga tcgcaacact gcactccagc 8400
ctgggcgaca gagcaagact ccatctcaaa aacaaaaaca aaaaaaaaac ctttttaaag 8460
taaattgagt gtaaaatgtt ttcaccatga agccattgca ccctctcccc caggaaacag 8520
tgcctaagga tgatgtcaag ttacagccag tttgctctga tcccccagga cctaagaaaa 8580
ctgtgagtgc ttcagcatgc aaaggtagag ctaccagata aggagaataa cttggccatt 8640
tatagcaaac tgcctgactt tggagtgaga aaccaagcac cttctaggtc ctaggatagg 8700
tgaattggag gtgcagctgc tgaagttccc attttctaca tacttcagga gccagggtgt 8760
ttcatttggt tttgtaaaac tttgctgtaa gtcatcacag ttaattcttt gaatggacat 8820
tttagagtgt aacttttttt caaaattagc agaacagata tctctgctca ttctctttat 8880
acatttagtt ttttaaagtt cccctagtat acctgttagg cactctaaat aggacacagt 8940
taaagctaaa agaaggacca gttgctccct agagcctaca gttcagaact atcttgttgt 9000
catgtcctgg tttggtttgg tttgttttta actcaacaag ttggaaacca tgagtttaaa 9060
gcaaactatt tccttttctt aagcttaaag agaatttttt ttttaatctt tagctcctca 9120
tttaggtagt gaaggcttcc ctgaaagaca gcagtgccct tctctggaag aagaggctgg 9180
gaccaaaagc ctgagtctat gggttgagga ttttgagcat tcctttgaca agcttctatg 9240
tatttttaat ataagactca taaatgaacc tgatgtggtg ttcatgaccc atcccttaag 9300

catgcttgct ttttttaact ttactttttg agtatagtac ttgtcttgtt tttcctggag 9360

ccatgctagg atttaattcc tatgtgccac tacccgccca tgtcctggaa gatggccatg 9420

cctgcagcaa tgctcatgtc ttgaggagaa gcaatgtgaa aagcatcagg aaagctgaac 9480

cttacgttca agtcagcaaa catttgtgag cattctagta tgcttgcgac cttgacactt 9540

tttaaaaatt atctttttat ttgcttcgag tatcaaccgt atagttgact ctagtggggc 9600

gaagggagca gagatgagag caaaattcag acttgctgcc cctgtgtgtc tcagttctca 9660

tgtctctctg agaaatgtgt tggcccaatt ccctatctgc cactctgagt ctcttgagta 9720

aatgtgtgta aatgagaaaa attatctcaa agatttaact tattatttag atattttctt 9780

ctttttaaac tcaggaataa acctggtttg tagataatat aatatggcta ctagagctat 9840

aattgagtag gtgactagaa gcatttttc aaatatattt cttgtaaaga catctacttt 9900

gttttaacta ggaaaagaaa ctcctcatat taaaataaac atttccataa gcactttcta 9960

gaagagtgta gcatccatct accatccatc ccagaatgtt tgtatcttgc tcatagtatt 10020

tcaaaaattt ttatgggtga aaaaaatgca ctttttttatt tattggaaaa gtgtagagtt 10080

attgaattaa attctcaagt gggacaattt agtgtaaagg gaaacatggg ttttggagac 10140

aagagcacag ggctgtggtc cctgttccag cattgacttt actatgtgac ctcgagcaaa 10200

tgatttaact tctctgtgcc tcagtttctc catatacaaa atggggataa tgatactaac 10260

cattgcctac ctcatagaga tgttatgggg acaaacgaaa taatagagat aagcacgaat 10320

gctttcaact cttcggaaga aaggtgctat ataaattgaa gttgtgtttt taatgatcca 10380

attattaatt atgtttaggg tttaaataac aatactgtta gtcatgttaa gaataagttt 10440

tgttttgatg catatttcca cttctcttct gtggccagat cttgatcatt caaccagtaa 10500

tggtacctga ggaactgaaa tgggtatttg ttttcgtatg tttctgccag tagtatttca 10560

attgaatatc cagaaaagac tggagtttaa cttgtaatat cttatagttt acatgtaata 10620

aaacttattc aagctatata taaaagtatg attacatgta aatagcaggt atgttgtaat 10680

taaaggcact tatcaaattg tctttgttct tttttaattg taataaaagt cagttttaca 10740

taaa 10744

<210> 90
<211> 432
<212> PRT
<213> Homo sapiens

<400> 90

Met Gly Pro Ala Gly Ser Leu Leu Gly Ser Gly Gln Met Gln Ile Thr
1 5 10 15

Leu Trp Gly Ser Leu Ala Ala Val Ala Ile Phe Phe Val Ile Thr Phe
20 25 30

Leu Ile Phe Leu Cys Ser Ser Cys Asp Arg Glu Lys Lys Pro Arg Gln
35 40 45

His Ser Gly Asp His Glu Asn Leu Met Asn Val Pro Ser Asp Lys Glu
50 55 60

Met Phe Ser Arg Ser Val Thr Ser Leu Ala Thr Asp Ala Pro Ala Ser
65 70 75 80

Ser Glu Gln Asn Gly Ala Leu Thr Asn Gly Asp Ile Leu Ser Glu Asp
85 90 95

Ser Thr Leu Thr Cys Met Gln His Tyr Glu Glu Val Gln Thr Ser Ala
100 105 110

Ser Asp Leu Leu Asp Ser Gln Asp Ser Thr Gly Lys Pro Lys Cys His
115 120 125

Gln Ser Arg Glu Leu Pro Arg Ile Pro Pro Glu Ser Ala Val Asp Thr
130 135 140

Met Leu Thr Ala Arg Ser Val Asp Gly Asp Gln Gly Leu Gly Met Glu
145 150 155 160

Gly Pro Tyr Glu Val Leu Lys Asp Ser Ser Ser Gln Glu Asn Met Val
165 170 175

Glu Asp Cys Leu Tyr Glu Thr Val Lys Glu Ile Lys Glu Val Ala Ala
180 185 190

Ala Ala His Leu Glu Lys Gly His Ser Gly Lys Ala Lys Ser Thr Ser
195 200 205

Ala Ser Lys Glu Leu Pro Gly Pro Gln Thr Glu Gly Lys Ala Glu Phe
210 215 220

Ala Glu Tyr Ala Ser Val Asp Arg Asn Lys Lys Cys Arg Gln Ser Val
225 230 235 240

Asn Val Glu Ser Ile Leu Gly Asn Ser Cys Asp Pro Glu Glu Glu Ala
245 250 255

Pro Pro Pro Val Pro Val Lys Leu Leu Asp Glu Asn Glu Asn Leu Gln
260 265 270

Glu Lys Glu Gly Gly Glu Ala Glu Glu Ser Ala Thr Asp Thr Thr Ser
275 280 285

Glu Thr Asn Lys Arg Phe Ser Ser Leu Ser Tyr Lys Ser Arg Glu Glu
290 295 300

Asp Pro Thr Leu Thr Glu Glu Glu Ile Ser Ala Met Tyr Ser Ser Val
305 310 315 320

Asn Lys Pro Gly Gln Leu Val Asn Lys Ser Gly Gln Ser Leu Thr Val
325 330 335

Pro Glu Ser Thr Tyr Thr Ser Ile Gln Gly Asp Pro Gln Arg Ser Pro
340 345 350

Ser Ser Cys Asn Asp Leu Tyr Ala Thr Val Lys Asp Phe Glu Lys Thr
355 360 365

Pro Asn Ser Thr Leu Pro Pro Ala Gly Arg Pro Ser Glu Glu Pro Glu
370 375 380

Pro Asp Tyr Glu Ala Ile Gln Thr Leu Asn Arg Glu Glu Glu Lys Ala
385 390 395 400

Thr Leu Gly Thr Asn Gly His His Gly Leu Val Pro Lys Glu Asn Asp
405 410 415

Tyr Glu Ser Ile Ser Asp Leu Gln Gln Gly Arg Asp Ile Thr Arg Leu
420 425 430

<210> 91
<211> 8240
<212> DNA
<213> Homo sapiens

<400> 91
cccctctcgg tagccctgag gctctggcgc cttcaagtga gaagctaagc accagcctct 60

gctgggctgc agaagcggcg gcggcggcag cagcagcagc agcatcagga aggcgctcgg 120

gccagcgcgg tgaacccggg ctgggcagca ggtcgcggag ccgcgagcca ggatggaggc 180

agagggcagc agcgcgccgg cccgggcggg cagcggagag ggcagcgaca gcgccggcgg 240

ggccacgctc aaagccccca agcatctctg gaggcacgag cagcaccacc agtacccgct 300

ccggcagccc cagttccgcc tcctgcatcc ccatcaccac ctgcccccgc cgccgccacc 360

ctcgccccag ccccagcccc agtgtccgct acagccgccg ccgccgcccc ccctgccgcc 420

gcccccgccg ccgcccgggg ctgcccgcgg ccgctacgcc tcgagcgggg ccaccggccg 480

cgtccggcat cgcggctact cggacaccga gcgctacctg tactgtcgcg ccatggaccg 540
cacctcctac gcggtggaga ccggccaccg gcccggcctg aagaaatcca ggatgtcctg 600
gccctcctcg ttccagggac tcaggcgttt tgatgtggac aatggcacat ctgcgggacg 660
gagtcccttg gatcccatga ccagcccagg atccgggcta attctccaag caaattttgt 720
ccacagtcaa cgacgggagt ccttcctgta tcgatccgac agcgattatg acctctctcc 780
aaagtctatg tcccggaact cctccattgc cagtgatata cacggagatg acttgattgt 840
gactccattt gctcaggtct tggccagtct gcgaactgta cgaaacaact ttgctgcatt 900
aactaatttg caagatcgag cacctagcaa aagatcaccc atgtgcaacc aaccatccat 960
caacaaagcc accataacag aggaggccta ccagaaactg gccagcgaga ccctggagga 1020
gctggactgg tgtctggacc agctagagac cctacagacc aggcactccg tcagtgagat 1080
ggcctccaac aagtttaaaa ggatgcttaa tcgggagctc acccatctct ctgaaatgag 1140
tcggtctgga aatcaagtgt cagagtttat atcaaacaca ttcttagata agcaacatga 1200
agtggaaatt ccttctccaa ctcagaagga aaaggagaaa aagaaaagac caatgtctca 1260
gatcagtgga gtcaagaaat tgatgcacag ctctagtctg actaattcaa gtatcccaag 1320
gtttggagtt aaaactgaac aagaagatgt ccttgccaag gaactagaag atgtgaacaa 1380
atggggtctt catgttttca gaatagcaga gttgtctggt aaccggccct tgactgttat 1440
catgcacacc atttttcagg aacgggattt attaaaaaca tttaaaattc cagtagatac 1500
tttaattaca tatcttatga ctctcgaaga ccattaccat gctgatgtgg cctatcacaa 1560
caatatccat gctgcagatg ttgtccagtc tactcatgtg ctattatcta cacctgcttt 1620
ggaggctgtg tttacagatt tggagattct tgcagcaatt tttgccagtg caatacatga 1680
tgtagatcat cctggtgtgt ccaatcaatt tctgatcaat acaaactctg aacttgcctt 1740
gatgtacaat gattcctcag tcttagagaa ccatcatttg gctgtgggct ttaaattgct 1800
tcaggaagaa aactgtgaca ttttccagaa tttgaccaaa aaacaaagac aatctttaag 1860
gaaaatggtc attgacatcg tacttgcaac agatatgtca aaacacatga atctactggc 1920
tgatttgaag actatggttg aaactaagaa agtgacaagc tctggagttc ttcttcttga 1980
taattattcc gataggattc aggttcttca gaatatggtg cactgtgcag atctgagcaa 2040
cccaacaaag cctctccagc tgtaccgcca gtggacggac cggataatgg aggagttctt 2100
ccgccaagga gaccgagaga gggaacgtgg catggagata agccccatgt gtgacaagca 2160
caatgcttcc gtggaaaaat cacaggtggg cttcatagac tatattgttc atcccctctg 2220
ggagacatgg gcagacctcg tccaccctga cgcccaggat attttggaca ctttggagga 2280
caatcgtgaa tggtaccaga gcacaatccc tcagagcccc tctcctgcac ctgatgaccc 2340
agaggagggc cggcagggtc aaactgagaa attccagttt gaactaactt tagaggaaga 2400

tggtgagtca gacacggaaa aggacagtgg cagtcaagtg gaagaagaca ctagctgcag 2460
tgactccaag actctttgta ctcaagactc agagtctact gaaattcccc ttgatgaaca 2520
ggttgaagag gaggcagtag gggaagaaga ggaaagccag cctgaagcct gtgtcataga 2580
tgatcgttct cctgacacgt aacagtgcaa aaactttcat gccttttttt tttttaagta 2640
gaaaaattgt ttccaaagtg catgtcacat gccacaacca cggtcacacc tcactgtcat 2700
ctgccaggac gtttgttgaa caaaactgac cttgactact cagtccagcg ctcaggaata 2760
tcgtaaccag ttttttcacc tccatgtcat ccgagcaagg tggacatctt cacgaacagc 2820
gtttttaaca agatttcagc ttggtagagc tgacaaagca gataaaatct actccaaatt 2880
attttcaaga gagtgtgact catcaggcag cccaaaagtt tattggactt ggggtttcta 2940
ttccttttta tttgtttgca atattttcag aagaaaggca ttgcacagag tgaacttaat 3000
ggacgaagca acaaatatgt caagaacagg acatagcacg aatctgttac cagtaggagg 3060
aggatgagcc acagaaattg cataattttc taatttcaag tcttcctgat acatgactga 3120
atagtgtggt tcagtgagct gcactgacct ctacattttg tatgatatgt aaaacagatt 3180
ttttgtagag cttactttta ttattaaatg tattgaggta ttatatttaa aaaaaactat 3240
gttcagaact tcatctgcca ctggttattt ttttctaagg agtaacttgc aagttttcag 3300
tacaaatctg tgctacactg gataaaaatc taatttatga attttacttg caccttatag 3360
ttcatagcaa ttaactgatt tgtagtgatt cattgtttgt tttatatacc aatgacttcc 3420
atattttaaa agagaaaaac aactttatgt tgcaggaaac ccttttgta agtctttatt 3480
atttactttg cattttgttt cactctttcc agataagcag agttgctctt caccagtgtt 3540
tttcttcatg tgcaaagtga ctatttgttc tataatactt ttatgtgtgt tatatcaaat 3600
gtgtcttaag cttcatgcaa actcagtcat cagttcgtgt tgtctgaagc aagtgggaga 3660
tatataaata cccagtagct aaaatggtca gtctttttta gatgttttcc tacttagtat 3720
ctcctaataa cgttttgctg tgtcactaga tgttcatttc acaagtgcat gtctttctaa 3780
taatccacac atttcatgct ctaataatcc acacatttca tgctcatttt tattgttttt 3840
acagccagtt atagtaagaa aaaggttttt cccctgtgc tgctttataa tttagcgtgt 3900
gtctgaacct tatccatgtt tgctagatga ggtcttgtca aatatatcac taccattgtc 3960
accggtgaaa agaaacaggt agttaagtta gggttaacat tcatttcaac cacgaggttg 4020
tatatcatga ctagctttta ctcttggttt acagagaaaa gttaaacagc caactaggca 4080
gtttttaaga atattaacaa tatattaaca aacaccaata caactaatcc tatttggttt 4140
taatgatttc accatgggat taagaactat atcaggaaca tccctgagaa acggttttaa 4200
gtgtagcaac tactcttcct taatggacag ccacataacg tgtaggaagt cctttatcac 4260

ttatcctcga tccataagca tatcttgcag aggggaacta cttctttaaa cacatggagg 4320
gaaagaagat gatgccactg gcaccagagg gttagtactg tgatgcatcc taaaatattt 4380
attatattgg taaaaattct ggttaaataa aaaattagag atcactcttg gctgatttca 4440
gcaccaggaa ctgtattaca gttttagaga ttaattccta gtgtttacct gattatagca 4500
gttggcatca tggggcattt aattctgact ttatccccac gtcagcctta ataaagtctt 4560
ctttaccttc tctatgaaga ctttaaagcc caaataatca tttttcacat tgatattcaa 4620
gaattgagat agatagaagc caaagtgggt atctgacaag tggaaaatca aacgtttaag 4680
aagaattaca actctgaaaa gcatttatat gtggaacttc tcaaggagcc tcctggggac 4740
tggaaagtaa gtcatcagcc aggcaaatga ctcatgctga agagagtccc catttcagtc 4800
ccctgagatc tagctgatgc ttagatcctt tgaaataaaa attatgtctt tataactctg 4860
atcttttaca taaagcagaa gaggaatcaa ctagttaatt gcaaggtttc tactctgttt 4920
cctctgtaaa gatcagatgg taatctttca aataagaaaa aaataaagac gtatgtttga 4980
ccaagtagtt tcacaagaat atttgggaac ttgtttcttt taattttatt tgtccctgag 5040
tgaagtctag aaagaaaggt aaagagtcta gagtttattc ctctttccaa aacattctca 5100
ttcctctcct ccctacactt agtatttccc ccacagagtg cctagaatct taataatgaa 5160
taaaataaaa agcagcaata tgtcattaac aaatccagac ctgaaagggt aaagggttta 5220
taactgcact aataaagaga ggctcttttt ttttcttcca gtttgttggt ttttaatggt 5280
accgtgttgt aaagataccc actaatggac aatcaaattg cagaaaaggc tcaatatcca 5340
agagacaggg actaatgcac tgtacaatct gcttatcctt gcccttctct cttgccaaag 5400
tgtgcttcag aaatatatac tgctttaaaa aagaataaaa gaatatcctt ttacaagtgg 5460
ctttacattt cctaaaatgc cataagaaaa tgcaatatct gggtactgta tggggaaaaa 5520
aatgtccaag tttgtgtaaa accagtgcat ttcagcttgc aagttactga acacaataat 5580
gctgttttaa ttttgtttta tatcagttaa aattcacaat aatgtagata gaacaaatta 5640
cagacaagga aagaaaaaac ttgaatgaaa tggattttac agaaagcttt atgataattt 5700
ttgaatgcat tatttatttt ttgtgccatg catttttttt ctcaccaaat gaccttacct 5760
gtaatacagt cttgtttgtc tgtttacaac catgtattta ttgcaatgta catactgtaa 5820
tgttaattgt aaattatctg ttcttattaa aacatcatcc catgatggga tggtgttgat 5880
atatttggaa actcttggtg agagaatgaa tggtgtgtat acatactctg tacatttttc 5940
ttttctcctg taatatagtc ttgtcacctt agagcttgtt tatggaagat tcaagaaaac 6000
tataaaatac ttaaagatat ataaatttaa aaaaacatag ctgcaggtct ttggtcccag 6060
ggctgtgcct taactttaac caatattttc ttctgttttg ctgcatttga aaggtaacag 6120
tggagctagg gctgggcatt ttacatccag gcttttaatt gattagaatt ctgccaatag 6180

gtggatttta caaaaccaca gacaacctct gaaagattct gagacccttt tgagacagaa 6240

gctcttaagt acttcttgcc agggagcagc actgcatgtg tgatggttgt ttgccatctg 6300

ttgatcagga actacttcag ctacttgcat ttgattattt cctttttttt tttttttaac 6360

tcggaaacac aactggggaa atatattctt tcccagtgat tataaacaat ctttttcttt 6420

tttttaagtc cttttggctt ctagagctca taggaaaatg gacttgattt gaaattggag 6480

ccagagttta ctcgtgttgg ttatctattc atcagcttcc tgacatgtta agagaataca 6540

ttaaagagaa aatactgttt tttaatccta aaatttttct tccactaaga taaaccaaat 6600

gtccttacat atatgtaaac ccatctattt aaacgcaaag gtgggttgat gtcagtttac 6660

atagcagaaa gcattcacta tcctctaaga tttgtttctg caaaactttc attgctttag 6720

aattttaaaa tttcaccttg tacaatggcc agcccctaaa gcaggaaaca tttataatgg 6780

attatatgga aacatcctcc cagtacttgc ccagcccttg aatcatgtgg cttttcagtg 6840

aaaggaaaga ttctttttct aggaaaaatg agcctatttt attttatttt attttatttt 6900

ttgacacaaa ctgtagattt tagcagccct ggcccaaagg aatttgatta cttttgtttt 6960

aaacagtaca aaggggacac tataattaca aaaacatcct taactgattt gagttgtttt 7020

tatttctttg gatatatttt cagagtggta aattgtgtgt gagaattaca aatgattatt 7080

cttttagtgg tttcttagcc tctcttacag cccacgggga tagtactgta catcaatacc 7140

ttcatatgaa atttttatat gcaatgaaaa taaaagcatg ggttgattct gcctatttat 7200

gactcaatct tttacaaata aaagattatt cattttaaat tatagttcaa tcagcatgtc 7260

tcttaggata ctgaacgtgg ttgaaatgaa aggatagtga catcataagt tagtactgat 7320

attcataacc aaataaagcc aacttgagta attttgctac attaaaaatt accaaaatta 7380

cttagatggc ctataagatt aagcatggtg ttttctaagc aagctttgaa aggggccttc 7440

catacttact taattgaata ttctgggata ttgaaaatta ttcagatact tgacaattat 7500

ttttggttac ctactccgca aactacaaag ttttaaggac tcaacaataa gttaatgaga 7560

cacagtgttt gctttcatgg agcttacagt ctggagggga caaaggctta aacaatactc 7620

atataattat atatgtgatc agtacaatga aggagctcag tggggtaaat aagcaggaac 7680

ctgaacttga tctgttccgg agggccacag aaggcttcct tgaggccttg agaaagtgat 7740

ttgcatctga gttctgaagg attgtaagag gtaactaggg aaaaagttga caggaagagg 7800

aaggggatcc agacaagaaa catttgcaaa gatcttgagg cataaatgag cttgagacat 7860

ctggagaaac tgaggaaaag tgagagagta ggcagggcct ggagccgcag agccattgct 7920

aaccatcctg tgtgagatat cccccattct gtagctttat tctcataacc ctgctcaatt 7980

ttctttataa cacttctcac agatttatat acgtgtttgt ttttgttatc tgtctctccc 8040

accagaccac agctccatga gagcaaggtc tttgcttacc aatatatcac tagcacttaa 8100

aactatgcct ggtacacagt aggttcttaa tatgtgttga atatagccat caaattgata 8160

ttggatataa ttcaatctga taagatattt tgagatatta aagagttttt aacttgatac 8220

cataaaaaaa aaaaaaaaaa 8240

<210> 92
<211> 8153
<212> DNA
<213> Homo sapiens

<400> 92
aatacttgtt gcaataattg cccacgatag ctgctcaaac aagagagttg gaattcatct 60

gtaaaaatca ctacatgtaa cgtaggagac aagaaaaata ttaatgacag aagatctgcg 120

aacatgatgc acgtgaataa ttttcccttt agaaggcatt cctggatatg ctgatggtaa 180

cagcagaaaa atgtgaactc tgaataaaga ggtgggagtt tttcagcaca taaagaatat 240

ttaaagccaa ttcattggat gcattgacca gtaagtgtag agatcaaaat caagaacaac 300

tccaagaatt gagagcagat gcaaacccca atttttgtgg gtctccagtc cagccttccc 360

aggaatgctg gtctgactac tcagatttgc ttttacttct tgccttttgg atataatgag 420

tttgccaagc agctgtgagt acctgactct ggggaaggtg gctagattcc gaagcgctta 480

tgttcatgga tcaccatacg cgatcaacat gccaattgat attaagccac agaggagacg 540

ttttgatgtg gacaatggca catctgcggg acggagtccc ttggatccca tgaccagccc 600

aggatccggg ctaattctcc aagcaaattt tgtccacagt caacgacggg agtccttcct 660

gtatcgatcc gacagcgatt atgacctctc tccaaagtct atgtcccgga actcctccat 720

tgccagtgat atacacggag atgacttgat tgtgactcca tttgctcagg tcttggccag 780

tctgcgaact gtacgaaaca actttgctgc attaactaat ttgcaagatc gagcacctag 840

caaaagatca cccatgtgca accaaccatc catcaacaaa gccaccataa cagaggaggc 900

ctaccagaaa ctggccagcg agaccctgga ggagctggac tggtgtctgg accagctaga 960

gaccctacag accaggcact ccgtcagtga gatggcctcc aacaagttta aaaggatgct 1020

taatcgggag ctcacccatc tctctgaaat gagtcggtct ggaaatcaag tgtcagagtt 1080

tatatcaaac acattcttag ataagcaaca tgaagtggaa attccttctc caactcagaa 1140

ggaaaaggag aaaaagaaaa gaccaatgtc tcagatcagt ggagtcaaga aattgatgca 1200

cagctctagt ctgactaatt caagtatccc aaggtttgga gttaaaactg aacaagaaga 1260

tgtccttgcc aaggaactag aagatgtgaa caaatggggt cttcatgttt tcagaatagc 1320

agagttgtct ggtaaccggc ccttgactgt tatcatgcac accattttttc aggaacggga 1380

tttattaaaa acatttaaaa ttccagtaga tactttaatt acatatctta tgactctcga 1440

agaccattac catgctgatg tggcctatca caacaatatc catgctgcag atgttgtcca 1500
gtctactcat gtgctattat ctacacctgc tttggaggct gtgtttacag atttggagat 1560
tcttgcagca atttttgcca gtgcaataca tgatgtagat catcctggtg tgtccaatca 1620
atttctgatc aatacaaact ctgaacttgc cttgatgtac aatgattcct cagtcttaga 1680
gaaccatcat ttggctgtgg gctttaaatt gcttcaggaa gaaaactgtg acattttcca 1740
gaatttgacc aaaaaacaaa gacaatcttt aaggaaaatg gtcattgaca tcgtacttgc 1800
aacagatatg tcaaaacaca tgaatctact ggctgatttg aagactatgg ttgaaactaa 1860
gaaagtgaca agctctggag ttcttcttct tgataattat tccgatagga ttcaggttct 1920
tcagaatatg gtgcactgtg cagatctgag caacccaaca aagcctctcc agctgtaccg 1980
ccagtggacg gaccggataa tggaggagtt cttccgccaa ggagaccgag agagggaacg 2040
tggcatggag ataagcccca tgtgtgacaa gcacaatgct tccgtggaaa aatcacaggt 2100
gggcttcata gactatattg ttcatcccct ctgggagaca tgggcagacc tcgtccaccc 2160
tgacgcccag gatattttgg acactttgga ggacaatcgt gaatggtacc agagcacaat 2220
ccctcagagc ccctctcctg cacctgatga cccagaggag ggccggcagg gtcaaactga 2280
gaaattccag tttgaactaa ctttagagga agatggtgag tcagacacgg aaaaggacag 2340
tggcagtcaa gtggaagaag acactagctg cagtgactcc aagactcttt gtactcaaga 2400
ctcagagtct actgaaattc cccttgatga acaggttgaa gaggaggcag taggggaaga 2460
agaggaaagc cagcctgaag cctgtgtcat agatgatcgt tctcctgaca cgtaacagtg 2520
caaaaacttt catgcctttt tttttttaa gtagaaaaat tgtttccaaa gtgcatgtca 2580
catgccacaa ccacggtcac acctcactgt catctgccag gacgtttgtt gaacaaaact 2640
gaccttgact actcagtcca gcgctcagga atatcgtaac cagtttttc acctccatgt 2700
catccgagca aggtggacat cttcacgaac agcgttttta acaagatttc agcttggtag 2760
agctgacaaa gcagataaaa tctactccaa attattttca agagagtgtg actcatcagg 2820
cagcccaaaa gtttattgga cttggggttt ctattccttt ttatttgttt gcaatatttt 2880
cagaagaaag gcattgcaca gagtgaactt aatggacgaa gcaacaaata tgtcaagaac 2940
aggacatagc acgaatctgt taccagtagg aggaggatga gccacagaaa ttgcataatt 3000
ttctaatttc aagtcttcct gatacatgac tgaatagtgt ggttcagtga gctgcactga 3060
cctctacatt ttgtatgata tgtaaaacag atttttgta gagcttactt ttattattaa 3120
atgtattgag gtattatatt taaaaaaaac tatgttcaga acttcatctg ccactggtta 3180
ttttttcta aggagtaact tgcaagtttt cagtacaaat ctgtgctaca ctggataaaa 3240
atctaattta tgaattttac ttgcacctta tagttcatag caattaactg atttgtagtg 3300
attcattgtt tgttttatat accaatgact tccatatttt aaaagagaaa aacaacttta 3360

tgttgcagga aacccttttt gtaagtcttt attatttact ttgcattttg tttcactctt 3420

tccagataag cagagttgct cttcaccagt gtttttcttc atgtgcaaag tgactatttg 3480

ttctataata cttttatgtg tgttatatca aatgtgtctt aagcttcatg caaactcagt 3540

catcagttcg tgttgtctga agcaagtggg agatatataa atacccagta gctaaaatgg 3600

tcagtctttt ttagatgttt tcctacttag tatctcctaa taacgttttg ctgtgtcact 3660

agatgttcat ttcacaagtg catgtctttc taataatcca cacatttcat gctctaataa 3720

tccacacatt tcatgctcat ttttattgtt tttacagcca gttatagtaa gaaaaaggtt 3780

tttccccttg tgctgcttta taatttagcg tgtgtctgaa ccttatccat gtttgctaga 3840

tgaggtcttg tcaaatatat cactaccatt gtcaccggtg aaaagaaaca ggtagttaag 3900

ttagggttaa cattcatttc aaccacgagg ttgtatatca tgactagctt ttactcttgg 3960

tttacagaga aaagttaaac agccaactag gcagtttttta agaatattaa caatatatta 4020

acaaacacca atacaactaa tcctatttgg ttttaatgat ttcaccatgg gattaagaac 4080

tatatcagga acatccctga gaaacggttt taagtgtagc aactactctt ccttaatgga 4140

cagccacata acgtgtagga agtcctttat cacttatcct cgatccataa gcatatcttg 4200

cagaggggaa ctacttcttt aaacacatgg agggaaagaa gatgatgcca ctggcaccag 4260

agggttagta ctgtgatgca tcctaaaata tttattatat tggtaaaaat tctggttaaa 4320

taaaaaatta gagatcactc ttggctgatt tcagcaccag gaactgtatt acagttttag 4380

agattaattc ctagtgttta cctgattata gcagttggca tcatggggca tttaattctg 4440

actttatccc cacgtcagcc ttaataaagt cttctttacc ttctctatga agactttaaa 4500

gcccaaataa tcatttttca cattgatatt caagaattga gatagataga agccaaagtg 4560

ggtatctgac aagtggaaaa tcaaacgttt aagaagaatt acaactctga aaagcattta 4620

tatgtggaac ttctcaagga gcctcctggg gactggaaag taagtcatca gccaggcaaa 4680

tgactcatgc tgaagagagt ccccatttca gtcccctgag atctagctga tgcttagatc 4740

ctttgaaata aaaattatgt ctttataact ctgatctttt acataaagca gaagaggaat 4800

caactagtta attgcaaggt ttctactctg tttcctctgt aaagatcaga tggtaatctt 4860

tcaaataaga aaaaaataaa gacgtatgtt tgaccaagta gtttcacaag aatatttggg 4920

aacttgtttc ttttaatttt atttgtccct gagtgaagtc tagaaagaaa ggtaaagagt 4980

ctagagttta ttcctctttc caaaacattc tcattcctct cctccctaca cttagtattt 5040

cccccacaga gtgcctagaa tcttaataat gaataaaata aaaagcagca atatgtcatt 5100

aacaaatcca gacctgaaag ggtaaagggt ttataactgc actaataaag agaggctctt 5160

tttttttctt ccagtttgtt ggtttttaat ggtaccgtgt tgtaaagata cccactaatg 5220

gacaatcaaa ttgcagaaaa ggctcaatat ccaagagaca gggactaatg cactgtacaa 5280
tctgcttatc cttgcccttc tctcttgcca aagtgtgctt cagaaatata tactgcttta 5340
aaaaagaata aaagaatatc cttttacaag tggctttaca tttcctaaaa tgccataaga 5400
aaatgcaata tctgggtact gtatggggaa aaaaatgtcc aagtttgtgt aaaaccagtg 5460
catttcagct tgcaagttac tgaacacaat aatgctgttt taattttgtt ttatatcagt 5520
taaaattcac aataatgtag atagaacaaa ttacagacaa ggaaagaaaa aacttgaatg 5580
aaatggattt tacagaaagc tttatgataa tttttgaatg cattatttat tttttgtgcc 5640
atgcattttt tttctcacca aatgacctta cctgtaatac agtcttgttt gtctgtttac 5700
aaccatgtat ttattgcaat gtacatactg taatgttaat tgtaaattat ctgttcttat 5760
taaaacatca tcccatgatg ggatggtgtt gatatatttg gaaactcttg gtgagagaat 5820
gaatggtgtg tatacatact ctgtacattt ttcttttctc ctgtaatata gtcttgtcac 5880
cttagagctt gtttatggaa gattcaagaa aactataaaa tacttaaaga tatataaatt 5940
taaaaaaaca tagctgcagg tctttggtcc cagggctgtg ccttaacttt aaccaatatt 6000
ttcttctgtt ttgctgcatt tgaaaggtaa cagtggagct agggctgggc attttacatc 6060
caggctttta attgattaga attctgccaa taggtggatt ttacaaaacc acagacaacc 6120
tctgaaagat tctgagaccc ttttgagaca gaagctctta agtacttctt gccagggagc 6180
agcactgcat gtgtgatggt tgtttgccat ctgttgatca ggaactactt cagctacttg 6240
catttgatta tttccttttt tttttttttt aactcggaaa cacaactggg gaaatatatt 6300
ctttcccagt gattataaac aatctttttc tttttttaa gtcctttggg cttctagagc 6360
tcataggaaa atggacttga tttgaaattg gagccagagt ttactcgtgt tggttatcta 6420
ttcatcagct tcctgacatg ttaagagaat acattaaaga gaaaatactg tttttttaatc 6480
ctaaaatttt tcttccacta agataaacca aatgtcctta catatatgta aacccatcta 6540
tttaaacgca aaggtgggtt gatgtcagtt tacatagcag aaagcattca ctatcctcta 6600
agatttgttt ctgcaaaact ttcattgctt tagaatttta aaatttcacc ttgtacaatg 6660
gccagcccct aaagcaggaa acatttataa tggattatat ggaaacatcc tcccagtact 6720
tgcccagccc ttgaatcatg tggcttttca gtgaaaggaa agattctttt tctaggaaaa 6780
atgagcctat tttattttat tttattttat tttttgacac aaactgtaga ttttagcagc 6840
cctggcccaa aggaatttga ttacttttgt tttaaacagt acaaagggga cactataatt 6900
acaaaaacat ccttaactga tttgagttgt ttttatttct ttggatatat tttcagagtg 6960
gtaaattgtg tgtgagaatt acaaatgatt attcttttag tggtttctta gcctctctta 7020
cagcccacgg ggatagtact gtacatcaat accttcatat gaaattttta tatgcaatga 7080
aaataaaagc atgggttgat tctgcctatt tatgactcaa tcttttacaa ataaaagatt 7140

attcatttta aattatagtt caatcagcat gtctcttagg atactgaacg tggttgaaat 7200

gaaaggatag tgacatcata agttagtact gatattcata accaaataaa gccaacttga 7260

gtaattttgc tacattaaaa attaccaaaa ttacttagat ggcctataag attaagcatg 7320

gtgttttcta agcaagcttt gaaaggggcc ttccatactt acttaattga atattctggg 7380

atattgaaaa ttattcagat acttgacaat tatttttggt tacctactcc gcaaactaca 7440

aagttttaag gactcaacaa taagttaatg agacacagtg tttgctttca tggagcttac 7500

agtctggagg ggacaaaggc ttaaacaata ctcatataat tatatatgtg atcagtacaa 7560

tgaaggagct cagtggggta aataagcagg aacctgaact tgatctgttc cggagggcca 7620

cagaaggctt ccttgaggcc ttgagaaagt gatttgcatc tgagttctga aggattgtaa 7680

gaggtaacta gggaaaaagt tgacaggaag aggaagggga tccagacaag aaacatttgc 7740

aaagatcttg aggcataaat gagcttgaga catctggaga aactgaggaa aagtgagaga 7800

gtaggcaggg cctggagccg cagagccatt gctaaccatc ctgtgtgaga tatcccccat 7860

tctgtagctt tattctcata accctgctca attttcttta taacacttct cacagattta 7920

tatacgtgtt tgtttttgtt atctgtctct cccaccagac cacagctcca tgagagcaag 7980

gtctttgctt accaatatat cactagcact taaaactatg cctggtacac agtaggttct 8040

taatatgtgt tgaatatagc catcaaattg atattggata taattcaatc tgataagata 8100

ttttgagata ttaaagagtt tttaacttga taccataaaa aaaaaaaaaa aaa 8153

<210> 93
<211> 8203
<212> DNA
<213> Homo sapiens

<400> 93
gcggcgcatt agttccaata gtttaacagg ctgctttgta gcacggacaa agccgtgcga 60

gcgcggtgct ttccctcctt gttcatttgc tatgcgagct tgtcagtgat ctttggcagg 120

ggcttgggag aggaggggtg acgtttaata cgcttccgcg gaggagtgcg ctcgcctcct 180

ctgcacccag ccccaggctc tacagagaga ctgaggcagg cgactgaatg cactaacagc 240

agcaggctca gacctgcttc cctggacatt tccgggaccg tgagcgaggg aaccacgttg 300

ccctggattc ttgccagctg tacaaagttg accaggaaaa tggctcagca gacaagcccg 360

gacactttaa cagtacctga agtggataat ccgcattgtc caaacccgtg gctgaacgaa 420

gaccttgtga aatccttgcg agaaaacctg ttgcagcatg agaagtccaa gacagcgagg 480

aaatcggttt ctcccaagct ctctccagtg atctctccga gaaattcccc caggcttctg 540

cgcagaatgc ttctcagcag caacatcccc aaacagcggc gtttcacggt ggcacataca 600

tgttttgatg tggacaatgg cacatctgcg ggacggagtc ccttggatcc catgaccagc 660

ccaggatccg ggctaattct ccaagcaaat tttgtccaca gtcaacgacg ggagtccttc 720

ctgtatcgat ccgacagcga ttatgacctc tctccaaagt ctatgtcccg gaactcctcc 780

attgccagtg atatacacgg agatgacttg attgtgactc catttgctca ggtcttggcc 840

agtctgcgaa ctgtacgaaa caactttgct gcattaacta atttgcaaga tcgagcacct 900

agcaaaagat cacccatgtg caaccaacca tccatcaaca aagccaccat aacagaggag 960

gcctaccaga aactggccag cgagaccctg gaggagctgg actggtgtct ggaccagcta 1020

gagaccctac agaccaggca ctccgtcagt gagatggcct ccaacaagtt taaaaggatg 1080

cttaatcggg agctcaccca tctctctgaa atgagtcggt ctggaaatca agtgtcagag 1140

tttatatcaa acacattctt agataagcaa catgaagtgg aaattccttc tccaactcag 1200

aaggaaaagg agaaaaagaa aagaccaatg tctcagatca gtggagtcaa gaaattgatg 1260

cacagctcta gtctgactaa ttcaagtatc ccaaggtttg gagttaaaac tgaacaagaa 1320

gatgtccttg ccaaggaact agaagatgtg aacaaatggg gtcttcatgt tttcagaata 1380

gcagagttgt ctggtaaccg gcccttgact gttatcatgc acaccatttt tcaggaacgg 1440

gatttattaa aaacatttaa aattccagta gatactttaa ttacatatct tatgactctc 1500

gaagaccatt accatgctga tgtggcctat cacaacaata tccatgctgc agatgttgtc 1560

cagtctactc atgtgctatt atctacacct gctttggagg ctgtgtttac agatttggag 1620

attcttgcag caatttttgc cagtgcaata catgatgtag atcatcctgg tgtgtccaat 1680

caatttctga tcaatacaaa ctctgaactt gccttgatgt acaatgattc ctcagtctta 1740

gagaaccatc atttggctgt gggctttaaa ttgcttcagg aagaaaactg tgacattttc 1800

cagaatttga ccaaaaaaca aagacaatct ttaaggaaaa tggtcattga catcgtactt 1860

gcaacagata tgtcaaaaca catgaatcta ctggctgatt tgaagactat ggttgaaact 1920

aagaaagtga caagctctgg agttcttctt cttgataatt attccgatag gattcaggtt 1980

cttcagaata tggtgcactg tgcagatctg agcaacccaa caaagcctct ccagctgtac 2040

cgccagtgga cggaccggat aatggaggag ttcttccgcc aaggagaccg agagagggaa 2100

cgtggcatgg agataagccc catgtgtgac aagcacaatg cttccgtgga aaaatcacag 2160

gtgggcttca tagactatat tgttcatccc ctctgggaga catgggcaga cctcgtccac 2220

cctgacgccc aggatatttt ggacactttg gaggacaatc gtgaatggta ccagagcaca 2280

atccctcaga gcccctctcc tgcacctgat gacccagagg agggccggca gggtcaaact 2340

gagaaattcc agtttgaact aactttagag gaagatggtg agtcagacac ggaaaaggac 2400

agtggcagtc aagtggaaga agacactagc tgcagtgact ccaagactct ttgtactcaa 2460

gactcagagt ctactgaaat tccccttgat gaacaggttg aagaggaggc agtaggggaa 2520

gaagaggaaa gccagcctga agcctgtgtc atagatgatc gttctcctga cacgtaacag 2580
tgcaaaaact ttcatgcctt tttttttttt aagtagaaaa attgtttcca aagtgcatgt 2640
cacatgccac aaccacggtc acacctcact gtcatctgcc aggacgtttg ttgaacaaaa 2700
ctgaccttga ctactcagtc cagcgctcag gaatatcgta accagttttt tcacctccat 2760
gtcatccgag caaggtggac atcttcacga acagcgtttt taacaagatt tcagcttggt 2820
agagctgaca aagcagataa aatctactcc aaattatttt caagagagtg tgactcatca 2880
ggcagcccaa aagtttattg gacttggggt ttctattcct ttttatttgt ttgcaatatt 2940
ttcagaagaa aggcattgca cagagtgaac ttaatggacg aagcaacaaa tatgtcaaga 3000
acaggacata gcacgaatct gttaccagta ggaggaggat gagccacaga aattgcataa 3060
ttttctaatt tcaagtcttc ctgatacatg actgaatagt gtggttcagt gagctgcact 3120
gacctctaca ttttgtatga tatgtaaaac agattttttg tagagcttac ttttattatt 3180
aaatgtattg aggtattata tttaaaaaaa actatgttca gaacttcatc tgccactggt 3240
tatttttttc taaggagtaa cttgcaagtt ttcagtacaa atctgtgcta cactggataa 3300
aaatctaatt tatgaatttt acttgcacct tatagttcat agcaattaac tgatttgtag 3360
tgattcattg tttgttttat ataccaatga cttccatatt ttaaaagaga aaaacaactt 3420
tatgttgcag gaaacccttt ttgtaagtct ttattattta ctttgcattt tgtttcactc 3480
tttccagata agcagagttg ctcttcacca gtgtttttct tcatgtgcaa agtgactatt 3540
tgttctataa tacttttatg tgtgttatat caaatgtgtc ttaagcttca tgcaaactca 3600
gtcatcagtt cgtgttgtct gaagcaagtg ggagatatat aaatacccag tagctaaaat 3660
ggtcagtctt ttttagatgt tttcctactt agtatctcct aataacgttt tgctgtgtca 3720
ctagatgttc atttcacaag tgcatgtctt tctaataatc cacacatttc atgctctaat 3780
aatccacaca tttcatgctc atttttattg tttttacagc cagttatagt aagaaaaagg 3840
tttttcccct tgtgctgctt tataatttag cgtgtgtctg aaccttatcc atgtttgcta 3900
gatgaggtct tgtcaaatat atcactacca ttgtcaccgg tgaaaagaaa caggtagtta 3960
agttagggtt aacattcatt tcaaccacga ggttgtatat catgactagc ttttactctt 4020
ggtttacaga gaaaagttaa acagccaact aggcagtttt taagaatatt aacaatatat 4080
taacaaacac caatacaact aatcctattt ggttttaatg atttcaccat gggattaaga 4140
actatatcag gaacatccct gagaaacggt tttaagtgta gcaactactc ttccttaatg 4200
gacagccaca taacgtgtag gaagtccttt atcacttatc ctcgatccat aagcatatct 4260
tgcagagggg aactacttct ttaaacacat ggagggaaag aagatgatgc cactggcacc 4320
agagggttag tactgtgatg catcctaaaa tatttattat attggtaaaa attctggtta 4380
aataaaaaat tagagatcac tcttggctga tttcagcacc aggaactgta ttacagtttt 4440

agagattaat tcctagtgtt tacctgatta tagcagttgg catcatgggg catttaattc 4500

tgactttatc cccacgtcag ccttaataaa gtcttcttta ccttctctat gaagacttta 4560

aagcccaaat aatcattttt cacattgata ttcaagaatt gagatagata gaagccaaag 4620

tgggtatctg acaagtggaa aatcaaacgt ttaagaagaa ttacaactct gaaaagcatt 4680

tatatgtgga acttctcaag gagcctcctg gggactggaa agtaagtcat cagccaggca 4740

aatgactcat gctgaagaga gtccccattt cagtcccctg agatctagct gatgcttaga 4800

tcctttgaaa taaaaattat gtctttataa ctctgatctt ttacataaag cagaagagga 4860

atcaactagt taattgcaag gtttctactc tgtttcctct gtaaagatca gatggtaatc 4920

tttcaaataa gaaaaaaata aagacgtatg tttgaccaag tagtttcaca agaatatttg 4980

ggaacttgtt tcttttaatt ttatttgtcc ctgagtgaag tctagaaaga aaggtaaaga 5040

gtctagagtt tattcctctt tccaaaacat tctcattcct ctcctcccta cacttagtat 5100

ttcccccaca gagtgcctag aatcttaata atgaataaaa taaaaagcag caatatgtca 5160

ttaacaaatc cagacctgaa agggtaaagg gtttataact gcactaataa agagaggctc 5220

tttttttttc ttccagtttg ttggttttta atggtaccgt gttgtaaaga tacccactaa 5280

tggacaatca aattgcagaa aaggctcaat atccaagaga cagggactaa tgcactgtac 5340

aatctgctta tccttgccct tctctcttgc caaagtgtgc ttcagaaata tatactgctt 5400

taaaaaagaa taaaagaata tccttttaca agtggcttta catttcctaa aatgccataa 5460

gaaaatgcaa tatctgggta ctgtatgggg aaaaaaatgt ccaagtttgt gtaaaaccag 5520

tgcatttcag cttgcaagtt actgaacaca ataatgctgt tttaattttg ttttatatca 5580

gttaaaattc acaataatgt agatagaaca aattacagac aaggaaagaa aaaacttgaa 5640

tgaaatggat tttacagaaa gctttatgat aatttttgaa tgcattattt atttttttgtg 5700

ccatgcattt tttttctcac caaatgacct tacctgtaat acagtcttgt ttgtctgttt 5760

acaaccatgt atttattgca atgtacatac tgtaatgtta attgtaaatt atctgttctt 5820

attaaaacat catcccatga tgggatggtg ttgatatatt tggaaactct tggtgagaga 5880

atgaatggtg tgtatacata ctctgtacat ttttcttttc tcctgtaata tagtcttgtc 5940

accttagagc ttgtttatgg aagattcaag aaaactataa aatacttaaa gatatataaa 6000

tttaaaaaaa catagctgca ggtctttggt cccagggctg tgccttaact ttaaccaata 6060

ttttcttctg ttttgctgca tttgaaaggt aacagtggag ctagggctgg gcattttaca 6120

tccaggcttt taattgatta gaattctgcc aataggtgga ttttacaaaa ccacagacaa 6180

cctctgaaag attctgagac ccttttgaga cagaagctct taagtacttc ttgccaggga 6240

gcagcactgc atgtgtgatg gttgtttgcc atctgttgat caggaactac ttcagctact 6300

tgcatttgat tatttccttt tttttttttt ttaactcgga aacacaactg gggaaatata 6360
ttctttccca gtgattataa acaatctttt tctttttttt aagtcctttt ggcttctaga 6420
gctcatagga aaatggactt gatttgaaat tggagccaga gtttactcgt gttggttatc 6480
tattcatcag cttcctgaca tgttaagaga atacattaaa gagaaaatac tgttttttaa 6540
tcctaaaatt tttcttccac taagataaac caaatgtcct tacatatatg taaacccatc 6600
tatttaaacg caaaggtggg ttgatgtcag tttacatagc agaaagcatt cactatcctc 6660
taagatttgt ttctgcaaaa ctttcattgc tttagaattt taaaatttca ccttgtacaa 6720
tggccagccc ctaaagcagg aaacatttat aatggattat atggaaacat cctcccagta 6780
cttgcccagc ccttgaatca tgtggctttt cagtgaaagg aaagattctt tttctaggaa 6840
aaatgagcct attttatttt attttatttt atttttgac acaaactgta gattttagca 6900
gccctggccc aaaggaattt gattactttt gttttaaaca gtacaaaggg gacactataa 6960
ttacaaaaac atccttaact gatttgagtt gtttttattt ctttggatat attttcagag 7020
tggtaaattg tgtgtgagaa ttacaaatga ttattctttt agtggtttct tagcctctct 7080
tacagcccac ggggatagta ctgtacatca ataccttcat atgaaatttt tatatgcaat 7140
gaaaataaaa gcatgggttg attctgccta tttatgactc aatcttttac aaataaaaga 7200
ttattcattt taaattatag ttcaatcagc atgtctctta ggatactgaa cgtggttgaa 7260
atgaaaggat agtgacatca taagttagta ctgatattca taaccaaata aagccaactt 7320
gagtaatttt gctacattaa aaattaccaa aattacttag atggcctata agattaagca 7380
tggtgttttc taagcaagct ttgaaagggg ccttccatac ttacttaatt gaatattctg 7440
ggatattgaa aattattcag atacttgaca attatttttg gttacctact ccgcaaacta 7500
caaagtttta aggactcaac aataagttaa tgagacacag tgtttgcttt catggagctt 7560
acagtctgga ggggacaaag gcttaaacaa tactcatata attatatatg tgatcagtac 7620
aatgaaggag ctcagtgggg taaataagca ggaacctgaa cttgatctgt tccggagggc 7680
cacagaaggc ttccttgagg ccttgagaaa gtgatttgca tctgagttct gaaggattgt 7740
aagaggtaac tagggaaaaa gttgacagga agaggaaggg gatccagaca agaaacattt 7800
gcaaagatct tgaggcataa atgagcttga gacatctgga gaaactgagg aaaagtgaga 7860
gagtaggcag ggcctggagc cgcagagcca ttgctaacca tcctgtgtga gatatccccc 7920
attctgtagc tttattctca taaccctgct caattttctt tataacactt ctcacagatt 7980
tatatacgtg tttgtttttg ttatctgtct ctcccaccag accacagctc catgagagca 8040
aggtctttgc ttaccaatat atcactagca cttaaaacta tgcctggtac acagtaggtt 8100
cttaatatgt gttgaatata gccatcaaat tgatattgga tataattcaa tctgataaga 8160
tattttgaga tattaaagag tttttaactt gataccataa aaa 8203

<210> 94
<211> 7783
<212> DNA
<213> Homo sapiens

<400> 94
aatacttgtt gcaataattg cccacgatag ctgctcaaac aagagagttg gaattcatct 60

gtaaaaatca ctacatgtaa cgtaggagac aagaaaaata ttaatgacag aagatctgcg 120

aacatgatgc acgtgaataa ttttcccttt agaaggcatt cctggatatg ttttgatgtg 180

gacaatggca catctgcggg acggagtccc ttggatccca tgaccagccc aggatccggg 240

ctaattctcc aagcaaattt tgtccacagt caacgacggg agtccttcct gtatcgatcc 300

gacagcgatt atgacctctc tccaaagtct atgtcccgga actcctccat tgccagtgat 360

atacacggag atgacttgat tgtgactcca tttgctcagg tcttggccag tctgcgaact 420

gtacgaaaca actttgctgc attaactaat ttgcaagatc gagcacctag caaaagatca 480

cccatgtgca accaaccatc catcaacaaa gccaccataa cagaggaggc ctaccagaaa 540

ctggccagcg agaccctgga ggagctggac tggtgtctgg accagctaga gaccctacag 600

accaggcact ccgtcagtga gatggcctcc aacaagttta aaaggatgct taatcgggag 660

ctcacccatc tctctgaaat gagtcggtct ggaaatcaag tgtcagagtt tatatcaaac 720

acattcttag ataagcaaca tgaagtggaa attccttctc caactcagaa ggaaaaggag 780

aaaaagaaaa gaccaatgtc tcagatcagt ggagtcaaga aattgatgca cagctctagt 840

ctgactaatt caagtatccc aaggtttgga gttaaaactg aacaagaaga tgtccttgcc 900

aaggaactag aagatgtgaa caaatggggt cttcatgttt tcagaatagc agagttgtct 960

ggtaaccggc ccttgactgt tatcatgcac accatttttc aggaacggga tttattaaaa 1020

acatttaaaa ttccagtaga tactttaatt acatatctta tgactctcga agaccattac 1080

catgctgatg tggcctatca caacaatatc catgctgcag atgttgtcca gtctactcat 1140

gtgctattat ctacacctgc tttggaggct gtgtttacag atttggagat tcttgcagca 1200

atttttgcca gtgcaataca tgatgtagat catcctggtg tgtccaatca atttctgatc 1260

aatacaaact ctgaacttgc cttgatgtac aatgattcct cagtcttaga gaaccatcat 1320

ttggctgtgg gctttaaatt gcttcaggaa gaaaactgtg acattttcca gaatttgacc 1380

aaaaaacaaa gacaatcttt aaggaaaatg gtcattgaca tcgtacttgc aacagatatg 1440

tcaaaacaca tgaatctact ggctgatttg aagactatgg ttgaaactaa gaaagtgaca 1500

agctctggag ttcttcttct tgataattat tccgatagga ttcaggttct tcagaatatg 1560

gtgcactgtg cagatctgag caacccaaca aagcctctcc agctgtaccg ccagtggacg 1620

gaccggataa tggaggagtt cttccgccaa ggagaccgag agagggaacg tggcatggag 1680

ataagcccca tgtgtgacaa gcacaatgct tccgtggaaa aatcacaggt gggcttcata 1740
gactatattg ttcatcccct ctgggagaca tgggcagacc tcgtccaccc tgacgcccag 1800
gatattttgg acactttgga ggacaatcgt gaatggtacc agagcacaat ccctcagagc 1860
ccctctcctg cacctgatga cccagaggag ggccggcagg gtcaaactga gaaattccag 1920
tttgaactaa ctttagagga agatggtgag tcagacacgg aaaaggacag tggcagtcaa 1980
gtggaagaag acactagctg cagtgactcc aagactcttt gtactcaaga ctcagagtct 2040
actgaaattc cccttgatga acaggttgaa gaggaggcag taggggaaga agaggaaagc 2100
cagcctgaag cctgtgtcat agatgatcgt tctcctgaca cgtaacagtg caaaaacttt 2160
catgcctttt tttttttaa gtagaaaaat tgtttccaaa gtgcatgtca catgccacaa 2220
ccacggtcac acctcactgt catctgccag gacgtttgtt gaacaaaact gaccttgact 2280
actcagtcca gcgctcagga atatcgtaac cagttttttc acctccatgt catccgagca 2340
aggtggacat cttcacgaac agcgttttta acaagatttc agcttggtag agctgacaaa 2400
gcagataaaa tctactccaa attattttca agagagtgtg actcatcagg cagcccaaaa 2460
gtttattgga cttggggttt ctattccttt ttatttgttt gcaatatttt cagaagaaag 2520
gcattgcaca gagtgaactt aatggacgaa gcaacaaata tgtcaagaac aggacatagc 2580
acgaatctgt taccagtagg aggaggatga gccacagaaa ttgcataatt ttctaatttc 2640
aagtcttcct gatacatgac tgaatagtgt ggttcagtga gctgcactga cctctacatt 2700
ttgtatgata tgtaaaacag atttttgta gagcttactt ttattattaa atgtattgag 2760
gtattatatt taaaaaaaac tatgttcaga acttcatctg ccactggtta tttttttcta 2820
aggagtaact tgcaagtttt cagtacaaat ctgtgctaca ctggataaaa atctaattta 2880
tgaattttac ttgcacctta tagttcatag caattaactg atttgtagtg attcattgtt 2940
tgttttatat accaatgact tccatatttt aaaagagaaa aacaacttta tgttgcagga 3000
aacccttttt gtaagtcttt attatttact ttgcattttg tttcactctt tccagataag 3060
cagagttgct cttcaccagt gtttttcttc atgtgcaaag tgactatttg ttctataata 3120
cttttatgtg tgttatatca aatgtgtctt aagcttcatg caaactcagt catcagttcg 3180
tgttgtctga agcaagtggg agatatataa atacccagta gctaaaatgg tcagtctttt 3240
ttagatgttt tcctacttag tatctcctaa taacgttttg ctgtgtcact agatgttcat 3300
ttcacaagtg catgtctttc taataatcca cacatttcat gctctaataa tccacacatt 3360
tcatgctcat ttttattgtt tttacagcca gttatagtaa gaaaaaggtt tttccccttg 3420
tgctgcttta taatttagcg tgtgtctgaa ccttatccat gtttgctaga tgaggtcttg 3480
tcaaatatat cactaccatt gtcaccggtg aaaagaaaca ggtagttaag ttagggttaa 3540

cattcatttc aaccacgagg ttgtatatca tgactagctt ttactcttgg tttacagaga 3600
aaagttaaac agccaactag gcagttttta agaatattaa caatatatta acaaacacca 3660
atacaactaa tcctatttgg ttttaatgat ttcaccatgg gattaagaac tatatcagga 3720
acatccctga gaaacggttt taagtgtagc aactactctt ccttaatgga cagccacata 3780
acgtgtagga agtcctttat cacttatcct cgatccataa gcatatcttg cagaggggaa 3840
ctacttcttt aaacacatgg agggaaagaa gatgatgcca ctggcaccag agggttagta 3900
ctgtgatgca tcctaaaata tttattatat tggtaaaaat tctggttaaa taaaaaatta 3960
gagatcactc ttggctgatt tcagcaccag gaactgtatt acagttttag agattaattc 4020
ctagtgttta cctgattata gcagttggca tcatggggca tttaattctg actttatccc 4080
cacgtcagcc ttaataaagt cttctttacc ttctctatga agactttaaa gcccaaataa 4140
tcatttttca cattgatatt caagaattga gatagataga agccaaagtg ggtatctgac 4200
aagtggaaaa tcaaacgttt aagaagaatt acaactctga aaagcattta tatgtggaac 4260
ttctcaagga gcctcctggg gactggaaag taagtcatca gccaggcaaa tgactcatgc 4320
tgaagagagt ccccatttca gtcccctgag atctagctga tgcttagatc ctttgaaata 4380
aaaattatgt ctttataact ctgatctttt acataaagca gaagaggaat caactagtta 4440
attgcaaggt ttctactctg tttcctctgt aaagatcaga tggtaatctt tcaaataaga 4500
aaaaaataaa gacgtatgtt tgaccaagta gtttcacaag aatatttggg aacttgtttc 4560
ttttaatttt atttgtccct gagtgaagtc tagaaagaaa ggtaaagagt ctagagttta 4620
ttcctctttc caaaacattc tcattcctct cctccctaca cttagtattt cccccacaga 4680
gtgcctagaa tcttaataat gaataaaata aaaagcagca atatgtcatt aacaaatcca 4740
gacctgaaag ggtaaagggt ttataactgc actaataaag agaggctctt tttttttctt 4800
ccagtttgtt ggtttttaat ggtaccgtgt tgtaaagata cccactaatg gacaatcaaa 4860
ttgcagaaaa ggctcaatat ccaagagaca gggactaatg cactgtacaa tctgcttatc 4920
cttgcccttc tctcttgcca aagtgtgctt cagaaatata tactgcttta aaaaagaata 4980
aaagaatatc cttttacaag tggctttaca tttcctaaaa tgccataaga aaatgcaata 5040
tctgggtact gtatggggaa aaaaatgtcc aagtttgtgt aaaaccagtg catttcagct 5100
tgcaagttac tgaacacaat aatgctgttt taattttgtt ttatatcagt taaaattcac 5160
aataatgtag atagaacaaa ttacagacaa ggaaagaaaa aacttgaatg aaatggattt 5220
tacagaaagc tttatgataa tttttgaatg cattatttat tttttgtgcc atgcattttt 5280
tttctcacca aatgacctta cctgtaatac agtcttgttt gtctgtttac aaccatgtat 5340
ttattgcaat gtacatactg taatgttaat tgtaaattat ctgttcttat taaaacatca 5400
tcccatgatg ggatggtgtt gatatatttg gaaactcttg gtgagagaat gaatggtgtg 5460

tatacatact ctgtacattt ttcttttctc ctgtaatata gtcttgtcac cttagagctt 5520
gtttatggaa gattcaagaa aactataaaa tacttaaaga tatataaatt taaaaaaaca 5580
tagctgcagg tctttggtcc cagggctgtg ccttaacttt aaccaatatt ttcttctgtt 5640
ttgctgcatt tgaaaggtaa cagtggagct agggctgggc attttacatc caggctttta 5700
attgattaga attctgccaa taggtggatt ttacaaaacc acagacaacc tctgaaagat 5760
tctgagaccc ttttgagaca gaagctctta agtacttctt gccagggagc agcactgcat 5820
gtgtgatggt tgtttgccat ctgttgatca ggaactactt cagctacttg catttgatta 5880
tttccttttt tttttttttt aactcggaaa cacaactggg gaaatatatt ctttcccagt 5940
gattataaac aatctttttc tttttttaa gtccttttgg cttctagagc tcataggaaa 6000
atggacttga tttgaaattg gagccagagt ttactcgtgt tggttatcta ttcatcagct 6060
tcctgacatg ttaagagaat acattaaaga gaaaatactg tttttaatc ctaaaatttt 6120
tcttccacta agataaacca aatgtcctta catatatgta aacccatcta tttaaacgca 6180
aaggtgggtt gatgtcagtt tacatagcag aaagcattca ctatcctcta agatttgttt 6240
ctgcaaaact ttcattgctt tagaatttta aaatttcacc ttgtacaatg gccagcccct 6300
aaagcaggaa acatttataa tggattatat ggaaacatcc tcccagtact tgcccagccc 6360
ttgaatcatg tggcttttca gtgaaaggaa agattctttt tctaggaaaa atgagcctat 6420
tttattttat tttattttat tttttgacac aaactgtaga ttttagcagc cctggcccaa 6480
aggaatttga ttacttttgt tttaaacagt acaaagggga cactataatt acaaaaacat 6540
ccttaactga tttgagttgt ttttatttct ttggatatat tttcagagtg gtaaattgtg 6600
tgtgagaatt acaaatgatt attcttttag tggtttctta gcctctctta cagcccacgg 6660
ggatagtact gtacatcaat accttcatat gaaattttta tatgcaatga aaataaaagc 6720
atgggttgat tctgcctatt tatgactcaa tcttttacaa ataaaagatt attcatttta 6780
aattatagtt caatcagcat gtctcttagg atactgaacg tggttgaaat gaaaggatag 6840
tgacatcata agttagtact gatattcata accaaataaa gccaacttga gtaattttgc 6900
tacattaaaa attaccaaaa ttacttagat ggcctataag attaagcatg gtgttttcta 6960
agcaagcttt gaaaggggcc ttccatactt acttaattga atattctggg atattgaaaa 7020
ttattcagat acttgacaat tatttttggt tacctactcc gcaaactaca aagttttaag 7080
gactcaacaa taagttaatg agacacagtg tttgctttca tggagcttac agtctggagg 7140
ggacaaaggc ttaaacaata ctcatataat tatatatgtg atcagtacaa tgaaggagct 7200
cagtggggta aataagcagg aacctgaact tgatctgttc cggagggcca cagaaggctt 7260
ccttgaggcc ttgagaaagt gatttgcatc tgagttctga aggattgtaa gaggtaacta 7320

gggaaaaagt tgacaggaag aggaagggga tccagacaag aaacatttgc aaagatcttg 7380

aggcataaat gagcttgaga catctggaga aactgaggaa aagtgagaga gtaggcaggg 7440

cctggagccg cagagccatt gctaaccatc ctgtgtgaga tatcccccat tctgtagctt 7500

tattctcata accctgctca attttcttta taacacttct cacagattta tatacgtgtt 7560

tgtttttgtt atctgtctct cccaccagac cacagctcca tgagagcaag gtctttgctt 7620

accaatatat cactagcact taaaactatg cctggtacac agtaggttct taatatgtgt 7680

tgaatatagc catcaaattg atattggata taattcaatc tgataagata ttttgagata 7740

ttaaagagtt tttaacttga taccataaaa aaaaaaaaaa aaa 7783

<210> 95
<211> 7667
<212> DNA
<213> Homo sapiens

<400> 95
acttcaagtg ccgtgcagaa ggctcggcag gcggggcggg cgtggggccg cggctccggg 60

ttggggaccg aggagatccg gctgtggacc agacgctcct ctgcggggcg ggcacccaag 120

cgcgctcgcc accccctcgc catccgctag agccgggctc ctggactggg actcgggccc 180

gccgcacagt tgaaaagtcg catagtggtt tttccgctcg cgtcgctgtg tgaaagttgg 240

ctcgccgctc tttgcacgcc ctccctggag gccgacccga gacgccaagc tggagagacc 300

gtgcctcccc gaggccggcc gccccgcgag cacagcctcc gcccccgttg cactgccggg 360

ctgggcaata tgaaggagca gccctcatgt gccggcaccg ggcatccgag catggcggga 420

ggaggcctac cagaaactgg ccagcgagac cctggaggag ctggactggt gtctggacca 480

gctagagacc ctacagacca ggcactccgt cagtgagatg gcctccaaca agtttaaaag 540

gatgcttaat cgggagctca cccatctctc tgaaatgagt cggtctggaa atcaagtgtc 600

agagtttata tcaaacacat tcttagataa gcaacatgaa gtggaaattc cttctccaac 660

tcagaaggaa aaggagaaaa agaaaagacc aatgtctcag atcagtggag tcaagaaatt 720

gatgcacagc tctagtctga ctaattcaag tatcccaagg tttggagtta aaactgaaca 780

agaagatgtc cttgccaagg aactagaaga tgtgaacaaa tggggtcttc atgttttcag 840

aatagcagag ttgtctggta accggccctt gactgttatc atgcacacca tttttcagga 900

acgggattta ttaaaaacat ttaaaattcc agtagatact ttaattacat atcttatgac 960

tctcgaagac cattaccatg ctgatgtggc ctatcacaac aatatccatg ctgcagatgt 1020

tgtccagtct actcatgtgc tattatctac acctgctttg gaggctgtgt ttacagattt 1080

ggagattctt gcagcaattt ttgccagtgc aatacatgat gtagatcatc ctggtgtgtc 1140

caatcaattt ctgatcaata caaactctga acttgccttg atgtacaatg attcctcagt 1200

cttagagaac catcatttgg ctgtgggctt taaattgctt caggaagaaa actgtgacat 1260

tttccagaat ttgaccaaaa aacaaagaca atctttaagg aaaatggtca ttgacatcgt 1320

acttgcaaca gatatgtcaa aacacatgaa tctactggct gatttgaaga ctatggttga 1380

aactaagaaa gtgacaagct ctggagttct tcttcttgat aattattccg ataggattca 1440

ggttcttcag aatatggtgc actgtgcaga tctgagcaac ccaacaaagc ctctccagct 1500

gtaccgccag tggacggacc ggataatgga ggagttcttc cgccaaggag accgagagag 1560

ggaacgtggc atggagataa gccccatgtg tgacaagcac aatgcttccg tggaaaaatc 1620

acaggtgggc ttcatagact atattgttca tcccctctgg gagacatggg cagacctcgt 1680

ccaccctgac gcccaggata ttttggacac tttggaggac aatcgtgaat ggtaccagag 1740

cacaatccct cagagcccct ctcctgcacc tgatgaccca gaggagggcc ggcagggtca 1800

aactgagaaa ttccagtttg aactaacttt agaggaagat ggtgagtcag acacggaaaa 1860

ggacagtggc agtcaagtgg aagaagacac tagctgcagt gactccaaga ctctttgtac 1920

tcaagactca gagtctactg aaattcccct tgatgaacag gttgaagagg aggcagtagg 1980

ggaagaagag gaaagccagc ctgaagcctg tgtcatagat gatcgttctc ctgacacgta 2040

acagtgcaaa aactttcatg cctttttttt ttttaagtag aaaaattgtt tccaaagtgc 2100

atgtcacatg ccacaaccac ggtcacacct cactgtcatc tgccaggacg tttgttgaac 2160

aaaactgacc ttgactactc agtccagcgc tcaggaatat cgtaaccagt tttttcacct 2220

ccatgtcatc cgagcaaggt ggacatcttc acgaacagcg tttttaacaa gatttcagct 2280

tggtagagct gacaaagcag ataaaatcta ctccaaatta ttttcaagag agtgtgactc 2340

atcaggcagc ccaaaagttt attggacttg ggtttctat tcctttttat ttgtttgcaa 2400

tattttcaga agaaaggcat tgcacagagt gaacttaatg gacgaagcaa caaatatgtc 2460

aagaacagga catagcacga atctgttacc agtaggagga ggatgagcca cagaaattgc 2520

ataattttct aatttcaagt cttcctgata catgactgaa tagtgtggtt cagtgagctg 2580

cactgacctc tacattttgt atgatatgta aaacagattt tttgtagagc ttacttttat 2640

tattaaatgt attgaggtat tatatttaaa aaaaactatg ttcagaactt catctgccac 2700

tggttatttt tttctaagga gtaacttgca agttttcagt acaaatctgt gctacactgg 2760

ataaaaatct aatttatgaa ttttacttgc accttatagt tcatagcaat taactgattt 2820

gtagtgattc attgtttgtt ttatatacca atgacttcca tattttaaaa gagaaaaaca 2880

actttatgtt gcaggaaacc ctttttgtaa gtctttatta tttactttgc attttgtttc 2940

actctttcca gataagcaga gttgctcttc accagtgttt ttcttcatgt gcaaagtgac 3000

tatttgttct ataatacttt tatgtgtgtt atatcaaatg tgtcttaagc ttcatgcaaa 3060

ctcagtcatc agttcgtgtt gtctgaagca agtgggagat atataaatac ccagtagcta 3120

aaatggtcag tctttttag atgttttcct acttagtatc tcctaataac gttttgctgt 3180
gtcactagat gttcatttca caagtgcatg tctttctaat aatccacaca tttcatgctc 3240
taataatcca cacatttcat gctcattttt attgttttta cagccagtta tagtaagaaa 3300
aaggtttttc cccttgtgct gctttataat ttagcgtgtg tctgaacctt atccatgttt 3360
gctagatgag gtcttgtcaa atatatcact accattgtca ccggtgaaaa gaaacaggta 3420
gttaagttag ggttaacatt catttcaacc acgaggttgt atatcatgac tagcttttac 3480
tcttggttta cagagaaaag ttaaacagcc aactaggcag tttttaagaa tattaacaat 3540
atattaacaa acaccaatac aactaatcct atttggtttt aatgatttca ccatgggatt 3600
aagaactata tcaggaacat ccctgagaaa cggttttaag tgtagcaact actcttcctt 3660
aatggacagc cacataacgt gtaggaagtc ctttatcact tatcctcgat ccataagcat 3720
atcttgcaga ggggaactac ttctttaaac acatggaggg aaagaagatg atgccactgg 3780
caccagaggg ttagtactgt gatgcatcct aaaatattta ttatattggt aaaaattctg 3840
gttaaataaa aaattagaga tcactcttgg ctgatttcag caccaggaac tgtattacag 3900
ttttagagat taattcctag tgtttacctg attatagcag ttggcatcat ggggcattta 3960
attctgactt tatccccacg tcagccttaa taaagtcttc tttaccttct ctatgaagac 4020
tttaaagccc aaataatcat ttttcacatt gatattcaag aattgagata gatagaagcc 4080
aaagtgggta tctgacaagt ggaaaatcaa acgtttaaga agaattacaa ctctgaaaag 4140
catttatatg tggaacttct caaggagcct cctggggact ggaaagtaag tcatcagcca 4200
ggcaaatgac tcatgctgaa gagagtcccc atttcagtcc cctgagatct agctgatgct 4260
tagatccttt gaaataaaaa ttatgtcttt ataactctga tcttttacat aaagcagaag 4320
aggaatcaac tagttaattg caaggtttct actctgtttc ctctgtaaag atcagatggt 4380
aatctttcaa ataagaaaaa aataaagacg tatgtttgac caagtagttt cacaagaata 4440
tttgggaact tgtttctttt aattttattt gtccctgagt gaagtctaga aagaaaggta 4500
aagagtctag agtttattcc tctttccaaa acattctcat tcctctcctc cctacactta 4560
gtatttcccc cacagagtgc ctagaatctt aataatgaat aaaataaaaa gcagcaatat 4620
gtcattaaca aatccagacc tgaaagggta aagggtttat aactgcacta ataaagagag 4680
gctctttttt tttcttccag tttgttggtt tttaatggta ccgtgttgta aagataccca 4740
ctaatggaca atcaaattgc agaaaaggct caatatccaa gagacaggga ctaatgcact 4800
gtacaatctg cttatccttg cccttctctc ttgccaaagt gtgcttcaga aatatatact 4860
gctttaaaaa agaataaaag aatatccttt tacaagtggc tttacatttc ctaaaatgcc 4920
ataagaaaat gcaatatctg ggtactgtat ggggaaaaaa atgtccaagt ttgtgtaaaa 4980

ccagtgcatt tcagcttgca agttactgaa cacaataatg ctgttttaat tttgttttat 5040
atcagttaaa attcacaata atgtagatag aacaaattac agacaaggaa agaaaaaact 5100
tgaatgaaat ggattttaca gaaagcttta tgataatttt tgaatgcatt atttattttt 5160
tgtgccatgc attttttttc tcaccaaatg accttacctg taatacagtc ttgtttgtct 5220
gtttacaacc atgtatttat tgcaatgtac atactgtaat gttaattgta aattatctgt 5280
tcttattaaa acatcatccc atgatgggat ggtgttgata tatttggaaa ctcttggtga 5340
gagaatgaat ggtgtgtata catactctgt acatttttct tttctcctgt aatatagtct 5400
tgtcacctta gagcttgttt atggaagatt caagaaaact ataaaatact taaagatata 5460
taaatttaaa aaaacatagc tgcaggtctt tggtcccagg gctgtgcctt aactttaacc 5520
aatattttct tctgttttgc tgcatttgaa aggtaacagt ggagctaggg ctgggcattt 5580
tacatccagg cttttaattg attagaattc tgccaatagg tggattttac aaaaccacag 5640
acaacctctg aaagattctg agaccctttt gagacagaag ctcttaagta cttcttgcca 5700
gggagcagca ctgcatgtgt gatggttgtt tgccatctgt tgatcaggaa ctacttcagc 5760
tacttgcatt tgattatttc cttttttttt ttttttaact cggaaacaca actggggaaa 5820
tatattcttt cccagtgatt ataaacaatc tttttctttt ttttaagtcc ttttggcttc 5880
tagagctcat aggaaaatgg acttgatttg aaattggagc cagagtttac tcgtgttggt 5940
tatctattca tcagcttcct gacatgttaa gagaatacat taaagagaaa atactgtttt 6000
ttaatcctaa aatttttctt ccactaagat aaaccaaatg tccttacata tatgtaaacc 6060
catctattta aacgcaaagg tgggttgatg tcagtttaca tagcagaaag cattcactat 6120
cctctaagat ttgtttctgc aaaactttca ttgctttaga attttaaaat ttcaccttgt 6180
acaatggcca gcccctaaag caggaaacat ttataatgga ttatatggaa acatcctccc 6240
agtacttgcc cagcccttga atcatgtggc ttttcagtga aaggaaagat tctttttcta 6300
ggaaaaatga gcctatttta ttttatttta ttttattttt tgacacaaac tgtagatttt 6360
agcagccctg gcccaaagga atttgattac ttttgtttta aacagtacaa aggggacact 6420
ataattacaa aaacatcctt aactgatttg agttgttttt atttctttgg atatattttc 6480
agagtggtaa attgtgtgtg agaattacaa atgattattc ttttagtggt ttcttagcct 6540
ctcttacagc ccacggggat agtactgtac atcaatacct tcatatgaaa tttttatatg 6600
caatgaaaat aaaagcatgg gttgattctg cctatttatg actcaatctt ttacaaataa 6660
aagattattc attttaaatt atagttcaat cagcatgtct cttaggatac tgaacgtggt 6720
tgaaatgaaa ggatagtgac atcataagtt agtactgata ttcataacca aataaagcca 6780
acttgagtaa ttttgctaca ttaaaaatta ccaaaattac ttagatggcc tataagatta 6840
agcatggtgt tttctaagca agctttgaaa ggggccttcc atacttactt aattgaatat 6900

tctgggatat tgaaaattat tcagatactt gacaattatt tttggttacc tactccgcaa 6960

actacaaagt tttaaggact caacaataag ttaatgagac acagtgtttg ctttcatgga 7020

gcttacagtc tggaggggac aaaggcttaa acaatactca tataattata tatgtgatca 7080

gtacaatgaa ggagctcagt ggggtaaata agcaggaacc tgaacttgat ctgttccgga 7140

gggccacaga aggcttcctt gaggccttga gaaagtgatt tgcatctgag ttctgaagga 7200

ttgtaagagg taactaggga aaaagttgac aggaagagga aggggatcca gacaagaaac 7260

atttgcaaag atcttgaggc ataaatgagc ttgagacatc tggagaaact gaggaaaagt 7320

gagagagtag gcagggcctg gagccgcaga gccattgcta accatcctgt gtgagatatc 7380

ccccattctg tagctttatt ctcataaccc tgctcaattt tctttataac acttctcaca 7440

gatttatata cgtgtttgtt tttgttatct gtctctccca ccagaccaca gctccatgag 7500

agcaaggtct ttgcttacca atatatcact agcacttaaa actatgcctg gtacacagta 7560

ggttcttaat atgtgttgaa tatagccatc aaattgatat tggatataat tcaatctgat 7620

aagatatttt gagatattaa agagttttta acttgatacc ataaaaa 7667

<210> 96
<211> 8379
<212> DNA
<213> Homo sapiens

<400> 96
cactgccctg cggtgttttg aactgccttc ttacagacgt catacagccc ttgaggaata 60

gtttctgcct ggtgagattg aatgatagtt ctcattcaca aaaccctgga ttctaagcag 120

ggacacacag aaattacttt cgcaggtaaa tcagcccacc cagccaaagt gtggagagat 180

ttgttccttg gctgacttct ttgctccacg gagaggagtg ttttcctgtg cttgccctga 240

aatggaactt ccttgacagc tctcccgtgt tacagtacct cccggtcatt ttctttttct 300

ctctctctac ctgcgctctt cgagtgtcag aaacctttaa agctgttact atggaattgc 360

aaaaaagaga tcaagtgact ctttcactat gctggtttcc cttgtgaccc agatgaagaa 420

tcaattcaga attcagttcc tcccttggca ttgcaagaca cagaagaaac tgtcacttcc 480

taacagccta gtactggagt aaattcagta tgaaggaaga aagcgctcct gcgtgttaga 540

accttgccca tgagctggac cgaggacagg agatggactc caggaaaatt ggatttcttc 600

aagcagcctc ccttggaaat ggaatatctt taaaatcttc tttgcagaaa gacagttaga 660

atgtattaat cagaatagtt gaagacttat tttccttttt attttttttc aaaatgagca 720

ttattatgaa gccaagatcc cgatctacaa gttccctaag gactgcagag gcagtttgtt 780

ttgatgtgga caatggcaca tctgcgggac ggagtccctt ggatcccatg accagcccag 840

gatccgggct aattctccaa gcaaattttg tccacagtca acgacgggag tccttcctgt 900

atcgatccga cagcgattat gacctctctc caaagtctat gtcccggaac tcctccattg 960

ccagtgatat acacggagat gacttgattg tgactccatt tgctcaggtc ttggccagtc 1020

tgcgaactgt acgaaacaac tttgctgcat taactaattt gcaagatcga gcacctagca 1080

aaagatcacc catgtgcaac caaccatcca tcaacaaagc caccataaca gaggaggcct 1140

accagaaact ggccagcgag accctggagg agctggactg gtgtctggac cagctagaga 1200

ccctacagac caggcactcc gtcagtgaga tggcctccaa caagtttaaa aggatgctta 1260

atcgggagct cacccatctc tctgaaatga gtcggtctgg aaatcaagtg tcagagttta 1320

tatcaaacac attcttagat aagcaacatg aagtggaaat tccttctcca actcagaagg 1380

aaaaggagaa aaagaaaaga ccaatgtctc agatcagtgg agtcaagaaa ttgatgcaca 1440

gctctagtct gactaattca agtatcccaa ggtttggagt taaaactgaa caagaagatg 1500

tccttgccaa ggaactagaa gatgtgaaca aatggggtct tcatgttttc agaatagcag 1560

agttgtctgg taaccggccc ttgactgtta tcatgcacac catttttcag gaacgggatt 1620

tattaaaaac atttaaaatt ccagtagata ctttaattac atatcttatg actctcgaag 1680

accattacca tgctgatgtg gcctatcaca acaatatcca tgctgcagat gttgtccagt 1740

ctactcatgt gctattatct acacctgctt tggaggctgt gtttacagat ttggagattc 1800

ttgcagcaat tttgccagt gcaatacatg atgtagatca tcctggtgtg tccaatcaat 1860

ttctgatcaa tacaaactct gaacttgcct tgatgtacaa tgattcctca gtcttagaga 1920

accatcattt ggctgtgggc tttaaattgc ttcaggaaga aaactgtgac attttccaga 1980

atttgaccaa aaaacaaaga caatctttaa ggaaaatggt cattgacatc gtacttgcaa 2040

cagatatgtc aaaacacatg aatctactgg ctgatttgaa gactatggtt gaaactaaga 2100

aagtgacaag ctctggagtt cttcttcttg ataattattc cgataggatt caggttcttc 2160

agaatatggt gcactgtgca gatctgagca acccaacaaa gcctctccag ctgtaccgcc 2220

agtggacgga ccggataatg gaggagttct tccgccaagg agaccgagag agggaacgtg 2280

gcatggagat aagccccatg tgtgacaagc acaatgcttc cgtggaaaaa tcacaggtgg 2340

gcttcataga ctatattgtt catcccctct gggagacatg ggcagacctc gtccaccctg 2400

acgcccagga tattttggac actttggagg acaatcgtga atggtaccag agcacaatcc 2460

ctcagagccc ctctcctgca cctgatgacc cagaggaggg ccggcagggt caaactgaga 2520

aattccagtt tgaactaact ttagaggaag atggtgagtc agacacggaa aaggacagtg 2580

gcagtcaagt ggaagaagac actagctgca gtgactccaa gactctttgt actcaagact 2640

cagagtctac tgaaattccc cttgatgaac aggttgaaga ggaggcagta ggggaagaag 2700

aggaaagcca gcctgaagcc tgtgtcatag atgatcgttc tcctgacacg taacagtgca 2760

aaaactttca tgcctttttt ttttttaagt agaaaaattg tttccaaagt gcatgtcaca 2820
tgccacaacc acggtcacac ctcactgtca tctgccagga cgtttgttga acaaaactga 2880
ccttgactac tcagtccagc gctcaggaat atcgtaacca gtttttcac ctccatgtca 2940
tccgagcaag gtggacatct tcacgaacag cgtttttaac aagatttcag cttggtagag 3000
ctgacaaagc agataaaatc tactccaaat tattttcaag agagtgtgac tcatcaggca 3060
gcccaaaagt ttattggact tggggtttct attccttttt atttgtttgc aatattttca 3120
gaagaaaggc attgcacaga gtgaacttaa tggacgaagc aacaaatatg tcaagaacag 3180
gacatagcac gaatctgtta ccagtaggag gaggatgagc cacagaaatt gcataatttt 3240
ctaatttcaa gtcttcctga tacatgactg aatagtgtgg ttcagtgagc tgcactgacc 3300
tctacatttt gtatgatatg taaaacagat tttttgtaga gcttactttt attattaaat 3360
gtattgaggt attatattta aaaaaaacta tgttcagaac ttcatctgcc actggttatt 3420
tttttctaag gagtaacttg caagttttca gtacaaatct gtgctacact ggataaaaat 3480
ctaatttatg aattttactt gcaccttata gttcatagca attaactgat ttgtagtgat 3540
tcattgtttg ttttatatac caatgacttc catattttaa aagagaaaaa caactttatg 3600
ttgcaggaaa cccttttttgt aagtctttat tatttacttt gcattttgtt tcactctttc 3660
cagataagca gagttgctct tcaccagtgt ttttcttcat gtgcaaagtg actatttgtt 3720
ctataatact tttatgtgtg ttatatcaaa tgtgtcttaa gcttcatgca aactcagtca 3780
tcagttcgtg ttgtctgaag caagtgggag atatataaat acccagtagc taaaatggtc 3840
agtctttttt agatgttttc ctacttagta tctcctaata acgttttgct gtgtcactag 3900
atgttcattt cacaagtgca tgtctttcta ataatccaca catttcatgc tctaataatc 3960
cacacatttc atgctcattt ttattgtttt tacagccagt tatagtaaga aaaaggtttt 4020
tccccttgtg ctgctttata atttagcgtg tgtctgaacc ttatccatgt ttgctagatg 4080
aggtcttgtc aaatatatca ctaccattgt caccggtgaa aagaaacagg tagttaagtt 4140
agggttaaca ttcatttcaa ccacgaggtt gtatatcatg actagctttt actcttggtt 4200
tacagagaaa agttaaacag ccaactaggc agtttttaag aatattaaca atatattaac 4260
aaacaccaat acaactaatc ctatttggtt ttaatgattt caccatggga ttaagaacta 4320
tatcaggaac atccctgaga aacggtttta agtgtagcaa ctactcttcc ttaatggaca 4380
gccacataac gtgtaggaag tcctttatca cttatcctcg atccataagc atatcttgca 4440
gaggggaact acttctttaa acacatggag ggaaagaaga tgatgccact ggcaccagag 4500
ggttagtact gtgatgcatc ctaaaatatt tattatattg gtaaaaattc tggttaaata 4560
aaaaattaga gatcactctt ggctgatttc agcaccagga actgtattac agttttagag 4620
attaattcct agtgtttacc tgattatagc agttggcatc atggggcatt taattctgac 4680

tttatcccca cgtcagcctt aataaagtct tctttacctt ctctatgaag actttaaagc 4740

ccaaataatc atttttcaca ttgatattca agaattgaga tagatagaag ccaaagtggg 4800

tatctgacaa gtggaaaatc aaacgtttaa gaagaattac aactctgaaa agcatttata 4860

tgtggaactt ctcaaggagc ctcctgggga ctggaaagta agtcatcagc caggcaaatg 4920

actcatgctg aagagagtcc ccatttcagt cccctgagat ctagctgatg cttagatcct 4980

ttgaaataaa aattatgtct ttataactct gatcttttac ataaagcaga agaggaatca 5040

actagttaat tgcaaggttt ctactctgtt tcctctgtaa agatcagatg gtaatctttc 5100

aaataagaaa aaaataaaga cgtatgtttg accaagtagt ttcacaagaa tatttgggaa 5160

cttgtttctt ttaattttat ttgtccctga gtgaagtcta gaaagaaagg taaagagtct 5220

agagtttatt cctctttcca aaacattctc attcctctcc tccctacact tagtatttcc 5280

cccacagagt gcctagaatc ttaataatga ataaaataaa aagcagcaat atgtcattaa 5340

caaatccaga cctgaaaggg taaagggttt ataactgcac taataaagag aggctctttt 5400

tttttcttcc agtttgttgg tttttaatgg taccgtgttg taaagatacc cactaatgga 5460

caatcaaatt gcagaaaagg ctcaatatcc aagagacagg gactaatgca ctgtacaatc 5520

tgcttatcct tgcccttctc tcttgccaaa gtgtgcttca gaaatatata ctgctttaaa 5580

aaagaataaa agaatatcct tttacaagtg gctttacatt tcctaaaatg ccataagaaa 5640

atgcaatatc tgggtactgt atggggaaaa aaatgtccaa gtttgtgtaa aaccagtgca 5700

tttcagcttg caagttactg aacacaataa tgctgtttta attttgtttt atatcagtta 5760

aaattcacaa taatgtagat agaacaaatt acagacaagg aaagaaaaaa cttgaatgaa 5820

atggatttta cagaaagctt tatgataatt tttgaatgca ttatttattt tttgtgccat 5880

gcattttttt tctcaccaaa tgaccttacc tgtaatacag tcttgtttgt ctgtttacaa 5940

ccatgtattt attgcaatgt acatactgta atgttaattg taaattatct gttcttatta 6000

aaacatcatc ccatgatggg atggtgttga tatatttgga aactcttggt gagagaatga 6060

atggtgtgta tacatactct gtacattttt cttttctcct gtaatatagt cttgtcacct 6120

tagagcttgt ttatggaaga ttcaagaaaa ctataaaata cttaaagata tataaattta 6180

aaaaaacata gctgcaggtc tttggtccca gggctgtgcc ttaactttaa ccaatatttt 6240

cttctgtttt gctgcatttg aaaggtaaca gtggagctag ggctgggcat tttacatcca 6300

ggcttttaat tgattagaat tctgccaata ggtggatttt acaaaaccac agacaacctc 6360

tgaaagattc tgagaccctt ttgagacaga agctcttaag tacttcttgc cagggagcag 6420

cactgcatgt gtgatggttg tttgccatct gttgatcagg aactacttca gctacttgca 6480

tttgattatt tccttttttt ttttttttaa ctcggaaaca caactgggga aatatattct 6540

ttcccagtga ttataaacaa tctttttctt ttttttaagt ccttttggct tctagagctc 6600
ataggaaaat ggacttgatt tgaaattgga gccagagttt actcgtgttg gttatctatt 6660
catcagcttc ctgacatgtt aagagaatac attaaagaga aaatactgtt ttttaatcct 6720
aaaattttc ttccactaag ataaaccaaa tgtccttaca tatatgtaaa cccatctatt 6780
taaacgcaaa ggtgggttga tgtcagttta catagcagaa agcattcact atcctctaag 6840
atttgtttct gcaaaacttt cattgcttta gaattttaaa atttcacctt gtacaatggc 6900
cagcccctaa agcaggaaac atttataatg gattatatgg aaacatcctc ccagtacttg 6960
cccagccctt gaatcatgtg gcttttcagt gaaaggaaag attctttttc taggaaaaat 7020
gagcctattt tattttattt tattttattt tttgacacaa actgtagatt ttagcagccc 7080
tggcccaaag gaatttgatt acttttgttt taaacagtac aaaggggaca ctataattac 7140
aaaaacatcc ttaactgatt tgagttgttt ttatttcttt ggatatattt tcagagtggt 7200
aaattgtgtg tgagaattac aaatgattat tcttttagtg gtttcttagc ctctcttaca 7260
gcccacgggg atagtactgt acatcaatac cttcatatga aatttttata tgcaatgaaa 7320
ataaaagcat gggttgattc tgcctattta tgactcaatc ttttacaaat aaaagattat 7380
tcattttaaa ttatagttca atcagcatgt ctcttaggat actgaacgtg gttgaaatga 7440
aaggatagtg acatcataag ttagtactga tattcataac caaataaagc caacttgagt 7500
aattttgcta cattaaaaat taccaaaatt acttagatgg cctataagat taagcatggt 7560
gttttctaag caagctttga aaggggcctt ccatacttac ttaattgaat attctgggat 7620
attgaaaatt attcagatac ttgacaatta tttttggtta cctactccgc aaactacaaa 7680
gttttaagga ctcaacaata agttaatgag acacagtgtt tgctttcatg gagcttacag 7740
tctggagggg acaaaggctt aaacaatact catataatta tatatgtgat cagtacaatg 7800
aaggagctca gtggggtaaa taagcaggaa cctgaacttg atctgttccg gagggccaca 7860
gaaggcttcc ttgaggcctt gagaaagtga tttgcatctg agttctgaag gattgtaaga 7920
ggtaactagg gaaaaagttg acaggaagag gaaggggatc cagacaagaa acatttgcaa 7980
agatcttgag gcataaatga gcttgagaca tctggagaaa ctgaggaaaa gtgagagagt 8040
aggcagggcc tggagccgca gagccattgc taaccatcct gtgtgagata tcccccattc 8100
tgtagcttta ttctcataac cctgctcaat tttctttata acacttctca cagatttata 8160
tacgtgtttg tttttgttat ctgtctctcc caccagacca cagctccatg agagcaaggt 8220
ctttgcttac caatatatca ctagcactta aaactatgcc tggtacacag taggttctta 8280
atatgtgttg aatatagcca tcaaattgat attggatata attcaatctg ataagatatt 8340
ttgagatatt aaagagtttt taacttgata ccataaaaa 8379

<210> 97
<211> 7545
<212> DNA
<213> Homo sapiens

<400> 97
ctttcttgca actaagcatc ttgacataca ttattcatta agccctggag ctcgggagag 60

aaagatgcag acccttagat ctttagatat tcctttatca cgtggatttt ctttattcag 120

aatagttgct gaattttgtg ccattctgga gtcttacaaa tggcatgtat tcgatgggaa 180

gacggctgga tgggatttaa tgcgaggctt tcttatgtat acttaattac caaaaatctt 240

taaaaactca tactctgcgt ggcttgtgga ggttgttaaa gtgtcgagat tttgaagcta 300

aatacatttt agagcttact atatatatac atatatatat atatacatat aatcaatcaa 360

aaatgcctga agcaaactat ttactgtcag tgtcttgggg ctacataaag tttaaaagga 420

tgcttaatcg ggagctcacc catctctctg aaatgagtcg gtctggaaat caagtgtcag 480

agtttatatc aaacacattc ttagataagc aacatgaagt ggaaattcct tctccaactc 540

agaaggaaaa ggagaaaaag aaaagaccaa tgtctcagat cagtggagtc aagaaattga 600

tgcacagctc tagtctgact aattcaagta tcccaaggtt tggagttaaa actgaacaag 660

aagatgtcct tgccaaggaa ctagaagatg tgaacaaatg gggtcttcat gttttcagaa 720

tagcagagtt gtctggtaac cggcccttga ctgttatcat gcacaccatt tttcaggaac 780

gggatttatt aaaaacattt aaaattccag tagatacttt aattacatat cttatgactc 840

tcgaagacca ttaccatgct gatgtggcct atcacaacaa tatccatgct gcagatgttg 900

tccagtctac tcatgtgcta ttatctacac ctgctttgga ggctgtgttt acagatttgg 960

agattcttgc agcaattttt gccagtgcaa tacatgatgt agatcatcct ggtgtgtcca 1020

atcaatttct gatcaataca aactctgaac ttgccttgat gtacaatgat tcctcagtct 1080

tagagaacca tcatttggct gtgggcttta aattgcttca ggaagaaaac tgtgacattt 1140

tccagaattt gaccaaaaaa caaagacaat ctttaaggaa aatggtcatt gacatcgtac 1200

ttgcaacaga tatgtcaaaa cacatgaatc tactggctga tttgaagact atggttgaaa 1260

ctaagaaagt gacaagctct ggagttcttc ttcttgataa ttattccgat aggattcagg 1320

ttcttcagaa tatggtgcac tgtgcagatc tgagcaaccc aacaaagcct ctccagctgt 1380

accgccagtg gacggaccgg ataatggagg agttcttccg ccaaggagac cgagagaggg 1440

aacgtggcat ggagataagc cccatgtgtg acaagcacaa tgcttccgtg gaaaaatcac 1500

aggtgggctt catagactat attgttcatc ccctctggga gacatgggca gacctcgtcc 1560

accctgacgc ccaggatatt ttggacactt tggaggacaa tcgtgaatgg taccagagca 1620

caatccctca gagcccctct cctgcacctg atgacccaga ggagggccgg caggtcaaa 1680

ctgagaaatt ccagtttgaa ctaactttag aggaagatgg tgagtcagac acggaaaagg 1740

acagtggcag tcaagtggaa gaagacacta gctgcagtga ctccaagact ctttgtactc 1800
aagactcaga gtctactgaa attccccttg atgaacaggt tgaagaggag gcagtagggg 1860
aagaagagga aagccagcct gaagcctgtg tcatagatga tcgttctcct gacacgtaac 1920
agtgcaaaaa ctttcatgcc tttttttttt ttaagtagaa aaattgtttc caaagtgcat 1980
gtcacatgcc acaaccacgg tcacacctca ctgtcatctg ccaggacgtt tgttgaacaa 2040
aactgacctt gactactcag tccagcgctc aggaatatcg taaccagttt tttcacctcc 2100
atgtcatccg agcaaggtgg acatcttcac gaacagcgtt tttaacaaga tttcagcttg 2160
gtagagctga caaagcagat aaaatctact ccaaattatt ttcaagagag tgtgactcat 2220
caggcagccc aaaagtttat tggacttggg gtttctattc ctttttattt gtttgcaata 2280
ttttcagaag aaaggcattg cacagagtga acttaatgga cgaagcaaca aatatgtcaa 2340
gaacaggaca tagcacgaat ctgttaccag taggaggagg atgagccaca gaaattgcat 2400
aattttctaa tttcaagtct tcctgataca tgactgaata gtgtggttca gtgagctgca 2460
ctgacctcta cattttgtat gatatgtaaa acagattttt tgtagagctt acttttatta 2520
ttaaatgtat tgaggtatta tatttaaaaa aaactatgtt cagaacttca tctgccactg 2580
gttatttttt tctaaggagt aacttgcaag ttttcagtac aaatctgtgc tacactggat 2640
aaaaatctaa tttatgaatt ttacttgcac cttatagttc atagcaatta actgatttgt 2700
agtgattcat tgtttgtttt atataccaat gacttccata ttttaaaaga gaaaaacaac 2760
tttatgttgc aggaaaccct ttttgtaagt ctttattatt tactttgcat tttgtttcac 2820
tctttccaga taagcagagt tgctcttcac cagtgttttt cttcatgtgc aaagtgacta 2880
tttgttctat aatactttta tgtgtgttat atcaaatgtg tcttaagctt catgcaaact 2940
cagtcatcag ttcgtgttgt ctgaagcaag tgggagatat ataaataccc agtagctaaa 3000
atggtcagtc ttttttagat gttttcctac ttagtatctc ctaataacgt tttgctgtgt 3060
cactagatgt tcatttcaca agtgcatgtc tttctaataa tccacacatt tcatgctcta 3120
ataatccaca catttcatgc tcatttttat tgtttttaca gccagttata gtaagaaaaa 3180
ggtttttccc cttgtgctgc tttataattt agcgtgtgtc tgaaccttat ccatgtttgc 3240
tagatgaggt cttgtcaaat atatcactac cattgtcacc ggtgaaaaga aacaggtagt 3300
taagttaggg ttaacattca tttcaaccac gaggttgtat atcatgacta gcttttactc 3360
ttggtttaca gagaaaagtt aaacagccaa ctaggcagtt tttaagaata ttaacaatat 3420
attaacaaac accaatacaa ctaatcctat ttggttttaa tgatttcacc atgggattaa 3480
gaactatatc aggaacatcc ctgagaaacg gttttaagtg tagcaactac tcttccttaa 3540
tggacagcca cataacgtgt aggaagtcct ttatcactta tcctcgatcc ataagcatat 3600

cttgcagagg ggaactactt ctttaaacac atggagggaa agaagatgat gccactggca 3660
ccagagggtt agtactgtga tgcatcctaa aatatttatt atattggtaa aaattctggt 3720
taaataaaaa attagagatc actcttggct gatttcagca ccaggaactg tattacagtt 3780
ttagagatta attcctagtg tttacctgat tatagcagtt ggcatcatgg ggcatttaat 3840
tctgacttta tccccacgtc agccttaata aagtcttctt taccttctct atgaagactt 3900
taaagcccaa ataatcattt ttcacattga tattcaagaa ttgagataga tagaagccaa 3960
agtgggtatc tgacaagtgg aaaatcaaac gtttaagaag aattacaact ctgaaaagca 4020
tttatatgtg gaacttctca aggagcctcc tggggactgg aaagtaagtc atcagccagg 4080
caaatgactc atgctgaaga gagtccccat ttcagtcccc tgagatctag ctgatgctta 4140
gatcctttga aataaaaatt atgtctttat aactctgatc ttttacataa agcagaagag 4200
gaatcaacta gttaattgca aggtttctac tctgtttcct ctgtaaagat cagatggtaa 4260
tctttcaaat aagaaaaaaa taaagacgta tgtttgacca agtagtttca caagaatatt 4320
tgggaacttg tttcttttaa ttttatttgt ccctgagtga agtctagaaa gaaaggtaaa 4380
gagtctagag tttattcctc tttccaaaac attctcattc ctctcctccc tacacttagt 4440
atttccccca cagagtgcct agaatcttaa taatgaataa aataaaaagc agcaatatgt 4500
cattaacaaa tccagacctg aaagggtaaa gggtttataa ctgcactaat aaagagaggc 4560
tctttttttt tcttccagtt tgttggtttt taatggtacc gtgttgtaaa gatacccact 4620
aatggacaat caaattgcag aaaaggctca atatccaaga gacagggact aatgcactgt 4680
acaatctgct tatccttgcc cttctctctt gccaaagtgt gcttcagaaa tatatactgc 4740
tttaaaaaag aataaaagaa tatcctttta caagtggctt tacatttcct aaaatgccat 4800
aagaaaatgc aatatctggg tactgtatgg ggaaaaaaat gtccaagttt gtgtaaaacc 4860
agtgcatttc agcttgcaag ttactgaaca caataatgct gttttaattt tgttttatat 4920
cagttaaaat tcacaataat gtagatagaa caaattacag acaaggaaag aaaaaacttg 4980
aatgaaatgg attttacaga aagctttatg ataatttttg aatgcattat ttattttttg 5040
tgccatgcat tttttttctc accaaatgac cttacctgta atacagtctt gtttgtctgt 5100
ttacaaccat gtatttattg caatgtacat actgtaatgt taattgtaaa ttatctgttc 5160
ttattaaaac atcatcccat gatgggatgg tgttgatata tttggaaact cttggtgaga 5220
gaatgaatgg tgtgtataca tactctgtac atttttcttt tctcctgtaa tatagtcttg 5280
tcaccttaga gcttgtttat ggaagattca agaaaactat aaaatactta aagatatata 5340
aatttaaaaa aacatagctg caggtctttg gtcccagggc tgtgccttaa ctttaaccaa 5400
tattttcttc tgttttgctg catttgaaag gtaacagtgg agctagggct gggcatttta 5460
catccaggct tttaattgat tagaattctg ccaataggtg gattttacaa aaccacagac 5520

aacctctgaa agattctgag accctttga gacagaagct cttaagtact tcttgccagg 5580
gagcagcact gcatgtgtga tggttgtttg ccatctgttg atcaggaact acttcagcta 5640
cttgcatttg attatttcct ttttttttt ttttaactcg gaaacacaac tggggaaata 5700
tattctttcc cagtgattat aaacaatctt tttcttttt ttaagtcctt ttggcttcta 5760
gagctcatag gaaaatggac ttgatttgaa attggagcca gagtttactc gtgttggtta 5820
tctattcatc agcttcctga catgttaaga gaatacatta aagagaaaat actgtttttt 5880
aatcctaaaa tttttcttcc actaagataa accaaatgtc cttacatata tgtaaaccca 5940
tctatttaaa cgcaaaggtg ggttgatgtc agtttacata gcagaaagca ttcactatcc 6000
tctaagattt gtttctgcaa aactttcatt gctttagaat tttaaaattt caccttgtac 6060
aatggccagc ccctaaagca ggaaacattt ataatggatt atatggaaac atcctcccag 6120
tacttgccca gcccttgaat catgtggctt ttcagtgaaa ggaaagattc tttttctagg 6180
aaaaatgagc ctattttatt ttattttatt ttatttttg acacaaactg tagattttag 6240
cagccctggc ccaaaggaat ttgattactt ttgttttaaa cagtacaaag gggacactat 6300
aattacaaaa acatccttaa ctgatttgag ttgtttttat ttctttggat atattttcag 6360
agtggtaaat tgtgtgtgag aattacaaat gattattctt ttagtggttt cttagcctct 6420
cttacagccc acggggatag tactgtacat caataccttc atatgaaatt tttatatgca 6480
atgaaaataa aagcatgggt tgattctgcc tatttatgac tcaatctttt acaaataaaa 6540
gattattcat tttaaattat agttcaatca gcatgtctct taggatactg aacgtggttg 6600
aaatgaaagg atagtgacat cataagttag tactgatatt cataaccaaa taaagccaac 6660
ttgagtaatt ttgctacatt aaaaattacc aaaattactt agatggccta taagattaag 6720
catggtgttt tctaagcaag ctttgaaagg ggccttccat acttacttaa ttgaatattc 6780
tgggatattg aaaattattc agatacttga caattatttt tggttaccta ctccgcaaac 6840
tacaaagttt taaggactca acaataagtt aatgagacac agtgtttgct ttcatggagc 6900
ttacagtctg gaggggacaa aggcttaaac aatactcata taattatata tgtgatcagt 6960
acaatgaagg agctcagtgg ggtaaataag caggaacctg aacttgatct gttccggagg 7020
gccacagaag gcttccttga ggccttgaga aagtgatttg catctgagtt ctgaaggatt 7080
gtaagaggta actagggaaa aagttgacag gaagaggaag gggatccaga caagaaacat 7140
ttgcaaagat cttgaggcat aaatgagctt gagacatctg gagaaactga ggaaaagtga 7200
gagagtaggc agggcctgga gccgcagagc cattgctaac catcctgtgt gagatatccc 7260
ccattctgta gctttattct cataaccctg ctcaattttc tttataacac ttctcacaga 7320
tttatatacg tgtttgtttt tgttatctgt ctctcccacc agaccacagc tccatgagag 7380

caaggtcttt gcttaccaat atatcactag cacttaaaac tatgcctggt acacagtagg 7440

ttcttaatat gtgttgaata tagccatcaa attgatattg gatataattc aatctgataa 7500

gatattttga gatattaaag agtttttaac ttgataccat aaaaa 7545

<210> 98
<211> 8130
<212> DNA
<213> Homo sapiens

<400> 98
agattatagc ccagcgtacg agaagcacga gtcctatagt tggcgtaccc tgaggcctgc 60

cagttcctgc cttaatgcat atgtagtcgt aattgagttc tgacacggcc ttggatgttt 120

ctgtcctaaa tagctgacat tgcatcttca agactgtcat tccagttggc ttttgagtgg 180

atacgtgcag tgagatcatt gacactggaa acactagttc ccattttaat tacttaaaac 240

accacgatga aaagaaatac ctgtgatttg ctttctcgga gcaaaagtgc ctctgaggaa 300

acactacatt ccagtaatga agaggaagac cctttccgcg gaatggaacc ctatcttgtc 360

cggagacttt catgtcgcaa tattcagctt ccccctctcg ccttcagaca gttggaacaa 420

gctgacttga aaagtgaatc agagaacatt caacgaccaa ccagcctccc cctgaagatt 480

ctgccgctga ttgctatcac ttctgcagaa tccagtggtt ttgatgtgga caatggcaca 540

tctgcgggac ggagtccctt ggatcccatg accagcccag gatccgggct aattctccaa 600

gcaaattttg tccacagtca acgacgggag tccttcctgt atcgatccga cagcgattat 660

gacctctctc caaagtctat gtcccggaac tcctccattg ccagtgatat acacggagat 720

gacttgattg tgactccatt tgctcaggtc ttggccagtc tgcgaactgt acgaaacaac 780

tttgctgcat taactaattt gcaagatcga gcacctagca aaagatcacc catgtgcaac 840

caaccatcca tcaacaaagc caccataaca gaggaggcct accagaaact ggccagcgag 900

accctggagg agctggactg gtgtctggac cagctagaga ccctacagac caggcactcc 960

gtcagtgaga tggcctccaa caagtttaaa aggatgctta atcgggagct cacccatctc 1020

tctgaaatga gtcggtctgg aaatcaagtg tcagagttta tatcaaacac attcttagat 1080

aagcaacatg aagtggaaat tccttctcca actcagaagg aaaaggagaa aaagaaaaga 1140

ccaatgtctc agatcagtgg agtcaagaaa ttgatgcaca gctctagtct gactaattca 1200

agtatcccaa ggtttggagt taaaactgaa caagaagatg tccttgccaa ggaactagaa 1260

gatgtgaaca aatggggtct tcatgttttc agaatagcag agttgtctgg taaccggccc 1320

ttgactgtta tcatgcacac cattttttcag gaacgggatt tattaaaaac atttaaaatt 1380

ccagtagata ctttaattac atatcttatg actctcgaag accattacca tgctgatgtg 1440

gcctatcaca acaatatcca tgctgcagat gttgtccagt ctactcatgt gctattatct 1500

acacctgctt tggaggctgt gtttacagat ttggagattc ttgcagcaat ttttgccagt 1560
gcaatacatg atgtagatca tcctggtgtg tccaatcaat ttctgatcaa tacaaactct 1620
gaacttgcct tgatgtacaa tgattcctca gtcttagaga accatcattt ggctgtgggc 1680
tttaaattgc ttcaggaaga aaactgtgac attttccaga atttgaccaa aaaacaaaga 1740
caatctttaa ggaaaatggt cattgacatc gtacttgcaa cagatatgtc aaaacacatg 1800
aatctactgg ctgatttgaa gactatggtt gaaactaaga aagtgacaag ctctggagtt 1860
cttcttcttg ataattattc cgataggatt caggttcttc agaatatggt gcactgtgca 1920
gatctgagca acccaacaaa gcctctccag ctgtaccgcc agtggacgga ccggataatg 1980
gaggagttct tccgccaagg agaccgagag agggaacgtg gcatggagat aagccccatg 2040
tgtgacaagc acaatgcttc cgtggaaaaa tcacaggtgg gcttcataga ctatattgtt 2100
catcccctct gggagacatg ggcagacctc gtccaccctg acgcccagga tattttggac 2160
actttggagg acaatcgtga atggtaccag agcacaatcc ctcagagccc ctctcctgca 2220
cctgatgacc cagaggaggg ccggcagggt caaactgaga aattccagtt tgaactaact 2280
ttagaggaag atggtgagtc agacacggaa aaggacagtg gcagtcaagt ggaagaagac 2340
actagctgca gtgactccaa gactctttgt actcaagact cagagtctac tgaaattccc 2400
cttgatgaac aggttgaaga ggaggcagta ggggaagaag aggaaagcca gcctgaagcc 2460
tgtgtcatag atgatcgttc tcctgacacg taacagtgca aaaactttca tgcctttttt 2520
ttttttaagt agaaaaattg tttccaaagt gcatgtcaca tgccacaacc acggtcacac 2580
ctcactgtca tctgccagga cgtttgttga acaaaactga ccttgactac tcagtccagc 2640
gctcaggaat atcgtaacca gttttttcac ctccatgtca tccgagcaag gtggacatct 2700
tcacgaacag cgtttttaac aagatttcag cttggtagag ctgacaaagc agataaaatc 2760
tactccaaat tattttcaag agagtgtgac tcatcaggca gcccaaaagt ttattggact 2820
tggggtttct attccttttt atttgtttgc aatattttca gaagaaaggc attgcacaga 2880
gtgaacttaa tggacgaagc aacaaatatg tcaagaacag gacatagcac gaatctgtta 2940
ccagtaggag gaggatgagc cacagaaatt gcataatttt ctaatttcaa gtcttcctga 3000
tacatgactg aatagtgtgg ttcagtgagc tgcactgacc tctacatttt gtatgatatg 3060
taaaacagat tttttgtaga gcttactttt attattaaat gtattgaggt attatattta 3120
aaaaaaacta tgttcagaac ttcatctgcc actggttatt tttttctaag gagtaacttg 3180
caagttttca gtacaaatct gtgctacact ggataaaaat ctaatttatg aattttactt 3240
gcaccttata gttcatagca attaactgat ttgtagtgat tcattgtttg ttttatatac 3300
caatgacttc catattttaa aagagaaaaa caactttatg ttgcaggaaa cccttttttgt 3360
aagtctttat tatttacttt gcattttgtt tcactctttc cagataagca gagttgctct 3420

tcaccagtgt ttttcttcat gtgcaaagtg actatttgtt ctataatact tttatgtgtg 3480
ttatatcaaa tgtgtcttaa gcttcatgca aactcagtca tcagttcgtg ttgtctgaag 3540
caagtgggag atatataaat acccagtagc taaaatggtc agtctttttt agatgttttc 3600
ctacttagta tctcctaata acgttttgct gtgtcactag atgttcattt cacaagtgca 3660
tgtctttcta ataatccaca catttcatgc tctaataatc cacacatttc atgctcattt 3720
ttattgtttt tacagccagt tatagtaaga aaaaggtttt tccccttgtg ctgctttata 3780
atttagcgtg tgtctgaacc ttatccatgt ttgctagatg aggtcttgtc aaatatatca 3840
ctaccattgt caccggtgaa aagaaacagg tagttaagtt agggttaaca ttcatttcaa 3900
ccacgaggtt gtatatcatg actagctttt actcttggtt tacagagaaa agttaaacag 3960
ccaactaggc agtttttaag aatattaaca atatattaac aaacaccaat acaactaatc 4020
ctatttggtt ttaatgattt caccatggga ttaagaacta tatcaggaac atccctgaga 4080
aacggtttta agtgtagcaa ctactcttcc ttaatggaca gccacataac gtgtaggaag 4140
tcctttatca cttatcctcg atccataagc atatcttgca gaggggaact acttctttaa 4200
acacatggag ggaaagaaga tgatgccact ggcaccagag ggttagtact gtgatgcatc 4260
ctaaaatatt tattatattg gtaaaaattc tggttaaata aaaaattaga gatcactctt 4320
ggctgatttc agcaccagga actgtattac agttttagag attaattcct agtgtttacc 4380
tgattatagc agttggcatc atggggcatt taattctgac tttatcccca cgtcagcctt 4440
aataaagtct tctttacctt ctctatgaag actttaaagc ccaaataatc atttttcaca 4500
ttgatattca agaattgaga tagatagaag ccaaagtggg tatctgacaa gtggaaaatc 4560
aaacgtttaa gaagaattac aactctgaaa agcatttata tgtggaactt ctcaaggagc 4620
ctcctgggga ctggaaagta agtcatcagc caggcaaatg actcatgctg aagagagtcc 4680
ccatttcagt cccctgagat ctagctgatg cttagatcct ttgaaataaa aattatgtct 4740
ttataactct gatcttttac ataaagcaga agaggaatca actagttaat tgcaaggttt 4800
ctactctgtt tcctctgtaa agatcagatg gtaatctttc aaataagaaa aaaataaaga 4860
cgtatgtttg accaagtagt ttcacaagaa tatttgggaa cttgtttctt ttaattttat 4920
ttgtccctga gtgaagtcta gaaagaaagg taaagagtct agagtttatt cctctttcca 4980
aaacattctc attcctctcc tccctacact tagtatttcc cccacagagt gcctagaatc 5040
ttaataatga ataaaataaa aagcagcaat atgtcattaa caaatccaga cctgaaaggg 5100
taaagggttt ataactgcac taataaagag aggctctttt tttttcttcc agtttgttgg 5160
tttttaatgg taccgtgttg taaagatacc cactaatgga caatcaaatt gcagaaaagg 5220
ctcaatatcc aagagacagg gactaatgca ctgtacaatc tgcttatcct tgcccttctc 5280

tcttgccaaa gtgtgcttca gaaatatata ctgctttaaa aaagaataaa agaatatcct 5340

tttacaagtg gctttacatt tcctaaaatg ccataagaaa atgcaatatc tgggtactgt 5400

atggggaaaa aaatgtccaa gtttgtgtaa aaccagtgca tttcagcttg caagttactg 5460

aacacaataa tgctgtttta attttgtttt atatcagtta aaattcacaa taatgtagat 5520

agaacaaatt acagacaagg aaagaaaaaa cttgaatgaa atggatttta cagaaagctt 5580

tatgataatt tttgaatgca ttatttattt tttgtgccat gcattttttt tctcaccaaa 5640

tgaccttacc tgtaatacag tcttgtttgt ctgtttacaa ccatgtattt attgcaatgt 5700

acatactgta atgttaattg taaattatct gttcttatta aaacatcatc ccatgatggg 5760

atggtgttga tatatttgga aactcttggt gagagaatga atggtgtgta tacatactct 5820

gtacattttt cttttctcct gtaatatagt cttgtcacct tagagcttgt ttatggaaga 5880

ttcaagaaaa ctataaaata cttaaagata tataaattta aaaaaacata gctgcaggtc 5940

tttggtccca gggctgtgcc ttaactttaa ccaatatttt cttctgtttt gctgcatttg 6000

aaaggtaaca gtggagctag ggctgggcat tttacatcca ggcttttaat tgattagaat 6060

tctgccaata ggtggatttt acaaaaccac agacaacctc tgaaagattc tgagaccctt 6120

ttgagacaga agctcttaag tacttcttgc cagggagcag cactgcatgt gtgatggttg 6180

tttgccatct gttgatcagg aactacttca gctacttgca tttgattatt tccttttttt 6240

ttttttttaa ctcggaaaca caactgggga aatatattct ttcccagtga ttataaacaa 6300

tctttttctt ttttttaagt ccttttggct tctagagctc ataggaaaat ggacttgatt 6360

tgaaattgga gccagagttt actcgtgttg gttatctatt catcagcttc ctgacatgtt 6420

aagagaatac attaaagaga aaatactgtt ttttaatcct aaaatttttc ttccactaag 6480

ataaaccaaa tgtccttaca tatatgtaaa cccatctatt taaacgcaaa ggtgggttga 6540

tgtcagttta catagcagaa agcattcact atcctctaag atttgtttct gcaaaacttt 6600

cattgcttta gaattttaaa atttcacctt gtacaatggc cagcccctaa agcaggaaac 6660

atttataatg gattatatgg aaacatcctc ccagtacttg cccagccctt gaatcatgtg 6720

gcttttcagt gaaaggaaag attctttttc taggaaaaat gagcctattt tattttattt 6780

tattttattt tttgacacaa actgtagatt ttagcagccc tggcccaaag gaatttgatt 6840

acttttgttt taaacagtac aaaggggaca ctataattac aaaaacatcc ttaactgatt 6900

tgagttgttt ttatttcttt ggatatattt tcagagtggt aaattgtgtg tgagaattac 6960

aaatgattat tcttttagtg gtttcttagc ctctcttaca gcccacgggg atagtactgt 7020

acatcaatac cttcatatga aatttttata tgcaatgaaa ataaaagcat gggttgattc 7080

tgcctattta tgactcaatc ttttacaaat aaaagattat tcattttaaa ttatagttca 7140

atcagcatgt ctcttaggat actgaacgtg gttgaaatga aaggatagtg acatcataag 7200

ttagtactga tattcataac caaataaagc caacttgagt aattttgcta cattaaaaat 7260

taccaaaatt acttagatgg cctataagat taagcatggt gttttctaag caagctttga 7320

aaggggcctt ccatacttac ttaattgaat attctgggat attgaaaatt attcagatac 7380

ttgacaatta tttttggtta cctactccgc aaactacaaa gttttaagga ctcaacaata 7440

agttaatgag acacagtgtt tgctttcatg gagcttacag tctggagggg acaaaggctt 7500

aaacaatact catataatta tatatgtgat cagtacaatg aaggagctca gtggggtaaa 7560

taagcaggaa cctgaacttg atctgttccg gagggccaca gaaggcttcc ttgaggcctt 7620

gagaaagtga tttgcatctg agttctgaag gattgtaaga ggtaactagg gaaaaagttg 7680

acaggaagag gaaggggatc cagacaagaa acatttgcaa agatcttgag gcataaatga 7740

gcttgagaca tctggagaaa ctgaggaaaa gtgagagagt aggcagggcc tggagccgca 7800

gagccattgc taaccatcct gtgtgagata tcccccattc tgtagcttta ttctcataac 7860

cctgctcaat tttctttata acacttctca cagatttata tacgtgtttg tttttgttat 7920

ctgtctctcc caccagacca cagctccatg agagcaaggt ctttgcttac caatatatca 7980

ctagcactta aaactatgcc tggtacacag taggttctta atatgtgttg aatatagcca 8040

tcaaattgat attggatata attcaatctg ataagatatt ttgagatatt aaagagtttt 8100

taacttgata ccataaaaaa aaaaaaaaaa 8130

<210> 99
<211> 7814
<212> DNA
<213> Homo sapiens

<400> 99
acaaaggaaa tgccctcact cagctacaag tgcctcctgc tccgtgcggg gcctcagggc 60

cccagagccc cgcaccagct ctgacttctc gtggttcctg gggatcttgg catcgtcctt 120

aaaaatggct tttgtttggg atcctctggg agccacggtg ccaggaccat ctacaagagc 180

caaatcaaga ttgcgtttct caaagtccta cagttttgat gtggacaatg gcacatctgc 240

gggacggagt cccttggatc ccatgaccag cccaggatcc gggctaattc tccaagcaaa 300

ttttgtccac agtcaacgac gggagtcctt cctgtatcga tccgacagcg attatgacct 360

ctctccaaag tctatgtccc ggaactcctc cattgccagt gatatacacg gagatgactt 420

gattgtgact ccatttgctc aggtcttggc cagtctgcga actgtacgaa acaactttgc 480

tgcattaact aatttgcaag atcgagcacc tagcaaaaga tcacccatgt gcaaccaacc 540

atccatcaac aaagccacca taacagagga ggcctaccag aaactggcca gcgagaccct 600

ggaggagctg gactggtgtc tggaccagct agagacccta cagaccaggc actccgtcag 660

tgagatggcc tccaacaagt ttaaaaggat gcttaatcgg gagctcaccc atctctctga 720

aatgagtcgg tctggaaatc aagtgtcaga gtttatatca aacacattct tagataagca 780
acatgaagtg gaaattcctt ctccaactca gaaggaaaag gagaaaaaga aaagaccaat 840
gtctcagatc agtggagtca agaaattgat gcacagctct agtctgacta attcaagtat 900
cccaaggttt ggagttaaaa ctgaacaaga agatgtcctt gccaaggaac tagaagatgt 960
gaacaaatgg ggtcttcatg ttttcagaat agcagagttg tctggtaacc ggcccttgac 1020
tgttatcatg cacaccattt ttcaggaacg ggatttatta aaaacattta aaattccagt 1080
agatacttta attacatatc ttatgactct cgaagaccat taccatgctg atgtggccta 1140
tcacaacaat atccatgctg cagatgttgt ccagtctact catgtgctat tatctacacc 1200
tgctttggag gctgtgttta cagatttgga gattcttgca gcaatttttg ccagtgcaat 1260
acatgatgta gatcatcctg gtgtgtccaa tcaatttctg atcaatacaa actctgaact 1320
tgccttgatg tacaatgatt cctcagtctt agagaaccat catttggctg tgggctttaa 1380
attgcttcag gaagaaaact gtgacatttt ccagaatttg accaaaaaac aaagacaatc 1440
tttaaggaaa atggtcattg acatcgtact tgcaacagat atgtcaaaac acatgaatct 1500
actggctgat ttgaagacta tggttgaaac taagaaagtg acaagctctg gagttcttct 1560
tcttgataat tattccgata ggattcaggt tcttcagaat atggtgcact gtgcagatct 1620
gagcaaccca acaaagcctc tccagctgta ccgccagtgg acggaccgga taatggagga 1680
gttcttccgc caaggagacc gagagaggga acgtggcatg gagataagcc ccatgtgtga 1740
caagcacaat gcttccgtgg aaaaatcaca ggtgggcttc atagactata ttgttcatcc 1800
cctctgggag acatgggcag acctcgtcca ccctgacgcc caggatattt tggacacttt 1860
ggaggacaat cgtgaatggt accagagcac aatccctcag agcccctctc ctgcacctga 1920
tgacccagag gagggccggc agggtcaaac tgagaaattc cagtttgaac taactttaga 1980
ggaagatggt gagtcagaca cggaaaagga cagtggcagt caagtggaag aagacactag 2040
ctgcagtgac tccaagactc tttgtactca agactcagag tctactgaaa ttccccttga 2100
tgaacaggtt gaagaggagg cagtagggga agaagaggaa agccagcctg aagcctgtgt 2160
catagatgat cgttctcctg acacgtaaca gtgcaaaaac tttcatgcct tttttttttt 2220
taagtagaaa aattgtttcc aaagtgcatg tcacatgcca caaccacggt cacacctcac 2280
tgtcatctgc caggacgttt gttgaacaaa actgaccttg actactcagt ccagcgctca 2340
ggaatatcgt aaccagtttt ttcacctcca tgtcatccga gcaaggtgga catcttcacg 2400
aacagcgttt ttaacaagat ttcagcttgg tagagctgac aaagcagata aaatctactc 2460
caaattattt tcaagagagt gtgactcatc aggcagccca aaagtttatt ggacttgggg 2520
tttctattcc tttttatttg tttgcaatat tttcagaaga aaggcattgc acagagtgaa 2580

cttaatggac gaagcaacaa atatgtcaag aacaggacat agcacgaatc tgttaccagt 2640
aggaggagga tgagccacag aaattgcata attttctaat ttcaagtctt cctgatacat 2700
gactgaatag tgtggttcag tgagctgcac tgacctctac attttgtatg atatgtaaaa 2760
cagatttttt gtagagctta cttttattat taaatgtatt gaggtattat atttaaaaaa 2820
aactatgttc agaacttcat ctgccactgg ttattttttt ctaaggagta acttgcaagt 2880
tttcagtaca aatctgtgct acactggata aaaatctaat ttatgaattt tacttgcacc 2940
ttatagttca tagcaattaa ctgatttgta gtgattcatt gtttgtttta tataccaatg 3000
acttccatat tttaaaagag aaaaacaact ttatgttgca ggaaaccctt tttgtaagtc 3060
tttattattt actttgcatt ttgtttcact ctttccagat aagcagagtt gctcttcacc 3120
agtgtttttc ttcatgtgca aagtgactat ttgttctata atacttttat gtgtgttata 3180
tcaaatgtgt cttaagcttc atgcaaactc agtcatcagt tcgtgttgtc tgaagcaagt 3240
gggagatata taaataccca gtagctaaaa tggtcagtct tttttagatg ttttcctact 3300
tagtatctcc taataacgtt ttgctgtgtc actagatgtt catttcacaa gtgcatgtct 3360
ttctaataat ccacacattt catgctctaa taatccacac atttcatgct catttttatt 3420
gtttttacag ccagttatag taagaaaaag gtttttcccc ttgtgctgct ttataattta 3480
gcgtgtgtct gaaccttatc catgtttgct agatgaggtc ttgtcaaata tatcactacc 3540
attgtcaccg gtgaaaagaa acaggtagtt aagttagggt taacattcat ttcaaccacg 3600
aggttgtata tcatgactag cttttactct tggtttacag agaaaagtta aacagccaac 3660
taggcagttt ttaagaatat taacaatata ttaacaaaca ccaatacaac taatcctatt 3720
tggttttaat gatttcacca tgggattaag aactatatca ggaacatccc tgagaaacgg 3780
ttttaagtgt agcaactact cttccttaat ggacagccac ataacgtgta ggaagtcctt 3840
tatcacttat cctcgatcca taagcatatc ttgcagaggg gaactacttc tttaaacaca 3900
tggagggaaa gaagatgatg ccactggcac cagagggtta gtactgtgat gcatcctaaa 3960
atatttatta tattggtaaa aattctggtt aaataaaaaa ttagagatca ctcttggctg 4020
atttcagcac caggaactgt attacagttt tagagattaa ttcctagtgt ttacctgatt 4080
atagcagttg gcatcatggg gcatttaatt ctgactttat ccccacgtca gccttaataa 4140
agtcttcttt accttctcta tgaagacttt aaagcccaaa taatcatttt tcacattgat 4200
attcaagaat tgagatagat agaagccaaa gtgggtatct gacaagtgga aaatcaaacg 4260
tttaagaaga attacaactc tgaaaagcat ttatatgtgg aacttctcaa ggagcctcct 4320
ggggactgga aagtaagtca tcagccaggc aaatgactca tgctgaagag agtccccatt 4380
tcagtcccct gagatctagc tgatgcttag atcctttgaa ataaaaatta tgtctttata 4440
actctgatct tttacataaa gcagaagagg aatcaactag ttaattgcaa ggtttctact 4500

ctgtttcctc tgtaaagatc agatggtaat ctttcaaata agaaaaaaat aaagacgtat 4560
gtttgaccaa gtagtttcac aagaatattt gggaacttgt ttcttttaat tttatttgtc 4620
cctgagtgaa gtctagaaag aaaggtaaag agtctagagt ttattcctct ttccaaaaca 4680
ttctcattcc tctcctccct acacttagta tttcccccac agagtgccta gaatcttaat 4740
aatgaataaa ataaaaagca gcaatatgtc attaacaaat ccagacctga aagggtaaag 4800
ggtttataac tgcactaata aagagaggct cttttttttt cttccagttt gttggttttt 4860
aatggtaccg tgttgtaaag atacccacta atggacaatc aaattgcaga aaaggctcaa 4920
tatccaagag acagggacta atgcactgta caatctgctt atccttgccc ttctctcttg 4980
ccaaagtgtg cttcagaaat atatactgct ttaaaaaaga ataaaagaat atccttttac 5040
aagtggcttt acatttccta aaatgccata agaaaatgca atatctgggt actgtatggg 5100
gaaaaaaatg tccaagtttg tgtaaaacca gtgcatttca gcttgcaagt tactgaacac 5160
aataatgctg ttttaatttt gttttatatc agttaaaatt cacaataatg tagatagaac 5220
aaattacaga caaggaaaga aaaaacttga atgaaatgga ttttacagaa agctttatga 5280
taatttttga atgcattatt tatttttgt gccatgcatt ttttttctca ccaaatgacc 5340
ttacctgtaa tacagtcttg tttgtctgtt tacaaccatg tatttattgc aatgtacata 5400
ctgtaatgtt aattgtaaat tatctgttct tattaaaaca tcatcccatg atgggatggt 5460
gttgatatat ttggaaactc ttggtgagag aatgaatggt gtgtatacat actctgtaca 5520
tttttctttt ctcctgtaat atagtcttgt caccttagag cttgtttatg gaagattcaa 5580
gaaaactata aaatacttaa agatatataa atttaaaaaa acatagctgc aggtctttgg 5640
tcccagggct gtgccttaac tttaaccaat attttcttct gttttgctgc atttgaaagg 5700
taacagtgga gctagggctg ggcattttac atccaggctt ttaattgatt agaattctgc 5760
caataggtgg attttacaaa accacagaca acctctgaaa gattctgaga ccctttttgag 5820
acagaagctc ttaagtactt cttgccaggg agcagcactg catgtgtgat ggttgtttgc 5880
catctgttga tcaggaacta cttcagctac ttgcatttga ttatttcctt tttttttttt 5940
tttaactcgg aaacacaact ggggaaatat attctttccc agtgattata aacaatcttt 6000
ttcttttttt taagtccttt tggcttctag agctcatagg aaaatggact tgatttgaaa 6060
ttggagccag agtttactcg tgttggttat ctattcatca gcttcctgac atgttaagag 6120
aatacattaa agagaaaata ctgtttttta atcctaaaat ttttcttcca ctaagataaa 6180
ccaaatgtcc ttacatatat gtaaacccat ctatttaaac gcaaaggtgg gttgatgtca 6240
gtttacatag cagaaagcat tcactatcct ctaagatttg tttctgcaaa actttcattg 6300
ctttagaatt ttaaaatttc accttgtaca atggccagcc cctaaagcag gaaacattta 6360

taatggatta tatggaaaca tcctcccagt acttgcccag cccttgaatc atgtggcttt 6420

tcagtgaaag gaaagattct ttttctagga aaaatgagcc tattttattt tattttattt 6480

tattttttga cacaaactgt agattttagc agccctggcc caaaggaatt tgattacttt 6540

tgttttaaac agtacaaagg ggacactata attacaaaaa catccttaac tgatttgagt 6600

tgtttttatt tctttggata tattttcaga gtggtaaatt gtgtgtgaga attacaaatg 6660

attattcttt tagtggtttc ttagcctctc ttacagccca cggggatagt actgtacatc 6720

aataccttca tatgaaattt ttatatgcaa tgaaaataaa agcatgggtt gattctgcct 6780

atttatgact caatctttta caaataaaag attattcatt ttaaattata gttcaatcag 6840

catgtctctt aggatactga acgtggttga aatgaaagga tagtgacatc ataagttagt 6900

actgatattc ataaccaaat aaagccaact tgagtaattt tgctacatta aaaattacca 6960

aaattactta gatggcctat aagattaagc atggtgtttt ctaagcaagc tttgaaaggg 7020

gccttccata cttacttaat tgaatattct gggatattga aaattattca gatacttgac 7080

aattattttt ggttacctac tccgcaaact acaaagtttt aaggactcaa caataagtta 7140

atgagacaca gtgtttgctt tcatggagct tacagtctgg aggggacaaa ggcttaaaca 7200

atactcatat aattatatat gtgatcagta caatgaagga gctcagtggg gtaaataagc 7260

aggaacctga acttgatctg ttccggaggg ccacagaagg cttccttgag gccttgagaa 7320

agtgatttgc atctgagttc tgaaggattg taagaggtaa ctagggaaaa agttgacagg 7380

aagaggaagg ggatccagac aagaaacatt tgcaaagatc ttgaggcata aatgagcttg 7440

agacatctgg agaaactgag gaaaagtgag agagtaggca gggcctggag ccgcagagcc 7500

attgctaacc atcctgtgtg agatatcccc cattctgtag ctttattctc ataaccctgc 7560

tcaattttct ttataacact tctcacagat ttatatacgt gtttgttttt gttatctgtc 7620

tctcccacca gaccacagct ccatgagagc aaggtctttg cttaccaata tatcactagc 7680

acttaaaact atgcctggta cacagtaggt tcttaatatg tgttgaatat agccatcaaa 7740

ttgatattgg atataattca atctgataag atattttgag atattaaaga gtttttaact 7800

tgataccata aaaa 7814

<210> 100
<211> 809
<212> PRT
<213> Homo sapiens

<400> 100

Met Glu Ala Glu Gly Ser Ser Ala Pro Ala Arg Ala Gly Ser Gly Glu
1 5 10 15

Gly Ser Asp Ser Ala Gly Gly Ala Thr Leu Lys Ala Pro Lys His Leu

20 25 30

Trp Arg His Glu Gln His His Gln Tyr Pro Leu Arg Gln Pro Gln Phe
35 40 45

Arg Leu Leu His Pro His His His Leu Pro Pro Pro Pro Pro Pro Ser
50 55 60

Pro Gln Pro Gln Pro Gln Cys Pro Leu Gln Pro Pro Pro Pro Pro Pro
65 70 75 80

Leu Pro Pro Pro Pro Pro Pro Pro Gly Ala Ala Arg Gly Arg Tyr Ala
85 90 95

Ser Ser Gly Ala Thr Gly Arg Val Arg His Arg Gly Tyr Ser Asp Thr
100 105 110

Glu Arg Tyr Leu Tyr Cys Arg Ala Met Asp Arg Thr Ser Tyr Ala Val
115 120 125

Glu Thr Gly His Arg Pro Gly Leu Lys Lys Ser Arg Met Ser Trp Pro
130 135 140

Ser Ser Phe Gln Gly Leu Arg Arg Phe Asp Val Asp Asn Gly Thr Ser
145 150 155 160

Ala Gly Arg Ser Pro Leu Asp Pro Met Thr Ser Pro Gly Ser Gly Leu
165 170 175

Ile Leu Gln Ala Asn Phe Val His Ser Gln Arg Arg Glu Ser Phe Leu
180 185 190

Tyr Arg Ser Asp Ser Asp Tyr Asp Leu Ser Pro Lys Ser Met Ser Arg
195 200 205

Asn Ser Ser Ile Ala Ser Asp Ile His Gly Asp Asp Leu Ile Val Thr
210 215 220

Pro Phe Ala Gln Val Leu Ala Ser Leu Arg Thr Val Arg Asn Asn Phe
225 230 235 240

Ala Ala Leu Thr Asn Leu Gln Asp Arg Ala Pro Ser Lys Arg Ser Pro
245 250 255

Met Cys Asn Gln Pro Ser Ile Asn Lys Ala Thr Ile Thr Glu Glu Ala
260 265 270

Tyr Gln Lys Leu Ala Ser Glu Thr Leu Glu Glu Leu Asp Trp Cys Leu
275 280 285

Asp Gln Leu Glu Thr Leu Gln Thr Arg His Ser Val Ser Glu Met Ala
290 295 300

Ser Asn Lys Phe Lys Arg Met Leu Asn Arg Glu Leu Thr His Leu Ser
305 310 315 320

Glu Met Ser Arg Ser Gly Asn Gln Val Ser Glu Phe Ile Ser Asn Thr
325 330 335

Phe Leu Asp Lys Gln His Glu Val Glu Ile Pro Ser Pro Thr Gln Lys
340 345 350

Glu Lys Glu Lys Lys Lys Arg Pro Met Ser Gln Ile Ser Gly Val Lys
355 360 365

Lys Leu Met His Ser Ser Ser Leu Thr Asn Ser Ser Ile Pro Arg Phe
370 375 380

Gly Val Lys Thr Glu Gln Glu Asp Val Leu Ala Lys Glu Leu Glu Asp
385 390 395 400

Val Asn Lys Trp Gly Leu His Val Phe Arg Ile Ala Glu Leu Ser Gly
405 410 415

Asn Arg Pro Leu Thr Val Ile Met His Thr Ile Phe Gln Glu Arg Asp
420 425 430

Leu Leu Lys Thr Phe Lys Ile Pro Val Asp Thr Leu Ile Thr Tyr Leu
435 440 445

Met Thr Leu Glu Asp His Tyr His Ala Asp Val Ala Tyr His Asn Asn
450 455 460

Ile His Ala Ala Asp Val Val Gln Ser Thr His Val Leu Leu Ser Thr
465 470 475 480

Pro Ala Leu Glu Ala Val Phe Thr Asp Leu Glu Ile Leu Ala Ala Ile
485 490 495

Phe Ala Ser Ala Ile His Asp Val Asp His Pro Gly Val Ser Asn Gln
500 505 510

Phe Leu Ile Asn Thr Asn Ser Glu Leu Ala Leu Met Tyr Asn Asp Ser
515 520 525

Ser Val Leu Glu Asn His His Leu Ala Val Gly Phe Lys Leu Leu Gln
    530                 535                 540

Glu Glu Asn Cys Asp Ile Phe Gln Asn Leu Thr Lys Lys Gln Arg Gln
545                 550                 555                 560

Ser Leu Arg Lys Met Val Ile Asp Ile Val Leu Ala Thr Asp Met Ser
                565                 570                 575

Lys His Met Asn Leu Leu Ala Asp Leu Lys Thr Met Val Glu Thr Lys
            580                 585                 590

Lys Val Thr Ser Ser Gly Val Leu Leu Leu Asp Asn Tyr Ser Asp Arg
        595                 600                 605

Ile Gln Val Leu Gln Asn Met Val His Cys Ala Asp Leu Ser Asn Pro
    610                 615                 620

Thr Lys Pro Leu Gln Leu Tyr Arg Gln Trp Thr Asp Arg Ile Met Glu
625                 630                 635                 640

Glu Phe Phe Arg Gln Gly Asp Arg Glu Arg Glu Arg Gly Met Glu Ile
                645                 650                 655

Ser Pro Met Cys Asp Lys His Asn Ala Ser Val Glu Lys Ser Gln Val
            660                 665                 670

Gly Phe Ile Asp Tyr Ile Val His Pro Leu Trp Glu Thr Trp Ala Asp
        675                 680                 685

Leu Val His Pro Asp Ala Gln Asp Ile Leu Asp Thr Leu Glu Asp Asn
    690                 695                 700

Arg Glu Trp Tyr Gln Ser Thr Ile Pro Gln Ser Pro Ser Pro Ala Pro
705                 710                 715                 720

Asp Asp Pro Glu Glu Gly Arg Gln Gly Gln Thr Glu Lys Phe Gln Phe
                725                 730                 735

Glu Leu Thr Leu Glu Glu Asp Gly Glu Ser Asp Thr Glu Lys Asp Ser
            740                 745                 750

Gly Ser Gln Val Glu Glu Asp Thr Ser Cys Ser Asp Ser Lys Thr Leu
        755                 760                 765

Cys Thr Gln Asp Ser Glu Ser Thr Glu Ile Pro Leu Asp Glu Gln Val
    770                 775                 780

Glu Glu Glu Ala Val Gly Glu Glu Glu Glu Ser Gln Pro Glu Ala Cys
785                 790                 795                 800

Val Ile Asp Asp Arg Ser Pro Asp Thr
                805

<210>  101
<211>  699
<212>  PRT
<213>  Homo sapiens

<400>  101

Met Ser Leu Pro Ser Ser Cys Glu Tyr Leu Thr Leu Gly Lys Val Ala
1               5                   10                  15

Arg Phe Arg Ser Ala Tyr Val His Gly Ser Pro Tyr Ala Ile Asn Met
            20                  25                  30

Pro Ile Asp Ile Lys Pro Gln Arg Arg Arg Phe Asp Val Asp Asn Gly
        35                  40                  45

Thr Ser Ala Gly Arg Ser Pro Leu Asp Pro Met Thr Ser Pro Gly Ser
    50                  55                  60

Gly Leu Ile Leu Gln Ala Asn Phe Val His Ser Gln Arg Arg Glu Ser
65                  70                  75                  80

Phe Leu Tyr Arg Ser Asp Ser Asp Tyr Asp Leu Ser Pro Lys Ser Met
                85                  90                  95

Ser Arg Asn Ser Ser Ile Ala Ser Asp Ile His Gly Asp Asp Leu Ile
            100                 105                 110

Val Thr Pro Phe Ala Gln Val Leu Ala Ser Leu Arg Thr Val Arg Asn
        115                 120                 125

Asn Phe Ala Ala Leu Thr Asn Leu Gln Asp Arg Ala Pro Ser Lys Arg
    130                 135                 140

Ser Pro Met Cys Asn Gln Pro Ser Ile Asn Lys Ala Thr Ile Thr Glu
145                 150                 155                 160

Glu Ala Tyr Gln Lys Leu Ala Ser Glu Thr Leu Glu Glu Leu Asp Trp
                165                 170                 175

Cys Leu Asp Gln Leu Glu Thr Leu Gln Thr Arg His Ser Val Ser Glu
            180                 185                 190

Met Ala Ser Asn Lys Phe Lys Arg Met Leu Asn Arg Glu Leu Thr His
195 200 205

Leu Ser Glu Met Ser Arg Ser Gly Asn Gln Val Ser Glu Phe Ile Ser
210 215 220

Asn Thr Phe Leu Asp Lys Gln His Glu Val Glu Ile Pro Ser Pro Thr
225 230 235 240

Gln Lys Glu Lys Glu Lys Lys Lys Arg Pro Met Ser Gln Ile Ser Gly
245 250 255

Val Lys Lys Leu Met His Ser Ser Ser Leu Thr Asn Ser Ser Ile Pro
260 265 270

Arg Phe Gly Val Lys Thr Glu Gln Glu Asp Val Leu Ala Lys Glu Leu
275 280 285

Glu Asp Val Asn Lys Trp Gly Leu His Val Phe Arg Ile Ala Glu Leu
290 295 300

Ser Gly Asn Arg Pro Leu Thr Val Ile Met His Thr Ile Phe Gln Glu
305 310 315 320

Arg Asp Leu Leu Lys Thr Phe Lys Ile Pro Val Asp Thr Leu Ile Thr
325 330 335

Tyr Leu Met Thr Leu Glu Asp His Tyr His Ala Asp Val Ala Tyr His
340 345 350

Asn Asn Ile His Ala Ala Asp Val Val Gln Ser Thr His Val Leu Leu
355 360 365

Ser Thr Pro Ala Leu Glu Ala Val Phe Thr Asp Leu Glu Ile Leu Ala
370 375 380

Ala Ile Phe Ala Ser Ala Ile His Asp Val Asp His Pro Gly Val Ser
385 390 395 400

Asn Gln Phe Leu Ile Asn Thr Asn Ser Glu Leu Ala Leu Met Tyr Asn
405 410 415

Asp Ser Ser Val Leu Glu Asn His His Leu Ala Val Gly Phe Lys Leu
420 425 430

Leu Gln Glu Glu Asn Cys Asp Ile Phe Gln Asn Leu Thr Lys Lys Gln

435 440 445

Arg Gln Ser Leu Arg Lys Met Val Ile Asp Ile Val Leu Ala Thr Asp
450 455 460

Met Ser Lys His Met Asn Leu Leu Ala Asp Leu Lys Thr Met Val Glu
465 470 475 480

Thr Lys Lys Val Thr Ser Ser Gly Val Leu Leu Leu Asp Asn Tyr Ser
485 490 495

Asp Arg Ile Gln Val Leu Gln Asn Met Val His Cys Ala Asp Leu Ser
500 505 510

Asn Pro Thr Lys Pro Leu Gln Leu Tyr Arg Gln Trp Thr Asp Arg Ile
515 520 525

Met Glu Glu Phe Phe Arg Gln Gly Asp Arg Glu Arg Glu Arg Gly Met
530 535 540

Glu Ile Ser Pro Met Cys Asp Lys His Asn Ala Ser Val Glu Lys Ser
545 550 555 560

Gln Val Gly Phe Ile Asp Tyr Ile Val His Pro Leu Trp Glu Thr Trp
565 570 575

Ala Asp Leu Val His Pro Asp Ala Gln Asp Ile Leu Asp Thr Leu Glu
580 585 590

Asp Asn Arg Glu Trp Tyr Gln Ser Thr Ile Pro Gln Ser Pro Ser Pro
595 600 605

Ala Pro Asp Asp Pro Glu Glu Gly Arg Gln Gly Gln Thr Glu Lys Phe
610 615 620

Gln Phe Glu Leu Thr Leu Glu Glu Asp Gly Glu Ser Asp Thr Glu Lys
625 630 635 640

Asp Ser Gly Ser Gln Val Glu Glu Asp Thr Ser Cys Ser Asp Ser Lys
645 650 655

Thr Leu Cys Thr Gln Asp Ser Glu Ser Thr Glu Ile Pro Leu Asp Glu
660 665 670

Gln Val Glu Glu Glu Ala Val Gly Glu Glu Glu Glu Ser Gln Pro Glu
675 680 685

Ala Cys Val Ile Asp Asp Arg Ser Pro Asp Thr
690 695

<210> 102
<211> 745
<212> PRT
<213> Homo sapiens

<400> 102

Met Ala Gln Gln Thr Ser Pro Asp Thr Leu Thr Val Pro Glu Val Asp
1 5 10 15

Asn Pro His Cys Pro Asn Pro Trp Leu Asn Glu Asp Leu Val Lys Ser
20 25 30

Leu Arg Glu Asn Leu Leu Gln His Glu Lys Ser Lys Thr Ala Arg Lys
35 40 45

Ser Val Ser Pro Lys Leu Ser Pro Val Ile Ser Pro Arg Asn Ser Pro
50 55 60

Arg Leu Leu Arg Arg Met Leu Leu Ser Ser Asn Ile Pro Lys Gln Arg
65 70 75 80

Arg Phe Thr Val Ala His Thr Cys Phe Asp Val Asp Asn Gly Thr Ser
85 90 95

Ala Gly Arg Ser Pro Leu Asp Pro Met Thr Ser Pro Gly Ser Gly Leu
100 105 110

Ile Leu Gln Ala Asn Phe Val His Ser Gln Arg Arg Glu Ser Phe Leu
115 120 125

Tyr Arg Ser Asp Ser Asp Tyr Asp Leu Ser Pro Lys Ser Met Ser Arg
130 135 140

Asn Ser Ser Ile Ala Ser Asp Ile His Gly Asp Asp Leu Ile Val Thr
145 150 155 160

Pro Phe Ala Gln Val Leu Ala Ser Leu Arg Thr Val Arg Asn Asn Phe
165 170 175

Ala Ala Leu Thr Asn Leu Gln Asp Arg Ala Pro Ser Lys Arg Ser Pro
180 185 190

Met Cys Asn Gln Pro Ser Ile Asn Lys Ala Thr Ile Thr Glu Glu Ala
195 200 205

Tyr Gln Lys Leu Ala Ser Glu Thr Leu Glu Glu Leu Asp Trp Cys Leu
    210                 215                 220

Asp Gln Leu Glu Thr Leu Gln Thr Arg His Ser Val Ser Glu Met Ala
225                 230                 235                 240

Ser Asn Lys Phe Lys Arg Met Leu Asn Arg Glu Leu Thr His Leu Ser
                245                 250                 255

Glu Met Ser Arg Ser Gly Asn Gln Val Ser Glu Phe Ile Ser Asn Thr
            260                 265                 270

Phe Leu Asp Lys Gln His Glu Val Glu Ile Pro Ser Pro Thr Gln Lys
        275                 280                 285

Glu Lys Glu Lys Lys Lys Arg Pro Met Ser Gln Ile Ser Gly Val Lys
    290                 295                 300

Lys Leu Met His Ser Ser Ser Leu Thr Asn Ser Ser Ile Pro Arg Phe
305                 310                 315                 320

Gly Val Lys Thr Glu Gln Glu Asp Val Leu Ala Lys Glu Leu Glu Asp
                325                 330                 335

Val Asn Lys Trp Gly Leu His Val Phe Arg Ile Ala Glu Leu Ser Gly
            340                 345                 350

Asn Arg Pro Leu Thr Val Ile Met His Thr Ile Phe Gln Glu Arg Asp
        355                 360                 365

Leu Leu Lys Thr Phe Lys Ile Pro Val Asp Thr Leu Ile Thr Tyr Leu
    370                 375                 380

Met Thr Leu Glu Asp His Tyr His Ala Asp Val Ala Tyr His Asn Asn
385                 390                 395                 400

Ile His Ala Ala Asp Val Val Gln Ser Thr His Val Leu Leu Ser Thr
                405                 410                 415

Pro Ala Leu Glu Ala Val Phe Thr Asp Leu Glu Ile Leu Ala Ala Ile
            420                 425                 430

Phe Ala Ser Ala Ile His Asp Val Asp His Pro Gly Val Ser Asn Gln
        435                 440                 445

Phe Leu Ile Asn Thr Asn Ser Glu Leu Ala Leu Met Tyr Asn Asp Ser
    450                 455                 460

Ser Val Leu Glu Asn His His Leu Ala Val Gly Phe Lys Leu Leu Gln
465 470 475 480

Glu Glu Asn Cys Asp Ile Phe Gln Asn Leu Thr Lys Lys Gln Arg Gln
485 490 495

Ser Leu Arg Lys Met Val Ile Asp Ile Val Leu Ala Thr Asp Met Ser
500 505 510

Lys His Met Asn Leu Leu Ala Asp Leu Lys Thr Met Val Glu Thr Lys
515 520 525

Lys Val Thr Ser Ser Gly Val Leu Leu Leu Asp Asn Tyr Ser Asp Arg
530 535 540

Ile Gln Val Leu Gln Asn Met Val His Cys Ala Asp Leu Ser Asn Pro
545 550 555 560

Thr Lys Pro Leu Gln Leu Tyr Arg Gln Trp Thr Asp Arg Ile Met Glu
565 570 575

Glu Phe Phe Arg Gln Gly Asp Arg Glu Arg Glu Arg Gly Met Glu Ile
580 585 590

Ser Pro Met Cys Asp Lys His Asn Ala Ser Val Glu Lys Ser Gln Val
595 600 605

Gly Phe Ile Asp Tyr Ile Val His Pro Leu Trp Glu Thr Trp Ala Asp
610 615 620

Leu Val His Pro Asp Ala Gln Asp Ile Leu Asp Thr Leu Glu Asp Asn
625 630 635 640

Arg Glu Trp Tyr Gln Ser Thr Ile Pro Gln Ser Pro Ser Pro Ala Pro
645 650 655

Asp Asp Pro Glu Glu Gly Arg Gln Gly Gln Thr Glu Lys Phe Gln Phe
660 665 670

Glu Leu Thr Leu Glu Glu Asp Gly Glu Ser Asp Thr Glu Lys Asp Ser
675 680 685

Gly Ser Gln Val Glu Glu Asp Thr Ser Cys Ser Asp Ser Lys Thr Leu
690 695 700

Cys Thr Gln Asp Ser Glu Ser Thr Glu Ile Pro Leu Asp Glu Gln Val
705 710 715 720

Glu Glu Glu Ala Val Gly Glu Glu Glu Glu Ser Gln Pro Glu Ala Cys
725 730 735

Val Ile Asp Asp Arg Ser Pro Asp Thr
740 745

<210> 103
<211> 673
<212> PRT
<213> Homo sapiens

<400> 103

Met Met His Val Asn Asn Phe Pro Phe Arg Arg His Ser Trp Ile Cys
1 5 10 15

Phe Asp Val Asp Asn Gly Thr Ser Ala Gly Arg Ser Pro Leu Asp Pro
20 25 30

Met Thr Ser Pro Gly Ser Gly Leu Ile Leu Gln Ala Asn Phe Val His
35 40 45

Ser Gln Arg Arg Glu Ser Phe Leu Tyr Arg Ser Asp Ser Asp Tyr Asp
50 55 60

Leu Ser Pro Lys Ser Met Ser Arg Asn Ser Ser Ile Ala Ser Asp Ile
65 70 75 80

His Gly Asp Asp Leu Ile Val Thr Pro Phe Ala Gln Val Leu Ala Ser
85 90 95

Leu Arg Thr Val Arg Asn Asn Phe Ala Ala Leu Thr Asn Leu Gln Asp
100 105 110

Arg Ala Pro Ser Lys Arg Ser Pro Met Cys Asn Gln Pro Ser Ile Asn
115 120 125

Lys Ala Thr Ile Thr Glu Glu Ala Tyr Gln Lys Leu Ala Ser Glu Thr
130 135 140

Leu Glu Glu Leu Asp Trp Cys Leu Asp Gln Leu Glu Thr Leu Gln Thr
145 150 155 160

Arg His Ser Val Ser Glu Met Ala Ser Asn Lys Phe Lys Arg Met Leu
165 170 175

Asn Arg Glu Leu Thr His Leu Ser Glu Met Ser Arg Ser Gly Asn Gln
180 185 190

```
Val Ser Glu Phe Ile Ser Asn Thr Phe Leu Asp Lys Gln His Glu Val
        195                 200                 205

Glu Ile Pro Ser Pro Thr Gln Lys Glu Lys Glu Lys Lys Lys Arg Pro
    210                 215                 220

Met Ser Gln Ile Ser Gly Val Lys Lys Leu Met His Ser Ser Ser Leu
225                 230                 235                 240

Thr Asn Ser Ser Ile Pro Arg Phe Gly Val Lys Thr Glu Gln Glu Asp
                245                 250                 255

Val Leu Ala Lys Glu Leu Glu Asp Val Asn Lys Trp Gly Leu His Val
            260                 265                 270

Phe Arg Ile Ala Glu Leu Ser Gly Asn Arg Pro Leu Thr Val Ile Met
        275                 280                 285

His Thr Ile Phe Gln Glu Arg Asp Leu Leu Lys Thr Phe Lys Ile Pro
    290                 295                 300

Val Asp Thr Leu Ile Thr Tyr Leu Met Thr Leu Glu Asp His Tyr His
305                 310                 315                 320

Ala Asp Val Ala Tyr His Asn Asn Ile His Ala Ala Asp Val Val Gln
                325                 330                 335

Ser Thr His Val Leu Leu Ser Thr Pro Ala Leu Glu Ala Val Phe Thr
            340                 345                 350

Asp Leu Glu Ile Leu Ala Ala Ile Phe Ala Ser Ala Ile His Asp Val
        355                 360                 365

Asp His Pro Gly Val Ser Asn Gln Phe Leu Ile Asn Thr Asn Ser Glu
    370                 375                 380

Leu Ala Leu Met Tyr Asn Asp Ser Ser Val Leu Glu Asn His His Leu
385                 390                 395                 400

Ala Val Gly Phe Lys Leu Leu Gln Glu Glu Asn Cys Asp Ile Phe Gln
                405                 410                 415

Asn Leu Thr Lys Lys Gln Arg Gln Ser Leu Arg Lys Met Val Ile Asp
            420                 425                 430

Ile Val Leu Ala Thr Asp Met Ser Lys His Met Asn Leu Leu Ala Asp
```

435 440 445

Leu Lys Thr Met Val Glu Thr Lys Lys Val Thr Ser Ser Gly Val Leu
450 455 460

Leu Leu Asp Asn Tyr Ser Asp Arg Ile Gln Val Leu Gln Asn Met Val
465 470 475 480

His Cys Ala Asp Leu Ser Asn Pro Thr Lys Pro Leu Gln Leu Tyr Arg
485 490 495

Gln Trp Thr Asp Arg Ile Met Glu Glu Phe Phe Arg Gln Gly Asp Arg
500 505 510

Glu Arg Glu Arg Gly Met Glu Ile Ser Pro Met Cys Asp Lys His Asn
515 520 525

Ala Ser Val Glu Lys Ser Gln Val Gly Phe Ile Asp Tyr Ile Val His
530 535 540

Pro Leu Trp Glu Thr Trp Ala Asp Leu Val His Pro Asp Ala Gln Asp
545 550 555 560

Ile Leu Asp Thr Leu Glu Asp Asn Arg Glu Trp Tyr Gln Ser Thr Ile
565 570 575

Pro Gln Ser Pro Ser Pro Ala Pro Asp Asp Pro Glu Glu Gly Arg Gln
580 585 590

Gly Gln Thr Glu Lys Phe Gln Phe Glu Leu Thr Leu Glu Glu Asp Gly
595 600 605

Glu Ser Asp Thr Glu Lys Asp Ser Gly Ser Gln Val Glu Glu Asp Thr
610 615 620

Ser Cys Ser Asp Ser Lys Thr Leu Cys Thr Gln Asp Ser Glu Ser Thr
625 630 635 640

Glu Ile Pro Leu Asp Glu Gln Val Glu Glu Glu Ala Val Gly Glu Glu
645 650 655

Glu Glu Ser Gln Pro Glu Ala Cys Val Ile Asp Asp Arg Ser Pro Asp
660 665 670

Thr

<210> 104
<211> 507
<212> PRT
<213> Homo sapiens

<400> 104

Met Ala Ser Asn Lys Phe Lys Arg Met Leu Asn Arg Glu Leu Thr His
1               5                   10                  15

Leu Ser Glu Met Ser Arg Ser Gly Asn Gln Val Ser Glu Phe Ile Ser
            20                  25                  30

Asn Thr Phe Leu Asp Lys Gln His Glu Val Glu Ile Pro Ser Pro Thr
        35                  40                  45

Gln Lys Glu Lys Glu Lys Lys Lys Arg Pro Met Ser Gln Ile Ser Gly
    50                  55                  60

Val Lys Lys Leu Met His Ser Ser Ser Leu Thr Asn Ser Ser Ile Pro
65                  70                  75                  80

Arg Phe Gly Val Lys Thr Glu Gln Glu Asp Val Leu Ala Lys Glu Leu
                85                  90                  95

Glu Asp Val Asn Lys Trp Gly Leu His Val Phe Arg Ile Ala Glu Leu
            100                 105                 110

Ser Gly Asn Arg Pro Leu Thr Val Ile Met His Thr Ile Phe Gln Glu
        115                 120                 125

Arg Asp Leu Leu Lys Thr Phe Lys Ile Pro Val Asp Thr Leu Ile Thr
    130                 135                 140

Tyr Leu Met Thr Leu Glu Asp His Tyr His Ala Asp Val Ala Tyr His
145                 150                 155                 160

Asn Asn Ile His Ala Ala Asp Val Val Gln Ser Thr His Val Leu Leu
                165                 170                 175

Ser Thr Pro Ala Leu Glu Ala Val Phe Thr Asp Leu Glu Ile Leu Ala
            180                 185                 190

Ala Ile Phe Ala Ser Ala Ile His Asp Val Asp His Pro Gly Val Ser
        195                 200                 205

Asn Gln Phe Leu Ile Asn Thr Asn Ser Glu Leu Ala Leu Met Tyr Asn
    210                 215                 220

Asp Ser Ser Val Leu Glu Asn His His Leu Ala Val Gly Phe Lys Leu
225 230 235 240

Leu Gln Glu Glu Asn Cys Asp Ile Phe Gln Asn Leu Thr Lys Lys Gln
245 250 255

Arg Gln Ser Leu Arg Lys Met Val Ile Asp Ile Val Leu Ala Thr Asp
260 265 270

Met Ser Lys His Met Asn Leu Leu Ala Asp Leu Lys Thr Met Val Glu
275 280 285

Thr Lys Lys Val Thr Ser Ser Gly Val Leu Leu Leu Asp Asn Tyr Ser
290 295 300

Asp Arg Ile Gln Val Leu Gln Asn Met Val His Cys Ala Asp Leu Ser
305 310 315 320

Asn Pro Thr Lys Pro Leu Gln Leu Tyr Arg Gln Trp Thr Asp Arg Ile
325 330 335

Met Glu Glu Phe Phe Arg Gln Gly Asp Arg Glu Arg Glu Arg Gly Met
340 345 350

Glu Ile Ser Pro Met Cys Asp Lys His Asn Ala Ser Val Glu Lys Ser
355 360 365

Gln Val Gly Phe Ile Asp Tyr Ile Val His Pro Leu Trp Glu Thr Trp
370 375 380

Ala Asp Leu Val His Pro Asp Ala Gln Asp Ile Leu Asp Thr Leu Glu
385 390 395 400

Asp Asn Arg Glu Trp Tyr Gln Ser Thr Ile Pro Gln Ser Pro Ser Pro
405 410 415

Ala Pro Asp Asp Pro Glu Glu Gly Arg Gln Gly Gln Thr Glu Lys Phe
420 425 430

Gln Phe Glu Leu Thr Leu Glu Glu Asp Gly Glu Ser Asp Thr Glu Lys
435 440 445

Asp Ser Gly Ser Gln Val Glu Glu Asp Thr Ser Cys Ser Asp Ser Lys
450 455 460

Thr Leu Cys Thr Gln Asp Ser Glu Ser Thr Glu Ile Pro Leu Asp Glu
465 470 475 480

Gln Val Glu Glu Glu Ala Val Gly Glu Glu Glu Glu Ser Gln Pro Glu
485 490 495

Ala Cys Val Ile Asp Asp Arg Ser Pro Asp Thr
500 505

<210> 105
<211> 679
<212> PRT
<213> Homo sapiens

<400> 105

Met Ser Ile Ile Met Lys Pro Arg Ser Arg Ser Thr Ser Ser Leu Arg
1 5 10 15

Thr Ala Glu Ala Val Cys Phe Asp Val Asp Asn Gly Thr Ser Ala Gly
20 25 30

Arg Ser Pro Leu Asp Pro Met Thr Ser Pro Gly Ser Gly Leu Ile Leu
35 40 45

Gln Ala Asn Phe Val His Ser Gln Arg Arg Glu Ser Phe Leu Tyr Arg
50 55 60

Ser Asp Ser Asp Tyr Asp Leu Ser Pro Lys Ser Met Ser Arg Asn Ser
65 70 75 80

Ser Ile Ala Ser Asp Ile His Gly Asp Asp Leu Ile Val Thr Pro Phe
85 90 95

Ala Gln Val Leu Ala Ser Leu Arg Thr Val Arg Asn Asn Phe Ala Ala
100 105 110

Leu Thr Asn Leu Gln Asp Arg Ala Pro Ser Lys Arg Ser Pro Met Cys
115 120 125

Asn Gln Pro Ser Ile Asn Lys Ala Thr Ile Thr Glu Glu Ala Tyr Gln
130 135 140

Lys Leu Ala Ser Glu Thr Leu Glu Glu Leu Asp Trp Cys Leu Asp Gln
145 150 155 160

Leu Glu Thr Leu Gln Thr Arg His Ser Val Ser Glu Met Ala Ser Asn
165 170 175

Lys Phe Lys Arg Met Leu Asn Arg Glu Leu Thr His Leu Ser Glu Met
180 185 190

```
Ser Arg Ser Gly Asn Gln Val Ser Glu Phe Ile Ser Asn Thr Phe Leu
        195                 200                 205

Asp Lys Gln His Glu Val Glu Ile Pro Ser Pro Thr Gln Lys Glu Lys
    210                 215                 220

Glu Lys Lys Lys Arg Pro Met Ser Gln Ile Ser Gly Val Lys Lys Leu
225                 230                 235                 240

Met His Ser Ser Ser Leu Thr Asn Ser Ser Ile Pro Arg Phe Gly Val
                245                 250                 255

Lys Thr Glu Gln Glu Asp Val Leu Ala Lys Glu Leu Glu Asp Val Asn
            260                 265                 270

Lys Trp Gly Leu His Val Phe Arg Ile Ala Glu Leu Ser Gly Asn Arg
        275                 280                 285

Pro Leu Thr Val Ile Met His Thr Ile Phe Gln Glu Arg Asp Leu Leu
    290                 295                 300

Lys Thr Phe Lys Ile Pro Val Asp Thr Leu Ile Thr Tyr Leu Met Thr
305                 310                 315                 320

Leu Glu Asp His Tyr His Ala Asp Val Ala Tyr His Asn Asn Ile His
                325                 330                 335

Ala Ala Asp Val Val Gln Ser Thr His Val Leu Leu Ser Thr Pro Ala
            340                 345                 350

Leu Glu Ala Val Phe Thr Asp Leu Glu Ile Leu Ala Ala Ile Phe Ala
        355                 360                 365

Ser Ala Ile His Asp Val Asp His Pro Gly Val Ser Asn Gln Phe Leu
    370                 375                 380

Ile Asn Thr Asn Ser Glu Leu Ala Leu Met Tyr Asn Asp Ser Ser Val
385                 390                 395                 400

Leu Glu Asn His His Leu Ala Val Gly Phe Lys Leu Leu Gln Glu Glu
                405                 410                 415

Asn Cys Asp Ile Phe Gln Asn Leu Thr Lys Lys Gln Arg Gln Ser Leu
            420                 425                 430

Arg Lys Met Val Ile Asp Ile Val Leu Ala Thr Asp Met Ser Lys His
        435                 440                 445
```

Met Asn Leu Leu Ala Asp Leu Lys Thr Met Val Glu Thr Lys Lys Val
    450                 455                 460

Thr Ser Ser Gly Val Leu Leu Leu Asp Asn Tyr Ser Asp Arg Ile Gln
465                 470                 475                 480

Val Leu Gln Asn Met Val His Cys Ala Asp Leu Ser Asn Pro Thr Lys
                485                 490                 495

Pro Leu Gln Leu Tyr Arg Gln Trp Thr Asp Arg Ile Met Glu Glu Phe
            500                 505                 510

Phe Arg Gln Gly Asp Arg Glu Arg Glu Arg Gly Met Glu Ile Ser Pro
        515                 520                 525

Met Cys Asp Lys His Asn Ala Ser Val Glu Lys Ser Gln Val Gly Phe
    530                 535                 540

Ile Asp Tyr Ile Val His Pro Leu Trp Glu Thr Trp Ala Asp Leu Val
545                 550                 555                 560

His Pro Asp Ala Gln Asp Ile Leu Asp Thr Leu Glu Asp Asn Arg Glu
                565                 570                 575

Trp Tyr Gln Ser Thr Ile Pro Gln Ser Pro Ser Pro Ala Pro Asp Asp
            580                 585                 590

Pro Glu Glu Gly Arg Gln Gly Gln Thr Glu Lys Phe Gln Phe Glu Leu
        595                 600                 605

Thr Leu Glu Glu Asp Gly Glu Ser Asp Thr Glu Lys Asp Ser Gly Ser
    610                 615                 620

Gln Val Glu Glu Asp Thr Ser Cys Ser Asp Ser Lys Thr Leu Cys Thr
625                 630                 635                 640

Gln Asp Ser Glu Ser Thr Glu Ile Pro Leu Asp Glu Gln Val Glu Glu
                645                 650                 655

Glu Ala Val Gly Glu Glu Glu Glu Ser Gln Pro Glu Ala Cys Val Ile
            660                 665                 670

Asp Asp Arg Ser Pro Asp Thr
        675

<210>  106

<211> 518
<212> PRT
<213> Homo sapiens

<400> 106

Met Pro Glu Ala Asn Tyr Leu Leu Ser Val Ser Trp Gly Tyr Ile Lys
1 5 10 15

Phe Lys Arg Met Leu Asn Arg Glu Leu Thr His Leu Ser Glu Met Ser
20 25 30

Arg Ser Gly Asn Gln Val Ser Glu Phe Ile Ser Asn Thr Phe Leu Asp
35 40 45

Lys Gln His Glu Val Glu Ile Pro Ser Pro Thr Gln Lys Glu Lys Glu
50 55 60

Lys Lys Lys Arg Pro Met Ser Gln Ile Ser Gly Val Lys Lys Leu Met
65 70 75 80

His Ser Ser Ser Leu Thr Asn Ser Ser Ile Pro Arg Phe Gly Val Lys
85 90 95

Thr Glu Gln Glu Asp Val Leu Ala Lys Glu Leu Glu Asp Val Asn Lys
100 105 110

Trp Gly Leu His Val Phe Arg Ile Ala Glu Leu Ser Gly Asn Arg Pro
115 120 125

Leu Thr Val Ile Met His Thr Ile Phe Gln Glu Arg Asp Leu Leu Lys
130 135 140

Thr Phe Lys Ile Pro Val Asp Thr Leu Ile Thr Tyr Leu Met Thr Leu
145 150 155 160

Glu Asp His Tyr His Ala Asp Val Ala Tyr His Asn Asn Ile His Ala
165 170 175

Ala Asp Val Val Gln Ser Thr His Val Leu Leu Ser Thr Pro Ala Leu
180 185 190

Glu Ala Val Phe Thr Asp Leu Glu Ile Leu Ala Ala Ile Phe Ala Ser
195 200 205

Ala Ile His Asp Val Asp His Pro Gly Val Ser Asn Gln Phe Leu Ile
210 215 220

Asn Thr Asn Ser Glu Leu Ala Leu Met Tyr Asn Asp Ser Ser Val Leu

```
225                 230                 235                 240

Glu Asn His His Leu Ala Val Gly Phe Lys Leu Leu Gln Glu Glu Asn
                245                 250                 255

Cys Asp Ile Phe Gln Asn Leu Thr Lys Lys Gln Arg Gln Ser Leu Arg
            260                 265                 270

Lys Met Val Ile Asp Ile Val Leu Ala Thr Asp Met Ser Lys His Met
        275                 280                 285

Asn Leu Leu Ala Asp Leu Lys Thr Met Val Glu Thr Lys Lys Val Thr
    290                 295                 300

Ser Ser Gly Val Leu Leu Leu Asp Asn Tyr Ser Asp Arg Ile Gln Val
305                 310                 315                 320

Leu Gln Asn Met Val His Cys Ala Asp Leu Ser Asn Pro Thr Lys Pro
                325                 330                 335

Leu Gln Leu Tyr Arg Gln Trp Thr Asp Arg Ile Met Glu Glu Phe Phe
            340                 345                 350

Arg Gln Gly Asp Arg Glu Arg Glu Arg Gly Met Glu Ile Ser Pro Met
        355                 360                 365

Cys Asp Lys His Asn Ala Ser Val Glu Lys Ser Gln Val Gly Phe Ile
    370                 375                 380

Asp Tyr Ile Val His Pro Leu Trp Glu Thr Trp Ala Asp Leu Val His
385                 390                 395                 400

Pro Asp Ala Gln Asp Ile Leu Asp Thr Leu Glu Asp Asn Arg Glu Trp
                405                 410                 415

Tyr Gln Ser Thr Ile Pro Gln Ser Pro Ser Pro Ala Pro Asp Asp Pro
            420                 425                 430

Glu Glu Gly Arg Gln Gly Gln Thr Glu Lys Phe Gln Phe Glu Leu Thr
        435                 440                 445

Leu Glu Glu Asp Gly Glu Ser Asp Thr Glu Lys Asp Ser Gly Ser Gln
    450                 455                 460

Val Glu Glu Asp Thr Ser Cys Ser Asp Ser Lys Thr Leu Cys Thr Gln
465                 470                 475                 480
```

Asp Ser Glu Ser Thr Glu Ile Pro Leu Asp Glu Gln Val Glu Glu Glu
485 490 495

Ala Val Gly Glu Glu Glu Glu Ser Gln Pro Glu Ala Cys Val Ile Asp
500 505 510

Asp Arg Ser Pro Asp Thr
515

<210> 107
<211> 748
<212> PRT
<213> Homo sapiens

<400> 107

Met Lys Arg Asn Thr Cys Asp Leu Leu Ser Arg Ser Lys Ser Ala Ser
1 5 10 15

Glu Glu Thr Leu His Ser Ser Asn Glu Glu Glu Asp Pro Phe Arg Gly
20 25 30

Met Glu Pro Tyr Leu Val Arg Arg Leu Ser Cys Arg Asn Ile Gln Leu
35 40 45

Pro Pro Leu Ala Phe Arg Gln Leu Glu Gln Ala Asp Leu Lys Ser Glu
50 55 60

Ser Glu Asn Ile Gln Arg Pro Thr Ser Leu Pro Leu Lys Ile Leu Pro
65 70 75 80

Leu Ile Ala Ile Thr Ser Ala Glu Ser Ser Gly Phe Asp Val Asp Asn
85 90 95

Gly Thr Ser Ala Gly Arg Ser Pro Leu Asp Pro Met Thr Ser Pro Gly
100 105 110

Ser Gly Leu Ile Leu Gln Ala Asn Phe Val His Ser Gln Arg Arg Glu
115 120 125

Ser Phe Leu Tyr Arg Ser Asp Ser Asp Tyr Asp Leu Ser Pro Lys Ser
130 135 140

Met Ser Arg Asn Ser Ser Ile Ala Ser Asp Ile His Gly Asp Asp Leu
145 150 155 160

Ile Val Thr Pro Phe Ala Gln Val Leu Ala Ser Leu Arg Thr Val Arg
165 170 175

Asn Asn Phe Ala Ala Leu Thr Asn Leu Gln Asp Arg Ala Pro Ser Lys
180 185 190

Arg Ser Pro Met Cys Asn Gln Pro Ser Ile Asn Lys Ala Thr Ile Thr
195 200 205

Glu Glu Ala Tyr Gln Lys Leu Ala Ser Glu Thr Leu Glu Glu Leu Asp
210 215 220

Trp Cys Leu Asp Gln Leu Glu Thr Leu Gln Thr Arg His Ser Val Ser
225 230 235 240

Glu Met Ala Ser Asn Lys Phe Lys Arg Met Leu Asn Arg Glu Leu Thr
245 250 255

His Leu Ser Glu Met Ser Arg Ser Gly Asn Gln Val Ser Glu Phe Ile
260 265 270

Ser Asn Thr Phe Leu Asp Lys Gln His Glu Val Glu Ile Pro Ser Pro
275 280 285

Thr Gln Lys Glu Lys Glu Lys Lys Lys Arg Pro Met Ser Gln Ile Ser
290 295 300

Gly Val Lys Lys Leu Met His Ser Ser Ser Leu Thr Asn Ser Ser Ile
305 310 315 320

Pro Arg Phe Gly Val Lys Thr Glu Gln Glu Asp Val Leu Ala Lys Glu
325 330 335

Leu Glu Asp Val Asn Lys Trp Gly Leu His Val Phe Arg Ile Ala Glu
340 345 350

Leu Ser Gly Asn Arg Pro Leu Thr Val Ile Met His Thr Ile Phe Gln
355 360 365

Glu Arg Asp Leu Leu Lys Thr Phe Lys Ile Pro Val Asp Thr Leu Ile
370 375 380

Thr Tyr Leu Met Thr Leu Glu Asp His Tyr His Ala Asp Val Ala Tyr
385 390 395 400

His Asn Asn Ile His Ala Ala Asp Val Val Gln Ser Thr His Val Leu
405 410 415

Leu Ser Thr Pro Ala Leu Glu Ala Val Phe Thr Asp Leu Glu Ile Leu
420 425 430

Ala Ala Ile Phe Ala Ser Ala Ile His Asp Val Asp His Pro Gly Val
435 440 445

Ser Asn Gln Phe Leu Ile Asn Thr Asn Ser Glu Leu Ala Leu Met Tyr
450 455 460

Asn Asp Ser Ser Val Leu Glu Asn His His Leu Ala Val Gly Phe Lys
465 470 475 480

Leu Leu Gln Glu Glu Asn Cys Asp Ile Phe Gln Asn Leu Thr Lys Lys
485 490 495

Gln Arg Gln Ser Leu Arg Lys Met Val Ile Asp Ile Val Leu Ala Thr
500 505 510

Asp Met Ser Lys His Met Asn Leu Leu Ala Asp Leu Lys Thr Met Val
515 520 525

Glu Thr Lys Lys Val Thr Ser Ser Gly Val Leu Leu Leu Asp Asn Tyr
530 535 540

Ser Asp Arg Ile Gln Val Leu Gln Asn Met Val His Cys Ala Asp Leu
545 550 555 560

Ser Asn Pro Thr Lys Pro Leu Gln Leu Tyr Arg Gln Trp Thr Asp Arg
565 570 575

Ile Met Glu Glu Phe Phe Arg Gln Gly Asp Arg Glu Arg Glu Arg Gly
580 585 590

Met Glu Ile Ser Pro Met Cys Asp Lys His Asn Ala Ser Val Glu Lys
595 600 605

Ser Gln Val Gly Phe Ile Asp Tyr Ile Val His Pro Leu Trp Glu Thr
610 615 620

Trp Ala Asp Leu Val His Pro Asp Ala Gln Asp Ile Leu Asp Thr Leu
625 630 635 640

Glu Asp Asn Arg Glu Trp Tyr Gln Ser Thr Ile Pro Gln Ser Pro Ser
645 650 655

Pro Ala Pro Asp Asp Pro Glu Glu Gly Arg Gln Gly Gln Thr Glu Lys
660 665 670

Phe Gln Phe Glu Leu Thr Leu Glu Glu Asp Gly Glu Ser Asp Thr Glu
675 680 685

Lys Asp Ser Gly Ser Gln Val Glu Glu Asp Thr Ser Cys Ser Asp Ser
    690                 695                 700

Lys Thr Leu Cys Thr Gln Asp Ser Glu Ser Thr Glu Ile Pro Leu Asp
705                 710                 715                 720

Glu Gln Val Glu Glu Glu Ala Val Gly Glu Glu Glu Glu Ser Gln Pro
                725                 730                 735

Glu Ala Cys Val Ile Asp Asp Arg Ser Pro Asp Thr
            740                 745

<210> 108
<211> 687
<212> PRT
<213> Homo sapiens

<400> 108

Met Ala Phe Val Trp Asp Pro Leu Gly Ala Thr Val Pro Gly Pro Ser
1               5                   10                  15

Thr Arg Ala Lys Ser Arg Leu Arg Phe Ser Lys Ser Tyr Ser Phe Asp
            20                  25                  30

Val Asp Asn Gly Thr Ser Ala Gly Arg Ser Pro Leu Asp Pro Met Thr
        35                  40                  45

Ser Pro Gly Ser Gly Leu Ile Leu Gln Ala Asn Phe Val His Ser Gln
    50                  55                  60

Arg Arg Glu Ser Phe Leu Tyr Arg Ser Asp Ser Asp Tyr Asp Leu Ser
65                  70                  75                  80

Pro Lys Ser Met Ser Arg Asn Ser Ser Ile Ala Ser Asp Ile His Gly
                85                  90                  95

Asp Asp Leu Ile Val Thr Pro Phe Ala Gln Val Leu Ala Ser Leu Arg
            100                 105                 110

Thr Val Arg Asn Asn Phe Ala Ala Leu Thr Asn Leu Gln Asp Arg Ala
        115                 120                 125

Pro Ser Lys Arg Ser Pro Met Cys Asn Gln Pro Ser Ile Asn Lys Ala
    130                 135                 140

Thr Ile Thr Glu Glu Ala Tyr Gln Lys Leu Ala Ser Glu Thr Leu Glu
145                 150                 155                 160

Glu Leu Asp Trp Cys Leu Asp Gln Leu Glu Thr Leu Gln Thr Arg His
                165                 170                 175

Ser Val Ser Glu Met Ala Ser Asn Lys Phe Lys Arg Met Leu Asn Arg
            180                 185                 190

Glu Leu Thr His Leu Ser Glu Met Ser Arg Ser Gly Asn Gln Val Ser
        195                 200                 205

Glu Phe Ile Ser Asn Thr Phe Leu Asp Lys Gln His Glu Val Glu Ile
    210                 215                 220

Pro Ser Pro Thr Gln Lys Glu Lys Glu Lys Lys Lys Arg Pro Met Ser
225                 230                 235                 240

Gln Ile Ser Gly Val Lys Lys Leu Met His Ser Ser Ser Leu Thr Asn
                245                 250                 255

Ser Ser Ile Pro Arg Phe Gly Val Lys Thr Glu Gln Glu Asp Val Leu
            260                 265                 270

Ala Lys Glu Leu Glu Asp Val Asn Lys Trp Gly Leu His Val Phe Arg
        275                 280                 285

Ile Ala Glu Leu Ser Gly Asn Arg Pro Leu Thr Val Ile Met His Thr
    290                 295                 300

Ile Phe Gln Glu Arg Asp Leu Leu Lys Thr Phe Lys Ile Pro Val Asp
305                 310                 315                 320

Thr Leu Ile Thr Tyr Leu Met Thr Leu Glu Asp His Tyr His Ala Asp
                325                 330                 335

Val Ala Tyr His Asn Asn Ile His Ala Ala Asp Val Val Gln Ser Thr
            340                 345                 350

His Val Leu Leu Ser Thr Pro Ala Leu Glu Ala Val Phe Thr Asp Leu
        355                 360                 365

Glu Ile Leu Ala Ala Ile Phe Ala Ser Ala Ile His Asp Val Asp His
    370                 375                 380

Pro Gly Val Ser Asn Gln Phe Leu Ile Asn Thr Asn Ser Glu Leu Ala
385                 390                 395                 400

Leu Met Tyr Asn Asp Ser Ser Val Leu Glu Asn His His Leu Ala Val

405 410 415

Gly Phe Lys Leu Leu Gln Glu Glu Asn Cys Asp Ile Phe Gln Asn Leu
420 425 430

Thr Lys Lys Gln Arg Gln Ser Leu Arg Lys Met Val Ile Asp Ile Val
435 440 445

Leu Ala Thr Asp Met Ser Lys His Met Asn Leu Leu Ala Asp Leu Lys
450 455 460

Thr Met Val Glu Thr Lys Lys Val Thr Ser Ser Gly Val Leu Leu Leu
465 470 475 480

Asp Asn Tyr Ser Asp Arg Ile Gln Val Leu Gln Asn Met Val His Cys
485 490 495

Ala Asp Leu Ser Asn Pro Thr Lys Pro Leu Gln Leu Tyr Arg Gln Trp
500 505 510

Thr Asp Arg Ile Met Glu Glu Phe Phe Arg Gln Gly Asp Arg Glu Arg
515 520 525

Glu Arg Gly Met Glu Ile Ser Pro Met Cys Asp Lys His Asn Ala Ser
530 535 540

Val Glu Lys Ser Gln Val Gly Phe Ile Asp Tyr Ile Val His Pro Leu
545 550 555 560

Trp Glu Thr Trp Ala Asp Leu Val His Pro Asp Ala Gln Asp Ile Leu
565 570 575

Asp Thr Leu Glu Asp Asn Arg Glu Trp Tyr Gln Ser Thr Ile Pro Gln
580 585 590

Ser Pro Ser Pro Ala Pro Asp Asp Pro Glu Glu Gly Arg Gln Gly Gln
595 600 605

Thr Glu Lys Phe Gln Phe Glu Leu Thr Leu Glu Glu Asp Gly Glu Ser
610 615 620

Asp Thr Glu Lys Asp Ser Gly Ser Gln Val Glu Glu Asp Thr Ser Cys
625 630 635 640

Ser Asp Ser Lys Thr Leu Cys Thr Gln Asp Ser Glu Ser Thr Glu Ile
645 650 655

```
Pro Leu Asp Glu Gln Val Glu Glu Glu Ala Val Gly Glu Glu Glu Glu
            660                 665                 670

Ser Gln Pro Glu Ala Cys Val Ile Asp Asp Arg Ser Pro Asp Thr
        675                 680                 685


<210>  109
<211>  2695
<212>  DNA
<213>  Homo sapiens

<400>  109
acagacagcg gcagagatct tgggctgagg ttcccgggcg ggcgggcgcg gagagacgcg      60

ggaagcaggg gctgggcggg ggtcgcggcg ccgcagctag cgcagccagc ccgagggccg     120

ccgccgccgc cgcccagcgc gctccggggc cgccggccgc agccagcacc cgccgcgccg     180

cagctccggg accggccccg gccgccgccg ccgcgatggg caacgccgcc gccgccaaga     240

agggcagcga gcaggagagc gtgaaagaat tcttagccaa agccaaagaa gattttctta     300

aaaaatggga aagtcccgct cagaacacag cccacttgga tcagtttgaa cgaatcaaga     360

ccctcggcac gggctccttc gggcgggtga tgctggtgaa acacaaggag accgggaacc     420

actatgccat gaagatcctc gacaaacaga aggtggtgaa actgaaacag atcgaacaca     480

ccctgaatga aaagcgcatc ctgcaagctg tcaactttcc gttcctcgtc aaactcgagt     540

tctccttcaa ggacaactca aacttataca tggtcatgga gtacgtgccc ggcggggaga     600

tgttctcaca cctacggcgg atcggaaggt tcagtgagcc ccatgcccgt ttctacgcgg     660

cccagatcgt cctgaccttt gagtatctgc actcgctgga tctcatctac agggacctga     720

agccggagaa tctgctcatt gaccagcagg gctacattca ggtgacagac ttcggtttcg     780

ccaagcgcgt gaagggccgc acttggacct tgtgcggcac ccctgagtac ctggcccctg     840

agattatcct gagcaaaggc tacaacaagg ccgtggactg gtgggccctg gggggttctta    900

tctatgaaat ggccgctggc tacccgccct tcttcgcaga ccagcccatc cagatctatg     960

agaagatcgt ctctgggaag gtgcgcttcc cttcccactt cagctctgac ttgaaggacc    1020

tgctgcggaa cctcctgcag gtagatctca ccaagcgctt tgggaacctc aagaatgggg    1080

tcaacgatat caagaaccac aagtggtttg ccacaactga ctggattgcc atctaccaga    1140

ggaaggtgga agctcccttc ataccaaagt ttaaaggccc tggggatacg agtaactttg    1200

acgactatga ggaagaagaa atccgggtct ccatcaatga gaagtgtggc aaggagtttt    1260

ctgagtttta ggggcatgcc tgtgccccca tgggttttct tttttctttt ttcttttttt    1320

tggtcggggg ggtgggaggg ttggattgaa cagccagagg gccccagagt tccttgcatc    1380

taatttcacc cccaccccac cctccagggt tagggggagc aggaagccca gataatcaga    1440

gggacagaaa caccagctgc tccccctcat ccccttcacc ctcctgcccc ctctcccact    1500
```

tttcccttcc tctttcccca cagcccccca gcccctcagc cctcccagcc cacttctgcc 1560

tgttttaaac gagtttctca actccagtca gaccaggtct tgctggtgta tccagggaca 1620

gggtatggaa agaggggctc acgcttaact ccagccccca cccacacccc catcccaccc 1680

aaccacaggc cccacttgct aagggcaaat gaacgaagcg ccaaccttcc tttcggagta 1740

atcctgcctg ggaaggagag atttttagtg acatgttcag tgggttgctt gctagaattt 1800

ttttaaaaaa acaacaattt aaaatcttat ttaagttcca ccagtgcctc cctccctcct 1860

tcctctactc ccacccctcc catgtccccc cattcctcaa atccatttta aagagaagca 1920

gactgacttt ggaaagggag gcgctggggt ttgaacctcc ccgctgctaa tctcccctgg 1980

gcccctcccc ggggaatcct ctctgccaat cctgcgaggg tctaggcccc tttaggaagc 2040

ctccgctctc tttttcccca acagacctgt cttcaccctt gggctttgaa agccagacaa 2100

agcagctgcc cctctccctg ccaaagagga gtcatccccc aaaaagacag agggggagcc 2160

ccaagcccaa gtctttcctc ccagcagcgt ttccccccaa ctccttaatt ttattctccg 2220

ctagatttta acgtccagcc ttccctcagc tgagtgggga gggcatccct gcaaaaggga 2280

acagaagagg ccaagtcccc ccaagccacg gcccggggtt caaggctaga gctgctgggg 2340

aggggctgcc tgttttactc acccaccagc ttccgcctcc cccatcctgg gcgcccctcc 2400

tccagcttag ctgtcagctg tccatcacct ctcccccact ttctcatttg tgcttttttc 2460

tctcgtaata gaaaagtggg gagccgctgg ggagccaccc cattcatccc cgtatttccc 2520

cctctcataa cttctcccca tcccaggagg agttctcagg cctggggtgg ggccccgggt 2580

gggtgcgggg gcgattcaac ctgtgtgctg cgaaggacga gacttcctct tgaacagtgt 2640

gctgttgtaa acatatttga aaactattac caataaagtt ttgtttaaaa aaaaa 2695

<210> 110
<211> 2492
<212> DNA
<213> Homo sapiens

<400> 110
accgtagtgc cggtgccctg agaacaggac tgagtgatgg cttccaactc cagcgatgtg 60

aaagaattct tagccaaagc caaagaagat ttcttaaaaa aatgggaaag tcccgctcag 120

aacacagccc acttggatca gtttgaacga atcaagaccc tcggcacggg ctccttcggg 180

cgggtgatgc tggtgaaaca caaggagacc gggaaccact atgccatgaa gatcctcgac 240

aaacagaagg tggtgaaact gaaacagatc gaacacaccc tgaatgaaaa gcgcatcctg 300

caagctgtca actttccgtt cctcgtcaaa ctcgagttct ccttcaagga caactcaaac 360

ttatacatgg tcatggagta cgtgcccggc ggggagatgt tctcacacct acggcggatc 420

ggaaggttca gtgagcccca tgcccgtttc tacgcggccc agatcgtcct gacctttgag 480

tatctgcact cgctggatct catctacagg gacctgaagc cggagaatct gctcattgac 540
cagcagggct acattcaggt gacagacttc ggtttcgcca agcgcgtgaa gggccgcact 600
tggaccttgt gcggcacccc tgagtacctg gcccctgaga ttatcctgag caaaggctac 660
aacaaggccg tggactggtg ggccctgggg gttcttatct atgaaatggc cgctggctac 720
ccgcccttct tcgcagacca gcccatccag atctatgaga agatcgtctc tgggaaggtg 780
cgcttccctt cccacttcag ctctgacttg aaggacctgc tgcggaacct cctgcaggta 840
gatctcacca agcgctttgg gaacctcaag aatggggtca acgatatcaa gaaccacaag 900
tggtttgcca caactgactg gattgccatc taccagagga aggtggaagc tcccttcata 960
ccaaagttta aaggccctgg ggatacgagt aactttgacg actatgagga agaagaaatc 1020
cgggtctcca tcaatgagaa gtgtggcaag gagttttctg agttttaggg gcatgcctgt 1080
gcccccatgg gttttctttt ttcttttttc ttttttttgg tcgggggggt gggagggttg 1140
gattgaacag ccagagggcc ccagagttcc ttgcatctaa tttcaccccc accccaccct 1200
ccagggttag ggggagcagg aagcccagat aatcagaggg acagaaacac cagctgctcc 1260
ccctcatccc cttcaccctc ctgcccccctc tcccactttt cccttcctct ttccccacag 1320
ccccccagcc cctcagccct cccagcccac ttctgcctgt tttaaacgag tttctcaact 1380
ccagtcagac caggtcttgc tggtgtatcc agggacaggg tatggaaaga ggggctcacg 1440
cttaactcca gcccccaccc acacccccat cccacccaac cacaggcccc acttgctaag 1500
ggcaaatgaa cgaagcgcca accttccttt cggagtaatc ctgcctggga aggagagatt 1560
tttagtgaca tgttcagtgg gttgcttgct agaatttttt taaaaaaaca acaatttaaa 1620
atcttattta agttccacca gtgcctccct ccctccttcc tctactccca cccctcccat 1680
gtccccccat tcctcaaatc cattttaaag agaagcagac tgactttgga aagggaggcg 1740
ctggggtttg aacctccccg ctgctaatct cccctgggcc cctccccggg gaatcctctc 1800
tgccaatcct gcgagggtct aggccccttt aggaagcctc cgctctcttt ttccccaaca 1860
gacctgtctt caccccttggg ctttgaaagc cagacaaagc agctgcccct ctccctgcca 1920
aagaggagtc atcccccaaa aagacagagg gggagcccca agcccaagtc tttcctccca 1980
gcagcgtttc cccccaactc cttaatttta ttctccgcta gattttaacg tccagccttc 2040
cctcagctga gtggggaggg catccctgca aaagggaaca gaagaggcca agtcccccca 2100
agccacggcc cggggttcaa ggctagagct gctggggagg ggctgcctgt tttactcacc 2160
caccagcttc cgcctccccc atcctgggcg cccctcctcc agcttagctg tcagctgtcc 2220
atcacctctc ccccactttc tcatttgtgc ttttttctct cgtaatagaa aagtggggag 2280
ccgctgggga gccaccccat tcatccccgt atttcccccct ctcataactt ctccccatcc 2340

caggaggagt tctcaggcct ggggtggggc cccgggtggg tgcgggggcg attcaacctg 2400

tgtgctgcga aggacgagac ttcctcttga acagtgtgct gttgtaaaca tatttgaaaa 2460

ctattaccaa taaagttttg tttaaaaaaa aa 2492

<210> 111
<211> 351
<212> PRT
<213> Homo sapiens

<400> 111

Met Gly Asn Ala Ala Ala Ala Lys Lys Gly Ser Glu Gln Glu Ser Val
1               5                   10                  15

Lys Glu Phe Leu Ala Lys Ala Lys Glu Asp Phe Leu Lys Lys Trp Glu
            20                  25                  30

Ser Pro Ala Gln Asn Thr Ala His Leu Asp Gln Phe Glu Arg Ile Lys
        35                  40                  45

Thr Leu Gly Thr Gly Ser Phe Gly Arg Val Met Leu Val Lys His Lys
    50                  55                  60

Glu Thr Gly Asn His Tyr Ala Met Lys Ile Leu Asp Lys Gln Lys Val
65                  70                  75                  80

Val Lys Leu Lys Gln Ile Glu His Thr Leu Asn Glu Lys Arg Ile Leu
                85                  90                  95

Gln Ala Val Asn Phe Pro Phe Leu Val Lys Leu Glu Phe Ser Phe Lys
            100                 105                 110

Asp Asn Ser Asn Leu Tyr Met Val Met Glu Tyr Val Pro Gly Gly Glu
        115                 120                 125

Met Phe Ser His Leu Arg Arg Ile Gly Arg Phe Ser Glu Pro His Ala
    130                 135                 140

Arg Phe Tyr Ala Ala Gln Ile Val Leu Thr Phe Glu Tyr Leu His Ser
145                 150                 155                 160

Leu Asp Leu Ile Tyr Arg Asp Leu Lys Pro Glu Asn Leu Leu Ile Asp
                165                 170                 175

Gln Gln Gly Tyr Ile Gln Val Thr Asp Phe Gly Phe Ala Lys Arg Val
            180                 185                 190

Lys Gly Arg Thr Trp Thr Leu Cys Gly Thr Pro Glu Tyr Leu Ala Pro

195 200 205

Glu Ile Ile Leu Ser Lys Gly Tyr Asn Lys Ala Val Asp Trp Trp Ala
210 215 220

Leu Gly Val Leu Ile Tyr Glu Met Ala Ala Gly Tyr Pro Pro Phe Phe
225 230 235 240

Ala Asp Gln Pro Ile Gln Ile Tyr Glu Lys Ile Val Ser Gly Lys Val
245 250 255

Arg Phe Pro Ser His Phe Ser Ser Asp Leu Lys Asp Leu Leu Arg Asn
260 265 270

Leu Leu Gln Val Asp Leu Thr Lys Arg Phe Gly Asn Leu Lys Asn Gly
275 280 285

Val Asn Asp Ile Lys Asn His Lys Trp Phe Ala Thr Thr Asp Trp Ile
290 295 300

Ala Ile Tyr Gln Arg Lys Val Glu Ala Pro Phe Ile Pro Lys Phe Lys
305 310 315 320

Gly Pro Gly Asp Thr Ser Asn Phe Asp Asp Tyr Glu Glu Glu Glu Ile
325 330 335

Arg Val Ser Ile Asn Glu Lys Cys Gly Lys Glu Phe Ser Glu Phe
340 345 350

<210> 112
<211> 343
<212> PRT
<213> Homo sapiens

<400> 112

Met Ala Ser Asn Ser Ser Asp Val Lys Glu Phe Leu Ala Lys Ala Lys
1 5 10 15

Glu Asp Phe Leu Lys Lys Trp Glu Ser Pro Ala Gln Asn Thr Ala His
20 25 30

Leu Asp Gln Phe Glu Arg Ile Lys Thr Leu Gly Thr Gly Ser Phe Gly
35 40 45

Arg Val Met Leu Val Lys His Lys Glu Thr Gly Asn His Tyr Ala Met
50 55 60

Lys Ile Leu Asp Lys Gln Lys Val Val Lys Leu Lys Gln Ile Glu His

65 70 75 80

Thr Leu Asn Glu Lys Arg Ile Leu Gln Ala Val Asn Phe Pro Phe Leu
85 90 95

Val Lys Leu Glu Phe Ser Phe Lys Asp Asn Ser Asn Leu Tyr Met Val
100 105 110

Met Glu Tyr Val Pro Gly Gly Glu Met Phe Ser His Leu Arg Arg Ile
115 120 125

Gly Arg Phe Ser Glu Pro His Ala Arg Phe Tyr Ala Ala Gln Ile Val
130 135 140

Leu Thr Phe Glu Tyr Leu His Ser Leu Asp Leu Ile Tyr Arg Asp Leu
145 150 155 160

Lys Pro Glu Asn Leu Leu Ile Asp Gln Gln Gly Tyr Ile Gln Val Thr
165 170 175

Asp Phe Gly Phe Ala Lys Arg Val Lys Gly Arg Thr Trp Thr Leu Cys
180 185 190

Gly Thr Pro Glu Tyr Leu Ala Pro Glu Ile Ile Leu Ser Lys Gly Tyr
195 200 205

Asn Lys Ala Val Asp Trp Trp Ala Leu Gly Val Leu Ile Tyr Glu Met
210 215 220

Ala Ala Gly Tyr Pro Pro Phe Phe Ala Asp Gln Pro Ile Gln Ile Tyr
225 230 235 240

Glu Lys Ile Val Ser Gly Lys Val Arg Phe Pro Ser His Phe Ser Ser
245 250 255

Asp Leu Lys Asp Leu Leu Arg Asn Leu Leu Gln Val Asp Leu Thr Lys
260 265 270

Arg Phe Gly Asn Leu Lys Asn Gly Val Asn Asp Ile Lys Asn His Lys
275 280 285

Trp Phe Ala Thr Thr Asp Trp Ile Ala Ile Tyr Gln Arg Lys Val Glu
290 295 300

Ala Pro Phe Ile Pro Lys Phe Lys Gly Pro Gly Asp Thr Ser Asn Phe
305 310 315 320

Asp Asp Tyr Glu Glu Glu Glu Ile Arg Val Ser Ile Asn Glu Lys Cys
325 330 335

Gly Lys Glu Phe Ser Glu Phe
340

<210> 113
<211> 4616
<212> DNA
<213> Homo sapiens

<400> 113
tgcgaagata cagtcgggcc agggcctggc ctgggcgcgc ggctgcccgg gggcgcgcag 60

agagggcgga ccgcgcgaag gggagtgtc tgcccgccgc cgccactgct gctgccaccg 120

ccgtcgccgc cgccgccgcc gccgccgctg ctgctgccgg tgctaaggag ttcgctggag 180

ccctttcctc agacccggcc cggtcttcgc gcccggactc ctggcgccag cgctaggcgc 240

actcaccgct ctgacgggtg cagacgcggg agttgtccca gactgtggag tggcgggcac 300

ggccccagcc ccccttccct tccctgaccc cttcttgcca tcgccccaga catggggaac 360

gcggcgaccg ccaagaaagg cagcgaggtg gagagcgtga aagagtttct agccaaagcc 420

aaagaagact tttgaaaaa atgggagaat ccaactcaga ataatgccgg acttgaagat 480

tttgaaagga aaaaaccct tggaacaggt tcatttggaa gagtcatgtt ggtaaaacac 540

aaagccactg aacagtatta tgccatgaag atcttagata agcagaaggt tgttaaactg 600

aagcaaatag agcatacttt gaatgagaaa agaatattac aggcagtgaa ttttcctttc 660

cttgttcgac tggagtatgc ttttaaggat aattctaatt tatacatggt tatggaatat 720

gtccctgggg gtgaaatgtt ttcacatcta agaagaattg gaaggttcag tgagccccat 780

gcacggttct atgcagctca gatagtgcta acattcgagt acctccattc actagacctc 840

atctacagag atctaaaacc tgaaaatctc ttaattgacc atcaaggcta tatccaggtc 900

acagactttg ggtttgccaa aagagttaaa ggcagaactt ggacattatg tggaactcca 960

gagtatttgg ctccagaaat aattctcagc aagggctaca ataaggcagt ggattggtgg 1020

gcattaggag tgctaatcta tgaaatggca gctggctatc cccccattctt tgcagaccaa 1080

ccaattcaga tttatgaaaa gattgtttct ggaaaggtcc gattcccatc ccacttcagt 1140

tcagatctca aggaccttct acggaacctg ctgcaggtgg atttgaccaa gagatttgga 1200

aatctaaaga atggtgtcag tgatataaaa actcacaagt ggtttgccac gacagattgg 1260

attgctattt accagaggaa ggttgaagct ccattcatac caaagtttag aggctctgga 1320

gataccagca actttgatga ctatgaagaa gaagatatcc gtgtctctat aacagaaaaa 1380

tgtgcaaaag aatttggtga attttaaaga ggaacaagat gacatctgag ctcacactca 1440

gtgtttgcac tctgttgaga gataaggtag agctgagacc gtccttgttg aagcagttac 1500

ctagttcctt cattccaacg actgagtgag gtctttattg ccatcatccc gtgtgcgcac 1560
tctgcatcca cctatgtaac aaggcaccgc taagcaagca ttgtctgtgc cataacacag 1620
tactagacca ctttcttact tctctttggg ttgtctttct cctctcctat atccatttct 1680
tccttttcca atttcattgg ttttctctaa acagtgctcc attttatttt gttggtgttt 1740
cagatgggca gtgttatggc tacgtgatat ttgaagggaa ggataagtgt tgctttcagt 1800
agttattgcc aatattgttg ttggtcaatg gcttgaagat aaactttcta ataattatta 1860
tttctttgag tagctcagac ttggttttgc caaaactctt ggtaattttt gaagatagac 1920
tgtcttatca ccaaggaaat ttatacaaat taagactaac tttcttggaa ttcactattc 1980
tggcaataaa ttttggtaga ctaatacagt acagctagac ccagaaattt ggaaggctgt 2040
agatcagagg ttctagttcc ctttccctcc ttttatatcc tcctctcctt gagtaatgaa 2100
gtgaccagcc tgtgtagtgt gacaaacgtg tctcattcag caggaaaaac taatgatatg 2160
gatcatcacc cagattctct cacttggtac cagcatttct gtaggtatta gagaagagtt 2220
ctaagttttc taaaccttaa ctgttcctta aggattttag ccagtatttt aatagaacat 2280
gattaatgaa agtgacaaat tttaaatttt ctctaatagt cctcatcata aactttttaa 2340
aggaaaataa gcaaactaaa aagaacattg gtttagataa atacttatac tttgcaaagt 2400
caaaaatggc ttgatttttg gaaacaatat agaggtattc atatttaaat gagggtttac 2460
atttgttttg ttttgtaacc gttaaaaaga agttgtttcc agctaattat tgtggtgtac 2520
tatatttgtg agcctagggt aggggcactg ctgcaacttc tgctttcatc ccatgcctca 2580
tcaatgagga aagggaacaa agtgtataaa actgccacaa ttgtatttta attttgaggt 2640
atgatatttt cagatatttc ataatttcta acctctgttc tctcagtaaa cagaatgtct 2700
gatcgatcat gcagatacaa tgttggtatt tgagaggtta gttttttcc tacacttttt 2760
tttgccaact gacttaacaa cattgctgtc aggtggaaat ttcaagcact tttgcacatt 2820
tagttcagtg tttgttgaga atccatggct taacccactt gttttgctat tttttcttt 2880
gcttttaatt ttccccatct gattttatct ctgcgtttca gtgacctacc ttaaaacaac 2940
acacgagaag agttaaactg ggttcatttt aatgatcaat ttacctgcat ataaaattta 3000
tttttaatca agctgatctt aatgtatata atcattctat ttgctttatt atcggtgcag 3060
gtaggtcatt aacaccactt cttttcatct gtaccacacc ctggtgaaac ctttgaagac 3120
ataaaaaaaa cctgtctgag atgttctttc taccaatcta tatgtctttc ggttatcaag 3180
tgtttctgca tggtaatgtc atgtaaatgc tgatattgat ttcactggtc catctatatt 3240
taaaacgtgc aagaaaaaaa taaaatactc tgctctagca agttttgtgt aacaaaggca 3300
tatcgtcatg ttaataaatt taaaacatca ttcgtataaa atattttaat tttcttgtat 3360

ttcatttaga cccaagaaca tgctgaccaa tgtgttctat atgtaaacta caaattctat 3420

ggtagctttg ttgtatatta ttgtaaaatt attttaataa gtcatgggga tgacaatttg 3480

attattacaa tttagttttc agtaatcaaa aagatttcta tgaattctaa aaaatatttt 3540

tttctatgaa attactagtg cccagctgta gaatctacct taggtagatg atccctagac 3600

atacgttggt tttgagggct attcagccat tccattttac tctctattta aaggccgtga 3660

gcaagcttgt catgagcaaa tatgtcaagg gagtcaattt ctgaccaatc aagtacacta 3720

aattagaata tttttaaagt atgtaacatt cccagtttca gccacaattt agccaagaat 3780

aagataaaaa cttgaataag aagtaagtag cataaatcag tatttaacct aaaattacat 3840

atttgaaaca gaagatatta tgttatgctc agtaaataat taagagatgg cattgtgtaa 3900

gaaggagccc tagactgaaa gtcaagacat ctgaatttca ggctggaaaa ctatcagtat 3960

gatctcagcc tcagttctct tgtctgtaaa atggaagaac tggattaggc agtttgtaag 4020

attcctccta actttcacag tcgatgacaa gattgtcttt ttatctgata ttttgaaggg 4080

tatattgctt tgaagtaagt ctcaataagg caatatattt tagggcatct ttcttcttat 4140

ctctgacagt gttcttaaaa ttatttgaat atcataagag ccttggtgtc tgtcctaatt 4200

cctttctcac tcaccgatgc tgaataccca gttgaatcaa actgtcaacc taccaaaaac 4260

gatattgtgg cttatgggta ttgctgtctc attcttggta tattcttgtg ttaactgccc 4320

attggcctga aaatactcat tgtaagcctg aaaaaaaaaa tctttcccac tgttttttct 4380

gcttgttgta agaatcaaat gaaataatgt atgtgaaagc accttgtaaa ctgtaaccta 4440

tcaatgtaaa atgttaaggt gtgttgttat ttcattaatt acttctttgt ttagaatgga 4500

atttcctatg cactactgta gctaggaaat gctgaaaaca actgtgtttt ttaattaatc 4560

aataactgca aaattaaagt accttcaatg gataagacaa aaaaaaaaaa aaaaaa 4616

<210> 114
<211> 4481
<212> DNA
<213> Homo sapiens

<400> 114
acatgcatag ctcttagctt ctgtgtaaga agttgtgagc tccttctgga aacatttgca 60

gttacattaa gtaaagtgta aatgcacatg aatggcagct tatagagaac caccttgtaa 120

ccagtataca ggtacaacta cagctcttca gaaattggaa ggttttgcta gccggttatt 180

tcatagacac tctaaaggta ctgcacatga tcagaaaaca gctctggaaa atgacagcct 240

tcatttctct gaacatactg ccttatggga cagatcaatg aaagagtttc tagccaaagc 300

caaagaagac tttttgaaaa aatgggagaa tccaactcag aataatgccg gacttgaaga 360

ttttgaaagg aaaaaaaccc ttggaacagg ttcatttgga agagtcatgt tggtaaaaca 420

caaagccact gaacagtatt atgccatgaa gatcttagat aagcagaagg ttgttaaact 480
gaagcaaata gagcatactt tgaatgagaa aagaatatta caggcagtga attttccttt 540
ccttgttcga ctggagtatg cttttaagga taattctaat ttatacatgg ttatggaata 600
tgtccctggg ggtgaaatgt tttcacatct aagaagaatt ggaaggttca gtgagcccca 660
tgcacggttc tatgcagctc agatagtgct aacattcgag tacctccatt cactagacct 720
catctacaga gatctaaaac ctgaaaatct cttaattgac catcaaggct atatccaggt 780
cacagacttt gggtttgcca aaagagttaa aggcagaact tggacattat gtggaactcc 840
agagtatttg gctccagaaa taattctcag caagggctac aataaggcag tggattggtg 900
ggcattagga gtgctaatct atgaaatggc agctggctat cccccattct ttgcagacca 960
accaattcag atttatgaaa agattgtttc tggaaaggtc cgattcccat cccacttcag 1020
ttcagatctc aaggaccttc tacggaacct gctgcaggtg gatttgacca agagatttgg 1080
aaatctaaag aatggtgtca gtgatataaa aactcacaag tggtttgcca cgacagattg 1140
gattgctatt taccagagga aggttgaagc tccattcata ccaaagttta gaggctctgg 1200
agataccagc aactttgatg actatgaaga agaagatatc cgtgtctcta taacagaaaa 1260
atgtgcaaaa gaatttggtg aattttaaag aggaacaaga tgacatctga gctcacactc 1320
agtgtttgca ctctgttgag agataaggta gagctgagac cgtccttgtt gaagcagtta 1380
cctagttcct tcattccaac gactgagtga ggtctttatt gccatcatcc cgtgtgcgca 1440
ctctgcatcc acctatgtaa caaggcaccg ctaagcaagc attgtctgtg ccataacaca 1500
gtactagacc actttcttac ttctctttgg gttgtctttc tcctctccta tatccatttc 1560
ttccttttcc aatttcattg gttttctcta aacagtgctc cattttattt tgttggtgtt 1620
tcagatgggc agtgttatgg ctacgtgata tttgaaggga aggataagtg ttgctttcag 1680
tagttattgc caatattgtt gttggtcaat ggcttgaaga taaactttct aataattatt 1740
atttctttga gtagctcaga cttggttttg ccaaaactct tggtaatttt tgaagataga 1800
ctgtcttatc accaaggaaa tttatacaaa ttaagactaa ctttcttgga attcactatt 1860
ctggcaataa attttggtag actaatacag tacagctaga cccagaaatt tggaaggctg 1920
tagatcagag gttctagttc cctttccctc cttttatatc ctcctctcct tgagtaatga 1980
agtgaccagc ctgtgtagtg tgacaaacgt gtctcattca gcaggaaaaa ctaatgatat 2040
ggatcatcac ccagattctc tcacttggta ccagcatttc tgtaggtatt agagaagagt 2100
tctaagtttt ctaaacctta actgttcctt aaggatttta gccagtattt taatagaaca 2160
tgattaatga aagtgacaaa ttttaaattt tctctaatag tcctcatcat aaacttttta 2220
aaggaaaata agcaaactaa aaagaacatt ggtttagata aatacttata ctttgcaaag 2280
tcaaaaatgg cttgattttt ggaaacaata tagaggtatt catatttaaa tgagggttta 2340

catttgtttt gttttgtaac cgttaaaaag aagttgtttc cagctaatta ttgtggtgta 2400
ctatatttgt gagcctaggg taggggcact gctgcaactt ctgctttcat cccatgcctc 2460
atcaatgagg aaagggaaca aagtgtataa aactgccaca attgtatttt aattttgagg 2520
tatgatattt tcagatattt cataatttct aacctctgtt ctctcagtaa acagaatgtc 2580
tgatcgatca tgcagataca atgttggtat ttgagaggtt agttttttc ctacactttt 2640
ttttgccaac tgacttaaca acattgctgt caggtggaaa tttcaagcac ttttgcacat 2700
ttagttcagt gtttgttgag aatccatggc ttaacccact tgttttgcta tttttttctt 2760
tgcttttaat tttccccatc tgattttatc tctgcgtttc agtgacctac cttaaaacaa 2820
cacacgagaa gagttaaact gggttcattt taatgatcaa tttacctgca tataaaattt 2880
atttttaatc aagctgatct taatgtatat aatcattcta tttgctttat tatcggtgca 2940
ggtaggtcat taacaccact tcttttcatc tgtaccacac cctggtgaaa cctttgaaga 3000
cataaaaaaa acctgtctga gatgttcttt ctaccaatct atatgtcttt cggttatcaa 3060
gtgtttctgc atggtaatgt catgtaaatg ctgatattga tttcactggt ccatctatat 3120
ttaaaacgtg caagaaaaaa ataaaatact ctgctctagc aagttttgtg taacaaaggc 3180
atatcgtcat gttaataaat ttaaaacatc attcgtataa aatattttaa ttttcttgta 3240
tttcatttag acccaagaac atgctgacca atgtgttcta tatgtaaact acaaattcta 3300
tggtagcttt gttgtatatt attgtaaaat tattttaata agtcatgggg atgacaattt 3360
gattattaca atttagtttt cagtaatcaa aaagatttct atgaattcta aaaaatattt 3420
ttttctatga aattactagt gcccagctgt agaatctacc ttaggtagat gatccctaga 3480
catacgttgg ttttgagggc tattcagcca ttccatttta ctctctattt aaaggccgtg 3540
agcaagcttg tcatgagcaa atatgtcaag ggagtcaatt tctgaccaat caagtacact 3600
aaattagaat atttttaaag tatgtaacat tcccagtttc agccacaatt tagccaagaa 3660
taagataaaa acttgaataa gaagtaagta gcataaatca gtatttaacc taaaattaca 3720
tatttgaaac agaagatatt atgttatgct cagtaaataa ttaagagatg gcattgtgta 3780
agaaggagcc ctagactgaa agtcaagaca tctgaatttc aggctggaaa actatcagta 3840
tgatctcagc ctcagttctc ttgtctgtaa aatggaagaa ctggattagg cagtttgtaa 3900
gattcctcct aactttcaca gtcgatgaca agattgtctt tttatctgat attttgaagg 3960
gtatattgct ttgaagtaag tctcaataag gcaatatatt ttagggcatc tttcttctta 4020
tctctgacag tgttcttaaa attatttgaa tatcataaga gccttggtgt ctgtcctaat 4080
tcctttctca ctcaccgatg ctgaataccc agttgaatca aactgtcaac ctaccaaaaa 4140
cgatattgtg gcttatgggt attgctgtct cattcttggt atattcttgt gttaactgcc 4200

cattggcctg aaaatactca ttgtaagcct gaaaaaaaaa atctttccca ctgtttttc 4260

tgcttgttgt aagaatcaaa tgaaataatg tatgtgaaag caccttgtaa actgtaacct 4320

atcaatgtaa aatgttaagg tgtgttgtta tttcattaat tacttctttg tttagaatgg 4380

aatttcctat gcactactgt agctaggaaa tgctgaaaac aactgtgttt tttaattaat 4440

caataactgc aaaattaaag taccttcaat ggataagaca a 4481

<210> 115
<211> 4650
<212> DNA
<213> Homo sapiens

<400> 115
atgctgatat aattgagaac atcttataca tcctggttcg aacattttct ccctgccatt 60

ttgagttgtt ctagtggtat atgaaggagg ctgggataac tagcttgaaa gaaattcagt 120

ctagttatag acatctttgg cattaatctg atgtttacta gtgatatctc atgctaggca 180

gttatgcttt gcttctaggg gcttctcttt ttaaaacaaa agaaagctct tttcgttttc 240

tgtgtgctgc atgctccagt gtgtgtgttt acaccatcgg ttcttctccc tctagagatt 300

agcataactc cctttgctgt tggattgtta ttttgagcaa tatgttttgg aaaggttggt 360

tttcatcatg agtgcacgca aatcatcaga tgcatctgct tgctcctctt cagaaatatc 420

tgattccttt gtgaaagagt ttctagccaa agccaaagaa gactttttga aaaaatggga 480

gaatccaact cagaataatg ccggacttga agattttgaa aggaaaaaaa cccttggaac 540

aggttcattt ggaagagtca tgttggtaaa acacaaagcc actgaacagt attatgccat 600

gaagatctta gataagcaga aggttgttaa actgaagcaa atagagcata ctttgaatga 660

gaaaagaata ttacaggcag tgaattttcc tttccttgtt cgactggagt atgcttttaa 720

ggataattct aatttataca tggttatgga atatgtccct gggggtgaaa tgttttcaca 780

tctaagaaga attggaaggt tcagtgagcc ccatgcacgg ttctatgcag ctcagatagt 840

gctaacattc gagtacctcc attcactaga cctcatctac agagatctaa aacctgaaaa 900

tctcttaatt gaccatcaag gctatatcca ggtcacagac tttgggtttg ccaaaagagt 960

taaaggcaga acttggacat tatgtggaac tccagagtat ttggctccag aaataattct 1020

cagcaagggc tacaataagg cagtggattg gtgggcatta ggagtgctaa tctatgaaat 1080

ggcagctggc tatcccccat tctttgcaga ccaaccaatt cagatttatg aaaagattgt 1140

ttctggaaag gtccgattcc catcccactt cagttcagat ctcaaggacc ttctacggaa 1200

cctgctgcag gtggatttga ccaagagatt tggaaatcta aagaatggtg tcagtgatat 1260

aaaaactcac aagtggtttg ccacgacaga ttggattgct atttaccaga ggaaggttga 1320

agctccattc ataccaaagt ttagaggctc tggagatacc agcaactttg atgactatga 1380

agaagaagat atccgtgtct ctataacaga aaaatgtgca aaagaatttg gtgaatttta 1440
aagaggaaca agatgacatc tgagctcaca ctcagtgttt gcactctgtt gagagataag 1500
gtagagctga gaccgtcctt gttgaagcag ttacctagtt ccttcattcc aacgactgag 1560
tgaggtcttt attgccatca tcccgtgtgc gcactctgca tccacctatg taacaaggca 1620
ccgctaagca agcattgtct gtgccataac acagtactag accactttct tacttctctt 1680
tgggttgtct ttctcctctc ctatatccat ttcttccttt tccaatttca ttggttttct 1740
ctaaacagtg ctccatttta ttttgttggt gtttcagatg ggcagtgtta tggctacgtg 1800
atatttgaag ggaaggataa gtgttgcttt cagtagttat tgccaatatt gttgttggtc 1860
aatggcttga agataaactt tctaataatt attatttctt tgagtagctc agacttggtt 1920
ttgccaaaac tcttggtaat ttttgaagat agactgtctt atcaccaagg aaatttatac 1980
aaattaagac taactttctt ggaattcact attctggcaa taaattttgg tagactaata 2040
cagtacagct agacccagaa atttggaagg ctgtagatca gaggttctag ttccctttcc 2100
ctccttttat atcctcctct ccttgagtaa tgaagtgacc agcctgtgta gtgtgacaaa 2160
cgtgtctcat tcagcaggaa aaactaatga tatggatcat cacccagatt ctctcacttg 2220
gtaccagcat ttctgtaggt attagagaag agttctaagt tttctaaacc ttaactgttc 2280
cttaaggatt ttagccagta ttttaataga acatgattaa tgaaagtgac aaattttaaa 2340
ttttctctaa tagtcctcat cataaacttt ttaaaggaaa ataagcaaac taaaaagaac 2400
attggtttag ataaatactt atactttgca aagtcaaaaa tggcttgatt tttggaaaca 2460
atatagaggt attcatattt aaatgagggt ttacatttgt tttgttttgt aaccgttaaa 2520
aagaagttgt ttccagctaa ttattgtggt gtactatatt tgtgagccta gggtaggggc 2580
actgctgcaa cttctgcttt catcccatgc ctcatcaatg aggaaaggga acaaagtgta 2640
taaaactgcc acaattgtat tttaattttg aggtatgata ttttcagata tttcataatt 2700
tctaacctct gttctctcag taaacagaat gtctgatcga tcatgcagat acaatgttgg 2760
tatttgagag gttagttttt ttcctacact ttttttttgcc aactgactta acaacattgc 2820
tgtcaggtgg aaatttcaag cacttttgca catttagttc agtgtttgtt gagaatccat 2880
ggcttaaccc acttgttttg ctattttttt ctttgctttt aattttcccc atctgatttt 2940
atctctgcgt ttcagtgacc taccttaaaa caacacacga gaagagttaa actgggttca 3000
ttttaatgat caatttacct gcatataaaa tttattttta atcaagctga tcttaatgta 3060
tataatcatt ctatttgctt tattatcggt gcaggtaggt cattaacacc acttcttttc 3120
atctgtacca caccctggtg aaacctttga agacataaaa aaaacctgtc tgagatgttc 3180
tttctaccaa tctatatgtc tttcggttat caagtgtttc tgcatggtaa tgtcatgtaa 3240
atgctgatat tgatttcact ggtccatcta tatttaaaac gtgcaagaaa aaaataaaat 3300

actctgctct agcaagtttt gtgtaacaaa ggcatatcgt catgttaata aatttaaaac 3360

atcattcgta taaaatattt taattttctt gtatttcatt tagacccaag aacatgctga 3420

ccaatgtgtt ctatatgtaa actacaaatt ctatggtagc tttgttgtat attattgtaa 3480

aattatttta ataagtcatg gggatgacaa tttgattatt acaatttagt tttcagtaat 3540

caaaaagatt tctatgaatt ctaaaaaata tttttttcta tgaaattact agtgcccagc 3600

tgtagaatct accttaggta gatgatccct agacatacgt tggttttgag ggctattcag 3660

ccattccatt ttactctcta tttaaaggcc gtgagcaagc ttgtcatgag caaatatgtc 3720

aagggagtca atttctgacc aatcaagtac actaaattag aatattttta aagtatgtaa 3780

cattcccagt ttcagccaca atttagccaa gaataagata aaaacttgaa taagaagtaa 3840

gtagcataaa tcagtattta acctaaaatt acatatttga aacagaagat attatgttat 3900

gctcagtaaa taattaagag atggcattgt gtaagaagga gccctagact gaaagtcaag 3960

acatctgaat ttcaggctgg aaaactatca gtatgatctc agcctcagtt ctcttgtctg 4020

taaaatggaa gaactggatt aggcagtttg taagattcct cctaactttc acagtcgatg 4080

acaagattgt ctttttatct gatattttga agggtatatt gctttgaagt aagtctcaat 4140

aaggcaatat attttagggc atctttcttc ttatctctga cagtgttctt aaaattattt 4200

gaatatcata agagccttgg tgtctgtcct aattcctttc tcactcaccg atgctgaata 4260

cccagttgaa tcaaactgtc aacctaccaa aaacgatatt gtggcttatg ggtattgctg 4320

tctcattctt ggtatattct tgtgttaact gcccattggc ctgaaaatac tcattgtaag 4380

cctgaaaaaa aaaatctttc ccactgtttt ttctgcttgt tgtaagaatc aaatgaaata 4440

atgtatgtga aagcaccttg taaactgtaa cctatcaatg taaaatgtta aggtgtgttg 4500

ttatttcatt aattacttct ttgtttagaa tggaatttcc tatgcactac tgtagctagg 4560

aaatgctgaa aacaactgtg ttttttaatt aatcaataac tgcaaaatta aagtaccttc 4620

aatggataag acaaaaaaaa aaaaaaaaaa 4650

<210> 116
<211> 4506
<212> DNA
<213> Homo sapiens

<400> 116
taacacagaa tgtttaaatg gttaacattt gtgctgcagt atagctttct ggctcatgaa 60

aaatgaaagc tatcagcgat ctcggcaata agattcatcg ccaatagtca ctagcaacag 120

cacacagcat tttaatatca gtgaggtcca cagctagcag taagagctgg tgtaattgaa 180

agacgtttag gtgcaatcat tctgctgttt gctccttgcc aggttcaaca tgggattgtc 240

acgcaaatca tcagatgcat ctgcttgctc ctcttcagaa atatctgtga aagagtttct 300

agccaaagcc aaagaagact ttttgaaaaa atgggagaat ccaactcaga ataatgccgg 360
acttgaagat tttgaaagga aaaaaaccct tggaacaggt tcatttggaa gagtcatgtt 420
ggtaaaacac aaagccactg aacagtatta tgccatgaag atcttagata agcagaaggt 480
tgttaaactg aagcaaatag agcatacttt gaatgagaaa agaatattac aggcagtgaa 540
ttttcctttc cttgttcgac tggagtatgc ttttaaggat aattctaatt tatacatggt 600
tatggaatat gtccctgggg gtgaaatgtt ttcacatcta agaagaattg gaaggttcag 660
tgagccccat gcacggttct atgcagctca gatagtgcta acattcgagt acctccattc 720
actagacctc atctacagag atctaaaacc tgaaaatctc ttaattgacc atcaaggcta 780
tatccaggtc acagactttg ggtttgccaa aagagttaaa ggcagaactt ggacattatg 840
tggaactcca gagtatttgg ctccagaaat aattctcagc aagggctaca ataaggcagt 900
ggattggtgg gcattaggag tgctaatcta tgaaatggca gctggctatc ccccattctt 960
tgcagaccaa ccaattcaga tttatgaaaa gattgtttct ggaaaggtcc gattcccatc 1020
ccacttcagt tcagatctca aggaccttct acggaacctg ctgcaggtgg atttgaccaa 1080
gagatttgga aatctaaaga atggtgtcag tgatataaaa actcacaagt ggtttgccac 1140
gacagattgg attgctattt accagaggaa ggttgaagct ccattcatac caaagtttag 1200
aggctctgga gataccagca actttgatga ctatgaagaa gaagatatcc gtgtctctat 1260
aacagaaaaa tgtgcaaaag aatttggtga attttaaaga ggaacaagat gacatctgag 1320
ctcacactca gtgtttgcac tctgttgaga gataaggtag agctgagacc gtccttgttg 1380
aagcagttac ctagttcctt cattccaacg actgagtgag gtctttattg ccatcatccc 1440
gtgtgcgcac tctgcatcca cctatgtaac aaggcaccgc taagcaagca ttgtctgtgc 1500
cataacacag tactagacca ctttcttact tctctttggg ttgtctttct cctctcctat 1560
atccatttct tccttttcca atttcattgg ttttctctaa acagtgctcc attttatttt 1620
gttggtgttt cagatgggca gtgttatggc tacgtgatat ttgaagggaa ggataagtgt 1680
tgctttcagt agttattgcc aatattgttg ttggtcaatg gcttgaagat aaactttcta 1740
ataattatta tttctttgag tagctcagac ttggttttgc caaaactctt ggtaattttt 1800
gaagatagac tgtcttatca ccaaggaaat ttatacaaat taagactaac tttcttggaa 1860
ttcactattc tggcaataaa ttttggtaga ctaatacagt acagctagac ccagaaattt 1920
ggaaggctgt agatcagagg ttctagttcc ctttccctcc ttttatatcc tcctctcctt 1980
gagtaatgaa gtgaccagcc tgtgtagtgt gacaaacgtg tctcattcag caggaaaaac 2040
taatgatatg gatcatcacc cagattctct cacttggtac cagcatttct gtaggtatta 2100
gagaagagtt ctaagttttc taaaccttaa ctgttcctta aggattttag ccagtatttt 2160

aatagaacat gattaatgaa agtgacaaat tttaaatttt ctctaatagt cctcatcata 2220
aactttttaa aggaaaataa gcaaactaaa aagaacattg gtttagataa atacttatac 2280
tttgcaaagt caaaaatggc ttgatttttg gaaacaatat agaggtattc atatttaaat 2340
gagggtttac atttgttttg ttttgtaacc gttaaaaaga agttgtttcc agctaattat 2400
tgtggtgtac tatatttgtg agcctagggt aggggcactg ctgcaacttc tgctttcatc 2460
ccatgcctca tcaatgagga aagggaacaa agtgtataaa actgccacaa ttgtatttta 2520
attttgaggt atgatatttt cagatatttc ataatttcta acctctgttc tctcagtaaa 2580
cagaatgtct gatcgatcat gcagatacaa tgttggtatt tgagaggtta gttttttttcc 2640
tacacttttt tttgccaact gacttaacaa cattgctgtc aggtggaaat ttcaagcact 2700
tttgcacatt tagttcagtg tttgttgaga atccatggct taacccactt gttttgctat 2760
ttttttcttt gcttttaatt ttccccatct gattttatct ctgcgtttca gtgacctacc 2820
ttaaaacaac acacgagaag agttaaactg ggttcatttt aatgatcaat ttacctgcat 2880
ataaaattta tttttaatca agctgatctt aatgtatata atcattctat ttgctttatt 2940
atcggtgcag gtaggtcatt aacaccactt cttttcatct gtaccacacc ctggtgaaac 3000
ctttgaagac ataaaaaaaa cctgtctgag atgttctttc taccaatcta tatgtctttc 3060
ggttatcaag tgtttctgca tggtaatgtc atgtaaatgc tgatattgat ttcactggtc 3120
catctatatt taaaacgtgc aagaaaaaaa taaaatactc tgctctagca agttttgtgt 3180
aacaaaggca tatcgtcatg ttaataaatt taaaacatca ttcgtataaa atattttaat 3240
tttcttgtat ttcatttaga cccaagaaca tgctgaccaa tgtgttctat atgtaaacta 3300
caaattctat ggtagctttg ttgtatatta ttgtaaaatt attttaataa gtcatgggga 3360
tgacaatttg attattacaa tttagttttc agtaatcaaa aagatttcta tgaattctaa 3420
aaaatatttt tttctatgaa attactagtg cccagctgta gaatctacct taggtagatg 3480
atccctagac atacgttggt tttgagggct attcagccat tccattttac tctctattta 3540
aaggccgtga gcaagcttgt catgagcaaa tatgtcaagg gagtcaattt ctgaccaatc 3600
aagtacacta aattagaata tttttaaagt atgtaacatt cccagtttca gccacaattt 3660
agccaagaat aagataaaaa cttgaataag aagtaagtag cataaatcag tatttaacct 3720
aaaattacat atttgaaaca gaagatatta tgttatgctc agtaaataat taagagatgg 3780
cattgtgtaa gaaggagccc tagactgaaa gtcaagacat ctgaatttca ggctggaaaa 3840
ctatcagtat gatctcagcc tcagttctct tgtctgtaaa atggaagaac tggattaggc 3900
agtttgtaag attcctccta actttcacag tcgatgacaa gattgtcttt ttatctgata 3960
ttttgaaggg tatattgctt tgaagtaagt ctcaataagg caatatattt tagggcatct 4020
ttcttcttat ctctgacagt gttcttaaaa ttatttgaat atcataagag ccttggtgtc 4080

tgtcctaatt cctttctcac tcaccgatgc tgaataccca gttgaatcaa actgtcaacc 4140

taccaaaaac gatattgtgg cttatgggta ttgctgtctc attcttggta tattcttgtg 4200

ttaactgccc attggcctga aaatactcat tgtaagcctg aaaaaaaaaa tctttcccac 4260

tgtttttttct gcttgttgta agaatcaaat gaaataatgt atgtgaaagc accttgtaaa 4320

ctgtaaccta tcaatgtaaa atgttaaggt gtgttgttat ttcattaatt acttctttgt 4380

ttagaatgga atttcctatg cactactgta gctaggaaat gctgaaaaca actgtgtttt 4440

ttaattaatc aataactgca aaattaaagt accttcaatg gataagacaa aaaaaaaaaa 4500

aaaaaa 4506

<210> 117
<211> 4458
<212> DNA
<213> Homo sapiens

<400> 117
taacacagaa tgtttaaatg gttaacattt gtgctgcagt atagctttct ggctcatgaa 60

aaatgaaagc tatcagcgat ctcggcaata agattcatcg ccaatagtca ctagcaacag 120

cacacagcat tttaatatca gtgaggtcca cagctagcag taagagctgg tgtaattgaa 180

agacgtttag gtgcaatcat tctgctgttt gctccttgcc aggttcaaca tgggattgtt 240

gaaagagttt ctagccaaag ccaaagaaga ctttttgaaa aaatgggaga atccaactca 300

gaataatgcc ggacttgaag attttgaaag gaaaaaaacc cttggaacag gttcatttgg 360

aagagtcatg ttggtaaaac acaaagccac tgaacagtat tatgccatga agatcttaga 420

taagcagaag gttgttaaac tgaagcaaat agagcatact ttgaatgaga aaagaatatt 480

acaggcagtg aattttcctt tccttgttcg actggagtat gcttttaagg ataattctaa 540

tttatacatg gttatggaat atgtccctgg gggtgaaatg ttttcacatc taagaagaat 600

tggaaggttc agtgagcccc atgcacggtt ctatgcagct cagatagtgc taacattcga 660

gtacctccat tcactagacc tcatctacag agatctaaaa cctgaaaatc tcttaattga 720

ccatcaaggc tatatccagg tcacagactt tgggtttgcc aaaagagtta aaggcagaac 780

ttggacatta tgtggaactc cagagtattt ggctccagaa ataattctca gcaagggcta 840

caataaggca gtggattggt gggcattagg agtgctaatc tatgaaatgg cagctggcta 900

tcccccattc tttgcagacc aaccaattca gatttatgaa aagattgttt ctggaaaggt 960

ccgattccca tcccacttca gttcagatct caaggacctt ctacggaacc tgctgcaggt 1020

ggatttgacc aagagatttg gaaatctaaa gaatggtgtc agtgatataa aaactcacaa 1080

gtggtttgcc acgacagatt ggattgctat ttaccagagg aaggttgaag ctccattcat 1140

accaaagttt agaggctctg gagataccag caactttgat gactatgaag aagaagatat 1200

ccgtgtctct ataacagaaa aatgtgcaaa agaatttggt gaattttaaa gaggaacaag 1260
atgacatctg agctcacact cagtgtttgc actctgttga gagataaggt agagctgaga 1320
ccgtccttgt tgaagcagtt acctagttcc ttcattccaa cgactgagtg aggtctttat 1380
tgccatcatc ccgtgtgcgc actctgcatc cacctatgta acaaggcacc gctaagcaag 1440
cattgtctgt gccataacac agtactagac cactttctta cttctctttg ggttgtcttt 1500
ctcctctcct atatccattt cttccttttc caatttcatt ggttttctct aaacagtgct 1560
ccattttatt ttgttggtgt ttcagatggg cagtgttatg gctacgtgat atttgaaggg 1620
aaggataagt gttgctttca gtagttattg ccaatattgt tgttggtcaa tggcttgaag 1680
ataaactttc taataattat tatttctttg agtagctcag acttggtttt gccaaaactc 1740
ttggtaattt ttgaagatag actgtcttat caccaaggaa atttatacaa attaagacta 1800
actttcttgg aattcactat tctggcaata aattttggta gactaataca gtacagctag 1860
acccagaaat ttggaaggct gtagatcaga ggttctagtt ccctttccct ccttttatat 1920
cctcctctcc ttgagtaatg aagtgaccag cctgtgtagt gtgacaaacg tgtctcattc 1980
agcaggaaaa actaatgata tggatcatca cccagattct ctcacttggt accagcattt 2040
ctgtaggtat tagagaagag ttctaagttt tctaaacctt aactgttcct taaggatttt 2100
agccagtatt ttaatagaac atgattaatg aaagtgacaa attttaaatt ttctctaata 2160
gtcctcatca taaacttttt aaaggaaaat aagcaaacta aaaagaacat tggtttagat 2220
aaatacttat actttgcaaa gtcaaaaatg gcttgatttt tggaaacaat atagaggtat 2280
tcatatttaa atgagggttt acatttgttt tgttttgtaa ccgttaaaaa gaagttgttt 2340
ccagctaatt attgtggtgt actatatttg tgagcctagg gtaggggcac tgctgcaact 2400
tctgctttca tcccatgcct catcaatgag gaaagggaac aaagtgtata aaactgccac 2460
aattgtattt taattttgag gtatgatatt ttcagatatt tcataatttc taacctctgt 2520
tctctcagta aacagaatgt ctgatcgatc atgcagatac aatgttggta tttgagaggt 2580
tagttttttt cctacacttt tttttgccaa ctgacttaac aacattgctg tcaggtggaa 2640
atttcaagca cttttgcaca tttagttcag tgtttgttga gaatccatgg cttaacccac 2700
ttgttttgct attttttttct ttgcttttaa ttttccccat ctgattttat ctctgcgttt 2760
cagtgaccta ccttaaaaca acacacgaga agagttaaac tgggttcatt ttaatgatca 2820
atttacctgc atataaaatt tatttttaat caagctgatc ttaatgtata taatcattct 2880
atttgcttta ttatcggtgc aggtaggtca ttaacaccac ttcttttcat ctgtaccaca 2940
ccctggtgaa acctttgaag acataaaaaa aacctgtctg agatgttctt tctaccaatc 3000
tatatgtctt tcggttatca agtgtttctg catggtaatg tcatgtaaat gctgatattg 3060

atttcactgg tccatctata tttaaaacgt gcaagaaaaa aataaaatac tctgctctag 3120

caagttttgt gtaacaaagg catatcgtca tgttaataaa tttaaaacat cattcgtata 3180

aaatatttta attttcttgt atttcattta gacccaagaa catgctgacc aatgtgttct 3240

atatgtaaac tacaaattct atggtagctt tgttgtatat tattgtaaaa ttattttaat 3300

aagtcatggg gatgacaatt tgattattac aatttagttt tcagtaatca aaaagatttc 3360

tatgaattct aaaaaatatt tttttctatg aaattactag tgcccagctg tagaatctac 3420

cttaggtaga tgatccctag acatacgttg gttttgaggg ctattcagcc attccatttt 3480

actctctatt taaaggccgt gagcaagctt gtcatgagca aatatgtcaa gggagtcaat 3540

ttctgaccaa tcaagtacac taaattagaa tatttttaaa gtatgtaaca ttcccagttt 3600

cagccacaat ttagccaaga ataagataaa aacttgaata agaagtaagt agcataaatc 3660

agtatttaac ctaaaattac atatttgaaa cagaagatat tatgttatgc tcagtaaata 3720

attaagagat ggcattgtgt aagaaggagc cctagactga aagtcaagac atctgaattt 3780

caggctggaa aactatcagt atgatctcag cctcagttct cttgtctgta aaatggaaga 3840

actggattag gcagtttgta agattcctcc taactttcac agtcgatgac aagattgtct 3900

ttttatctga tattttgaag ggtatattgc tttgaagtaa gtctcaataa ggcaatatat 3960

tttagggcat ctttcttctt atctctgaca gtgttcttaa aattatttga atatcataag 4020

agccttggtg tctgtcctaa ttcctttctc actcaccgat gctgaatacc cagttgaatc 4080

aaactgtcaa cctaccaaaa acgatattgt ggcttatggg tattgctgtc tcattcttgg 4140

tatattcttg tgttaactgc ccattggcct gaaaatactc attgtaagcc tgaaaaaaaa 4200

aatctttccc actgtttttt ctgcttgttg taagaatcaa atgaaataat gtatgtgaaa 4260

gcaccttgta aactgtaacc tatcaatgta aaatgttaag gtgtgttgtt atttcattaa 4320

ttacttcttt gtttagaatg gaatttccta tgcactactg tagctaggaa atgctgaaaa 4380

caactgtgtt ttttaattaa tcaataactg caaaattaaa gtaccttcaa tggataagac 4440

aaaaaaaaaa aaaaaaaa 4458

<210> 118
<211> 4254
<212> DNA
<213> Homo sapiens

<400> 118
taaagagaga aagccactag gctctctcat gctgctacta tctagttcta taagcttata 60

taaagacaga aatgaagcaa gactgatttc ttcacagaat aatgccggac ttgaagattt 120

tgaaaggaaa aaaacccttg gaacaggttc atttggaaga gtcatgttgg taaaacacaa 180

agccactgaa cagtattatg ccatgaagat cttagataag cagaaggttg ttaaactgaa 240

gcaaatagag catactttga atgagaaaag aatattacag gcagtgaatt ttcctttcct 300

tgttcgactg gagtatgctt ttaaggataa ttctaattta tacatggtta tggaatatgt 360

ccctgggggt gaaatgtttt cacatctaag aagaattgga aggttcagtg agccccatgc 420

acggttctat gcagctcaga tagtgctaac attcgagtac ctccattcac tagacctcat 480

ctacagagat ctaaaacctg aaaatctctt aattgaccat caaggctata tccaggtcac 540

agactttggg tttgccaaaa gagttaaagg cagaacttgg acattatgtg gaactccaga 600

gtatttggct ccagaaataa ttctcagcaa gggctacaat aaggcagtgg attggtgggc 660

attaggagtg ctaatctatg aaatggcagc tggctatccc ccattctttg cagaccaacc 720

aattcagatt tatgaaaaga ttgtttctgg aaaggtccga ttcccatccc acttcagttc 780

agatctcaag gaccttctac ggaacctgct gcaggtggat ttgaccaaga gatttggaaa 840

tctaaagaat ggtgtcagtg atataaaaac tcacaagtgg tttgccacga cagattggat 900

tgctatttac cagaggaagg ttgaagctcc attcatacca aagtttagag gctctggaga 960

taccagcaac tttgatgact atgaagaaga agatatccgt gtctctataa cagaaaaatg 1020

tgcaaaagaa tttggtgaat tttaaagagg aacaagatga catctgagct cacactcagt 1080

gtttgcactc tgttgagaga taaggtagag ctgagaccgt ccttgttgaa gcagttacct 1140

agttccttca ttccaacgac tgagtgaggt ctttattgcc atcatcccgt gtgcgcactc 1200

tgcatccacc tatgtaacaa ggcaccgcta agcaagcatt gtctgtgcca taacacagta 1260

ctagaccact ttcttacttc tctttgggtt gtctttctcc tctcctatat ccatttcttc 1320

cttttccaat ttcattggtt ttctctaaac agtgctccat tttattttgt tggtgtttca 1380

gatgggcagt gttatggcta cgtgatattt gaagggaagg ataagtgttg ctttcagtag 1440

ttattgccaa tattgttgtt ggtcaatggc ttgaagataa actttctaat aattattatt 1500

tctttgagta gctcagactt ggttttgcca aaactcttgg taatttttga agatagactg 1560

tcttatcacc aaggaaattt atacaaatta agactaactt tcttggaatt cactattctg 1620

gcaataaatt ttggtagact aatacagtac agctagaccc agaaatttgg aaggctgtag 1680

atcagaggtt ctagttccct ttccctcctt ttatatcctc ctctccttga gtaatgaagt 1740

gaccagcctg tgtagtgtga caaacgtgtc tcattcagca ggaaaaacta atgatatgga 1800

tcatcaccca gattctctca cttggtacca gcatttctgt aggtattaga gaagagttct 1860

aagttttcta aaccttaact gttccttaag gattttagcc agtattttaa tagaacatga 1920

ttaatgaaag tgacaaattt taaattttct ctaatagtcc tcatcataaa ctttttaaag 1980

gaaaataagc aaactaaaaa gaacattggt ttagataaat acttatactt tgcaaagtca 2040

aaaatggctt gatttttgga aacaatatag aggtattcat atttaaatga gggtttacat 2100

ttgttttgtt ttgtaaccgt taaaaagaag ttgtttccag ctaattattg tggtgtacta 2160

tatttgtgag cctagggtag gggcactgct gcaacttctg ctttcatccc atgcctcatc 2220

aatgaggaaa gggaacaaag tgtataaaac tgccacaatt gtattttaat tttgaggtat 2280

gatattttca gatatttcat aatttctaac ctctgttctc tcagtaaaca gaatgtctga 2340

tcgatcatgc agatacaatg ttggtatttg agaggttagt ttttttccta cacttttttt 2400

tgccaactga cttaacaaca ttgctgtcag gtggaaattt caagcacttt tgcacattta 2460

gttcagtgtt tgttgagaat ccatggctta acccacttgt tttgctattt ttttctttgc 2520

ttttaatttt ccccatctga ttttatctct gcgtttcagt gacctacctt aaaacaacac 2580

acgagaagag ttaaactggg ttcattttaa tgatcaattt acctgcatat aaaatttatt 2640

tttaatcaag ctgatcttaa tgtatataat cattctattt gctttattat cggtgcaggt 2700

aggtcattaa caccacttct tttcatctgt accacaccct ggtgaaacct ttgaagacat 2760

aaaaaaaacc tgtctgagat gttctttcta ccaatctata tgtctttcgg ttatcaagtg 2820

tttctgcatg gtaatgtcat gtaaatgctg atattgattt cactggtcca tctatattta 2880

aaacgtgcaa gaaaaaaata aaatactctg ctctagcaag ttttgtgtaa caaaggcata 2940

tcgtcatgtt aataaattta aaacatcatt cgtataaaat attttaattt tcttgtattt 3000

catttagacc caagaacatg ctgaccaatg tgttctatat gtaaactaca aattctatgg 3060

tagctttgtt gtatattatt gtaaaattat tttaataagt catggggatg acaatttgat 3120

tattacaatt tagttttcag taatcaaaaa gatttctatg aattctaaaa aatatttttt 3180

tctatgaaat tactagtgcc cagctgtaga atctacctta ggtagatgat ccctagacat 3240

acgttggttt tgagggctat tcagccattc cattttactc tctatttaaa ggccgtgagc 3300

aagcttgtca tgagcaaata tgtcaaggga gtcaatttct gaccaatcaa gtacactaaa 3360

ttagaatatt tttaaagtat gtaacattcc cagtttcagc cacaatttag ccaagaataa 3420

gataaaaact tgaataagaa gtaagtagca taaatcagta tttaacctaa aattacatat 3480

ttgaaacaga agatattatg ttatgctcag taaataatta agagatggca ttgtgtaaga 3540

aggagcccta gactgaaagt caagacatct gaatttcagg ctggaaaact atcagtatga 3600

tctcagcctc agttctcttg tctgtaaaat ggaagaactg gattaggcag tttgtaagat 3660

tcctcctaac tttcacagtc gatgacaaga ttgtcttttt atctgatatt ttgaagggta 3720

tattgctttg aagtaagtct caataaggca atatatttta gggcatcttt cttcttatct 3780

ctgacagtgt tcttaaaatt atttgaatat cataagagcc ttggtgtctg tcctaattcc 3840

tttctcactc accgatgctg aatacccagt tgaatcaaac tgtcaaccta ccaaaaacga 3900

tattgtggct tatgggtatt gctgtctcat tcttggtata ttcttgtgtt aactgcccat 3960

tggcctgaaa atactcattg taagcctgaa aaaaaaaatc tttcccactg tttttttctgc 4020

ttgttgtaag aatcaaatga aataatgtat gtgaaagcac cttgtaaact gtaacctatc 4080

aatgtaaaat gttaaggtgt gttgttattt cattaattac ttctttgttt agaatggaat 4140

ttcctatgca ctactgtagc taggaaatgc tgaaaacaac tgtgtttttt aattaatcaa 4200

taactgcaaa attaaagtac cttcaatgga taagacaaaa aaaaaaaaaa aaaa 4254

<210> 119
<211> 4542
<212> DNA
<213> Homo sapiens

<400> 119
atgctgatat aattgagaac atcttataca tcctggttcg aacattttct ccctgccatt 60

ttgagttgtt ctagtggtat atgaaggagg ctgggataac tagcttgaaa gaaattcagt 120

ctagttatag acatctttgg cattaatctg atgtttacta gtgatatctc atgctaggca 180

gttatgcttt gcttctaggg gcttctcttt ttaaaacaaa agaaagctct tttcgttttc 240

tgtgtgctgc atgctccagt gtgtgtgttt acaccatcgg ttcttctccc tctagagatt 300

agcataactc cctttgctgt tggattgtta ttttgagcaa tatgttttgg aaaggttggt 360

tttcatcatg agtgcacgca aatcatcaga tgcatctgct tgctcctctt cagaaatatc 420

tgtgaaagag tttctagcca aagccaaaga agactttttg aaaaaatggg agaatccaac 480

tcagaataat gccggacttg aagattttga aaggaaaaaa acccttggaa caggttcatt 540

tggaagagtc atgttggtaa aacacaaagc cactgaacag tattatgcca tgaagatctt 600

agataagcag aaggataatt ctaatttata catggttatg gaatatgtcc ctgggggtga 660

aatgttttca catctaagaa gaattggaag gttcagtgag ccccatgcac ggttctatgc 720

agctcagata gtgctaacat tcgagtacct ccattcacta gacctcatct acagagatct 780

aaaacctgaa aatctcttaa ttgaccatca aggctatatc caggtcacag actttgggtt 840

tgccaaaaga gttaaaggca gaacttggac attatgtgga actccagagt atttggctcc 900

agaaataatt ctcagcaagg gctacaataa ggcagtggat tggtgggcat taggagtgct 960

aatctatgaa atggcagctg gctatccccc attctttgca gaccaaccaa ttcagattta 1020

tgaaaagatt gtttctggaa aggtccgatt cccatcccac ttcagttcag atctcaagga 1080

ccttctacgg aacctgctgc aggtggattt gaccaagaga tttggaaatc taaagaatgg 1140

tgtcagtgat ataaaaactc acaagtggtt tgccacgaca gattggattg ctatttacca 1200

gaggaaggtt gaagctccat tcataccaaa gtttagaggc tctggagata ccagcaactt 1260

tgatgactat gaagaagaag atatccgtgt ctctataaca gaaaaatgtg caaaagaatt 1320

tggtgaattt taaagaggaa caagatgaca tctgagctca cactcagtgt ttgcactctg 1380

ttgagagata aggtagagct gagaccgtcc ttgttgaagc agttacctag ttccttcatt 1440

ccaacgactg agtgaggtct ttattgccat catcccgtgt gcgcactctg catccaccta 1500

tgtaacaagg caccgctaag caagcattgt ctgtgccata acacagtact agaccacttt 1560

cttacttctc tttgggttgt ctttctcctc tcctatatcc atttcttcct tttccaattt 1620

cattggtttt ctctaaacag tgctccattt tattttgttg gtgtttcaga tgggcagtgt 1680

tatggctacg tgatatttga agggaaggat aagtgttgct ttcagtagtt attgccaata 1740

ttgttgttgg tcaatggctt gaagataaac tttctaataa ttattatttc tttgagtagc 1800

tcagacttgg ttttgccaaa actcttggta atttttgaag atagactgtc ttatcaccaa 1860

ggaaatttat acaaattaag actaactttc ttggaattca ctattctggc aataaatttt 1920

ggtagactaa tacagtacag ctagacccag aaatttggaa ggctgtagat cagaggttct 1980

agttcccttt ccctcctttt atatcctcct ctccttgagt aatgaagtga ccagcctgtg 2040

tagtgtgaca aacgtgtctc attcagcagg aaaaactaat gatatggatc atcacccaga 2100

ttctctcact tggtaccagc atttctgtag gtattagaga agagttctaa gttttctaaa 2160

ccttaactgt tccttaagga ttttagccag tattttaata gaacatgatt aatgaaagtg 2220

acaaatttta aattttctct aatagtcctc atcataaact ttttaaagga aaataagcaa 2280

actaaaaaga acattggttt agataaatac ttatactttg caaagtcaaa aatggcttga 2340

tttttggaaa caatatagag gtattcatat ttaaatgagg gtttacattt gttttgtttt 2400

gtaaccgtta aaaagaagtt gtttccagct aattattgtg gtgtactata tttgtgagcc 2460

tagggtaggg gcactgctgc aacttctgct ttcatcccat gcctcatcaa tgaggaaagg 2520

gaacaaagtg tataaaactg ccacaattgt attttaattt tgaggtatga tattttcaga 2580

tatttcataa tttctaacct ctgttctctc agtaaacaga atgtctgatc gatcatgcag 2640

atacaatgtt ggtatttgag aggttagttt ttttcctaca cttttttttg ccaactgact 2700

taacaacatt gctgtcaggt ggaaatttca agcacttttg cacatttagt tcagtgtttg 2760

ttgagaatcc atggcttaac ccacttgttt tgctattttt ttctttgctt ttaattttcc 2820

ccatctgatt ttatctctgc gtttcagtga cctaccttaa aacaacacac gagaagagtt 2880

aaactgggtt cattttaatg atcaatttac ctgcatataa aatttatttt taatcaagct 2940

gatcttaatg tatataatca ttctatttgc tttattatcg gtgcaggtag gtcattaaca 3000

ccacttcttt tcatctgtac cacaccctgg tgaaaccttt gaagacataa aaaaaacctg 3060

tctgagatgt tctttctacc aatctatatg tctttcggtt atcaagtgtt tctgcatggt 3120

aatgtcatgt aaatgctgat attgatttca ctggtccatc tatatttaaa acgtgcaaga 3180

aaaaaataaa atactctgct ctagcaagtt ttgtgtaaca aaggcatatc gtcatgttaa 3240

taaatttaaa acatcattcg tataaaatat tttaattttc ttgtatttca tttagaccca 3300

agaacatgct gaccaatgtg ttctatatgt aaactacaaa ttctatggta gctttgttgt 3360

atattattgt aaaattattt taataagtca tggggatgac aatttgatta ttacaattta 3420

gttttcagta atcaaaaaga tttctatgaa ttctaaaaaa tatttttttc tatgaaatta 3480

ctagtgccca gctgtagaat ctaccttagg tagatgatcc ctagacatac gttggttttg 3540

agggctattc agccattcca ttttactctc tatttaaagg ccgtgagcaa gcttgtcatg 3600

agcaaatatg tcaagggagt caatttctga ccaatcaagt acactaaatt agaatatttt 3660

taaagtatgt aacattccca gtttcagcca caatttagcc aagaataaga taaaaacttg 3720

aataagaagt aagtagcata aatcagtatt taacctaaaa ttacatattt gaaacagaag 3780

atattatgtt atgctcagta aataattaag agatggcatt gtgtaagaag gagccctaga 3840

ctgaaagtca agacatctga atttcaggct ggaaaactat cagtatgatc tcagcctcag 3900

ttctcttgtc tgtaaaatgg aagaactgga ttaggcagtt tgtaagattc ctcctaactt 3960

tcacagtcga tgacaagatt gtctttttat ctgatatttt gaagggtata ttgctttgaa 4020

gtaagtctca ataaggcaat atattttagg gcatctttct tcttatctct gacagtgttc 4080

ttaaaattat ttgaatatca taagagcctt ggtgtctgtc ctaattcctt tctcactcac 4140

cgatgctgaa tacccagttg aatcaaactg tcaacctacc aaaaacgata ttgtggctta 4200

tgggtattgc tgtctcattc ttggtatatt cttgtgttaa ctgcccattg gcctgaaaat 4260

actcattgta agcctgaaaa aaaaaatctt tcccactgtt ttttctgctt gttgtaagaa 4320

tcaaatgaaa taatgtatgt gaaagcacct tgtaaactgt aacctatcaa tgtaaaatgt 4380

taaggtgtgt tgttatttca ttaattactt ctttgtttag aatggaattt cctatgcact 4440

actgtagcta ggaaatgctg aaaacaactg tgttttttaa ttaatcaata actgcaaaat 4500

taaagtacct tcaatggata agacaaaaaa aaaaaaaaaa aa 4542

<210> 120
<211> 2055
<212> DNA
<213> Homo sapiens

<400> 120
gccattttga gttgttctag tggtatatga aggaggctgg gataactagc ttgaaagaaa 60

ttcagtctag ttatagacat ctttggcatt aatctgatgt ttactagtga tatctcatgc 120

taggcagtta tgctttgctt ctaggggctt ctctttttaa aacaaaagaa agctcttttc 180

gttttctgtg tgctgcatgc tccagtgtgt gtgtttacac catcggttct tctccctcta 240

gagattagca taactccctt tgctgttgga ttgttatttt gagcaatatg ttttggaaag 300

gttggttttc atcatgagtg cacgcaaatc atcagatgca tctgcttgct cctcttcaga 360

aatatctgat tcctttgtga aagagtttct agccaaagcc aaagaagact ttttgaaaaa 420

atgggagaat ccaactcaga ataatgccgg acttgaagat tttgaaagga aaaaaaccct 480

tggaacaggt tcatttggaa gagtcatgtt ggtaaaacac aaagccactg aacagtatta 540

tgccatgaag atcttagata agcagaaggt tgttaaactg aagcaaatag agcatacttt 600

gaatgagaaa agaatattac aggcagtgaa ttttcctttc cttgttcgac tggagtatgc 660

ttttaaggat aattctaatt tatacatggt tatggaatat gtccctgggg gtgaaatgtt 720

ttcacatcta agaagaattg gaaggttcag tgagccccat gcacggttct atgcagctca 780

gatagtgcta acattcgagt acctccattc actagacctc atctacagag atctaaaacc 840

tgaaaatctc ttaattgacc atcaaggcta tatccaggtc acagactttg ggtttgccaa 900

aagagttaaa ggcagaactt ggacattatg tggaactcca gagtatttgg ctccagaaat 960

aattctcagc aagggctaca ataaggcagt ggattggtgg gcattaggag tgctaatcta 1020

tgaaatggca gctggctatc ccccattctt tgcagaccaa ccaattcaga tttatgaaaa 1080

gattgtttct ggaaagcaga acttttgata tgaacaaaac aaaactttga gaaaaattaa 1140

cagacaaggc agtgatttat tttgaagaa tttgagaagt gtagactctc aagaggacta 1200

aaggtcatat gaagaatgat gagagaacca aaatacatta aaatcacaaa tggaagaaga 1260

atattttact aatacaaaaa ctaagaatgt aaatgttata ataattgttt caaatcattt 1320

aattgacagt aattataaag ttcttgaatc tttactatat tacttttatt tatattcata 1380

taagaaatcc aattttctaa caaggatact gtcataacta aatttacatt tattaagaaa 1440

aactgcttta gttaaaatta atgtgtcttc atttttatgc attggcctcg atttgccaat 1500

cattctctat tggttaaatt ttatattcag ctgtttatga atatatattc attttatatc 1560

aaactttaaa attttgtatc taataatcag catatattct aaaatcataa cagtctaaat 1620

cctgggcacc ttagaagaat gacaccagaa aaccttatta tatcacaata ttctgttttc 1680

cccttcattt atttagaaat atgacaggat atttggtgta cttttgtttt ttaactaaaa 1740

gtaccagatt atctctcccc atgtgggata taaaattatc cccatctctt actcccttta 1800

ctcatctaaa gtagaagtca tgaaagtgga atttttgcca ttaaaaggct ctgtattatg 1860

tgaagttaga ttgtattaac catttcccaa taaatcatct gtttcaaaac tcaaattcaa 1920

actagaatgt gtctctattc acattgcaaa aatattattg tctctctggt tagtggctaa 1980

aagccaaatt ggaaactaac tagtttttta aattttttaa attgtgcaaa ttattaaaaa 2040

tccaatttgg tctta 2055

<210> 121
<211> 1968
<212> DNA
<213> Homo sapiens

<400> 121
actgctgctg ccaccgccgt cgccgccgcc gccgccgccg ccgctgctgc tgccggtgct 60

aaggagttcg ctggagccct ttcctcagac ccggcccggt cttcgcgccc ggactcctgg 120
cgccagcgct aggcgcactc accgctctga cgggtgcaga cgcgggagtt gtcccagact 180
gtggagtggc gggcacggcc ccagcccccc ttcccttccc tgaccccttc ttgccatcgc 240
cccagacatg gggaacgcgg cgaccgccaa gaaaggcagc gaggtggaga gcgtgaaaga 300
gtttctagcc aaagccaaag aagacttttt gaaaaaatgg gagaatccaa ctcagaataa 360
tgccggactt gaagattttg aaaggaaaaa aacccttgga acaggttcat ttggaagagt 420
catgttggta aaacacaaag ccactgaaca gtattatgcc atgaagatct tagataagca 480
gaaggttgtt aaactgaagc aaatagagca tactttgaat gagaaaagaa tattacaggc 540
agtgaatttt cctttccttg ttcgactgga gtatgctttt aaggataatt ctaatttata 600
catggttatg gaatatgtcc ctgggggtga aatgttttca catctaagaa gaattggaag 660
gttcagtgag ccccatgcac ggttctatgc agctcagata gtgctaacat tcgagtacct 720
ccattcacta gacctcatct acagagatct aaaacctgaa aatctcttaa ttgaccatca 780
aggctatatc caggtcacag actttgggtt tgccaaaaga gttaaaggca gaacttggac 840
attatgtgga actccagagt atttggctcc agaaataatt ctcagcaagg gctacaataa 900
ggcagtggat tggtgggcat taggagtgct aatctatgaa atggcagctg gctatccccc 960
attctttgca gaccaaccaa ttcagattta tgaaaagatt gtttctggaa agaacttttg 1020
atatgaacaa aacaaaactt tgagaaaaat taacagacaa ggcagtgatt tatttttgaa 1080
gaatttgaga agtgtagact ctcaagagga ctaaaggtca tatgaagaat gatgagagaa 1140
ccaaaataca ttaaaatcac aaatggaaga agaatatttt actaatacaa aaactaagaa 1200
tgtaaatgtt ataataattg tttcaaatca tttaattgac agtaattata aagttcttga 1260
atctttacta tattactttt atttatattc atataagaaa tccaattttc taacaaggat 1320
actgtcataa ctaaatttac atttattaag aaaaactgct ttagttaaaa ttaatgtgtc 1380
ttcattttta tgcattggcc tcgatttgcc aatcattctc tattggttaa attttatatt 1440
cagctgttta tgaatatata ttcattttat atcaaacttt aaaattttgt atctaataat 1500
cagcatatat tctaaaatca taacagtcta aatcctgggc accttagaag aatgacacca 1560
gaaaacctta ttatatcaca atattctgtt ttccccttca tttatttaga aatatgacag 1620
gatatttggt gtacttttgt tttttaacta aaagtaccag attatctctc cccatgtggg 1680
atataaaatt atccccatct cttactccct ttactcatct aaagtagaag tcatgaaagt 1740
ggaatttttg ccattaaaag gctctgtatt atgtgaagtt agattgtatt aaccatttcc 1800
caataaatca tctgtttcaa aactcaaatt caaactagaa tgtgtctcta ttcacattgc 1860
aaaaatatta ttgtctctct ggttagtggc taaaagccaa attggaaact aactagtttt 1920

ttaaattttt taaattgtgc aaattattaa aaatccaatt tggtctta 1968

<210> 122
<211> 4515
<212> DNA
<213> Homo sapiens

<400> 122
taacacagaa tgtttaaatg gttaacattt gtgctgcagt atagctttct ggctcatgaa 60

aaatgaaagc tatcagcgat ctcggcaata agattcatcg ccaatagtca ctagcaacag 120

cacacagcat tttaatatca gtgaggtcca cagctagcag taagagctgg tgtaattgaa 180

agacgtttag gtgcaatcat tctgctgttt gctccttgcc aggttcaaca tgggattgtc 240

acgcaaatca tcagatgcat ctgcttgctc ctcttcagaa atatctgatt cctttgtgaa 300

agagtttcta gccaaagcca aagaagactt tttgaaaaaa tgggagaatc caactcagaa 360

taatgccgga cttgaagatt ttgaaaggaa aaaaaccctt ggaacaggtt catttggaag 420

agtcatgttg gtaaaacaca aagccactga acagtattat gccatgaaga tcttagataa 480

gcagaaggtt gttaaactga agcaaataga gcatactttg aatgagaaaa gaatattaca 540

ggcagtgaat tttcctttcc ttgttcgact ggagtatgct tttaaggata attctaattt 600

atacatggtt atggaatatg tccctggggg tgaaatgttt tcacatctaa gaagaattgg 660

aaggttcagt gagccccatg cacggttcta tgcagctcag atagtgctaa cattcgagta 720

cctccattca ctagacctca tctacagaga tctaaaacct gaaaatctct taattgacca 780

tcaaggctat atccaggtca cagactttgg gtttgccaaa agagttaaag gcagaacttg 840

gacattatgt ggaactccag agtatttggc tccagaaata attctcagca agggctacaa 900

taaggcagtg gattggtggg cattaggagt gctaatctat gaaatggcag ctggctatcc 960

cccattcttt gcagaccaac caattcagat ttatgaaaag attgtttctg gaaaggtccg 1020

attcccatcc cacttcagtt cagatctcaa ggaccttcta cggaacctgc tgcaggtgga 1080

tttgaccaag agatttggaa atctaaagaa tggtgtcagt gatataaaaa ctcacaagtg 1140

gtttgccacg acagattgga ttgctattta ccagaggaag gttgaagctc cattcatacc 1200

aaagtttaga ggctctggag ataccagcaa ctttgatgac tatgaagaag aagatatccg 1260

tgtctctata acagaaaaat gtgcaaaaga atttggtgaa ttttaaagag gaacaagatg 1320

acatctgagc tcacactcag tgtttgcact ctgttgagag ataaggtaga gctgagaccg 1380

tccttgttga agcagttacc tagttccttc attccaacga ctgagtgagg tctttattgc 1440

catcatcccg tgtgcgcact ctgcatccac ctatgtaaca aggcaccgct aagcaagcat 1500

tgtctgtgcc ataacacagt actagaccac tttcttactt ctctttgggt tgtctttctc 1560

ctctcctata tccatttctt ccttttccaa tttcattggt tttctctaaa cagtgctcca 1620

ttttattttg ttggtgtttc agatgggcag tgttatggct acgtgatatt tgaagggaag 1680
gataagtgtt gctttcagta gttattgcca atattgttgt tggtcaatgg cttgaagata 1740
aactttctaa taattattat ttctttgagt agctcagact tggttttgcc aaaactcttg 1800
gtaatttttg aagatagact gtcttatcac caaggaaatt tatacaaatt aagactaact 1860
ttcttggaat tcactattct ggcaataaat tttggtagac taatacagta cagctagacc 1920
cagaaatttg gaaggctgta gatcagaggt tctagttccc tttccctcct tttatatcct 1980
cctctccttg agtaatgaag tgaccagcct gtgtagtgtg acaaacgtgt ctcattcagc 2040
aggaaaaact aatgatatgg atcatcaccc agattctctc acttggtacc agcatttctg 2100
taggtattag agaagagttc taagttttct aaaccttaac tgttccttaa ggattttagc 2160
cagtatttta atagaacatg attaatgaaa gtgacaaatt ttaaattttc tctaatagtc 2220
ctcatcataa actttttaaa ggaaaataag caaactaaaa agaacattgg tttagataaa 2280
tacttatact ttgcaaagtc aaaaatggct tgatttttgg aaacaatata gaggtattca 2340
tatttaaatg agggtttaca tttgttttgt tttgtaaccg ttaaaaagaa gttgtttcca 2400
gctaattatt gtggtgtact atatttgtga gcctagggta ggggcactgc tgcaacttct 2460
gctttcatcc catgcctcat caatgaggaa agggaacaaa gtgtataaaa ctgccacaat 2520
tgtattttaa ttttgaggta tgatattttc agatatttca taatttctaa cctctgttct 2580
ctcagtaaac agaatgtctg atcgatcatg cagatacaat gttggtattt gagaggttag 2640
tttttttcct acactttttt ttgccaactg acttaacaac attgctgtca ggtggaaatt 2700
tcaagcactt ttgcacattt agttcagtgt ttgttgagaa tccatggctt aacccacttg 2760
ttttgctatt tttttctttg cttttaattt tccccatctg attttatctc tgcgtttcag 2820
tgacctacct taaaacaaca cacgagaaga gttaaactgg gttcatttta atgatcaatt 2880
tacctgcata taaaatttat ttttaatcaa gctgatctta atgtatataa tcattctatt 2940
tgctttatta tcggtgcagg taggtcatta acaccacttc ttttcatctg taccacaccc 3000
tggtgaaacc tttgaagaca taaaaaaaac ctgtctgaga tgttctttct accaatctat 3060
atgtctttcg gttatcaagt gtttctgcat ggtaatgtca tgtaaatgct gatattgatt 3120
tcactggtcc atctatattt aaaacgtgca agaaaaaaat aaaatactct gctctagcaa 3180
gttttgtgta acaaaggcat atcgtcatgt taataaattt aaaacatcat tcgtataaaa 3240
tattttaatt ttcttgtatt tcatttagac ccaagaacat gctgaccaat gtgttctata 3300
tgtaaactac aaattctatg gtagctttgt tgtatattat tgtaaaatta ttttaataag 3360
tcatggggat gacaatttga ttattacaat ttagttttca gtaatcaaaa agatttctat 3420
gaattctaaa aaatattttt ttctatgaaa ttactagtgc ccagctgtag aatctacctt 3480
aggtagatga tccctagaca tacgttggtt ttgagggcta ttcagccatt ccattttact 3540

ctctatttaa aggccgtgag caagcttgtc atgagcaaat atgtcaaggg agtcaatttc 3600

tgaccaatca agtacactaa attagaatat ttttaaagta tgtaacattc ccagtttcag 3660

ccacaattta gccaagaata agataaaaac ttgaataaga agtaagtagc ataaatcagt 3720

atttaaccta aaattacata tttgaaacag aagatattat gttatgctca gtaaataatt 3780

aagagatggc attgtgtaag aaggagccct agactgaaag tcaagacatc tgaatttcag 3840

gctggaaaac tatcagtatg atctcagcct cagttctctt gtctgtaaaa tggaagaact 3900

ggattaggca gtttgtaaga ttcctcctaa ctttcacagt cgatgacaag attgtctttt 3960

tatctgatat tttgaagggt atattgcttt gaagtaagtc tcaataaggc aatatatttt 4020

agggcatctt tcttcttatc tctgacagtg ttcttaaaat tatttgaata tcataagagc 4080

cttggtgtct gtcctaattc ctttctcact caccgatgct gaatacccag ttgaatcaaa 4140

ctgtcaacct accaaaaacg atattgtggc ttatgggtat tgctgtctca ttcttggtat 4200

attcttgtgt taactgccca ttggcctgaa aatactcatt gtaagcctga aaaaaaaaat 4260

ctttcccact gtttttctg cttgttgtaa gaatcaaatg aaataatgta tgtgaaagca 4320

ccttgtaaac tgtaacctat caatgtaaaa tgttaaggtg tgttgttatt tcattaatta 4380

cttctttgtt tagaatggaa tttcctatgc actactgtag ctaggaaatg ctgaaaacaa 4440

ctgtgttttt taattaatca ataactgcaa aattaaagta ccttcaatgg ataagacaaa 4500

aaaaaaaaaa aaaaa 4515

<210> 123
<211> 1793
<212> DNA
<213> Homo sapiens

<400> 123
acacagcatt ttaatatcag tgaggtccac agctagcagt aagagctggt gtaattgaaa 60

gacgtttagg tgcaatcatt ctgctgtttg ctccttgcca ggttcaacat gggattgttg 120

aaagagtttc tagccaaagc caaagaagac tttttgaaaa aatgggagaa tccaactcag 180

aataatgccg gacttgaaga ttttgaaagg aaaaaaaccc ttggaacagg ttcatttgga 240

agagtcatgt tggtaaaaca caaagccact gaacagtatt atgccatgaa gatcttagat 300

aagcagaagg ttgttaaact gaagcaaata gagcatactt tgaatgagaa aagaatatta 360

caggcagtga attttccttt ccttgttcga ctggagtatg cttttaagga taattctaat 420

ttatacatgg ttatggaata tgtccctggg ggtgaaatgt tttcacatct aagaagaatt 480

ggaaggttca gtgagcccca tgcacggttc tatgcagctc agatagtgct aacattcgag 540

tacctccatt cactagacct catctacaga gatctaaaac ctgaaaatct cttaattgac 600

catcaaggct atatccaggt cacagacttt gggtttgcca aaagagttaa aggcagaact 660

```
tggacattat gtggaactcc agagtatttg gctccagaaa taattctcag caagggctac      720
aataaggcag tggattggtg ggcattagga gtgctaatct atgaaatggc agctggctat      780
cccccattct ttgcagacca accaattcag atttatgaaa agattgtttc tggaaagaac      840
ttttgatatg aacaaaacaa aactttgaga aaaattaaca gacaaggcag tgatttattt      900
ttgaagaatt tgagaagtgt agactctcaa gaggactaaa ggtcatatga agaatgatga      960
gagaaccaaa atacattaaa atcacaaatg gaagaagaat attttactaa tacaaaaact     1020
aagaatgtaa atgttataat aattgtttca aatcatttaa ttgacagtaa ttataaagtt     1080
cttgaatctt tactatatta cttttattta tattcatata agaaatccaa ttttctaaca     1140
aggatactgt cataactaaa tttacattta ttaagaaaaa ctgctttagt taaaattaat     1200
gtgtcttcat ttttatgcat tggcctcgat ttgccaatca ttctctattg gttaaatttt     1260
atattcagct gtttatgaat atatattcat tttatatcaa actttaaaat tttgtatcta     1320
ataatcagca tatattctaa aatcataaca gtctaaatcc tgggcacctt agaagaatga     1380
caccagaaaa ccttattata tcacaatatt ctgttttccc cttcatttat ttagaaatat     1440
gacaggatat ttggtgtact tttgtttttt aactaaaagt accagattat ctctccccat     1500
gtgggatata aaattatccc catctcttac tccctttact catctaaagt agaagtcatg     1560
aaagtggaat ttttgccatt aaaaggctct gtattatgtg aagttagatt gtattaacca     1620
tttcccaata aatcatctgt ttcaaaactc aaattcaaac tagaatgtgt ctctattcac     1680
attgcaaaaa tattattgtc tctctggtta gtggctaaaa gccaaattgg aaactaacta     1740
gtttttaaaa ttttttaaat tgtgcaaatt attaaaaatc caatttggtc tta            1793
```

<210> 124
<211> 351
<212> PRT
<213> Homo sapiens

<400> 124

```
Met Gly Asn Ala Ala Thr Ala Lys Lys Gly Ser Glu Val Glu Ser Val
1               5                   10                  15

Lys Glu Phe Leu Ala Lys Ala Lys Glu Asp Phe Leu Lys Lys Trp Glu
            20                  25                  30

Asn Pro Thr Gln Asn Asn Ala Gly Leu Glu Asp Phe Glu Arg Lys Lys
        35                  40                  45

Thr Leu Gly Thr Gly Ser Phe Gly Arg Val Met Leu Val Lys His Lys
    50                  55                  60
```

```
Ala Thr Glu Gln Tyr Tyr Ala Met Lys Ile Leu Asp Lys Gln Lys Val
65                  70                  75                  80

Val Lys Leu Lys Gln Ile Glu His Thr Leu Asn Glu Lys Arg Ile Leu
                85                  90                  95

Gln Ala Val Asn Phe Pro Phe Leu Val Arg Leu Glu Tyr Ala Phe Lys
            100                 105                 110

Asp Asn Ser Asn Leu Tyr Met Val Met Glu Tyr Val Pro Gly Gly Glu
        115                 120                 125

Met Phe Ser His Leu Arg Arg Ile Gly Arg Phe Ser Glu Pro His Ala
    130                 135                 140

Arg Phe Tyr Ala Ala Gln Ile Val Leu Thr Phe Glu Tyr Leu His Ser
145                 150                 155                 160

Leu Asp Leu Ile Tyr Arg Asp Leu Lys Pro Glu Asn Leu Leu Ile Asp
                165                 170                 175

His Gln Gly Tyr Ile Gln Val Thr Asp Phe Gly Phe Ala Lys Arg Val
            180                 185                 190

Lys Gly Arg Thr Trp Thr Leu Cys Gly Thr Pro Glu Tyr Leu Ala Pro
        195                 200                 205

Glu Ile Ile Leu Ser Lys Gly Tyr Asn Lys Ala Val Asp Trp Trp Ala
    210                 215                 220

Leu Gly Val Leu Ile Tyr Glu Met Ala Ala Gly Tyr Pro Pro Phe Phe
225                 230                 235                 240

Ala Asp Gln Pro Ile Gln Ile Tyr Glu Lys Ile Val Ser Gly Lys Val
                245                 250                 255

Arg Phe Pro Ser His Phe Ser Ser Asp Leu Lys Asp Leu Leu Arg Asn
            260                 265                 270

Leu Leu Gln Val Asp Leu Thr Lys Arg Phe Gly Asn Leu Lys Asn Gly
        275                 280                 285

Val Ser Asp Ile Lys Thr His Lys Trp Phe Ala Thr Thr Asp Trp Ile
    290                 295                 300

Ala Ile Tyr Gln Arg Lys Val Glu Ala Pro Phe Ile Pro Lys Phe Arg
305                 310                 315                 320
```

Gly Ser Gly Asp Thr Ser Asn Phe Asp Asp Tyr Glu Glu Glu Asp Ile
325 330 335

Arg Val Ser Ile Thr Glu Lys Cys Ala Lys Glu Phe Gly Glu Phe
340 345 350

<210> 125
<211> 398
<212> PRT
<213> Homo sapiens

<400> 125

Met Ala Ala Tyr Arg Glu Pro Pro Cys Asn Gln Tyr Thr Gly Thr Thr
1 5 10 15

Thr Ala Leu Gln Lys Leu Glu Gly Phe Ala Ser Arg Leu Phe His Arg
20 25 30

His Ser Lys Gly Thr Ala His Asp Gln Lys Thr Ala Leu Glu Asn Asp
35 40 45

Ser Leu His Phe Ser Glu His Thr Ala Leu Trp Asp Arg Ser Met Lys
50 55 60

Glu Phe Leu Ala Lys Ala Lys Glu Asp Phe Leu Lys Lys Trp Glu Asn
65 70 75 80

Pro Thr Gln Asn Asn Ala Gly Leu Glu Asp Phe Glu Arg Lys Lys Thr
85 90 95

Leu Gly Thr Gly Ser Phe Gly Arg Val Met Leu Val Lys His Lys Ala
100 105 110

Thr Glu Gln Tyr Tyr Ala Met Lys Ile Leu Asp Lys Gln Lys Val Val
115 120 125

Lys Leu Lys Gln Ile Glu His Thr Leu Asn Glu Lys Arg Ile Leu Gln
130 135 140

Ala Val Asn Phe Pro Phe Leu Val Arg Leu Glu Tyr Ala Phe Lys Asp
145 150 155 160

Asn Ser Asn Leu Tyr Met Val Met Glu Tyr Val Pro Gly Gly Glu Met
165 170 175

Phe Ser His Leu Arg Arg Ile Gly Arg Phe Ser Glu Pro His Ala Arg
180 185 190

Phe Tyr Ala Ala Gln Ile Val Leu Thr Phe Glu Tyr Leu His Ser Leu
        195                 200                 205

Asp Leu Ile Tyr Arg Asp Leu Lys Pro Glu Asn Leu Leu Ile Asp His
    210                 215                 220

Gln Gly Tyr Ile Gln Val Thr Asp Phe Gly Phe Ala Lys Arg Val Lys
225                 230                 235                 240

Gly Arg Thr Trp Thr Leu Cys Gly Thr Pro Glu Tyr Leu Ala Pro Glu
                245                 250                 255

Ile Ile Leu Ser Lys Gly Tyr Asn Lys Ala Val Asp Trp Trp Ala Leu
            260                 265                 270

Gly Val Leu Ile Tyr Glu Met Ala Ala Gly Tyr Pro Pro Phe Phe Ala
        275                 280                 285

Asp Gln Pro Ile Gln Ile Tyr Glu Lys Ile Val Ser Gly Lys Val Arg
    290                 295                 300

Phe Pro Ser His Phe Ser Ser Asp Leu Lys Asp Leu Leu Arg Asn Leu
305                 310                 315                 320

Leu Gln Val Asp Leu Thr Lys Arg Phe Gly Asn Leu Lys Asn Gly Val
                325                 330                 335

Ser Asp Ile Lys Thr His Lys Trp Phe Ala Thr Thr Asp Trp Ile Ala
            340                 345                 350

Ile Tyr Gln Arg Lys Val Glu Ala Pro Phe Ile Pro Lys Phe Arg Gly
        355                 360                 365

Ser Gly Asp Thr Ser Asn Phe Asp Asp Tyr Glu Glu Glu Asp Ile Arg
    370                 375                 380

Val Ser Ile Thr Glu Lys Cys Ala Lys Glu Phe Gly Glu Phe
385                 390                 395

<210> 126
<211> 357
<212> PRT
<213> Homo sapiens

<400> 126

Met Ser Ala Arg Lys Ser Ser Asp Ala Ser Ala Cys Ser Ser Ser Glu
1               5                   10                  15

```
Ile Ser Asp Ser Phe Val Lys Glu Phe Leu Ala Lys Ala Lys Glu Asp
            20                  25                  30

Phe Leu Lys Lys Trp Glu Asn Pro Thr Gln Asn Asn Ala Gly Leu Glu
        35                  40                  45

Asp Phe Glu Arg Lys Lys Thr Leu Gly Thr Gly Ser Phe Gly Arg Val
    50                  55                  60

Met Leu Val Lys His Lys Ala Thr Glu Gln Tyr Tyr Ala Met Lys Ile
65                  70                  75                  80

Leu Asp Lys Gln Lys Val Val Lys Leu Lys Gln Ile Glu His Thr Leu
                85                  90                  95

Asn Glu Lys Arg Ile Leu Gln Ala Val Asn Phe Pro Phe Leu Val Arg
            100                 105                 110

Leu Glu Tyr Ala Phe Lys Asp Asn Ser Asn Leu Tyr Met Val Met Glu
        115                 120                 125

Tyr Val Pro Gly Gly Glu Met Phe Ser His Leu Arg Arg Ile Gly Arg
    130                 135                 140

Phe Ser Glu Pro His Ala Arg Phe Tyr Ala Ala Gln Ile Val Leu Thr
145                 150                 155                 160

Phe Glu Tyr Leu His Ser Leu Asp Leu Ile Tyr Arg Asp Leu Lys Pro
                165                 170                 175

Glu Asn Leu Leu Ile Asp His Gln Gly Tyr Ile Gln Val Thr Asp Phe
            180                 185                 190

Gly Phe Ala Lys Arg Val Lys Gly Arg Thr Trp Thr Leu Cys Gly Thr
        195                 200                 205

Pro Glu Tyr Leu Ala Pro Glu Ile Ile Leu Ser Lys Gly Tyr Asn Lys
    210                 215                 220

Ala Val Asp Trp Trp Ala Leu Gly Val Leu Ile Tyr Glu Met Ala Ala
225                 230                 235                 240

Gly Tyr Pro Pro Phe Phe Ala Asp Gln Pro Ile Gln Ile Tyr Glu Lys
                245                 250                 255

Ile Val Ser Gly Lys Val Arg Phe Pro Ser His Phe Ser Ser Asp Leu
            260                 265                 270
```

Lys Asp Leu Leu Arg Asn Leu Leu Gln Val Asp Leu Thr Lys Arg Phe
275 280 285

Gly Asn Leu Lys Asn Gly Val Ser Asp Ile Lys Thr His Lys Trp Phe
290 295 300

Ala Thr Thr Asp Trp Ile Ala Ile Tyr Gln Arg Lys Val Glu Ala Pro
305 310 315 320

Phe Ile Pro Lys Phe Arg Gly Ser Gly Asp Thr Ser Asn Phe Asp Asp
325 330 335

Tyr Glu Glu Glu Asp Ile Arg Val Ser Ile Thr Glu Lys Cys Ala Lys
340 345 350

Glu Phe Gly Glu Phe
355

<210> 127
<211> 355
<212> PRT
<213> Homo sapiens

<400> 127

Met Gly Leu Ser Arg Lys Ser Ser Asp Ala Ser Ala Cys Ser Ser Ser
1 5 10 15

Glu Ile Ser Val Lys Glu Phe Leu Ala Lys Ala Lys Glu Asp Phe Leu
20 25 30

Lys Lys Trp Glu Asn Pro Thr Gln Asn Asn Ala Gly Leu Glu Asp Phe
35 40 45

Glu Arg Lys Lys Thr Leu Gly Thr Gly Ser Phe Gly Arg Val Met Leu
50 55 60

Val Lys His Lys Ala Thr Glu Gln Tyr Tyr Ala Met Lys Ile Leu Asp
65 70 75 80

Lys Gln Lys Val Val Lys Leu Lys Gln Ile Glu His Thr Leu Asn Glu
85 90 95

Lys Arg Ile Leu Gln Ala Val Asn Phe Pro Phe Leu Val Arg Leu Glu
100 105 110

Tyr Ala Phe Lys Asp Asn Ser Asn Leu Tyr Met Val Met Glu Tyr Val
115 120 125

```
Pro Gly Gly Glu Met Phe Ser His Leu Arg Arg Ile Gly Arg Phe Ser
    130                 135                 140

Glu Pro His Ala Arg Phe Tyr Ala Ala Gln Ile Val Leu Thr Phe Glu
145                 150                 155                 160

Tyr Leu His Ser Leu Asp Leu Ile Tyr Arg Asp Leu Lys Pro Glu Asn
                165                 170                 175

Leu Leu Ile Asp His Gln Gly Tyr Ile Gln Val Thr Asp Phe Gly Phe
            180                 185                 190

Ala Lys Arg Val Lys Gly Arg Thr Trp Thr Leu Cys Gly Thr Pro Glu
        195                 200                 205

Tyr Leu Ala Pro Glu Ile Ile Leu Ser Lys Gly Tyr Asn Lys Ala Val
    210                 215                 220

Asp Trp Trp Ala Leu Gly Val Leu Ile Tyr Glu Met Ala Ala Gly Tyr
225                 230                 235                 240

Pro Pro Phe Phe Ala Asp Gln Pro Ile Gln Ile Tyr Glu Lys Ile Val
                245                 250                 255

Ser Gly Lys Val Arg Phe Pro Ser His Phe Ser Ser Asp Leu Lys Asp
            260                 265                 270

Leu Leu Arg Asn Leu Leu Gln Val Asp Leu Thr Lys Arg Phe Gly Asn
        275                 280                 285

Leu Lys Asn Gly Val Ser Asp Ile Lys Thr His Lys Trp Phe Ala Thr
    290                 295                 300

Thr Asp Trp Ile Ala Ile Tyr Gln Arg Lys Val Glu Ala Pro Phe Ile
305                 310                 315                 320

Pro Lys Phe Arg Gly Ser Gly Asp Thr Ser Asn Phe Asp Asp Tyr Glu
                325                 330                 335

Glu Glu Asp Ile Arg Val Ser Ile Thr Glu Lys Cys Ala Lys Glu Phe
            340                 345                 350

Gly Glu Phe
        355
```

<210> 128

<211> 339
<212> PRT
<213> Homo sapiens

<400> 128

```
Met Gly Leu Leu Lys Glu Phe Leu Ala Lys Ala Lys Glu Asp Phe Leu
1               5                   10                  15

Lys Lys Trp Glu Asn Pro Thr Gln Asn Asn Ala Gly Leu Glu Asp Phe
            20                  25                  30

Glu Arg Lys Lys Thr Leu Gly Thr Gly Ser Phe Gly Arg Val Met Leu
        35                  40                  45

Val Lys His Lys Ala Thr Glu Gln Tyr Tyr Ala Met Lys Ile Leu Asp
    50                  55                  60

Lys Gln Lys Val Val Lys Leu Lys Gln Ile Glu His Thr Leu Asn Glu
65                  70                  75                  80

Lys Arg Ile Leu Gln Ala Val Asn Phe Pro Phe Leu Val Arg Leu Glu
                85                  90                  95

Tyr Ala Phe Lys Asp Asn Ser Asn Leu Tyr Met Val Met Glu Tyr Val
            100                 105                 110

Pro Gly Gly Glu Met Phe Ser His Leu Arg Arg Ile Gly Arg Phe Ser
        115                 120                 125

Glu Pro His Ala Arg Phe Tyr Ala Ala Gln Ile Val Leu Thr Phe Glu
    130                 135                 140

Tyr Leu His Ser Leu Asp Leu Ile Tyr Arg Asp Leu Lys Pro Glu Asn
145                 150                 155                 160

Leu Leu Ile Asp His Gln Gly Tyr Ile Gln Val Thr Asp Phe Gly Phe
                165                 170                 175

Ala Lys Arg Val Lys Gly Arg Thr Trp Thr Leu Cys Gly Thr Pro Glu
            180                 185                 190

Tyr Leu Ala Pro Glu Ile Ile Leu Ser Lys Gly Tyr Asn Lys Ala Val
        195                 200                 205

Asp Trp Trp Ala Leu Gly Val Leu Ile Tyr Glu Met Ala Ala Gly Tyr
    210                 215                 220

Pro Pro Phe Phe Ala Asp Gln Pro Ile Gln Ile Tyr Glu Lys Ile Val
```

225 230 235 240

Ser Gly Lys Val Arg Phe Pro Ser His Phe Ser Ser Asp Leu Lys Asp
245 250 255

Leu Leu Arg Asn Leu Leu Gln Val Asp Leu Thr Lys Arg Phe Gly Asn
260 265 270

Leu Lys Asn Gly Val Ser Asp Ile Lys Thr His Lys Trp Phe Ala Thr
275 280 285

Thr Asp Trp Ile Ala Ile Tyr Gln Arg Lys Val Glu Ala Pro Phe Ile
290 295 300

Pro Lys Phe Arg Gly Ser Gly Asp Thr Ser Asn Phe Asp Asp Tyr Glu
305 310 315 320

Glu Glu Asp Ile Arg Val Ser Ile Thr Glu Lys Cys Ala Lys Glu Phe
325 330 335

Gly Glu Phe

<210> 129
<211> 338
<212> PRT
<213> Homo sapiens

<400> 129

Met Leu Leu Leu Ser Ser Ser Ile Ser Leu Tyr Lys Asp Arg Asn Glu
1 5 10 15

Ala Arg Leu Ile Ser Ser Gln Asn Asn Ala Gly Leu Glu Asp Phe Glu
20 25 30

Arg Lys Lys Thr Leu Gly Thr Gly Ser Phe Gly Arg Val Met Leu Val
35 40 45

Lys His Lys Ala Thr Glu Gln Tyr Tyr Ala Met Lys Ile Leu Asp Lys
50 55 60

Gln Lys Val Val Lys Leu Lys Gln Ile Glu His Thr Leu Asn Glu Lys
65 70 75 80

Arg Ile Leu Gln Ala Val Asn Phe Pro Phe Leu Val Arg Leu Glu Tyr
85 90 95

Ala Phe Lys Asp Asn Ser Asn Leu Tyr Met Val Met Glu Tyr Val Pro

```
                100                 105                 110

Gly Gly Glu Met Phe Ser His Leu Arg Arg Ile Gly Arg Phe Ser Glu
            115                 120                 125

Pro His Ala Arg Phe Tyr Ala Ala Gln Ile Val Leu Thr Phe Glu Tyr
    130                 135                 140

Leu His Ser Leu Asp Leu Ile Tyr Arg Asp Leu Lys Pro Glu Asn Leu
145                 150                 155                 160

Leu Ile Asp His Gln Gly Tyr Ile Gln Val Thr Asp Phe Gly Phe Ala
                165                 170                 175

Lys Arg Val Lys Gly Arg Thr Trp Thr Leu Cys Gly Thr Pro Glu Tyr
            180                 185                 190

Leu Ala Pro Glu Ile Ile Leu Ser Lys Gly Tyr Asn Lys Ala Val Asp
        195                 200                 205

Trp Trp Ala Leu Gly Val Leu Ile Tyr Glu Met Ala Ala Gly Tyr Pro
    210                 215                 220

Pro Phe Phe Ala Asp Gln Pro Ile Gln Ile Tyr Glu Lys Ile Val Ser
225                 230                 235                 240

Gly Lys Val Arg Phe Pro Ser His Phe Ser Ser Asp Leu Lys Asp Leu
                245                 250                 255

Leu Arg Asn Leu Leu Gln Val Asp Leu Thr Lys Arg Phe Gly Asn Leu
            260                 265                 270

Lys Asn Gly Val Ser Asp Ile Lys Thr His Lys Trp Phe Ala Thr Thr
        275                 280                 285

Asp Trp Ile Ala Ile Tyr Gln Arg Lys Val Glu Ala Pro Phe Ile Pro
    290                 295                 300

Lys Phe Arg Gly Ser Gly Asp Thr Ser Asn Phe Asp Asp Tyr Glu Glu
305                 310                 315                 320

Glu Asp Ile Arg Val Ser Ile Thr Glu Lys Cys Ala Lys Glu Phe Gly
                325                 330                 335

Glu Phe
```

<210> 130
<211> 321
<212> PRT
<213> Homo sapiens

<400> 130

Met Ser Ala Arg Lys Ser Ser Asp Ala Ser Ala Cys Ser Ser Ser Glu
1 5 10 15

Ile Ser Val Lys Glu Phe Leu Ala Lys Ala Lys Glu Asp Phe Leu Lys
20 25 30

Lys Trp Glu Asn Pro Thr Gln Asn Asn Ala Gly Leu Glu Asp Phe Glu
35 40 45

Arg Lys Lys Thr Leu Gly Thr Gly Ser Phe Gly Arg Val Met Leu Val
50 55 60

Lys His Lys Ala Thr Glu Gln Tyr Tyr Ala Met Lys Ile Leu Asp Lys
65 70 75 80

Gln Lys Asp Asn Ser Asn Leu Tyr Met Val Met Glu Tyr Val Pro Gly
85 90 95

Gly Glu Met Phe Ser His Leu Arg Arg Ile Gly Arg Phe Ser Glu Pro
100 105 110

His Ala Arg Phe Tyr Ala Ala Gln Ile Val Leu Thr Phe Glu Tyr Leu
115 120 125

His Ser Leu Asp Leu Ile Tyr Arg Asp Leu Lys Pro Glu Asn Leu Leu
130 135 140

Ile Asp His Gln Gly Tyr Ile Gln Val Thr Asp Phe Gly Phe Ala Lys
145 150 155 160

Arg Val Lys Gly Arg Thr Trp Thr Leu Cys Gly Thr Pro Glu Tyr Leu
165 170 175

Ala Pro Glu Ile Ile Leu Ser Lys Gly Tyr Asn Lys Ala Val Asp Trp
180 185 190

Trp Ala Leu Gly Val Leu Ile Tyr Glu Met Ala Ala Gly Tyr Pro Pro
195 200 205

Phe Phe Ala Asp Gln Pro Ile Gln Ile Tyr Glu Lys Ile Val Ser Gly
210 215 220

Lys Val Arg Phe Pro Ser His Phe Ser Ser Asp Leu Lys Asp Leu Leu
225 230 235 240

Arg Asn Leu Leu Gln Val Asp Leu Thr Lys Arg Phe Gly Asn Leu Lys
245 250 255

Asn Gly Val Ser Asp Ile Lys Thr His Lys Trp Phe Ala Thr Thr Asp
260 265 270

Trp Ile Ala Ile Tyr Gln Arg Lys Val Glu Ala Pro Phe Ile Pro Lys
275 280 285

Phe Arg Gly Ser Gly Asp Thr Ser Asn Phe Asp Asp Tyr Glu Glu Glu
290 295 300

Asp Ile Arg Val Ser Ile Thr Glu Lys Cys Ala Lys Glu Phe Gly Glu
305 310 315 320

Phe

<210> 131
<211> 264
<212> PRT
<213> Homo sapiens

<400> 131

Met Ser Ala Arg Lys Ser Ser Asp Ala Ser Ala Cys Ser Ser Ser Glu
1 5 10 15

Ile Ser Asp Ser Phe Val Lys Glu Phe Leu Ala Lys Ala Lys Glu Asp
20 25 30

Phe Leu Lys Lys Trp Glu Asn Pro Thr Gln Asn Asn Ala Gly Leu Glu
35 40 45

Asp Phe Glu Arg Lys Lys Thr Leu Gly Thr Gly Ser Phe Gly Arg Val
50 55 60

Met Leu Val Lys His Lys Ala Thr Glu Gln Tyr Tyr Ala Met Lys Ile
65 70 75 80

Leu Asp Lys Gln Lys Val Val Lys Leu Lys Gln Ile Glu His Thr Leu
85 90 95

Asn Glu Lys Arg Ile Leu Gln Ala Val Asn Phe Pro Phe Leu Val Arg
100 105 110

Leu Glu Tyr Ala Phe Lys Asp Asn Ser Asn Leu Tyr Met Val Met Glu
115 120 125

Tyr Val Pro Gly Gly Glu Met Phe Ser His Leu Arg Arg Ile Gly Arg
130 135 140

Phe Ser Glu Pro His Ala Arg Phe Tyr Ala Ala Gln Ile Val Leu Thr
145 150 155 160

Phe Glu Tyr Leu His Ser Leu Asp Leu Ile Tyr Arg Asp Leu Lys Pro
165 170 175

Glu Asn Leu Leu Ile Asp His Gln Gly Tyr Ile Gln Val Thr Asp Phe
180 185 190

Gly Phe Ala Lys Arg Val Lys Gly Arg Thr Trp Thr Leu Cys Gly Thr
195 200 205

Pro Glu Tyr Leu Ala Pro Glu Ile Ile Leu Ser Lys Gly Tyr Asn Lys
210 215 220

Ala Val Asp Trp Trp Ala Leu Gly Val Leu Ile Tyr Glu Met Ala Ala
225 230 235 240

Gly Tyr Pro Pro Phe Phe Ala Asp Gln Pro Ile Gln Ile Tyr Glu Lys
245 250 255

Ile Val Ser Gly Lys Gln Asn Phe
260

<210> 132
<211> 257
<212> PRT
<213> Homo sapiens

<400> 132

Met Gly Asn Ala Ala Thr Ala Lys Lys Gly Ser Glu Val Glu Ser Val
1 5 10 15

Lys Glu Phe Leu Ala Lys Ala Lys Glu Asp Phe Leu Lys Lys Trp Glu
20 25 30

Asn Pro Thr Gln Asn Asn Ala Gly Leu Glu Asp Phe Glu Arg Lys Lys
35 40 45

Thr Leu Gly Thr Gly Ser Phe Gly Arg Val Met Leu Val Lys His Lys
50 55 60

```
Ala Thr Glu Gln Tyr Tyr Ala Met Lys Ile Leu Asp Lys Gln Lys Val
65                  70                  75                  80

Val Lys Leu Lys Gln Ile Glu His Thr Leu Asn Glu Lys Arg Ile Leu
                85                  90                  95

Gln Ala Val Asn Phe Pro Phe Leu Val Arg Leu Glu Tyr Ala Phe Lys
            100                 105                 110

Asp Asn Ser Asn Leu Tyr Met Val Met Glu Tyr Val Pro Gly Gly Glu
        115                 120                 125

Met Phe Ser His Leu Arg Arg Ile Gly Arg Phe Ser Glu Pro His Ala
    130                 135                 140

Arg Phe Tyr Ala Ala Gln Ile Val Leu Thr Phe Glu Tyr Leu His Ser
145                 150                 155                 160

Leu Asp Leu Ile Tyr Arg Asp Leu Lys Pro Glu Asn Leu Leu Ile Asp
                165                 170                 175

His Gln Gly Tyr Ile Gln Val Thr Asp Phe Gly Phe Ala Lys Arg Val
            180                 185                 190

Lys Gly Arg Thr Trp Thr Leu Cys Gly Thr Pro Glu Tyr Leu Ala Pro
        195                 200                 205

Glu Ile Ile Leu Ser Lys Gly Tyr Asn Lys Ala Val Asp Trp Trp Ala
    210                 215                 220

Leu Gly Val Leu Ile Tyr Glu Met Ala Ala Gly Tyr Pro Pro Phe Phe
225                 230                 235                 240

Ala Asp Gln Pro Ile Gln Ile Tyr Glu Lys Ile Val Ser Gly Lys Asn
                245                 250                 255

Phe


<210>  133
<211>  358
<212>  PRT
<213>  Homo sapiens

<400>  133

Met Gly Leu Ser Arg Lys Ser Ser Asp Ala Ser Ala Cys Ser Ser Ser
1               5                   10                  15
```

Glu Ile Ser Asp Ser Phe Val Lys Glu Phe Leu Ala Lys Ala Lys Glu
20 25 30

Asp Phe Leu Lys Lys Trp Glu Asn Pro Thr Gln Asn Asn Ala Gly Leu
35 40 45

Glu Asp Phe Glu Arg Lys Lys Thr Leu Gly Thr Gly Ser Phe Gly Arg
50 55 60

Val Met Leu Val Lys His Lys Ala Thr Glu Gln Tyr Tyr Ala Met Lys
65 70 75 80

Ile Leu Asp Lys Gln Lys Val Val Lys Leu Lys Gln Ile Glu His Thr
85 90 95

Leu Asn Glu Lys Arg Ile Leu Gln Ala Val Asn Phe Pro Phe Leu Val
100 105 110

Arg Leu Glu Tyr Ala Phe Lys Asp Asn Ser Asn Leu Tyr Met Val Met
115 120 125

Glu Tyr Val Pro Gly Gly Glu Met Phe Ser His Leu Arg Arg Ile Gly
130 135 140

Arg Phe Ser Glu Pro His Ala Arg Phe Tyr Ala Ala Gln Ile Val Leu
145 150 155 160

Thr Phe Glu Tyr Leu His Ser Leu Asp Leu Ile Tyr Arg Asp Leu Lys
165 170 175

Pro Glu Asn Leu Leu Ile Asp His Gln Gly Tyr Ile Gln Val Thr Asp
180 185 190

Phe Gly Phe Ala Lys Arg Val Lys Gly Arg Thr Trp Thr Leu Cys Gly
195 200 205

Thr Pro Glu Tyr Leu Ala Pro Glu Ile Ile Leu Ser Lys Gly Tyr Asn
210 215 220

Lys Ala Val Asp Trp Trp Ala Leu Gly Val Leu Ile Tyr Glu Met Ala
225 230 235 240

Ala Gly Tyr Pro Pro Phe Phe Ala Asp Gln Pro Ile Gln Ile Tyr Glu
245 250 255

Lys Ile Val Ser Gly Lys Val Arg Phe Pro Ser His Phe Ser Ser Asp
260 265 270

Leu Lys Asp Leu Leu Arg Asn Leu Leu Gln Val Asp Leu Thr Lys Arg
275 280 285

Phe Gly Asn Leu Lys Asn Gly Val Ser Asp Ile Lys Thr His Lys Trp
290 295 300

Phe Ala Thr Thr Asp Trp Ile Ala Ile Tyr Gln Arg Lys Val Glu Ala
305 310 315 320

Pro Phe Ile Pro Lys Phe Arg Gly Ser Gly Asp Thr Ser Asn Phe Asp
325 330 335

Asp Tyr Glu Glu Glu Asp Ile Arg Val Ser Ile Thr Glu Lys Cys Ala
340 345 350

Lys Glu Phe Gly Glu Phe
355

<210> 134
<211> 245
<212> PRT
<213> Homo sapiens

<400> 134

Met Gly Leu Leu Lys Glu Phe Leu Ala Lys Ala Lys Glu Asp Phe Leu
1 5 10 15

Lys Lys Trp Glu Asn Pro Thr Gln Asn Asn Ala Gly Leu Glu Asp Phe
20 25 30

Glu Arg Lys Lys Thr Leu Gly Thr Gly Ser Phe Gly Arg Val Met Leu
35 40 45

Val Lys His Lys Ala Thr Glu Gln Tyr Tyr Ala Met Lys Ile Leu Asp
50 55 60

Lys Gln Lys Val Val Lys Leu Lys Gln Ile Glu His Thr Leu Asn Glu
65 70 75 80

Lys Arg Ile Leu Gln Ala Val Asn Phe Pro Phe Leu Val Arg Leu Glu
85 90 95

Tyr Ala Phe Lys Asp Asn Ser Asn Leu Tyr Met Val Met Glu Tyr Val
100 105 110

Pro Gly Gly Glu Met Phe Ser His Leu Arg Arg Ile Gly Arg Phe Ser
115 120 125

Glu Pro His Ala Arg Phe Tyr Ala Ala Gln Ile Val Leu Thr Phe Glu
130 135 140

Tyr Leu His Ser Leu Asp Leu Ile Tyr Arg Asp Leu Lys Pro Glu Asn
145 150 155 160

Leu Leu Ile Asp His Gln Gly Tyr Ile Gln Val Thr Asp Phe Gly Phe
165 170 175

Ala Lys Arg Val Lys Gly Arg Thr Trp Thr Leu Cys Gly Thr Pro Glu
180 185 190

Tyr Leu Ala Pro Glu Ile Ile Leu Ser Lys Gly Tyr Asn Lys Ala Val
195 200 205

Asp Trp Trp Ala Leu Gly Val Leu Ile Tyr Glu Met Ala Ala Gly Tyr
210 215 220

Pro Pro Phe Phe Ala Asp Gln Pro Ile Gln Ile Tyr Glu Lys Ile Val
225 230 235 240

Ser Gly Lys Asn Phe
245

<210> 135
<211> 5429
<212> DNA
<213> Homo sapiens

<400> 135
agaacaactt ttttgacttc ctgcaaagag gacccttaca gtatttttgg agaagttagt 60

aaaaccgaat ctgacatcat cacctagcag ttcatgcagc tagcaagtgg tttgttctta 120

gggtaacaga ggaggaaatt gttcctcgtc tgataagaca acagtggaga aaggacgcat 180

gctgtttctt agggacacgg ctgacttcca gatatgacca tgtatttgtg gcttaaactc 240

ttggcatttg gctttgcctt tctggacaca gaagtatttg tgacagggca aagcccaaca 300

ccttccccca ctggattgac tacagcaaag atgcccagtg ttccactttc aagtgacccc 360

ttacctactc acaccactgc attctcaccc gcaagcacct ttgaaagaga aaatgacttc 420

tcagagacca caacttctct tagtccagac aatacttcca cccaagtatc cccggactct 480

ttggataatg ctagtgcttt taataccaca ggtgtttcat cagtacagac gcctcacctt 540

cccacgcacg cagactcgca gacgccctct gctggaactg acacgcagac attcagcggc 600

tccgccgcca atgcaaaact caaccctacc ccaggcagca atgctatctc agatgtccca 660

ggagagagga gtacagccag cacctttcct acagacccag tttccccatt gacaaccacc 720

ctcagccttg cacaccacag ctctgctgcc ttacctgcac gcacctccaa caccaccatc 780
acagcgaaca cctcagatgc ctaccttaat gcctctgaaa caaccactct gagcccttct 840
ggaagcgctg tcatttcaac cacaacaata gctactactc catctaagcc aacatgtgat 900
gaaaaatatg caaacatcac tgtggattac ttatataaca aggaaactaa attatttaca 960
gcaaagctaa atgttaatga gaatgtggaa tgtggaaaca atacttgcac aaacaatgag 1020
gtgcataacc ttacagaatg taaaaatgcg tctgtttcca tatctcataa ttcatgtact 1080
gctcctgata agacattaat attagatgtg ccaccagggg ttgaaaagtt tcagttacat 1140
gattgtacac aagttgaaaa agcagatact actatttgtt taaaatggaa aaatattgaa 1200
acctttactt gtgatacaca gaatattacc tacagatttc agtgtggtaa tatgatattt 1260
gataataaag aaattaaatt agaaaacctt gaacccgaac atgagtataa gtgtgactca 1320
gaaatactct ataataacca caagtttact aacgcaagta aaattattaa aacagatttt 1380
gggagtccag gagagcctca gattattttt tgtagaagtg aagctgcaca tcaaggagta 1440
attacctgga atccccctca aagatcattt cataatttta ccctctgtta tataaaagag 1500
acagaaaaag attgcctcaa tctggataaa aacctgatca aatatgattt gcaaaattta 1560
aaaccttata cgaaatatgt tttatcatta catgcctaca tcattgcaaa agtgcaacgt 1620
aatggaagtg ctgcaatgtg tcatttcaca actaaaagtg ctcctccaag ccaggtctgg 1680
aacatgactg tctccatgac atcagataat agtatgcatg tcaagtgtag gcctcccagg 1740
gaccgtaatg gcccccatga acgttaccat ttggaagttg aagctggaaa tactctggtt 1800
agaaatgagt cgcataagaa ttgcgatttc cgtgtaaaag atcttcaata ttcaacagac 1860
tacactttta aggcctattt tcacaatgga gactatcctg gagaaccctt tattttacat 1920
cattcaacat cttataattc taaggcactg atagcatttc tggcatttct gattattgtg 1980
acatcaatag ccctgcttgt tgttctctac aaaatctatg atctacataa gaaaagatcc 2040
tgcaatttag atgaacagca ggagcttgtt gaaagggatg atgaaaaaca actgatgaat 2100
gtggagccaa tccatgcaga tattttgttg gaaacttata agaggaagat tgctgatgaa 2160
ggaagacttt ttctggctga atttcagagc atcccgcggg tgttcagcaa gtttcctata 2220
aaggaagctc gaaagccctt taaccagaat aaaaaccgtt atgttgacat tcttccttat 2280
gattataacc gtgttgaact ctctgagata aacggagatg cagggtcaaa ctacataaat 2340
gccagctata ttgatggttt caaagaaccc aggaaataca ttgctgcaca aggtcccagg 2400
gatgaaactg ttgatgattt ctggaggatg atttgggaac agaaagccac agttattgtc 2460
atggtcactc gatgtgaaga aggaaacagg aacaagtgtg cagaatactg gccgtcaatg 2520
gaagagggca ctcgggcttt tggagatgtt gttgtaaaga tcaaccagca caaaagatgt 2580
ccagattaca tcattcagaa attgaacatt gtaaataaaa aagaaaaagc aactggaaga 2640

gaggtgactc acattcagtt caccagctgg ccagaccacg gggtgcctga ggatcctcac 2700
ttgctcctca aactgagaag gagagtgaat gccttcagca atttcttcag tggtcccatt 2760
gtggtgcact gcagtgctgg tgttgggcgc acaggaacct atatcggaat tgatgccatg 2820
ctagaaggcc tggaagccga gaacaaagtg gatgtttatg gttatgttgt caagctaagg 2880
cgacagagat gcctgatggt tcaagtagag gcccagtaca tcttgatcca tcaggctttg 2940
gtggaataca atcagtttgg agaaacagaa gtgaatttgt ctgaattaca tccatatcta 3000
cataacatga agaaaaggga tccacccagt gagccgtctc cactagaggc tgaattccag 3060
agacttcctt catataggag ctggaggaca cagcacattg gaaatcaaga agaaaataaa 3120
agtaaaaaca ggaattctaa tgtcatccca tatgactata acagagtgcc acttaaacat 3180
gagctggaaa tgagtaaaga gagtgagcat gattcagatg aatcctctga tgatgacagt 3240
gattcagagg aaccaagcaa atacatcaat gcatctttta taatgagcta ctggaaacct 3300
gaagtgatga ttgctgctca gggaccactg aaggagacca ttggtgactt ttggcagatg 3360
atcttccaaa gaaaagtcaa agttattgtt atgctgacag aactgaaaca tggagaccag 3420
gaaatctgtg ctcagtactg gggagaagga aagcaaacat atggagatat tgaagttgac 3480
ctgaaagaca cagacaaatc ttcaacttat acccttcgtg tctttgaact gagacattcc 3540
aagaggaaag actctcgaac tgtgtaccag taccaatata caaactggag tgtggagcag 3600
cttcctgcag aacccaagga attaatctct atgattcagg tcgtcaaaca aaaacttccc 3660
cagaagaatt cctctgaagg gaacaagcat cacaagagta cacctctact cattcactgc 3720
agggatggat ctcagcaaac gggaatattt tgtgctttgt taaatctctt agaaagtgcg 3780
gaaacagaag aggtagtgga tatttttcaa gtggtaaaag ctctacgcaa agctaggcca 3840
ggcatggttt ccacattcga gcaatatcaa ttcctatatg acgtcattgc cagcacctac 3900
cctgctcaga atggacaagt aaagaaaaac aaccatcaag aagataaaat tgaatttgat 3960
aatgaagtgg acaaagtaaa gcaggatgct aattgtgtta atccacttgg tgccccagaa 4020
aagctccctg aagcaaagga acaggctgaa ggttctgaac ccacgagtgg cactgagggg 4080
ccagaacatt ctgtcaatgg tcctgcaagt ccagctttaa atcaaggttc ataggaaaag 4140
acataaatga ggaaactcca aacctcctgt tagctgttat ttctattttt gtagaagtag 4200
gaagtgaaaa taggtataca gtggattaat taaatgcagc gaaccaatat ttgtagaagg 4260
gttatatttt actactgtgg aaaaatattt aagatagttt tgccagaaca gtttgtacag 4320
acgtatgctt attttaaaat tttatctctt attcagtaaa aaacaacttc tttgtaatcg 4380
ttatgtgtgt atatgtatgt gtgtatgggt gtgtgtttgt gtgagagaca gagaaagaga 4440
gagaattctt tcaagtgaat ctaaaagctt ttgcttttcc tttgttttta tgaagaaaaa 4500

atacatttta tattagaagt gttaacttag cttgaaggat ctgtttttaa aaatcataaa 4560

ctgtgtgcag actcaataaa atcatgtaca tttctgaaat gacctcaaga tgtcctcctt 4620

gttctactca tatatatcta tcttatatag tttactattt tacttctaga gatagtacat 4680

aaaggtggta tgtgtgtgta tgctactaca aaaaagttgt taactaaatt aacattggga 4740

aatcttatat tccatatatt agcatttagt ccaatgtctt tttaagctta tttaattaaa 4800

aaatttccag tgagcttatc atgctgtctt tacatggggt tttcaatttt gcatgctcga 4860

ttattccctg tacaatattt aaaatttatt gcttgatact tttgacaaca aattaggttt 4920

tgtacaattg aacttaaata aatgtcatta aaataaataa atgcaatatg tattaatatt 4980

cattgtataa aaatagaaga atacaaacat atttgttaaa tatttacata tgaaatttaa 5040

tatagctatt tttatggaat ttttcattga tatgaaaaat atgatattgc atatgcatag 5100

ttcccatgtt aaatcccatt cataactttc attaaagcat ttactttgaa tttctccaat 5160

gcttagaatg tttttaccag gaatggatgt cgctaatcat aataaaattc aaccattatt 5220

tttttcttgt ttataataca ttgtgttata tgttcaaata tgaaatgtgt atgcacctat 5280

tgaaatatgt ttaatgcatt tattaacatt tgcaggacac ttttacaggc cccaattatc 5340

caatagtcta ataattgttt aagatctaga aaaaaaaaat caagaatagt ggtatttttc 5400

atgaagtaat aaaaactcgt tttggtgaa 5429

<210> 136
<211> 4946
<212> DNA
<213> Homo sapiens

<400> 136
agaacaactt ttttgacttc ctgcaaagag gacccttaca gtatttttgg agaagttagt 60

aaaaccgaat ctgacatcat cacctagcag ttcatgcagc tagcaagtgg tttgttctta 120

gggtaacaga ggaggaaatt gttcctcgtc tgataagaca acagtggaga aaggacgcat 180

gctgtttctt agggacacgg ctgacttcca gatatgacca tgtatttgtg gcttaaactc 240

ttggcatttg gctttgcctt tctggacaca gaagtatttg tgacagggca aagcccaaca 300

ccttccccca ctgatgccta ccttaatgcc tctgaaacaa ccactctgag cccttctgga 360

agcgctgtca tttcaaccac aacaatagct actactccat ctaagccaac atgtgatgaa 420

aaatatgcaa acatcactgt ggattactta tataacaagg aaactaaatt atttacagca 480

aagctaaatg ttaatgagaa tgtggaatgt ggaaacaata cttgcacaaa caatgaggtg 540

cataacctta cagaatgtaa aaatgcgtct gtttccatat ctcataattc atgtactgct 600

cctgataaga cattaatatt agatgtgcca ccaggggttg aaaagtttca gttacatgat 660

tgtacacaag ttgaaaaagc agatactact atttgtttaa aatggaaaaa tattgaaacc 720

tttacttgtg atacacagaa tattacctac agatttcagt gtggtaatat gatatttgat 780
aataaagaaa ttaaattaga aaaccttgaa cccgaacatg agtataagtg tgactcagaa 840
atactctata ataaccacaa gtttactaac gcaagtaaaa ttattaaaac agattttggg 900
agtccaggag agcctcagat tattttttgt agaagtgaag ctgcacatca aggagtaatt 960
acctggaatc cccctcaaag atcatttcat aattttaccc tctgttatat aaaagagaca 1020
gaaaaagatt gcctcaatct ggataaaaac ctgatcaaat atgatttgca aaatttaaaa 1080
ccttatacga aatatgtttt atcattacat gcctacatca ttgcaaaagt gcaacgtaat 1140
ggaagtgctg caatgtgtca tttcacaact aaaagtgctc ctccaagcca ggtctggaac 1200
atgactgtct ccatgacatc agataatagt atgcatgtca agtgtaggcc tcccagggac 1260
cgtaatggcc cccatgaacg ttaccatttg gaagttgaag ctggaaatac tctggttaga 1320
aatgagtcgc ataagaattg cgatttccgt gtaaaagatc ttcaatattc aacagactac 1380
acttttaagg cctattttca caatggagac tatcctggag aacccttttat tttacatcat 1440
tcaacatctt ataattctaa ggcactgata gcatttctgg catttctgat tattgtgaca 1500
tcaatagccc tgcttgttgt tctctacaaa atctatgatc tacataagaa aagatcctgc 1560
aatttagatg aacagcagga gcttgttgaa agggatgatg aaaaacaact gatgaatgtg 1620
gagccaatcc atgcagatat tttgttggaa acttataaga ggaagattgc tgatgaagga 1680
agactttttc tggctgaatt tcagagcatc ccgcgggtgt tcagcaagtt tcctataaag 1740
gaagctcgaa agccctttaa ccagaataaa aaccgttatg ttgacattct tccttatgat 1800
tataaccgtg ttgaactctc tgagataaac ggagatgcag ggtcaaacta cataaatgcc 1860
agctatattg atggtttcaa agaacccagg aaatacattg ctgcacaagg tcccagggat 1920
gaaactgttg atgatttctg gaggatgatt tgggaacaga aagccacagt tattgtcatg 1980
gtcactcgat gtgaagaagg aaacaggaac aagtgtgcag aatactggcc gtcaatggaa 2040
gagggcactc gggcttttgg agatgttgtt gtaaagatca accagcacaa aagatgtcca 2100
gattacatca ttcagaaatt gaacattgta aataaaaaag aaaaagcaac tggaagagag 2160
gtgactcaca ttcagttcac cagctggcca gaccacgggg tgcctgagga tcctcacttg 2220
ctcctcaaac tgagaaggag agtgaatgcc ttcagcaatt tcttcagtgg tcccattgtg 2280
gtgcactgca gtgctggtgt tgggcgcaca ggaacctata tcggaattga tgccatgcta 2340
gaaggcctgg aagccgagaa caaagtggat gtttatggtt atgttgtcaa gctaaggcga 2400
cagagatgcc tgatggttca agtagaggcc cagtacatct tgatccatca ggctttggtg 2460
gaatacaatc agtttggaga aacagaagtg aatttgtctg aattacatcc atatctacat 2520
aacatgaaga aaagggatcc acccagtgag ccgtctccac tagaggctga attccagaga 2580
cttccttcat ataggagctg gaggacacag cacattggaa atcaagaaga aaataaaagt 2640

aaaaacagga attctaatgt catcccatat gactataaca gagtgccact taaacatgag 2700
ctggaaatga gtaaagagag tgagcatgat tcagatgaat cctctgatga tgacagtgat 2760
tcagaggaac caagcaaata catcaatgca tcttttataa tgagctactg gaaacctgaa 2820
gtgatgattg ctgctcaggg accactgaag gagaccattg gtgacttttg gcagatgatc 2880
ttccaaagaa aagtcaaagt tattgttatg ctgacagaac tgaaacatgg agaccaggaa 2940
atctgtgctc agtactgggg agaaggaaag caaacatatg gagatattga agttgacctg 3000
aaagacacag acaaatcttc aacttatacc cttcgtgtct ttgaactgag acattccaag 3060
aggaaagact ctcgaactgt gtaccagtac caatatacaa actggagtgt ggagcagctt 3120
cctgcagaac ccaaggaatt aatctctatg attcaggtcg tcaaacaaaa acttccccag 3180
aagaattcct ctgaagggaa caagcatcac aagagtacac ctctactcat tcactgcagg 3240
gatggatctc agcaaacggg aatattttgt gctttgttaa atctcttaga aagtgcggaa 3300
acagaagagg tagtggatat ttttcaagtg gtaaaagctc tacgcaaagc taggccaggc 3360
atggtttcca cattcgagca atatcaattc ctatatgacg tcattgccag cacctaccct 3420
gctcagaatg gacaagtaaa gaaaaacaac catcaagaag ataaaattga atttgataat 3480
gaagtggaca aagtaaagca ggatgctaat tgtgttaatc cacttggtgc cccagaaaag 3540
ctccctgaag caaaggaaca ggctgaaggt tctgaaccca cgagtggcac tgagggggcca 3600
gaacattctg tcaatggtcc tgcaagtcca gctttaaatc aaggttcata ggaaaagaca 3660
taaatgagga aactccaaac ctcctgttag ctgttatttc tatttttgta gaagtaggaa 3720
gtgaaaatag gtatacagtg gattaattaa atgcagcgaa ccaatatttg tagaagggtt 3780
atattttact actgtggaaa aatatttaag atagttttgc cagaacagtt tgtacagacg 3840
tatgcttatt ttaaaatttt atctcttatt cagtaaaaaa caacttcttt gtaatcgtta 3900
tgtgtgtata tgtatgtgtg tatgggtgtg tgtttgtgtg agagacagag aaagagagag 3960
aattctttca agtgaatcta aaagcttttg cttttccttt gtttttatga agaaaaaata 4020
cattttatat tagaagtgtt aacttagctt gaaggatctg tttttaaaaa tcataaactg 4080
tgtgcagact caataaaatc atgtacattt ctgaaatgac ctcaagatgt cctccttgtt 4140
ctactcatat atatctatct tatatagttt actattttac ttctagagat agtacataaa 4200
ggtggtatgt gtgtgtatgc tactacaaaa aagttgttaa ctaaattaac attgggaaat 4260
cttatattcc atatattagc atttagtcca atgtcttttt aagcttattt aattaaaaaa 4320
tttccagtga gcttatcatg ctgtctttac atggggtttt caattttgca tgctcgatta 4380
ttccctgtac aatatttaaa atttattgct tgatactttt gacaacaaat taggttttgt 4440
acaattgaac ttaaataaat gtcattaaaa taaataaatg caatatgtat taatattcat 4500

tgtataaaaa tagaagaata caaacatatt tgttaaatat ttacatatga aatttaatat 4560

agctattttt atggaatttt tcattgatat gaaaaatatg atattgcata tgcatagttc 4620

ccatgttaaa tcccattcat aactttcatt aaagcattta ctttgaattt ctccaatgct 4680

tagaatgttt ttaccaggaa tggatgtcgc taatcataat aaaattcaac cattattttt 4740

ttcttgttta taatacattg tgttatatgt tcaaatatga aatgtgtatg cacctattga 4800

aatatgttta atgcatttat taacatttgc aggacacttt tacaggcccc aattatccaa 4860

tagtctaata attgtttaag atctagaaaa aaaaaatcaa gaatagtggt atttttcatg 4920

aagtaataaa aactcgtttt ggtgaa 4946

<210> 137
<211> 1306
<212> PRT
<213> Homo sapiens

<400> 137

Met Thr Met Tyr Leu Trp Leu Lys Leu Leu Ala Phe Gly Phe Ala Phe
1 5 10 15

Leu Asp Thr Glu Val Phe Val Thr Gly Gln Ser Pro Thr Pro Ser Pro
20 25 30

Thr Gly Leu Thr Thr Ala Lys Met Pro Ser Val Pro Leu Ser Ser Asp
35 40 45

Pro Leu Pro Thr His Thr Thr Ala Phe Ser Pro Ala Ser Thr Phe Glu
50 55 60

Arg Glu Asn Asp Phe Ser Glu Thr Thr Thr Ser Leu Ser Pro Asp Asn
65 70 75 80

Thr Ser Thr Gln Val Ser Pro Asp Ser Leu Asp Asn Ala Ser Ala Phe
85 90 95

Asn Thr Thr Gly Val Ser Ser Val Gln Thr Pro His Leu Pro Thr His
100 105 110

Ala Asp Ser Gln Thr Pro Ser Ala Gly Thr Asp Thr Gln Thr Phe Ser
115 120 125

Gly Ser Ala Ala Asn Ala Lys Leu Asn Pro Thr Pro Gly Ser Asn Ala
130 135 140

Ile Ser Asp Val Pro Gly Glu Arg Ser Thr Ala Ser Thr Phe Pro Thr
145 150 155 160

Asp Pro Val Ser Pro Leu Thr Thr Thr Leu Ser Leu Ala His His Ser
165 170 175

Ser Ala Ala Leu Pro Ala Arg Thr Ser Asn Thr Thr Ile Thr Ala Asn
180 185 190

Thr Ser Asp Ala Tyr Leu Asn Ala Ser Glu Thr Thr Thr Leu Ser Pro
195 200 205

Ser Gly Ser Ala Val Ile Ser Thr Thr Thr Ile Ala Thr Thr Pro Ser
210 215 220

Lys Pro Thr Cys Asp Glu Lys Tyr Ala Asn Ile Thr Val Asp Tyr Leu
225 230 235 240

Tyr Asn Lys Glu Thr Lys Leu Phe Thr Ala Lys Leu Asn Val Asn Glu
245 250 255

Asn Val Glu Cys Gly Asn Asn Thr Cys Thr Asn Asn Glu Val His Asn
260 265 270

Leu Thr Glu Cys Lys Asn Ala Ser Val Ser Ile Ser His Asn Ser Cys
275 280 285

Thr Ala Pro Asp Lys Thr Leu Ile Leu Asp Val Pro Pro Gly Val Glu
290 295 300

Lys Phe Gln Leu His Asp Cys Thr Gln Val Glu Lys Ala Asp Thr Thr
305 310 315 320

Ile Cys Leu Lys Trp Lys Asn Ile Glu Thr Phe Thr Cys Asp Thr Gln
325 330 335

Asn Ile Thr Tyr Arg Phe Gln Cys Gly Asn Met Ile Phe Asp Asn Lys
340 345 350

Glu Ile Lys Leu Glu Asn Leu Glu Pro Glu His Glu Tyr Lys Cys Asp
355 360 365

Ser Glu Ile Leu Tyr Asn Asn His Lys Phe Thr Asn Ala Ser Lys Ile
370 375 380

Ile Lys Thr Asp Phe Gly Ser Pro Gly Glu Pro Gln Ile Ile Phe Cys
385 390 395 400

Arg Ser Glu Ala Ala His Gln Gly Val Ile Thr Trp Asn Pro Pro Gln
405 410 415

```
Arg Ser Phe His Asn Phe Thr Leu Cys Tyr Ile Lys Glu Thr Glu Lys
            420                 425                 430

Asp Cys Leu Asn Leu Asp Lys Asn Leu Ile Lys Tyr Asp Leu Gln Asn
        435                 440                 445

Leu Lys Pro Tyr Thr Lys Tyr Val Leu Ser Leu His Ala Tyr Ile Ile
    450                 455                 460

Ala Lys Val Gln Arg Asn Gly Ser Ala Ala Met Cys His Phe Thr Thr
465                 470                 475                 480

Lys Ser Ala Pro Pro Ser Gln Val Trp Asn Met Thr Val Ser Met Thr
                485                 490                 495

Ser Asp Asn Ser Met His Val Lys Cys Arg Pro Pro Arg Asp Arg Asn
            500                 505                 510

Gly Pro His Glu Arg Tyr His Leu Glu Val Glu Ala Gly Asn Thr Leu
        515                 520                 525

Val Arg Asn Glu Ser His Lys Asn Cys Asp Phe Arg Val Lys Asp Leu
    530                 535                 540

Gln Tyr Ser Thr Asp Tyr Thr Phe Lys Ala Tyr Phe His Asn Gly Asp
545                 550                 555                 560

Tyr Pro Gly Glu Pro Phe Ile Leu His His Ser Thr Ser Tyr Asn Ser
                565                 570                 575

Lys Ala Leu Ile Ala Phe Leu Ala Phe Leu Ile Ile Val Thr Ser Ile
            580                 585                 590

Ala Leu Leu Val Val Leu Tyr Lys Ile Tyr Asp Leu His Lys Lys Arg
        595                 600                 605

Ser Cys Asn Leu Asp Glu Gln Gln Glu Leu Val Glu Arg Asp Asp Glu
    610                 615                 620

Lys Gln Leu Met Asn Val Glu Pro Ile His Ala Asp Ile Leu Leu Glu
625                 630                 635                 640

Thr Tyr Lys Arg Lys Ile Ala Asp Glu Gly Arg Leu Phe Leu Ala Glu
                645                 650                 655

Phe Gln Ser Ile Pro Arg Val Phe Ser Lys Phe Pro Ile Lys Glu Ala
```

660 665 670

Arg Lys Pro Phe Asn Gln Asn Lys Asn Arg Tyr Val Asp Ile Leu Pro
675 680 685

Tyr Asp Tyr Asn Arg Val Glu Leu Ser Glu Ile Asn Gly Asp Ala Gly
690 695 700

Ser Asn Tyr Ile Asn Ala Ser Tyr Ile Asp Gly Phe Lys Glu Pro Arg
705 710 715 720

Lys Tyr Ile Ala Ala Gln Gly Pro Arg Asp Glu Thr Val Asp Asp Phe
725 730 735

Trp Arg Met Ile Trp Glu Gln Lys Ala Thr Val Ile Val Met Val Thr
740 745 750

Arg Cys Glu Glu Gly Asn Arg Asn Lys Cys Ala Glu Tyr Trp Pro Ser
755 760 765

Met Glu Glu Gly Thr Arg Ala Phe Gly Asp Val Val Val Lys Ile Asn
770 775 780

Gln His Lys Arg Cys Pro Asp Tyr Ile Ile Gln Lys Leu Asn Ile Val
785 790 795 800

Asn Lys Lys Glu Lys Ala Thr Gly Arg Glu Val Thr His Ile Gln Phe
805 810 815

Thr Ser Trp Pro Asp His Gly Val Pro Glu Asp Pro His Leu Leu Leu
820 825 830

Lys Leu Arg Arg Arg Val Asn Ala Phe Ser Asn Phe Phe Ser Gly Pro
835 840 845

Ile Val Val His Cys Ser Ala Gly Val Gly Arg Thr Gly Thr Tyr Ile
850 855 860

Gly Ile Asp Ala Met Leu Glu Gly Leu Glu Ala Glu Asn Lys Val Asp
865 870 875 880

Val Tyr Gly Tyr Val Val Lys Leu Arg Arg Gln Arg Cys Leu Met Val
885 890 895

Gln Val Glu Ala Gln Tyr Ile Leu Ile His Gln Ala Leu Val Glu Tyr
900 905 910

Asn Gln Phe Gly Glu Thr Glu Val Asn Leu Ser Glu Leu His Pro Tyr
915 920 925

Leu His Asn Met Lys Lys Arg Asp Pro Pro Ser Glu Pro Ser Pro Leu
930 935 940

Glu Ala Glu Phe Gln Arg Leu Pro Ser Tyr Arg Ser Trp Arg Thr Gln
945 950 955 960

His Ile Gly Asn Gln Glu Glu Asn Lys Ser Lys Asn Arg Asn Ser Asn
965 970 975

Val Ile Pro Tyr Asp Tyr Asn Arg Val Pro Leu Lys His Glu Leu Glu
980 985 990

Met Ser Lys Glu Ser Glu His Asp Ser Asp Glu Ser Ser Asp Asp Asp
995 1000 1005

Ser Asp Ser Glu Glu Pro Ser Lys Tyr Ile Asn Ala Ser Phe Ile
1010 1015 1020

Met Ser Tyr Trp Lys Pro Glu Val Met Ile Ala Ala Gln Gly Pro
1025 1030 1035

Leu Lys Glu Thr Ile Gly Asp Phe Trp Gln Met Ile Phe Gln Arg
1040 1045 1050

Lys Val Lys Val Ile Val Met Leu Thr Glu Leu Lys His Gly Asp
1055 1060 1065

Gln Glu Ile Cys Ala Gln Tyr Trp Gly Glu Gly Lys Gln Thr Tyr
1070 1075 1080

Gly Asp Ile Glu Val Asp Leu Lys Asp Thr Asp Lys Ser Ser Thr
1085 1090 1095

Tyr Thr Leu Arg Val Phe Glu Leu Arg His Ser Lys Arg Lys Asp
1100 1105 1110

Ser Arg Thr Val Tyr Gln Tyr Gln Tyr Thr Asn Trp Ser Val Glu
1115 1120 1125

Gln Leu Pro Ala Glu Pro Lys Glu Leu Ile Ser Met Ile Gln Val
1130 1135 1140

Val Lys Gln Lys Leu Pro Gln Lys Asn Ser Ser Glu Gly Asn Lys
1145 1150 1155

His His Lys Ser Thr Pro Leu Leu Ile His Cys Arg Asp Gly Ser
1160 1165 1170

Gln Gln Thr Gly Ile Phe Cys Ala Leu Leu Asn Leu Leu Glu Ser
1175 1180 1185

Ala Glu Thr Glu Glu Val Val Asp Ile Phe Gln Val Val Lys Ala
1190 1195 1200

Leu Arg Lys Ala Arg Pro Gly Met Val Ser Thr Phe Glu Gln Tyr
1205 1210 1215

Gln Phe Leu Tyr Asp Val Ile Ala Ser Thr Tyr Pro Ala Gln Asn
1220 1225 1230

Gly Gln Val Lys Lys Asn Asn His Gln Glu Asp Lys Ile Glu Phe
1235 1240 1245

Asp Asn Glu Val Asp Lys Val Lys Gln Asp Ala Asn Cys Val Asn
1250 1255 1260

Pro Leu Gly Ala Pro Glu Lys Leu Pro Glu Ala Lys Glu Gln Ala
1265 1270 1275

Glu Gly Ser Glu Pro Thr Ser Gly Thr Glu Gly Pro Glu His Ser
1280 1285 1290

Val Asn Gly Pro Ala Ser Pro Ala Leu Asn Gln Gly Ser
1295 1300 1305

<210> 138
<211> 1145
<212> PRT
<213> Homo sapiens

<400> 138

Met Thr Met Tyr Leu Trp Leu Lys Leu Leu Ala Phe Gly Phe Ala Phe
1 5 10 15

Leu Asp Thr Glu Val Phe Val Thr Gly Gln Ser Pro Thr Pro Ser Pro
20 25 30

Thr Asp Ala Tyr Leu Asn Ala Ser Glu Thr Thr Thr Leu Ser Pro Ser
35 40 45

Gly Ser Ala Val Ile Ser Thr Thr Thr Ile Ala Thr Thr Pro Ser Lys
50 55 60

```
Pro Thr Cys Asp Glu Lys Tyr Ala Asn Ile Thr Val Asp Tyr Leu Tyr
65                  70                  75                  80

Asn Lys Glu Thr Lys Leu Phe Thr Ala Lys Leu Asn Val Asn Glu Asn
                85                  90                  95

Val Glu Cys Gly Asn Asn Thr Cys Thr Asn Asn Glu Val His Asn Leu
            100                 105                 110

Thr Glu Cys Lys Asn Ala Ser Val Ser Ile Ser His Asn Ser Cys Thr
        115                 120                 125

Ala Pro Asp Lys Thr Leu Ile Leu Asp Val Pro Pro Gly Val Glu Lys
    130                 135                 140

Phe Gln Leu His Asp Cys Thr Gln Val Glu Lys Ala Asp Thr Thr Ile
145                 150                 155                 160

Cys Leu Lys Trp Lys Asn Ile Glu Thr Phe Thr Cys Asp Thr Gln Asn
                165                 170                 175

Ile Thr Tyr Arg Phe Gln Cys Gly Asn Met Ile Phe Asp Asn Lys Glu
            180                 185                 190

Ile Lys Leu Glu Asn Leu Glu Pro Glu His Glu Tyr Lys Cys Asp Ser
        195                 200                 205

Glu Ile Leu Tyr Asn Asn His Lys Phe Thr Asn Ala Ser Lys Ile Ile
    210                 215                 220

Lys Thr Asp Phe Gly Ser Pro Gly Glu Pro Gln Ile Ile Phe Cys Arg
225                 230                 235                 240

Ser Glu Ala Ala His Gln Gly Val Ile Thr Trp Asn Pro Pro Gln Arg
                245                 250                 255

Ser Phe His Asn Phe Thr Leu Cys Tyr Ile Lys Glu Thr Glu Lys Asp
            260                 265                 270

Cys Leu Asn Leu Asp Lys Asn Leu Ile Lys Tyr Asp Leu Gln Asn Leu
        275                 280                 285

Lys Pro Tyr Thr Lys Tyr Val Leu Ser Leu His Ala Tyr Ile Ile Ala
    290                 295                 300

Lys Val Gln Arg Asn Gly Ser Ala Ala Met Cys His Phe Thr Thr Lys
305                 310                 315                 320
```

Ser Ala Pro Pro Ser Gln Val Trp Asn Met Thr Val Ser Met Thr Ser
325 330 335

Asp Asn Ser Met His Val Lys Cys Arg Pro Pro Arg Asp Arg Asn Gly
340 345 350

Pro His Glu Arg Tyr His Leu Glu Val Glu Ala Gly Asn Thr Leu Val
355 360 365

Arg Asn Glu Ser His Lys Asn Cys Asp Phe Arg Val Lys Asp Leu Gln
370 375 380

Tyr Ser Thr Asp Tyr Thr Phe Lys Ala Tyr Phe His Asn Gly Asp Tyr
385 390 395 400

Pro Gly Glu Pro Phe Ile Leu His His Ser Thr Ser Tyr Asn Ser Lys
405 410 415

Ala Leu Ile Ala Phe Leu Ala Phe Leu Ile Ile Val Thr Ser Ile Ala
420 425 430

Leu Leu Val Val Leu Tyr Lys Ile Tyr Asp Leu His Lys Lys Arg Ser
435 440 445

Cys Asn Leu Asp Glu Gln Gln Glu Leu Val Glu Arg Asp Asp Glu Lys
450 455 460

Gln Leu Met Asn Val Glu Pro Ile His Ala Asp Ile Leu Leu Glu Thr
465 470 475 480

Tyr Lys Arg Lys Ile Ala Asp Glu Gly Arg Leu Phe Leu Ala Glu Phe
485 490 495

Gln Ser Ile Pro Arg Val Phe Ser Lys Phe Pro Ile Lys Glu Ala Arg
500 505 510

Lys Pro Phe Asn Gln Asn Lys Asn Arg Tyr Val Asp Ile Leu Pro Tyr
515 520 525

Asp Tyr Asn Arg Val Glu Leu Ser Glu Ile Asn Gly Asp Ala Gly Ser
530 535 540

Asn Tyr Ile Asn Ala Ser Tyr Ile Asp Gly Phe Lys Glu Pro Arg Lys
545 550 555 560

Tyr Ile Ala Ala Gln Gly Pro Arg Asp Glu Thr Val Asp Asp Phe Trp

565 570 575

Arg Met Ile Trp Glu Gln Lys Ala Thr Val Ile Val Met Val Thr Arg
580 585 590

Cys Glu Glu Gly Asn Arg Asn Lys Cys Ala Glu Tyr Trp Pro Ser Met
595 600 605

Glu Glu Gly Thr Arg Ala Phe Gly Asp Val Val Val Lys Ile Asn Gln
610 615 620

His Lys Arg Cys Pro Asp Tyr Ile Ile Gln Lys Leu Asn Ile Val Asn
625 630 635 640

Lys Lys Glu Lys Ala Thr Gly Arg Glu Val Thr His Ile Gln Phe Thr
645 650 655

Ser Trp Pro Asp His Gly Val Pro Glu Asp Pro His Leu Leu Leu Lys
660 665 670

Leu Arg Arg Arg Val Asn Ala Phe Ser Asn Phe Phe Ser Gly Pro Ile
675 680 685

Val Val His Cys Ser Ala Gly Val Gly Arg Thr Gly Thr Tyr Ile Gly
690 695 700

Ile Asp Ala Met Leu Glu Gly Leu Glu Ala Glu Asn Lys Val Asp Val
705 710 715 720

Tyr Gly Tyr Val Val Lys Leu Arg Arg Gln Arg Cys Leu Met Val Gln
725 730 735

Val Glu Ala Gln Tyr Ile Leu Ile His Gln Ala Leu Val Glu Tyr Asn
740 745 750

Gln Phe Gly Glu Thr Glu Val Asn Leu Ser Glu Leu His Pro Tyr Leu
755 760 765

His Asn Met Lys Lys Arg Asp Pro Pro Ser Glu Pro Ser Pro Leu Glu
770 775 780

Ala Glu Phe Gln Arg Leu Pro Ser Tyr Arg Ser Trp Arg Thr Gln His
785 790 795 800

Ile Gly Asn Gln Glu Glu Asn Lys Ser Lys Asn Arg Asn Ser Asn Val
805 810 815

Ile Pro Tyr Asp Tyr Asn Arg Val Pro Leu Lys His Glu Leu Glu Met
820 825 830

Ser Lys Glu Ser Glu His Asp Ser Asp Glu Ser Ser Asp Asp Asp Ser
835 840 845

Asp Ser Glu Glu Pro Ser Lys Tyr Ile Asn Ala Ser Phe Ile Met Ser
850 855 860

Tyr Trp Lys Pro Glu Val Met Ile Ala Ala Gln Gly Pro Leu Lys Glu
865 870 875 880

Thr Ile Gly Asp Phe Trp Gln Met Ile Phe Gln Arg Lys Val Lys Val
885 890 895

Ile Val Met Leu Thr Glu Leu Lys His Gly Asp Gln Glu Ile Cys Ala
900 905 910

Gln Tyr Trp Gly Glu Gly Lys Gln Thr Tyr Gly Asp Ile Glu Val Asp
915 920 925

Leu Lys Asp Thr Asp Lys Ser Ser Thr Tyr Thr Leu Arg Val Phe Glu
930 935 940

Leu Arg His Ser Lys Arg Lys Asp Ser Arg Thr Val Tyr Gln Tyr Gln
945 950 955 960

Tyr Thr Asn Trp Ser Val Glu Gln Leu Pro Ala Glu Pro Lys Glu Leu
965 970 975

Ile Ser Met Ile Gln Val Val Lys Gln Lys Leu Pro Gln Lys Asn Ser
980 985 990

Ser Glu Gly Asn Lys His His Lys Ser Thr Pro Leu Leu Ile His Cys
995 1000 1005

Arg Asp Gly Ser Gln Gln Thr Gly Ile Phe Cys Ala Leu Leu Asn
1010 1015 1020

Leu Leu Glu Ser Ala Glu Thr Glu Glu Val Val Asp Ile Phe Gln
1025 1030 1035

Val Val Lys Ala Leu Arg Lys Ala Arg Pro Gly Met Val Ser Thr
1040 1045 1050

Phe Glu Gln Tyr Gln Phe Leu Tyr Asp Val Ile Ala Ser Thr Tyr
1055 1060 1065

Pro Ala Gln Asn Gly Gln Val Lys Lys Asn Asn His Gln Glu Asp
1070 1075 1080

Lys Ile Glu Phe Asp Asn Glu Val Asp Lys Val Lys Gln Asp Ala
1085 1090 1095

Asn Cys Val Asn Pro Leu Gly Ala Pro Glu Lys Leu Pro Glu Ala
1100 1105 1110

Lys Glu Gln Ala Glu Gly Ser Glu Pro Thr Ser Gly Thr Glu Gly
1115 1120 1125

Pro Glu His Ser Val Asn Gly Pro Ala Ser Pro Ala Leu Asn Gln
1130 1135 1140

Gly Ser
1145

<210> 139
<211> 6663
<212> DNA
<213> Homo sapiens

<400> 139
actgaccccg ctgtaccacg gccctcttgc ggacagcccc ggggacgtcg ttgggacatc 60

gctgggaccc cgggctctgc agccacaacc atgtttggcc ggaagcgcag tgtctccttt 120

gggggcttcg gatggatcga caagaccatg ctggcaagtc tgaaggtcaa gaagcaggag 180

ctggccaaca gctcggatgc gaccctccca gaccggccgc tctcccctcc tctcacggca 240

cctcccacca tgaagtcgtc ggagttcttt gagatgctgg agaaaatgca ggggatcaag 300

cttgaagagc agaagccggg accccagaag aacaaggacg actatatccc ataccccagc 360

atcgacgagg ttgtggagaa gggaggcccg taccctcagg tcatcctgcc acagtttggg 420

ggctattgga tcgaggaccc ggagaacgtg ggcaccccaa catcgctggg gagcagcatc 480

tgtgaggagg aggaagagga caacctcagc cccaacacat ttggctacaa gctcgagtgc 540

aagggtgaag ccagggccta ccggaggcac ttcctgggga aggatcatct aaacttttac 600

tgtaccggca gcagcctggg gaacttgatc ctgtccgtca agtgcgagga agcagagggg 660

atcgagtacc tccgggtcat ccttaggtcc aaactgaaga cggtacatga gcggatcccc 720

ttggctggac tgagcaagct tcccagtgtc cctcagattg caaaggcttt ctgtgatgat 780

gcagtgggac tgagattcaa tcctgtcctg taccccaagg cctcccaaat gattgtgtcc 840

tatgatgagc atgaagtcaa caacacattc aaattcggag tcatttatca aaaagccagg 900

cagaccctgg aggaggagct atttgggaac aatgaggaga gcccagcttt taaggagttc 960

ttggacctgc tgggggacac gatcacactg caggatttca aaggtttccg aggaggcctg 1020
gacgtgaccc acggacagac aggggtggaa tcagtgtaca caacattccg ggacagggag 1080
atcatgtttc acgtttccac aaagctgcca tttaccgacg gagacgccca gcagctccag 1140
agaaagagac acattggaaa tgacatcgtg gccatcatct tccaagagga aaacacgccg 1200
tttgtcccag acatgatagc ctccaatttc ttacatgcct acatcgtcgt gcaggtcgag 1260
accccaggca cagagacccc atcctacaag gtctctgtca ctgcgcggga agatgtgccc 1320
acctttggtc cacctctgcc cagtcccccc gttttccaga agggcccgga attcagggag 1380
tttctgctca ccaagctcac caatgccgag aacgcctgct gcaagtcgga caagtttgca 1440
aagctggagg accggaccag ggctgccctc ctggacaacc ttcacgatga gctccacgcc 1500
cacacacagg ccatgctggg actgggccca gaggaggaca agtttgagaa tggaggccac 1560
gggggggttcc tggagtcttt taagagggcc atccgcgtac gcagccactc catggagacc 1620
atggtgggcg gccagaagaa gtcgcacagt gggggcatcc ctggcagcct cagcgggggc 1680
atctcccaca acagcatgga ggtcaccaag accaccttct cgcctccagt ggtggcggca 1740
acggtgaaga accagtcacg gagtcccatc aagcgacgct cggggctctt cccccgcctg 1800
cacacgggct cagaaggcca gggcgacagc cgggcacgat gtgacagcac atccagcaca 1860
cccaagaccc cagatggtgg acactcctct caggagataa agtctgagac ctcatccaat 1920
cccagctctc cggaaatctg ccccaacaag gagaagccct tcatgaagtt gaaggaaaac 1980
ggccgtgcca tctcccgctc ctcctccagc accagcagcg tcagcagcac tgcaggggag 2040
ggcgaggcca tggaggaggg cgacagtggg ggcagccagc cgtccacgac ctcacccttc 2100
aagcaggagg tgtttgtcta cagcccgtcc ccgagcagcg agagccccag cctgggggca 2160
gctgccaccc cgatcatcat gagccggagt cccacagatg ccaaaagcag aaactccccg 2220
agatcgaacc tgaaattccg ctttgacaag ctcagccatg ccagctctgg tgcgggtcac 2280
taatgtgaaa gtggagtcct tcgcctgtcc aagggaatcc cctcttctgt cctggaaaag 2340
gctcctgtac cagcagtttg ggagtgccgt ccacgaccct gacagtccca gccctgctgc 2400
cccatggcca cgtgcccaca gatgtgctgt tggtccaggt gtcccagtct ggccacagcc 2460
ctgcctccgc cctcacctac atgccctccc agcccctccc atctctggac gaggcctcct 2520
tcctcaggtt cctcctgctc ctgacctccc agtgtgatgt ccgggtcctt tatcatccta 2580
ttcatcctgg agaggaaaag tgtcgggcaa agggggatct ggggggagct cagcagtgac 2640
tggggagctg gtctgcctca gagacagagt agggggtggg agcagagcct cggtgagggt 2700
cttggccaca gggcagtgcc ttcctgaacg tggcaggctt tactaccagg aacgcactcg 2760
gtggtggagg ccccatgttc ccaggagcca agattcgtag catccttgag gccatcctga 2820
taaaattcgg cgctattgcc cccgtagctc tggagctcta aaccgtctat ctgcttctgt 2880

gctgaacgcc tttcccatct gctgacgtag gcccagggct gccctgcccc tgctgccagt 2940

gtaccgtgag cggggctcca gccagttcaa gctcagagcc agagctggac gggccagaac 3000

tgcgctgcac acttcctgga ctgaggcggg gactttgggt cccacccggt ttctcctgat 3060

tatggctgct gtggggtgag gggagggagg ggcagccccg aggcagtctc ttccctttga 3120

gaagatattt tcccacaaag ggtgggaag ccaggagtga gaaggaattc agggagagca 3180

aaggagccag tgctgagatg ctgctgtgtt ggttgaggaa aacctcgggc ctgagggcca 3240

ggccggagcc caggtctctg ctgacaatgg gggttcaagg aagacgtcgt tatctcccct 3300

ccccacttac tcgaggagag aggtgagggg gggatgactt gcgggttctg atcaggcccc 3360

tgggtgggga aggggcacag tgtccctcag cagcttacgc ccctggagtc ttggggggcc 3420

cagcctggcc ctggggcctt ttccagctac tgtgcccttg ggcagctgcg tctggggctc 3480

aaccccccaa tcctgttccc ctctccagct gcgggtctgt aggcagctgt cacatctgaa 3540

gggtttctgc aacctggacc ccatctgggt gtgggtcaga ccctgtgacc cacatgccac 3600

ccccaccctc cacagagccc ccttgctggg acagccagct cacctccaag gacatcccct 3660

cctggcttct ccccccttccg agtctgcagc gccgtgggct tctctgccga tgggcccggg 3720

ttggggttaa ggtgggcatc ctccaggtac aacgagcctg aagagcccct ttcagtgcag 3780

acggggctgc agagtgacac tggctgggca cctgccccac gaccaatgac aaggatttcc 3840

agctgaatgc tttattccca tagggatctg gacctgtgcc caagatataa atactacact 3900

tttttttttt ttttttaact gacattgtga aattctccct atagcttttg ccattcaagc 3960

aacattgtga tctttcttcc ccgccacgtg tgtgggaatg attgagtcct gtttgcaagc 4020

tggagaggag ctctcccttt gctagtactt tctctaaagt actagtctag taaaatttat 4080

tcttgttaga aggtcaacaa aatatctgtt tagcttttat gaagagtcac cgtagcagcc 4140

cccacggctg gaaagaggcc tgtacgttct ggacgcgttt tgttggctgg gcttctggag 4200

gcactggcaa ggtcaaactg catttcttta agaacagttg caggatctgg ctcgcctctg 4260

tgggaagccg gcattacagg tgcttggtgg atgggccgtg tcacattgcc atctggggtc 4320

ctttggggtt tccaggttgt caccatgctg tcccatttgg gaatcccata cctgcctgtc 4380

cccactgcgc tggctgaccc ttgctgcctg ctgcctcttg ggagggcttt gtccctgcct 4440

ctgagctggt gggcaggatg gctgggtggc ccccagagaa gcacagacct gagatggggt 4500

ctccatgccc ggtttgctgt tggaatgatc tgaacaggac cccaaatgcc tcttccctct 4560

ggtcatgcct cactatctct aggagctcca tcctgtggct tccagagtgt gcacttccag 4620

cccacccggg cagtgctgag agggaggagg agaacaagga tggcccagcc tcccctccct 4680

cccctagacc acggggcggg cagctcgggt tcctggaggg ctgtttcccc cacgctgtcc 4740

ctacatctgc tctgatctaa aatgtctttc cttttatgct gcgcccagtc ttggggctca 4800
aagatttgcc caaacctcat tggccctcgt gactaggctc atctagatgg tgctcacgct 4860
ggtgtttgag gcatttccac tgtgatctcc acgaggggat gttttccggg acacatctct 4920
ggctctggga actgcctgac tcactgaaga aactactttt caggcactgt agggtcaccc 4980
atatgcctcc agctcagttg acgcttaaaa acaggtgcag aaaagctcgc gatggaaggt 5040
cttaatgaga gtgtctgtct atgccaatca tgtaaaatga cgtttcttga aaaagattca 5100
gtggttcagc tttgtcagca tcatctcaac acaagcctgc tggctctttt tagcatctca 5160
tccaaccatg tcatcgtcca gatgagaaat cttagcccag gtgaggggag taacttgctt 5220
gaggtcacac agctggctgt tggcaaagct gggattagaa ccctcaaccc agggtccctt 5280
cctctgcagc gcctacatgg ttggttgaat aagtggctgc gtttcctggg gccctgggtt 5340
ttggggaagc cagttagctg ctgctttggc actggcatgg aggtgagcag tcaaggatgc 5400
tggtgaggct gcagtttctg ctctttttca tcagggggga tagtctctag gatttttcag 5460
tgaggaccct tgggctttgg atgcagcttg aaccaagaaa acgaggaggg aaagggattc 5520
agtgaactat tcctcagtgg gatcggttct tcagctcctg atgggggctg tgtaatgggg 5580
gcagaggcca gggaaaaaga tgctgttcac ccaccctcag cttccctttt cctaaattaa 5640
gaggaaaagt ggtcaaagaa aaactcttca tttctccctg attcttaaac gaaggtggtt 5700
aatagaaact caggctcccg tgacaaggca ggacaagagc ctgtttcgct ttcctccctg 5760
accctgccag gtgccaactc aaacactacc tttctcattg gtttctaagt cagtagagac 5820
agatctgttt taagcagttg gggttcgag tagatctcat gggtacagga ggccagcagg 5880
gaccaggcca gtcagccatg ctcaggaccc ctcggctcct cccccagcct ctagctaccc 5940
tgtatcgagg caaggggagg ccagtaaagt ttgccaagcc tgatcctgca gcctggtggg 6000
gctggctggg gtattctttt accaaactct gttttaccgc cagccccttg tacaccccaa 6060
tcccatgtct ccctcccttc agctggaccg tgtgcccctt tgggaggaag aagacaagcc 6120
ccactagggc caagggcagc agagccctgc cgagtgagag gctgtggggc agcggctctg 6180
tcctgtgcct taccagccct ggggaggggg gcatttggct ggaagactgg aatttaattg 6240
ccatcgtctt tgattttgtg acatttctgc ttggaagtgt gaactacccc cccccccccg 6300
cttcctgctc cttagcatgc gtgcagctct ctcctgtttt gggtgttccc cttggacact 6360
ccagctcggg gactgctggc gtgtgaatgt gcagattccc ctgtgtggtc gaacctaaga 6420
actgtggctt ggaagtgatg ctccatgtga cgacgacttt gctttctttc ctcttagtga 6480
ggaggtgatt cgtagatccc aactgcctat gtaatgtaaa taatgtacat ttaatttatt 6540
gctatggtag cacattgtat ttgttaatgt acaaaacaaa ttctaaaagg ttgacaaatg 6600
tatattttgt tgcttaaatg tgtctttgca gaaattgaca ataaataaca tattttgtgt 6660

cca 6663

<210> 140
<211> 6618
<212> DNA
<213> Homo sapiens

<400> 140
actgaccccg ctgtaccacg gccctcttgc ggacagcccc ggggacgtcg ttgggacatc 60

gctgggaccc cgggctctgc agccacaacc atgtttggcc ggaagcgcag tgtctccttt 120

gggggcttcg gatggatcga caagaccatg ctggcaagtc tgaaggtcaa gaagcaggag 180

ctggccaaca gctcggatgc gaccctccca gaccggccgc tctcccctcc tctcacggca 240

cctcccacca tgaagtcgtc ggagttcttt gagatgctgg agaaaatgca ggacgactat 300

atcccatacc ccagcatcga cgaggttgtg gagaagggag gcccgtaccc tcaggtcatc 360

ctgccacagt ttgggggcta ttggatcgag gacccggaga acgtgggcac cccaacatcg 420

ctggggagca gcatctgtga ggaggaggaa gaggacaacc tcagccccaa cacatttggc 480

tacaagctcg agtgcaaggg tgaagccagg gcctaccgga ggcacttcct ggggaaggat 540

catctaaact tttactgtac cggcagcagc ctggggaact tgatcctgtc cgtcaagtgc 600

gaggaagcag aggggatcga gtacctccgg gtcatcctta ggtccaaact gaagacggta 660

catgagcgga tccccttggc tggactgagc aagcttccca gtgtccctca gattgcaaag 720

gctttctgtg atgatgcagt gggactgaga ttcaatcctg tcctgtaccc caaggcctcc 780

caaatgattg tgtcctatga tgagcatgaa gtcaacaaca cattcaaatt cggagtcatt 840

tatcaaaaag ccaggcagac cctggaggag gagctatttg ggaacaatga ggagagccca 900

gcttttaagg agttcttgga cctgctgggg gacacgatca cactgcagga tttcaaaggt 960

ttccgaggag gcctggacgt gacccacgga cagacagggg tggaatcagt gtacacaaca 1020

ttccgggaca gggagatcat gtttcacgtt tccacaaagc tgccatttac cgacggagac 1080

gcccagcagc tccagagaaa gagacacatt ggaaatgaca tcgtggccat catcttccaa 1140

gaggaaaaca cgccgtttgt cccagacatg atagcctcca atttcttaca tgcctacatc 1200

gtcgtgcagg tcgagacccc aggcacagag accccatcct acaaggtctc tgtcactgcg 1260

cgggaagatg tgcccacctt tggtccacct ctgcccagtc cccccgtttt ccagaagggc 1320

ccggaattca gggagtttct gctcaccaag ctcaccaatg ccgagaacgc ctgctgcaag 1380

tcggacaagt ttgcaaagct ggaggaccgg accagggctg ccctcctgga caaccttcac 1440

gatgagctcc acgcccacac acaggccatg ctgggactgg gcccagagga ggacaagttt 1500

gagaatggag gccacggggg gttcctggag tcttttaaga gggccatccg cgtacgcagc 1560

cactccatgg agaccatggt gggcggccag aagaagtcgc acagtggggg catccctggc 1620

agcctcagcg ggggcatctc ccacaacagc atggaggtca ccaagaccac cttctcgcct 1680

ccagtggtgg cggcaacggt gaagaaccag tcacggagtc ccatcaagcg acgctcgggg 1740

ctcttccccc gcctgcacac gggctcagaa ggccagggcg acagccgggc acgatgtgac 1800

agcacatcca gcacacccaa gaccccagat ggtggacact cctctcagga gataaagtct 1860

gagacctcat ccaatcccag ctctccggaa atctgcccca acaaggagaa gcccttcatg 1920

aagttgaagg aaaacggccg tgccatctcc cgctcctcct ccagcaccag cagcgtcagc 1980

agcactgcag gggagggcga ggccatggag gagggcgaca gtgggggcag ccagccgtcc 2040

acgacctcac ccttcaagca ggaggtgttt gtctacagcc cgtccccgag cagcgagagc 2100

cccagcctgg gggcagctgc cacccccgatc atcatgagcc ggagtcccac agatgccaaa 2160

agcagaaact ccccgagatc gaacctgaaa ttccgctttg acaagctcag ccatgccagc 2220

tctggtgcgg gtcactaatg tgaaagtgga gtccttcgcc tgtccaaggg aatcccctct 2280

tctgtcctgg aaaaggctcc tgtaccagca gtttgggagt gccgtccacg accctgacag 2340

tcccagccct gctgccccat ggccacgtgc ccacagatgt gctgttggtc caggtgtccc 2400

agtctggcca cagccctgcc tccgccctca cctacatgcc ctcccagccc ctcccatctc 2460

tggacgaggc ctccttcctc aggttcctcc tgctcctgac ctcccagtgt gatgtccggg 2520

tcctttatca tcctattcat cctggagagg aaaagtgtcg ggcaaagggg gatctggggg 2580

gagctcagca gtgactgggg agctggtctg cctcagagac agagtagggg gtgggagcag 2640

agcctcggtg agggtcttgg ccacagggca gtgccttcct gaacgtggca ggctttacta 2700

ccaggaacgc actcggtggt ggaggcccca tgttcccagg agccaagatt cgtagcatcc 2760

ttgaggccat cctgataaaa ttcggcgcta ttgcccccgt agctctggag ctctaaaccg 2820

tctatctgct tctgtgctga acgcctttcc catctgctga cgtaggccca gggctgccct 2880

gcccctgctg ccagtgtacc gtgagcgggg ctccagccag ttcaagctca gagccagagc 2940

tggacgggcc agaactgcgc tgcacacttc ctggactgag gcggggactt tgggtcccac 3000

ccggtttctc ctgattatgg ctgctgtggg gtgaggggag ggaggggcag ccccgaggca 3060

gtctcttccc tttgagaaga tattttccca caaaggggtg ggaagccagg agtgagaagg 3120

aattcaggga gagcaaagga gccagtgctg agatgctgct gtgttggttg aggaaaacct 3180

cgggcctgag ggccaggccg gagcccaggt ctctgctgac aatgggggtt caaggaagac 3240

gtcgttatct cccctcccca cttactcgag gagagaggtg aggggggat gacttgcggg 3300

ttctgatcag gcccctgggt ggggaagggg cacagtgtcc ctcagcagct tacgcccctg 3360

gagtcttggg gggcccagcc tggccctggg gccttttcca gctactgtgc ccttgggcag 3420

ctgcgtctgg ggctcaaccc cccaatcctg ttcccctctc cagctgcggg tctgtaggca 3480

gctgtcacat ctgaagggtt tctgcaacct ggaccccatc tgggtgtggg tcagaccctg 3540

tgacccacat gccaccccca ccctccacag agcccccttg ctgggacagc cagctcacct 3600

ccaaggacat cccctcctgg cttctccccc ttccgagtct gcagcgccgt gggcttctct 3660

gccgatgggc ccgggttggg gttaaggtgg gcatcctcca ggtacaacga gcctgaagag 3720

ccccttttcag tgcagacggg gctgcagagt gacactggct gggcacctgc cccacgacca 3780

atgacaagga tttccagctg aatgctttat tcccataggg atctggacct gtgcccaaga 3840

tataaatact acactttttt tttttttttt taactgacat tgtgaaattc tccctatagc 3900

ttttgccatt caagcaacat tgtgatcttt cttccccgcc acgtgtgtgg gaatgattga 3960

gtcctgtttg caagctggag aggagctctc cctttgctag tactttctct aaagtactag 4020

tctagtaaaa tttattcttg ttagaaggtc aacaaaatat ctgtttagct tttatgaaga 4080

gtcaccgtag cagcccccac ggctggaaag aggcctgtac gttctggacg cgttttgttg 4140

gctgggcttc tggaggcact ggcaaggtca aactgcattt ctttaagaac agttgcagga 4200

tctggctcgc ctctgtggga agccggcatt acaggtgctt ggtggatggg ccgtgtcaca 4260

ttgccatctg ggtcctttg gggtttccag gttgtcacca tgctgtccca tttgggaatc 4320

ccatacctgc ctgtccccac tgcgctggct gacccttgct gcctgctgcc tcttgggagg 4380

gctttgtccc tgcctctgag ctggtgggca ggatggctgg gtggccccca gagaagcaca 4440

gacctgagat ggggtctcca tgcccggttt gctgttggaa tgatctgaac aggaccccaa 4500

atgcctcttc cctctggtca tgcctcacta tctctaggag ctccatcctg tggcttccag 4560

agtgtgcact tccagcccac ccgggcagtg ctgagaggga ggaggagaac aaggatggcc 4620

cagcctcccc tccctcccct agaccacggg gcgggcagct cgggttcctg gagggctgtt 4680

tccccccacgc tgtccctaca tctgctctga tctaaaatgt ctttcctttt atgctgcgcc 4740

cagtcttggg gctcaaagat ttgcccaaac ctcattggcc ctcgtgacta ggctcatcta 4800

gatggtgctc acgctggtgt ttgaggcatt tccactgtga tctccacgag ggatgtttt 4860

ccgggacaca tctctggctc tgggaactgc ctgactcact gaagaaacta cttttcaggc 4920

actgtagggt cacccatatg cctccagctc agttgacgct taaaaacagg tgcagaaaag 4980

ctcgcgatgg aaggtcttaa tgagagtgtc tgtctatgcc aatcatgtaa aatgacgttt 5040

cttgaaaaag attcagtggt tcagctttgt cagcatcatc tcaacacaag cctgctggct 5100

ctttttagca tctcatccaa ccatgtcatc gtccagatga gaaatcttag cccaggtgag 5160

gggagtaact tgcttgaggt cacacagctg gctgttggca aagctgggat tagaaccctc 5220

aacccagggt cccttcctct gcagcgccta catggttggt tgaataagtg gctgcgtttc 5280

ctggggccct gggttttggg gaagccagtt agctgctgct ttggcactgg catggaggtg 5340

agcagtcaag gatgctggtg aggctgcagt ttctgctctt tttcatcagg ggggatagtc 5400

tctaggattt ttcagtgagg acccttgggc tttggatgca gcttgaacca agaaaacgag 5460

gagggaaagg gattcagtga actattcctc agtgggatcg gttcttcagc tcctgatggg 5520

ggctgtgtaa tgggggcaga ggccagggaa aaagatgctg ttcacccacc ctcagcttcc 5580

cttttcctaa attaagagga aaagtggtca aagaaaaact cttcatttct ccctgattct 5640

taaacgaagg tggttaatag aaactcaggc tcccgtgaca aggcaggaca agagcctgtt 5700

tcgctttcct ccctgaccct gccaggtgcc aactcaaaca ctacctttct cattggtttc 5760

taagtcagta gagacagatc tgttttaagc agttgggggt tcgagtagat ctcatgggta 5820

caggaggcca gcagggacca ggccagtcag ccatgctcag gacccctcgg ctcctccccc 5880

agcctctagc taccctgtat cgaggcaagg ggaggccagt aaagtttgcc aagcctgatc 5940

ctgcagcctg gtggggctgg ctggggtatt cttttaccaa actctgtttt accgccagcc 6000

ccttgtacac cccaatccca tgtctccctc ccttcagctg gaccgtgtgc ccctttggga 6060

ggaagaagac aagccccact agggccaagg gcagcagagc cctgccgagt gagaggctgt 6120

ggggcagcgg ctctgtcctg tgccttacca gccctgggga ggggggcatt tggctggaag 6180

actggaattt aattgccatc gtctttgatt ttgtgacatt tctgcttgga agtgtgaact 6240

accccccccc ccccgcttcc tgctccttag catgcgtgca gctctctcct gttttgggtg 6300

ttccccttgg acactccagc tcggggactg ctggcgtgtg aatgtgcaga ttcccctgtg 6360

tggtcgaacc taagaactgt ggcttggaag tgatgctcca tgtgacgacg actttgcttt 6420

ctttcctctt agtgaggagg tgattcgtag atcccaactg cctatgtaat gtaaataatg 6480

tacatttaat ttattgctat ggtagcacat tgtatttgtt aatgtacaaa acaaattcta 6540

aaaggttgac aaatgtatat tttgttgctt aaatgtgtct ttgcagaaat tgacaataaa 6600

taacatattt tgtgtcca 6618

<210> 141
<211> 6719
<212> DNA
<213> Homo sapiens

<400> 141
gtgcgctggg gccggccggg gtgcgcgcgt gcgcggcagg actgctgcga gggaccccgc 60

cgcccccggag gaggaggagg aggaggagga ggaggaggag ggggctgggc cgagggaggg 120

ggcgaccgcg gggactgagg tgcccttcgc tccgggtcca gaggcagccg cgcgccaggc 180

ggcgcagaag gatcgacaag accatgctgg caagtctgaa ggtcaagaag caggagctgg 240

ccaacagctc ggatgcgacc ctcccagacc ggccgctctc ccctcctctc acggcacctc 300

ccaccatgaa gtcgtcggag ttctttgaga tgctggagaa aatgcagggg atcaagcttg 360

aagagcagaa gccgggaccc cagaagaaca aggacgacta tatcccatac cccagcatcg 420

acgaggttgt ggagaaggga ggcccgtacc ctcaggtcat cctgccacag tttgggggct 480

attggatcga ggacccggag aacgtgggca ccccaacatc gctggggagc agcatctgtg 540

aggaggagga agaggacaac ctcagcccca acacatttgg ctacaagctc gagtgcaagg 600

gtgaagccag ggcctaccgg aggcacttcc tggggaagga tcatctaaac ttttactgta 660

ccggcagcag cctggggaac ttgatcctgt ccgtcaagtg cgaggaagca gaggggatcg 720

agtacctccg ggtcatcctt aggtccaaac tgaagacggt acatgagcgg atccccttgg 780

ctggactgag caagcttccc agtgtccctc agattgcaaa ggctttctgt gatgatgcag 840

tgggactgag attcaatcct gtcctgtacc ccaaggcctc ccaaatgatt gtgtcctatg 900

atgagcatga agtcaacaac acattcaaat tcggagtcat ttatcaaaaa gccaggcaga 960

ccctggagga ggagctattt gggaacaatg aggagagccc agcttttaag gagttcttgg 1020

acctgctggg ggacacgatc acactgcagg atttcaaagg tttccgagga ggcctggacg 1080

tgacccacgg acagacaggg gtggaatcag tgtacacaac attccgggac agggagatca 1140

tgtttcacgt ttccacaaag ctgccattta ccgacggaga cgcccagcag ctccagagaa 1200

agagacacat tggaaatgac atcgtggcca tcatcttcca agaggaaaac acgccgtttg 1260

tcccagacat gatagcctcc aatttcttac atgcctacat cgtcgtgcag gtcgagaccc 1320

caggcacaga gaccccatcc tacaaggtct ctgtcactgc gcgggaagat gtgcccacct 1380

ttggtccacc tctgcccagt ccccccgttt tccagaaggg cccggaattc agggagtttc 1440

tgctcaccaa gctcaccaat gccgagaacg cctgctgcaa gtcggacaag tttgcaaagc 1500

tggaggaccg gaccagggct gccctcctgg acaaccttca cgatgagctc cacgcccaca 1560

cacaggccat gctgggactg ggcccagagg aggacaagtt tgagaatgga ggccacgggg 1620

ggttcctgga gtcttttaag agggccatcc gcgtacgcag ccactccatg gagaccatgg 1680

tgggcggcca gaagaagtcg cacagtgggg gcatccctgg cagcctcagc gggggcatct 1740

cccacaacag catggaggtc accaagacca ccttctcgcc tccagtggtg gcggcaacgg 1800

tgaagaacca gtcacggagt cccatcaagc gacgctcggg gctcttcccc cgcctgcaca 1860

cgggctcaga aggccagggc gacagccggg cacgatgtga cagcacatcc agcacaccca 1920

agaccccaga tggtggacac tcctctcagg agataaagtc tgagacctca tccaatccca 1980

gctctccgga aatctgcccc aacaaggaga agcccttcat gaagttgaag gaaaacggcc 2040

gtgccatctc ccgctcctcc tccagcacca gcagcgtcag cagcactgca ggggagggcg 2100

aggccatgga ggagggcgac agtgggggca gccagccgtc cacgacctca cccttcaagc 2160

aggaggtgtt tgtctacagc ccgtccccga gcagcgagag ccccagcctg gggcagctg 2220

ccaccccgat catcatgagc cggagtccca cagatgccaa aagcagaaac tccccgagat 2280

cgaacctgaa attccgcttt gacaagctca gccatgccag ctctggtgcg ggtcactaat 2340
gtgaaagtgg agtccttcgc ctgtccaagg gaatcccctc ttctgtcctg gaaaaggctc 2400
ctgtaccagc agtttgggag tgccgtccac gaccctgaca gtcccagccc tgctgcccca 2460
tggccacgtg cccacagatg tgctgttggt ccaggtgtcc cagtctggcc acagccctgc 2520
ctccgccctc acctacatgc cctcccagcc cctcccatct ctggacgagg cctccttcct 2580
caggttcctc ctgctcctga cctcccagtg tgatgtccgg gtcctttatc atcctattca 2640
tcctggagag gaaaagtgtc gggcaaaggg ggatctgggg ggagctcagc agtgactggg 2700
gagctggtct gcctcagaga cagagtaggg ggtgggagca gagcctcggt gagggtcttg 2760
gccacagggc agtgccttcc tgaacgtggc aggctttact accaggaacg cactcggtgg 2820
tggaggcccc atgttcccag gagccaagat tcgtagcatc cttgaggcca tcctgataaa 2880
attcggcgct attgcccccg tagctctgga gctctaaacc gtctatctgc ttctgtgctg 2940
aacgcctttc ccatctgctg acgtaggccc agggctgccc tgcccctgct gccagtgtac 3000
cgtgagcggg gctccagcca gttcaagctc agagccagag ctggacgggc cagaactgcg 3060
ctgcacactt cctggactga ggcggggact ttgggtccca cccggtttct cctgattatg 3120
gctgctgtgg ggtgagggga gggaggggca gccccgaggc agtctcttcc ctttgagaag 3180
atattttccc acaaaggggt gggaagccag gagtgagaag gaattcaggg agagcaaagg 3240
agccagtgct gagatgctgc tgtgttggtt gaggaaaacc tcgggcctga gggccaggcc 3300
ggagcccagg tctctgctga caatgggggt tcaaggaaga cgtcgttatc tcccctcccc 3360
acttactcga ggagagaggt gagggggga tgacttgcgg gttctgatca ggcccctggg 3420
tggggaaggg gcacagtgtc cctcagcagc ttacgcccct ggagtcttgg ggggcccagc 3480
ctggccctgg ggccttttcc agctactgtg cccttgggca gctgcgtctg gggctcaacc 3540
ccccaatcct gttcccctct ccagctgcgg gtctgtaggc agctgtcaca tctgaagggt 3600
ttctgcaacc tggaccccat ctgggtgtgg gtcagaccct gtgacccaca tgccaccccc 3660
accctccaca gagcccctt gctgggacag ccagctcacc tccaaggaca tcccctcctg 3720
gcttctcccc cttccgagtc tgcagcgccg tgggcttctc tgccgatggg cccgggttgg 3780
ggttaaggtg ggcatcctcc aggtacaacg agcctgaaga gccccttca gtgcagacgg 3840
ggctgcagag tgacactggc tgggcacctg ccccacgacc aatgacaagg atttccagct 3900
gaatgcttta ttcccatagg gatctggacc tgtgcccaag atataaatac tacactttt 3960
tttttttt ttaactgaca ttgtgaaatt ctccctatag cttttgccat tcaagcaaca 4020
ttgtgatctt tcttccccgc cacgtgtgtg ggaatgattg agtcctgttt gcaagctgga 4080
gaggagctct ccctttgcta gtactttctc taaagtacta gtctagtaaa atttattctt 4140
gttagaaggt caacaaaata tctgtttagc ttttatgaag agtcaccgta gcagccccca 4200

cggctggaaa gaggcctgta cgttctggac gcgttttgtt ggctgggctt ctggaggcac 4260
tggcaaggtc aaactgcatt tctttaagaa cagttgcagg atctggctcg cctctgtggg 4320
aagccggcat tacaggtgct tggtggatgg gccgtgtcac attgccatct ggggtccttt 4380
ggggtttcca ggttgtcacc atgctgtccc atttgggaat cccatacctg cctgtcccca 4440
ctgcgctggc tgacccttgc tgcctgctgc ctcttgggag ggctttgtcc ctgcctctga 4500
gctggtgggc aggatggctg ggtggccccc agagaagcac agacctgaga tggggtctcc 4560
atgcccggtt tgctgttgga atgatctgaa caggacccca aatgcctctt ccctctggtc 4620
atgcctcact atctctagga gctccatcct gtggcttcca gagtgtgcac ttccagccca 4680
cccgggcagt gctgagaggg aggaggagaa caaggatggc ccagcctccc ctccctcccc 4740
tagaccacgg ggcgggcagc tcgggttcct ggagggctgt ttcccccacg ctgtccctac 4800
atctgctctg atctaaaatg tctttccttt tatgctgcgc ccagtcttgg ggctcaaaga 4860
tttgcccaaa cctcattggc cctcgtgact aggctcatct agatggtgct cacgctggtg 4920
tttgaggcat ttccactgtg atctccacga ggggatgttt tccgggacac atctctggct 4980
ctgggaactg cctgactcac tgaagaaact acttttcagg cactgtaggg tcacccatat 5040
gcctccagct cagttgacgc ttaaaaacag gtgcagaaaa gctcgcgatg gaaggtctta 5100
atgagagtgt ctgtctatgc caatcatgta aaatgacgtt tcttgaaaaa gattcagtgg 5160
ttcagctttg tcagcatcat ctcaacacaa gcctgctggc tctttttagc atctcatcca 5220
accatgtcat cgtccagatg agaaatctta gcccaggtga ggggagtaac ttgcttgagg 5280
tcacacagct ggctgttggc aaagctggga ttagaaccct caacccaggg tcccttcctc 5340
tgcagcgcct acatggttgg ttgaataagt ggctgcgttt cctggggccc tgggttttgg 5400
ggaagccagt tagctgctgc tttggcactg gcatggaggt gagcagtcaa ggatgctggt 5460
gaggctgcag tttctgctct ttttcatcag gggggatagt ctctaggatt tttcagtgag 5520
gacccttggg ctttggatgc agcttgaacc aagaaaacga ggagggaaag ggattcagtg 5580
aactattcct cagtgggatc ggttcttcag ctcctgatgg gggctgtgta atgggggcag 5640
aggccaggga aaaagatgct gttcacccac cctcagcttc ccttttccta aattaagagg 5700
aaaagtggtc aaagaaaaac tcttcatttc tccctgattc ttaaacgaag gtggttaata 5760
gaaactcagg ctcccgtgac aaggcaggac aagagcctgt ttcgctttcc tccctgaccc 5820
tgccaggtgc caactcaaac actacctttc tcattggttt ctaagtcagt agagacagat 5880
ctgttttaag cagttggggg ttcgagtaga tctcatgggt acaggaggcc agcagggacc 5940
aggccagtca gccatgctca ggacccctcg gctcctcccc cagcctctag ctaccctgta 6000
tcgaggcaag ggaggccag taaagtttgc caagcctgat cctgcagcct ggtggggctg 6060

gctggggtat tcttttacca aactctgttt taccgccagc cccttgtaca ccccaatccc 6120

atgtctccct cccttcagct ggaccgtgtg cccctttggg aggaagaaga caagccccac 6180

tagggccaag ggcagcagag ccctgccgag tgagaggctg tggggcagcg gctctgtcct 6240

gtgccttacc agccctgggg agggggcat ttggctggaa gactggaatt taattgccat 6300

cgtctttgat tttgtgacat ttctgcttgg aagtgtgaac taccccccc cccccgcttc 6360

ctgctcctta gcatgcgtgc agctctctcc tgttttgggt gttccccttg gacactccag 6420

ctcggggact gctggcgtgt gaatgtgcag attcccctgt gtggtcgaac ctaagaactg 6480

tggcttggaa gtgatgctcc atgtgacgac gactttgctt tctttcctct tagtgaggag 6540

gtgattcgta gatcccaact gcctatgtaa tgtaaataat gtacatttaa tttattgcta 6600

tggtagcaca ttgtatttgt taatgtacaa aacaaattct aaaaggttga caaatgtata 6660

ttttgttgct taaatgtgtc tttgcagaaa ttgacaataa ataacatatt ttgtgtcca 6719

<210> 142
<211> 730
<212> PRT
<213> Homo sapiens

<400> 142

Met Phe Gly Arg Lys Arg Ser Val Ser Phe Gly Gly Phe Gly Trp Ile
1 5 10 15

Asp Lys Thr Met Leu Ala Ser Leu Lys Val Lys Lys Gln Glu Leu Ala
20 25 30

Asn Ser Ser Asp Ala Thr Leu Pro Asp Arg Pro Leu Ser Pro Pro Leu
35 40 45

Thr Ala Pro Pro Thr Met Lys Ser Ser Glu Phe Phe Glu Met Leu Glu
50 55 60

Lys Met Gln Gly Ile Lys Leu Glu Glu Gln Lys Pro Gly Pro Gln Lys
65 70 75 80

Asn Lys Asp Asp Tyr Ile Pro Tyr Pro Ser Ile Asp Glu Val Val Glu
85 90 95

Lys Gly Gly Pro Tyr Pro Gln Val Ile Leu Pro Gln Phe Gly Gly Tyr
100 105 110

Trp Ile Glu Asp Pro Glu Asn Val Gly Thr Pro Thr Ser Leu Gly Ser
115 120 125

Ser Ile Cys Glu Glu Glu Glu Glu Asp Asn Leu Ser Pro Asn Thr Phe

130 135 140

Gly Tyr Lys Leu Glu Cys Lys Gly Glu Ala Arg Ala Tyr Arg Arg His
145 150 155 160

Phe Leu Gly Lys Asp His Leu Asn Phe Tyr Cys Thr Gly Ser Ser Leu
165 170 175

Gly Asn Leu Ile Leu Ser Val Lys Cys Glu Glu Ala Glu Gly Ile Glu
180 185 190

Tyr Leu Arg Val Ile Leu Arg Ser Lys Leu Lys Thr Val His Glu Arg
195 200 205

Ile Pro Leu Ala Gly Leu Ser Lys Leu Pro Ser Val Pro Gln Ile Ala
210 215 220

Lys Ala Phe Cys Asp Asp Ala Val Gly Leu Arg Phe Asn Pro Val Leu
225 230 235 240

Tyr Pro Lys Ala Ser Gln Met Ile Val Ser Tyr Asp Glu His Glu Val
245 250 255

Asn Asn Thr Phe Lys Phe Gly Val Ile Tyr Gln Lys Ala Arg Gln Thr
260 265 270

Leu Glu Glu Glu Leu Phe Gly Asn Asn Glu Glu Ser Pro Ala Phe Lys
275 280 285

Glu Phe Leu Asp Leu Leu Gly Asp Thr Ile Thr Leu Gln Asp Phe Lys
290 295 300

Gly Phe Arg Gly Gly Leu Asp Val Thr His Gly Gln Thr Gly Val Glu
305 310 315 320

Ser Val Tyr Thr Thr Phe Arg Asp Arg Glu Ile Met Phe His Val Ser
325 330 335

Thr Lys Leu Pro Phe Thr Asp Gly Asp Ala Gln Gln Leu Gln Arg Lys
340 345 350

Arg His Ile Gly Asn Asp Ile Val Ala Ile Ile Phe Gln Glu Glu Asn
355 360 365

Thr Pro Phe Val Pro Asp Met Ile Ala Ser Asn Phe Leu His Ala Tyr
370 375 380

```
Ile Val Val Gln Val Glu Thr Pro Gly Thr Glu Thr Pro Ser Tyr Lys
385                 390                 395                 400

Val Ser Val Thr Ala Arg Glu Asp Val Pro Thr Phe Gly Pro Pro Leu
                405                 410                 415

Pro Ser Pro Pro Val Phe Gln Lys Gly Pro Glu Phe Arg Glu Phe Leu
            420                 425                 430

Leu Thr Lys Leu Thr Asn Ala Glu Asn Ala Cys Cys Lys Ser Asp Lys
        435                 440                 445

Phe Ala Lys Leu Glu Asp Arg Thr Arg Ala Ala Leu Leu Asp Asn Leu
    450                 455                 460

His Asp Glu Leu His Ala His Thr Gln Ala Met Leu Gly Leu Gly Pro
465                 470                 475                 480

Glu Glu Asp Lys Phe Glu Asn Gly Gly His Gly Gly Phe Leu Glu Ser
                485                 490                 495

Phe Lys Arg Ala Ile Arg Val Arg Ser His Ser Met Glu Thr Met Val
            500                 505                 510

Gly Gly Gln Lys Lys Ser His Ser Gly Gly Ile Pro Gly Ser Leu Ser
        515                 520                 525

Gly Gly Ile Ser His Asn Ser Met Glu Val Thr Lys Thr Thr Phe Ser
    530                 535                 540

Pro Pro Val Val Ala Ala Thr Val Lys Asn Gln Ser Arg Ser Pro Ile
545                 550                 555                 560

Lys Arg Arg Ser Gly Leu Phe Pro Arg Leu His Thr Gly Ser Glu Gly
                565                 570                 575

Gln Gly Asp Ser Arg Ala Arg Cys Asp Ser Thr Ser Ser Thr Pro Lys
            580                 585                 590

Thr Pro Asp Gly Gly His Ser Ser Gln Glu Ile Lys Ser Glu Thr Ser
        595                 600                 605

Ser Asn Pro Ser Ser Pro Glu Ile Cys Pro Asn Lys Glu Lys Pro Phe
    610                 615                 620

Met Lys Leu Lys Glu Asn Gly Arg Ala Ile Ser Arg Ser Ser Ser Ser
625                 630                 635                 640
```

Thr Ser Ser Val Ser Ser Thr Ala Gly Glu Gly Glu Ala Met Glu Glu
645 650 655

Gly Asp Ser Gly Gly Ser Gln Pro Ser Thr Thr Ser Pro Phe Lys Gln
660 665 670

Glu Val Phe Val Tyr Ser Pro Ser Pro Ser Ser Glu Ser Pro Ser Leu
675 680 685

Gly Ala Ala Ala Thr Pro Ile Ile Met Ser Arg Ser Pro Thr Asp Ala
690 695 700

Lys Ser Arg Asn Ser Pro Arg Ser Asn Leu Lys Phe Arg Phe Asp Lys
705 710 715 720

Leu Ser His Ala Ser Ser Gly Ala Gly His
725 730

<210> 143
<211> 715
<212> PRT
<213> Homo sapiens

<400> 143

Met Phe Gly Arg Lys Arg Ser Val Ser Phe Gly Gly Phe Gly Trp Ile
1 5 10 15

Asp Lys Thr Met Leu Ala Ser Leu Lys Val Lys Lys Gln Glu Leu Ala
20 25 30

Asn Ser Ser Asp Ala Thr Leu Pro Asp Arg Pro Leu Ser Pro Pro Leu
35 40 45

Thr Ala Pro Pro Thr Met Lys Ser Ser Glu Phe Phe Glu Met Leu Glu
50 55 60

Lys Met Gln Asp Asp Tyr Ile Pro Tyr Pro Ser Ile Asp Glu Val Val
65 70 75 80

Glu Lys Gly Gly Pro Tyr Pro Gln Val Ile Leu Pro Gln Phe Gly Gly
85 90 95

Tyr Trp Ile Glu Asp Pro Glu Asn Val Gly Thr Pro Thr Ser Leu Gly
100 105 110

Ser Ser Ile Cys Glu Glu Glu Glu Glu Asp Asn Leu Ser Pro Asn Thr
115 120 125

```
Phe Gly Tyr Lys Leu Glu Cys Lys Gly Glu Ala Arg Ala Tyr Arg Arg
    130                 135                 140

His Phe Leu Gly Lys Asp His Leu Asn Phe Tyr Cys Thr Gly Ser Ser
145                 150                 155                 160

Leu Gly Asn Leu Ile Leu Ser Val Lys Cys Glu Glu Ala Glu Gly Ile
                165                 170                 175

Glu Tyr Leu Arg Val Ile Leu Arg Ser Lys Leu Lys Thr Val His Glu
            180                 185                 190

Arg Ile Pro Leu Ala Gly Leu Ser Lys Leu Pro Ser Val Pro Gln Ile
        195                 200                 205

Ala Lys Ala Phe Cys Asp Asp Ala Val Gly Leu Arg Phe Asn Pro Val
    210                 215                 220

Leu Tyr Pro Lys Ala Ser Gln Met Ile Val Ser Tyr Asp Glu His Glu
225                 230                 235                 240

Val Asn Asn Thr Phe Lys Phe Gly Val Ile Tyr Gln Lys Ala Arg Gln
                245                 250                 255

Thr Leu Glu Glu Glu Leu Phe Gly Asn Asn Glu Glu Ser Pro Ala Phe
            260                 265                 270

Lys Glu Phe Leu Asp Leu Leu Gly Asp Thr Ile Thr Leu Gln Asp Phe
        275                 280                 285

Lys Gly Phe Arg Gly Gly Leu Asp Val Thr His Gly Gln Thr Gly Val
    290                 295                 300

Glu Ser Val Tyr Thr Thr Phe Arg Asp Arg Glu Ile Met Phe His Val
305                 310                 315                 320

Ser Thr Lys Leu Pro Phe Thr Asp Gly Asp Ala Gln Gln Leu Gln Arg
                325                 330                 335

Lys Arg His Ile Gly Asn Asp Ile Val Ala Ile Ile Phe Gln Glu Glu
            340                 345                 350

Asn Thr Pro Phe Val Pro Asp Met Ile Ala Ser Asn Phe Leu His Ala
        355                 360                 365

Tyr Ile Val Val Gln Val Glu Thr Pro Gly Thr Glu Thr Pro Ser Tyr
    370                 375                 380
```

Lys Val Ser Val Thr Ala Arg Glu Asp Val Pro Thr Phe Gly Pro Pro
385 390 395 400

Leu Pro Ser Pro Pro Val Phe Gln Lys Gly Pro Glu Phe Arg Glu Phe
405 410 415

Leu Leu Thr Lys Leu Thr Asn Ala Glu Asn Ala Cys Cys Lys Ser Asp
420 425 430

Lys Phe Ala Lys Leu Glu Asp Arg Thr Arg Ala Ala Leu Leu Asp Asn
435 440 445

Leu His Asp Glu Leu His Ala His Thr Gln Ala Met Leu Gly Leu Gly
450 455 460

Pro Glu Glu Asp Lys Phe Glu Asn Gly Gly His Gly Gly Phe Leu Glu
465 470 475 480

Ser Phe Lys Arg Ala Ile Arg Val Arg Ser His Ser Met Glu Thr Met
485 490 495

Val Gly Gly Gln Lys Lys Ser His Ser Gly Gly Ile Pro Gly Ser Leu
500 505 510

Ser Gly Gly Ile Ser His Asn Ser Met Glu Val Thr Lys Thr Thr Phe
515 520 525

Ser Pro Pro Val Val Ala Ala Thr Val Lys Asn Gln Ser Arg Ser Pro
530 535 540

Ile Lys Arg Arg Ser Gly Leu Phe Pro Arg Leu His Thr Gly Ser Glu
545 550 555 560

Gly Gln Gly Asp Ser Arg Ala Arg Cys Asp Ser Thr Ser Ser Thr Pro
565 570 575

Lys Thr Pro Asp Gly Gly His Ser Ser Gln Glu Ile Lys Ser Glu Thr
580 585 590

Ser Ser Asn Pro Ser Ser Pro Glu Ile Cys Pro Asn Lys Glu Lys Pro
595 600 605

Phe Met Lys Leu Lys Glu Asn Gly Arg Ala Ile Ser Arg Ser Ser Ser
610 615 620

Ser Thr Ser Ser Val Ser Ser Thr Ala Gly Glu Gly Glu Ala Met Glu

625 630 635 640

Glu Gly Asp Ser Gly Gly Ser Gln Pro Ser Thr Thr Ser Pro Phe Lys
645 650 655

Gln Glu Val Phe Val Tyr Ser Pro Ser Pro Ser Ser Glu Ser Pro Ser
660 665 670

Leu Gly Ala Ala Ala Thr Pro Ile Ile Met Ser Arg Ser Pro Thr Asp
675 680 685

Ala Lys Ser Arg Asn Ser Pro Arg Ser Asn Leu Lys Phe Arg Phe Asp
690 695 700

Lys Leu Ser His Ala Ser Ser Gly Ala Gly His
705 710 715

<210> 144
<211> 711
<212> PRT
<213> Homo sapiens

<400> 144

Met Leu Ala Ser Leu Lys Val Lys Lys Gln Glu Leu Ala Asn Ser Ser
1 5 10 15

Asp Ala Thr Leu Pro Asp Arg Pro Leu Ser Pro Pro Leu Thr Ala Pro
20 25 30

Pro Thr Met Lys Ser Ser Glu Phe Phe Glu Met Leu Glu Lys Met Gln
35 40 45

Gly Ile Lys Leu Glu Glu Gln Lys Pro Gly Pro Gln Lys Asn Lys Asp
50 55 60

Asp Tyr Ile Pro Tyr Pro Ser Ile Asp Glu Val Val Glu Lys Gly Gly
65 70 75 80

Pro Tyr Pro Gln Val Ile Leu Pro Gln Phe Gly Gly Tyr Trp Ile Glu
85 90 95

Asp Pro Glu Asn Val Gly Thr Pro Thr Ser Leu Gly Ser Ser Ile Cys
100 105 110

Glu Glu Glu Glu Glu Asp Asn Leu Ser Pro Asn Thr Phe Gly Tyr Lys
115 120 125

Leu Glu Cys Lys Gly Glu Ala Arg Ala Tyr Arg Arg His Phe Leu Gly

```
        130                 135                 140

Lys Asp His Leu Asn Phe Tyr Cys Thr Gly Ser Ser Leu Gly Asn Leu
145                 150                 155                 160

Ile Leu Ser Val Lys Cys Glu Glu Ala Glu Gly Ile Glu Tyr Leu Arg
                165                 170                 175

Val Ile Leu Arg Ser Lys Leu Lys Thr Val His Glu Arg Ile Pro Leu
            180                 185                 190

Ala Gly Leu Ser Lys Leu Pro Ser Val Pro Gln Ile Ala Lys Ala Phe
        195                 200                 205

Cys Asp Asp Ala Val Gly Leu Arg Phe Asn Pro Val Leu Tyr Pro Lys
    210                 215                 220

Ala Ser Gln Met Ile Val Ser Tyr Asp Glu His Glu Val Asn Asn Thr
225                 230                 235                 240

Phe Lys Phe Gly Val Ile Tyr Gln Lys Ala Arg Gln Thr Leu Glu Glu
                245                 250                 255

Glu Leu Phe Gly Asn Asn Glu Glu Ser Pro Ala Phe Lys Glu Phe Leu
            260                 265                 270

Asp Leu Leu Gly Asp Thr Ile Thr Leu Gln Asp Phe Lys Gly Phe Arg
        275                 280                 285

Gly Gly Leu Asp Val Thr His Gly Gln Thr Gly Val Glu Ser Val Tyr
    290                 295                 300

Thr Thr Phe Arg Asp Arg Glu Ile Met Phe His Val Ser Thr Lys Leu
305                 310                 315                 320

Pro Phe Thr Asp Gly Asp Ala Gln Gln Leu Gln Arg Lys Arg His Ile
                325                 330                 335

Gly Asn Asp Ile Val Ala Ile Ile Phe Gln Glu Glu Asn Thr Pro Phe
            340                 345                 350

Val Pro Asp Met Ile Ala Ser Asn Phe Leu His Ala Tyr Ile Val Val
        355                 360                 365

Gln Val Glu Thr Pro Gly Thr Glu Thr Pro Ser Tyr Lys Val Ser Val
    370                 375                 380
```

Thr Ala Arg Glu Asp Val Pro Thr Phe Gly Pro Pro Leu Pro Ser Pro
385 390 395 400

Pro Val Phe Gln Lys Gly Pro Glu Phe Arg Glu Phe Leu Leu Thr Lys
405 410 415

Leu Thr Asn Ala Glu Asn Ala Cys Cys Lys Ser Asp Lys Phe Ala Lys
420 425 430

Leu Glu Asp Arg Thr Arg Ala Ala Leu Leu Asp Asn Leu His Asp Glu
435 440 445

Leu His Ala His Thr Gln Ala Met Leu Gly Leu Gly Pro Glu Glu Asp
450 455 460

Lys Phe Glu Asn Gly Gly His Gly Gly Phe Leu Glu Ser Phe Lys Arg
465 470 475 480

Ala Ile Arg Val Arg Ser His Ser Met Glu Thr Met Val Gly Gly Gln
485 490 495

Lys Lys Ser His Ser Gly Gly Ile Pro Gly Ser Leu Ser Gly Gly Ile
500 505 510

Ser His Asn Ser Met Glu Val Thr Lys Thr Thr Phe Ser Pro Pro Val
515 520 525

Val Ala Ala Thr Val Lys Asn Gln Ser Arg Ser Pro Ile Lys Arg Arg
530 535 540

Ser Gly Leu Phe Pro Arg Leu His Thr Gly Ser Glu Gly Gln Gly Asp
545 550 555 560

Ser Arg Ala Arg Cys Asp Ser Thr Ser Ser Thr Pro Lys Thr Pro Asp
565 570 575

Gly Gly His Ser Ser Gln Glu Ile Lys Ser Glu Thr Ser Ser Asn Pro
580 585 590

Ser Ser Pro Glu Ile Cys Pro Asn Lys Glu Lys Pro Phe Met Lys Leu
595 600 605

Lys Glu Asn Gly Arg Ala Ile Ser Arg Ser Ser Ser Ser Thr Ser Ser
610 615 620

Val Ser Ser Thr Ala Gly Glu Gly Glu Ala Met Glu Glu Gly Asp Ser
625 630 635 640

Gly Gly Ser Gln Pro Ser Thr Thr Ser Pro Phe Lys Gln Glu Val Phe
645 650 655

Val Tyr Ser Pro Ser Pro Ser Ser Glu Ser Pro Ser Leu Gly Ala Ala
660 665 670

Ala Thr Pro Ile Ile Met Ser Arg Ser Pro Thr Asp Ala Lys Ser Arg
675 680 685

Asn Ser Pro Arg Ser Asn Leu Lys Phe Arg Phe Asp Lys Leu Ser His
690 695 700

Ala Ser Ser Gly Ala Gly His
705 710

<210> 145
<211> 2732
<212> DNA
<213> Homo sapiens

<400> 145
gactgcggga gtggagccgg cgagagagtg gcagcggggg ctgatggaag tgcagtgggg 60

gctggagagg gcaccctact gtatccagca tgctccaagg ccacagctct gtgttccagg 120

ccttgctggg gaccttcttc acctggggga tgacagcagc tggggcagct ctcgtgttcg 180

tattctctag tggacagagg cggatcttag atggaagtct tggctttgct gcaggggtca 240

tgttggcagc ttcctattgg tctcttctgg ccccagcagt tgagatggcc acgtcctctg 300

ggggcttcgg tgcctttgcc ttcttccctg tggctgttgg cttcaccctt ggagcggctt 360

ttgtctactt ggctgacctc ctgatgcctc acttgggtgc agcagaagac ccccagacga 420

ccctggcact gaacttcggc tctacgttga tgaagaagaa gtctgatcct gagggtcccg 480

cgctgctctt ccctgagagt gaactttcca tccggataga caagagtgag aatggtgagg 540

catatcagag aaagaaggcg gcagccactg gccttccaga gggtcctgct gtccctgtgc 600

cttctcgagg gaatctggca cagcccggcg gcagcagctg gaggaggatc gcactgctca 660

tcttggccat cactatacac aacgttccag agggtctcgc tgttggagtt ggatttgggg 720

ctatagaaaa gacggcatct gctacctttg agagtgccag gaatttggcc attggaatcg 780

ggatccagaa tttccccgag ggcctggctg tcagccttcc cttgcgaggg gcaggcttct 840

ccacctggag agctttctgg tatgggcagc tgagcggcat ggtggagccc ctggccgggg 900

tctttggtgc ctttgccgtg gtgctggctg agcccatcct gccctacgct ctggcctttg 960

ctgccggtgc catggtctac gtggtcatgg acgacatcat ccccgaagcc cagatcagtg 1020

gtaatgggaa actggcatcc tgggcctcca tcctgggatt tgtagtgatg atgtcactgg 1080

acgttggcct gggctagggc tgagacgctt cggaccccgg gaaaggccat acgaagaaac 1140

agcagtggtt ggcttctatg ggacaacaag cttctttctt cacattaaaa ctttttttcct 1200

tcctctcttc ttcatctcat tatcctgatt gactctgatt ataatagaac catttttact 1260

ttgctttgag ggagattttt gatttaatgg ggaattttaa ggtgtcatgg aaatacagat 1320

tctttgtttt ggccactgaa tggactctct cttcagtggg attatcaagg aacttcagat 1380

cagggaaatc tccacttcgg gaccttctat ctgcctccca actcctcaag gtcacctata 1440

gaagcgagct accaaaagac gtctcctaag cattttggtg gcctagtgac tcagggcaga 1500

gtggccagca cacctctcat ccgcccctcc tgctccatca ctgctgagcc tctccccatc 1560

tagaatgttg gaactggagc atcataaaga tagcaagcta ccttccaagg ccgagccagc 1620

ccagagagga gcatgtcttc ctttacctcc ccctaaggag atactacatg ggagggggac 1680

acagaaaaag ggaaggaaat tggctagtct ggcttttttt tttttttttt ttaaaggcaa 1740

agattgacat tattgaagga aaggggatga ggacaactgt gaactcacag tgagccctgt 1800

ggaaagaaga gacagacaga gtgtgggttt gttcggaggc ctctgctgtc aatggattcc 1860

aggagcaagg ccatttgtcg cgctttccaa atttcttagg catttatttt gataagttta 1920

tagccatcat gtttctaaga gacttggaga caccagcaaa ctgctagaac tcaaactctt 1980

caattactca aagaaggagc catttcagtt aactcaagtg aatgaaagag ttttggaatc 2040

tgctgtgggt ccttccctgt tgaccatttg gtaacttata atctgacaaa aactcttgag 2100

ctgcaacagg ccttgccaga gggctcagga tgggaaagga agaaggggat aggaaaagaa 2160

gaggtaattt tacatttccc ctttaaagta aattttagcc aactcatcat tctgaaatgt 2220

ccctataaag aatgagtcga actagaccag aagccagcct actccttctt acatagcttc 2280

tccaacaggg gtagcaatga cctgtccact tcaaacacag ataaggcctg ccatcctcat 2340

tggttaaagg cacacgtgag actttcagtg ggctctgctg agaaggaagg cagcccagga 2400

gtcaggtatg caggcattgc attgtcagtg tctgctctca gagtttacac attcaattgc 2460

ttccaagggt gaatctcctg ctctgtgaat gctatcagac cccaaaggcc aaccttgggc 2520

tgggtctatg tacgttcttc cgaagcactg atgatcaaaa ttgaagacac attcagaggt 2580

ttgattggtt gagattaact ggtgtggtgg ttggtgtatg tatgttttat ttttatgtct 2640

ttgtatgtag ttctacataa tgcaaattgt gctttctgat ggacaagacc tcataactgt 2700

gattaatatc aataaaaagg ggatgttgtg ga 2732

<210> 146
<211> 2869
<212> DNA
<213> Homo sapiens

<400> 146

gactgcggga gtggagccgg cgagagagtg gcagcggggg ctgatggaag tgcagtgggg 60
gctggagagg gcaccctagt gagttggctt ttctctcctt cggtgccttt ggttgggctc 120
gaggcccggg gtcggaggcc atcaggacaa gagtgtggga cttggaaggg cagccacctg 180
gagcttggaa ggggctgtat ccagcatgct ccaaggccac agctctgtgt tccaggcctt 240
gctggggacc ttcttcacct ggggggatgac agcagctggg gcagctctcg tgttcgtatt 300
ctctagtgga cagaggcgga tcttagatgg aagtcttggc tttgctgcag ggtcatgtt 360
ggcagcttcc tattggtctc ttctggcccc agcagttgag atggccacgt cctctggggg 420
cttcggtgcc tttgccttct tccctgtggc tgttggcttc acccttggag cggcttttgt 480
ctacttggct gacctcctga tgcctcactt gggtgcagca gaagaccccc agacgaccct 540
ggcactgaac ttcggctcta cgttgatgaa gaagaagtct gatcctgagg gtcccgcgct 600
gctcttccct gagagtgaac tttccatccg gataggtaga gctgggcttc tttcagacaa 660
gagtgagaat ggtgaggcat atcagagaaa gaaggcggca gccactggcc ttccagaggg 720
tcctgctgtc cctgtgcctt ctcgagggaa tctggcacag cccggcggca gcagctggag 780
gaggatcgca ctgctcatct tggccatcac tatacacaac gttccagagg gtctcgctgt 840
tggagttgga tttggggcta tagaaaagac ggcatctgct acctttgaga gtgccaggaa 900
tttggccatt ggaatcggga tccagaattt ccccgagggc ctggctgtca gccttccctt 960
gcgaggggca ggcttctcca cctggagagc tttctggtat gggcagctga gcggcatggt 1020
ggagcccctg gccggggtct ttggtgcctt tgccgtggtg ctggctgagc ccatcctgcc 1080
ctacgctctg gcctttgctg ccggtgccat ggtctacgtg gtcatggacg acatcatccc 1140
cgaagcccag atcagtggta atgggaaact ggcatcctgg gcctccatcc tgggatttgt 1200
agtgatgatg tcactggacg ttggcctggg ctagggctga gacgcttcgg accccgggaa 1260
aggccatacg aagaaacagc agtggttggc ttctatggga caacaagctt ctttcttcac 1320
attaaaactt ttttccttcc tctcttcttc atctcattat cctgattgac tctgattata 1380
atagaaccat ttttactttg ctttgaggga gatttttgat ttaatgggga attttaaggt 1440
gtcatggaaa tacagattct ttgttttggc cactgaatgg actctctctt cagtgggatt 1500
atcaaggaac ttcagatcag ggaaatctcc acttcgggac cttctatctg cctcccaact 1560
cctcaaggtc acctatagaa gcgagctacc aaaagacgtc tcctaagcat tttggtggcc 1620
tagtgactca gggcagagtg gccagcacac ctctcatccg cccctcctgc tccatcactg 1680
ctgagcctct ccccatctag aatgttggaa ctggagcatc ataaagatag caagctacct 1740
tccaaggccg agccagccca gagaggagca tgtcttcctt tacctccccc taaggagata 1800
ctacatggga gggggacaca gaaaaaggga aggaaattgg ctagtctggc ttttttttt 1860
ttttttttta aaggcaaaga ttgacattat tgaaggaaag gggatgagga caactgtgaa 1920

ctcacagtga gccctgtgga aagaagagac agacagagtg tgggtttgtt cggaggcctc 1980

tgctgtcaat ggattccagg agcaaggcca tttgtcgcgc tttccaaatt tcttaggcat 2040

ttattttgat aagtttatag ccatcatgtt tctaagagac ttggagacac cagcaaactg 2100

ctagaactca aactcttcaa ttactcaaag aaggagccat ttcagttaac tcaagtgaat 2160

gaaagagttt tggaatctgc tgtgggtcct tccctgttga ccatttggta acttataatc 2220

tgacaaaaac tcttgagctg caacaggcct tgccagaggg ctcaggatgg gaaaggaaga 2280

aggggatagg aaaagaagag gtaattttac atttcccctt taaagtaaat tttagccaac 2340

tcatcattct gaaatgtccc tataaagaat gagtcgaact agaccagaag ccagcctact 2400

ccttcttaca tagcttctcc aacaggggta gcaatgacct gtccacttca aacacagata 2460

aggcctgcca tcctcattgg ttaaaggcac acgtgagact ttcagtgggc tctgctgaga 2520

aggaaggcag cccaggagtc aggtatgcag gcattgcatt gtcagtgtct gctctcagag 2580

tttacacatt caattgcttc caagggtgaa tctcctgctc tgtgaatgct atcagacccc 2640

aaaggccaac cttgggctgg gtctatgtac gttcttccga agcactgatg atcaaaattg 2700

aagacacatt cagaggtttg attggttgag attaactggt gtggtggttg gtgtatgtat 2760

gttttatttt tatgtctttg tatgtagttc tacataatgc aaattgtgct ttctgatgga 2820

caagacctca taactgtgat taatatcaat aaaaagggga tgttgtgga 2869

<210> 147
<211> 335
<212> PRT
<213> Homo sapiens

<400> 147

Met Leu Gln Gly His Ser Ser Val Phe Gln Ala Leu Leu Gly Thr Phe
1 5 10 15

Phe Thr Trp Gly Met Thr Ala Ala Gly Ala Ala Leu Val Phe Val Phe
20 25 30

Ser Ser Gly Gln Arg Arg Ile Leu Asp Gly Ser Leu Gly Phe Ala Ala
35 40 45

Gly Val Met Leu Ala Ala Ser Tyr Trp Ser Leu Leu Ala Pro Ala Val
50 55 60

Glu Met Ala Thr Ser Ser Gly Gly Phe Gly Ala Phe Ala Phe Phe Pro
65 70 75 80

Val Ala Val Gly Phe Thr Leu Gly Ala Ala Phe Val Tyr Leu Ala Asp
85 90 95

Leu Leu Met Pro His Leu Gly Ala Ala Glu Asp Pro Gln Thr Thr Leu
100 105 110

Ala Leu Asn Phe Gly Ser Thr Leu Met Lys Lys Lys Ser Asp Pro Glu
115 120 125

Gly Pro Ala Leu Leu Phe Pro Glu Ser Glu Leu Ser Ile Arg Ile Asp
130 135 140

Lys Ser Glu Asn Gly Glu Ala Tyr Gln Arg Lys Lys Ala Ala Ala Thr
145 150 155 160

Gly Leu Pro Glu Gly Pro Ala Val Pro Val Pro Ser Arg Gly Asn Leu
165 170 175

Ala Gln Pro Gly Gly Ser Ser Trp Arg Arg Ile Ala Leu Leu Ile Leu
180 185 190

Ala Ile Thr Ile His Asn Val Pro Glu Gly Leu Ala Val Gly Val Gly
195 200 205

Phe Gly Ala Ile Glu Lys Thr Ala Ser Ala Thr Phe Glu Ser Ala Arg
210 215 220

Asn Leu Ala Ile Gly Ile Gly Ile Gln Asn Phe Pro Glu Gly Leu Ala
225 230 235 240

Val Ser Leu Pro Leu Arg Gly Ala Gly Phe Ser Thr Trp Arg Ala Phe
245 250 255

Trp Tyr Gly Gln Leu Ser Gly Met Val Glu Pro Leu Ala Gly Val Phe
260 265 270

Gly Ala Phe Ala Val Val Leu Ala Glu Pro Ile Leu Pro Tyr Ala Leu
275 280 285

Ala Phe Ala Ala Gly Ala Met Val Tyr Val Val Met Asp Asp Ile Ile
290 295 300

Pro Glu Ala Gln Ile Ser Gly Asn Gly Lys Leu Ala Ser Trp Ala Ser
305 310 315 320

Ile Leu Gly Phe Val Val Met Met Ser Leu Asp Val Gly Leu Gly
325 330 335

<210> 148

<211> 342
<212> PRT
<213> Homo sapiens

<400> 148

Met Leu Gln Gly His Ser Ser Val Phe Gln Ala Leu Leu Gly Thr Phe
1 5 10 15

Phe Thr Trp Gly Met Thr Ala Ala Gly Ala Ala Leu Val Phe Val Phe
20 25 30

Ser Ser Gly Gln Arg Arg Ile Leu Asp Gly Ser Leu Gly Phe Ala Ala
35 40 45

Gly Val Met Leu Ala Ala Ser Tyr Trp Ser Leu Leu Ala Pro Ala Val
50 55 60

Glu Met Ala Thr Ser Ser Gly Gly Phe Gly Ala Phe Ala Phe Phe Pro
65 70 75 80

Val Ala Val Gly Phe Thr Leu Gly Ala Ala Phe Val Tyr Leu Ala Asp
85 90 95

Leu Leu Met Pro His Leu Gly Ala Ala Glu Asp Pro Gln Thr Thr Leu
100 105 110

Ala Leu Asn Phe Gly Ser Thr Leu Met Lys Lys Lys Ser Asp Pro Glu
115 120 125

Gly Pro Ala Leu Leu Phe Pro Glu Ser Glu Leu Ser Ile Arg Ile Gly
130 135 140

Arg Ala Gly Leu Leu Ser Asp Lys Ser Glu Asn Gly Glu Ala Tyr Gln
145 150 155 160

Arg Lys Lys Ala Ala Ala Thr Gly Leu Pro Glu Gly Pro Ala Val Pro
165 170 175

Val Pro Ser Arg Gly Asn Leu Ala Gln Pro Gly Gly Ser Ser Trp Arg
180 185 190

Arg Ile Ala Leu Leu Ile Leu Ala Ile Thr Ile His Asn Val Pro Glu
195 200 205

Gly Leu Ala Val Gly Val Gly Phe Gly Ala Ile Glu Lys Thr Ala Ser
210 215 220

Ala Thr Phe Glu Ser Ala Arg Asn Leu Ala Ile Gly Ile Gly Ile Gln

225 230 235 240

Asn Phe Pro Glu Gly Leu Ala Val Ser Leu Pro Leu Arg Gly Ala Gly
245 250 255

Phe Ser Thr Trp Arg Ala Phe Trp Tyr Gly Gln Leu Ser Gly Met Val
260 265 270

Glu Pro Leu Ala Gly Val Phe Gly Ala Phe Ala Val Val Leu Ala Glu
275 280 285

Pro Ile Leu Pro Tyr Ala Leu Ala Phe Ala Ala Gly Ala Met Val Tyr
290 295 300

Val Val Met Asp Asp Ile Ile Pro Glu Ala Gln Ile Ser Gly Asn Gly
305 310 315 320

Lys Leu Ala Ser Trp Ala Ser Ile Leu Gly Phe Val Val Met Met Ser
325 330 335

Leu Asp Val Gly Leu Gly
340

<210> 149
<211> 4510
<212> DNA
<213> Homo sapiens

<400> 149
gctgaggcca ggagggcgca ctggggattg gaggcgaggg aagtgcaggg cgcatcccag 60

gcggcagggc tcccagcatc ggcagtcgcc atcaccgcca gaccgcagag acaggttcgg 120

atccgcggtc ctcttgcctc tttccaggcc tcgatgagtg ttaaatcgcc atttaatgtg 180

atgtcaagaa ataatttgga agcacctcct tgtaagatga cagagccatt taattttgag 240

aaaaatgaaa acaagcttcc accacatgag tctttaagaa gtcctggaac acttcctaac 300

caccctaatt tcaggctgaa aagctcagag aatggaaata aaaagaacaa ttttttgctt 360

tgtgagcaaa ccaaacaata tttggctagt caggaagaca attcagtttc ttcaaacccg 420

aatggcatca acggagaagt agttggctcc aaaggagaca ggaaaaaatt gccagcagga 480

aactcagtgt caccaccaag tgctgaaagt aattcaccac ccaaagaagt gaatattaag 540

cctggaaata atgtacgtcc tgcaaaatca aaaaaactaa acaagttggt cgagaattcc 600

ttgtccataa gtaatccagg gctcttcacc tccttaggac ctcctcttcg gtccacaact 660

tgccatcgct gtggcctatt tggatcgctg aggtgctctc agtgcaagca gacctactat 720

tgctccacag catgtcaaag aagagactgg tctgcacaca gcatcgtgtg caggcctgtt 780

cagccaaatt tccacaaact tgaaaataaa tcatctattg aaacaaagga tgtggaggta 840

aacaataaga gtgactgtcc acttggagtt actaaggaaa tagccatttg ggctgagaga 900

ataatgtttt ctgatttgag aagtctacaa ctcaagaaaa ccatggaaat aaagggtacg 960

gttaccgaat tcaaacaccc aggggacttc tacgtgcagt tatattcttc agaagtttta 1020

gaatacatga accaactctc tgccagctta aaagaaacat atgcaaatgt gcatgaaaaa 1080

gactatattc ctgttaaggg ggaagtttgt attgccaagt acactgttga tcagacctgg 1140

aacagagcaa tcatacaaaa cgttgatgtg cagcaaaaga aggcacatgt cttatatatt 1200

gattatggaa atgaagaaat aattccatta aacagaattt accacctcaa caggaacatt 1260

gacttgtttc ctccttgtgc cataaagtgc tttgtagcca atgttatccc agcagaaggg 1320

aattggagca gtgattgtat caaagctact aaaccactgt taatggagca gtactgctcc 1380

ataaagattg tcgacatctt ggaagaggaa gtggttacct ttgctgtaga agttgagctg 1440

ccaaattcag gaaaactttt agaccatgtg cttatagaaa tgggatatgg cttgaaaccc 1500

agtggacaag attctaagaa ggaaaatgca gatcaaagtg atcctgaaga tgttggaaaa 1560

atgacaactg aaaacaacat tgtcgtagac aaaagtgacc taatcccaaa agtgttaact 1620

ttgaatgtag gtgatgagtt ttgtggtgtg gttgcccaca ttcaaacacc agaagacttc 1680

ttttgtcaac aactgcaaag tggccgaaag cttgctgaac ttcaggcatc ccttagcaag 1740

tactgtgatc agttgcctcc acgctctgat tttttatccag ccattggtga tatatgttgt 1800

gctcagttct cagaggatga tcagtggtac cgtgcctctg ttttggctta cgcttctgaa 1860

gaatctgtac tggtcggata tgtagattat ggaaactttg aaatccttag tttgatgaga 1920

ctttgtccca taatcccaaa gttgttggaa ttgccaatgc aagctataaa gtgtgtacta 1980

gcaggagtaa agccatcatt aggaatttgg actccagaag ctatttgtct catgaaaaaa 2040

cttgtacaga acaaaataat cacagtgaaa gtggtggaca agttggaaaa cagttccctg 2100

gtggagctta ttgataaatc cgagacgcct catgtcagtg ttagcaaagt tctcctagat 2160

gcaggctttg ctgtgggaga acagagtatg gtgacagata aacccagtga cgtgaaagaa 2220

accagtgttc ccttgggtgt ggaaggaaaa gtaaatccat tggagtggac atgggttgaa 2280

cttggtgttg accaaacagt agatgttgtg gtctgtgtga tatatagtcc tggagaattt 2340

tattgccatg tgcttaaaga ggatgcttta aagaaactca atgatttgaa caagtcatta 2400

gcagaacact gccagcagaa gttacctaat ggtttcaagg cagagatagg acaaccttgt 2460

tgtgcttttt ttgcaggtga tggtagttgg tatcgtgctt tagtcaagga aatcttacca 2520

aatggacatg ttaaagtaca ttttgtggat tatggaaaca tcgaagaagt tactgcagat 2580

gaactccgaa tgatatcatc aacattttta aaccttccct ttcagggaat acggtgccag 2640

ttagcagata tacagtctag aaacaaacat tggtctgaag aagccataac aagattccag 2700

atgtgtgttg ctgggataaa attgcaagcc agagtggttg aagtcactga aaatgggata 2760
ggagttgaac tcaccgatct ctccacttgt tatcccagaa taattagtga tgttctgatt 2820
gatgaacatc tggttttaaa atctgcttca ccacataaag acttaccaaa tgacagactt 2880
gttaataaac atgagcttca agttcatgta cagggacttc aagctacctc ttcagctgag 2940
caatggaaga cgatagaatt gccagtggat aaaactatac aagcaaatgt attagaaatc 3000
ataagcccaa acttgtttta tgctctacca aaagggatgc cagaaaatca ggaaaagctg 3060
tgcatgttga cagctgaatt attagaatac tgcaatgctc cgaaaagtcg accaccctat 3120
agaccaagaa ttggagacgc atgctgtgcc aaatacacaa gtgatgattt ttggtatcgt 3180
gcagttgttc tggggacatc agacactgat gtggaagtgc tctatgcaga ctatggaaac 3240
attgaaaccc tgcctctttg cagagtgcaa ccaatcacct ctagccacct ggcgcttcct 3300
ttccaaatta ttagatgttc acttgaagga ttaatggaat tgaatggaag ctcttctcaa 3360
ttaataataa tgctattaaa aaatttcatg ttgaatcaga atgtaatgct ttctgtgaaa 3420
ggaattacaa agaatgtcca tacagtgtca gttgagaaat gttctgagaa tgggactgtc 3480
gatgtagctg ataagctagt gacatttggt ctggcaaaaa acatcacacc tcaaaggcag 3540
agtgctttaa atacagaaaa gatgtatagg atgaattgct gctgcacaga gttacagaaa 3600
caagttgaaa aacatgaaca tattcttctc ttcctcttaa acaattcaac caatcaaaat 3660
aaatttattg aaatgaaaaa actgttaaaa aaaacagcat ctcttggagg taaaccctta 3720
tgagacagga aacagcaaag gctagcttta ggagagaaag tacagcacct ggtgttttta 3780
tttatgagaa ccttttcttt gtccactttc tctgtaatga ccttctatcc ctccgttttt 3840
gcctgcctgc cattctccta ttaggttggt ggtttttatt ttcctctaag ttccttccac 3900
caaataaata ttacgtaaaa aattcatacc aaatcaatga gaatactggc aaggaataca 3960
tagggacttt ctgctatata tgtaactttt tattacttaa aggtaccgaa ggaaggccag 4020
gtgcagtggc tcacgcccag cactttggga ggctgaggtg ggaggatccc ttgaggccag 4080
gagttcaagg ttacagtgag ctatgatagt gccactgcac tccagcctgg gtgacagatt 4140
ttgtcttaaa aaaaaaaaaa aaaaagttga tatgagtttt attttctgtc cgtttgaaat 4200
attttgtaat attccctgca ttctctgtcg tctgcctctt ccacataatg tcctttgctt 4260
tcatgtttgt tatcttcttt ttctgttcac tcagaggtca tcaatttctt tctctccgtc 4320
cttaattgga ttatttttct tttggccttt gggcacagag tctgacctct ggaccactct 4380
aactggagaa ggaactttat gttccctctc ctgctgtgtc cacaacctta gaaatctgta 4440
gctagatttt tgttgttata gatagaattt actgtttctg aaacccaaat acagttatca 4500
gtttaaggtt 4510

<210> 150
<211> 1189
<212> PRT
<213> Homo sapiens

<400> 150

Met Ser Val Lys Ser Pro Phe Asn Val Met Ser Arg Asn Asn Leu Glu
1 5 10 15

Ala Pro Pro Cys Lys Met Thr Glu Pro Phe Asn Phe Glu Lys Asn Glu
20 25 30

Asn Lys Leu Pro Pro His Glu Ser Leu Arg Ser Pro Gly Thr Leu Pro
35 40 45

Asn His Pro Asn Phe Arg Leu Lys Ser Ser Glu Asn Gly Asn Lys Lys
50 55 60

Asn Asn Phe Leu Leu Cys Glu Gln Thr Lys Gln Tyr Leu Ala Ser Gln
65 70 75 80

Glu Asp Asn Ser Val Ser Ser Asn Pro Asn Gly Ile Asn Gly Glu Val
85 90 95

Val Gly Ser Lys Gly Asp Arg Lys Lys Leu Pro Ala Gly Asn Ser Val
100 105 110

Ser Pro Pro Ser Ala Glu Ser Asn Ser Pro Pro Lys Glu Val Asn Ile
115 120 125

Lys Pro Gly Asn Asn Val Arg Pro Ala Lys Ser Lys Lys Leu Asn Lys
130 135 140

Leu Val Glu Asn Ser Leu Ser Ile Ser Asn Pro Gly Leu Phe Thr Ser
145 150 155 160

Leu Gly Pro Pro Leu Arg Ser Thr Thr Cys His Arg Cys Gly Leu Phe
165 170 175

Gly Ser Leu Arg Cys Ser Gln Cys Lys Gln Thr Tyr Tyr Cys Ser Thr
180 185 190

Ala Cys Gln Arg Arg Asp Trp Ser Ala His Ser Ile Val Cys Arg Pro
195 200 205

Val Gln Pro Asn Phe His Lys Leu Glu Asn Lys Ser Ser Ile Glu Thr
210 215 220

Lys Asp Val Glu Val Asn Asn Lys Ser Asp Cys Pro Leu Gly Val Thr
225 230 235 240

Lys Glu Ile Ala Ile Trp Ala Glu Arg Ile Met Phe Ser Asp Leu Arg
245 250 255

Ser Leu Gln Leu Lys Lys Thr Met Glu Ile Lys Gly Thr Val Thr Glu
260 265 270

Phe Lys His Pro Gly Asp Phe Tyr Val Gln Leu Tyr Ser Ser Glu Val
275 280 285

Leu Glu Tyr Met Asn Gln Leu Ser Ala Ser Leu Lys Glu Thr Tyr Ala
290 295 300

Asn Val His Glu Lys Asp Tyr Ile Pro Val Lys Gly Glu Val Cys Ile
305 310 315 320

Ala Lys Tyr Thr Val Asp Gln Thr Trp Asn Arg Ala Ile Ile Gln Asn
325 330 335

Val Asp Val Gln Gln Lys Lys Ala His Val Leu Tyr Ile Asp Tyr Gly
340 345 350

Asn Glu Glu Ile Ile Pro Leu Asn Arg Ile Tyr His Leu Asn Arg Asn
355 360 365

Ile Asp Leu Phe Pro Pro Cys Ala Ile Lys Cys Phe Val Ala Asn Val
370 375 380

Ile Pro Ala Glu Gly Asn Trp Ser Ser Asp Cys Ile Lys Ala Thr Lys
385 390 395 400

Pro Leu Leu Met Glu Gln Tyr Cys Ser Ile Lys Ile Val Asp Ile Leu
405 410 415

Glu Glu Glu Val Val Thr Phe Ala Val Glu Val Glu Leu Pro Asn Ser
420 425 430

Gly Lys Leu Leu Asp His Val Leu Ile Glu Met Gly Tyr Gly Leu Lys
435 440 445

Pro Ser Gly Gln Asp Ser Lys Lys Glu Asn Ala Asp Gln Ser Asp Pro
450 455 460

Glu Asp Val Gly Lys Met Thr Thr Glu Asn Asn Ile Val Val Asp Lys
465 470 475 480

Ser Asp Leu Ile Pro Lys Val Leu Thr Leu Asn Val Gly Asp Glu Phe
485 490 495

Cys Gly Val Val Ala His Ile Gln Thr Pro Glu Asp Phe Phe Cys Gln
500 505 510

Gln Leu Gln Ser Gly Arg Lys Leu Ala Glu Leu Gln Ala Ser Leu Ser
515 520 525

Lys Tyr Cys Asp Gln Leu Pro Pro Arg Ser Asp Phe Tyr Pro Ala Ile
530 535 540

Gly Asp Ile Cys Cys Ala Gln Phe Ser Glu Asp Asp Gln Trp Tyr Arg
545 550 555 560

Ala Ser Val Leu Ala Tyr Ala Ser Glu Glu Ser Val Leu Val Gly Tyr
565 570 575

Val Asp Tyr Gly Asn Phe Glu Ile Leu Ser Leu Met Arg Leu Cys Pro
580 585 590

Ile Ile Pro Lys Leu Leu Glu Leu Pro Met Gln Ala Ile Lys Cys Val
595 600 605

Leu Ala Gly Val Lys Pro Ser Leu Gly Ile Trp Thr Pro Glu Ala Ile
610 615 620

Cys Leu Met Lys Lys Leu Val Gln Asn Lys Ile Ile Thr Val Lys Val
625 630 635 640

Val Asp Lys Leu Glu Asn Ser Ser Leu Val Glu Leu Ile Asp Lys Ser
645 650 655

Glu Thr Pro His Val Ser Val Ser Lys Val Leu Leu Asp Ala Gly Phe
660 665 670

Ala Val Gly Glu Gln Ser Met Val Thr Asp Lys Pro Ser Asp Val Lys
675 680 685

Glu Thr Ser Val Pro Leu Gly Val Glu Gly Lys Val Asn Pro Leu Glu
690 695 700

Trp Thr Trp Val Glu Leu Gly Val Asp Gln Thr Val Asp Val Val Val
705 710 715 720

Cys Val Ile Tyr Ser Pro Gly Glu Phe Tyr Cys His Val Leu Lys Glu

725 730 735

Asp Ala Leu Lys Lys Leu Asn Asp Leu Asn Lys Ser Leu Ala Glu His
740 745 750

Cys Gln Gln Lys Leu Pro Asn Gly Phe Lys Ala Glu Ile Gly Gln Pro
755 760 765

Cys Cys Ala Phe Phe Ala Gly Asp Gly Ser Trp Tyr Arg Ala Leu Val
770 775 780

Lys Glu Ile Leu Pro Asn Gly His Val Lys Val His Phe Val Asp Tyr
785 790 795 800

Gly Asn Ile Glu Glu Val Thr Ala Asp Glu Leu Arg Met Ile Ser Ser
805 810 815

Thr Phe Leu Asn Leu Pro Phe Gln Gly Ile Arg Cys Gln Leu Ala Asp
820 825 830

Ile Gln Ser Arg Asn Lys His Trp Ser Glu Glu Ala Ile Thr Arg Phe
835 840 845

Gln Met Cys Val Ala Gly Ile Lys Leu Gln Ala Arg Val Val Glu Val
850 855 860

Thr Glu Asn Gly Ile Gly Val Glu Leu Thr Asp Leu Ser Thr Cys Tyr
865 870 875 880

Pro Arg Ile Ile Ser Asp Val Leu Ile Asp Glu His Leu Val Leu Lys
885 890 895

Ser Ala Ser Pro His Lys Asp Leu Pro Asn Asp Arg Leu Val Asn Lys
900 905 910

His Glu Leu Gln Val His Val Gln Gly Leu Gln Ala Thr Ser Ser Ala
915 920 925

Glu Gln Trp Lys Thr Ile Glu Leu Pro Val Asp Lys Thr Ile Gln Ala
930 935 940

Asn Val Leu Glu Ile Ile Ser Pro Asn Leu Phe Tyr Ala Leu Pro Lys
945 950 955 960

Gly Met Pro Glu Asn Gln Glu Lys Leu Cys Met Leu Thr Ala Glu Leu
965 970 975

Leu Glu Tyr Cys Asn Ala Pro Lys Ser Arg Pro Pro Tyr Arg Pro Arg
980 985 990

Ile Gly Asp Ala Cys Cys Ala Lys Tyr Thr Ser Asp Asp Phe Trp Tyr
995 1000 1005

Arg Ala Val Val Leu Gly Thr Ser Asp Thr Asp Val Glu Val Leu
1010 1015 1020

Tyr Ala Asp Tyr Gly Asn Ile Glu Thr Leu Pro Leu Cys Arg Val
1025 1030 1035

Gln Pro Ile Thr Ser Ser His Leu Ala Leu Pro Phe Gln Ile Ile
1040 1045 1050

Arg Cys Ser Leu Glu Gly Leu Met Glu Leu Asn Gly Ser Ser Ser
1055 1060 1065

Gln Leu Ile Ile Met Leu Leu Lys Asn Phe Met Leu Asn Gln Asn
1070 1075 1080

Val Met Leu Ser Val Lys Gly Ile Thr Lys Asn Val His Thr Val
1085 1090 1095

Ser Val Glu Lys Cys Ser Glu Asn Gly Thr Val Asp Val Ala Asp
1100 1105 1110

Lys Leu Val Thr Phe Gly Leu Ala Lys Asn Ile Thr Pro Gln Arg
1115 1120 1125

Gln Ser Ala Leu Asn Thr Glu Lys Met Tyr Arg Met Asn Cys Cys
1130 1135 1140

Cys Thr Glu Leu Gln Lys Gln Val Glu Lys His Glu His Ile Leu
1145 1150 1155

Leu Phe Leu Leu Asn Asn Ser Thr Asn Gln Asn Lys Phe Ile Glu
1160 1165 1170

Met Lys Lys Leu Leu Lys Lys Thr Ala Ser Leu Gly Gly Lys Pro
1175 1180 1185

Leu

<210> 151
<211> 4216
<212> DNA

<213> Homo sapiens

<400> 151
agtttctctt ctcggccacc tcctgcatag agggtaccat tctgcgctgc tgcaagttac 60

ggaatgaaaa attagaacaa cagaaacatg gaaaacatgt tccttcagtc gtcaatgctg 120

acctgcattt tcctgctaat atctggttcc tgtgagttat gcgccgaaga aaatttttct 180

agaagctatc cttgtgatga gaaaaagcaa aatgactcag ttattgcaga gtgcagcaat 240

cgtcgactac aggaagttcc ccaaacggtg ggcaaatatg tgacagaact agacctgtct 300

gataatttca tcacacacat aacgaatgaa tcatttcaag ggctgcaaaa tctcactaaa 360

ataaatctaa accacaaccc caatgtacag caccagaacg gaaatcccgg tatacaatca 420

aatggcttga atatcacaga cggggcattc ctcaacctaa aaaacctaag ggagttactg 480

cttgaagaca accagttacc ccaaataccc tctggtttgc cagagtcttt gacagaactt 540

agtctaattc aaaacaatat atacaacata actaaagagg gcatttcaag acttataaac 600

ttgaaaaatc tctatttggc ctggaactgc tattttaaca aagtttgcga gaaaactaac 660

atagaagatg gagtatttga aacgctgaca aatttggagt tgctatcact atctttcaat 720

tctctttcac acgtgccacc caaactgcca agctccctac gcaaactttt tctgagcaac 780

acccagatca aatacattag tgaagaagat ttcaagggat tgataaattt aacattacta 840

gatttaagcg ggaactgtcc gaggtgcttc aatgccccat ttccatgcgt gccttgtgat 900

ggtggtgctt caattaatat agatcgtttt gcttttcaaa acttgaccca acttcgatac 960

ctaaacctct ctagcacttc cctcaggaag attaatgctg cctggtttaa aaatatgcct 1020

catctgaagg tgctggatct tgaattcaac tatttagtgg gagaaatagc ctctggggca 1080

tttttaacga tgctgccccg cttagaaata cttgacttgt cttttaacta tataaagggg 1140

agttatccac agcatattaa tatttccaga aacttctcta aacttttgtc tctacgggca 1200

ttgcatttaa gaggttatgt gttccaggaa ctcagagaag atgatttcca gcccctgatg 1260

cagcttccaa acttatcgac tatcaacttg ggtattaatt ttattaagca aatcgatttc 1320

aaacttttcc aaaatttctc caatctggaa attatttact tgtcagaaaa cagaatatca 1380

ccgttggtaa aagatacccg gcagagttat gcaaatagtt cctcttttca acgtcatatc 1440

cggaaacgac gctcaacaga ttttgagttt gacccacatt cgaactttta tcatttcacc 1500

cgtcctttaa taaagccaca atgtgctgct tatggaaaag ccttagattt aagcctcaac 1560

agtattttct tcattgggcc aaaccaattt gaaaatcttc ctgacattgc ctgtttaaat 1620

ctgtctgcaa atagcaatgc tcaagtgtta agtggaactg aattttcagc cattcctcat 1680

gtcaaatatt tggatttgac aaacaataga ctagactttg ataatgctag tgctcttact 1740

gaattgtccg acttggaagt tctagatctc agctataatt cacactattt cagaatagca 1800

ggcgtaacac atcatctaga atttattcaa aatttcacaa atctaaaagt tttaaacttg 1860
agccacaaca acatttatac tttaacagat aagtataacc tggaaagcaa gtccctggta 1920
gaattagttt tcagtggcaa tcgccttgac attttgtgga atgatgatga caacaggtat 1980
atctccattt tcaaaggtct caagaatctg acacgtctgg atttatccct taataggctg 2040
aagcacatcc caaatgaagc attccttaat ttgccagcga gtctcactga actacatata 2100
aatgataata tgttaaagtt ttttaactgg acattactcc agcagtttcc tcgtctcgag 2160
ttgcttgact tacgtggaaa caaactactc tttttaactg atagcctatc tgactttaca 2220
tcttcccttc ggacactgct gctgagtcat aacaggattt cccacctacc ctctggcttt 2280
ctttctgaag tcagtagtct gaagcacctc gatttaagtt ccaatctgct aaaaacaatc 2340
aacaaatccg cacttgaaac taagaccacc accaaattat ctatgttgga actacacgga 2400
aacccctttg aatgcacctg tgacattgga gatttccgaa gatggatgga tgaacatctg 2460
aatgtcaaaa ttcccagact ggtagatgtc atttgtgcca gtcctgggga tcaaagaggg 2520
aagagtattg tgagtctgga gctaacaact tgtgtttcag atgtcactgc agtgatatta 2580
tttttcttca cgttctttat caccaccatg gttatgttgg ctgccctggc tcaccatttg 2640
ttttactggg atgtttggtt tatatataat gtgtgtttag ctaaggtaaa aggctacagg 2700
tctctttcca catcccaaac tttctatgat gcttacattt cttatgacac caaagatgcc 2760
tctgttactg actgggtgat aaatgagctg cgctaccacc ttgaagagag ccgagacaaa 2820
aacgttctcc tttgtctaga ggagagggat tgggacccgg gattggccat catcgacaac 2880
ctcatgcaga gcatcaacca aagcaagaaa acagtatttg ttttaaccaa aaaatatgca 2940
aaaagctgga actttaaaac agctttttac ttggctttgc agaggctaat ggatgagaac 3000
atggatgtga ttatatttat cctgctggag ccagtgttac agcattctca gtatttgagg 3060
ctacggcagc ggatctgtaa gagctccatc ctccagtggc ctgacaaccc gaaggcagaa 3120
ggcttgtttt ggcaaactct gagaaatgtg gtcttgactg aaaatgattc acggtataac 3180
aatatgtatg tcgattccat taagcaatac taactgacgt taagtcatga tttcgcgcca 3240
taataaagat gcaaaggaat gacatttctg tattagttat ctattgctat gtaacaaatt 3300
atcccaaaac ttagtggttt aaaacaacac atttgctggc ccacagtttt tgagggtcag 3360
gagtccaggc ccagcataac tgggtcctct gctcagggtg tctcagaggc tgcaatgtag 3420
gtgttcacca gagacatagg catcactggg gtcacactca tgtggttgtt ttctggattc 3480
aattcctcct gggctattgg ccaaaggcta tactcatgta agccatgcga gcctctccca 3540
caaggcagct tgcttcatca gagctagcaa aaaagagagg ttgctagcaa gatgaagtca 3600
caatcttttg taatcgaatc aaaaaagtga tatctcatca ctttggccat attctatttg 3660
ttagaagtaa accacaggtc ccaccagctc catgggagtg accacctcag tccagggaaa 3720

acagctgaag accaagatgg tgagctctga ttgcttcagt tggtcatcaa ctattttccc 3780

ttgactgctg tcctgggatg gcctgctatc ttgatgatag attgtgaata tcaggaggca 3840

gggatcactg tggaccatct tagcagttga cctaacacat cttcttttca atatctaaga 3900

acttttgcca ctgtgactaa tggtcctaat attaagctgt tgtttatatt tatcatatat 3960

ctatggctac atggttatat tatgctgtgg ttgcgttcgg ttttatttac agttgctttt 4020

acaaatattt gctgtaacat ttgacttcta aggtttagat gccatttaag aactgagatg 4080

gatagctttt aaagcatctt ttacttctta ccattttta aaagtatgca gctaaattcg 4140

aagcttttgg tctatattgt taattgccat tgctgtaaat cttaaaatga atgaataaaa 4200

atgtttcatt ttacaa 4216

<210> 152
<211> 4353
<212> DNA
<213> Homo sapiens

<400> 152
agtttctctt ctcggccacc tcctgcatag agggtaccat tctgcgctgc tgcaagttac 60

ggaatgaaaa attagaacaa cagaaacatg gttctcttga cacttcagtg ttagggaaca 120

tcagcaagac ccatcccagg agaccttgaa ggaagccttt gaaagggaga atgaaggagt 180

catctttgca aaatagctcc tgcagcctgg gaaaggagac taaaaaggaa aacatgttcc 240

ttcagtcgtc aatgctgacc tgcattttcc tgctaatatc tggttcctgt gagttatgcg 300

ccgaagaaaa tttttctaga agctatcctt gtgatgagaa aaagcaaaat gactcagtta 360

ttgcagagtg cagcaatcgt cgactacagg aagttcccca aacggtgggc aaatatgtga 420

cagaactaga cctgtctgat aatttcatca cacacataac gaatgaatca tttcaagggc 480

tgcaaaatct cactaaaata aatctaaacc acaaccccaa tgtacagcac cagaacggaa 540

atcccggtat acaatcaaat ggcttgaata tcacagacgg ggcattcctc aacctaaaaa 600

acctaaggga gttactgctt gaagacaacc agttacccca aataccctct ggtttgccag 660

agtctttgac agaacttagt ctaattcaaa acaatatata caacataact aaagagggca 720

tttcaagact tataaacttg aaaaatctct atttggcctg gaactgctat tttaacaaag 780

tttgcgagaa aactaacata gaagatggag tatttgaaac gctgacaaat ttggagttgc 840

tatcactatc tttcaattct ctttcacacg tgccacccaa actgccaagc tccctacgca 900

aacttttctt gagcaacacc cagatcaaat acattagtga agaagatttc aagggattga 960

taaatttaac attactagat ttaagcggga actgtccgag gtgcttcaat gccccatttc 1020

catgcgtgcc ttgtgatggt ggtgcttcaa ttaatataga tcgttttgct tttcaaaact 1080

tgacccaact tcgataccta aacctctcta gcacttccct caggaagatt aatgctgcct 1140

ggtttaaaaa tatgcctcat ctgaaggtgc tggatcttga attcaactat ttagtgggag 1200

aaatagcctc tggggcattt ttaacgatgc tgccccgctt agaaatactt gacttgtctt 1260

ttaactatat aaaggggagt tatccacagc atattaatat ttccagaaac ttctctaaac 1320

ttttgtctct acgggcattg catttaagag gttatgtgtt ccaggaactc agagaagatg 1380

atttccagcc cctgatgcag cttccaaact tatcgactat caacttgggt attaatttta 1440

ttaagcaaat cgatttcaaa cttttccaaa atttctccaa tctggaaatt atttacttgt 1500

cagaaaacag aatatcaccg ttggtaaaag atacccggca gagttatgca aatagttcct 1560

cttttcaacg tcatatccgg aaacgacgct caacagattt tgagtttgac ccacattcga 1620

acttttatca tttcacccgt cctttaataa agccacaatg tgctgcttat ggaaaagcct 1680

tagatttaag cctcaacagt attttcttca ttgggccaaa ccaatttgaa aatcttcctg 1740

acattgcctg tttaaatctg tctgcaaata gcaatgctca agtgttaagt ggaactgaat 1800

tttcagccat tcctcatgtc aaatatttgg atttgacaaa caatagacta gactttgata 1860

atgctagtgc tcttactgaa ttgtccgact tggaagttct agatctcagc tataattcac 1920

actatttcag aatagcaggc gtaacacatc atctagaatt tattcaaaat ttcacaaatc 1980

taaaagtttt aaacttgagc cacaacaaca tttatacttt aacagataag tataacctgg 2040

aaagcaagtc cctggtagaa ttagttttca gtggcaatcg ccttgacatt ttgtggaatg 2100

atgatgacaa caggtatatc tccattttca aaggtctcaa gaatctgaca cgtctggatt 2160

tatcccttaa taggctgaag cacatcccaa atgaagcatt ccttaatttg ccagcgagtc 2220

tcactgaact acatataaat gataatatgt taaagttttt taactggaca ttactccagc 2280

agtttcctcg tctcgagttg cttgacttac gtggaaacaa actactcttt ttaactgata 2340

gcctatctga ctttacatct tcccttcgga cactgctgct gagtcataac aggatttccc 2400

acctaccctc tggctttctt tctgaagtca gtagtctgaa gcacctcgat ttaagttcca 2460

atctgctaaa aacaatcaac aaatccgcac ttgaaactaa gaccaccacc aaattatcta 2520

tgttggaact acacggaaac ccctttgaat gcacctgtga cattggagat ttccgaagat 2580

ggatggatga acatctgaat gtcaaaattc ccagactggt agatgtcatt tgtgccagtc 2640

ctggggatca aagagggaag agtattgtga gtctggagct aacaacttgt gtttcagatg 2700

tcactgcagt gatattattt ttcttcacgt tctttatcac caccatggtt atgttggctg 2760

ccctggctca ccatttgttt tactgggatg tttggtttat atataatgtg tgtttagcta 2820

aggtaaaagg ctacaggtct ctttccacat cccaaacttt ctatgatgct tacatttctt 2880

atgacaccaa agatgcctct gttactgact gggtgataaa tgagctgcgc taccaccttg 2940

aagagagccg agacaaaaac gttctccttt gtctagagga gagggattgg gacccgggat 3000

tggccatcat cgacaacctc atgcagagca tcaaccaaag caagaaaaca gtatttgttt 3060

taaccaaaaa atatgcaaaa agctggaact ttaaaacagc tttttacttg gctttgcaga 3120

ggctaatgga tgagaacatg gatgtgatta tatttatcct gctggagcca gtgttacagc 3180

attctcagta tttgaggcta cggcagcgga tctgtaagag ctccatcctc cagtggcctg 3240

acaacccgaa ggcagaaggc ttgttttggc aaactctgag aaatgtggtc ttgactgaaa 3300

atgattcacg gtataacaat atgtatgtcg attccattaa gcaatactaa ctgacgttaa 3360

gtcatgattt cgcgccataa taaagatgca aaggaatgac atttctgtat tagttatcta 3420

ttgctatgta acaaattatc ccaaaactta gtggtttaaa acaacacatt tgctggccca 3480

cagtttttga gggtcaggag tccaggccca gcataactgg gtcctctgct cagggtgtct 3540

cagaggctgc aatgtaggtg ttcaccagag acataggcat cactggggtc acactcatgt 3600

ggttgttttc tggattcaat tcctcctggg ctattggcca aaggctatac tcatgtaagc 3660

catgcgagcc tctcccacaa ggcagcttgc ttcatcagag ctagcaaaaa agagaggttg 3720

ctagcaagat gaagtcacaa tcttttgtaa tcgaatcaaa aaagtgatat ctcatcactt 3780

tggccatatt ctatttgtta gaagtaaacc acaggtccca ccagctccat gggagtgacc 3840

acctcagtcc agggaaaaca gctgaagacc aagatggtga gctctgattg cttcagttgg 3900

tcatcaacta ttttcccttg actgctgtcc tgggatggcc tgctatcttg atgatagatt 3960

gtgaatatca ggaggcaggg atcactgtgg accatcttag cagttgacct aacacatctt 4020

cttttcaata tctaagaact tttgccactg tgactaatgg tcctaatatt aagctgttgt 4080

ttatatttat catatatcta tggctacatg gttatattat gctgtggttg cgttcggttt 4140

tatttacagt tgcttttaca aatatttgct gtaacatttg acttctaagg tttagatgcc 4200

atttaagaac tgagatggat agcttttaaa gcatctttta cttcttacca tttttaaaa 4260

gtatgcagct aaattcgaag cttttggtct atattgttaa ttgccattgc tgtaaatctt 4320

aaaatgaatg aataaaaatg tttcatttta caa 4353

<210> 153
<211> 1041
<212> PRT
<213> Homo sapiens

<400> 153

Met Glu Asn Met Phe Leu Gln Ser Ser Met Leu Thr Cys Ile Phe Leu
1 5 10 15

Leu Ile Ser Gly Ser Cys Glu Leu Cys Ala Glu Glu Asn Phe Ser Arg
20 25 30

Ser Tyr Pro Cys Asp Glu Lys Lys Gln Asn Asp Ser Val Ile Ala Glu

35 40 45

Cys Ser Asn Arg Arg Leu Gln Glu Val Pro Gln Thr Val Gly Lys Tyr
50 55 60

Val Thr Glu Leu Asp Leu Ser Asp Asn Phe Ile Thr His Ile Thr Asn
65 70 75 80

Glu Ser Phe Gln Gly Leu Gln Asn Leu Thr Lys Ile Asn Leu Asn His
85 90 95

Asn Pro Asn Val Gln His Gln Asn Gly Asn Pro Gly Ile Gln Ser Asn
100 105 110

Gly Leu Asn Ile Thr Asp Gly Ala Phe Leu Asn Leu Lys Asn Leu Arg
115 120 125

Glu Leu Leu Leu Glu Asp Asn Gln Leu Pro Gln Ile Pro Ser Gly Leu
130 135 140

Pro Glu Ser Leu Thr Glu Leu Ser Leu Ile Gln Asn Asn Ile Tyr Asn
145 150 155 160

Ile Thr Lys Glu Gly Ile Ser Arg Leu Ile Asn Leu Lys Asn Leu Tyr
165 170 175

Leu Ala Trp Asn Cys Tyr Phe Asn Lys Val Cys Glu Lys Thr Asn Ile
180 185 190

Glu Asp Gly Val Phe Glu Thr Leu Thr Asn Leu Glu Leu Leu Ser Leu
195 200 205

Ser Phe Asn Ser Leu Ser His Val Pro Pro Lys Leu Pro Ser Ser Leu
210 215 220

Arg Lys Leu Phe Leu Ser Asn Thr Gln Ile Lys Tyr Ile Ser Glu Glu
225 230 235 240

Asp Phe Lys Gly Leu Ile Asn Leu Thr Leu Leu Asp Leu Ser Gly Asn
245 250 255

Cys Pro Arg Cys Phe Asn Ala Pro Phe Pro Cys Val Pro Cys Asp Gly
260 265 270

Gly Ala Ser Ile Asn Ile Asp Arg Phe Ala Phe Gln Asn Leu Thr Gln
275 280 285

Leu Arg Tyr Leu Asn Leu Ser Ser Thr Ser Leu Arg Lys Ile Asn Ala
    290                 295                 300

Ala Trp Phe Lys Asn Met Pro His Leu Lys Val Leu Asp Leu Glu Phe
305                 310                 315                 320

Asn Tyr Leu Val Gly Glu Ile Ala Ser Gly Ala Phe Leu Thr Met Leu
                325                 330                 335

Pro Arg Leu Glu Ile Leu Asp Leu Ser Phe Asn Tyr Ile Lys Gly Ser
            340                 345                 350

Tyr Pro Gln His Ile Asn Ile Ser Arg Asn Phe Ser Lys Leu Leu Ser
        355                 360                 365

Leu Arg Ala Leu His Leu Arg Gly Tyr Val Phe Gln Glu Leu Arg Glu
    370                 375                 380

Asp Asp Phe Gln Pro Leu Met Gln Leu Pro Asn Leu Ser Thr Ile Asn
385                 390                 395                 400

Leu Gly Ile Asn Phe Ile Lys Gln Ile Asp Phe Lys Leu Phe Gln Asn
                405                 410                 415

Phe Ser Asn Leu Glu Ile Ile Tyr Leu Ser Glu Asn Arg Ile Ser Pro
            420                 425                 430

Leu Val Lys Asp Thr Arg Gln Ser Tyr Ala Asn Ser Ser Ser Phe Gln
        435                 440                 445

Arg His Ile Arg Lys Arg Arg Ser Thr Asp Phe Glu Phe Asp Pro His
    450                 455                 460

Ser Asn Phe Tyr His Phe Thr Arg Pro Leu Ile Lys Pro Gln Cys Ala
465                 470                 475                 480

Ala Tyr Gly Lys Ala Leu Asp Leu Ser Leu Asn Ser Ile Phe Phe Ile
                485                 490                 495

Gly Pro Asn Gln Phe Glu Asn Leu Pro Asp Ile Ala Cys Leu Asn Leu
            500                 505                 510

Ser Ala Asn Ser Asn Ala Gln Val Leu Ser Gly Thr Glu Phe Ser Ala
        515                 520                 525

Ile Pro His Val Lys Tyr Leu Asp Leu Thr Asn Asn Arg Leu Asp Phe
    530                 535                 540

Asp Asn Ala Ser Ala Leu Thr Glu Leu Ser Asp Leu Glu Val Leu Asp
545 550 555 560

Leu Ser Tyr Asn Ser His Tyr Phe Arg Ile Ala Gly Val Thr His His
565 570 575

Leu Glu Phe Ile Gln Asn Phe Thr Asn Leu Lys Val Leu Asn Leu Ser
580 585 590

His Asn Asn Ile Tyr Thr Leu Thr Asp Lys Tyr Asn Leu Glu Ser Lys
595 600 605

Ser Leu Val Glu Leu Val Phe Ser Gly Asn Arg Leu Asp Ile Leu Trp
610 615 620

Asn Asp Asp Asp Asn Arg Tyr Ile Ser Ile Phe Lys Gly Leu Lys Asn
625 630 635 640

Leu Thr Arg Leu Asp Leu Ser Leu Asn Arg Leu Lys His Ile Pro Asn
645 650 655

Glu Ala Phe Leu Asn Leu Pro Ala Ser Leu Thr Glu Leu His Ile Asn
660 665 670

Asp Asn Met Leu Lys Phe Phe Asn Trp Thr Leu Leu Gln Gln Phe Pro
675 680 685

Arg Leu Glu Leu Leu Asp Leu Arg Gly Asn Lys Leu Leu Phe Leu Thr
690 695 700

Asp Ser Leu Ser Asp Phe Thr Ser Ser Leu Arg Thr Leu Leu Leu Ser
705 710 715 720

His Asn Arg Ile Ser His Leu Pro Ser Gly Phe Leu Ser Glu Val Ser
725 730 735

Ser Leu Lys His Leu Asp Leu Ser Ser Asn Leu Leu Lys Thr Ile Asn
740 745 750

Lys Ser Ala Leu Glu Thr Lys Thr Thr Thr Lys Leu Ser Met Leu Glu
755 760 765

Leu His Gly Asn Pro Phe Glu Cys Thr Cys Asp Ile Gly Asp Phe Arg
770 775 780

Arg Trp Met Asp Glu His Leu Asn Val Lys Ile Pro Arg Leu Val Asp
785 790 795 800

Val Ile Cys Ala Ser Pro Gly Asp Gln Arg Gly Lys Ser Ile Val Ser
805 810 815

Leu Glu Leu Thr Thr Cys Val Ser Asp Val Thr Ala Val Ile Leu Phe
820 825 830

Phe Phe Thr Phe Phe Ile Thr Thr Met Val Met Leu Ala Ala Leu Ala
835 840 845

His His Leu Phe Tyr Trp Asp Val Trp Phe Ile Tyr Asn Val Cys Leu
850 855 860

Ala Lys Val Lys Gly Tyr Arg Ser Leu Ser Thr Ser Gln Thr Phe Tyr
865 870 875 880

Asp Ala Tyr Ile Ser Tyr Asp Thr Lys Asp Ala Ser Val Thr Asp Trp
885 890 895

Val Ile Asn Glu Leu Arg Tyr His Leu Glu Glu Ser Arg Asp Lys Asn
900 905 910

Val Leu Leu Cys Leu Glu Glu Arg Asp Trp Asp Pro Gly Leu Ala Ile
915 920 925

Ile Asp Asn Leu Met Gln Ser Ile Asn Gln Ser Lys Lys Thr Val Phe
930 935 940

Val Leu Thr Lys Lys Tyr Ala Lys Ser Trp Asn Phe Lys Thr Ala Phe
945 950 955 960

Tyr Leu Ala Leu Gln Arg Leu Met Asp Glu Asn Met Asp Val Ile Ile
965 970 975

Phe Ile Leu Leu Glu Pro Val Leu Gln His Ser Gln Tyr Leu Arg Leu
980 985 990

Arg Gln Arg Ile Cys Lys Ser Ser Ile Leu Gln Trp Pro Asp Asn Pro
995 1000 1005

Lys Ala Glu Gly Leu Phe Trp Gln Thr Leu Arg Asn Val Val Leu
1010 1015 1020

Thr Glu Asn Asp Ser Arg Tyr Asn Asn Met Tyr Val Asp Ser Ile
1025 1030 1035

Lys Gln Tyr

1040

<210> 154
<211> 1059
<212> PRT
<213> Homo sapiens

<400> 154

Met Lys Glu Ser Ser Leu Gln Asn Ser Ser Cys Ser Leu Gly Lys Glu
1 5 10 15

Thr Lys Lys Glu Asn Met Phe Leu Gln Ser Ser Met Leu Thr Cys Ile
20 25 30

Phe Leu Leu Ile Ser Gly Ser Cys Glu Leu Cys Ala Glu Glu Asn Phe
35 40 45

Ser Arg Ser Tyr Pro Cys Asp Glu Lys Lys Gln Asn Asp Ser Val Ile
50 55 60

Ala Glu Cys Ser Asn Arg Arg Leu Gln Glu Val Pro Gln Thr Val Gly
65 70 75 80

Lys Tyr Val Thr Glu Leu Asp Leu Ser Asp Asn Phe Ile Thr His Ile
85 90 95

Thr Asn Glu Ser Phe Gln Gly Leu Gln Asn Leu Thr Lys Ile Asn Leu
100 105 110

Asn His Asn Pro Asn Val Gln His Gln Asn Gly Asn Pro Gly Ile Gln
115 120 125

Ser Asn Gly Leu Asn Ile Thr Asp Gly Ala Phe Leu Asn Leu Lys Asn
130 135 140

Leu Arg Glu Leu Leu Leu Glu Asp Asn Gln Leu Pro Gln Ile Pro Ser
145 150 155 160

Gly Leu Pro Glu Ser Leu Thr Glu Leu Ser Leu Ile Gln Asn Asn Ile
165 170 175

Tyr Asn Ile Thr Lys Glu Gly Ile Ser Arg Leu Ile Asn Leu Lys Asn
180 185 190

Leu Tyr Leu Ala Trp Asn Cys Tyr Phe Asn Lys Val Cys Glu Lys Thr
195 200 205

Asn Ile Glu Asp Gly Val Phe Glu Thr Leu Thr Asn Leu Glu Leu Leu

210 215 220

Ser Leu Ser Phe Asn Ser Leu Ser His Val Pro Pro Lys Leu Pro Ser
225 230 235 240

Ser Leu Arg Lys Leu Phe Leu Ser Asn Thr Gln Ile Lys Tyr Ile Ser
245 250 255

Glu Glu Asp Phe Lys Gly Leu Ile Asn Leu Thr Leu Leu Asp Leu Ser
260 265 270

Gly Asn Cys Pro Arg Cys Phe Asn Ala Pro Phe Pro Cys Val Pro Cys
275 280 285

Asp Gly Gly Ala Ser Ile Asn Ile Asp Arg Phe Ala Phe Gln Asn Leu
290 295 300

Thr Gln Leu Arg Tyr Leu Asn Leu Ser Ser Thr Ser Leu Arg Lys Ile
305 310 315 320

Asn Ala Ala Trp Phe Lys Asn Met Pro His Leu Lys Val Leu Asp Leu
325 330 335

Glu Phe Asn Tyr Leu Val Gly Glu Ile Ala Ser Gly Ala Phe Leu Thr
340 345 350

Met Leu Pro Arg Leu Glu Ile Leu Asp Leu Ser Phe Asn Tyr Ile Lys
355 360 365

Gly Ser Tyr Pro Gln His Ile Asn Ile Ser Arg Asn Phe Ser Lys Leu
370 375 380

Leu Ser Leu Arg Ala Leu His Leu Arg Gly Tyr Val Phe Gln Glu Leu
385 390 395 400

Arg Glu Asp Asp Phe Gln Pro Leu Met Gln Leu Pro Asn Leu Ser Thr
405 410 415

Ile Asn Leu Gly Ile Asn Phe Ile Lys Gln Ile Asp Phe Lys Leu Phe
420 425 430

Gln Asn Phe Ser Asn Leu Glu Ile Ile Tyr Leu Ser Glu Asn Arg Ile
435 440 445

Ser Pro Leu Val Lys Asp Thr Arg Gln Ser Tyr Ala Asn Ser Ser Ser
450 455 460

Phe Gln Arg His Ile Arg Lys Arg Arg Ser Thr Asp Phe Glu Phe Asp
465 470 475 480

Pro His Ser Asn Phe Tyr His Phe Thr Arg Pro Leu Ile Lys Pro Gln
485 490 495

Cys Ala Ala Tyr Gly Lys Ala Leu Asp Leu Ser Leu Asn Ser Ile Phe
500 505 510

Phe Ile Gly Pro Asn Gln Phe Glu Asn Leu Pro Asp Ile Ala Cys Leu
515 520 525

Asn Leu Ser Ala Asn Ser Asn Ala Gln Val Leu Ser Gly Thr Glu Phe
530 535 540

Ser Ala Ile Pro His Val Lys Tyr Leu Asp Leu Thr Asn Asn Arg Leu
545 550 555 560

Asp Phe Asp Asn Ala Ser Ala Leu Thr Glu Leu Ser Asp Leu Glu Val
565 570 575

Leu Asp Leu Ser Tyr Asn Ser His Tyr Phe Arg Ile Ala Gly Val Thr
580 585 590

His His Leu Glu Phe Ile Gln Asn Phe Thr Asn Leu Lys Val Leu Asn
595 600 605

Leu Ser His Asn Asn Ile Tyr Thr Leu Thr Asp Lys Tyr Asn Leu Glu
610 615 620

Ser Lys Ser Leu Val Glu Leu Val Phe Ser Gly Asn Arg Leu Asp Ile
625 630 635 640

Leu Trp Asn Asp Asp Asp Asn Arg Tyr Ile Ser Ile Phe Lys Gly Leu
645 650 655

Lys Asn Leu Thr Arg Leu Asp Leu Ser Leu Asn Arg Leu Lys His Ile
660 665 670

Pro Asn Glu Ala Phe Leu Asn Leu Pro Ala Ser Leu Thr Glu Leu His
675 680 685

Ile Asn Asp Asn Met Leu Lys Phe Phe Asn Trp Thr Leu Leu Gln Gln
690 695 700

Phe Pro Arg Leu Glu Leu Leu Asp Leu Arg Gly Asn Lys Leu Leu Phe
705 710 715 720

```
Leu Thr Asp Ser Leu Ser Asp Phe Thr Ser Ser Leu Arg Thr Leu Leu
                725                 730                 735

Leu Ser His Asn Arg Ile Ser His Leu Pro Ser Gly Phe Leu Ser Glu
            740                 745                 750

Val Ser Ser Leu Lys His Leu Asp Leu Ser Ser Asn Leu Leu Lys Thr
        755                 760                 765

Ile Asn Lys Ser Ala Leu Glu Thr Lys Thr Thr Thr Lys Leu Ser Met
    770                 775                 780

Leu Glu Leu His Gly Asn Pro Phe Glu Cys Thr Cys Asp Ile Gly Asp
785                 790                 795                 800

Phe Arg Arg Trp Met Asp Glu His Leu Asn Val Lys Ile Pro Arg Leu
                805                 810                 815

Val Asp Val Ile Cys Ala Ser Pro Gly Asp Gln Arg Gly Lys Ser Ile
            820                 825                 830

Val Ser Leu Glu Leu Thr Thr Cys Val Ser Asp Val Thr Ala Val Ile
        835                 840                 845

Leu Phe Phe Phe Thr Phe Phe Ile Thr Thr Met Val Met Leu Ala Ala
    850                 855                 860

Leu Ala His His Leu Phe Tyr Trp Asp Val Trp Phe Ile Tyr Asn Val
865                 870                 875                 880

Cys Leu Ala Lys Val Lys Gly Tyr Arg Ser Leu Ser Thr Ser Gln Thr
                885                 890                 895

Phe Tyr Asp Ala Tyr Ile Ser Tyr Asp Thr Lys Asp Ala Ser Val Thr
            900                 905                 910

Asp Trp Val Ile Asn Glu Leu Arg Tyr His Leu Glu Glu Ser Arg Asp
        915                 920                 925

Lys Asn Val Leu Leu Cys Leu Glu Glu Arg Asp Trp Asp Pro Gly Leu
    930                 935                 940

Ala Ile Ile Asp Asn Leu Met Gln Ser Ile Asn Gln Ser Lys Lys Thr
945                 950                 955                 960

Val Phe Val Leu Thr Lys Lys Tyr Ala Lys Ser Trp Asn Phe Lys Thr
                965                 970                 975
```

Ala Phe Tyr Leu Ala Leu Gln Arg Leu Met Asp Glu Asn Met Asp Val
980 985 990

Ile Ile Phe Ile Leu Leu Glu Pro Val Leu Gln His Ser Gln Tyr Leu
995 1000 1005

Arg Leu Arg Gln Arg Ile Cys Lys Ser Ser Ile Leu Gln Trp Pro
1010 1015 1020

Asp Asn Pro Lys Ala Glu Gly Leu Phe Trp Gln Thr Leu Arg Asn
1025 1030 1035

Val Val Leu Thr Glu Asn Asp Ser Arg Tyr Asn Asn Met Tyr Val
1040 1045 1050

Asp Ser Ile Lys Gln Tyr
1055

<210> 155
<211> 5799
<212> DNA
<213> Homo sapiens

<400> 155
agtgccggct gccgatgacc gattcacgct cccggtactg ggccccctc tgcccagcca 60

cccctaccca gagcacccgg gctgcgcggc cccgccgagg gtgcgggctt tgttcgcatt 120

gtctgcgcgc acctgagcgc ggccttcctg gcacggcggc gtcgggggaa gagcgcacct 180

ggcgcgcgcc tccctcgtgg ccactcgcgg tccgtcccgg gcgagctggc ggggttttgg 240

gaggggtgcg gtcagcagtt atatcaacat gcccccttc ctgttgctgg aagccgtctg 300

tgttttcctg ttttccagag tgccccatc tctccctctc caggaagtcc atgtaagcaa 360

agaaaccatc gggaagattt cagctgccag caaaatgatg tggtgctcgg ctgcagtgga 420

catcatgttt ctgttagatg ggtctaacag cgtcgggaaa gggagctttg aaaggtccaa 480

gcactttgcc atcacagtct gtgacggtct ggacatcagc cccgagaggg tcagagtggg 540

agcattccag ttcagttcca ctcctcatct ggaattcccc ttggattcat tttcaaccca 600

acaggaagtg aaggcaagaa tcaagaggat ggttttcaaa ggagggcgca cggagacgga 660

acttgctctg aaataccttc tgcacagagg gttgcctgga ggcagaaatg cttctgtgcc 720

ccagatcctc atcatcgtca ctgatgggaa gtcccagggg gatgtggcac tgccatccaa 780

gcagctgaag gaaaggggtg tcactgtgtt tgctgtgggg gtcaggtttc ccaggtggga 840

ggagctgcat gcactggcca gcgagcctag agggcagcac gtgctgttgg ctgagcaggt 900

ggaggatgcc accaacggcc tcttcagcac cctcagcagc tcggccatct gctccagcgc 960

cacgccagac tgcagggtcg aggctcaccc ctgtgagcac aggacgctgg agatggtccg 1020
ggagttcgct ggcaatgccc catgctggag aggatcgcgg cggacccttg cggtgctggc 1080
tgcacactgt cccttctaca gctggaagag agtgttccta acccaccctg ccacctgcta 1140
caggaccacc tgcccaggcc cctgtgactc gcagccctgc cagaatggag gcacatgtgt 1200
tccagaagga ctggacggct accagtgcct ctgcccgctg gcctttggag gggaggctaa 1260
ctgtgccctg aagctgagcc tggaatgcag ggtcgacctc ctcttcctgc tggacagctc 1320
tgcgggcacc actctggacg gcttcctgcg ggccaaagtc ttcgtgaagc ggtttgtgcg 1380
ggccgtgctg agcgaggact ctcgggcccg agtgggtgtg gccacataca gcagggagct 1440
gctggtggcg gtgcctgtgg gggagtacca ggatgtgcct gacctggtct ggagcctcga 1500
tggcattccc ttccgtggtg gccccaccct gacgggcagt gccttgcggc aggcggcaga 1560
gcgtggcttc gggagcgcca ccaggacagg ccaggaccgg ccacgtagag tggtggtttt 1620
gctcactgag tcacactccg aggatgaggt tgcgggccca gcgcgtcacg caagggcgcg 1680
agagctgctc ctgctgggtg taggcagtga ggccgtgcgg gcagagctgg aggagatcac 1740
aggcagccca aagcatgtga tggtctactc ggatcctcag gatctgttca accaaatccc 1800
tgagctgcag gggaagctgt gcagccggca gcggccaggg tgccggacac aagccctgga 1860
cctcgtcttc atgttggaca cctctgcctc agtagggccc gagaattttg ctcagatgca 1920
gagctttgtg agaagctgtg ccctccagtt tgaggtgaac cctgacgtga cacaggtcgg 1980
cctggtggtg tatggcagcc aggtgcagac tgccttcggg ctggacacca aacccacccg 2040
ggctgcgatg ctgcgggcca ttagccaggc cccctaccta ggtggggtgg gctcagccgg 2100
caccgccctg ctgcacatct atgacaaagt gatgaccgtc cagaggggtg cccggcctgg 2160
tgtccccaaa gctgtggtgg tgctcacagg cgggagaggc gcagaggatg cagccgttcc 2220
tgcccagaag ctgaggaaca atggcatctc tgtcttggtc gtgggcgtgg ggcctgtcct 2280
aagtgagggt ctgcggaggc ttgcaggtcc ccgggattcc ctgatccacg tggcagctta 2340
cgccgacctg cggtaccacc aggacgtgct cattgagtgg ctgtgtggag aagccaagca 2400
gccagtcaac ctctgcaaac ccagcccgtg catgaatgag ggcagctgcg tcctgcagaa 2460
tgggagctac cgctgcaagt gtcgggatgg ctgggagggc cccccactgcg agaaccgatt 2520
cttgagacgc ccctgaggca catggctccc gtgcaggagg gcagcagccg taccccctccc 2580
agcaactaca gagaaggcct gggcactgaa atggtgccta ccttctggaa tgtctgtgcc 2640
ccaggtcctt agaatgtctg cttcccgccg tggccaggac cactattctc actgagggag 2700
gaggatgtcc caactgcagc catgctgctt agagacaaga aagcagctga tgtcacccac 2760
aaacgatgtt gttgaaaagt tttgatgtgt aagtaaatac ccactttctg tacctgctgt 2820

gccttgttga ggctatgtca tctgccacct ttcccttgag gataaacaag gggtcctgaa 2880
gacttaaatt tagcggcctg acgttccttt gcacacaatc aatgctcgcc agaatgttgt 2940
tgacacagta atgcccagca gaggccttta ctagagcatc ctttggacgg cgaaggccac 3000
ggcctttcaa gatggaaagc agcagctttt ccacttcccc agagacattc tggatgcatt 3060
tgcattgagt ctgaaagggg gcttgaggga cgtttgtgac ttctggcgac tgccttttgt 3120
gtgtggaaga gacttggaaa ggtctcagac tgaaatgtga ccaattaacc agcttgtttg 3180
atgatggggg aggggctgag tgtgcaatgg gcccaggtct ggagggggcca cgtaaaatcg 3240
ttctgagtcg tgagcagtgt ccacctgaag ggtcttcctt tcaaaagagg ctgcggccag 3300
agactgtggc tcatgcctgt aatcccagca ctttggaggc tgaggcaggt ggatcacccg 3360
aggtcaggag tttgagacca gcctggccaa cgtggtgaaa gtttgtcttt actaaaaata 3420
caaaaggtag ccgggggtgg tggtggatgc ctataatccc agctactcgg gaggctgaga 3480
caggagaatg gcttgaacct gggaggcgga agttacagtg agccgagatc tcaccactgc 3540
actccagcct gggcaacaag agtaaaaatc tgtctcaaaa agaaaaaaat gtacttagga 3600
ggggttaatt gtggcgtgtt tatggaattc tttccttatt ctccttttag tggggcaaag 3660
agaagtaaga ttcttaaact caaaaatata ggataaagaa acttacagag attttgcttt 3720
ttaaagcatt gatcttacgc tgtctaggtt ttaattttgt tttgctttgc ttttctacac 3780
agtttttaaa gaaatatttc aagaaatgtt ggttatttat taaacaggga tatttgtacc 3840
tatgtggcaa agaggcatat ttggaatatt ctctggcaaa ctagatactt acttccctat 3900
cgctgcagta tttaggaatt acttcttctc cttggttgtg ttgtttagag ttggattttc 3960
tgtagaaatc tttctagagc tctgatgtga ctccagacac tttatcgttt ttcttttttt 4020
tgagacggag ttttgctctt gttgcccagg ctagagtgca gtggtacaat cttggctcac 4080
tgcaaccttt gcctcccagg ttgaagtgat tctcatgcct cagcctcttg agtagctggg 4140
attagaggca ggtgccacca tgcctagcta atttttgtat ttttagttag agacagggtt 4200
tcaccatgtt ggccaggcta gtctcgaact tctgacctca ggtgatcccc ctgccttggc 4260
ctcccaaagt tctgggatta caggggtgaa ccactgtgcc tggcccattt ttctttataa 4320
atattgtaac ataatgtttt atagacaaac attcaagggt actttggctt taagaacttc 4380
aggatttctg gtgctagaaa agcgcttgaa gcagtatcac caagatttta gatattaaaa 4440
agtctggtgt accagacatt gagtcataat catctatatt caagggatac tttcattgat 4500
aactttgtta ttatgctgcc cttcacagaa gacaacgtcc ggggcaggat cacatgctcc 4560
ctagcagatg ctgatcagtg atgtcataga aattacatga atgcattgtc tttaaatagc 4620
agtttaacca tgttataatg taggcttttt gtcttgttcc gggctggtat ttgggtgccc 4680
tgattgaatt acttggattt aaacagcaaa ctgtgggctc tcgacttaca tagtaagggc 4740

ctttactgtt tctttgtagg aaatgggttt ctcgcctttg aaacattttt tccccttttg 4800
tagtgacagt gccactaaat agttcagctt ttgtcagtcc cccaggaaag tgctatccta 4860
tggcctaact agccaagcct tttttctttc atttaaaaga aattagcttt aatttttacc 4920
tttaattact tattcaaata agacagaaat atattttcct tgcaataatt aaaacattgc 4980
atataggcca taaatttcct tattttctct gaacgatcct gattccagtc atcttgttga 5040
ataccctagt tctaataatt gactcttgct tttctagaga aatatttcca aatgatgcta 5100
gttttgtctc ttcctttcaa agttgtatac cacttctttt tcttgtcatt ttgcattgcc 5160
tgggacctcc agaataatgt ttcatgaagt agcatgtatc catatctggt tcttgacttt 5220
ttcatcataa taattgtttt ctatgggtta cttatcagtt taagaatgct taattcctag 5280
atgaactaag agtgtttatt acatgttgag atttatggta tgctttttct tcctcaagat 5340
aatgcatttt ttgtattatc tgttaatgtg ataggttatc catttgtgta ttttcaatca 5400
ttgaacaacc cttgattttt ttggataaac tctatttggt cattatgcat cattctataa 5460
accctgctga atttttcatt tgccaacatc ttatttcaga ttcttttaat ctgtgtccaa 5520
caatgagatt tgttttcttt tgcaatttgt tttgaatttt tggtatcaga gctatactaa 5580
ccttataatg gaaaatacat atttctcaaa cttttacact gatatattca tagtattttt 5640
ttataatttg aaaaatcttg tcagtatctg tattaaggcc tccatttcag ttctgctatt 5700
tcatattgcc ttaggttgtc tatttgtctc tttaatgaac ccgattttga ttttgtcatt 5760
tttaaaataa acacatttat gctataaact tccttaatt 5799

<210> 156
<211> 755
<212> PRT
<213> Homo sapiens

<400> 156

Met Pro Pro Phe Leu Leu Leu Glu Ala Val Cys Val Phe Leu Phe Ser
1               5                   10                  15

Arg Val Pro Pro Ser Leu Pro Leu Gln Glu Val His Val Ser Lys Glu
            20                  25                  30

Thr Ile Gly Lys Ile Ser Ala Ala Ser Lys Met Met Trp Cys Ser Ala
        35                  40                  45

Ala Val Asp Ile Met Phe Leu Leu Asp Gly Ser Asn Ser Val Gly Lys
    50                  55                  60

Gly Ser Phe Glu Arg Ser Lys His Phe Ala Ile Thr Val Cys Asp Gly
65                  70                  75                  80

```
Leu Asp Ile Ser Pro Glu Arg Val Arg Val Gly Ala Phe Gln Phe Ser
                85                  90                  95

Ser Thr Pro His Leu Glu Phe Pro Leu Asp Ser Phe Ser Thr Gln Gln
            100                 105                 110

Glu Val Lys Ala Arg Ile Lys Arg Met Val Phe Lys Gly Gly Arg Thr
        115                 120                 125

Glu Thr Glu Leu Ala Leu Lys Tyr Leu Leu His Arg Gly Leu Pro Gly
    130                 135                 140

Gly Arg Asn Ala Ser Val Pro Gln Ile Leu Ile Ile Val Thr Asp Gly
145                 150                 155                 160

Lys Ser Gln Gly Asp Val Ala Leu Pro Ser Lys Gln Leu Lys Glu Arg
                165                 170                 175

Gly Val Thr Val Phe Ala Val Gly Val Arg Phe Pro Arg Trp Glu Glu
            180                 185                 190

Leu His Ala Leu Ala Ser Glu Pro Arg Gly Gln His Val Leu Leu Ala
        195                 200                 205

Glu Gln Val Glu Asp Ala Thr Asn Gly Leu Phe Ser Thr Leu Ser Ser
    210                 215                 220

Ser Ala Ile Cys Ser Ser Ala Thr Pro Asp Cys Arg Val Glu Ala His
225                 230                 235                 240

Pro Cys Glu His Arg Thr Leu Glu Met Val Arg Glu Phe Ala Gly Asn
                245                 250                 255

Ala Pro Cys Trp Arg Gly Ser Arg Arg Thr Leu Ala Val Leu Ala Ala
            260                 265                 270

His Cys Pro Phe Tyr Ser Trp Lys Arg Val Phe Leu Thr His Pro Ala
        275                 280                 285

Thr Cys Tyr Arg Thr Thr Cys Pro Gly Pro Cys Asp Ser Gln Pro Cys
    290                 295                 300

Gln Asn Gly Gly Thr Cys Val Pro Glu Gly Leu Asp Gly Tyr Gln Cys
305                 310                 315                 320

Leu Cys Pro Leu Ala Phe Gly Gly Glu Ala Asn Cys Ala Leu Lys Leu
```

325 330 335

Ser Leu Glu Cys Arg Val Asp Leu Leu Phe Leu Leu Asp Ser Ser Ala
340 345 350

Gly Thr Thr Leu Asp Gly Phe Leu Arg Ala Lys Val Phe Val Lys Arg
355 360 365

Phe Val Arg Ala Val Leu Ser Glu Asp Ser Arg Ala Arg Val Gly Val
370 375 380

Ala Thr Tyr Ser Arg Glu Leu Leu Val Ala Val Pro Val Gly Glu Tyr
385 390 395 400

Gln Asp Val Pro Asp Leu Val Trp Ser Leu Asp Gly Ile Pro Phe Arg
405 410 415

Gly Gly Pro Thr Leu Thr Gly Ser Ala Leu Arg Gln Ala Ala Glu Arg
420 425 430

Gly Phe Gly Ser Ala Thr Arg Thr Gly Gln Asp Arg Pro Arg Arg Val
435 440 445

Val Val Leu Leu Thr Glu Ser His Ser Glu Asp Glu Val Ala Gly Pro
450 455 460

Ala Arg His Ala Arg Ala Arg Glu Leu Leu Leu Leu Gly Val Gly Ser
465 470 475 480

Glu Ala Val Arg Ala Glu Leu Glu Glu Ile Thr Gly Ser Pro Lys His
485 490 495

Val Met Val Tyr Ser Asp Pro Gln Asp Leu Phe Asn Gln Ile Pro Glu
500 505 510

Leu Gln Gly Lys Leu Cys Ser Arg Gln Arg Pro Gly Cys Arg Thr Gln
515 520 525

Ala Leu Asp Leu Val Phe Met Leu Asp Thr Ser Ala Ser Val Gly Pro
530 535 540

Glu Asn Phe Ala Gln Met Gln Ser Phe Val Arg Ser Cys Ala Leu Gln
545 550 555 560

Phe Glu Val Asn Pro Asp Val Thr Gln Val Gly Leu Val Val Tyr Gly
565 570 575

Ser Gln Val Gln Thr Ala Phe Gly Leu Asp Thr Lys Pro Thr Arg Ala
580 585 590

Ala Met Leu Arg Ala Ile Ser Gln Ala Pro Tyr Leu Gly Gly Val Gly
595 600 605

Ser Ala Gly Thr Ala Leu Leu His Ile Tyr Asp Lys Val Met Thr Val
610 615 620

Gln Arg Gly Ala Arg Pro Gly Val Pro Lys Ala Val Val Val Leu Thr
625 630 635 640

Gly Gly Arg Gly Ala Glu Asp Ala Ala Val Pro Ala Gln Lys Leu Arg
645 650 655

Asn Asn Gly Ile Ser Val Leu Val Val Gly Val Gly Pro Val Leu Ser
660 665 670

Glu Gly Leu Arg Arg Leu Ala Gly Pro Arg Asp Ser Leu Ile His Val
675 680 685

Ala Ala Tyr Ala Asp Leu Arg Tyr His Gln Asp Val Leu Ile Glu Trp
690 695 700

Leu Cys Gly Glu Ala Lys Gln Pro Val Asn Leu Cys Lys Pro Ser Pro
705 710 715 720

Cys Met Asn Glu Gly Ser Cys Val Leu Gln Asn Gly Ser Tyr Arg Cys
725 730 735

Lys Cys Arg Asp Gly Trp Glu Gly Pro His Cys Glu Asn Arg Phe Leu
740 745 750

Arg Arg Pro
755

<210> 157
<211> 1860
<212> DNA
<213> Homo sapiens

<400> 157
atgccagacc ccgcggcgca cctgcccttc ttctacggca gcatctcgcg tgccgaggcc 60

gaggagcacc tgaagctggc gggcatggcg gacgggctct tcctgctgcg ccagtgcctg 120

cgctcgctgg gcggctatgt gctgtcgctc gtgcacgatg tgcgcttcca ccactttccc 180

atcgagcgcc agctcaacgg cacctacgcc attgccggcg gcaaagcgca ctgtggaccg 240

gcagagctct gcgagttcta ctcgcgcgac cccgacgggc tgccctgcaa cctgcgcaag 300

ccgtgcaacc ggccgtcggg cctcgagccg cagccggggg tcttcgactg cctgcgagac 360

gccatggtgc gtgactacgt gcgccagacg tggaagctgg agggcgaggc cctggagcag 420

gccatcatca gccaggcccc gcaggtggag aagctcattg ctacgacggc ccacgagcgg 480

atgccctggt accacagcag cctgacgcgt gaggaggccg agcgcaaact ttactctggg 540

gcgcagaccg acggcaagtt cctgctgagg ccgcggaagg agcagggcac atacgccctg 600

tccctcatct atgggaagac ggtgtaccac tacctcatca gccaagacaa ggcgggcaag 660

tactgcattc ccgagggcac caagtttgac acgctctggc agctggtgga gtatctgaag 720

ctgaaggcgg acgggctcat ctactgcctg aaggaggcct gccccaacag cagtgccagc 780

aacgcctcag ggctgctgc tcccacactc ccagcccacc catccacgtt gactcatcct 840

cagagacgaa tcgacaccct caactcagat ggatacaccc ctgagccagc acgcataacg 900

tccccagaca aaccgcggcc gatgcccatg gacacgagcg tgtatgagag cccctacagc 960

gacccagagg agctcaagga caagaagctc ttcctgaagc gcgataacct cctcatagct 1020

gacattgaac ttggctgcgg caactttggc tcagtgcgcc agggcgtgta ccgcatgcgc 1080

aagaagcaga tcgacgtggc catcaaggtg ctgaagcagg gcacggagaa ggcagacacg 1140

gaagagatga tgcgcgaggc gcagatcatg caccagctgg acaaccccta catcgtgcgg 1200

ctcattggcg tctgccaggc cgaggccctc atgctggtca tggagatggc tgggggcggg 1260

ccgctgcaca agttcctggt cggcaagagg gaggagatcc ctgtgagcaa tgtggccgag 1320

ctgctgcacc aggtgtccat ggggatgaag tacctggagg agaagaactt tgtgcaccgt 1380

gacctggcgg cccgcaacgt cctgctggtt aaccggcact acgccaagat cagcgacttt 1440

ggcctctcca aagcactggg tgccgacgac agctactaca ctgcccgctc agcagggaag 1500

tggccgctca agtggtacgc acccgaatgc atcaacttcc gcaagttctc cagccgcagc 1560

gatgtctgga gctatggggt caccatgtgg gaggccttgt cctacggcca gaagccctac 1620

aagaagatga aagggccgga ggtcatggcc ttcatcgagc agggcaagcg gatggagtgc 1680

ccaccagagt gtccacccga actgtacgca ctcatgagtg actgctggat ctacaagtgg 1740

gaggatcgcc ccgacttcct gaccgtggag cagcgcatgc gagcctgtta ctacagcctg 1800

gccagcaagg tggaagggcc cccaggcagc acacagaagg ctgaggctgc ctgtgcctga 1860

<210> 158
<211> 1468
<212> DNA
<213> Homo sapiens

<400> 158
tgcatgctcc agggacggca cccttgtctg gggcaggtgc ttgtgggggc tgaggctgcc 60

ttgctcccca cacccctgcc cctgacctgg gagtgtaccg ctgtgtgtgc ccagcacgca 120

```
taacgtcccc agacaaaccg cggccgatgc ccatggacac gagcgtgtat gagagcccct       180
acagcgaccc agaggagctc aaggacaaga agctcttcct gaagcgcgat aacctcctca       240
tagctgacat tgaacttggc tgcggcaact ttggctcagt gcgccagggc gtgtaccgca       300
tgcgcaagaa gcagatcgac gtggccatca aggtgctgaa gcagggcacg gagaaggcag       360
acacggaaga gatgatgcgc gaggcgcaga tcatgcacca gctggacaac ccctacatcg       420
tgcggctcat tggcgtctgc caggccgagg ccctcatgct ggtcatggag atggctgggg       480
gcgggccgct gcacaagttc ctggtcggca agagggagga gatccctgtg agcaatgtgg       540
ccgagctgct gcaccaggtg tccatgggga tgaagtacct ggaggagaag aactttgtgc       600
accgtgacct ggcggcccgc aacgtcctgc tggttaaccg gcactacgcc aagatcagcg       660
actttggcct ctccaaagca ctgggtgccg acgacagcta ctacactgcc cgctcagcag       720
ggaagtggcc gctcaagtgg tacgcacccg aatgcatcaa cttccgcaag ttctccagcc       780
gcagcgatgt ctggagctat ggggtcacca tgtgggaggc cttgtcctac ggccagaagc       840
cctacaagaa gatgaaaggg ccggaggtca tggccttcat cgagcagggc aagcggatgg       900
agtgcccacc agagtgtcca cccgaactgt acgcactcat gagtgactgc tggatctaca       960
agtgggagga tcgccccgac ttcctgaccg tggagcagcg catgcgagcc tgttactaca      1020
gcctggccag caaggtggaa gggcccccag gcagcacaca gaaggctgag gctgcctgtg      1080
cctgagctcc cgctgcccag gggagccctc cacgccggct cttccccacc ctcagcccca      1140
ccccaggtcc tgcagtctgg ctgagccctg cttggttgtc tccacacaca gctgggctgt      1200
ggtagggggt gtctcaggcc acaccggcct tgcattgcct gcctggcccc ctgtcctctc      1260
tggctgggga gcagggaggt ccgggagggt gcggctgtgc agcctgtcct gggctggtgg      1320
ctcccggagg gccctgagct gagggcattg cttacacgga tgccttcccc tgggccctga      1380
cattggagcc tgggcatcct caggtggtca ggcgtagatc accagaataa acccagcttc      1440
cctcttgaaa aaaaaaaaaa aaaaaaa                                          1468
```

<210>  159
<211>  619
<212>  PRT
<213>  Homo sapiens

<400>  159

```
Met Pro Asp Pro Ala Ala His Leu Pro Phe Phe Tyr Gly Ser Ile Ser
1               5                   10                  15

Arg Ala Glu Ala Glu Glu His Leu Lys Leu Ala Gly Met Ala Asp Gly
            20                  25                  30
```

```
Leu Phe Leu Leu Arg Gln Cys Leu Arg Ser Leu Gly Gly Tyr Val Leu
        35                  40                  45

Ser Leu Val His Asp Val Arg Phe His His Phe Pro Ile Glu Arg Gln
    50                  55                  60

Leu Asn Gly Thr Tyr Ala Ile Ala Gly Gly Lys Ala His Cys Gly Pro
65                  70                  75                  80

Ala Glu Leu Cys Glu Phe Tyr Ser Arg Asp Pro Asp Gly Leu Pro Cys
                85                  90                  95

Asn Leu Arg Lys Pro Cys Asn Arg Pro Ser Gly Leu Glu Pro Gln Pro
            100                 105                 110

Gly Val Phe Asp Cys Leu Arg Asp Ala Met Val Arg Asp Tyr Val Arg
        115                 120                 125

Gln Thr Trp Lys Leu Glu Gly Glu Ala Leu Glu Gln Ala Ile Ile Ser
    130                 135                 140

Gln Ala Pro Gln Val Glu Lys Leu Ile Ala Thr Thr Ala His Glu Arg
145                 150                 155                 160

Met Pro Trp Tyr His Ser Ser Leu Thr Arg Glu Glu Ala Glu Arg Lys
                165                 170                 175

Leu Tyr Ser Gly Ala Gln Thr Asp Gly Lys Phe Leu Leu Arg Pro Arg
            180                 185                 190

Lys Glu Gln Gly Thr Tyr Ala Leu Ser Leu Ile Tyr Gly Lys Thr Val
        195                 200                 205

Tyr His Tyr Leu Ile Ser Gln Asp Lys Ala Gly Lys Tyr Cys Ile Pro
    210                 215                 220

Glu Gly Thr Lys Phe Asp Thr Leu Trp Gln Leu Val Glu Tyr Leu Lys
225                 230                 235                 240

Leu Lys Ala Asp Gly Leu Ile Tyr Cys Leu Lys Glu Ala Cys Pro Asn
                245                 250                 255

Ser Ser Ala Ser Asn Ala Ser Gly Ala Ala Ala Pro Thr Leu Pro Ala
            260                 265                 270

His Pro Ser Thr Leu Thr His Pro Gln Arg Arg Ile Asp Thr Leu Asn
        275                 280                 285
```

Ser Asp Gly Tyr Thr Pro Glu Pro Ala Arg Ile Thr Ser Pro Asp Lys
290 295 300

Pro Arg Pro Met Pro Met Asp Thr Ser Val Tyr Glu Ser Pro Tyr Ser
305 310 315 320

Asp Pro Glu Glu Leu Lys Asp Lys Lys Leu Phe Leu Lys Arg Asp Asn
325 330 335

Leu Leu Ile Ala Asp Ile Glu Leu Gly Cys Gly Asn Phe Gly Ser Val
340 345 350

Arg Gln Gly Val Tyr Arg Met Arg Lys Lys Gln Ile Asp Val Ala Ile
355 360 365

Lys Val Leu Lys Gln Gly Thr Glu Lys Ala Asp Thr Glu Glu Met Met
370 375 380

Arg Glu Ala Gln Ile Met His Gln Leu Asp Asn Pro Tyr Ile Val Arg
385 390 395 400

Leu Ile Gly Val Cys Gln Ala Glu Ala Leu Met Leu Val Met Glu Met
405 410 415

Ala Gly Gly Gly Pro Leu His Lys Phe Leu Val Gly Lys Arg Glu Glu
420 425 430

Ile Pro Val Ser Asn Val Ala Glu Leu Leu His Gln Val Ser Met Gly
435 440 445

Met Lys Tyr Leu Glu Glu Lys Asn Phe Val His Arg Asp Leu Ala Ala
450 455 460

Arg Asn Val Leu Leu Val Asn Arg His Tyr Ala Lys Ile Ser Asp Phe
465 470 475 480

Gly Leu Ser Lys Ala Leu Gly Ala Asp Asp Ser Tyr Tyr Thr Ala Arg
485 490 495

Ser Ala Gly Lys Trp Pro Leu Lys Trp Tyr Ala Pro Glu Cys Ile Asn
500 505 510

Phe Arg Lys Phe Ser Ser Arg Ser Asp Val Trp Ser Tyr Gly Val Thr
515 520 525

Met Trp Glu Ala Leu Ser Tyr Gly Gln Lys Pro Tyr Lys Lys Met Lys
530 535 540

Gly Pro Glu Val Met Ala Phe Ile Glu Gln Gly Lys Arg Met Glu Cys
545 550 555 560

Pro Pro Glu Cys Pro Pro Glu Leu Tyr Ala Leu Met Ser Asp Cys Trp
565 570 575

Ile Tyr Lys Trp Glu Asp Arg Pro Asp Phe Leu Thr Val Glu Gln Arg
580 585 590

Met Arg Ala Cys Tyr Tyr Ser Leu Ala Ser Lys Val Glu Gly Pro Pro
595 600 605

Gly Ser Thr Gln Lys Ala Glu Ala Ala Cys Ala
610 615

<210> 160
<211> 312
<212> PRT
<213> Homo sapiens

<400> 160

Met Pro Met Asp Thr Ser Val Tyr Glu Ser Pro Tyr Ser Asp Pro Glu
1 5 10 15

Glu Leu Lys Asp Lys Lys Leu Phe Leu Lys Arg Asp Asn Leu Leu Ile
20 25 30

Ala Asp Ile Glu Leu Gly Cys Gly Asn Phe Gly Ser Val Arg Gln Gly
35 40 45

Val Tyr Arg Met Arg Lys Lys Gln Ile Asp Val Ala Ile Lys Val Leu
50 55 60

Lys Gln Gly Thr Glu Lys Ala Asp Thr Glu Glu Met Met Arg Glu Ala
65 70 75 80

Gln Ile Met His Gln Leu Asp Asn Pro Tyr Ile Val Arg Leu Ile Gly
85 90 95

Val Cys Gln Ala Glu Ala Leu Met Leu Val Met Glu Met Ala Gly Gly
100 105 110

Gly Pro Leu His Lys Phe Leu Val Gly Lys Arg Glu Glu Ile Pro Val
115 120 125

Ser Asn Val Ala Glu Leu Leu His Gln Val Ser Met Gly Met Lys Tyr
130 135 140

Leu Glu Glu Lys Asn Phe Val His Arg Asp Leu Ala Ala Arg Asn Val
145 150 155 160

Leu Leu Val Asn Arg His Tyr Ala Lys Ile Ser Asp Phe Gly Leu Ser
165 170 175

Lys Ala Leu Gly Ala Asp Asp Ser Tyr Tyr Thr Ala Arg Ser Ala Gly
180 185 190

Lys Trp Pro Leu Lys Trp Tyr Ala Pro Glu Cys Ile Asn Phe Arg Lys
195 200 205

Phe Ser Ser Arg Ser Asp Val Trp Ser Tyr Gly Val Thr Met Trp Glu
210 215 220

Ala Leu Ser Tyr Gly Gln Lys Pro Tyr Lys Lys Met Lys Gly Pro Glu
225 230 235 240

Val Met Ala Phe Ile Glu Gln Gly Lys Arg Met Glu Cys Pro Pro Glu
245 250 255

Cys Pro Pro Glu Leu Tyr Ala Leu Met Ser Asp Cys Trp Ile Tyr Lys
260 265 270

Trp Glu Asp Arg Pro Asp Phe Leu Thr Val Glu Gln Arg Met Arg Ala
275 280 285

Cys Tyr Tyr Ser Leu Ala Ser Lys Val Glu Gly Pro Pro Gly Ser Thr
290 295 300

Gln Lys Ala Glu Ala Ala Cys Ala
305 310

<210> 161
<211> 2142
<212> DNA
<213> Homo sapiens

<400> 161
agagctgcaa gaagcaccag gctcggccac ttcagaagcc ccagcctcga cctagcccac 60

cctctcaggg ccacagtgca gaagcctgca cacctgccaa gtctctccga ctccttgcag 120

ctgctgtcag catggcccag gctcctgctg acccgggcag agaaggccac cttgaacaaa 180

gaatcctgca ggtgctgaca gaggctggct ccccggtgaa acttgcccag ctggtgaagg 240

aatgccaagc acccaagagg gagctcaacc aagtcctcta ccgaatgaaa aaggagttga 300

aagtctccct cacatcccct gccacctggt gcttgggcgg gactgatcct gaaggcgagg 360

gtcctgcaga gctggccttg tccagccctg ccgagaggcc ccagcaacat gcagctacaa 420

ttccagagac ccctggccct cagttcagcc aacaacggga ggaagacatc tacaggtttc 480

tcaaagacaa tggtccccag agggccctgg tcatcgccca agcactggga atgaggacag 540

caaaagatgt gaaccgagac ttgtacagga tgaagagcag gcaccttctg gacatggatg 600

agcagtccaa agcatggacg atttaccgcc cagaagattc tggaagaaga gcaaagtcag 660

cctcaattat ttaccagcac aatccaatca acatgatctg ccagaatgga cccaacagct 720

ggatttccat tgcaaactcc gaagccatcc agattggaca cgggaacatc attacaagac 780

agacagtctc cagggaggac ggttccgccg gtccacgcca cctcccttca atggcaccag 840

gtgattcctc aacttggggg accctagttg atccctgggg gccccaggac atccacatgg 900

agcagtccat actgagacgg gtgcagctgg gacacagcaa tgagatgagg ctccacggcg 960

tcccgtccga gggccctgcc cacatccccc ctggcagccc cccagtctct gccactgctg 1020

ccggcccaga agcttcgttt gaagcaagaa ttcccagtcc aggaactcac cctgaggggg 1080

aagccgccca gagaatccac atgaaatcgt gctttctcga ggacgccacc atcggcaaca 1140

gcaacaaaat gtctatcagc ccaggggtgg ctggcccagg aggagtcgca gggtctggag 1200

agggggagcc aggggaggac gcaggtcgtc gtcccgcaga cacacaatcc agaagtcact 1260

ttcctcgaga cattggtcag cccatcactc ccagccactc gaagctcacc cccaagctgg 1320

aaactatgac tcttggaaac aggagtcaca aagctgcaga aggcagccac tatgtggatg 1380

aagcctcaca cgaggggagc tggtggggag gtgggattta gtgcacagcc tcacgtgggg 1440

cttggacaca ggctgggggt gggcgcatgc tagggagact agcctgctgc tctctgcatt 1500

ccttagcgtc ttgtttgacc tgcttgcttc cagacataac ctgcatgaat cagttttggg 1560

ggaatggacc tggcatgggg atgggttcag gccaggtctt ttgatggcca ggagtagatg 1620

acagggagtt gccttgggga acctttggtg tgccaagagg aggtgggtag atgggagtgg 1680

ggctcggtcc cccaggccca ggggactctc tccactcttt cctgggctcg gggcatctgc 1740

ctggagttac cttccatcat ggctacctgc tgtggtttga atgtttgagt cccaacaaaa 1800

ttcatatcaa aacataatcc caactgggtg cagtggctca cgcctgtaat cccagcactt 1860

tgggaggccg aggcgggcgg atcaataggt caggaaatcc agaccgtcct ggctaacatg 1920

gtgaaacccc gtctctacta aaaaaaaaaa tacaaaaaat tagccgggcg ttgtggcggg 1980

cacctggagt cccagctact ccggaggctg agggaggaga atggtgtgaa cccgggaggt 2040

ggagcttcca gtgagccgag atcgcgccac tgcactccag gctgggcgac agagcgagac 2100

tccgtctcaa aaaaataaat acataaataa aaaataaacc aa 2142

<210> 162

<211> 1917
<212> DNA
<213> Homo sapiens

<400> 162
agagctgcaa gaagcaccag gctcggccac ttcagaagcc ccagcctcga cctagcccac 60

cctctcaggg ccacagtgca gaagcctgca cacctgccaa gtctctccga ctccttgcag 120

ctgctgtcag catggcccag gctcctgctg acccgggcag agaagccgag aggccccagc 180

aacatgcagc tacaattcca gagacccctg gccctcagtt cagccaacaa cgggaggaag 240

acatctacag gtttctcaaa gacaatggtc cccagagggc cctggtcatc gcccaagcac 300

tgggaatgag gacagcaaaa gatgtgaacc gagacttgta caggatgaag agcaggcacc 360

ttctggacat ggatgagcag tccaaagcat ggacgattta ccgcccagaa gattctggaa 420

gaagagcaaa gtcagcctca attatttacc agcacaatcc aatcaacatg atctgccaga 480

atggacccaa cagctggatt tccattgcaa actccgaagc catccagatt ggacacggga 540

acatcattac aagacagaca gtctccaggg aggacggttc cgccggtcca cgccacctcc 600

cttcaatggc accaggtgat tcctcaactt gggggaccct agttgatccc tgggggcccc 660

aggacatcca catggagcag tccatactga gacgggtgca gctgggacac agcaatgaga 720

tgaggctcca cggcgtcccg tccgagggcc ctgcccacat ccccccctggc agccccccag 780

tctctgccac tgctgccggc ccagaagctt cgtttgaagc aagaattccc agtccaggaa 840

ctcaccctga gggggaagcc gcccagagaa tccacatgaa atcgtgcttt ctcgaggacg 900

ccaccatcgg caacagcaac aaaatgtcta tcagcccagg ggtggctggc ccaggaggag 960

tcgcagggtc tggagagggg gagccagggg aggacgcagg tcgtcgtccc gcagacacac 1020

aatccagaag tcactttcct cgagacattg gtcagcccat cactcccagc cactcgaagc 1080

tcacccccaa gctggaaact atgactcttg gaaacaggag tcacaaagct gcagaaggca 1140

gccactatgt ggatgaagcc tcacacgagg ggagctggtg gggaggtggg atttagtgca 1200

cagcctcacg tggggcttgg acacaggctg ggggtgggcg catgctaggg agactagcct 1260

gctgctctct gcattcctta gcgtcttgtt tgacctgctt gcttccagac ataacctgca 1320

tgaatcagtt ttgggggaat ggacctggca tggggatggg ttcaggccag gtcttttgat 1380

ggccaggagt agatgacagg gagttgcctt ggggaacctt tggtgtgcca agaggaggtg 1440

ggtagatggg agtggggctc ggtcccccag gcccagggga ctctctccac tctttcctgg 1500

gctcggggca tctgcctgga gttaccttcc atcatggcta cctgctgtgg tttgaatgtt 1560

tgagtcccaa caaaattcat atcaaaacat aatcccaact gggtgcagtg gctcacgcct 1620

gtaatcccag cactttggga ggccgaggcg ggcggatcaa taggtcagga aatccagacc 1680

gtcctggcta acatggtgaa accccgtctc tactaaaaaa aaaaatacaa aaaattagcc 1740

gggcgttgtg gcgggcacct ggagtcccag ctactccgga ggctgaggga ggagaatggt 1800

gtgaacccgg gaggtggagc ttccagtgag ccgagatcgc gccactgcac tccaggctgg 1860

gcgacagagc gagactccgt ctcaaaaaaa taaatacata aataaaaaat aaaccaa 1917

<210> 163
<211> 1255
<212> DNA
<213> Homo sapiens

<400> 163
ttttttttc tttcaaaaga cgaacagaga agtttcattt tctttttctc ctgaaaccga 60

atctggccgg cctggctagg catctatttc cgggctgtaa gcagctgaca cctgcccagt 120

ggaagctggc atccctcccc ttgtgggttc agagctgcaa gaagcaccag gctcggccac 180

ttcagaagcc ccagcctcga cctagcccac cctctcaggg ccacagtgca gaagcctgca 240

cacctgccaa gtctctccga ctccttgcag ctgctgtcag catggcccag gctcctgctg 300

acccgggcag agaaggccac cttgaacaaa gaatcctgca ggtgctgaca gaggctggct 360

ccccggtgaa acttgcccag ctggtgaagg aatgccaagc acccaagagg gagctcaacc 420

aagtcctcta ccgaatgaaa aaggagttga aagtctccct cacatcccct gccacctggt 480

gcttgggcgg gactgatcct gaaggcgagg gtcctgcaga gctggccttg tccagccctg 540

ccgagaggcc ccagcaacat gcagctacaa ttccagagac ccctggccct cagttcagcc 600

aacaacggga ggaagacatc tacaggtttc tcaaagacaa tggtccccag agggccctgg 660

tcatcgccca agcactggga atgaggacag caaaagatgt gaaccgagac ttgtacagga 720

tgaagagcag gcaccttctg gacatggatg agcagtccaa agcatggacg atttaccgcc 780

cagaagattc tggaagaaga gcaaagtcag cctcaattat ttaccagcac aatccaatca 840

acatgatctg ccagaatgga cccaacagct ggatttccat tgcaaactcc gaagccatcc 900

agattggaca cgggaacatc attacaagac agacagtctc cagggaggac ggtaagtcac 960

ccaagagagc ccagggaggg gacctcggtg gggagccacc ggatcctctg ggtgggggca 1020

aagggtaggg atggggagtg gggggattct gccctccaag gggaaagggt tgcttccaga 1080

cccccacagt ccacctttac ggccgtcctg agaatgagga cacctggatc aaagctctcc 1140

tgatttccct gtactctgat actttctacc tcattttaaa gtttaattta atttttattt 1200

ttctaataaa attttaaaat taagatggga aaaaaaaaaa aaaaaaaaaa aaaaa 1255

<210> 164
<211> 429
<212> PRT
<213> Homo sapiens

<400> 164

```
Met Ala Gln Ala Pro Ala Asp Pro Gly Arg Glu Gly His Leu Glu Gln
1               5                   10                  15

Arg Ile Leu Gln Val Leu Thr Glu Ala Gly Ser Pro Val Lys Leu Ala
            20                  25                  30

Gln Leu Val Lys Glu Cys Gln Ala Pro Lys Arg Glu Leu Asn Gln Val
        35                  40                  45

Leu Tyr Arg Met Lys Lys Glu Leu Lys Val Ser Leu Thr Ser Pro Ala
    50                  55                  60

Thr Trp Cys Leu Gly Gly Thr Asp Pro Glu Gly Glu Gly Pro Ala Glu
65                  70                  75                  80

Leu Ala Leu Ser Ser Pro Ala Glu Arg Pro Gln Gln His Ala Ala Thr
                85                  90                  95

Ile Pro Glu Thr Pro Gly Pro Gln Phe Ser Gln Gln Arg Glu Glu Asp
            100                 105                 110

Ile Tyr Arg Phe Leu Lys Asp Asn Gly Pro Gln Arg Ala Leu Val Ile
        115                 120                 125

Ala Gln Ala Leu Gly Met Arg Thr Ala Lys Asp Val Asn Arg Asp Leu
    130                 135                 140

Tyr Arg Met Lys Ser Arg His Leu Leu Asp Met Asp Glu Gln Ser Lys
145                 150                 155                 160

Ala Trp Thr Ile Tyr Arg Pro Glu Asp Ser Gly Arg Arg Ala Lys Ser
                165                 170                 175

Ala Ser Ile Ile Tyr Gln His Asn Pro Ile Asn Met Ile Cys Gln Asn
            180                 185                 190

Gly Pro Asn Ser Trp Ile Ser Ile Ala Asn Ser Glu Ala Ile Gln Ile
        195                 200                 205

Gly His Gly Asn Ile Ile Thr Arg Gln Thr Val Ser Arg Glu Asp Gly
    210                 215                 220

Ser Ala Gly Pro Arg His Leu Pro Ser Met Ala Pro Gly Asp Ser Ser
225                 230                 235                 240

Thr Trp Gly Thr Leu Val Asp Pro Trp Gly Pro Gln Asp Ile His Met
                245                 250                 255
```

Glu Gln Ser Ile Leu Arg Arg Val Gln Leu Gly His Ser Asn Glu Met
260 265 270

Arg Leu His Gly Val Pro Ser Glu Gly Pro Ala His Ile Pro Pro Gly
275 280 285

Ser Pro Pro Val Ser Ala Thr Ala Ala Gly Pro Glu Ala Ser Phe Glu
290 295 300

Ala Arg Ile Pro Ser Pro Gly Thr His Pro Glu Gly Glu Ala Ala Gln
305 310 315 320

Arg Ile His Met Lys Ser Cys Phe Leu Glu Asp Ala Thr Ile Gly Asn
325 330 335

Ser Asn Lys Met Ser Ile Ser Pro Gly Val Ala Gly Pro Gly Gly Val
340 345 350

Ala Gly Ser Gly Glu Gly Glu Pro Gly Glu Asp Ala Gly Arg Arg Pro
355 360 365

Ala Asp Thr Gln Ser Arg Ser His Phe Pro Arg Asp Ile Gly Gln Pro
370 375 380

Ile Thr Pro Ser His Ser Lys Leu Thr Pro Lys Leu Glu Thr Met Thr
385 390 395 400

Leu Gly Asn Arg Ser His Lys Ala Ala Glu Gly Ser His Tyr Val Asp
405 410 415

Glu Ala Ser His Glu Gly Ser Trp Trp Gly Gly Gly Ile
420 425

<210> 165
<211> 354
<212> PRT
<213> Homo sapiens

<400> 165

Met Ala Gln Ala Pro Ala Asp Pro Gly Arg Glu Ala Glu Arg Pro Gln
1 5 10 15

Gln His Ala Ala Thr Ile Pro Glu Thr Pro Gly Pro Gln Phe Ser Gln
20 25 30

Gln Arg Glu Glu Asp Ile Tyr Arg Phe Leu Lys Asp Asn Gly Pro Gln
35 40 45

Arg Ala Leu Val Ile Ala Gln Ala Leu Gly Met Arg Thr Ala Lys Asp
    50                  55                  60

Val Asn Arg Asp Leu Tyr Arg Met Lys Ser Arg His Leu Leu Asp Met
65                  70                  75                  80

Asp Glu Gln Ser Lys Ala Trp Thr Ile Tyr Arg Pro Glu Asp Ser Gly
                85                  90                  95

Arg Arg Ala Lys Ser Ala Ser Ile Ile Tyr Gln His Asn Pro Ile Asn
            100                 105                 110

Met Ile Cys Gln Asn Gly Pro Asn Ser Trp Ile Ser Ile Ala Asn Ser
        115                 120                 125

Glu Ala Ile Gln Ile Gly His Gly Asn Ile Ile Thr Arg Gln Thr Val
    130                 135                 140

Ser Arg Glu Asp Gly Ser Ala Gly Pro Arg His Leu Pro Ser Met Ala
145                 150                 155                 160

Pro Gly Asp Ser Ser Thr Trp Gly Thr Leu Val Asp Pro Trp Gly Pro
                165                 170                 175

Gln Asp Ile His Met Glu Gln Ser Ile Leu Arg Arg Val Gln Leu Gly
            180                 185                 190

His Ser Asn Glu Met Arg Leu His Gly Val Pro Ser Glu Gly Pro Ala
        195                 200                 205

His Ile Pro Pro Gly Ser Pro Pro Val Ser Ala Thr Ala Ala Gly Pro
    210                 215                 220

Glu Ala Ser Phe Glu Ala Arg Ile Pro Ser Pro Gly Thr His Pro Glu
225                 230                 235                 240

Gly Glu Ala Ala Gln Arg Ile His Met Lys Ser Cys Phe Leu Glu Asp
                245                 250                 255

Ala Thr Ile Gly Asn Ser Asn Lys Met Ser Ile Ser Pro Gly Val Ala
            260                 265                 270

Gly Pro Gly Gly Val Ala Gly Ser Gly Glu Gly Glu Pro Gly Glu Asp
        275                 280                 285

Ala Gly Arg Arg Pro Ala Asp Thr Gln Ser Arg Ser His Phe Pro Arg
    290                 295                 300

Asp Ile Gly Gln Pro Ile Thr Pro Ser His Ser Lys Leu Thr Pro Lys
305 310 315 320

Leu Glu Thr Met Thr Leu Gly Asn Arg Ser His Lys Ala Ala Glu Gly
325 330 335

Ser His Tyr Val Asp Glu Ala Ser His Glu Gly Ser Trp Trp Gly Gly
340 345 350

Gly Ile

<210> 166
<211> 248
<212> PRT
<213> Homo sapiens

<400> 166

Met Ala Gln Ala Pro Ala Asp Pro Gly Arg Glu Gly His Leu Glu Gln
1 5 10 15

Arg Ile Leu Gln Val Leu Thr Glu Ala Gly Ser Pro Val Lys Leu Ala
20 25 30

Gln Leu Val Lys Glu Cys Gln Ala Pro Lys Arg Glu Leu Asn Gln Val
35 40 45

Leu Tyr Arg Met Lys Lys Glu Leu Lys Val Ser Leu Thr Ser Pro Ala
50 55 60

Thr Trp Cys Leu Gly Gly Thr Asp Pro Glu Gly Glu Gly Pro Ala Glu
65 70 75 80

Leu Ala Leu Ser Ser Pro Ala Glu Arg Pro Gln Gln His Ala Ala Thr
85 90 95

Ile Pro Glu Thr Pro Gly Pro Gln Phe Ser Gln Gln Arg Glu Glu Asp
100 105 110

Ile Tyr Arg Phe Leu Lys Asp Asn Gly Pro Gln Arg Ala Leu Val Ile
115 120 125

Ala Gln Ala Leu Gly Met Arg Thr Ala Lys Asp Val Asn Arg Asp Leu
130 135 140

Tyr Arg Met Lys Ser Arg His Leu Leu Asp Met Asp Glu Gln Ser Lys
145 150 155 160

```
Ala Trp Thr Ile Tyr Arg Pro Glu Asp Ser Gly Arg Arg Ala Lys Ser
                165                 170                 175

Ala Ser Ile Ile Tyr Gln His Asn Pro Ile Asn Met Ile Cys Gln Asn
            180                 185                 190

Gly Pro Asn Ser Trp Ile Ser Ile Ala Asn Ser Glu Ala Ile Gln Ile
        195                 200                 205

Gly His Gly Asn Ile Ile Thr Arg Gln Thr Val Ser Arg Glu Asp Gly
    210                 215                 220

Lys Ser Pro Lys Arg Ala Gln Gly Gly Asp Leu Gly Gly Glu Pro Pro
225                 230                 235                 240

Asp Pro Leu Gly Gly Gly Lys Gly
                245
```

## Description

### FIELD OF THE INVENTION

The invention relates to a method of predicting an outcome of a colorectal cancer subject, and to an apparatus for predicting an outcome of a colorectal cancer subject. Moreover, the invention relates to a diagnostic kit, to a use of the kit, to a use of the kit in a method of predicting an outcome of a colorectal cancer subject, to a use of first, second, and/or third gene expression profile(s) in a method of predicting an outcome of a colorectal cancer subject, and to a corresponding computer program product.

### BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Colorectal cancer (CRC) is fairly common cancer, ranking 4$^{th}$ (8.2%) in representing new cases, and even ranking 2$^{nd}$ in number of estimated deaths for 2020 in the US. For 2020, it is estimated that almost 148,000 new cases of colorectal cancer will be diagnosed, and more than 53,000 will die from the disease. The median age of diagnosis is 67 with a 5-year relative survival of 64.6%. The earlier colorectal cancer is diagnosed, the higher the chance of surviving more than 5 years after diagnosis; the 5-year survival for localized colorectal cancer is 90.2%, while only 14.3% of patients with distant colorectal cancer survive for at least 5 years. About 38% of the patients present with localized disease. Men are affected more than women, and incidence increases with age (see National Cancer Institute, SEER Cancer Stat Facts: Colorectal Cancer, https://seer.cancer. gov/statfacts/html/colorect.html, accessed 09/09/2020). Risk factors for colorectal cancer include older age, having a family history of colorectal cancer in a first-degree relative, having a personal history of cancer of the colon, rectum, or ovary, having a personal history of high-risk adenomas, having inherited changes in certain genes that increase the risk of familial adenomatous polyposis (FAP) or Lynch syndrome, having a personal history of chronic ulcerative colitis or Crohn disease for +8 years, having >3 alcoholic drinks per day, smoking, being black, and being obese. In the past two decades, the rate of new cases is declining, although faster than the decline in the death rate.

Colorectal cancer starts in the colon or the rectum and can also be called colon cancer or rectal cancer, depending on where it started. Nevertheless, they are often grouped together because they have many features in common. Usually, CRC starts as a growth of the inner lining of the colon or rectum, called polyps. Whether these polyps change into cancer depends on the type of polyp. Adenomas sometimes change into cancer. Hyperplastic polyps and inflammatory polyps are more common, but generally not pre-cancerous. Sessile serrated polyps (SSP) and traditional serrated adenomas (TSA) are often treated as adenomas as they have a higher risk of colorectal cancer. In addition to the type of polyp, larger size of the polyp, multiple polyps, and dysplasia present in the polyp increase the risk of developing CRC.

Most CRCs are adenocarcinomas, which start in the cells that produce the mucus to lubricate the inside of the colon and rectum. Other, less common, types are carcinoid tumors (staring in hormone producing cells), Gastro-intestinal stromal tumors (GIST), lymphomas, and sarcomas.

Local treatments are more useful in early stage cancers. Depending on the type of CRC also systemic treatment may be given. For each type and stage different treatment types may be used, or combined at the same time, or used after one another. Some main treatment types are described at American Cancer Society, Colorectal Cancer, https://www.cancer.org/cancer/colon-rectal-cancer.html, accessed 09/09/20202 and National Cancer Institute, PDQ® Adult Treatment Editorial Board, PDQ Colorectal Cancer Treatment, https://www.cancer.gov/types/colorectal/patient/colon-treatment-pdq, accessed 09/09/2020.

The main treatment for early stage colorectal cancers is surgery. The type of surgery depends on the grade of the cancer. For rectal cancer, radiation and chemotherapy are often given before or after surgery. Small metastases can be removed by embolization or ablation. This is also an option for patients whose cancer cannot be cured with surgery or who cannot have surgery. Radiation therapy is not common in colon cancer treatment but may be used in special cases, such as in (neo)adjuvant setting combined with surgery, or to treat metastases. For rectal cancer, radiation therapy is much more common and can be used before, after or during the surgery. It can also be used with or without chemotherapy in case surgery is not possible. Chemotherapy may be used at different times during treatment of colorectal cancer, in a (neo)adjuvant setting or to treat metastases. It can be given systemically, or regionally. Targeted therapies used in colorectal cancer can be targeted at: VEGF to prevent blood vessel formation, EGFR and BRAF to prevent cancer growth. Kinase inhibitors can also be used to slow cancer cell growth. Immune checkpoint inhibitors (PD-1 inhibitors, CTLA4-inhibitors) can be used for people whose colorectal cancer cells have tested positive to specific gene changes, such as microsatellite instability (MSI-H) or mismatch repair (MMR) genes. These drugs are often used when surgery is not possible, or for recurring or metastasized CRC.

Traditional classification of tumours has been refined by comprehensive molecular characterizations using large numbers of tumours. Colorectal cancers can be sub grouped by means of chromosomal instability (CIN), microsatellite instability (MSI) and hypermethylation. The cancer genome Atlas Network published a comprehensive molecular characterization of CRC in 2012 (see Cancer Genome Atlas Network, "Comprehensive molecular characterization of human colon and rectal cancer", Nature, Vol. 487, No. 7407, pages 330-337, 2012). Many studies before have reported on genes and pathways important to initiation and progression of CRC, such as WNT, RAS-MAPK, PI3K, TGF-β, P53, and DNA mismatch repair pathways. The TCGA study concluded that non-hypermutated adenocarcinomas of the colon and rectum are not distinguishable at the genomic level. Activation of the WNT signalling pathway and inactivation of TGF-β signalling pathway, resulting in decreased activity of MYC, are nearly ubiquitous in CRC. Genomic aberrations frequently target PI3K and MAPK pathways, and less frequently receptor tyrosine kinases. Later, Liu Y. et al. provided a systematic effort to characterize shared molecular processes in gastrointestinal cancers (including CRC) (Liu Y. et al., "Comparative molecular analysis of gastrointestinal adenocarcinomas", Cancer Cell, Vol. 33, No. 4, pages 721-735, 2018). Particularly in the lower GI tract, they found enriched activation of WNT signalling. Also, disruptions in TGF-β and SMAD signalling components were found, consistent with previous findings. Consideration of the molecular subtypes will be essential to study and treat CRC. For more granular details please refer to Liu Y. et al., 2018, ibid and Cancer Genome Atlas Network, 2018, ibid).

The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the treatment response, in order to increase the success rate of these therapies.

SUMMARY OF THE INVENTION

It is an objective of the invention to provide a method of predicting an outcome of a colorectal cancer subject, and to an apparatus for predicting an outcome of a colorectal cancer subject, which allow to make better treatment decisions. It is a further aspect of the invention to provide a diagnostic kit, a use of the kit, a use of the kit in a method of predicting an outcome of a colorectal cancer subject, a use of first, second, and/or third gene expression profile(s) in a method of predicting an outcome of a colorectal cancer subject, and a corresponding computer program product.

In a first aspect of the present invention, a method of predicting an outcome of a colorectal cancer subject is presented, comprising:

- determining or receiving the result of a determination of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from the subject, and/or
- determining or receiving the result of a determination of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from the subject, and/or
- determining or receiving the result of a determination of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from the subject,
- determining the prediction of the outcome based on the first gene expression profile(s), or on the second gene expression profile(s), or on the third gene expression profile(s), or on the first, second, and third gene expression profile(s), and
- optionally, providing the prediction to a medical caregiver or the subject.

In recent years, the importance of the immune system in cancer inhibition as well as in cancer initiation, promotion and metastasis has become very evident (see Mantovani A. et al., "Cancer-related inflammation", Nature, Vol. 454, No. 7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated.

The present invention is based on the idea that, since the status of the immune system and of the immune microenvironment have an impact on therapy effectiveness, the ability to identify markers predictive for this effect might help to be better able to predict the outcome of a colorectal cancer subject.

Herein, the term "outcome" relates to a specific result or effect that can be measured. Examples of an outcome of a colorectal cancer subject include an outcome of the colorectal cancer, a pathology outcome, an outcome of a therapy for treating the colorectal cancer, such as a surgery outcome, a radiation therapy outcome, a chemotherapy outcome, and an immunotherapy outcome, as well as other outcomes, such as a biomarker related outcome (e.g., CEA (*Carcinoembryonic Antigen*)), a genomic profile related outcome, an imaging related outcome (e.g., a change in morphology or texture of the tumor), a biology related outcome (e.g., inflammation or immune response), a surrogate marker related outcome, a tumor size outcome, a treatment side effect outcome, a treatment toxicity outcome, a disease pain outcome, a quality of life outcome, a cancer specific survival, and an overall survival.

**Immune response defense genes**

The integrity and stability of genomics DNA is permanently under stress induced by various cell internal and external factors like exposure to radiation, viral or bacterial infections, but also oxidation and replication stress (see Gasser S. et al., "Sensing of dangerous DNA", Mechanisms of Aging and Development, Vol. 165, pages 33-46, 2017). In order to maintain DNA structure and stability, a cell must be able to recognize all types of DNA damages like single or double strand breaks etc. induced by various factors. This process involves the participation of a multitude of specific proteins depending on the kind of damage as part of DNA recognition pathways.

Recent evidence suggests that mis-localized DNA (e.g., DNA unnaturally appearing in the cytosolic fraction of the cell in contrast to the nucleus) and damaged DNA (e.g., through mutations occurring in cancer development) is used by the immune system to identify infected or otherwise diseased cells while genomic and mitochondrial DNA present in healthy cells is ignored by DNA recognition pathways. In diseased cells, cytosolic DNA sensor proteins have been demonstrated to be involved in the detection of DNA occurring unnaturally in the cytosol of the cell. Detection of such DNA by different nucleic acid sensors translates into similar responses leading to nuclear factor kappa-B (NF-kB) and interferon type I (IFN type I) signalling followed by the activation of innate immune system components. While the recognition of viral DNA is known to induce an IFN type I response, evidence that sensing of DNA damage can initiate immune responses has only recently been accumulating.

TLR9 (Toll-like receptor 9) located in the endosomes was one of the first DNA sensors molecules identified to be involved in the immune recognition of DNA by signalling downstream via the adaptor protein myeloid differentiation primary-response protein88 (MYD88). This interaction in turn activates mitogen-activated protein kinases (MAPKs) and NF-kB. TLR9 also induces the generation of type I interferons through the activation of IRF7 via IkB Kinase alpha (IKKalpha) in plasmacytoid dendritic cells (pDCs). Various other DNA immune receptors including IFI16 (IFN-gamma-inducible protein 16), cGAS (cyclic DMP-AMP synthase, DDX41 (DEAD-box helicase 41), as well as ZBP1 (Z-DNA-binding protein 1) interact with STING (stimulator of IFN genes), which activates the IKK complex and IRF3 through TBK1 (TANK binding kinase 1). ZBP1 also activates NF-kB via recruitment of RIP1 and RIP3 (receptor-interacting protein 1 and 3, respectively). While the helicase DHX36 (DEAH-box helicase 36) interacts in a complex with TRID to induce NF-kB and IRF-3/7 the DHX9 helicase stimulates MYD88-dependent signalling in plasmacytoid dendritic cells. The DNA sensor LRRFIP1 (leucine-rich repeat flightless-interacting protein) complexes with beta-catenin to activate the transcription of IRF3 whereas AIM2 (absent in melanoma 2) recruits the adaptor protein ASC (apoptosis speck-like protein) to induce a caspase-1-activating inflammasome complex leading to the secretion of interleukin-lbeta (IL-1beta) and IL-18 (see Fig. 1 of Gasser S. et al., 2017, ibid, which provides a schematic overview of DNA damage and DNA sensor pathways leading to the production of inflammatory cytokines and the expression of ligands for activating innate immune receptors. Members of the non-homologous end joining pathway (orange), homologous recombination (red), inflammasome (dark green), NF- kB and interferon responses (light green) are shown).

The factors and mechanisms responsible for activating the DNA sensor pathways in cancer are currently not well elucidated. It will be important to identify the intratumoral DNA species, sensors and pathways implicated in the expression of IFNs in different cancer types at all stages of the disease. In addition to therapeutic targets in cancer, such factors may also have prognostic and predictive value. Novel DNA sensor pathway agonists and antagonists are currently being developed and tested in preclinical trials. Such compounds will be useful in characterizing the role of DNA sensor pathways in the pathogenesis of cancer, autoimmunity and potentially other diseases.

**T-Cell receptor signaling genes**

An immune response against pathogens can be elicited at different levels: there are physical barriers, such as the skin, to keep invaders out. If breached, innate immunity comes into play; a first and fast non-specific response. If this is not sufficient, the adaptive immune response is elicited. This is much more specific and needs time to develop when encountering a pathogen for the first time. Lymphocytes are activated by interacting with activated antigen presenting cells from the innate immune system, and are also responsible for maintenance of memory for faster responses upon next encounters with the same pathogen.

As lymphocytes are highly specific and effective when activated, they are subject to negative selection for their ability to recognize self, a process known as central tolerance. As not all self-antigens are expressed at selection sites, peripheral tolerance mechanisms evolved as well, such as ligation of the TCR in absence of co-stimulation, expression of inhibitory co-receptors, and suppression by Tregs. A disturbed balance between activation and suppression may lead to autoimmune disorders, or immune deficiencies and cancer, respectively.

T-cell activation can have different functional consequences, depending on the location the type of T-cell involved. CD8+ T-cells differentiate into cytotoxic effector cells, whereas CD4+ T-cells can differentiate into Th1 (IFN$\gamma$ secretion and promotion of cell mediated immunity) or Th2 (IL4/5/13 secretion and promotion of B cell and humoral immunity). Differentiation towards other, more recently identified T-cell subsets is also possible, for example the Tregs, which have a suppressive effect on immune activation (see Mosenden R. and Tasken K., "Cyclic AMP-mediated immune regulation - Overview of mechanisms of action in T-cells", Cell Signal, Vol. 23, No. 6, pages 1009-1016, 2011, in particular, Fig. 4, which T-cell activation and its modulation by PKA, and Tasken K. and Ruppelt A., "Negative regulation of T-cell receptor activation by the cAMP-PKA-Csk signaling pathway in T-cell lipid rafts", Front Biosci, Vol. 11, pages 2929-2939, 2006).

Both PKA and PDE4 regulated signaling intersect with TCR induced T-cell activation to fine-tune its regulation, with opposing effects (see Abrahamsen H. et al., "TCR- and CD28-mediated recruitment of phosphodiesterase 4 to lipid rafts potentiates TCR signaling", J Immunol, Vol. 173, pages 4847-4848, 2004, in particular, Fig. 6, which shows opposing effects of PKA and PDE4 on TCR activation). The molecule that connects these effectors is cyclic AMP (cAMP), an intracellular second messenger of extracellular ligand action. In T-cells, it mediates effects of prostaglandins, adenosine, histamine, beta-adrenergic agonists, neuropeptide hormones and beta-endorphin. Binding of these extracellular molecules to GPCRs leads to their conformational change, release of stimulatory subunits and subsequent activation of adenylate cyclases (AC), which hydrolyze ATP to cAMP (see Fig. 6 of Abrahamsen H. et al., 2004, ibid). Although not the only one, PKA is the principal effector of cAMP signaling (see Mosenden R. and Tasken K., 2011, ibid, and Tasken K. and Ruppelt A., 2006, ibid). At a functional level, increased levels of cAMP lead to reduced IPN$\gamma$ and IL-2 production in T-cells (see Abrahamsen H. et al., 2004, ibid). Aside from interfering with TCR activation, PKA has many more effector (see Fig. 15 of Torheim E.A., "Immunity Leashed - Mechanisms of Regulation in the Human Immune System", Thesis for the degree of Philosophiae Doctor (PhD), The Biotechnology Centre of Ola, University of Oslo, Norway, 2009).

In naive T-cells, hyperphosphorylated PAG targets Csk to lipid rafts. Via the Ezrin-EBP50-PAG scaffold complex PKA is targeted to Csk. Through specific phosphorylation by PKA, Csk can negatively regulate Lck and Fyn to dampen their activity and downregulate T-cell activation (see Fig. 6 of Abrahamsen H. et al., 2004, ibid). Upon TCR activation, PAG is dephosphorylated and Csk is released from the rafts. Dissociation of Csk is needed for T-cell activation to proceed. Within the same time course, a Csk-G3BP complex is formed and seems to sequester Csk outside lipid rafts (see Mosenden R. and Tasken K., 2011, ibid, and Tasken K. and Ruppelt A., 2006, ibid).

In contrast, combined TCR and CD28 stimulation mediates recruitment of the cyclic nucleotide phosphodiesterase PDE4 to lipid rafts, which enhances cAMP degradation (see Fig. 6 of Abrahamsen H. et al., 2004, ibid). As such, TCR induced production of cAMP is countered, and the T-cell immune response potentiated. Upon TCR stimulation alone, PDE4 recruitment may be too low to fully reduce the cAMP levels and therefore maximal T-cell activation cannot occur (see Abrahamsen H. et al., 2004, ibid).

Thus, by active suppression of proximal TCR signaling, signaling via cAMP-PKA-Csk is thought to set the threshold for T-cell activation. Recruitment of PDEs can counter this suppression. Tissue or cell-type specific regulation is accomplished through expression of multiple isoforms of AC, PKA, and PDEs. As mentioned above, the balance between activation and suppression needs to be tightly regulated to prevent development of autoimmune disorders, immune deficiencies and cancer.

**PDE4D7 correlated genes**

Phosphodiesterases (PDEs) provide the sole means for the degradation of the second messenger 3'-5'-cyclic AMP. As such they are poised to provide a key regulatory role. Thus, aberrant changes in their expression, activity and intracellular location may all contribute to the underlying molecular pathology of particular disease states. Indeed, it has recently been shown that mutations in PDE genes are enriched in prostate cancer patients leading to elevated cAMP signalling and a potential predisposition to prostate cancer. However, varied expression profiles in different cell types coupled with complex arrays of isoform variants within each PDE family makes understanding the links between aberrant changes in PDE expression and functionality during disease progression challenging. Several studies have endeavored to describe the complement of PDEs in prostate, all of which identified significant levels of PDE4 expression alongside other PDEs, leading to the development of a PDE4D7 biomarker (see Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018). Since the PDE4D7 biomarker has been proven to be a good predictor, we assumed that the ability to identify markers that are highly correlated with the PDE47 biomarker might also be helpful in prognosticating the outcome of certain cancer subjects.

**Selection of the genes**

The lists of genes have originally been selected by us for prognostication in prostate cancer subjects. In this document, it is shown that they are also of prognostic value with respect to an outcome of a colorectal cancer subject.

The identified immune defense response genes ZBP1, and AIM2, APOBEC3A, CIAO1, DDX58, DHX9, IFI16, IFIH1, IFIT1, IFIT3, LRRFIP1, MYD88, OAS1, TLR8, and ZBP1, respectively, were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #2 demonstrated enrichment (enrichment score: 10.8) in 30 genes with a function in defense response to viruses, negative regulation of viral genome replication as well as type I interferon signaling. A further heat map analysis confirmed that these immune defense response genes were generally higher expressed in samples from patients in PDE4D7 score class 1 than from patients in PDE4D7 score class 4. The class of genes with a function in defense response to viruses, negative regulation of viral genome replication as well as type I interferon signaling was further enriched to 61 genes by literature search to identify additional genes with the same molecular function. A further selection from the 61 genes was made based on the combinatorial power to separate patients who died from prostate cancer vs. those who did not, resulting of a preferred set of 14 genes. It was found that the number of events (metastases, prostate cancer specific death) was enriched in sub-cohorts with a low expression of these genes compared to the total patient cohort (#538) and a sub-cohort of 151 patients undergoing salvage RT (SRT) after post-surgical disease recurrence.

The identified T-Cell receptor signaling genes CD2, CD247, CD28, CD3E, CD3G, CD4, CSK, EZR, FYN, LAT, LCK, PAG1, PDE4D, PRKACA, PRKACB, PTPRC, and ZAP70 were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #6 demonstrated enrichment (enrichment score: 5.9) in 17 genes with a function in primary immune deficiency and activation of T-Cell receptor signaling. A further heat map analysis confirmed that these T-Cell receptor signaling genes were generally higher expressed in samples from patients in PDE4D7 score class 1 than from patients in PDE4D7 score class 4.

The identified PDE4D7 correlated genes ABCC5, CUX2, KIAA1549, PDE4D, RAP1GAP2, SLC39A11, TDRD1, and VWA2 were identified as follows: We have identified in RNAseq data generated on 571 prostate cancer patients on close to 60,000 transcripts a range of genes that are correlated to the expression of the known biomarker PDE4D7 in this data. The correlation between the expression of any of these genes and PDE4D7 across the 571 samples was done by Pearson correlation and is expressed as a value between 0 to 1 in case of positive correlation or a value between -1 to 0 in case of negative correlation. As input data for the calculation of the correlation coefficient we used the PDE4D7 score (see Alves de Inda M. et al., 2018, ibid) and the RNAseq determined TPM gene expression value per gene of interest (see below).

The maximum negative correlation coefficient identified between the expression of any of the approximately 60,000 transcripts and the expression of PDE4D7 was -0.38 while the maximum positive correlation coefficient identified between the expression of any of the approximately 60,000 transcripts and the expression of PDE4D7 was +0.56. We selected genes in the range of correlation -0.31 to -0.38 as well as +0.41 to +0.56. We identified in total 77 transcripts matching these characteristics. From those 77 transcripts we selected the eight PDE4D7 correlated genes ABCC5, CUX2, KIAA1549, PDE4D, RAP1GAP2, SLC39A11, TDRD1, and VWA2 by testing Cox regression combination models iteratively in a sub-cohort of 186 patients who were undergoing salvage radiation treatment (SRT) due to post-surgical biochemical relapse. The clinical endpoint tested was prostate cancer specific death after start of SRT. The boundary condition for the selection of the eight genes was given by the restriction that the p-values in the multivariate Cox-regression were <0.1 for all genes retained in the model.

The term "ABCC5" refers to the human ATP binding cassette subfamily C member 5 gene (Ensembl: ENSG00000114770), for example, to the sequence as defined in NCBI Reference Sequence NM_001023587.2 or in NCBI Reference Sequence NM_005688.3, specifically, to the nucleotide sequence as set forth in SEQ ID NO:1 or in SEQ ID NO:2, which correspond to the sequences of the above indicated NCBI Reference Sequences of the ABCC5 transcript, and also relates to the corresponding amino acid sequence for example as set forth in SEQ ID NO:3 or in SEQ ID NO:4, which correspond to the protein sequences defined in NCBI Protein Accession Reference Sequence NP_001018881.1 and in NCBI Protein Accession Reference Sequence NP_005679 encoding the ABCC5 polypeptide.

The term "ABCC5" also comprises nucleotide sequences showing a high degree of homology to ABCC5, e.g., nucleic acid sequences being at least 75%, 80%, 85%, 90%, 91%, 92%, 93%, 94%, 95%, 96%, 97%, 98% or 99% identical to the sequence as set forth in SEQ ID NO:1 or in SEQ ID NO:2 or amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1 or in SEQ ID NO:2.

The term "AIM2" refers to the Absent in Melanoma 2 gene (Ensembl: ENSG00000163568), for example, to the sequence as defined in NCBI Reference Sequence NM_004833, specifically, to the nucleotide sequence as set forth in SEQ ID NO:5, which corresponds to the sequence of the above indicated NCBI Reference Sequence of the AIM2 transcript, and also relates to the corresponding amino acid sequence for example as set forth in SEQ ID NO:6, which corresponds to the protein sequence defined in NCBI Protein Accession Reference Sequence NP_004824 encoding the AIM2 polypeptide.

The term "AIM2" also comprises nucleotide sequences showing a high degree of homology to AIM2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "APOBEC3A" refers to the Apolipoprotein B mRNA Editing Enzyme Catalytic Subunit 3A gene (Ensembl: ENSG00000128383), for example, to the sequence as defined in NCBI Reference Sequence NM_145699, specifically, to the nucleotide sequence as set forth in SEQ ID NO:7, which corresponds to the sequence of the above indicated NCBI Reference Sequence of the APOBEC3A transcript, and also relates to the corresponding amino acid sequence for example as set forth in SEQ ID NO:8, which corresponds to the protein sequence defined in NCBI Protein Accession Reference Sequence NP_663745 encoding the APOBEC3A polypeptide.

The term "APOBEC3A" also comprises nucleotide sequences showing a high degree of homology to APOBEC3A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "CD2" refers to the Cluster Of Differentiation 2 gene (Ensembl: ENSG00000116824), for example, to the sequence as defined in NCBI Reference Sequence NM_001767, specifically, to the nucleotide sequence as set forth in SEQ ID NO:9, which corresponds to the sequence of the above indicated NCBI Reference Sequence of the CD2 transcript, and also relates to the corresponding amino acid sequence for example as set forth in SEQ ID NO:10, which corresponds to the protein sequence defined in NCBI Protein Accession Reference Sequence NP_001758 encoding the CD2 polypeptide.

The term "CD2" also comprises nucleotide sequences showing a high degree of homology to CD2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO: 10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "CD247" refers to the Cluster Of Differentiation 247 gene (Ensembl: ENSG00000198821), for example, to the sequence as defined in NCBI Reference Sequence NM_000734 or in NCBI Reference Sequence NM_198053, specifically, to the nucleotide sequence as set forth in SEQ ID NO:11 or in SEQ ID NO: 12, which correspond to the sequences of the above indicated NCBI Reference Sequences of the CD247 transcript, and also relates to the corresponding amino acid sequence for example as set forth in SEQ ID NO: 13 or in SEQ ID NO:14, which correspond to the protein sequences defined in NCBI Protein Accession Reference Sequence NP_000725 and in NCBI Protein Accession Reference Sequence NP_932170 encoding the CD247 polypeptide.

The term "CD247" also comprises nucleotide sequences showing a high degree of homology to CD247, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO: 12 or amino acid sequences being at least 75%, 80%, 85%, 90%, 91%, 92%, 93%, 94%, 95%, 96%, 97%, 98% or 99% identical to the sequence as set forth in SEQ ID NO: 13 or in SEQ ID NO:14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 13 or in SEQ ID NO: 14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO: 12.

The term "CD28" refers to the Cluster Of Differentiation 28 gene (Ensembl: ENSG00000178562), for example, to the sequence as defined in NCBI Reference Sequence NM_006139 or in NCBI Reference Sequence NM_001243078, specifically, to the nucleotide sequence as set forth in SEQ ID NO: 15 or in SEQ ID NO: 16, which correspond to the sequences of the above indicated NCBI Reference Sequences of the CD28 transcript, and also relates to the corresponding amino acid sequence for example as set forth in SEQ ID NO: 17 or in SEQ ID NO: 18, which correspond to the protein sequences defined in NCBI Protein Accession Reference Sequence NP_006130 and in NCBI Protein Accession Reference Sequence NP_001230007 encoding the CD28 polypeptide.

The term "CD28" also comprises nucleotide sequences showing a high degree of homology to CD28, e.g., nucleic acid sequences being at least 75%, 80%, 85%, 90%, 91%, 92%, 93%, 94%, 95%, 96%, 97%, 98% or 99% identical to the sequence as set forth in SEQ ID NO:15 or in SEQ ID NO:16 or amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:17 or in SEQ ID NO:18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:15 or in SEQ ID NO:16.

The term "CD3E" refers to the Cluster Of Differentiation 3E gene (Ensembl: ENSG00000198851), for example, to the sequence as defined in NCBI Reference Sequence NM_000733, specifically, to the nucleotide sequence as set forth in SEQ ID NO: 19, which corresponds to the sequence of the above indicated NCBI Reference Sequence of the CD3E transcript, and also relates to the corresponding amino acid sequence for example as set forth in SEQ ID NO:20, which corresponds to the protein sequence defined in NCBI Protein Accession Reference Sequence NP_000724 encoding the CD3E polypeptide.

The term "CD3E" also comprises nucleotide sequences showing a high degree of homology to CD3E, e.g., nucleic acid sequences being at least 75%, 80%, 85%, 90%, 91%, 92%, 93%, 94%, 95%, 96%, 97%, 98% or 99% identical to the sequence as set forth in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 19.

The term "CD3G" refers to the Cluster Of Differentiation 3G gene (Ensembl: ENSG00000160654), for example, to the sequence as defined in NCBI Reference Sequence NM_000073, specifically, to the nucleotide sequence as set forth in SEQ ID NO:21, which corresponds to the sequence of the above indicated NCBI Reference Sequence of the CD3G transcript, and also relates to the corresponding amino acid sequence for example as set forth in SEQ ID NO:22, which corresponds to the protein sequence defined in NCBI Protein Accession Reference Sequence NP_000064 encoding the CD3G polypeptide.

The term "CD3G" also comprises nucleotide sequences showing a high degree of homology to CD3G, e.g., nucleic acid sequences being at least 75%, 80%, 85%, 90%, 91%, 92%, 93%, 94%, 95%, 96%, 97%, 98% or 99% identical to the sequence as set forth in SEQ ID NO:21 or amino acid sequences being at least 75%, 80%, 85%, 90%, 91%, 92%, 93%, 94%, 95%, 96%, 97%, 98% or 99% identical to the sequence as set forth in SEQ ID NO:22 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:22 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21.

The term "CD4" refers to the Cluster Of Differentiation 4 gene (Ensembl: ENSG00000010610), for example, to the sequence as defined in NCBI Reference Sequence NM_000616, specifically, to the nucleotide sequence as set forth in SEQ ID NO:23, which corresponds to the sequence of the above indicated NCBI Reference Sequence of the CD4 transcript, and also relates to the corresponding amino acid sequence for example as set forth in SEQ ID NO:24, which corresponds to the protein sequence defined in NCBI Protein Accession Reference Sequence NP_000607 encoding the CD4 polypeptide.

The term "CD4" also comprises nucleotide sequences showing a high degree of homology to CD4, e.g., nucleic acid sequences being at least 75%, 80%, 85%, 90%, 91%, 92%, 93%, 94%, 95%, 96%, 97%, 98% or 99% identical to the sequence as set forth in SEQ ID NO:23 or amino acid sequences being at least 75%, 80%, 85%, 90%, 91%, 92%, 93%, 94%, 95%, 96%, 97%, 98% or 99% identical to the sequence as set forth in SEQ ID NO:24 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:24 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:23.

The term "CIAO1" refers to the Cytosolic Iron-Sulfur Assembly Component 1 gene (Ensembl: ENSG00000144021), for example, to the sequence as defined in NCBI Reference Sequence NM_004804, specifically, to the nucleotide sequence as set forth in SEQ ID NO:25, which corresponds to the sequence of the above indicated NCBI Reference Sequence of the CIAO1 transcript, and also relates to the corresponding amino acid sequence for example as set forth in SEQ ID NO:26, which corresponds to the protein sequence defined in NCBI Protein Accession Reference Sequence NP_663745 encoding the CIAO1 polypeptide.

The term "CIAO1" also comprises nucleotide sequences showing a high degree of homology to CIAO1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:25 or amino acid sequences being at least 75%, 80%, 85%, 90%, 91%, 92%, 93%, 94%, 95%, 96%, 97%, 98% or 99% identical to the sequence as set forth in SEQ ID NO:26 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:26 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:25.

The term "CSK" refers to the C-Terminal Src Kinase gene (Ensembl: ENSG00000103653), for example, to the sequence as defined in NCBI Reference Sequence NM_004383, specifically, to the nucleotide sequence as set forth in SEQ ID NO:27, which corresponds to the sequence of the above indicated NCBI Reference Sequence of the CSK transcript, and also relates to the corresponding amino acid sequence for example as set forth in SEQ ID NO:28, which corresponds to the protein sequence defined in NCBI Protein Accession Reference Sequence NP_004374 encoding the CSK polypeptide.

The term "CSK" also comprises nucleotide sequences showing a high degree of homology to CSK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:27 or amino acid sequences being at least 75%, 80%, 85%, 90%, 91%, 92%, 93%, 94%, 95%, 96%, 97%, 98% or 99% identical to the sequence as set forth in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:27.

The term "CUX2" refers to the human Cut Like Homeobox 2 gene (Ensembl: ENSG00000111249), for example, to the sequence as defined in NCBI Reference Sequence NM_015267.3, specifically, to the nucleotide sequence as set forth in SEQ ID NO:29, which corresponds to the sequence of the above indicated NCBI Reference Sequence of the CUX2 transcript, and also relates to the corresponding amino acid sequence for example as set forth in SEQ ID NO:30, which corresponds to the protein sequence defined in NCBI Protein Accession Reference Sequence NP_056082.2 encoding the CUX2 polypeptide.

The term "CUX2" also comprises nucleotide sequences showing a high degree of homology to CUX2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or amino acid sequences being at least 75%, 80%, 85%, 90%, 91%, 92%, 93%, 94%, 95%, 96%, 97%, 98% or 99% identical to the sequence as set forth in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29.

The term "DDX58" refers to the DExD/H-box Helicase 58 gene (Ensembl: ENSG00000107201), for example, to the sequence as defined in NCBI Reference Sequence NM_014314, specifically, to the nucleotide sequence as set forth in SEQ ID NO:31, which corresponds to the sequence of the above indicated NCBI Reference Sequence of the DDX58 transcript, and also relates to the corresponding amino acid sequence for example as set forth in SEQ ID NO:32, which corresponds to the protein sequence defined in NCBI Protein Accession Reference Sequence NP_055129 encoding the DDX58 polypeptide.

The term "DDX58" also comprises nucleotide sequences showing a high degree of homology to DDX58, e.g., nucleic acid sequences being at least 75%, 80%, 85%, 90%, 91%, 92%, 93%, 94%, 95%, 96%, 97%, 98% or 99% identical to the sequence as set forth in SEQ ID NO:31 or amino acid sequences being at least 75%, 80%, 85%, 90%, 91%, 92%, 93%, 94%, 95%, 96%, 97%, 98% or 99% identical to the sequence as set forth in SEQ ID NO:32 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:31.

The term "DHX9" refers to the DExD/H-box Helicase 9 gene (Ensembl: ENSG00000135829), for example, to the sequence as defined in NCBI Reference Sequence NM_001357, specifically, to the nucleotide sequence as set forth in SEQ ID NO:33, which corresponds to the sequence of the above indicated NCBI Reference Sequence of the DHX9 transcript, and also relates to the corresponding amino acid sequence for example as set forth in SEQ ID NO:34, which corresponds to the protein sequence defined in NCBI Protein Accession Reference Sequence NP_001348 encoding the DHX9 polypeptide.

The term "DHX9" also comprises nucleotide sequences showing a high degree of homology to DHX9, e.g., nucleic acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:33.

The term "EZR" refers to the Ezrin gene (Ensembl: ENSG00000092820), for example, to the sequence as defined in NCBI Reference Sequence NM_003379, specifically, to the nucleotide sequence as set forth in SEQ ID NO:35, which corresponds to the sequence of the above indicated NCBI Reference Sequence of the EZR transcript, and also relates to the corresponding amino acid sequence for example as set forth in SEQ ID NO:36, which corresponds to the protein sequence defined in NCBI Protein Accession Reference Sequence NP_003370 encoding the EZR polypeptide.

The term "EZR" also comprises nucleotide sequences showing a high degree of homology to EZR, e.g., nucleic acid sequences being at least 75%, 80%, 85%, 90%, 91%, 92%, 93%, 94%, 95%, 96%, 97%, 98% or 99% identical to the sequence as set forth in SEQ ID NO:35 or amino acid sequences being at least 75%, 80%, 85%, 90%, 91%, 92%, 93%, 94%, 95%, 96%, 97%, 98% or 99% identical to the sequence as set forth in SEQ ID NO:36 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:36 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:35.

The term "FYN" refers to the FYN Proto-Oncogene gene (Ensembl: ENSG00000010810), for example, to the sequence as defined in NCBI Reference Sequence NM_002037 or in NCBI Reference Sequence NM_153047 or in NCBI Reference Sequence NM_153048, specifically, to the nucleotide sequence as set forth in SEQ ID NO:37 or in SEQ ID NO:38 or in SEQ ID NO:39, which correspond to the sequences of the above indicated NCBI Reference Sequences of the FYN transcript, and also relates to the corresponding amino acid sequence for example as set forth in SEQ ID NO:40 or in SEQ ID NO:41 or in SEQ ID NO:42, which correspond to the protein sequences defined in NCBI Protein Accession Reference Sequence NP_002028 and in NCBI Protein Accession Reference Sequence NP_694592 and in NCBI Protein Accession Reference Sequence XP_005266949 encoding the FYN polypeptide.

The term "FYN" also comprises nucleotide sequences showing a high degree of homology to FYN, e.g., nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39 or amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39.

The term "IFI16" refers to the Interferon Gamma Inducible Protein 16 gene (Ensembl: ENSG00000163565), for example, to the sequence as defined in NCBI Reference Sequence NM_005531, specifically, to the nucleotide sequence as set forth in SEQ ID NO:43, which corresponds to the sequence of the above indicated NCBI Reference Sequence of the IFI16 transcript, and also relates to the corresponding amino acid sequence for example as set forth in SEQ ID NO:44, which corresponds to the protein sequence defined in NCBI Protein Accession Reference Sequence NP_005522 encoding the IFI16 polypeptide.

The term "IFI16" also comprises nucleotide sequences showing a high degree of homology to IFI16, e.g., nucleic acid sequences being at least 75%, 80%, 85%, 90%, 91%, 92%, 93%, 94%, 95%, 96%, 97%, 98% or 99% identical to the sequence as set forth in SEQ ID NO:43 or amino acid sequences being at least 75%, 80%, 85%, 90%, 91%, 92%, 93%, 94%, 95%, 96%, 97%, 98% or 99% identical to the sequence as set forth in SEQ ID NO:44 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:44 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:43.

The term "IFIH1" refers to the Interferon Induced With Helicase C Domain 1 gene (Ensembl: ENSG00000115267), for example, to the sequence as defined in NCBI Reference Sequence NM_022168, specifically, to the nucleotide sequence as set forth in SEQ ID NO:45, which corresponds to the sequence of the above indicated NCBI Reference Sequence of the IFIH1 transcript, and also relates to the corresponding amino acid sequence for example as set forth in SEQ ID NO:46, which corresponds to the protein sequence defined in NCBI Protein Accession Reference Sequence NP_071451 encoding the IFIH1 polypeptide.

The term "IFIH1" also comprises nucleotide sequences showing a high degree of homology to IFIH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:45 or amino acid sequences being at least 75%, 80%, 85%, 90%, 91%, 92%, 93%, 94%, 95%, 96%, 97%, 98% or 99% identical to the sequence as set forth in SEQ ID NO:46 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:46 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:45.

The term "IFIT1" refers to the Interferon Induced Protein With Tetratricopeptide Repeats 1 gene (Ensembl: ENSG00000185745), for example, to the sequence as defined in NCBI Reference Sequence NM_001270929 or in NCBI Reference Sequence NM_001548.5, specifically, to the nucleotide sequence as set forth in SEQ ID NO:47 or in SEQ ID NO:48, which correspond to the sequences of the above indicated NCBI Reference Sequences of the IFIT1 transcript, and also relates to the corresponding amino acid sequence for example as set forth in SEQ ID NO:49 or in SEQ ID NO:50, which correspond to the protein sequences defined in NCBI Protein Accession Reference Sequence NP_001257858 and in NCBI Protein Accession Reference Sequence NP_001539 encoding the IFIT1 polypeptide.

The term "IFIT1" also comprises nucleotide sequences showing a high degree of homology to IFIT1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48 or amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:49 or SEQ ID NO:50 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48.

The term "IFIT3" refers to the Interferon Induced Protein With Tetratricopeptide Repeats 3 gene (Ensembl: ENSG00000119917), for example, to the sequence as defined in NCBI Reference Sequence NM_001031683, specifically, to the nucleotide sequence as set forth in SEQ ID NO:51, which corresponds to the sequence of the above indicated NCBI Reference Sequence of the IFIT3 transcript, and also relates to the corresponding amino acid sequence for example as set forth in SEQ ID NO:52, which corresponds to the protein sequence defined in NCBI Protein Accession Reference Sequence NP_001026853 encoding the IFIT3 polypeptide.

The term "IFIT3" also comprises nucleotide sequences showing a high degree of homology to IFIT3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:51 or amino acid sequences being at least 75%, 80%, 85%, 90%, 91%, 92%, 93%, 94%, 95%, 96%, 97%, 98% or 99% identical to the sequence as set forth in SEQ ID NO:52 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:52 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:51.

The term "KIAA1549" refers to the human KIAA1549 gene (Ensembl: ENSG00000122778), for example, to the sequence as defined in NCBI Reference Sequence NM_020910 or in NCBI Reference Sequence NM_001164665, specifically, to the nucleotide sequence as set forth in SEQ ID NO:53 or in SEQ ID NO:54, which correspond to the sequences of the above indicated NCBI Reference Sequence of the KIAA1549 transcript, and also relates to the corresponding amino acid sequence for example as set forth in SEQ ID NO:55 or in SEQ ID NO:56, which correspond to the protein sequences defined in NCBI Protein Accession Reference Sequence NP_065961 and in NCBI Protein Accession Reference Sequence NP_001158137 encoding the KIAA1549 polypeptide.

The term "KIAA1549" also comprises nucleotide sequences showing a high degree of homology to KIAA1549, e.g., nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54 or amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54.

The term "LAT" refers to the Linker For Activation Of T-Cells gene (Ensembl: ENSG00000213658), for example, to the sequence as defined in NCBI Reference Sequence NM_001014987 or in NCBI Reference Sequence NM_014387, specifically, to the nucleotide sequence as set forth in SEQ ID NO:57 or in SEQ ID NO:58, which correspond to the sequences of the above indicated NCBI Reference Sequences of the LAT transcript, and also relates to the corresponding amino acid sequence for example as set forth in SEQ ID NO:59 or in SEQ ID NO:60, which corresponds to the protein sequences defined in NCBI Protein Accession Reference Sequence NP_001014987 and in NCBI Protein Accession Reference Sequence NP_055202 encoding the LAT polypeptide.

The term "LAT" also comprises nucleotide sequences showing a high degree of homology to LAT, e.g., nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58 or amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58.

The term "LCK" refers to the LCK Proto-Oncogene gene (Ensembl: ENSG00000182866), for example, to the sequence as defined in NCBI Reference Sequence NM_005356, specifically, to the nucleotide sequence as set forth in SEQ ID NO:61, which corresponds to the sequence of the above indicated NCBI Reference Sequence of the LCK transcript, and also relates to the corresponding amino acid sequence for example as set forth in SEQ ID NO:62, which corresponds to the protein sequence defined in NCBI Protein Accession Reference Sequence NP_005347 encoding the LCK polypeptide.

The term "LCK" also comprises nucleotide sequences showing a high degree of homology to LCK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:61 or amino acid sequences being at least 75%, 80%, 85%, 90%, 91%, 92%, 93%, 94%, 95%, 96%, 97%, 98% or 99% identical to the sequence as set forth in SEQ ID NO:62 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:62 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:61.

The term "LRRFIP1" refers to the LRR Binding FLII Interacting Protein 1 gene (Ensembl: ENSG00000124831), for example, to the sequence as defined in NCBI Reference Sequence NM_004735 or in NCBI Reference Sequence NM_001137550 or in NCBI Reference Sequence NM_001137553 or in NCBI Reference Sequence NM_001137552, specifically, to the nucleotide sequence as set forth in SEQ ID NO:63 or in SEQ ID NO:64 or in SEQ ID NO:65 or in SEQ ID NO:66, which correspond to the sequences of the above indicated NCBI Reference Sequences of the LRRFIP1 transcript, and also relates to the corresponding amino acid sequence for example as set forth in SEQ ID NO:67 or in SEQ ID NO:68 or in SEQ ID NO:69 or in SEQ ID NO:70, which correspond to the protein sequences defined in NCBI Protein Accession Reference Sequence NP_004726 and in NCBI Protein Accession Reference Sequence NP_001131022 and in NCBI Protein Accession Reference Sequence NP_001131025 and in NCBI Protein Accession Reference Sequence NP_001131024 encoding the LRRFIP1 polypeptide.

The term "LRRFIP1" also comprises nucleotide sequences showing a high degree of homology to LRRFIP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66 or amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66.

The term "MYD88" refers to the MYD88 Innate Immune Signal Transduction Adaptor gene (Ensembl: ENSG00000172936), for example, to the sequence as defined in NCBI Reference Sequence NM_001172567 or in NCBI Reference Sequence NM_001172568 or in NCBI Reference Sequence NM_001172569 or in NCBI Reference Sequence NM_001172566 or in NCBI Reference Sequence NM_002468, specifically, to the nucleotide sequences as set forth in SEQ ID NO:71 or in SEQ ID NO:72 or in SEQ ID NO:73 or in SEQ ID NO:74 or in SEQ ID NO:75, which correspond to the sequences of the above indicated NCBI Reference Sequences of the MYD88 transcript, and also relates to the corresponding amino acid sequence for example as set forth in SEQ ID NO:76 or in SEQ ID NO:77 or in SEQ ID NO:78 or in SEQ ID NO:79 or in SEQ ID NO:80, which correspond to the protein sequences defined in NCBI Protein Accession Reference Sequence NP_001166038 and in NCBI Protein Accession Reference Sequence NP_001166039 and in NCBI Protein Accession Reference Sequence NP_001166040 and in NCBI Protein Accession Reference Sequence NP_001166037 and in NCBI Protein Accession Reference Sequence NP_002459 encoding the MYD88 polypeptide.

The term "MYD88" also comprises nucleotide sequences showing a high degree of homology to MYD88, e.g., nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75 or amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75.

The term "OAS1" refers to the 2'-5'-Oligoadenylate Synthetase 1 gene (Ensembl: ENSG00000089127), for example, to the sequence as defined in NCBI Reference Sequence NM_001320151 or in NCBI Reference Sequence NM_002534 or in NCBI Reference Sequence NM_001032409 or in NCBI Reference Sequence NM_016816, specifically, to the nucleotide sequences as set forth in SEQ ID NO:81 or in SEQ ID NO:82 or in SEQ ID NO:83 or in SEQ ID NO:84, which correspond to the sequences of the above indicated NCBI Reference Sequences of the OAS1 transcript, and also relates to the corresponding amino acid sequences for example as set forth in SEQ ID NO:85 or in SEQ ID NO:86 or in SEQ ID NO:87 or in SEQ ID NO:88, which correspond to the protein sequences defined in NCBI Protein Accession Reference Sequence NP_001307080 and in NCBI Protein Accession Reference Sequence NP_002525 and in NCBI Protein Accession Reference Sequence NP_001027581 and in NCBI Protein Accession Reference Sequence NP_058132 encoding the OAS1 polypeptide.

The term "OAS1" also comprises nucleotide sequences showing a high degree of homology to OAS1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84 or amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84.

The term "PAG1" refers to the Phosphoprotein Membrane Anchor With Glycosphingolipid Microdomains 1 gene (Ensembl: ENSG00000076641), for example, to the sequence as defined in NCBI Reference Sequence NM_018440, specifically, to the nucleotide sequence as set forth in SEQ ID NO:89, which corresponds to the sequence of the above indicated NCBI Reference Sequence of the PAG1 transcript, and also relates to the corresponding amino acid sequence for example as set forth in SEQ ID NO:90, which corresponds to the protein sequence defined in NCBI Protein Accession Reference Sequence NP_060910 encoding the PAG1 polypeptide.

The term "PAG1" also comprises nucleotide sequences showing a high degree of homology to PAG1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:89 or amino acid sequences being at least 75%, 80%, 85%, 90%, 91%, 92%, 93%, 94%, 95%, 96%, 97%, 98% or 99% identical to the sequence as set forth in SEQ ID NO:90 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:90 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:89.

The term "PDE4D" refers to the human Phosphodiesterase 4D gene (Ensembl: ENSG00000113448), for example, to the sequence as defined in NCBI Reference Sequence NM_001104631 or in NCBI Reference Sequence NM_001349242 or in NCBI Reference Sequence NM_001197218 or in NCBI Reference Sequence NM_006203 or in NCBI Reference Sequence NM_001197221 or in NCBI Reference Sequence NM_001197220 or in NCBI Reference Sequence NM_001197223 or in NCBI Reference Sequence NM_001165899 or in NCBI Reference Sequence NM_001165899, specifically, to the nucleotide sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99, which correspond to the sequences of the above indicated NCBI Reference Sequence of the PDE4D transcript, and also relates to the corresponding amino acid sequence for example as set forth in SEQ ID NO: 100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO: 107 or in SEQ ID NO:108, which correspond to the protein sequences defined in NCBI Protein Accession Reference Sequence NP_001098101 and in NCBI Protein Accession Reference Sequence NP_001336171 and in NCBI Protein Accession Reference Sequence NP_001184147 and in NCBI Protein Accession Reference Sequence NP_006194 and in NCBI Protein Accession Reference Sequence NP_001184150 and in NCBI Protein Accession Reference Sequence NP_001184149 and in NCBI Protein Accession Reference Sequence NP_001184152 and in NCBI Protein Accession Reference Sequence NP_001159371 and in NCBI Protein Accession Reference Sequence NP_001184148 encoding the PDE4D polypeptide.

The term "PDE4D" also comprises nucleotide sequences showing a high degree of homology to PDE4D, e.g., nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99 or amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99.

The term "PRKACA" refers to the Protein Kinase cAMP-Activated Catalytic Subunit Alpha gene (Ensembl: ENSG00000072062), for example, to the sequence as defined in NCBI Reference Sequence NM_002730 or in NCBI Reference Sequence NM_207518, specifically, to the nucleotide sequences as set forth in SEQ ID NO:109 or in SEQ ID NO:110, which correspond to the sequences of the above indicated NCBI Reference Sequences of the PRKACA transcript, and also relates to the corresponding amino acid sequence for example as set forth in SEQ ID NO:111 or in SEQ ID NO:112, which correspond to the protein sequences defined in NCBI Protein Accession Reference Sequence NP_002721 and in NCBI Protein Accession Reference Sequence NP_997401 encoding the PRKACA polypeptide.

The term "PRKACA" also comprises nucleotide sequences showing a high degree of homology to PRKACA, e.g., nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110 or amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110.

The term "PRKACB" refers to the Protein Kinase cAMP-Activated Catalytic Subunit Beta gene (Ensembl: ENSG00000142875), for example, to the sequence as defined in NCBI Reference Sequence NM_002731 or in NCBI Reference Sequence NM_182948 or in NCBI Reference Sequence NM_001242860 or in NCBI Reference Sequence NM_001242859 or in NCBI Reference Sequence NM_001242858 or in NCBI Reference Sequence NM_001242862 or in NCBI Reference Sequence NM_001242861 or in NCBI Reference Sequence NM_001300915 or in NCBI Reference Sequence NM_207578 or in NCBI Reference Sequence NM_001242857 or in NCBI Reference Sequence NM_001300917, specifically, to the nucleotide sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123, which correspond to the sequences of the above indicated NCBI Reference Sequences of the PRKACB transcript, and also relates to the corresponding amino acid sequence for example as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134, which correspond to the protein sequences defined in NCBI Protein Accession Reference Sequence NP_002722 and in NCBI Protein Accession Reference Sequence NP_891993 and in NCBI Protein Accession Reference Sequence NP_001229789 and in NCBI Protein Accession Reference Sequence NP_001229788 and in NCBI Protein Accession Reference Sequence NP_001229787 and in NCBI Protein Accession Reference Sequence NP_001229791 and in NCBI Protein Accession Reference Sequence NP_001229790 and in NCBI Protein Accession Reference Sequence NP_001287844 and in NCBI Protein Accession Reference Sequence NP_997461 and in NCBI Protein Accession Reference Sequence NP_001229786 and in NCBI Protein Accession Reference Sequence NP_001287846 encoding the PRKACB polypeptide.

The term "PRKACB" also comprises nucleotide sequences showing a high degree of homology to PRKACB, e.g., nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID N0:123 or amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123.

The term "PTPRC" refers to the Protein Tyrosine Phosphatase Receptor Type C gene (Ensembl: ENSG00000081237), for example, to the sequence as defined in NCBI Reference Sequence NM_002838 or in NCBI Reference Sequence NM_080921, specifically, to the nucleotide sequence as set forth in SEQ ID NO:135 or in SEQ ID NO:136, which correspond to the sequences of the above indicated NCBI Reference Sequences of the PTPRC transcript, and also relates to the corresponding amino acid sequence for example as set forth in SEQ ID NO:137 or in SEQ ID NO:138, which correspond to the protein sequences defined in NCBI Protein Accession Reference Sequence NP_002829 encoding the PTPRC polypeptide and in NCBI Protein Accession Reference Sequence NP_563578.

The term "PTPRC" also comprises nucleotide sequences showing a high degree of homology to PTPRC, e.g., nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136 or amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO:138 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 137 or in SEQ ID NO:138 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136.

The term "RAP1GAP2" refers to the human RAP1 GTPase Activating Protein 2 gene (ENSG00000132359), for example, to the sequence as defined in NCBI Reference Sequence NM_015085 or in NCBI Reference Sequence NM_001100398 or in NCBI Reference Sequence NM_001330058, specifically, to the nucleotide sequence as set forth in SEQ ID NO:139 or in SEQ ID NO:140 or in SEQ ID NO:141, which correspond to the sequences of the above indicated NCBI Reference Sequences of the RAP1GAP2 transcript, and also relates to the corresponding amino acid sequence for example as set forth in SEQ ID NO: 142 or in SEQ ID NO: 143 or in SEQ ID NO: 144, which correspond to the protein sequences defined in NCBI Protein Accession Reference Sequence NP_055900 and in NCBI Protein Accession Reference Sequence NP_001093868 and in NCBI Protein Accession Reference Sequence NP_001316987 encoding the RAP1GAP2 polypeptide.

The term "RAP1GAP2" also comprises nucleotide sequences showing a high degree of homology to RAP1GAP2, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 139 or in SEQ ID NO: 140 or in SEQ ID NO: 141 or amino acid sequences being at least 75%, 80%, 85%, 90%, 91%, 92%, 93%, 94%, 95%, 96%, 97%, 98% or 99% identical to the sequence as set forth in SEQ ID NO: 142 or in SEQ ID NO: 143 or in SEQ ID NO: 144 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:142 or in SEQ ID NO:143 or in SEQ ID NO:144 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:139 or in SEQ ID NO:140 or in SEQ ID NO:141.

The term "SLC39A11" refers to the human Solute Carrier Family 39 Member 11 gene (Ensembl: ENSG00000133195), for example, to the sequence as defined in NCBI Reference Sequence NM_139177 or in NCBI Reference Sequence NM_001352692, specifically, to the nucleotide sequence as set forth in SEQ ID NO: 145 or in SEQ ID NO: 146, which correspond to the sequences of the above indicated NCBI Reference Sequences of the SLC39A11 transcript, and also relates to the corresponding amino acid sequence for example as set forth in SEQ ID NO: 147 or in SEQ ID NO: 148, which correspond to the protein sequences defined in NCBI Protein Accession Reference Sequence NP_631916 and in NCBI Protein Accession Reference Sequence NP_001339621 encoding the SLC39A11 polypeptide.

The term "SLC39A11" also comprises nucleotide sequences showing a high degree of homology to SLC39A11, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 145 or in SEQ ID NO: 146 or amino acid sequences being at least 75%, 80%, 85%, 90%, 91%, 92%, 93%, 94%, 95%, 96%, 97%, 98% or 99% identical to the sequence as set forth in SEQ ID NO: 147 or in SEQ ID NO: 148 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 147 or in SEQ ID NO:148 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:145 or in SEQ ID NO:146.

The term "TDRD1" refers to the human Tudor Domain Containing 1 gene (Ensembl: ENSG00000095627), for example, to the sequence as defined in NCBI Reference Sequence NM_198795, specifically, to the nucleotide sequence as set forth in SEQ ID NO: 149, which corresponds to the sequence of the above indicated NCBI Reference Sequence of the TDRD1 transcript, and also relates to the corresponding amino acid sequence for example as set forth in SEQ ID NO: 150, which corresponds to the protein sequence defined in NCBI Protein Accession Reference Sequence NP_942090 encoding the TDRD1 polypeptide.

The term "TDRD1" also comprises nucleotide sequences showing a high degree of homology to TDRD1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:149 or amino acid sequences being at least 75%, 80%, 85%, 90%, 91%, 92%, 93%, 94%, 95%, 96%, 97%, 98% or 99% identical to the sequence as set forth in SEQ ID NO: 150 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 150 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 149.

The term "TLR8" refers to the Toll Like Receptor 8 gene (Ensembl: ENSG00000101916), for example, to the sequence as defined in NCBI Reference Sequence NM_138636 or in NCBI Reference Sequence NM_016610, specifically, to the nucleotide sequences as set forth in SEQ ID NO:151 or in SEQ ID NO:152, which correspond to the sequences of the above indicated NCBI Reference Sequences of the TLR8 transcript, and also relates to the corresponding amino acid sequences for example as set forth in SEQ ID NO: 153 or in SEQ ID NO: 154, which corresponds to the protein sequences defined in NCBI Protein Accession Reference Sequence NP_619542 and in NCBI Protein Accession Reference Sequence NP_057694 encoding the TLR8 polypeptide.

The term "TLR8" also comprises nucleotide sequences showing a high degree of homology to TLR8, e.g., nucleic acid sequences being at least 75%, 80%, 85%, 90%, 91%, 92%, 93%, 94%, 95%, 96%, 97%, 98% or 99% identical to the sequence as set forth in SEQ ID NO:151 or in SEQ ID NO:152 or amino acid sequences being at least 75%, 80%, 85%, 90%, 91%, 92%, 93%, 94%, 95%, 96%, 97%, 98% or 99% identical to the sequence as set forth in SEQ ID NO: 153 or in SEQ ID NO: 154 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:153 or in SEQ ID NO:154 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:151 or in SEQ ID NO:152.

The term "VWA2" refers to the human Von Willebrand Factor A Domain Containing 2 gene (Ensembl: ENSG00000165816), for example, to the sequence as defined in NCBI Reference Sequence NM_001320804, specifically, to the nucleotide sequence as set forth in SEQ ID NO: 155, which corresponds to the sequence of the above indicated NCBI Reference Sequence of the VWA2 transcript, and also relates to the corresponding amino acid sequence for example as set forth in SEQ ID NO: 156, which corresponds to the protein sequence defined in NCBI Protein Accession Reference Sequence NP_001307733 encoding the VWA2 polypeptide.

The term "VWA2" also comprises nucleotide sequences showing a high degree of homology to VWA2, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 155 or amino acid sequences being at least 75%, 80%, 85%, 90%, 91%, 92%, 93%, 94%, 95%, 96%, 97%, 98% or 99% identical to the sequence as set forth in SEQ ID NO: 156 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:156 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 155.

The term "ZAP70" refers to the Zeta Chain Of T-Cell Receptor Associated Protein Kinase 70 gene (Ensembl: ENSG00000115085), for example, to the sequence as defined in NCBI Reference Sequence NM_001079 or in NCBI Reference Sequence NM_207519, specifically, to the nucleotide sequences as set forth in SEQ ID NO:157 or in SEQ ID NO: 158, which correspond to the sequences of the above indicated NCBI Reference Sequences of the ZAP70 transcript, and also relates to the corresponding amino acid sequence for example as set forth in SEQ ID NO: 159 or in SEQ ID NO: 160, which corresponds to the protein sequences defined in NCBI Protein Accession Reference Sequence NP_001070 and in NCBI Protein Accession Reference Sequence NP_997402 encoding the ZAP70 polypeptide.

The term "ZAP70" also comprises nucleotide sequences showing a high degree of homology to ZAP70, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 157 or in SEQ ID NO: 158or amino acid sequences being at least 75%, 80%, 85%, 90%, 91%, 92%, 93%, 94%, 95%, 96%, 97%, 98% or 99% identical to the sequence as set forth in SEQ ID NO: 159 or in SEQ ID NO: 160 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 159 or in SEQ ID NO:160 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158.

The term "ZBP1" refers to the Z-DNA Binding Protein 1 gene (Ensembl: ENSG00000124256), for example, to the sequence as defined in NCBI Reference Sequence NM_030776 or in NCBI Reference Sequence NM_001160418 or in NCBI Reference Sequence NM_001160419, specifically, to the nucleotide sequence as set forth in SEQ ID NO:161 or in SEQ ID NO:162 or in SEQ ID NO:163, which corresponds to the sequence of the above indicated NCBI Reference Sequence of the ZBP1 transcript, and also relates to the corresponding amino acid sequence for example as set forth in SEQ ID NO: 164 or in SEQ ID NO: 165 or in SEQ ID NO: 166, which corresponds to the protein sequences defined in NCBI Protein Accession Reference Sequence NP_110403 and in NCBI Protein Accession Reference Sequence NP_001153890 and in NCBI Protein Accession Reference Sequence NP_001153891 encoding the ZBP1 polypeptide.

The term "ZBP1" also comprises nucleotide sequences showing a high degree of homology to ZBP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163 or amino acid sequences being at least 75%, 80%, 85%, 90%, 91%, 92%, 93%, 94%, 95%, 96%, 97%, 98% or 99% identical to the sequence as set forth in SEQ ID NO: 164 or in SEQ ID NO: 165 or in SEQ ID NO: 166 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 164 or in SEQ ID NO: 165 or in SEQ ID NO: 166 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163.

The term "biological sample" or "sample obtained from a subject" refers to any biological material obtained via suitable methods known to the person skilled in the art from a subject, e.g., a colorectal cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood (or blood derived such as serum, plasma, or PBMC's (peripheral blood mononuclear cells)), sweat, saliva, urine, and a needle biopsy or resection biopsy. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the colorectal of a subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., colorectal cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "colorectal cancer" refers to a cancer of the colon or the rectum. Colorectal cancers start in either the colon or the rectum, which make up a large part of the intestine, which in turn is part of the gastrointestinal (GI) system. Colorectal cancers can also be called colon cancer or rectal cancer, depending on their site of origin. However, these cancers are often grouped together as colorectal cancer due to their common nature. The term "TNM" refers to a classification system, which is used to describe the characteristics of malignant tumors.

The term "T stage" refers to the extent and size of the tumor according to the TNM classification system. T stage can have the attributes T1 (small local tumor; typically < 2 cm in size); T2 (larger local tumor; typically 2-5 cm in size); T3 (larger locally advanced tumor; typically >5 cm in size; T4 (advanced/metastatic tumor).

The term "N stage" refers to the presence and extent of tumor positive lymph nodes according to the TNM classification system. N stage can have the attributes NO (no evidence of tumor positive lymph nodes); N1 (evidence of tumor positive lymph nodes; N2/N3 (more extended number of tumor positive lymph nodes); NX (no assessment of lymph node status possible).

The term "M stage" refers to the presence and extent of tumor metastases according to the TNM classification system. M stage can have the attributes M0 (no evidence of the presence of distant metastases); M1 (evidence of the presence of distant metastases).

The term "survival" refers to survival of a patient from his or her colorectal cancer.

It is preferred that:

- the one or more immune defense response genes comprise three or more, preferably, six or more, more preferably, nine or more, most preferably, all of the immune defense genes, and/or
- the one or more T-Cell receptor signaling genes comprise three or more, preferably, six or more, more preferably, nine or more, most preferably, all of the T-Cell receptor signaling genes, and/or
- the one or more PDE4D7 correlated genes comprise three or more, preferably, six or more, most preferably, all of the PDE4D7 correlated genes.

It is preferred that the determining of the outcome comprises:

- combining the first gene expression profiles for two or more, for example, 2, 3, 4, 5, 6, 7, 8, 9, 10, 11, 12, 13 or all, of the immune defense response genes with a regression function that had been derived from a population of colorectal cancer subjects, and/or
- combining the second gene expression profiles for two or more, for example, 2, 3, 4, 5, 6, 7, 8, 9, 10, 11, 12, 13, 14, 15, 16 or all, of the T-Cell receptor signaling genes with a regression function that had been derived from a population of colorectal cancer subjects, and/or
- combining the third gene expression profiles for two or more, for example, 2, 3, 4, 5, 6, 7 or all, of the PDE4D7 correlated genes with a regression function that had been derived from a population of colorectal cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t) = Ho(t) \cdot \exp(w_1 \cdot V_1 + w_2 \cdot V_2 + w_3 . V_3 + ...)$, where $V_1$, $V_2$, $V_3$ ... are predictor variables and $H_0(t)$ is the baseline hazard while $H(t)$ is the hazard at any time t. The hazard ratio (or the risk to reach the event) is represented by $Ln[H(t)/ Ho(t)] = w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

In one particular realization, the combination of the first gene expression profiles for the two or more, for example, 2, 3, 4, 5, 6, 7, 8, 9, 10, 11, 12, 13 or all, of the immune defense response genes with a regression function is determined as follows:

IDR_model:

$$(w_1 \cdot AIM2) + (w_2 \cdot APOBEC3A) + (w_3 \cdot CIAO1) + (w_4 \cdot DDX58) + (w_5 \cdot DHX9) + (w_6 \cdot IFI16) + (w_7 \cdot IFIH1) + (w_8 \cdot IFIT1) + (w_9 \cdot IFIT3) + (w_{10} \cdot LRRFIP1) + (w_{11} \cdot MYD88) + (w_{12} \cdot OAS1) + (w_{13} \cdot TLR8) + (w_{14} \cdot ZBP1) \quad (1)$$

where $w_1$ to $w_{14}$ are weights and AIM2, APOBEC3A, CIAO1, DDX58, DHX9, IFI16, IFIH1, IFIT1, IFIT3, LRRFIP1, MYD88, OAS1, TLR8, and ZBP1 are the expression levels of the immune defense response genes.

In one example, $w_1$ may be about -0.8 to 0.2, such as -0.32, $w_2$ may be about 0.0 to 1.0, such as 0.4743, $w_3$ may be about -0.2 to 0.8, such as 0.2864, $w_4$ may be about -1.2 to -0.2, such as -0.6683, $w_5$ may be about -0.9 to 0.1, such as -0.3665, $w_6$ may be about -0.4 to 0.6, such as 0.1357, $w_7$ may be about 0.0 to 1.0, such as 0.505, $w_8$ may be about -0.6 to 0.4, such as -0.1024, $w_9$ may be about 0.2 to 1.2, such as 0.7229, $w_{10}$ may be about -0.3 to 0.7, such as 0.2066, $w_{11}$ may be about -0.6 to 0.4, such as -0.1209, $w_{12}$ may be about -0.8 to 0.2, such as -0.2982, $w_{13}$ may be about -0.4 to 0.6, such as 0.1005, and $w_{14}$ may be about -0.7 to 0.3, such as -0.2253.

In one particular realization, the combination of the second gene expression profiles for the two or more, for example, 2, 3, 4, 5, 6, 7, 8, 9, 10, 11, 12, 13, 14, 15, 16 or all, of the T-Cell receptor signaling genes with a regression function is determined as follows:

TCR_SIGNALING_model:

$$(w_{15} \cdot C2) + (w_{16} \cdot CD247) + (w_{17} \cdot CD28) + (w_{18} \cdot CD3E) + (w_{19} \cdot CD3G) + (w_{20} \cdot CD4) + (w_{21} \cdot CSK) + (w_{22} \cdot EZR) + (w_{23} \cdot FYN) + (w_{24} \cdot LAT) + (w_{25} \cdot LCK) + (w_{26} \cdot PAG1) + (w_{27} \cdot PDE4D) + (w_{28} \cdot PRKACA) + (w_{29} \cdot PRKACB) + (w_{30} \cdot PTPRC) + (w_{31} \cdot ZAP70) \quad (2)$$

where $w_{15}$ to $w_{31}$ are weights and CD2, CD247, CD28, CD3E, CD3G, CD4, CSK, EZR, FYN, LAT, LCK, PAG1, PDE4D, PRKACA, PRKACB, PTPRC, and ZAP70 are the expression levels of the T-Cell receptor signaling genes.

In one example, $w_{15}$ may be about -1.2 to -0.2, such as -0.72, $w_{16}$ may be about 0.0 to 1.0, such as 0.5222, $w_{17}$ may be about -0.1 to 0.9, such as 0.4222, $w_{18}$ may be about 0.1 to 1.1, such as 0.5981, $w_{19}$ may be about -0.9 to 0.1, such as -0.3978, $w_{20}$ may be about 0.0 to 1.0, such as 0.5332, $w_{21}$ may be about -0.5 to 0.5, such as 0.007001, $w_{22}$ may be about -0.3 to 0.7, such as 0.1881, $w_{23}$ may be about -0.5 to 0.5, such as 0.08063, $w_{24}$ may be about -0.7 to 0.3, such as -0.2047, $w_{25}$ may be about -0.4 to 0.6, such as 0.1408, $w_{26}$ may be about -0.4 to 0.6, such as 0.1038, $w_{27}$ may be about -0.6 to 0.4, such as -0.1477, $w_{28}$ may be about -0.3 to 0.7, such as 0.2311, $w_{29}$ may be about -1.0 to 0.0, such as -0.4637, $w_{30}$ may be about -1.4 to -0.4, such as -0.8881, and $w_{31}$ may be about -0.3 to 0.7, such as 0.1936. In one particular realization, the combination of the third gene expression profiles for the two or more, for example, 2, 3, 4, 5, 6, 7 or all, of the PDE4D7 correlated genes with a regression function is determined as follows:
PDE4D7_CORR_model:

$$(w_{32} \cdot ABCC5) + (w_{33} \cdot CUX2) + (w_{34} \cdot KIAA1549) + (w_{35} \cdot PDE4D) + (w_{36} \cdot RAP1GAP2) + (w_{37} \cdot SLC39A11) + (w_{38} \cdot TDRD1) + (w_{39} \cdot VWA2) \quad (3)$$

where $w_{32}$ to $w_{39}$ are weights and ABCC5, CUX2, KIAA1549, PDE4D, RAP1GAP2, SLC39A11, TDRD1, and VWA2 are the expression levels of the PDE4D7 correlated genes.

In one example, $w_{32}$ may be about -0.4 to 0.6, such as 0.09275, $w_{33}$ may be about -0.2 to 0.7, such as 0.2824, $w_{34}$ may be about -0.8 to 0.2, such as -0.2594, $w_{35}$ may be about -0.5 to 0.5, such as -0.0217, $w_{36}$ may be about -0.4 to 0.6, such as 0.08958, $w_{37}$ may be about -0.7 to 0.3, such as -0.152, $w_{38}$ may be about -0.8 to 0.2, such as -0.2854, and $w_{39}$ may be about -0.6 to 0.4, such as -0.1182.

It is further preferred that the determining of the prediction of the outcome further comprises combining the combination of the first gene expression profiles, the combination of the second gene expression profiles, and the combination of the third gene expression profiles with a regression function that had been derived from a population of colorectal cancer subjects.

In one particular realization, the prediction of the outcome is determined as follows:
CRCAI_model:

$$(w_{40} \cdot IDR_model) + (w_{41} \cdot TCR_SIGNALING_model) + (w_{42} \cdot PDE4D7_CORR_model) \quad (4)$$

where $w_{40}$ to $w_{42}$ are weights, IDR_model is the above-described regression model based on the expression profiles for the two or more, for example, 2, 3, 4, 6, 7, 8, 9, 10, 11, 12, 13 or all, of the immune defense response genes, TCR_SIGNALING_model is the above-described regression model based on the expression profiles for the two or more, for example, 2, 3, 4, 6, 7, 8, 9, 10, 11, 12, 13, 14, 15, 16 or all, of the T-Cell receptor signaling genes, and PDE4D7_CORR_model is the above-described regression model based on the expression profiles for the two or more, for example, 2, 3, 4, 6, 7 or all, of the PDE4D7 correlated genes.

In one example, $w_{40}$ may be about 0.3 to 1.3, such as 0.7878, $w_{41}$ may be about 0.3 to 1.3, such as 0.7699, and $w_{42}$ may be about 0.1 to 1.1, such as 0.6176.

The prediction of the outcome may also be classified or categorized into one of at least two risk groups, based on the value of the prediction of the outcome. For example, there may be two risk groups, or three risk groups, or four risk groups, or more than four predefined risk groups. Each risk group covers a respective range of (non-overlapping) values of the prediction of the outcome. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the outcome is further based on one or more clinical parameters obtained from the subject.

As mentioned above, various measures based on clinical parameters have been investigated. By further basing the prediction of the outcome on such clinical parameter(s), it can be possible to further improve the prediction.

It is preferred that the clinical parameters comprise one or more of: (i) T stage attribute (T1, T2, T3, or T4); (ii) N stage attribute (N0, N1, or N2), and; (iii) M stage attribute (M0, M1). Additionally or alternatively, the clinical parameters comprise one or more other clinical parameters that is/are relevant for the diagnosis and/or prognosis of colorectal cancer.

It is further preferred that the determining of the prediction of the outcome comprises combining one or more of: (i) the first gene expression profile(s) for the one or more immune defense response genes; (ii) the second gene expression profile(s) for the one or more T-Cell receptor signaling genes; (iii) the third gene expression profile(s) for the one or more PDE4D7 correlated genes, and; (iv) the combination of the first gene expression profiles, the combination of the second gene expression profiles, and the combination of the third gene expression profiles, and the one or more clinical parameters obtained from the subject with a regression function that had been derived from a population of colorectal cancer subjects.

In one particular realization, the prediction of the outcome is determined as follows:

CRCAI&Clinical_model:

$$(w_{40} \cdot \text{IDR_model}) + (w_{41} \cdot \text{TCR_SIGNALING_model}) + (w_{42} \cdot \text{PDE4D7_CORR_model}) + (w_{43} \cdot \text{N_stage_N1}) + (w_{44} \cdot \text{N_stage_N2}) \tag{5}$$

where $w_{40}$ to $w_{44}$ are weights, IDR_model is the above-described regression model based on the expression profiles for the two or more, for example, 2, 3, 4, 6, 7, 8, 9, 10, 11, 12, 13 or all, of the immune defense response genes, TCR_SIGNALING_model is the above-described regression model based on the expression profiles for the two or more, for example, 2, 3, 4, 6, 7, 8, 9, 10, 11, 12, 13, 14, 15, 16 or all, of the T-Cell receptor signaling genes, and PDE4D7_CORR_model is the above-described regression model based on the expression profiles for the two or more, for example, 2, 3, 4, 6, 7 or all, of the PDE4D7 correlated genes, and N_stage_N1 and N_stage_N2 are clinical N stage attributes according to the TNM classification of malignant tumors.

In one example, $w_{40}$ may be about 0.3 to 1.3, such as 0.8098, $w_{41}$ may be about 0.2 to 1.2, such as 0.7297, $w_{42}$ may be about 0.1 to 1.1, such as 0.6221, $w_{43}$ may be about 0.0 to 1.0, such as 0.4875, and $w_{44}$ may be about 0.4 to 1.4, such as 0.9216.

It is preferred that the biological sample is obtained from the subject before the start of the therapy. The gene expression profile(s) may be determined in the form of mRNA or protein in tissue of colorectal cancer. Alternatively, if the genes are present in a soluble form, the gene expression profile(s) may be determined in blood.

It is further preferred that the therapy is surgery, radiotherapy, cytotoxic chemotherapy (CTX), short- or long-course chemo-radiation therapy (CRT), immunotherapy, or any combination thereof.

It is preferred that the prediction of the therapy response is unlikely or likely for the effectiveness of the therapy, wherein a therapy is recommended based on the prediction and, if the prediction is negative, the recommended therapy comprises one or more of: (i) therapy provided earlier than is the standard; (ii) radiotherapy with an increased effective dose; (iii) an adjuvant therapy, such as chemotherapy; (iv) long-course CRT (chemo-radiation therapy), and; (iv) an alternative therapy, such as immunotherapy.

In a further aspect of the present invention, an apparatus for predicting an outcome of a colorectal cancer subject is presented, comprising:

- an input adapted to receive data indicative of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from the subject, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from the subject, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from the subject,

- a processor adapted to determine the prediction of the outcome based on the first gene expression profile(s), or on the second gene expression profile(s), or on the third gene expression profile(s), or on the first, second, and third gene expression profile(s), and
- optionally, a providing unit adapted to provide the prediction to a medical caregiver or the subject.

In a further aspect of the present invention, a computer program product is presented comprising instructions which, when the program is executed by a computer, cause the computer to carry out a method comprising:

- receiving data indicative of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from a colorectal cancer subject, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from a colorectal cancer subject, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from a colorectal cancer subject,
- determining a prediction of an outcome of the subject based on the first gene expression profile(s), or on the second gene expression profile(s), or on the third gene expression profile(s), or on the first, second, and third gene expression profile(s), and
- optionally, providing the prediction to a medical caregiver or the subject.

In a further aspect of the present invention, a diagnostic kit is presented, comprising:

- at least one primer and/or probe for determining a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from a subject, and/or
- at least one primer and/or probe for determining a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in a biological sample obtained from a subject, and/or
- at least one primer and/or probe for determining a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a biological sample obtained from a subject, and
- optionally, an apparatus as defined in claim 11 or a computer program product as defined in claim 12.

In a further aspect of the present invention, a use of the kit as defined in claim 13 is presented.

It is preferred that the use as defined in claim 14 is in a method of predicting an outcome of a colorectal cancer subject.

In a further aspect of the present invention, a method is presented, comprising:

- receiving one or more biological sample(s) obtained from a colorectal cancer subj ect,
- using the kit as defined in claim 13 to determine a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from the subject, and/or
- using the kit as defined in claim 13 to determine a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in a biological sample obtained from the subject, and/or
- using the kit as defined in claim 13 to determine a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a biological sample obtained from the subj ect.

In a further aspect of the present invention, a use of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a method of predicting an outcome of a colorectal cancer subject is presented, comprising:

- determining the prediction of the outcome based on the first gene expression profile(s), or on the second gene expression profile(s), or on the third gene expression profile(s), or on the first, second, and third gene expression profile(s), and
- optionally, providing the prediction or the personalization or a therapy recommendation based on the prediction or the personalization to a medical caregiver or the subj ect.

It shall be understood that the method of claim 1, the apparatus of claim 11, the computer program product of claim 12, the diagnostic kit of claim 13, the use of the diagnostic kit of claim 14, the method of claim 16, and the use of first, second, and/or third gene expression profile(s) of claim 17 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting an outcome of a colorectal cancer subject,

Fig. 2 shows a Kaplan-Meier curve of the IDR_model in a 168 patient TCGA colorectal cancer cohort (training set used to develop the IDR model),

Fig. 3 shows a Kaplan-Meier curve of the IDR_model in a 83 patient TCGA colorectal cancer cohort (testing set used to validate the IDR model as developed on the 168 patient training set),

Fig. 4 shows a Kaplan-Meier curve of the TCR_SIGNALING_model in a 168 patient TCGA colorectal cancer cohort (training set used to develop the TCR SIGNALING model),

Fig. 5 shows a Kaplan-Meier curve of the TCR_SIGNALING_model in a 83 patient TCGA colorectal cancer cohort (testing set used to validate the TCR_SIGNALING_model as developed on the 168 patient training set),

Fig. 6 shows a Kaplan-Meier curve of the PDE4D7_CORR_model in a 168 patient TCGA colorectal cancer cohort (training set used to develop the PDE4D7_CORR_model),

Fig. 7 shows a Kaplan-Meier curve of the PDE4D7_CORR_model in a 83 patient TCGA colorectal cancer cohort (testing set used to validate the PDE4D7_CORR_model as developed on the 168 patient training set),

Fig. 8 shows a Kaplan-Meier curve of the CRCAI_model in a 168 patient TCGA colorectal cancer cohort (training set used to develop the CRCAI_model),

Fig. 9 shows a Kaplan-Meier curve of the CRCAI_model in a 83 patient TCGA colorectal cancer cohort (testing set used to validate the CRCAI_model as developed on the 168 patient training set),

Fig. 10 shows a Kaplan-Meier curve of the CRCAI&Clinical_model in a 168 patient TCGA colorectal cancer cohort (training set used to develop the CRCAI&Clinical_model),

Fig. 11 shows a Kaplan-Meier curve of the CRCAI&Clinical_model in a 83 patient TCGA colorectal cancer cohort (testing set used to validate the CRCAI&Clinical_model as developed on the 168 patient training set),

Fig. 12 shows a Kaplan-Meier curve of the CRCAI_model in a colorectal cancer cohort derived from the GSE41248 data set. The clinical endpoint that was tested was the overall death,

Fig. 13 shows a Kaplan-Meier curve of the CRCAI_model in a colorectal cancer cohort derived from the GSE41248 data set. The clinical endpoint that was tested was the cancer specific death,

Fig. 14 shows a Kaplan-Meier curve of the CRCAI&Clinical_model in a colorectal cancer cohort derived from the GSE41248 data set. The clinical endpoint that was tested was the overall death, and

Fig. 15 shows a Kaplan-Meier curve of the CRCAI&Clinical_model in a colorectal cancer cohort derived from the GSE41248 data set. The clinical endpoint that was tested was the cancer specific death.

DETAILED DESCRIPTION OF EMBODIMENTS

**Overview Of Outcome Prediction**

Fig. 1 shows schematically and exemplarily a flowchart of an embodiment of a method of predicting an outcome of a colorectal cancer subject.

The method begins at step S100.

At step S102, a biological sample is obtained from each of a first set of patients (subjects) diagnosed with colorectal cancer. Preferably, monitoring colorectal cancer has been performed for these colorectal cancer patients over a period of time, such as at least one year, or at least two years, or about five years, after obtaining the biological sample.

At step S104, a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or a third gene expression profile for each of two or more, for example, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more genes which can be normalized using value(s) for each of a set of reference genes, such as B2M, HPRT1, POLR2A, and/or PUM1. In one realization, the gene expression level for each of the two or more genes of the first gene expression profiles, and/or the second gene expression profiles, and/or the third gene expression profiles is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes.

At step S106, a regression function for assigning a prediction of the outcome is determined based on the first gene expression profiles for the two or more immune defense response genes, AIM2, APOBEC3A, CIAO1, DDX58, DHX9, IFI16, IFIH1, IFIT1, IFIT3, LRRFIP1, MYD88, OAS1, TLR8, and/or ZBP1, and/or the second gene expression profiles for the two or more T-Cell receptor signaling genes, CD2, CD247, CD28, CD3E, CD3G, CD4, CSK, EZR, FYN, LAT, LCK, PAG1, PDE4D, PRKACA, PRKACB, PTPRC, and/or ZAP70, and/or the third gene expression profiles for the two or more PDE4D7 correlated genes, ABCC5, CUX2, KIAA1549, PDE4D, RAP1GAP2, SLC39A11, TDRD1, and/or VWA2, obtained for at least some of the biological samples obtained for the first set of patients and respective results obtained from the monitoring. In one particular realization, the regression function is determined as specified in Eq. (4) above.

At step S108, a biological sample is obtained from a patient (subject or individual). The patient can be a new patient or one of the first set.

At step S110, a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or a third gene expression profile is obtained for each of the two or more, for example, 2, 3, 4, 5, 6, 7 or all, PDE4D7 correlated genes, e.g., by performing PCR on the biological sample. In one realization, the gene expression level for each of the two or more genes of the first gene expression profiles, and/or the second gene expression profiles, and/or the third gene expression profiles is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes. This is substantially the same as in step S104.

At step S112, a prediction of the outcome based on the first, second, and third gene expression profiles is determined for the patient using the regression function. This will be described in more detail later in the description.

At S114, a therapy recommendation may be provided, e.g., to the patient or his or her guardian, to a doctor, or to another healthcare worker, based on the prediction or the personalization. To this end, the prediction or personalization may be categorized into one of a predefined set of risk groups, based on the value of the prediction or personalization. In one particular realization, the therapy may be radiotherapy and the prediction of the therapy response may be unlikely or likely for the effectiveness of the therapy. If the prediction is unlikely, the recommended therapy may comprise one or more of: (i) therapy provided earlier than is the standard; (ii) radiotherapy with an increased effective dose; (iii) an adjuvant therapy, such as chemotherapy; (iv) long-course CRT (chemo-radiation therapy), and; (iv) an alternative therapy, such as immunotherapy.

The method ends at S116.

In one embodiment, the gene expression profiles at steps S104 and S110 are determined by detecting mRNA expression using two or more primers and/or probes and/or two or more sets thereof.

In one embodiment, steps S104 and S110 further comprise obtaining clinical parameters from the first set of patients and the patient, respectively. The clinical parameters may comprise one or more of: (i) T stage attribute (T1, T2, T3, or T4); (ii) N stage attribute (N0, N1, or N2), and; (iii) M stage attribute (M0, M1). Additionally or alternatively, the clinical parameters comprise one or more other clinical parameters that is/are relevant for the diagnosis and/or prognosis of colorectal cancer. The regression function for assigning the prediction of the outcome that is determined in step S 106 is then further based on the one or more clinical parameters obtained from at least some of the first set of patients. In step S112, the prediction of the outcome is then further based on the one or more clinical parameters, e.g., the N stage attribute, obtained from the patient and is determined for the patient using the regression function. In one particular realization, the regression function is determined as specified in Eq. (5) above.

Based on the significant correlation with survival outcome after therapy, we expect that the identified molecules will provide predictive value with regard to the effectiveness of the treatment of primary colorectal cancers.

RESULTS

For each gene, the log2 expression value as provided in the download from the TCGA database (TCGA Colorectal Adenocarcinoma, Firehose legacy, http://linkedomics.org/login.php, accessed March 6, 2020) was obtained.

The log2 expression values for each gene were transformed into z-scores by calculating:

$$\text{log2_gene transformed z-score} = ((\text{log2_gene}) - (\text{mean_samples}))/(\text{stdev_samples}) \quad (6)$$

where log2_gene is the log2 gene expression value per gene, mean_samples is the mathematical mean of the log2_gene values across all samples, and stdev_samples is the standard deviation of the log2_gene values across all samples.

This process distributes the transformed log2_gene values around the mean 0 with a standard deviation of 1.

For the multivariate analysis of the genes of interest we used the log2_gene transformed z-score value of each gene as input.

**Cox Regression Analysis**

We then set out to test whether the combination of the 14 immune defense response genes, the combination of the 17 T-Cell receptor signalling genes, the combination of the eight PDE4D7 correlated genes, and a combination thereof will exhibit a prognostic value for colorectal cancer. With Cox regression we modelled the expression levels of the 14 immune defense response genes, of the 17 T-Cell receptor signalling genes, and of the eight PDE4D7 correlated genes, respectively, to overall survival in a TCGA cohort of 377 colorectal cancer patients.

From the TCGA colorectal cancer cohort, only samples with combined presence of clinical parameters, gene expression values, and survival information were included. From this subset, we only included samples from patients with non-metastasized disease (m0), which resulted in a total number of 251 patients. These 251 patients were randomly split into 3 groups. Cohort 1 (n=168) was used as a train cohort and consisted of groups 1+2. Cohort 2 (n=83) consisted of group 3 and was used to validate the risk models as derived from the train cohort.

The Cox regression functions were derived as follows:

IDR_model:

$$(w_1 \cdot \text{AIM2}) + (w_2 \cdot \text{APOBEC3A}) + (w_3 \cdot \text{CIAO1}) + (w_4 \cdot \text{DDX58}) + (w_5 \cdot \text{DHX9}) + (w_6 \cdot \text{IFI16}) + (w_7 \cdot \text{IFIH1}) + (w_8 \cdot \text{IFIT1}) + (w_9 \cdot \text{IFIT3}) + (w_{10} \cdot \text{LRRFIP1}) + (w_{11} \cdot \text{MYD88}) + (w_{12} \cdot \text{OAS1}) + (w_{13} \cdot \text{TLR8}) + (w_{14} \cdot \text{ZBP1})$$

TCR_SIGNALING_model:

$$(w_{15} \cdot C2) + (w_{16} \cdot CD247) + (w_{17} \cdot CD28) + (w_{18} \cdot CD3E) + (w_{19} \cdot CD3G) + (w_{20} \cdot CD4) + (w_{21} \cdot CSK) + (w_{22} \cdot EZR) + (w_{23} \cdot FYN) + (w_{24} \cdot LAT) + (w_{25} \cdot LCK) + (w_{26} \cdot PAG1) + (w_{27} \cdot PDE4D) + (w_{28} \cdot PRKACA) + (w_{29} \cdot PRKACB) + (w_{30} \cdot PTPRC) + (w_{31} \cdot ZAP70)$$

PDE4D7_CORR_model:

$$(w_{32} \cdot ABCC5) + (w_{33} \cdot CUX2) + (w_{34} \cdot KIAA1549) + (w_{35} \cdot PDE4D) + (w_{36} \cdot RAP1GAP2) + (w_{37} \cdot SLC39A11) + (w_{38} \cdot TDRD1) + (w_{39} \cdot VWA2)$$

The details for the weights $w_1$ to $w_{39}$ are shown in the following TABLE 1.

TABLE 1: Variables and weights for the three individual Cox regression models, i.e., the immune defense response model (IDR model), the T-Cell receptor signaling model (TCR SIGNALING model), and the PDE4D7 correlation model (PDE4D7_CORR_model) for colorectal cancer; NA - not available.

| **Variable** | **Weights** | | | |
|---|---|---|---|---|
| **Model** | | **IDR_model** | **TCR _ SIGNALING_ model** | **PDE4D7_ CORR_ model** |
| **AIM2** | $w_1$ | -0.32 | NA | NA |
| **APOBEC3A** | $w_2$ | 0.4743 | NA | NA |
| **CIAO1** | $w_3$ | 0.2864 | NA | NA |
| **DDX58** | $w_4$ | -0.6683 | NA | NA |
| **DHX9** | $w_5$ | -0.3665 | NA | NA |
| **IFI16** | $w_6$ | 0.1357 | NA | NA |
| **IFIH1** | $w_7$ | 0.505 | NA | NA |
| **IFIT1** | $w_8$ | -0.1024 | NA | NA |
| **IFIT3** | $w_9$ | 0.7229 | NA | NA |
| **LRRFIP1** | $w_{10}$ | 0.2066 | NA | NA |
| **MYD88** | $w_{11}$ | -0.1209 | NA | NA |
| **OAS1** | $w_{12}$ | -0.2982 | NA | NA |
| **TLR8** | $w_{13}$ | 0.1005 | NA | NA |
| **ZBP1** | $w_{14}$ | -0.2253 | NA | NA |
| **CD2** | $w_{15}$ | NA | -0.72 | NA |
| **CD247** | $w_{16}$ | NA | 0.5222 | NA |
| **CD28** | $w_{17}$ | NA | 0.4222 | NA |
| **CD3E** | $w_{18}$ | NA | 0.5981 | NA |
| **CD3G** | $w_{19}$ | NA | -0.3978 | NA |
| **CD4** | $w_{20}$ | NA | 0.5332 | NA |

(continued)

| Variable | Weights | | | |
|---|---|---|---|---|
| **Model** | | **IDR_model** | **TCR _ SIGNALING_ model** | **PDE4D7_ CORR_ model** |
| **CSK** | $w_{21}$ | NA | 0.007001 | NA |
| **EZR** | $w_{22}$ | NA | 0.1881 | NA |
| **FYN** | $w_{23}$ | NA | 0.08063 | NA |
| **LAT** | $w_{24}$ | NA | -0.2047 | NA |
| **LCK** | $w_{25}$ | NA | 0.1408 | NA |
| **PAG1** | $w_{26}$ | NA | 0.1038 | NA |
| **PDE4D** | $w_{27}$ | NA | -0.1477 | NA |
| **PRKACA** | $w_{28}$ | NA | 0.2311 | NA |
| **PRKACB** | $w_{29}$ | NA | -0.4637 | NA |
| **PTPRC** | $w_{30}$ | NA | -0.8881 | NA |
| **ZAP70** | $w_{31}$ | NA | 0.1936 | NA |
| **ABCC5** | $w_{32}$ | NA | NA | 0.09275 |
| **CUX2** | $w_{33}$ | NA | NA | 0.2824 |
| **KIAA1549** | $w_{34}$ | NA | NA | -0.2594 |
| **PDE4D** | $w_{35}$ | NA | NA | -0.0217 |
| **RAP1GAP2** | $w_{36}$ | NA | NA | 0.08958 |
| **SLC39A11** | $w_{37}$ | NA | NA | -0.152 |
| **TDRD1** | $w_{38}$ | NA | NA | -0.2854 |
| **VWA2** | $w_{39}$ | NA | NA | -0.1182 |

Based on the three individual Cox regression models (IDR_model, TCR_SIGNALING_model, PDE4D7_CORR_model) we then again used Cox regression to model the combination thereof to overall survival with (CRCAI&Clinical_model) or without (CRCAI_model) the presence of the clinical variables (N stage attributes) in the respective cohorts of colorectal cancer patients. We tested the two models in Kaplan-Meier survival analysis.

The Cox regression functions were derived as follows:

CRCAI_model:

$$(w_{40} \cdot \text{IDR_model}) + (w_{41} \cdot \text{TCR_SIGNALING_model}) + (w_{42} \cdot \text{PDE4D7_CORR_model})$$

CRCAI&Clinical_model:

$$(w_{40} \cdot \text{IDR_model}) + (w_{41} \cdot \text{TCR_SIGNALING_model}) + (w_{42} \cdot \text{PDE4D7_CORR_model}) + (w_{43} \cdot \text{N_stage_N1}) + (w_{44} \cdot \text{N_stage_N2})$$

The details for the weights $w_{40}$ to $w_{44}$ are shown in the following TABLE 2.

TABLE 2: Variables and weights for two combination Cox regression models, i.e., colorectal cancer AI model (CRCAI_model) and the colorectal cancer & clinical model (CRCAI&Clinical_model); NA - not available.

| Variable | Weights | | |
|---|---|---|---|
| **Model** | | **CRCAI_model** | **CRCAI&Clinical_model** |
| **IDR_model** | $w_{40}$ | 0.7878 | 0.8098 |
| **TCR_SIGNALING_model** | $w_{41}$ | 0.7699 | 0.7297 |
| **PDE47_CORR_model** | $w_{42}$ | 0.6176 | 0.6221 |
| **N_stage_N1** | $w_{43}$ | NA | 0.3293 |
| **N_stage_N2** | $w_{44}$ | NA | 0.9216 |

**Kaplan-Meier Survival Analysis**

For Kaplan-Meier survival curve analysis, the Cox functions of the risk models (IDR_model, TCR_SIGNALING_model, PDE4D7_CORR_model, CRCAI_model, and CRCAI&Clinical_model) were categorized into two sub-cohorts based on a cut-off. The threshold for group separation into low and high risk was based on the risk to experience the clinical endpoint (outcome) as predicted by the respective Cox regression model

Fig. 2 shows a Kaplan-Meier curve of the IDR_model in a 168 patient TCGA colorectal cancer cohort (training set used to develop the IDR_model). The clinical endpoint that was tested was the overall death (logrank p=0.009; HR=2.5; 95% CI=1.3-5.0). The following supplementary lists indicate the number of patients at risk for the IDR_model classes analyzed (threshold=0), i.e., the patients at risk at any time interval +20 months are shown: Low risk: 80, 45, 21, 15, 9, 6, 4, 1, 0; High risk: 88, 52, 29, 14, 9, 4, 3, 0, 0.

Fig. 3 shows a Kaplan-Meier curve of the IDR_model in a 83 patient TCGA colorectal cancer cohort (testing set used to validate the IDR_model as developed on the 168 patient training set). The clinical endpoint that was tested was the overall death (logrank p=0.8; HR=0.9; 95% CI=0.3-2.6). The following supplementary lists indicate the number of patients at risk for the IDR_model classes analyzed (threshold=0), i.e., the patients at risk at any time interval +20 months are shown: Low risk: 42, 29, 8, 4, 4, 2, 2, 1, 0; High risk: 41, 19, 9, 4, 2, 0, 0, 0, 0.

Fig. 4 shows a Kaplan-Meier curve of the TCR_SIGNALING_model in a 168 patient TCGA colorectal cancer cohort (training set used to develop the TCR_SIGNALING_model). The clinical endpoint that was tested was the overall death (logrank p=0.003; HR=2.9; 95% CI=1.4-5.9). The following supplementary lists indicate the number of patients at risk for the TCR_SIGNALING_model classes analyzed (threshold=0), i.e., the patients at risk at any time interval +20 months are shown: Low risk: 81, 46, 23, 10, 4, 3, 1, 0, 0; High risk: 87, 51, 27, 19, 14, 7, 6, 1, 0.

Fig. 5 shows a Kaplan-Meier curve of the TCR_SIGNALING_model in a 83 patient TCGA colorectal cancer cohort (testing set used to validate the TCR_SIGNALING_model as developed on the 168 patient training set). The clinical endpoint that was tested was the overall death (logrank p=0.02; HR=3.7; 95% CI=1.3-10.9). The following supplementary lists indicate the number of patients at risk for the TCR_SIGNALING_model classes analyzed (threshold=0), i.e., the patients at risk at any time interval +20 months are shown: Low risk: 46, 26, 10, 6, 5, 2, 2, 1, 0; High risk: 37, 22, 7, 2, 1, 0, 0, 0, 0.

Fig. 6 shows a Kaplan-Meier curve of the PDE4D7_CORR_model in a 95 patient TCGA colorectal cancer cohort (training set used to develop the PDE4D7_CORR_model). The clinical endpoint that was tested was the overall death (logrank p<0.0001; HR=10.7; 95% CI=3.5-32.4). The following supplementary lists indicate the number of patients at risk for the PDE4D7_CORR_model classes analyzed (threshold=0), i.e., the patients at risk at any time interval +20 months are shown: Low risk: 143, 86, 45, 27, 17, 10, 7, 1, 0; High risk: 25, 11, 5, 2, 1, 0, 0, 0, 0.

Fig. 7 shows a Kaplan-Meier curve of the PDE4D7_CORR_model in a 83 patient TCGA colorectal cancer cohort (testing set used to validate the PDE4D7_CORR_model as developed on the 168 patient training set). The clinical endpoint that was tested was the overall death (logrank p<0.8; HR=1.2; 95% CI=0.2-5.9). The following supplementary lists indicate the number of patients at risk for the PDE4D7_CORR_model classes analyzed (threshold=0), i.e., the patients at risk at any time interval +20 months are shown: Low risk: 72, 42, 15, 7, 6, 2, 2, 1, 0; High risk: 11, 6, 2, 1, 0, 0, 0, 0, 0.

Fig. 8 shows a Kaplan-Meier curve of the CRCAI_model in a 168 patient TCGA colorectal cancer cohort (training set used to develop the CRCAI_model). The clinical endpoint that was tested was the overall death (logrank p=0.0001; HR=4.6; 95% CI=2.2-9.7). The following supplementary lists indicate the number of patients at risk for the CRCAI_model classes analyzed (threshold=0.5), i.e., the patients at risk at any time interval +20 months are shown: Low risk: 117, 69, 37, 19, 9, 6, 3, 0, 0; High risk: 51, 28, 13, 10, 9, 4, 4, 1, 0.

Fig. 9 shows a Kaplan-Meier curve of the CRCAI_model in a 83 patient TCGA colorectal cancer cohort (testing set used to validate the CRCAI_model as developed on the 168 patient training set). The clinical endpoint that was tested was the overall death (logrank p=0.005; HR=5.9; 95% CI=1.7-20.2). The following supplementary lists indicate the number of patients at risk for the CRCAI_model classes analyzed (threshold=0.5), i.e., the patients at risk at any time interval +20 months are shown: Low risk: 66, 39, 10, 6, 5, 2, 2, 1, 0; High risk: 17, 9, 7, 2, 1, 0, 0, 0, 0.

Fig. 10 shows a Kaplan-Meier curve of the CRCAI&Clinical_model in a 168 patient TCGA colorectal cancer cohort (training set used to develop the CRCAI&Clinical_model). The clinical endpoint that was tested was the overall death (logrank p=0.0001; HR=4.0; 95% CI=2.0-8.0). The following supplementary lists indicate the number of patients at risk for the CRCAI&Clinical_model classes analyzed (threshold=0.5), i.e., the patients at risk at any time interval +20 months are shown: Low risk: 105, 59, 29, 15, 7, 5, 3, 0, 0; High risk: 63, 38, 21, 14, 11, 5, 4, 1, 0.

Fig. 11 shows a Kaplan-Meier curve of the CRCAI&Clinical_model in a 83 patient TCGA colorectal cancer cohort (testing set used to validate the CRCAI&Clinical_model as developed on the 168 patient training set). The clinical endpoint that was tested was the overall death (logrank p=0. 01; HR=5.5; 95% CI=1.4-21.8). The following supplementary lists indicate the number of patients at risk for the CRCAI&Clinical_model classes analyzed (threshold=0.5), i.e., the patients at risk at any time interval +20 months are shown: Low risk: 62, 40, 14, 7, 6, 2, 2, 1, 0; High risk: 21, 8, 3, 1, 0, 0, 0, 0, 0.

In the following, we show some additional results for a colorectal cancer cohort derived from the GSE41248 (www.ncbi.nlm.nih.gov/geo) data set:

Fig. 12 shows a Kaplan-Meier curve of the CRCAI_model in a colorectal cancer cohort derived from the GSE41248 data set. The model was trained on 2/3 of the samples from patients with non-metastasized (m0) and metastasized disease (m1) and then tested on the remaining 1/3 of the m0 and m1 samples. This trained model was then tested only on samples from patients with non-metastasized disease (m0) in order to have the same situation as with the TCGA colorectal cancer cohort. The clinical endpoint that was tested was the overall death (logrank p=0.001; HR=3.2; 95% CI=1.6-6.5). The following supplementary lists indicate the number of patients at risk for the CRCAI_model classes analyzed (threshold=0.5), i.e., the patients at risk at any time interval +20 months are shown: Low risk: 132, 123, 113, 97, 75, 50, 32, 14, 6, 3, 2, 0; High risk: 31, 27, 23, 22, 12, 8, 4, 0, 0, 0, 0, 0.

Fig. 13 shows a Kaplan-Meier curve of the CRCAI_model in a colorectal cancer cohort derived from the GSE41248 data set. The model was trained on 2/3 of the samples from patients with non-metastasized (m0) and metastasized disease (m1) and then tested on the remaining 1/3 of the m0 and m1 samples. This trained model was then tested only on samples from patients with non-metastasized disease (m0) in order to have the same situation as with the TCGA colorectal cancer cohort. The clinical endpoint that was tested was the cancer specific death (logrank p<0.0001; HR=12.9; 95% CI=3.9-43.0). The following supplementary lists indicate the number of patients at risk for the CRCAI_model classes analyzed (threshold=0.5), i.e., the patients at risk at any time interval +20 months are shown: Low risk: 94, 86, 80, 67, 51, 34, 18, 8, 3, 2, 2, 0; High risk: 23, 19, 16, 15, 7, 4, 3, 0, 0, 0, 0, 0.

Fig. 14 shows a Kaplan-Meier curve of the CRCAI&Clinical_model in a colorectal cancer cohort derived from the GSE41248 data set. The model was trained on 2/3 of the samples from patients with non-metastasized (m0) and metastasized disease (m1) and then tested on the remaining 1/3 of the m0 and m1 samples. This trained model was then tested only on samples from patients with non-metastasized disease (m0) in order to have the same situation as with the TCGA colorectal cancer cohort. The clinical endpoint that was tested was the overall death (logrank p=0.0002; HR=3.3; 95% CI=1.8-6.1). The following supplementary lists indicate the number of patients at risk for the CRCAI&Clinical_model classes analyzed (threshold=0.5), i.e., the patients at risk at any time interval +20 months are shown: Low risk: 120, 111, 103, 90, 71, 45, 30, 14, 6, 3, 2, 0; High risk: 43, 39, 33, 29, 16, 13, 6, 0, 0, 0, 0, 0.

Fig. 15 shows a Kaplan-Meier curve of the CRCAI&Clinical_model in a colorectal cancer cohort derived from the GSE41248 data set. The model was trained on 2/3 of the samples from patients with non-metastasized (m0) and metastasized disease (m1) and then tested on the remaining 1/3 of the m0 and m1 samples. This trained model was then tested only on samples from patients with non-metastasized disease (m0) in order to have the same situation as with the TCGA colorectal cancer cohort. The clinical endpoint that was tested was the cancer specific death (logrank p=0.0001; HR=16.8; 95% CI=6.0-47.1). The following supplementary lists indicate the number of patients at risk for the CRCAI&Clinical_model classes analyzed (threshold=0.5), i.e., the patients at risk at any time interval +20 months are shown: Low risk: 84, 76, 71, 61, 48, 31, 17, 8, 3, 2, 2, 0; High risk: 33, 29, 25, 21, 10, 7, 4, 0, 0, 0, 0, 0.

The Kaplan-Meier survival curve analysis as shown in Figs. 2 to 15 demonstrates the presence of different patient risk groups. The risk group of a patient is determined by the probability to suffer from the respective clinical endpoint (overall death) as calculated by the respective risk model as shown in the figures. Depending on the predicted risk of a patient (i.e., depending on in which risk group the patient may belong) to die from colorectal cancer different types of interventions might be indicated. In the low risk group (probability <0.5) standard of care (SOC) delivers acceptable long-term oncological control. This is definitely not the case for the patient group with a risk >0.5 to experience any of the relevant outcomes. In this patient group escalation of intervention or application of alternative treatment needs to happen. Alternative options for treatment escalation are adjuvant therapies with radiation or cytotoxic drugs or alternative therapies like immunotherapies (e.g., atezolizumab; pembrolizumab; nivolumab; avelumab; durvalumab) or other experimental therapies.

**Discussion**

The effectiveness of therapies for colorectal cancers is limited, resulting in disease progression and ultimately death of patients, especially for those at high risk of recurrence of disease after primary intervention. The prediction of the therapy outcome is very challenging as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the treatment response in order to increase the success rate of colorectal cancer therapies.

We have identified molecules of which expression shows a significant relation to mortality after primer therapy of colorectal cancers and therefore are expected to improve the prediction of the effectiveness of secondary treatments. This can be achieved by 1) standard of care for those patients with low risk of progressive disease correlated with death from cancer and/or 2) guiding patients with high risk of progressive disease and subsequent death from cancer to an alternative, potentially more effective form of treatment as compared to currently applied standard of care. This would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting an outcome of a colorectal cancer subject, comprising determining or receiving the result of a determination of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from the subject, and/or determining or receiving the result of a determination of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from the subject, and/or determining or receiving the result of a determination of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from the subject, determining the prediction of outcome based on the first gene expression profile(s), or on the second gene expression profile(s), or on the third gene expression profile(s), or on the first, second, and third gene expression profile(s), and, optionally, providing the prediction to a medical caregiver or the subj ect.

In some embodiments, the prediction of the outcome can also be determined based on the first gene expression profile(s) and the second genes expression profile(s). In some embodiments, the prediction of the outcome can also be determined based on the first gene expression profile(s) and the third genes expression profile(s). In some embodiments, the prediction of the outcome can also be determined based on the second gene expression profile(s) and the third genes expression profile(s).

**The attached Sequence Listing, entitled 2020PF00762_Sequence Listing_ST25 is incorporated herein by reference, in its entirety.**

## Claims

**1.** A method of predicting an outcome of a colorectal cancer subject, comprising:

- determining or receiving the result of a determination of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from the subject, and/or
- determining or receiving the result of a determination of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from the subject, and/or
- determining or receiving the result of a determination of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from the subject,
- determining the prediction of the outcome based on the first gene expression profile(s), or on the second gene expression profile(s), or on the third gene expression profile(s), or on the first, second, and third gene expression profile(s), and
- optionally, providing the prediction to a medical caregiver or the subject.

**2.** The method as defined in claim 1, wherein:

- the one or more immune defense response genes comprise three or more, preferably, six or more, more preferably, nine or more, most preferably, all of the immune defense genes, and/or
- the one or more T-Cell receptor signaling genes comprise three or more, preferably, six or more, more preferably, nine or more, most preferably, all of the T-Cell receptor signaling genes, and/or
- the one or more PDE4D7 correlated genes comprise three or more, preferably, six or more, most preferably, all of the PDE4D7 correlated genes.

**3.** The method as defined in claim 1 or 2, wherein the determining of the prediction of the outcome comprises:

- combining the first gene expression profiles for two or more, for example, 2, 3, 4, 5, 6, 7, 8, 9, 10, 11, 12, 13 or all, of the immune defense response genes with a regression function that had been derived from a population of colorectal cancer subjects, and/or

- combining the second gene expression profiles for two or more, for example, 2, 3, 4, 5, 6, 7, 8, 9, 10, 11, 12, 13, 14, 15, 16 or all, of the T-Cell receptor signaling genes with a regression function that had been derived from a population of colorectal cancer subjects, and/or
- combining the third gene expression profiles for two or more, for example, 2, 3, 4, 5, 6, 7 or all, of the PDE4D7 correlated genes with a regression function that had been derived from a population of colorectal cancer subjects.

**4.** The method as defined in claim 3, wherein the determining of the prediction of the outcome further comprises combining the combination of the first gene expression profiles, the combination of the second gene expression profiles, and the combination of the third gene expression profiles with a regression function that had been derived from a population of colorectal cancer subjects.

**5.** The method as defined in any of claims 1 to 4, wherein the determining of the outcome is further based on one or more clinical parameters obtained from the subject.

**6.** The method as defined in claim 4, wherein the clinical parameters comprise one or more of: (i) T stage attribute (T1, T2, T3, or T4); (ii) N stage attribute (N0, N1, or N2), and; (iii) M stage attribute (M0, M1).

**7.** The method as defined in claim 5 or 6, wherein the determining of the prediction of the outcome comprises combining one or more of: (i) the first gene expression profile(s) for the one or more immune defense response genes; (ii) the second gene expression profile(s) for the one or more T-Cell receptor signaling genes; (iii) the third gene expression profile(s) for the one or more PDE4D7 correlated genes, and; (iv) the combination of the first gene expression profiles, the combination of the second gene expression profiles, and the combination of the third gene expression profiles, and the one or more clinical parameters obtained from the subject with a regression function that had been derived from a population of colorectal cancer subjects.

**8.** The method as defined in any of claims 1 to 7, wherein the biological sample(s) is/are obtained from the subject before the start of the therapy.

**9.** The method as defined in any of claims 1 to 8, wherein the therapy is surgery, radiotherapy, cytotoxic chemotherapy (CTX), short- or long-course chemo-radiation therapy (CRT), immunotherapy, or any combination thereof.

**10.** The method as defined in any of claims 1 to 9, wherein the prediction of the therapy response is unlikely or likely for the effectiveness of the therapy, wherein a therapy is recommended based on the prediction and, if the prediction is unlikely, the recommended therapy comprises one or more of: (i) therapy provided earlier than is the standard; (ii) radiotherapy with an increased effective dose; (iii) an adjuvant therapy, such as chemotherapy; (iv) long-course CRT (chemo-radiation therapy), and (iv); an alternative therapy, such as immunotherapy.

**11.** An apparatus for predicting an outcome of a colorectal cancer subject, comprising:

- an input adapted to receive data indicative of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from the subject, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from the subject, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from the subject,
- a processor adapted to determine the prediction of outcome based on the first gene expression profile(s), or on the second gene expression profile(s), or on the third gene expression profile(s), or on the first, second, and third gene expression profile(s), and
- optionally, a providing unit adapted to provide the prediction to a medical caregiver or the subject.

**12.** A computer program product comprising instructions which, when the program is executed by a computer, cause the computer to carry out a method comprising:

- receiving data indicative of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from a colorectal cancer subject, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from a colorectal cancer subject, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from a colorectal cancer subject,
- determining a prediction of an outcome of the subject based on the first gene expression profile(s), or on the second gene expression profile(s), or on the third gene expression profile(s), or on the first, second, and third gene expression profile(s), and
- optionally, providing the prediction to a medical caregiver or the subject.

**13.** A diagnostic kit, comprising:

- at least one primer and/or probe for determining a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from a subject, and/or
- at least one primer and/or probe for determining a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in a biological sample obtained from a subject, and/or
- at least one primer and/or probe for determining a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a biological sample obtained from a subject, and
- optionally, an apparatus as defined in claim 11 or a computer program product as defined in claim 12.

**14.** Use of the kit as defined in claim 13.

**15.** The use as defined in claim 14 in a method of predicting an outcome of a colorectal cancer subject.

**16.** A method, comprising:

- receiving one or more biological sample(s) obtained from a colorectal cancer subj ect,
- using the kit as defined in claim 13 to determine a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from the subject, and/or
- using the kit as defined in claim 13 to determine a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in a biological sample obtained from the subject, and/or
- using the kit as defined in claim 13 to determine a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a biological sample obtained from the subj ect.

**17.** Use of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a method of predicting an outcome of a colorectal cancer subject, comprising:

- determining the prediction of the outcome based on the first gene expression profile(s), or on the second gene expression profile(s), or on the third gene expression profile(s), or on the first, second, and third gene expression profile(s), and
- optionally, providing the prediction to a medical caregiver or the subject.

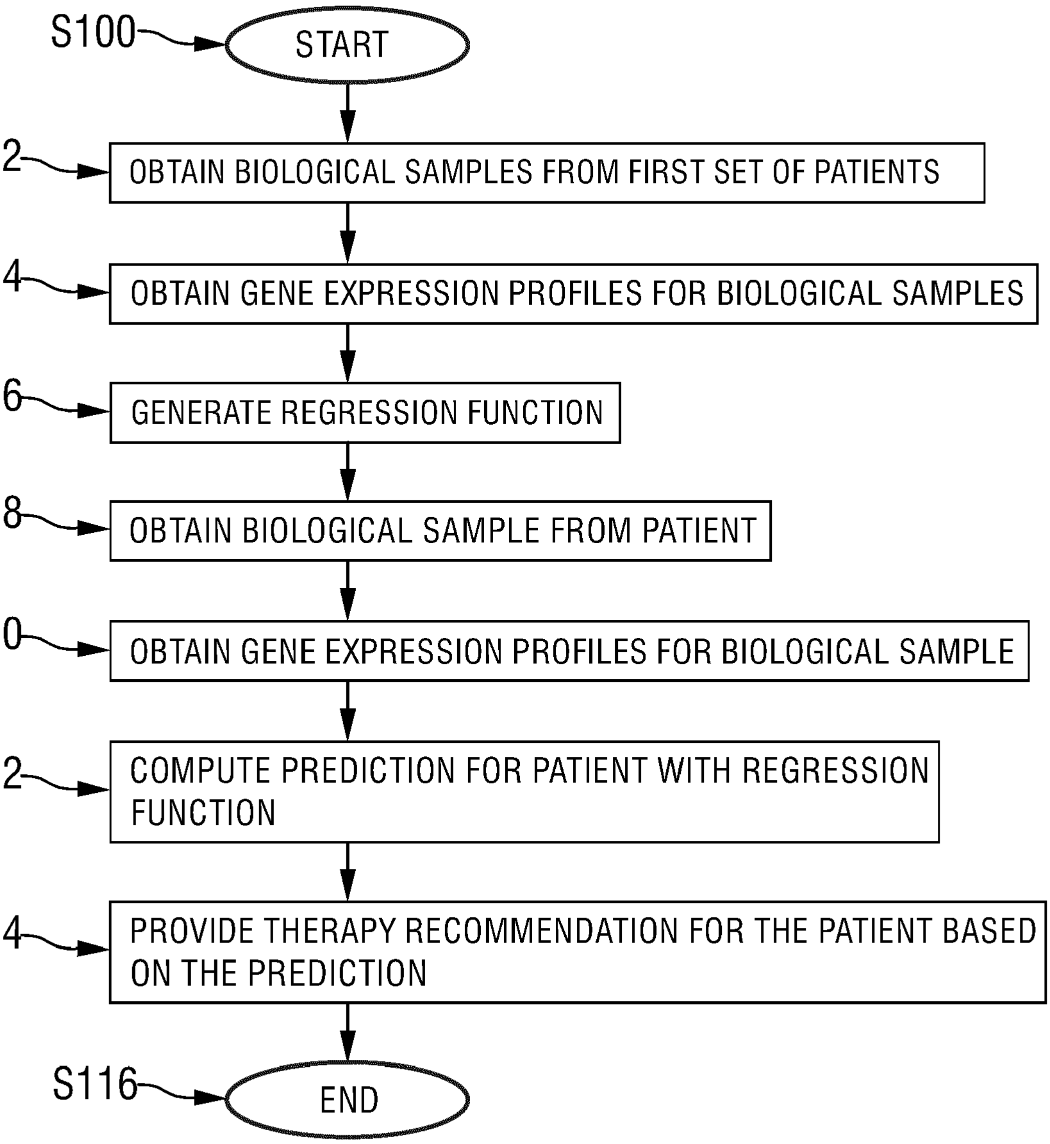
S100
START
S102
OBTAIN BIOLOGICAL SAMPLES FROM FIRST SET OF PATIENTS
S104
OBTAIN GENE EXPRESSION PROFILES FOR BIOLOGICAL SAMPLES
S106
GENERATE REGRESSION FUNCTION
S108
OBTAIN BIOLOGICAL SAMPLE FROM PATIENT
S110
OBTAIN GENE EXPRESSION PROFILES FOR BIOLOGICAL SAMPLE
S112
COMPUTE PREDICTION FOR PATIENT WITH REGRESSION FUNCTION
S114
PROVIDE THERAPY RECOMMENDATION FOR THE PATIENT BASED ON THE PREDICTION
S116
END

FIG. 1

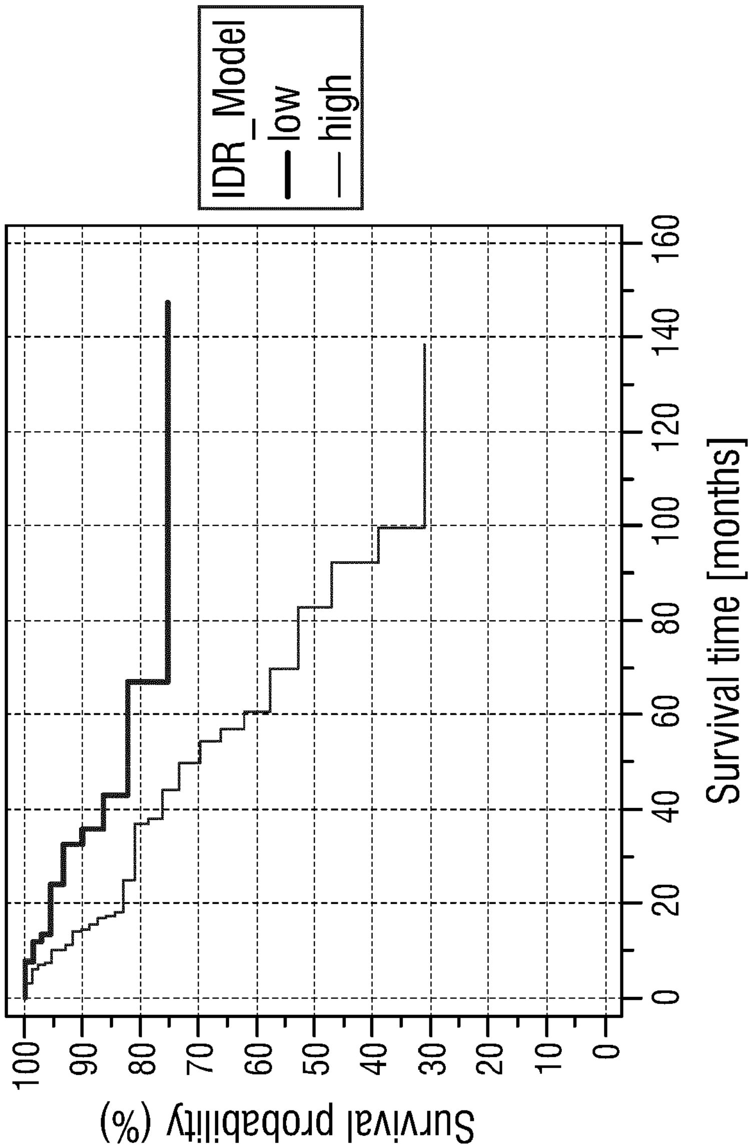
IDR_Model
low
high
Survival probability (%)
Survival time [months]
0
10
20
30
40
50
60
70
80
90
100
0
20
40
60
80
100
120
140
160

FIG. 2

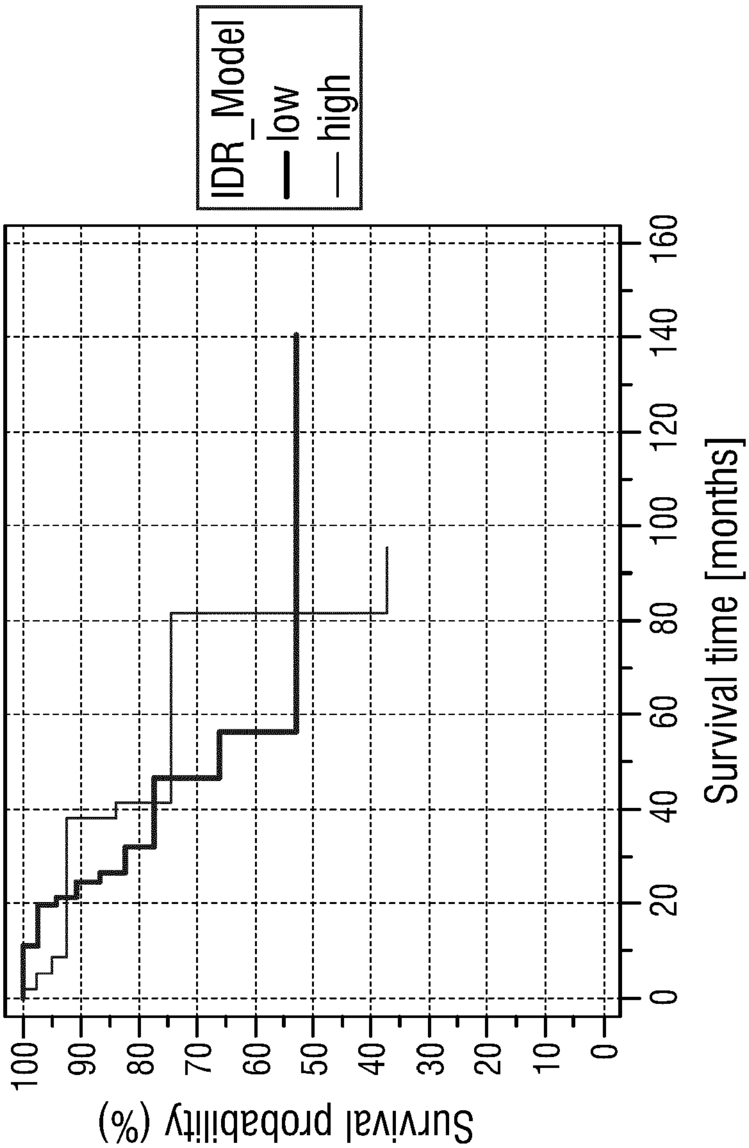
IDR_Model
low
high
Survival probability (%)
100
90
80
70
60
50
40
30
20
10
0
20
40
60
80
100
120
140
160
Survival time [months]

FIG. 3

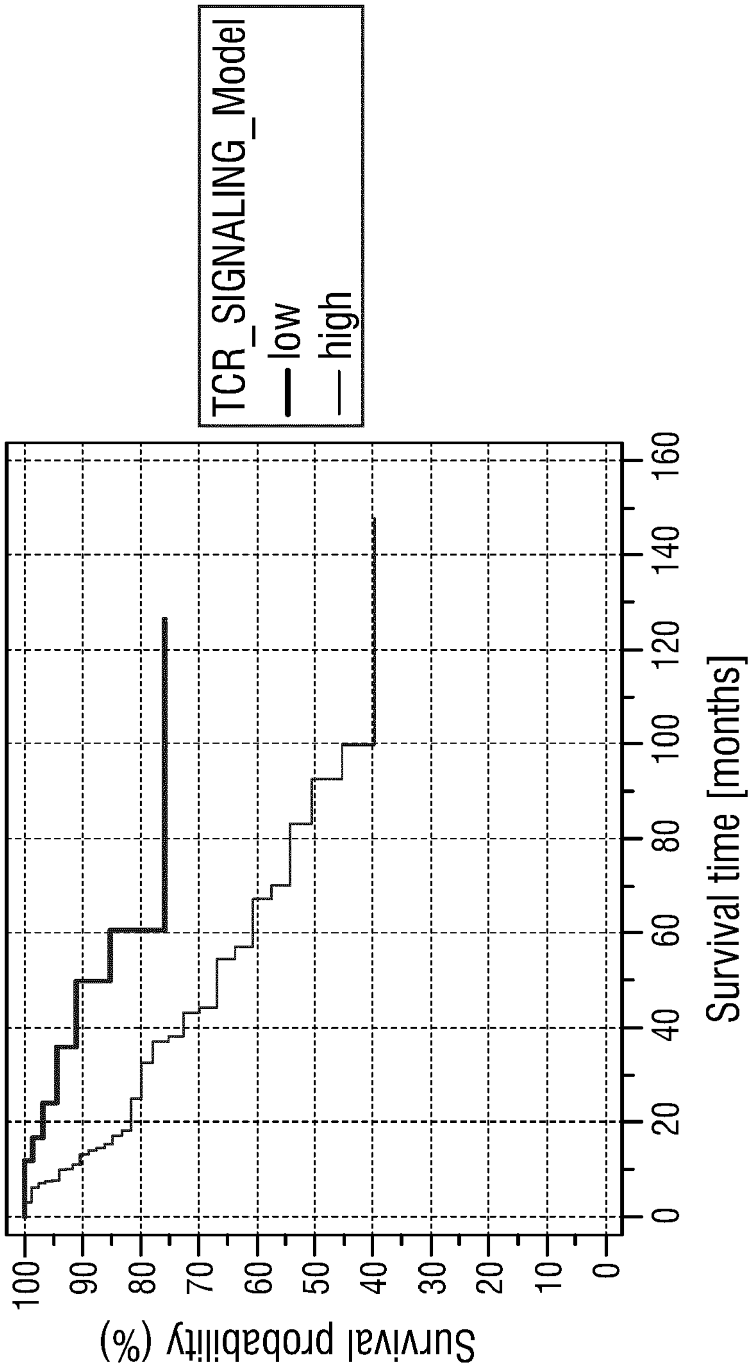
TCR_SIGNALING_Model
low
high
Survival probability (%)
100
90
80
70
60
50
40
30
20
10
0
Survival time [months]
0
20
40
60
80
100
120
140
160

FIG. 4

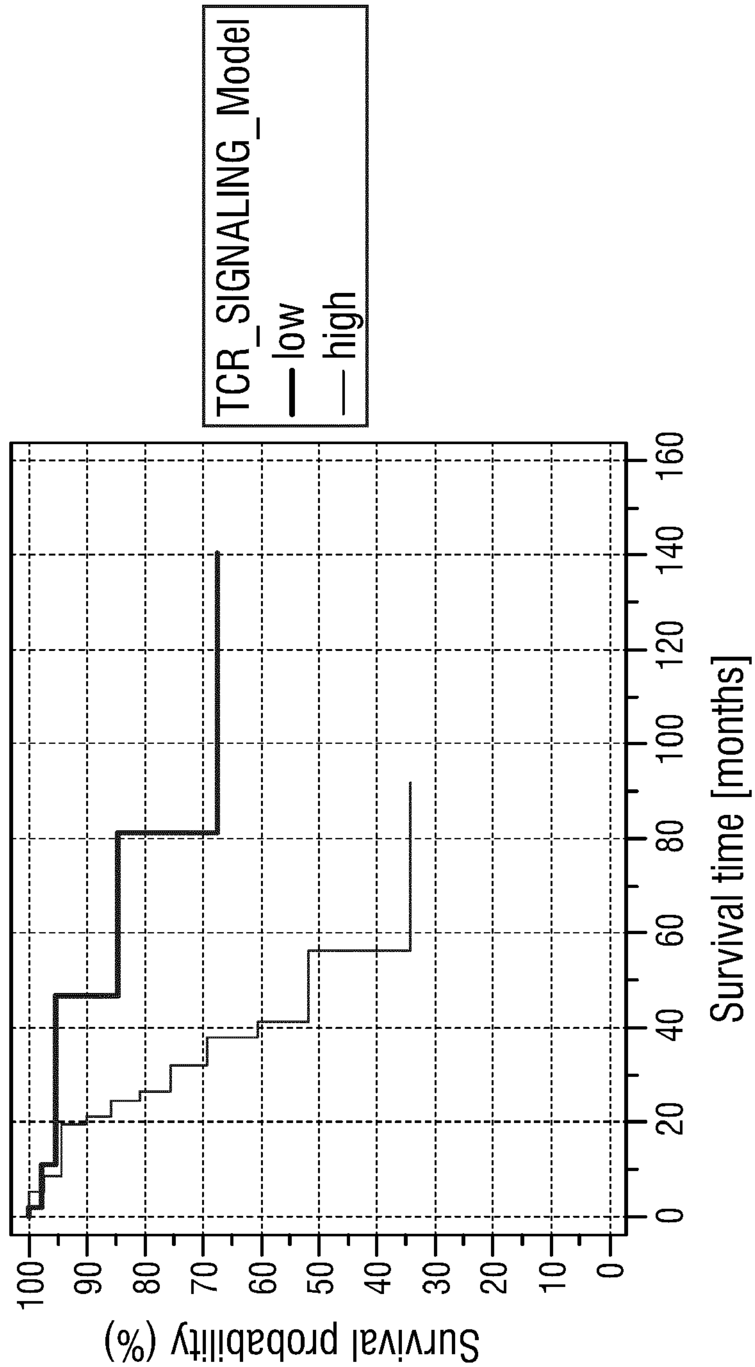
TCR_SIGNALING_Model
low
high
Survival probability (%)
100
90
80
70
60
50
40
30
20
10
0
0
20
40
60
80
100
120
140
160
Survival time [months]

FIG. 5

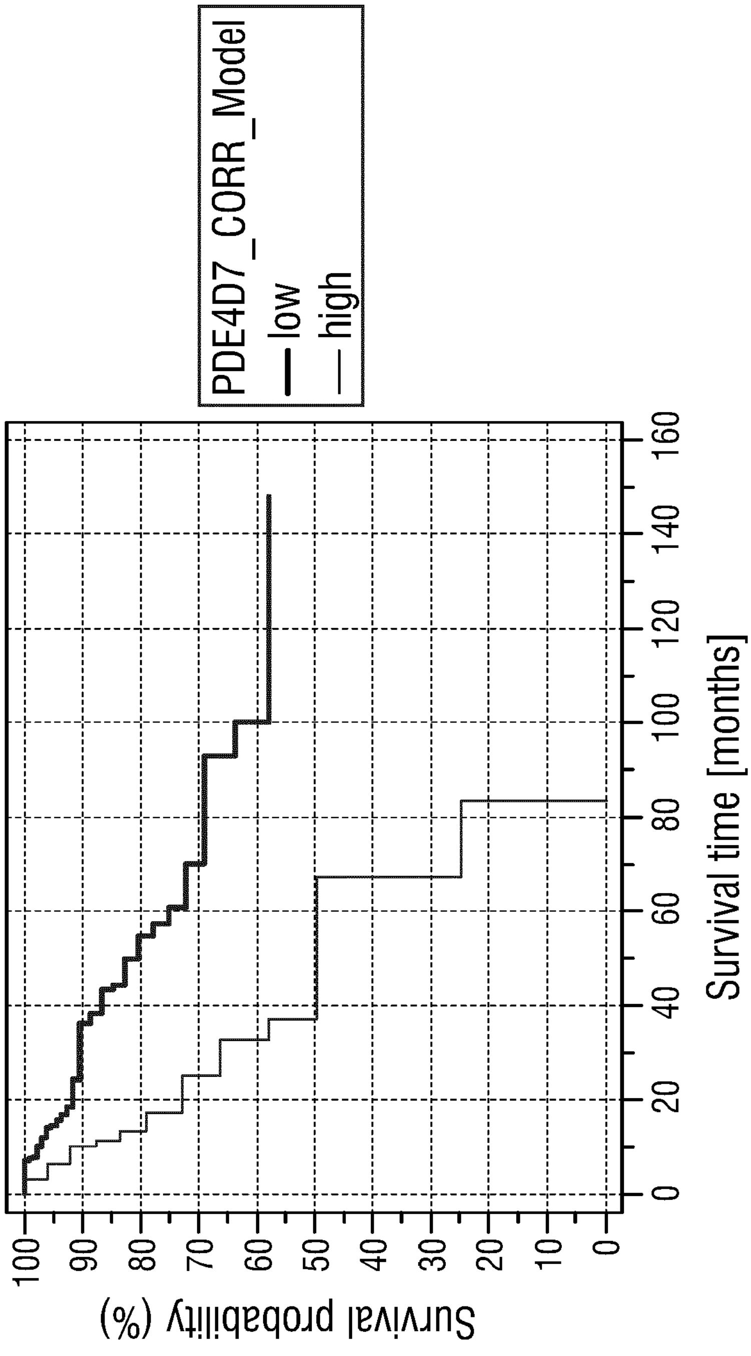
PDE4D7_CORR_Model
low
high
Survival probability (%)
100
90
80
70
60
50
40
30
20
10
0
Survival time [months]
0
20
40
60
80
100
120
140
160

FIG. 6

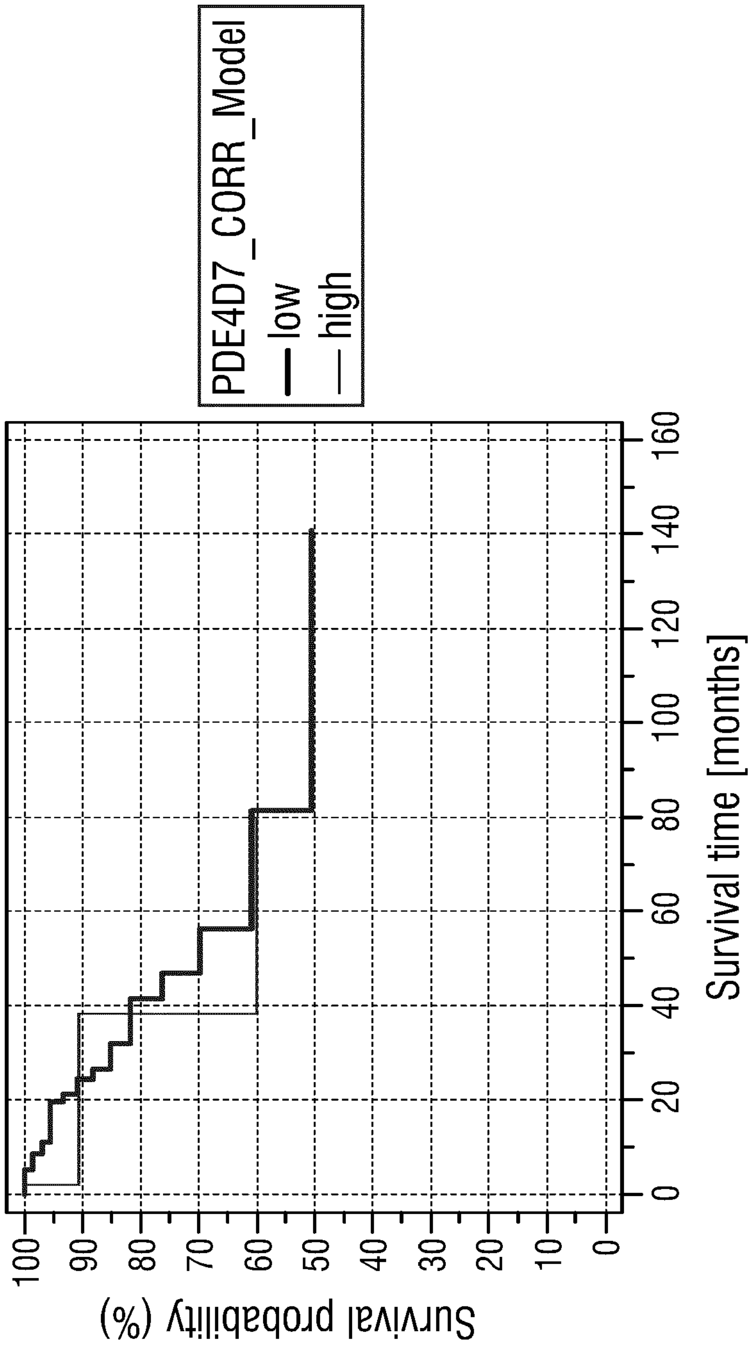
PDE4D7_CORR_Model
low
high
Survival probability (%)
100
90
80
70
60
50
40
30
20
10
0
Survival time [months]
0
20
40
60
80
100
120
140
160

FIG. 7

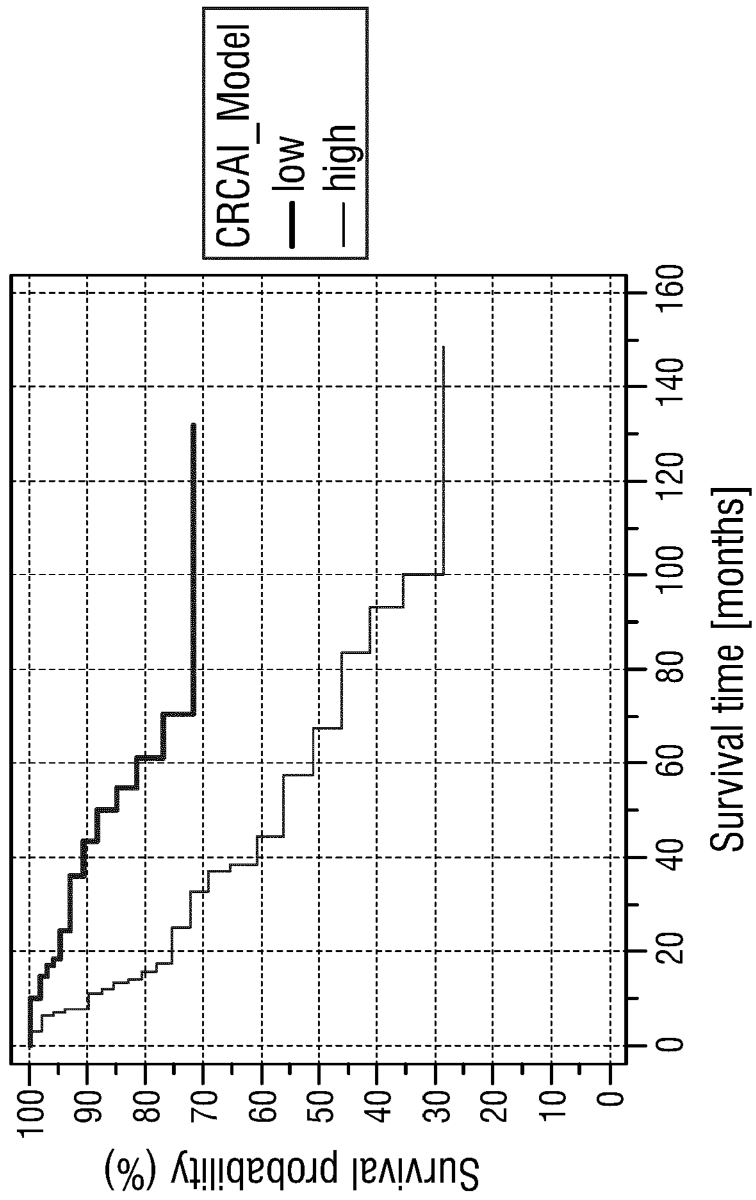
CRCAI_Model
low
high
Survival probability (%)
100
90
80
70
60
50
40
30
20
10
0
Survival time [months]
0
20
40
60
80
100
120
140
160

FIG. 8

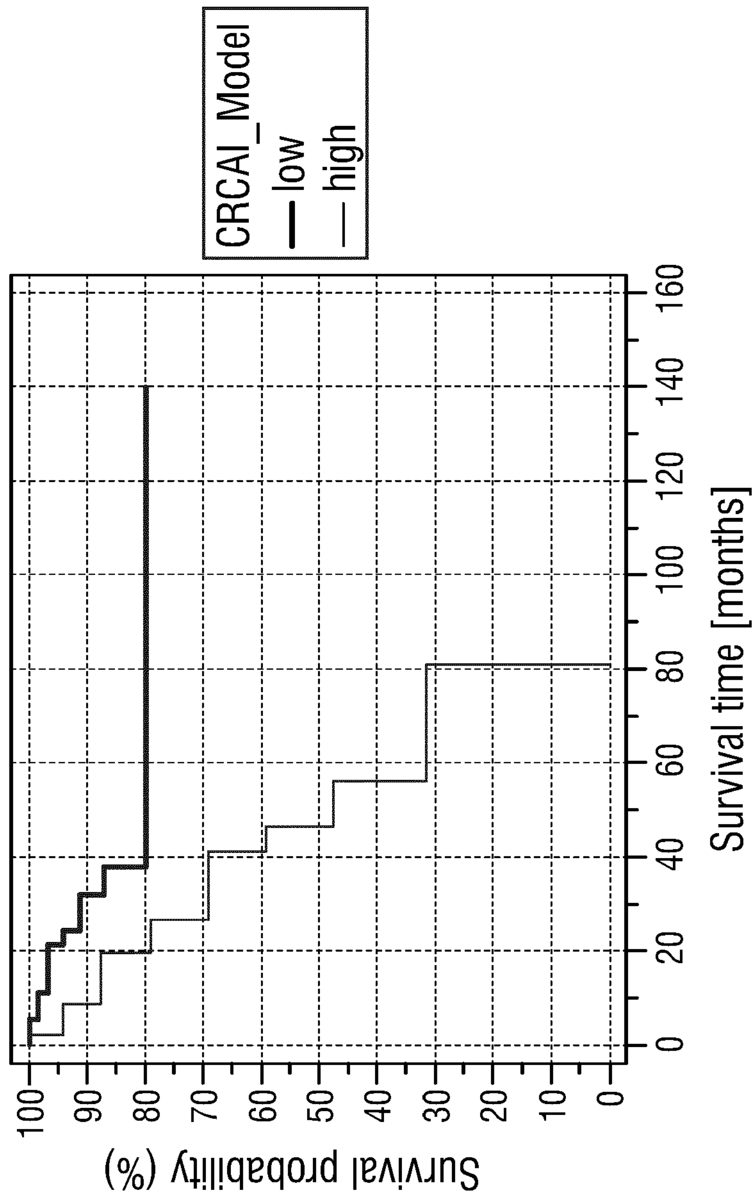

CRCAI_Model
low
high
Survival probability (%)
100
90
80
70
60
50
40
30
20
10
0
0
20
40
60
80
100
120
140
160
Survival time [months]


FIG. 9

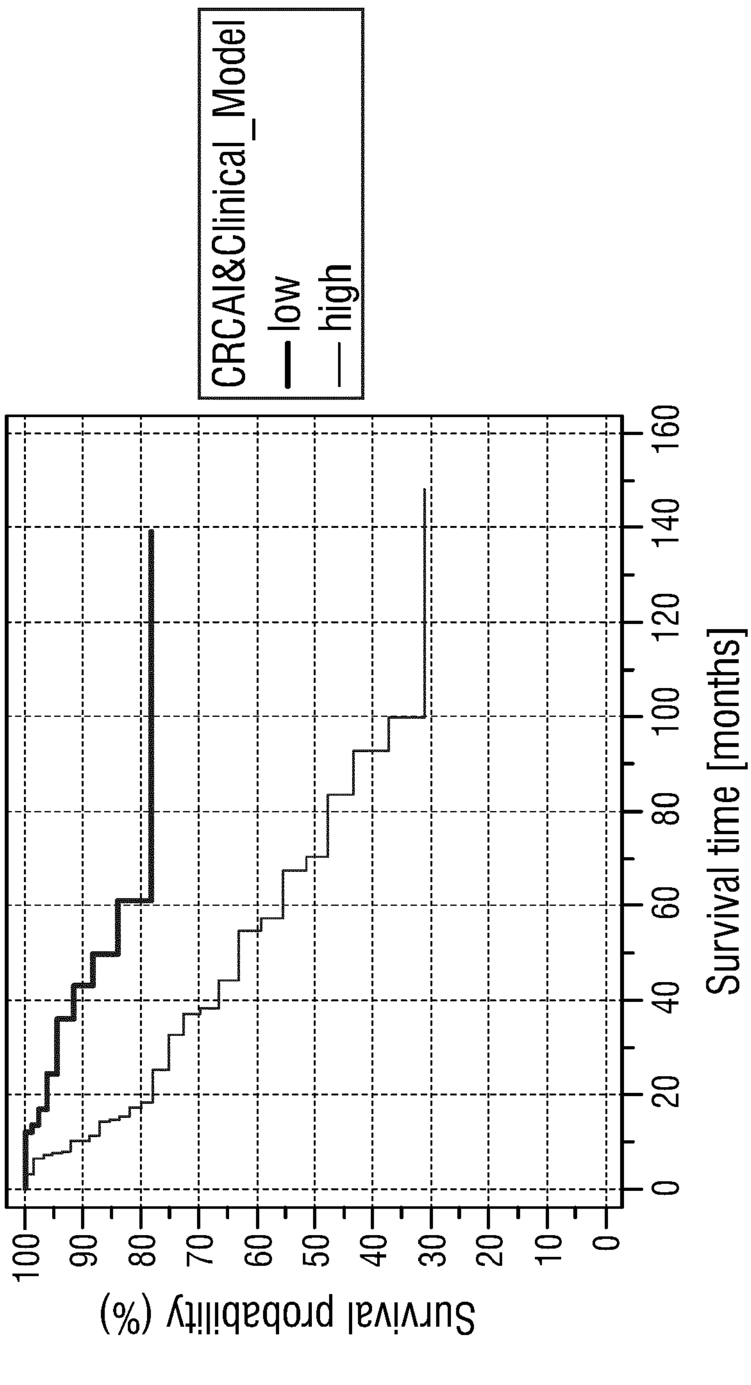
CRCAI&Clinical_Model
low
high
Survival probability (%)
100
90
80
70
60
50
40
30
20
10
0
Survival time [months]
0
20
40
60
80
100
120
140
160

FIG. 10

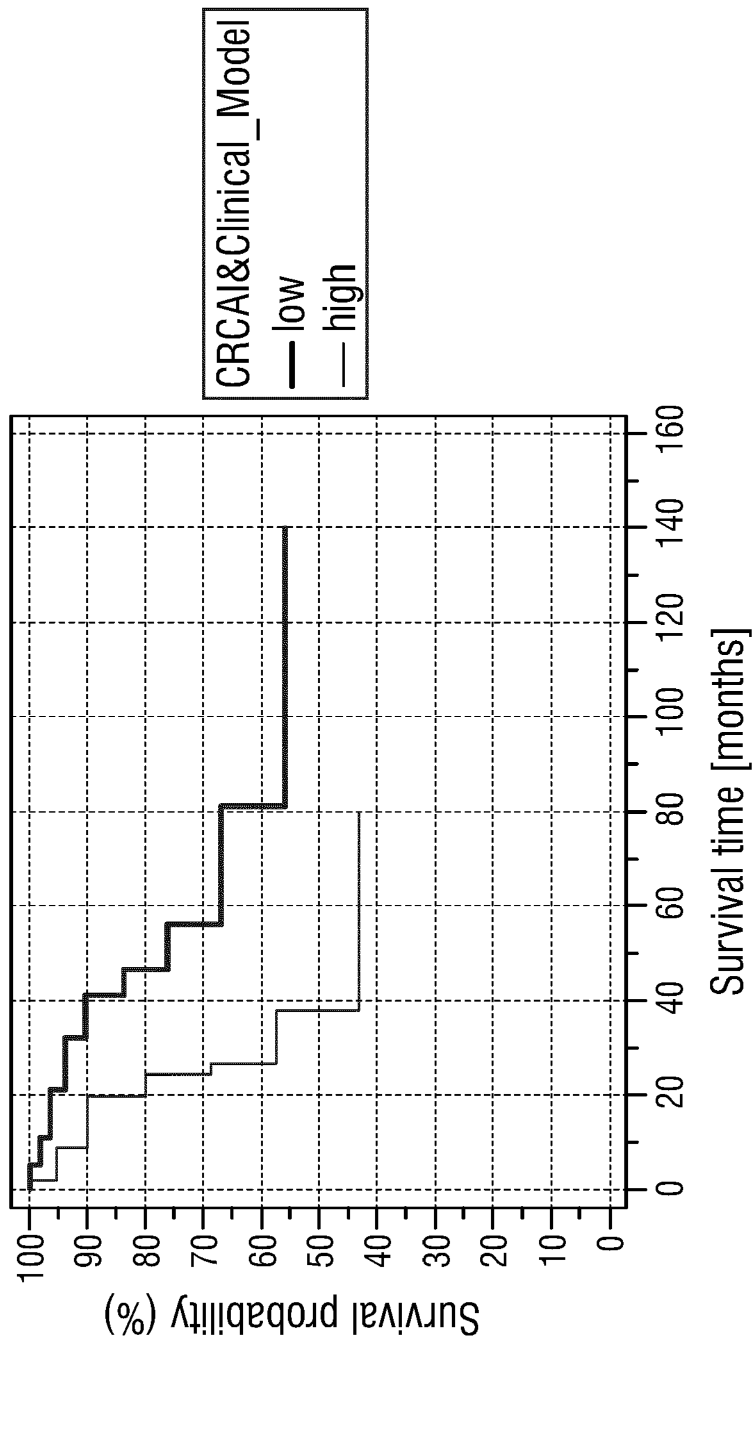
CRCAI&Clinical_Model
low
high
Survival probability (%)
100
90
80
70
60
50
40
30
20
10
0
0
20
40
60
80
100
120
140
160
Survival time [months]

FIG. 11

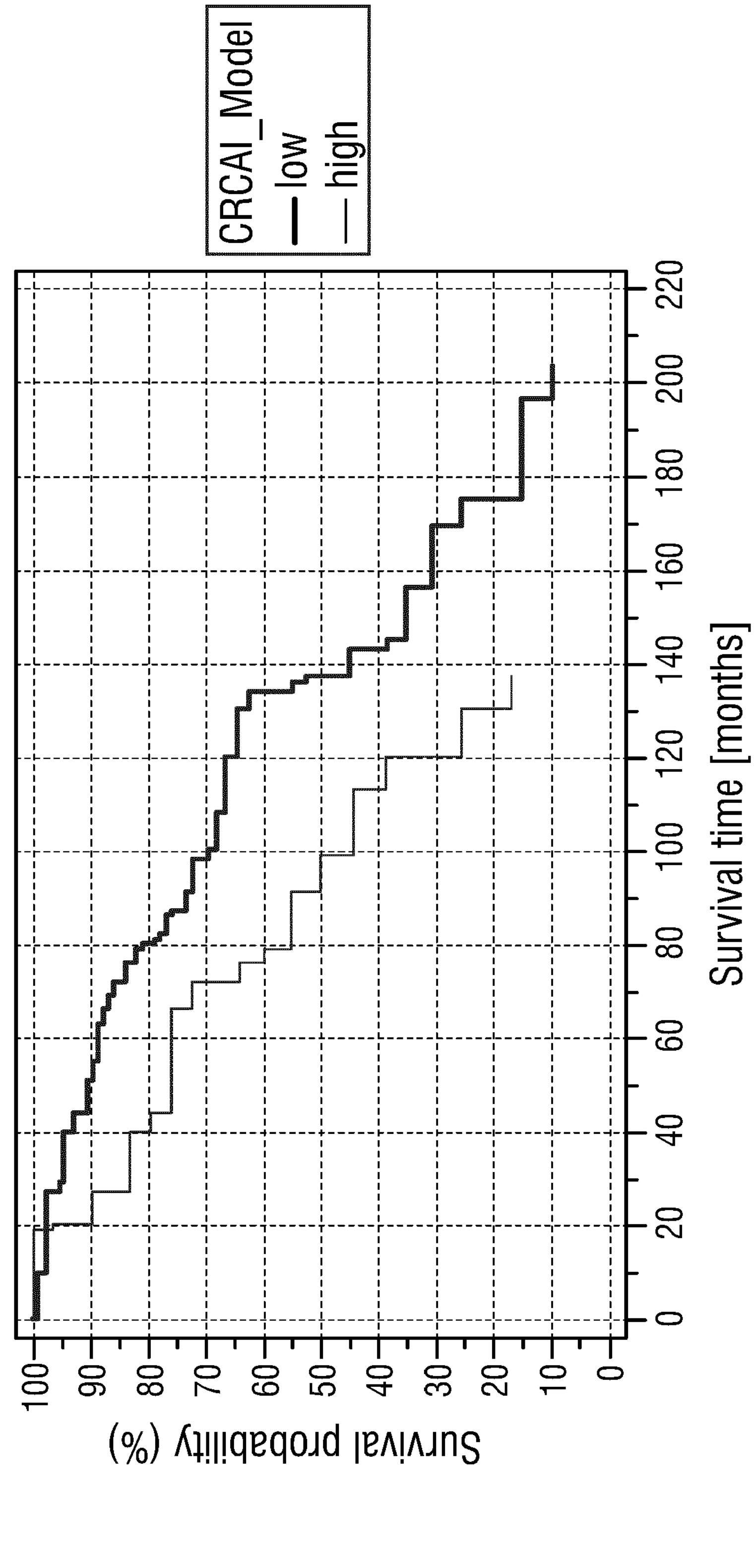
CRCAI_Model
low
high
Survival probability (%)
100
90
80
70
60
50
40
30
20
10
0
0
20
40
60
80
100
120
140
160
180
200
220
Survival time [months]

FIG. 12

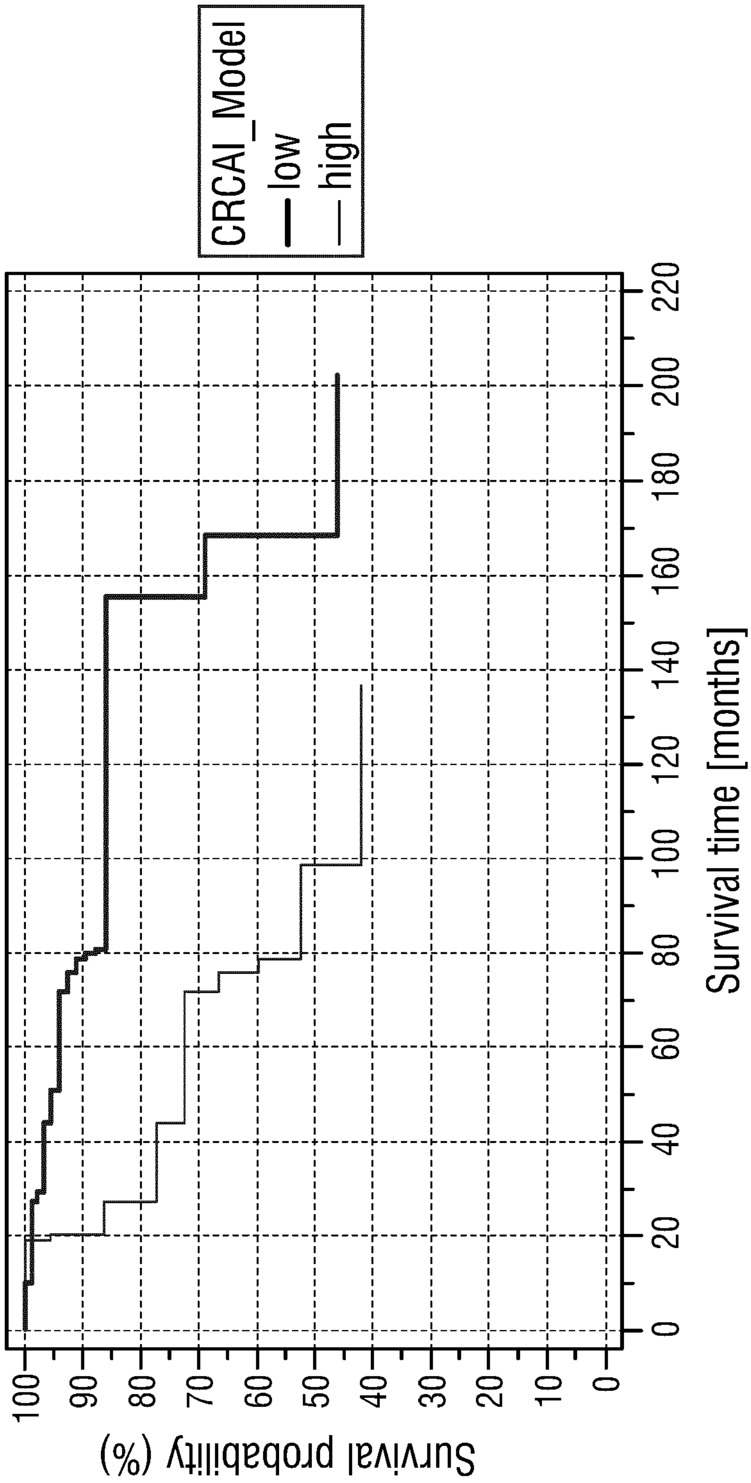
CRCAI_Model
low
high
Survival probability (%)
Survival time [months]
100
90
80
70
60
50
40
30
20
10
0
0
20
40
60
80
100
120
140
160
180
200
220

FIG. 13

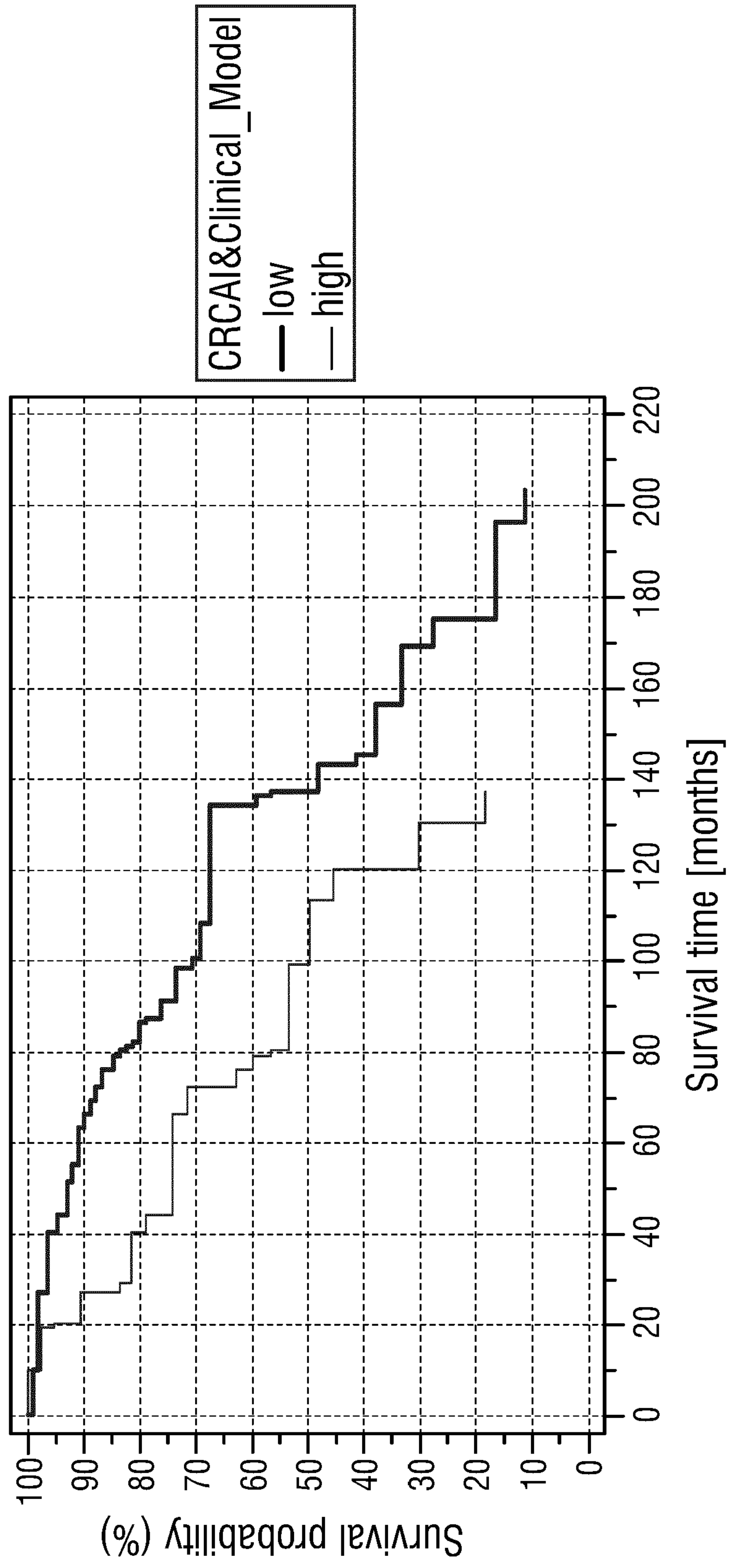
CRCAI&Clinical_Model
low
high
Survival probability (%)
100
90
80
70
60
50
40
30
20
10
0
0
20
40
60
80
100
120
140
160
180
200
220
Survival time [months]

FIG. 14

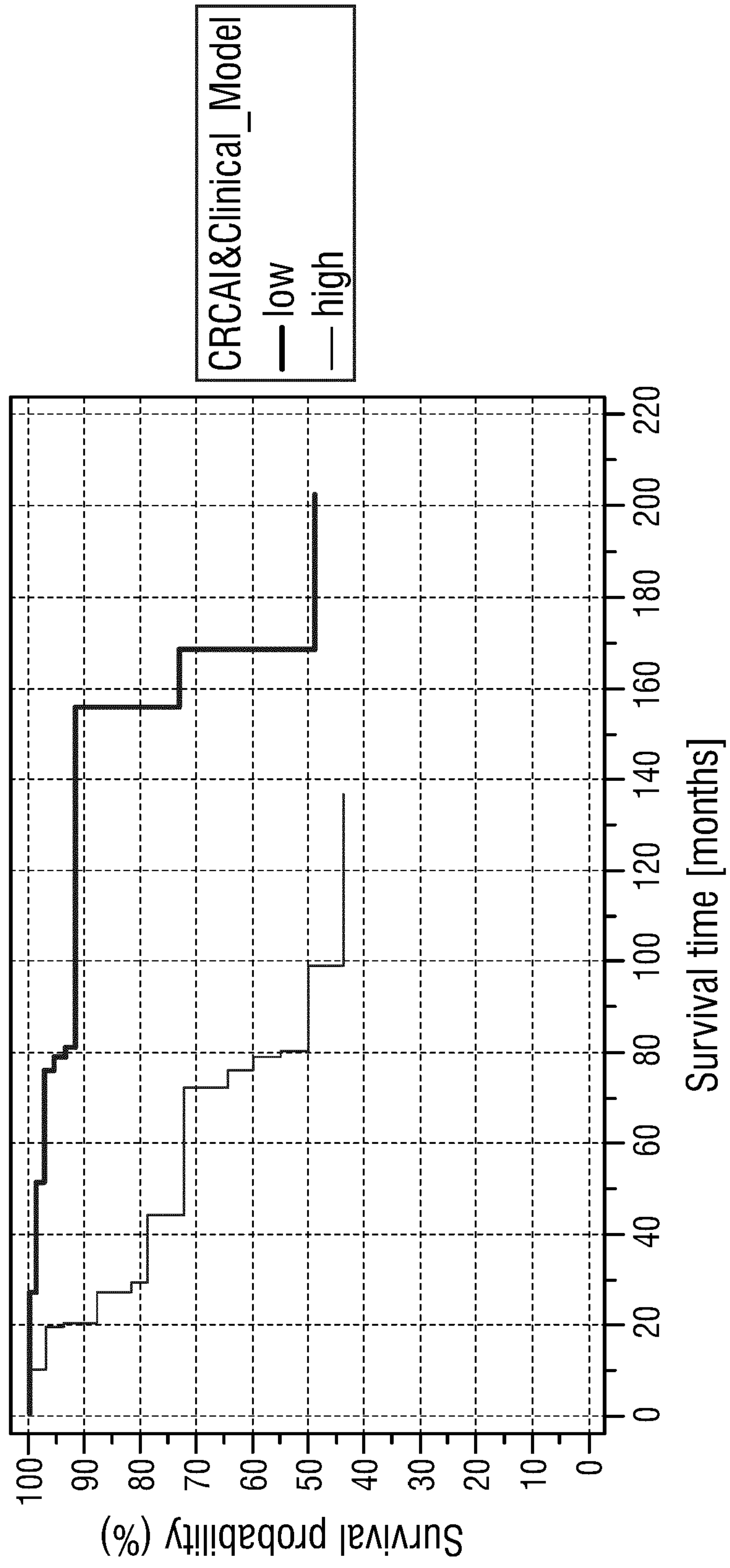
CRCAI&Clinical_Model
low
high
Survival probability (%)
100
90
80
70
60
50
40
30
20
10
0
Survival time [months]
0
20
40
60
80
100
120
140
160
180
200
220

FIG. 15

Europäisches Patentamt
European Patent Office
Office européen des brevets

# EUROPEAN SEARCH REPORT

Application Number
EP 21 15 1054

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | EP 1 777 523 A1 (INST NAT SANTE RECH MED [FR]) 25 April 2007 (2007-04-25)<br>* claims 1-13 *<br>* examples 1-3 *<br>* paragraphs [0185] - [0194] *<br>----- | 1-3,5,6, 8-17<br>1-17 | INV.<br>C12Q1/6886 |
| X | WO 2007/082099 A2 (GENOMIC HEALTH INC [US]; NASBP FOUNDATION INC [US] ET AL.) 19 July 2007 (2007-07-19)<br>* claims 1-19,28-44 *<br>* paragraphs [0037], [0052] - [0058], [0130], [0131] *<br>----- | 1,2,5,6, 8-17 | |
| X | HE LIANGMEI ET AL: "DNA sensors, crucial receptors to resist pathogens, are deregulated in colorectal cancer and associated with initiation and progression of the disease",<br>JOURNAL OF CANCER,<br>vol. 11, no. 4,<br>1 January 2020 (2020-01-01), pages 893-905, XP055816281,<br>AU<br>ISSN: 1837-9664, DOI: 10.7150/jca.34188<br>Retrieved from the Internet:<br>URL:https://www.jcancer.org/v11p0893.pdf><br>* the whole document *<br>----- | 1,2,8, 11-17 | TECHNICAL FIELDS SEARCHED (IPC)<br>C12Q |
| X | HE LIANGMEI ET AL: "The expression profile of RNA sensors in colorectal cancer and its correlation with cancer stages",<br>TRANSLATIONAL CANCER RESEARCH,<br>vol. 8, no. 4, 1 August 2019 (2019-08-01), pages 1351-1363, XP055816361,<br>ISSN: 2218-676X, DOI: 10.21037/tcr.2019.07.45<br>* the whole document *<br>-----<br>-/-- | 1,2,8, 11-17 | |

The present search report has been drawn up for all claims

2

| Place of search | Date of completion of the search | Examiner |
|---|---|---|
| The Hague | 22 June 2021 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 15 1054

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHEELU VARGHESE ET AL: "Site-Specific Gene Expression Profiles and Novel Molecular Prognostic Factors in Patients with Lower Gastrointestinal Adenocarcinoma Diffusely Metastatic to Liver or Peritoneum", ANNALS OF SURGICAL ONCOLOGY, SPRINGER-VERLAG, NE, vol. 14, no. 12, 25 September 2007 (2007-09-25), pages 3460-3471, XP019547540, ISSN: 1534-4681, DOI: 10.1245/S10434-007-9557-7 * the whole document * ----- | 1,2, 8-10, 13-17 | |
| X | WO 2016/109546 A2 (GENENTECH INC [US]) 7 July 2016 (2016-07-07) * claims 1-67 * ----- | 1-3,8-17 | |
| X | EP 3 636 779 A1 (KONINKLIJKE PHILIPS NV [NL]; UNIV GLASGOW COURT [GB]) 15 April 2020 (2020-04-15) | 13,14 | TECHNICAL FIELDS SEARCHED (IPC) |
| Y | * claims 1-13 * ----- | 1-17 | |
| X | WO 2019/028285 A2 (GENOMEDX INC [US]; UNIV MICHIGAN REGENTS [US]) 7 February 2019 (2019-02-07) * paragraphs [0010], [0108] - [0112] * ----- | 13,14 | |

The present search report has been drawn up for all claims

2

| Place of search | Date of completion of the search | Examiner |
|---|---|---|
| The Hague | 22 June 2021 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 21 15 1054

This annex lists the patent family members relating to the patent documents cited in the above-mentioned European search report. The members are as contained in the European Patent Office EDP file on
The European Patent Office is in no way liable for these particulars which are merely given for the purpose of information.

22-06-2021

| Patent document cited in search report | Publication date | Patent family member(s) | Publication date |
|---|---|---|---|
| EP 1777523 A1 | 25-04-2007 | AT 523786 T | 15-09-2011 |
| | | DK 2420840 T3 | 08-02-2016 |
| | | DK 2733493 T3 | 19-09-2016 |
| | | EP 1777523 A1 | 25-04-2007 |
| | | EP 1943520 A1 | 16-07-2008 |
| | | EP 2241891 A1 | 20-10-2010 |
| | | EP 2420835 A2 | 22-02-2012 |
| | | EP 2420836 A2 | 22-02-2012 |
| | | EP 2420837 A2 | 22-02-2012 |
| | | EP 2420838 A2 | 22-02-2012 |
| | | EP 2420839 A2 | 22-02-2012 |
| | | EP 2420840 A2 | 22-02-2012 |
| | | EP 2420841 A2 | 22-02-2012 |
| | | EP 2733493 A1 | 21-05-2014 |
| | | EP 3088894 A1 | 02-11-2016 |
| | | EP 3364191 A1 | 22-08-2018 |
| | | EP 3715849 A1 | 30-09-2020 |
| | | ES 2374783 T3 | 22-02-2012 |
| | | ES 2398907 T3 | 22-03-2013 |
| | | ES 2469102 T3 | 17-06-2014 |
| | | ES 2469103 T3 | 17-06-2014 |
| | | ES 2470165 T3 | 23-06-2014 |
| | | ES 2480690 T3 | 28-07-2014 |
| | | ES 2480691 T3 | 28-07-2014 |
| | | ES 2531659 T3 | 18-03-2015 |
| | | ES 2561527 T3 | 26-02-2016 |
| | | ES 2592169 T3 | 28-11-2016 |
| | | ES 2674560 T3 | 02-07-2018 |
| | | ES 2785033 T3 | 05-10-2020 |
| | | JP 5256038 B2 | 07-08-2013 |
| | | JP 5789216 B2 | 07-10-2015 |
| | | JP 5921602 B2 | 24-05-2016 |
| | | JP 2009515148 A | 09-04-2009 |
| | | JP 2012154944 A | 16-08-2012 |
| | | JP 2014158500 A | 04-09-2014 |
| | | PL 2733493 T3 | 30-12-2016 |
| | | US 2009215053 A1 | 27-08-2009 |
| | | US 2014057257 A1 | 27-02-2014 |
| | | US 2015268245 A1 | 24-09-2015 |
| | | US 2019178892 A1 | 13-06-2019 |
| | | WO 2007045996 A1 | 26-04-2007 |
| WO 2007082099 A2 | 19-07-2007 | AU 2007204826 A1 | 19-07-2007 |
| | | BR PI0706511 A2 | 29-03-2011 |
| | | CA 2636984 A1 | 19-07-2007 |
| | | DK 1974058 T3 | 01-09-2014 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 21 15 1054

This annex lists the patent family members relating to the patent documents cited in the above-mentioned European search report. The members are as contained in the European Patent Office EDP file on
The European Patent Office is in no way liable for these particulars which are merely given for the purpose of information.

22-06-2021

| Patent document cited in search report | Publication date | Patent family member(s) | Publication date |
|---|---|---|---|
| | | EP 1974058 A2 | 01-10-2008 |
| | | EP 2377950 A1 | 19-10-2011 |
| | | EP 2400036 A1 | 28-12-2011 |
| | | EP 2407553 A1 | 18-01-2012 |
| | | EP 2412820 A1 | 01-02-2012 |
| | | EP 2412821 A1 | 01-02-2012 |
| | | EP 2412822 A1 | 01-02-2012 |
| | | EP 2412823 A1 | 01-02-2012 |
| | | EP 2412824 A1 | 01-02-2012 |
| | | ES 2491222 T3 | 05-09-2014 |
| | | HK 1120089 A1 | 20-03-2009 |
| | | IL 192794 A | 31-07-2013 |
| | | JP 5297202 B2 | 25-09-2013 |
| | | JP 5486664 B2 | 07-05-2014 |
| | | JP 5486717 B2 | 07-05-2014 |
| | | JP 5486718 B2 | 07-05-2014 |
| | | JP 5486719 B2 | 07-05-2014 |
| | | JP 5486720 B2 | 07-05-2014 |
| | | JP 2009523028 A | 18-06-2009 |
| | | JP 2013074894 A | 25-04-2013 |
| | | JP 2013176398 A | 09-09-2013 |
| | | JP 2013215201 A | 24-10-2013 |
| | | JP 2013223503 A | 31-10-2013 |
| | | JP 2013226150 A | 07-11-2013 |
| | | JP 2013226151 A | 07-11-2013 |
| | | KR 20090003178 A | 09-01-2009 |
| | | NZ 569788 A | 26-08-2011 |
| | | NZ 593224 A | 26-10-2012 |
| | | NZ 593225 A | 26-10-2012 |
| | | NZ 593226 A | 26-10-2012 |
| | | NZ 593227 A | 26-10-2012 |
| | | NZ 593228 A | 26-10-2012 |
| | | NZ 593229 A | 26-10-2012 |
| | | US 2009291434 A1 | 26-11-2009 |
| | | US 2010190173 A1 | 29-07-2010 |
| | | US 2011039269 A1 | 17-02-2011 |
| | | US 2011039270 A1 | 17-02-2011 |
| | | US 2011039271 A1 | 17-02-2011 |
| | | US 2011039272 A1 | 17-02-2011 |
| | | US 2011097759 A1 | 28-04-2011 |
| | | US 2011111421 A1 | 12-05-2011 |
| | | US 2012171688 A1 | 05-07-2012 |
| | | US 2013102492 A1 | 25-04-2013 |
| | | WO 2007082099 A2 | 19-07-2007 |
| WO 2016109546 A2 | 07-07-2016 | CN 107208138 A | 26-09-2017 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 21 15 1054

This annex lists the patent family members relating to the patent documents cited in the above-mentioned European search report.
The members are as contained in the European Patent Office EDP file on
The European Patent Office is in no way liable for these particulars which are merely given for the purpose of information.

22-06-2021

| Patent document cited in search report | Publication date | Patent family member(s) | Publication date |
|---|---|---|---|
| | | EP 3240908 A2 | 08-11-2017 |
| | | JP 2018503373 A | 08-02-2018 |
| | | US 2017260594 A1 | 14-09-2017 |
| | | WO 2016109546 A2 | 07-07-2016 |
| EP 3636779 A1 | 15-04-2020 | EP 3636779 A1 | 15-04-2020 |
| | | WO 2020074679 A1 | 16-04-2020 |
| WO 2019028285 A2 | 07-02-2019 | AU 2018310987 A1 | 27-02-2020 |
| | | CA 3072061 A1 | 07-02-2019 |
| | | EP 3662082 A2 | 10-06-2020 |
| | | WO 2019028285 A2 | 07-02-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Cancer Genome Atlas Network, ''Comprehensive molecular characterization of human colon and rectal cancer. Nature. 2012, vol. 487, 330-337
- **LIU Y. et al.** Comparative molecular analysis of gastrointestinal adenocarcinomas. *Cancer Cell,* 2018, vol. 33 (4), 721-735
- **MANTOVANI A. et al.** Cancer-related inflammation. *Nature,* 2008, vol. 454 (7203), 436-444
- **GIRALDO N.A. et al.** The clinical role of the TME in solid cancer. *Br J Cancer,* 2019, vol. 120 (1), 45-53
- **SHIAO S.L. et al.** Regulation of prostate cancer progression by tumor microenvironment. *Cancer Lett,* 2016, vol. 380 (1), 340-348
- **BARKER H.E. et al.** The tumor microenvironment after radiotherapy: Mechanisms of resistance or recurrence. *Nat Rev Cancer,* 2015, vol. 15 (7), 409-425
- **GASSER S. et al.** Sensing of dangerous DNA. *Mechanisms of Aging and Development,* 2017, vol. 165, 33-46
- **MOSENDEN R. ; TASKEN K.** Cyclic AMP-mediated immune regulation - Overview of mechanisms of action in T-cells. *Cell Signal,* 2011, vol. 23 (6), 1009-1016
- **TASKEN K. ; RUPPELT A.** Negative regulation of T-cell receptor activation by the cAMP-PKA-Csk signaling pathway in T-cell lipid rafts. *Front Biosci,* 2006, vol. 11, 2929-2939
- **ABRAHAMSEN H. et al.** TCR- and CD28-mediated recruitment of phosphodiesterase 4 to lipid rafts potentiates TCR signaling. *J Immunol,* 2004, vol. 173, 4847-4848
- Immunity Leashed - Mechanisms of Regulation in the Human Immune System. **TORHEIM E.A.** Thesis for the degree of Philosophiae Doctor (PhD). The Biotechnology Centre of Ola, University of Oslo, 2009
- **ALVES DE INDA M. et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus,* 2018, vol. 4 (3), 376-384